(19) Europäisches Patentamt
European Patent Office
Office européen des brevets

(11) EP 4 755 885 A1

# (12) EUROPEAN PATENT APPLICATION

published in accordance with Art. 153(4) EPC

(43) Date of publication:
**10.06.2026 Bulletin 2026/24**

(21) Application number: **24848225.9**

(22) Date of filing: **29.07.2024**

(51) International Patent Classification (IPC):
***C07D 471/04*** *(2006.01)* ***C07D 498/22*** *(2006.01)*
***A61K 31/437*** *(2006.01)* ***A61K 31/4709*** *(2006.01)*
***A61P 1/16*** *(2006.01)* ***A61P 3/04*** *(2006.01)*
***A61P 3/10*** *(2006.01)*

(52) Cooperative Patent Classification (CPC):
**A61K 31/437; A61K 31/4709; A61K 31/496; A61K 31/5355; A61K 31/5386; A61K 31/675; A61P 1/16; A61P 3/04; A61P 3/10; A61P 29/00; C07D 471/04; C07D 471/10; C07D 471/18; C07D 498/18; C07D 498/22;** (Cont.)

(86) International application number:
**PCT/CN2024/108192**

(87) International publication number:
**WO 2025/026270 (06.02.2025 Gazette 2025/06)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority:
**28.07.2023 CN 202310946477**
**25.08.2023 CN 202311087184**
**30.08.2023 CN 202311110642**
**27.09.2023 CN 202311269491**
**20.10.2023 CN 202311372605**
**17.11.2023 CN 202311546779**
**08.12.2023 CN 202311690443**
**29.12.2023 CN 202311866263**
**31.01.2024 CN 202410140555**
**01.03.2024 CN 202410241733**
**29.03.2024 CN 202410385768**
**11.05.2024 CN 202410587643**
**02.07.2024 CN 202410882329**

(71) Applicants:
- **Shanghai Hansoh Biomedical Co., Ltd.**
  **Shanghai 201203 (CN)**
- **Jiangsu Hansoh Pharmaceutical Group Co., Ltd.**
  **Lianyungang, Jiangsu 222047 (CN)**

(72) Inventors:
- **MAO, Xiaofeng**
  **Shanghai 201203 (CN)**
- **WANG, Jun**
  **Shanghai 201203 (CN)**
- **SU, Yidong**
  **Shanghai 201203 (CN)**
- **YU, Wensheng**
  **Shanghai 201203 (CN)**

(74) Representative: **Bradley, Josephine Mary et al**
**Kilburn & Strode LLP**
**Lacon London**
**84 Theobalds Road**
**London WC1X 8NL (GB)**

## (54) BENZO NITROGEN-CONTAINING HETEROCYCLIC DERIVATIVE REGULATOR, PREPARATION METHOD THEREFOR AND USE THEREOF

(57) A benzo nitrogen-containing heterocyclic derivative regulator, a preparation method therefor and a use thereof. Particularly, the present invention relates to a compound represented by general formula (XIX), a preparation method therefor, a pharmaceutical composition containing the compound, and a use of the compound as a regulator in the preparation of drugs for treating metabolic diseases and related diseases, wherein each substituent in the general formula (XIX) is the same as the definition in the description.

EP 4 755 885 A1

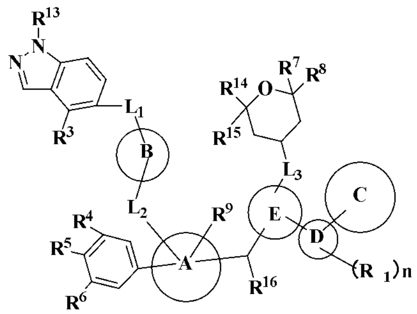

(XIX)

(52) Cooperative Patent Classification (CPC): (Cont.)
**C07D 519/00; C07F 9/6561; C07F 9/6568**

## Description

### Technical Field

The present invention belongs to the field of medicine and biology, and specifically relates to a benzene nitrogen-containing heterocyclic derivative regulator, and a preparation method and an application thereof.

### Background Art

Diabetes mellitus is a common endocrine metabolic disease. It is caused by metabolic disorders due to various reasons, leading to multi-system and multi-organ damage. Its incidence is high. There are about 425 million diabetic patients worldwide. The incidence of diabetes in China is about 10%, of which type 2 diabetes accounts for 90%. The incidence is still increasing, and the age of patients is getting younger.

Currently, there are many types of drugs on the market for the treatment of type II diabetes, including insulin, biguanides, glucagon-like peptide 1 (GLP-1) receptor agonists, dipeptidyl peptidase (DPP-IV) inhibitors, sodium-glucose cotransporter 2 (SGLT-2) inhibitors, $\alpha$-glucosidase inhibitors, etc., among which GLP-1 receptor agonists are the most popular.

GLP-1 is a peptide hormone secreted by human intestinal L cells. Its receptors are distributed in pancreatic islet cells, various gastrointestinal cells, central nervous system, and peripheral nervous system neurons. After GLP-1 receptor activation, it has physiological effects such as promoting insulin secretion, inhibiting glucagon secretion, suppressing appetite, and delaying gastric emptying. Clinical evidence shows that compared with other hypoglycemic drugs, GLP-1 receptor agonists have better hypoglycemic effects and are less likely to cause side effects such as hypoglycemia. In addition, it also has additional cardiovascular benefits, and can reduce food intake and delay gastric emptying, which is beneficial for weight control.

Currently, all GLP-1 receptor agonists on the market are peptide drugs, most of which require subcutaneous administration, and patients' compliance is poor, while the bioavailability of oral peptides is very low. Therefore, there is a great clinical demand for the development of oral small molecule GLP-1 receptor agonists.

Currently, no small molecule GLP-1 receptor agonists have been approved, and there is a huge clinical demand. Oral small molecule GLP-1 receptor agonists with lower cost and better compliance have the potential to treat a variety of metabolic diseases and thus have broad market prospects.

### Summary of the Invention

An object of the present invention is to provide a compound represented by general formula (XIX), a prodrug thereof, a stereoisomer thereof, or a pharmaceutically acceptable salt thereof:

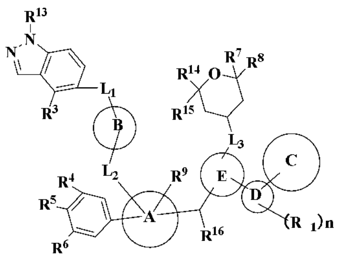

(XIX)

wherein,

$R^{13}$ is selected from hydrogen, deuterium, halogen, amino, nitro, hydroxyl, cyano, oxo, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl, the amino, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl, which are optionally further substituted by one or more substituents selected from deuterium, halogen, amino, nitro, hydroxyl, cyano, oxo, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated

alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

$L_1$, $L_2$ or $L_3$ are each independently selected from a bond, $C_{2-4}$ alkenylene, $C_{2-4}$ alkynylene or $-(CH_2)_{n1}-$;

ring A is selected from $C_{3-14}$ cycloalkyl, 3-14 membered heterocyclyl, $C_{6-14}$ aryl or 5-14 membered heteroaryl;

ring B is selected from $C_{3-12}$ cycloalkyl, 3-12 membered heterocyclyl, $C_{6-14}$ aryl or 5-14 membered heteroaryl, which are optionally further substituted with one or more substituents selected from deuterium, halogen, amino, nitro, hydroxyl, cyano, oxo, thiol, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy or $C_{1-6}$ deuterated haloalkoxy;

ring C is selected from $C_{3-14}$ cycloalkyl, 3-14 membered heterocyclyl, $C_{6-14}$ aryl or 5-14 membered heteroaryl, which are optionally further substituted by one or more substituents selected from deuterium, halogen, amino, nitro, hydroxyl, cyano, oxo, thiol, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy or $C_{1-6}$ deuterated haloalkoxy;

ring D is selected from $C_{3-12}$ cycloalkyl, 3-12 membered heterocyclyl, $C_{6-14}$ aryl or 5-14 membered heteroaryl;

ring E is selected from $C_{3-14}$ cycloalkyl, 3-14 membered heterocyclyl, $C_{6-14}$ aryl or 5-14 membered heteroaryl, which are optionally further substituted with one or more substituents selected from deuterium, halogen, amino, nitro, hydroxy, cyano, oxo, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

alternatively, ring E is

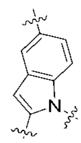,

which is optionally further substituted by one or more substituents selected from methyl, ethyl, methoxy or ethoxy;

$R^1$, $R^3$, $R^4$, $R^5$, $R^6$, $R^7$, $R^8$, $R^9$, $R^{14}$, $R^{15}$ or $R^{16}$ is each independently selected from hydrogen, deuterium, halogen, amino, nitro, hydroxyl, cyano, oxo, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, C3-8 cycloalkyl, 3-8 membered heterocyclyl, C6-10 aryl, 5-10 membered heteroaryl, $-(CH_2)_{m1}OR_{c1}$ or $(=C)R_{c3}R_{c4}$, the amino, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl, 5-10 membered heteroaryl, $-(CH_2)_{m1}OR_{c1}$ or $(=C)R_{c3}R_{c4}$ are optionally further substituted by one or more of deuterium, halogen, amino, nitro, hydroxyl, cyano, oxo, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

$R_{c1}$, $R_{c3}$ or $R_{c4}$ is each independently selected from hydrogen, deuterium, halogen, amino, nitro, hydroxyl, cyano, oxo, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl, 5-10 membered heteroaryl, $-(CH_2)_{m2}OR_{c2}$, $-(CH_2)_{m3}C(O)NR_{a1}R_{b1}$ or $-(CH_2)_{m4}C(O)OR_{a2}$, the amino, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, C3-8 cycloalkyl, 3-8 membered heterocyclic, C6-10 aryl, 5-10 membered heteroaryl, $-(CH_2)_{m2}OR_{c2}$, $-(CH_2)_{m3}C(O)NR_{a1}R_{b1}$ or $-(CH_2)_{m4}C(O)OR_{a2}$, are optionally further substituted by one or more of deuterium, halogen, amino, nitro, hydroxyl, cyano, oxo, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

$R_{a1}$, $R_{a2}$, $R_{b1}$ or $R_{c2}$ is each independently selected from hydrogen, deuterium, halogen, amino, nitro, hydroxyl, cyano, oxo, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated

hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, the amino, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl are optionally further substituted by one or more of deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

alternatively, $R^9$ or $R^{16}$ are respectively linked to atoms connected thereto to form $C_{3-10}$ cycloalkyl, 3-10 membered heterocyclic, $C_{6-12}$ aryl or 5-12 membered heteroaryl;

alternatively, when n is not 1,

$R^1$ and $R^1$, $R^2$ and $R^1$, $R^4$ and $R^5$, $R^5$ and $R^6$, $R^7$ and $R^8$ are linked to the atoms connected thereto to form $C_{3-10}$ cycloalkyl, 3-10 membered heterocyclic, $C_{6-12}$ aryl or 5-12 membered heteroaryl, which are optionally further substituted by one or more substituents selected from deuterium, halogen, amino, nitro, hydroxyl, cyano, oxo, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

n, n1, m1, m2, m3 or m4 is each independently selected from 0, 1, 2, 3, 4 or 5.

In a further preferred embodiment of the present invention, the compound of general formula (XIX) is as follows:

$R^{13}$ is selected from hydrogen, deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl or 5-8 membered heteroaryl, the amino, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl or 5-8 membered heteroaryl, which are optionally further substituted by one or more substituents selected from deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl or 5-8 membered heteroaryl;

Preferably, $R^{13}$ is selected from $-CD_3$, hydrogen, deuterium, fluorine, chlorine, bromine, methyl or cyclopropyl, which are optionally further substituted by one or more substituents selected from deuterium, halogen, or $C_{1-3}$ alkyl.

In a further preferred embodiment of the present invention, the compound of general formula (XIX) is as follows:

Ring A is selected from $C_{3-12}$ cycloalkyl, 3-12 membered heterocyclic containing 1-3 atoms selected from N, O or S, $C_{6-12}$ aryl or 5-12 membered heteroaryl containing 1-3 atoms selected from N, O or S;

Preferably, ring A is selected from $C_{6-12}$ cycloalkyl, 6-12 membered heterocyclic containing 1-3 N, O or S atoms, or 6-12 membered heteroaryl containing 1-3 N, O or S atoms;

More preferably, ring A is selected from

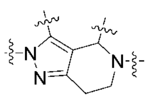 、 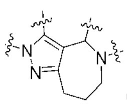 or 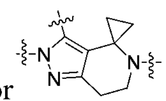 .

In a further preferred embodiment of the present invention, the compound of general formula (XIX) is as follows:

Ring B is selected from $C_{3-8}$ cycloalkyl, 3-8 membered heterocyclic containing 1-3 atoms selected from N, O or S, $C_{6-10}$ aryl or 5-10 membered heteroaryl containing 1-3 atoms selected from N, O or S; optionally further substituted by one or more substituents selected from deuterium, halogen, amino, oxo, thiol, thio or $C_{1-3}$ alkyl;

Preferably, ring B is selected from $C_{3-6}$ cycloalkyl, 3-6 membered heterocyclic containing 1-3 N, O or S atoms, or 5-8 membered heteroaryl containing 1-3 N, O or S atoms; optionally further substituted by one or more substituents selected from deuterium, halogen, amino, oxo, thio or $C_{1-3}$ alkyl;

More preferably, ring B is selected from

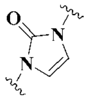 、 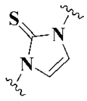 or 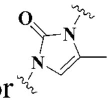 ,

optionally further substituted by one or more substituents selected from deuterium, halogen, amino, oxo, thio or $C_{1-3}$ alkyl.

In a further preferred embodiment of the present invention, the compound of general formula (XIX) is as follows:

Ring C is selected from $C_{3-8}$ cycloalkyl, 3-8 membered heterocyclic containing 1-3 atoms selected from N, O or S, $C_{6-10}$ aryl or 5-8 membered heteroaryl containing 1-3 atoms selected from N, O or S; optionally further substituted by one or more substituents selected from deuterium, halogen, amino, nitro, hydroxyl, cyano, oxo, thiol, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy or $C_{1-3}$ deuterated haloalkoxy;
Preferably, ring C is selected from $C_{3-8}$ cycloalkyl, 3-8 membered heterocyclic containing 1-3 atoms selected from N, O or S, or 5-8 membered heteroaryl containing 1-3 atoms selected from N, O or S; optionally further substituted by one or more substituents selected from deuterium, hydroxyl, thioxo, oxo or $C_{1-3}$ haloalkoxy;
More preferably, ring C is selected from

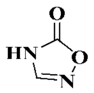 、 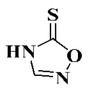 、 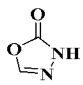 、 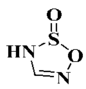 、 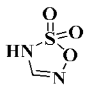 、

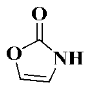 or 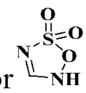 ;

optionally further substituted by one or more substituents selected from deuterium, hydroxyl, thio or oxo.

In a further preferred embodiment of the present invention, the compound of general formula (XIX) is as follows:

Ring D is selected from $C_{3-8}$ cycloalkyl, 3-8 membered heterocyclyl containing 1-3 atoms selected from N, O or S, $C_{6-10}$ aryl or 5-10 membered heteroaryl containing 1-3 atoms selected from N, O or S;
Preferably, ring D is selected from $C_{3-6}$ cycloalkyl, 3-6 membered heterocyclyl containing 1-3 N, O or S atoms, or 5-8 membered heteroaryl containing 1-3 N, O or S atoms;
More preferably, ring D is selected from

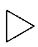 、 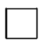 、 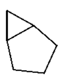 or 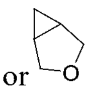 .

In a further preferred embodiment of the present invention, the compound of general formula (XIX) is as follows:

Ring E is selected from $C_{3-8}$ cycloalkyl, 3-10 membered heterocyclic containing 1-3 atoms selected from N, O or S, C6-10 aryl or 5-10 membered heteroaryl containing $_{1-3}$ atoms selected from N, O or S, which are optionally further substituted by one or more substituents selected from deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl, or 5-8 membered heteroaryl;

Preferably, ring E is selected from

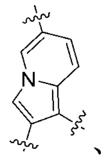 、 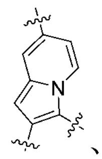 、 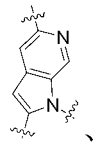 、 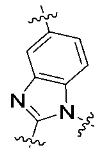 、 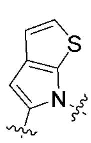 or 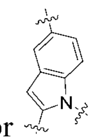,

optionally substituted with one or more substituents selected from methyl, ethyl, methoxy or ethoxy.

In a further preferred embodiment of the present invention, the compound of general formula (XIX) is as follows:

$R^1$, $R^3$, $R^4$, $R^5$, $R^6$, $R^7$, $R^8$, $R^9$, $R^{14}$, $R^{15}$ or $R^{16}$ is each independently selected from hydrogen, deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, $_{3-6}$ membered heterocyclyl, C6-10 aryl, 5-8 membered heteroaryl, $-(CH_2)_{m1}OR_{c1}$ or $(=C)R_{c3}R_{c4}$, the amino, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, $_{3-6}$ membered heterocyclic, $C_{6-10}$ aryl, 5-10 membered heteroaryl, $-(CH_2)_{m1}OR_{c1}$ or $(=C)R_{c3}R_{c4}$ are optionally further substituted by one or more of deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl;

Preferably, $R^1$, $R^3$, $R^4$, $R^5$, $R^6$, $R^7$, $R^8$, $R^9$, $R^{14}$, $R^{15}$ or $R^{16}$ is each independently selected from hydrogen, deuterium, halogen, amino, nitro, hydroxyl, cyano, oxo, thio, $C_{2-4}$ alkynyl, C3-6 cycloalkyl, $C_{1-3}$ alkyl, $C_{1-3}$ deuterated alkyl, $C_{1-3}$ haloalkyl, $-(CH_2)_{m1}OR_{c1}$ or $(=C)R_{c3}R_{c4}$, the amino, $C_{2-4}$ alkynyl, C3-6 cycloalkyl, $C_{1-3}$ alkyl, $C_{1-3}$ deuterated alkyl, $C_{1-3}$ haloalkyl, - $(CH_2)_{m1}OR_{c1}$ or $(=C)R_{c3}R_{c4}$ are optionally further substituted by one or more substituents selected from deuterium, amino, halogen, deuterated methyl, hydroxyl, methyl, cyclopropyl, oxo, thio, $C_{1-3}$ alkyl or $C_{1-3}$ alkoxy;

More preferably, $R^1$, $R^3$, $R^4$, $R^5$, $R^6$, $R^7$, $R^8$, $R^9$, $R^{14}$, $R^{15}$ or $R^{16}$ is each independently selected from hydrogen, oxo, thio, cyano, methyl, fluorine, chlorine, cyclopropyl, ethynyl, - $(CH_2)_{m1}OR_{c1}$ or $(=C)R_{c3}R_{c4}$, the cyclopropyl, ethynyl, $-(CH_2)_{m1}OR_{c1}$ or $(=C)R_{c3}R_{c4}$, are optionally further substituted by one or more substituents in deuterium, fluorine, chlorine, bromine, hydroxyl, $-CD_3$, methyl, ethyl, cyclopropyl, oxo, thio or

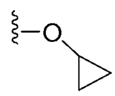.

In a further preferred embodiment of the present invention, the compound of general formula (XIX) is as follows:

$R_{c1}$, $R_{c3}$ or $R_{c4}$ is selected from hydrogen, deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclyl, $C_{6-10}$ aryl, 5-10 membered heteroaryl, -$(CH_2)_{m2}OR_{c2}$, $-(CH_2)_{m3}C(O)NR_{a1}R_{b1}$ or $-(CH_2)_{m4}C(O)OR_{a2}$, the amino, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, $_{3-6}$ membered heterocyclic, C6-10 aryl, 5-8 membered heteroaryl, $-(CH_2)_{m2}OR_{c2}$, $-(CH_2)_{m3}C(O)NR_{a1}R_{b1}$ or $-(CH_2)_{m4}C(O)OR_{a2}$, are optionally further substituted by one or more of deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl;

Preferably, $R_{c1}$, $R_{c3}$ or $R_{c4}$ is selected from fluorine, methyl, cyano, -CH2F, difluoromethyl, trifluoromethyl, ethynyl, methylthio, amino, cyclopropyl, oxetane, phenyl, - $(CH_2)_{m2}OR_{c2}$, $-(CH_2)_{m3}C(O)NR_{a1}R_{b1}$ or $-(CH_2)_{m4}C(O)OR_{a2}$, the amino, methyl, $-CH_2F$, difluoromethyl, trifluoromethyl, ethynyl, methylthio, cyclopropyl, oxetane, phenyl, -$(CH_2)_{m2}OR_{c2}$, $-(CH_2)_{m3}C(O)NR_{a1}R_{b1}$ or $-(CH_2)_{m4}C(O)OR_{a2}$ are optionally further substituted by one or more substituents of deuterium, halogen, methoxy, deuterated methyl or hydroxyl.

In a further preferred embodiment of the present invention, the compound of general formula (XIX) is as follows:

$R_{a1}$, $R_{a2}$, $R_{b1}$ or $R_{c2}$ is each independently selected from hydrogen, deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, $_{3-6}$ membered heterocyclyl, $C_{6-10}$ aryl or 5-10 membered heteroaryl, the amino, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl or 5-8 membered heteroaryl are optionally further substituted by one or more of deuterium, halogen, amino, nitro, hydroxyl, cyano, oxo, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl;

Preferably, $R_{a1}$, $R_{a2}$, $R_{b1}$ or $R_{c2}$ is each independently selected from fluorine, methyl, ethyl, $-CH_2F$, cyano, difluoromethyl, trifluoromethyl, ethynyl, methylthio, amino, cyclopropyl, oxetane or phenyl, the amino, methyl, ethyl, $-CH_2F$, difluoromethyl, trifluoromethyl, ethynyl, methylthio, cyclopropyl, oxetane or phenyl are optionally further substituted by one or more substituents of deuterium, halogen, methoxy, deuterated methyl or hydroxyl.

In a further preferred embodiment of the present invention, the compound of general formula (XIX) is as follows:

$R^9$ or $R^{16}$ are respectively linked to atoms connected thereto to form $C_{3-6}$ cycloalkyl, 3-6 membered heterocyclic containing 1-3 atoms selected from N, O or S, $C_{6-10}$ aryl, or 5-8 membered heteroaryl containing 1-3 atoms selected from N, O or S;

Preferably, $R^9$ or $R^{16}$ are linked to the atoms connected thereto to form cyclopropyl.

In a further preferred embodiment of the present invention, the compound of general formula (XIX) is as follows:

$R^1$ and $R^1$, $R^2$ and $R^1$, $R^4$ and $R^5$, $R^5$ and $R^6$, $R^7$ and $R^8$ are linked to the atoms connected thereto to form $C_{3-10}$ cycloalkyl, 3-6 membered heterocyclic, $C_{6-12}$ aryl or 5-12 membered heteroaryl, which are optionally further substituted by one or more substituents selected from deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl;

Preferably, $R^1$ and $R^1$, $R^2$ and $R^1$, $R^4$ and $R^5$, $R^5$ and $R^6$, $R^7$ and $R^8$ are linked to the atoms connected thereto to form cyclopropyl,

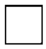、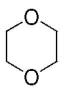、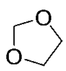、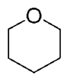、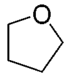、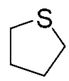 or 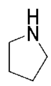,

optionally further substituted with one or more substituents selected from deuterium, fluorine, chlorine, bromine, amino, nitro, hydroxy, cyano, oxo, thioxo, methyl or ethyl.

An object of the present invention is to provide a compound represented by general formula (XVIII), a prodrug thereof, a stereoisomer thereof or a pharmaceutically acceptable salt thereof:

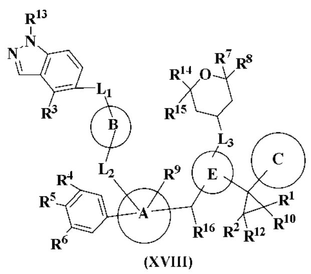

(XVIII)

wherein,

$R^1$, $R^2$, $R^3$, $R^4$, $R^5$, $R^6$, $R^7$, $R^8$, $R^9$, $R^{10}$, $R^{12}$, $R^{14}$, $R^{15}$ or $R^{16}$ is each independently selected from hydrogen, deuterium, halogen, amino, nitro, hydroxyl, cyano, oxo, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, $_{3-8}$ membered heterocyclyl, $C_{6-10}$ aryl, 5-8 $-(CH_2)_{m1}OR_{c1}$, the amino, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl, 5-10 membered heteroaryl or $-(CH_2)_{m1}OR_{c1}$, are optionally further substituted by one or more substituents selected from deuterium, halogen, amino, nitro, hydroxyl, cyano, oxo, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

$R_{c1}$ is selected from hydrogen, deuterium, halogen, amino, nitro, hydroxyl, cyano, oxo, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl, 5-10 membered heteroaryl, $-(CH_2)_{m2}OR_{c2}$, $-(CH_2)_{m3}C(O)NR_{a1}R_{b1}$ or $-(CH_2)_{m4}C(O)OR_{a2}$, the amino, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, $_{3-8}$ membered heterocyclic, C6-10 aryl, 5-8 membered heteroaryl, $-(CH_2)_{m2}OR_{c2}$, $-(CH_2)_{m3}C(O)NR_{a1}R_{b1}$ or $-(CH_2)_{m4}C(O)OR_{a2}$ are optionally further substituted by one or more substituents selected from deuterium, halogen, amino, nitro, hydroxyl, cyano, oxo, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

$R_{a1}$, $R_{a2}$, $R_{b1}$ or $R_{c2}$ are each independently selected from hydrogen, deuterium, halogen, amino, nitro, hydroxyl, cyano, oxo, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, $_{3-8}$ membered heterocyclyl, $C_{6-10}$ aryl or 5-8 membered heteroaryl, the amino, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, C6-10 aryl or 5-10 membered heteroaryl are optionally further substituted with one or more substituents selected from deuterium, halogen, amino, nitro, hydroxyl, cyano, oxo, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

Alternatively, $R^9$ or $R^{16}$ are linked to the atoms connected thereto to form a $C_{3-10}$ cycloalkyl, a 3-10 membered heterocyclic, a $C_{6-12}$ aryl or a 5-12 membered heteroaryl;

$R^1$ and $R^{10}$, $R^2$ and $R^{12}$, $R^4$ and $R^5$, $R^5$ and $R^6$, $R^7$ and $R^8$, $R^{10}$ and $R^{12}$ are linked to the atoms connected thereto to form $C_{3-10}$ cycloalkyl, 3-10 membered heterocyclyl, $C_{6-12}$ aryl or 5-12 membered heteroaryl, which are optionally further substituted by one or more substituents selected from deuterium, halogen, amino, nitro, hydroxyl, cyano, oxo, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

$R^{13}$, $L_1$, $L_2$, $L_3$, ring A, ring B, ring C, ring E are as set forth in general formula (XIX);

n1, m1, m2, m3 or m4 is each independently selected from 0, 1, 2, 3, 4 or 5.

In a further preferred embodiment of the present invention, the compound of general formula (XVIII) is as follows:

$R^1$, $R^2$, $R^3$, $R^4$, $R^5$, $R^6$, $R^7$, $R^8$, $R^9$, $R^{10}$, $R^{12}$, $R^{14}$, $R^{15}$ or $R^{16}$ is each independently selected from hydrogen, deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6

membered heterocyclyl, $C_{6-10}$ aryl, 5-8 -$(CH_2)_{m1}OR_{c1}$, the amino, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl, 5-8 membered heteroaryl or -$(CH_2)_{m1}OR_{c1}$, are optionally further substituted by one or more substituents selected from deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl;

Preferably, $R^1$, $R^2$, $R^3$, $R^4$, $R^5$, $R^6$, $R^7$, $R^8$, $R^9$, $R^{10}$, $R^{12}$, $R^{14}$, $R^{15}$ or $R^{16}$ is each independently selected from hydrogen, deuterium, halogen, amino, nitro, hydroxyl, cyano, oxo, thio, $C_{2-4}$ alkynyl, $C_{3-6}$ cycloalkyl, $C_{1-3}$ alkyl, $C_{1-3}$ deuterated alkyl, $C_{1-3}$ haloalkyl or - $(CH_2)_{m1}OR_{c1}$, the amino, $C_{2-4}$ alkynyl, $C_{3-6}$ cycloalkyl, $C_{1-3}$ alkyl, $C_{1-3}$ deuterated alkyl, $C_{1-3}$ haloalkyl or -$(CH_2)_{m1}OR_{c1}$ are optionally further substituted by one or more substituents selected from deuterium, amino, halogen, deuterated methyl, hydroxyl, methyl, cyclopropyl, oxo, thio, $C_{1-3}$ alkyl or $C_{1-3}$ alkoxy;

Further preferably, $R^1$, $R^2$, $R^3$, $R^4$, $R^5$, $R^6$, $R^7$, $R^8$, $R^9$, $R^{10}$, $R^{12}$, $R^{14}$, $R^{15}$ or $R^{16}$ is each independently selected from hydrogen, oxo, thio, cyano, methyl, fluorine, chlorine, cyclopropyl, ethynyl or -$(CH_2)_{m1}OR_{c1}$, the cyclopropyl, ethynyl or -$(CH_2)_{m1}OR_{c1}$ are optionally further substituted by one or more substituents in deuterium, fluorine, chlorine, bromine, hydroxyl, - $CD_3$, methyl, ethyl, cyclopropyl, oxo, thio or

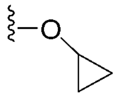.

In a further preferred embodiment of the present invention, the compound of general formula (XVIII) is as follows:

$R_{c1}$ is selected from hydrogen, deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclyl, $C_{6-10}$ aryl, 5-8 membered heteroaryl, -$(CH_2)_{m2}OR_{c2}$,-$(CH_2)_{m3}C(O)NR_{a1}R_{b1}$ or -$(CH_2)_{m4}C(O)OR_{a2}$, the amino, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, C3-6 cycloalkyl, 3-6 membered heterocyclic, C6-10 aryl, 5-8 membered heteroaryl, -$(CH_2)_{m2}OR_{c2}$, -$(CH_2)_{m3}C(O)NR_{a1}R_{b1}$ or-$(CH_2)_{m4}C(O)OR_{a2}$ are optionally further substituted by one or more substituents selected from deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl;

Preferably, $R_{c1}$ is selected from fluorine, methyl, cyano, -CH2F, difluoromethyl, trifluoromethyl, ethynyl, methylthio, amino, cyclopropyl, oxetane, phenyl, -$(CH_2)_{m2}OR_{c2}$,-$(CH_2)_{m3}C(O)NR_{a1}R_{b1}$ or -$(CH_2)_{m4}C(O)OR_{a2}$, the amino, methyl, -$CH_2F$, difluoromethyl, trifluoromethyl, ethynyl, methylthio, cyclopropyl, oxetane, phenyl, -$(CH_2)_{m2}OR_{c2}$,-$(CH_2)_{m3}C(O)NR_{a1}R_{b1}$ or -$(CH_2)_{m4}C(O)OR_{a2}$ are optionally further substituted by one or more substituents of deuterium, halogen, methoxy, deuterated methyl or hydroxyl.

In a further preferred embodiment of the present invention, the compound of general formula (XVIII) is as follows:

$R_{a1}$, $R_{a2}$, $R_{b1}$ or $R_{c2}$ are each independently selected from hydrogen, deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl, the amino, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, C3-6 cycloalkyl, 3-6 membered heterocyclic, C6-10 aryl or 5-8 membered heteroaryl are optionally further substituted with one or more substituents selected from deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl;

Preferably, $R_{a1}$, $R_{a2}$, $R_{b1}$ or $R_{c2}$ are each independently selected from fluorine, methyl, ethyl, -$CH_2F$, cyano,

difluoromethyl, trifluoromethyl, ethynyl, methylthio, amino, cyclopropyl, oxetane or phenyl, the methyl, ethyl, $-CH_2F$, difluoromethyl, trifluoromethyl, ethynyl, methylthio, amino, cyclopropyl, oxetane or phenyl are optionally further substituted by one or more substituents of deuterium, halogen, methoxy, deuterated methyl or hydroxyl.

In a further preferred embodiment of the present invention, the compound of general formula (XVIII) is as follows:

$R^9$ or $R^{16}$ are respectively linked to atoms connected thereto to form $C_{3-6}$ cycloalkyl, 3-6 membered heterocyclic containing 1-3 atoms selected from N, O or S, $C_{6-10}$ aryl, or 5-8 membered heteroaryl containing 1-3 atoms selected from N, O or S;
Preferably, $R^9$ or $R^{16}$ are linked to the atoms connected thereto to form cyclopropyl.

In a further preferred embodiment of the present invention, the compound of general formula (XVIII) is as follows:

$R^1$ and $R^{10}$, $R^2$ and $R^{12}$, $R^4$ and $R^5$, $R^5$ and $R^6$, $R^7$ and $R^8$, $R^{10}$ and $R^{12}$ are linked to the atoms connected thereto to form $C_{3-6}$ cycloalkyl, 3-6 membered heterocyclic containing $_{1-3}$ atoms selected from N, O or S, $C_{6-10}$ aryl or 5-8 membered heteroaryl containing $_{1-3}$ atoms selected from N, O or S, which are optionally further substituted with one or more substituents selected from deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl;
preferably, $R^1$ and $R^{10}$, $R^2$ and $R^{12}$, $R^4$ and $R^5$, $R^5$ and $R^6$, $R^7$ and $R^8$, $R^{10}$ and $R^{12}$ are linked to the atoms connected thereto to form cyclopropyl,

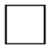、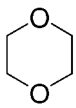、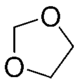、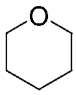

or

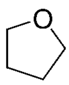,

which are optionally further substituted with one or more substituents selected from deuterium, fluorine, chlorine, bromine, amino, nitro, hydroxyl, cyano, oxo, thioxo, methyl or ethyl.

In a further preferred embodiment of the present invention, the compound of general formula (XVIII) is as follows: $R^1$ and $R^{10}$, $R^2$ and $R^{12}$, $R^4$ and $R^5$, $R^5$ and $R^6$, $R^7$ and $R^8$, $R^{10}$ and $R^{12}$ are linked to the atoms connected thereto to form cyclopropyl,

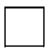、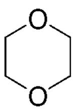、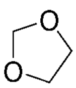、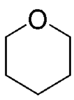、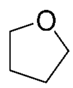、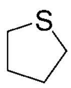,

or

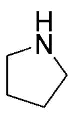,

which are optionally further substituted with one or more substituents selected from deuterium, fluorine, chlorine, bromine, amino, nitro, hydroxyl, cyano, oxo, thioxo, methyl or ethyl.
In a further preferred embodiment of the present invention, the compound of general formula (XVIII) is further shown in general formula (XVIIIA):

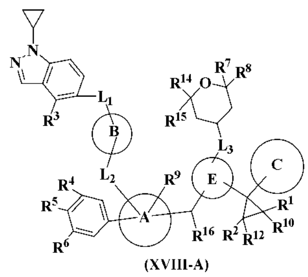

(XVIII-A)

wherein ring A, ring B, ring C, ring E, $R^1$, $R^2$, $R^3$, $R^4$, $R^5$, $R^6$, $R^7$, $R^8$, $R^9$, $R^{10}$, $R^{12}$, $R^{13}$, $R^{14}$, $R^{15}$, $R^{16}$, $L_1$, $L_2$ and $L_3$ are as defined in general formula (XVIII).

In a further preferred embodiment of the present invention, the compound of general formula (XVIII) is further shown in general formula (XVIIIB):

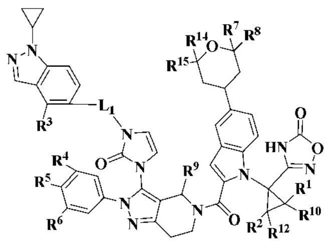

(XVIII-B)

wherein, $R^1$, $R^2$, $R^3$, $R^4$, $R^5$, $R^6$, $R^7$, $R^8$, $R^9$, $R^{10}$, $R^{12}$, $R^{13}$, $R^{14}$, $R^{15}$ and L1 are as defined in general formula (XVIII).

In a further preferred embodiment of the present invention, the compound of general formula (XVIII) is further shown in general formula (XVIII-1):

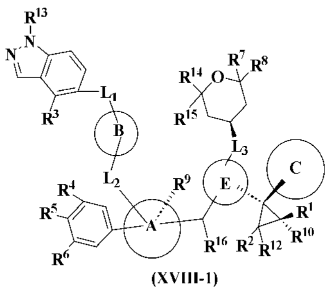

(XVIII-1)

.

In a further preferred embodiment of the present invention, the compound of general formula (XVIII) is further shown in general formula (XVIII-A-1):

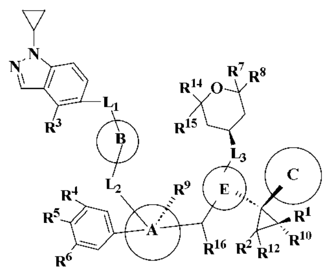

(XVIII-A-1)

.

In a further preferred embodiment of the present invention, the compound of general formula (XVIII) is further shown in general formula (XVIII-B-1):

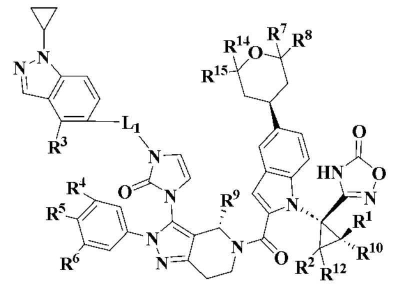

(XVIII-B-1) .

An object of the present invention is to provide a compound represented by general formula (V), a prodrug thereof, a stereoisomer thereof or a pharmaceutically acceptable salt thereof:

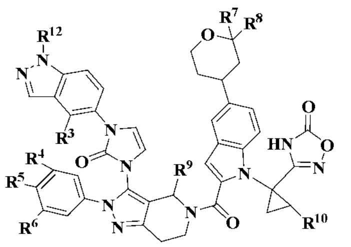

(V)

wherein,

$R^4$, $R^5$ or $R^6$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ sulfanyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, the amino, $C_{1-6}$ alkyl, $C_{1-6}$ deuterated alkyl, $C_{1-6}$ haloalkyl, $C_{1-6}$ hydroxyalkyl, $C_{1-6}$ sulfanyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl and 5-10 membered heteroaryl are optionally further substituted with one or more substituents selected from halogen, amino, nitro, hydroxy, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl and 5-10 membered heteroaryl;

Alternatively, $R^5$ and $R^6$ are linked with adjacent atoms to form a $C_{3-8}$ cycloalkyl, a 3-8 membered heterocyclyl, a $C_{6-10}$ aryl or a 5-10 membered heteroaryl, the $C_{3-8}$ cycloalkyl, 3-8 membered heterocyclyl, $C_{6-10}$ aryl or 5-10 membered heteroaryl are optionally further substituted by one or more substituents of halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ sulfanyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

$R^3$ is selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, 5-10 membered heteroaryl, $-NS(O)R_{a1}R_{b1}$, $-(CH2)_{n1}S(O)NR_{a2}R_{b2}$, $-P(O)R_{a3}R_{b3}$, $-NR_{a5}S(O)NR_{a4}R_{b4}$, $-(CH_2)_{n2}R_{a5}R_{b5}$ or

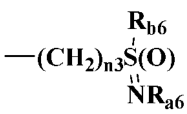

,

the amino, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl are optionally further substituted by one or more substituents selected from halogen, amino, nitro, hydroxy, cyano, oxo, -$NS(O)R_{a1}R_{b1}$, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

$R_{a1}$, $R_{b1}$, $R_{a2}$, $R_{b2}$, $R_{a3}$, $R_{b3}$, $R_{a4}$, $R_{b4}$, $R_{a5}$, $R_{b5}$, $R_{a6}$, or $R_{b6}$ is each independently selected from hydrogen, deuterium, halogen, amino, nitro, hydroxyl, cyano, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, 5-10 membered heteroaryl;

alternatively, $R_{a1}$ and $R_{b1}$, $R_{a2}$ and $R_{b2}$, $R_{a3}$ and $R_{b3}$, $R_{a4}$ and $R_{b4}$, $R_{a5}$ and $R_{b5}$, $R_{a6}$ and $R_{b6}$ are linked to adjacent atoms to form $C_{3-8}$ cycloalkyl, 3-8 membered heterocyclyl, $C_{6-10}$ aryl or 5-10 membered heteroaryl, and the $C_{3-8}$ cycloalkyl, 3-8 membered heterocyclyl, $C_{6-10}$ aryl or 5-10 membered heteroaryl, which are optionally further substituted by one or more substituents of halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

$R^7$, $R^8$, $R^9$ or $R^{10}$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, 5-10 membered heteroaryl or $-(CH_2)_{m1}OR_{c1}$, which are optionally further substituted with one or more substituents selected from halogen, amino, nitro, hydroxy, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

$R_{c1}$ is selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ sulfanyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, or 5-10 membered heteroaryl, the amino, $C_{1-6}$ alkyl, $C_{1-6}$ deuterated alkyl, $C_{1-6}$ haloalkyl, $C_{1-6}$ hydroxyalkyl, $C_{1-6}$ sulfanyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl are optionally further substituted with one or more substituents selected from halogen, amino, nitro, hydroxy, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

$R^{12}$ is selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl, 5-10 membered heteroaryl, $-(CH_2)_{n2}R_{a5}R_{b5}$ or

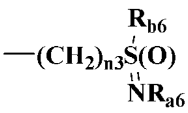,

which are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 $C_{6-10}$ aryl or 5-10 membered heteroaryl;

n1-n3 are integers from 0-5;

m1 is an integer from 0-5;

wherein when $R^7$ and $R^8$ are hydrogen, $R^9$, $R^{10}$, and $R^{12}$ are methyl, and $R^3$ and $R^5$ are fluorine, $R^4$ and $R^6$ are not methyl at the same time;

when $R^7$ and $R^8$ are hydrogen, $R^9$, $R^{10}$, and $R^{12}$ are methyl, $R^3$ is hydrogen, and $R^5$ is halogen, $R^4$ and $R^6$ are not methyl at the same time;

when $R^7$, $R^8$, $R^9$, $R^{10}$, and $R^{12}$ are methyl, $R^5$ is fluorine, and $R^4$ is halogen, trifluoromethyl, or cyclopropane, $R^6$ is not hydrogen;

when $R^7$, $R^8$, $R^9$, $R^{10}$ are methyl, $R^{12}$ is deuterated methyl, $R^3$ is fluorine, $R^5$ is halogen, $R^4$ is deuterated methyl, R6 is not deuterated methyl;

when $R^4$, $R^6$, $R^7$, $R^8$, $R^9$, $R^{10}$ are methyl, $R^3$, $R^5$ are fluorine, $R^{12}$ is not deuterated methyl;

when $R^7$, $R^8$, $R^9$, $R^{10}$, $R^{12}$ are methyl, $R^3$ is fluorine, $R^5$ is halogen, $R^4$ is methyl, $R^6$ is not methyl;

when $R^7$, $R^8$, $R^9$, $R^{10}$ are methyls, $R^{12}$ is deuterated methyl, $R^3$ is fluorine, $R^5$ is halogen, and $R^4$ is deuterated methyl, $R^6$ is not a deuterated methyl;

when $R^4$, $R^6$, $R^7$, $R^8$, $R^9$, $R^{10}$ are methyl, $R^3$ and $R^5$ are fluorine, $R^{12}$ is not deuterated methyl.

In a further preferred embodiment of the present invention, the compound of general formula (V) is as follows:

$R^4$, $R^5$ or $R^6$ is each independently selected from hydrogen, deuterium, halogen, amino, cyano, $C_{1-3}$ alkyl, $C_{1-3}$ sulfanyl, $C_{2-4}$ alkynyl, $C_{3-6}$ cycloalkyl, 3-6 membered heterocyclic containing $_{1-3}$ N, O or S atoms, which are optionally further substituted by one or more substituents selected from halogen, $C_{1-3}$ alkyl, $C_{1-3}$ deuterated alkyl and $C_{1-3}$ alkoxy;

Preferably, $R^4$, $R^5$ or $R^6$ is each independently selected from fluorine, methyl, cyano, ethynyl, methylthio, amino, cyclopropyl, oxetane, which are optionally further substituted by halogen, methoxy, deuterated methyl.

In a further preferred embodiment of the present invention, the compound of general formula (V) is as follows:

$R^5$ and $R^6$ are linked with adjacent atoms to form $C_{3-6}$ cycloalkyl, 3-8 membered heterocyclic, $C_{6-10}$ aryl or 5-8 membered heteroaryl, which may be further substituted by one or more substituents selected from halogen, amino, $C_{1-3}$ alkyl, and oxo;

Preferably, $R^5$ and $R^6$ are linked with adjacent atoms to form 5-7 membered heterocyclic containing 1-3 atoms selected from N, O or S; optionally further substituted by one or more substituents selected from halogen, amino, $C_{1-3}$ alkyl, oxo;

Further preferably, $R^5$ and $R^6$ are linked with adjacent atoms to form

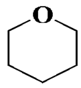 , 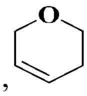 ,

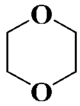 ;

and optionally further substituted by one or more substituents selected from halogen, amino, $C_{1-3}$ alkyl, and oxo.

In a further preferred embodiment of the present invention, the compound of general formula (V) is as follows:

$R^7$ and $R^8$ are each independently selected from deuterium or $C_{1-6}$ deuterated alkyl;

Preferably, $R^7$ and $R^8$ are each independently selected from deuterium or $C_{1-3}$ deuterated alkyl.

In a further preferred embodiment of the present invention, the compound of general formula (V) is as follows:

$R^9$ is selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, which are optionally further substituted by one or more substituents selected from halogen, amino, nitro, hydroxy, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, or 5-10 membered heteroaryl;

Preferably, $R^9$ is selected from hydrogen, deuterium, halogen, amino, nitro, hydroxyl, cyano, oxo, $C_{2-6}$ alkyl, $C_{2-6}$ deuterated alkyl, $C_{2-6}$ haloalkyl, $C_{2-6}$ hydroxyalkyl, $C_{2-6}$ alkoxy, $C_{2-6}$ haloalkoxy, $C_{2-4}$ alkenyl, $C_{2-4}$ alkynyl, $C_{3-6}$ cycloalkyl, 3-6 membered heterocyclyl, $C_{6-8}$ aryl or 5-8 membered heteroaryl, which are optionally further substituted by one or more substituents selected from halogen, amino, nitro, hydroxy, cyano, oxo, $C_{2-6}$ alkyl, $C_{2-6}$ deuterated alkyl, $C_{2-6}$ haloalkyl, $C_{2-6}$ hydroxyalkyl, $C_{2-6}$ alkoxy, $C_{2-6}$ haloalkoxy, $C_{2-4}$ alkenyl, $C_{2-4}$ alkynyl, $C_{3-6}$ cycloalkyl, 3-6 membered heterocyclyl, $C_{6-10}$ aryl, or 5-8 membered heteroaryl;

Further preferably, $R^9$ is selected from cyano, cyclopropyl, which are optionally further substituted by one or more substituents selected from halogen, amino, nitro, hydroxyl, cyano, oxo, $C_{2-6}$ alkyl, $C_{2-6}$ deuterated alkyl, $C_{2-6}$ haloalkyl, $C_{2-6}$ hydroxyalkyl, $C_{2-6}$ alkoxy, $C_{2-6}$ haloalkoxy, $C_{2-4}$ alkenyl, $C_{2-4}$ alkynyl, $C_{3-6}$ cycloalkyl, 3-6 membered heterocyclic, $C_{6-10}$ aryl or 5-8 membered heteroaryl.

In a further preferred embodiment of the present invention, the compound of general formula (V) is as follows:

$R^9$ is linked to the carbon atom connected thereto to form $C_{3-8}$ cycloalkyl, 3-8 membered heterocyclyl, a $C_{6-10}$ aryl or a 5-10 membered heteroaryl;

Preferably, $R^9$ is linked to the carbon atom connected thereto to form $C_{3-6}$ cycloalkyl, 3-6 membered heterocyclyl, $C_{6-10}$ aryl or 5-10 membered heteroaryl;

More preferably, $R^9$ is linked to the carbon atom connected thereto to further preferably form cyclopropyl.

In a further preferred embodiment of the present invention, the compound of general formula (V) is as follows:

$R^3$ is selected from hydrogen, deuterium, halogen, amino, nitro, hydroxyl, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-8}$ cycloalkyl, 3-8 membered heterocyclyl, $C_{6-10}$ aryl, 5-10 membered heteroaryl, $-NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$,$-(CH_2)_{n2}R_{a5}R_{b5}$ or

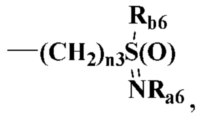

the amino, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclic, $C_{6-10}$ aryl or 5-8 membered heteroaryl are optionally further substituted by one or more substituents selected from halogen, amino, nitro, hydroxy, cyano, oxo,-NS(O)$R_{a1}R_{b1}$, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl;

Preferably, $R^3$ is selected from hydrogen, deuterium, halogen, amino, $C_{1-3}$ alkyl, $C_{1-3}$ deuterated alkyl, $C_{1-3}$ haloalkyl, -NS(O)$R_{a1}R_{b1}$, $-(CH_2)_{n1}S(O)NR_{a2}R_{b2}$, -P(O)$R_{a3}R_{b3}$,-$NR_{a5}S(O)NR_{a4}R_{b4}$, $-(CH_2)_{n2}R_{a5}R_{b5}$ or

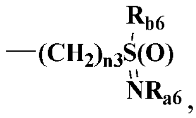

the amino, $C_{1-3}$ alkyl, $C_{1-3}$ deuterated alkyl or $C_{1-3}$ haloalkyl are optionally further substituted by one or more substituents selected from halogen, -NS(O)$R_{a1}R_{b1}$ or $C_{1-3}$ alkyl.

In a further preferred embodiment of the present invention, the compound of general formula (V) is as follows:

$R^3$ is selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, 5-10 membered heteroaryl, -NS(O)$R_{a1}R_{b1}$, $-(CH_2)_{n1}S(O)NR_{a2}R_{b2}$, -P(O)$R_{a3}R_{b3}$, -$NR_{a5}S(O)NR_{a4}R_{b4}$, the amino, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl and 5-10 membered heteroaryl are optionally further substituted by one or more of halogen, amino, nitro, hydroxyl, cyano, oxo, -NS(O)$R_{a1}R_{b1}$, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl and 5-10 membered heteroaryl;

Preferably, $R^3$ is selected from hydrogen, deuterium, halogen, amino, nitro, hydroxyl, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclyl, $C_{6-10}$ aryl, 5-8 membered heteroaryl, -NS(O)$R_{a1}R_{b1}$, $-(CH_2)_{n1}S(O)NR_{a2}R_{b2}$, -P(O)$R_{a3}R_{b3}$, -$NR_{a5}S(O)NR_{a4}R_{b4}$, the amino, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-8}$ cycloalkyl, 3-8 membered heterocyclic, $C_{6-10}$ aryl and 5-10 membered heteroaryl are optionally further substituted by one or more of halogen, amino, nitro, hydroxyl, cyano, oxo, -NS(O)$R_{a1}R_{b1}$, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclic, $C_{6-10}$ aryl and 5-8 membered heteroaryl;

Further preferably, $R^3$ is selected from hydrogen, deuterium, halogen, amino, $C_{1-3}$ alkyl, $C_{1-3}$ deuterated alkyl, $C_{1-3}$ haloalkyl, -NS(O)$R_{a1}R_{b1}$, $-(CH_2)_{n1}S(O)NR_{a2}R_{b2}$, -P(O)$R_{a3}R_{b3}$, or-$NR_{a5}S(O)NR_{a4}R_{b4}$, the amino, $C_{1-3}$ alkyl, $C_{1-3}$ deuterated alkyl or $C_{1-3}$ haloalkyl are optionally further substituted by one or more substituents selected from halogen, -NS(O)$R_{a1}R_{b1}$ or $C_{1-3}$ alkyl.

In a further preferred embodiment of the present invention, the compound of general formula (V) is as follows:

$R^{12}$ is selected from $C_{1-3}$ alkyl, $C_{3-6}$ cycloalkyl, $-(CH_2)_{n2}R_{a5}R_{b5}$ or

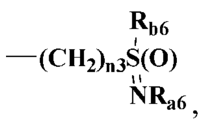

which are optionally further substituted by one or more substituents selected from halogen, amino, nitro, hydroxyl, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl;

Preferably, $R^{12}$ is selected from methyl, cyclopropyl, $-(CH_2)_{n2}R_{a5}R_{b5}$ or

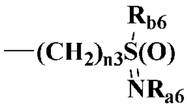,

which are optionally further substituted by one or more substituents selected from halogen, amino, nitro, hydroxyl, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl.

In a further preferred embodiment of the present invention, the compound of general formula (V) is as follows:

$R^{12}$ is selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, which are optionally further substituted by one or more substituents selected from halogen, amino, nitro, hydroxy, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, or 5-10 membered heteroaryl;
Preferably, $R^{12}$ is selected from $C_{1-3}$ alkyl;
Further preferably, $R^{12}$ is selected from methyl.

In a further preferred embodiment of the present invention, the compound of general formula (V) is as follows:

$R_{a1}$, $R_{b1}$, $R_{a2}$, $R_{b2}$, $R_{a3}$, $R_{b3}$, $R_{a4}$, $R_{b4}$, $R_{a5}$, $R_{b5}$, $R_{a6}$, or $R_{b6}$ is each independently selected from hydrogen, deuterium, halogen, amino, nitro, hydroxyl, cyano, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, 5-10 membered heteroaryl;
Preferably, $R_{a1}$, $R_{b1}$, $R_{a2}$, $R_{b2}$, $R_{a3}$, $R_{b3}$, $R_{a4}$, $R_{b4}$, $R_{a5}$, $R_{b5}$, $R_{a6}$, or $R_{b6}$ is each independently selected from hydrogen, deuterium, halogen, or methyl.

In a further preferred embodiment of the present invention, the compound of general formula (V) is as follows:

$R_{a1}$ and $R_{b1}$, $R_{a2}$ and $R_{b2}$, $R_{a3}$ and $R_{b3}$, $R_{a4}$ and $R_{b4}$, $R_{a5}$ and $R_{b5}$, $R_{a6}$ and $R_{b6}$ are linked to adjacent atoms to form $C_{3-8}$ cycloalkyl, 3-6 membered heterocyclyl, $C_{6-10}$ aryl or 5-8 membered heteroaryl;
Preferably, $R_{a2}$ and $R_{b2}$, $R_{a3}$ and $R_{b3}$, are linked to adjacent atoms to form $C_{3-8}$ cycloalkyl, 3-6 membered heterocyclic containing 1-3 heteroatoms.

In a further preferred embodiment of the present invention, the compound of general formula (V) is as follows:

$R_{a1}$, $R_{b1}$, $R_{a2}$, $R_{b2}$, $R_{a3}$, $R_{b3}$, $R_{a4}$ or $R_{b4}$ is each independently selected from hydrogen, deuterium, halogen, amino, nitro, hydroxyl, cyano, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;
Preferably, $R_{a1}$, $R_{b1}$, $R_{a2}$, $R_{b2}$, $R_{a3}$, $R_{b3}$, $R_{a4}$ or $R_{b4}$ is each independently selected from hydrogen, deuterium, halogen, amino, $C_{1-3}$ alkyl, $C_{1-3}$ deuterated alkyl or $C_{1-3}$ halogenated alkyl;
Further preferably, $R_{a1}$, $R_{b1}$, $R_{a2}$, $R_{b2}$, $R_{a3}$, $R_{b3}$, $R_{a4}$ or $R_{b4}$ is each independently selected from hydrogen, deuterium, halogen, or methyl.

In a further preferred embodiment of the present invention, the compound of general formula (V) is as follows:

$R_{a1}$ and $R_{b1}$, $R_{a2}$ and $R_{b2}$, $R_{a3}$ and $R_{b3}$, $R_{a4}$ and $R_{b4}$, are respectively linked to adjacent atoms to form $C_{3-8}$ cycloalkyl, 3-8 membered heterocyclyl, $C_{6-10}$ aryl or 5-10 membered heteroaryl, the $C_{3-8}$ cycloalkyl, 3-8 membered heterocyclyl, $C_{6-10}$ aryl or 5-10 membered heteroaryl are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;
Preferably, $R_{a1}$ and $R_{b1}$, $R_{a2}$ and $R_{b2}$, $R_{a3}$ and $R_{b3}$, $R_{a4}$ and $R_{b4}$, are respectively linked to adjacent atoms to form $C_{3-8}$ cycloalkyl, 3-6 membered heterocyclyl, $C_{6-10}$ aryl or 5-8 membered heteroaryl;
Further preferably, $R_{a2}$ and $R_{b2}$, $R_{a3}$ and $R_{b3}$, are respectively linked to adjacent atoms to form $C_{3-8}$ cycloalkyl, 3-6 membered heterocyclic containing $_{1-3}$ heteroatoms.

In a further preferred embodiment of the present invention, the compound of general formula (V) is as follows:

$R^7$, $R^8$, $R^9$ or $R^{10}$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, which are optionally further substituted by one or more of halogen, amino, nitro, hydroxy, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl and 5-10 membered heteroaryl;
Preferably, $R^7$, $R^8$, $R^9$ or $R^{10}$ is each independently selected from hydrogen, deuterium, halogen, $C_{1-3}$ alkyl, $C_{1-3}$ deuterated alkyl or $C_{1-3}$ haloalkyl;
Further preferably, $R^7$, $R^8$, $R^9$ or $R^{10}$ is each independently selected from methyl.

In a further preferred embodiment of the present invention, the compound of general formula (V) is as follows:

$R^7$, $R^8$, $R^9$ or $R^{10}$ is each independently selected from hydrogen, deuterium, halogen, amino, nitro, hydroxyl, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl, which are optionally further substituted by one or more of halogen, amino, nitro, hydroxy, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclyl, $C_{6-10}$ aryl and 5-8 membered heteroaryl;
Preferably, $R^7$, $R^8$, $R^9$ or $R^{10}$ is each independently selected from hydrogen, deuterium, halogen, cyano, $C_{2-4}$ alkynyl, C3-6 cycloalkyl, $C_{1-3}$ alkyl, $C_{1-3}$ deuterated alkyl or $C_{1-3}$ haloalkyl;
Further preferably, $R^7$, $R^8$, $R^9$ or $R^{10}$ is each independently selected from methyl, fluoro, cyclopropyl or ethynyl.

In a further preferred embodiment of the present invention, the compound of general formula (V) is as follows:

$R^7$, $R^8$, $R^9$ or $R^{10}$ is respectively linked to the atoms connected thereto to form $C_{3-10}$ cycloalkyl, 3-10 membered heterocyclic, $C_{6-12}$ aryl or 5-12 membered heteroaryl;
Preferably, $R^7$, $R^8$, $R^9$ or $R^{10}$ is respectively linked to the atoms connected thereto to form $C_{3-6}$ cycloalkyl, 3-6 membered heterocyclic, $C_{6-10}$ aryl or 5-8 membered heteroaryl;
Further preferably, $R^7$, $R^8$, $R^9$ or $R^{10}$ is respectively linked to the atoms connected thereto to form cyclopropyl.

In a further preferred embodiment of the present invention, the compound of general formula (V) is as follows: $R^{12}$ is selected from hydrogen, deuterium, halogen, amino, nitro, hydroxyl, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl, which are optionally further substituted by one or more substituents selected from halogen, amino, nitro, hydroxyl, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl.

In a further preferred embodiment of the present invention, the compound of general formula (V) is as follows:

$R^7$, $R^8$, $R^9$ or $R^{10}$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, 5-10 membered heteroaryl or $-(CH_2)_{m1}OR_{c1}$, which are optionally further substituted with one or more substituents selected from halogen, amino, nitro, hydroxy, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;
preferably, $R^7$, $R^8$, $R^9$ or $R^{10}$ is each independently selected from hydrogen, deuterium, halogen, amino, nitro, hydroxyl, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, C3-6 cycloalkyl, 3-6 membered heterocyclyl, $C_{6-10}$ aryl, 5-8 membered heteroaryl or $-(CH_2)_{m1}OR_{c1}$, which are optionally further substituted by one or more substituents selected from halogen, amino, nitro, hydroxy, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl;
Further preferably, $R^7$, $R^8$, $R^9$ or $R^{10}$ is each independently selected from hydrogen, deuterium, halogen, cyano, $C_{2-4}$ alkynyl, C3-6 cycloalkyl, $C_{1-3}$ alkyl, $C_{1-3}$ deuterated alkyl, $C_{1-3}$ haloalkyl or $-(CH_2)_{m1}OR_{c1}$;
More preferably, $R^7$, $R^8$, $R^9$ or $R^{10}$ is each independently selected from methyl, fluorine, cyclopropyl, ethynyl, or $-(CH_2)_{m1}OR_{c1}$.

In a further preferred embodiment of the present invention, the compound of general formula (V) is as follows:

$R_{c1}$ is selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ sulfanyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, or 5-10 membered heteroaryl, the amino, $C_{1-6}$ alkyl, $C_{1-6}$ deuterated alkyl, $C_{1-6}$ haloalkyl, $C_{1-6}$ hydroxyalkyl, $C_{1-6}$ sulfanyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl are optionally further substituted with one or more substituents selected from halogen, amino, nitro, hydroxy, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

Preferably, $R_{c1}$ is selected from hydrogen, deuterium, halogen, amino, nitro, hydroxyl, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ sulfanyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl, which are optionally further substituted with one or more substituents selected from halogen, amino, nitro, hydroxy, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl;

Further preferably, $R_{c1}$ is selected from fluoro, methyl, cyano, difluoromethyl, trifluoromethyl, ethynyl, methylthio, amino, cyclopropyl or oxetane; optionally further substituted by halogen, methoxy or deuterated methyl.

In a further preferred embodiment of the present invention, the compound of general formula (V) is as follows:

$R^{10}$ is linked to the atoms connected thereto and the adjacent atoms to form $C_{3-10}$ cycloalkyl, 3-10 membered heterocyclic, $C_{6-12}$ aryl, or 5-12 membered heteroaryl;

preferably, $R^{10}$ is linked to the atoms connected thereto and the adjacent atoms to form $C_{3-6}$ cycloalkyl, 3-6 membered heterocyclic, $C_{6-10}$ aryl or a 5-8 membered heteroaryl;

more preferably, $R^{10}$ is linked to the atoms connected thereto and the adjacent atoms to form

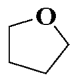
.

In a further preferred embodiment of the present invention, the compound of general formula (V) is as follows: $R^{10}$ is linked to the atoms connected thereto and the adjacent atoms to form

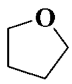

or

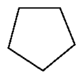
.

In a further preferred embodiment of the present invention, the compound of general formula (V) is as follows: $R^{10}$ is linked to the atoms connected thereto and the adjacent atoms to form

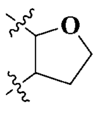
、

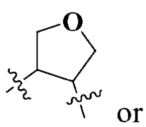
or
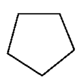
.

In a further preferred embodiment of the present invention, the compound of general formula (V) is further shown in general formula (V-1):

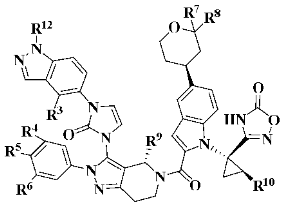

(V-1)

.

An object of the present invention is to provide a compound represented by general formula (XVI), a prodrug thereof, a stereoisomer thereof or a pharmaceutically acceptable salt thereof:

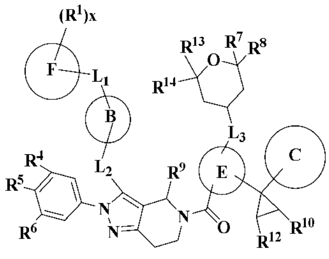

(XVI)

wherein,

ring F is selected from 3-18 membered heterocyclyl, $C_{3-18}$ cycloalkyl, $C_{6-18}$ aryl or 5-18 membered heteroaryl;
ring B is selected from $C_{3-12}$ cycloalkyl, 3-12 membered heterocyclyl, $C_{6-14}$ aryl or 5-14 membered heteroaryl, which are optionally further substituted with one or more substituents selected from halogen, amino, nitro, hydroxyl, cyano, oxo, thiol, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ alkoxy, $C_{1-6}$ deuterated alkoxy, $C_{1-6}$ haloalkoxy or $C_{1-6}$ deuterated haloalkoxy;
ring C is selected from $C_{3-12}$ cycloalkyl, 3-12 membered heterocyclyl, $C_{6-14}$ aryl or 5-14 membered heteroaryl, which are optionally further substituted by one or more substituents selected from halogen, amino, nitro, hydroxyl, cyano, oxo, thiol, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy or $C_{1-6}$ deuterated haloalkoxy;
ring E is selected from $C_{3-12}$ cycloalkyl, 3-12 membered heterocyclyl, $C_{6-14}$ aryl or 5-14 membered heteroaryl, which are optionally further substituted by one or more substituents selected from deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl,
Alternatively, ring E is

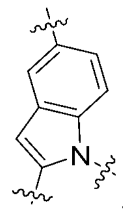

,

which is optionally further substituted by one or more substituents selected from halogen, methoxy or deuterated methyl;
$L_1$, $L_2$ or $L_3$ are each independently selected from a bond, $C_{2-4}$ alkenylene, $C_{2-4}$ alkynylene, $-(CH_2)_{n1}-$ or $-(CH_2)_{n5}NR_{b5}$, the $C_{2-4}$ alkenylene, $C_{2-4}$ alkynylene, $-(CH_2)_{n1}-$ or $-(CH_2)_{n5}NR_{b5}$ are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

$R^1$ is independently selected from

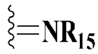,

, hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl, 5-10 membered heteroaryl, $-NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$ , $-(CH_2)_{n1}O(CH_2)_{n2}R_{a5}$ , $-(CH_2)_{n1}C(O)NR_{a6}R_{b6}$ , $-(CH_2)_{n1}P(O)R_{a3}R_{b3}$,

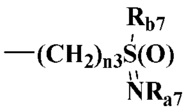,

$-(CH_2)_{n1}NR_{a8}C(O)R_{b8}$, $-(CR_{a8}R_{b8})_{n4}C(O)NR_{a9}R_{b9}$, $-(CH_2)_{n1}S(O)_2R_{b5}$ or $-(CH_2)_{n5}C(O)NR_{a10}R_{b10}$, the amino, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl, 5-10 membered heteroaryl, $-NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$ , $-(CH_2)_{n1}O(CH_2)_{n2}R_{a5}$ , $-(CH_2)_{n1}C(O)NR_{a6}R_{b6}$ , $-(CH_2)_{n1}P(O)R_{a3}R_{b3}$,

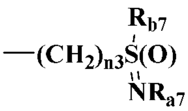,

$-(CH_2)_{n1}NR_{a8}C(O)R_{b8}$, $-(CR_{a8}R_{b8})_{n4}C(O)NR_{a9}R_{b9}$, $-(CH_2)_{n1}S(O)_2R_{b5}$ or $-(CH_2)_{n5}C(O)NR_{a10}R_{b10}$ are optionally further substituted by one or more substituents selected from

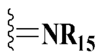,

, halogen, amino, nitro, hydroxyl, cyano, oxo, $-NS(O)R_{a1}R_{b1}$, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heteroaryl, $C_{6-10}$ aryl or 5-10 membered heteroaryl;

alternatively,

$R^1$ is respectively linked to the atoms connected thereto to form $C_{3-12}$ cycloalkyl, 3-12 membered heterocyclic, $C_{6-14}$ aryl or 5-14 membered heteroaryl, which are optionally further substituted by one or more substituents selected from

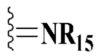,

, deuterium, halogen, amino, nitro, hydroxyl, cyano, oxo, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, C3-8 cycloalkyl, 3-8 membered heterocyclic, C6-10 aryl, 5-10 membered heteroaryl, $-NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$, $-(CH2)_{n1}S(O)_2R_{a5}$, $-(CH_2)_{n1}O(CH_2)_{n2}R_{b5}$,

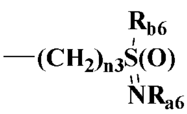,

$-(CH_2)_{n1}NR_{a8}C(O)R_{b8}$, $-(CR_{a8}R_{b8})_{n4}C(O)NR_{a9}R_{b9}$, $-(CH_2)_{n1}S(O)_2R_{b5}$ or $-(CH_2)_{n5}C(O)NR_{a10}R_{b10}$;

$R_{a1}$, $R_{b1}$, $R_{a2}$, $R_{b2}$, $R_{a3}$, $R_{b3}$, $R_{a4}$, $R_{b4}$, $R_{a5}$, $R_{a6}$, $R_{b6}$, $R_{a7}$, $R_{b7}$, $R_{a8}$, $R_{b8}$, $R_{a9}$, $R_{b9}$, $Ra_{10}$ or $Rb_{10}$ is each independently

selected from hydrogen, deuterium, halogen, amino, nitro, hydroxyl, cyano, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, the amino, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl are optionally further substituted by one or more substituents selected from halogen, amino, nitro, hydroxyl, cyano, oxo, $-NS(O)R_{a1}R_{b1}$, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

alternatively,

$R_{a1}$ and $R_{b1}$, $R_{a2}$ and $R_{b2}$, $R_{a3}$ and $R_{b3}$, $R_{a4}$ and $R_{b4}$, $R_{a6}$ and $R_{b6}$, $R_{a8}$ and $R_{b8}$, $R_{a9}$ and $R_{b9}$, $R_{a10}$ and $R_{b10}$ are linked with adjacent atoms to form $C_{3-10}$ cycloalkyl, 3-10 membered heterocyclic, C6-12 aryl or 5-12 membered heteroaryl, which are optionally further substituted by one or more substituents selected from halogen, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R^4$, $R^5$ or $R^6$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, the ammonia, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, C6-10 aryl or 5-10 membered heteroaromatic are optionally further substituted by one or more substituents selected from Halogen, amino, nitro, hydroxyl, cyano, oxo, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R^7$, $R^8$, $R^9$, $R^{10}$, $R^{12}$, $R^{13}$, $R^{14}$ or $R^{15}$ are each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl, 5-10 membered heteroaryl or $-(CH_2)_{m1}OR_{c1}$, the amino, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl, 5-10 membered heteroaryl or $-(CH_2)_{m1}OR_{c1}$ are optionally further substituted by one or more substituents selected from deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R_{c1}$ is selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, the amino, $C_{1-6}$ alkyl, $C_{1-6}$ deuterated alkyl, $C_{1-6}$ haloalkyl, $C_{1-6}$ hydroxyalkyl, $C_{1-6}$ sulfanyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl, the amino, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl, the amino, $C_{1-6}$ alkyl, $C_{1-6}$ deuterated alkyl, $C_{1-6}$ haloalkyl, $C_{1-6}$ hydroxyalkyl, $C_{1-6}$ sulfanyl, $C_{1-6}$ alkoxy, $C_{1-6}$ haloalkoxy, $C_{2-6}$ alkenyl, $C_{2-6}$ alkynyl, $C_{3-8}$ Cycloalkyl, 3-8 membered heterocyclyl, $C_{6-10}$ aryl or 5-10 membered heteroaryl are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

alternatively, $R^7$, $R^8$, $R^9$, $R^{10}$, $R^{12}$, $R^{13}$, $R^{14}$ or $R^{15}$ are linked to the atoms connected thereto to form a $C_{3-10}$ cycloalkyl, a 3-10 membered heterocyclic, a $C_{6-12}$ aryl, or 5-12 membered heteroaryl;
alternatively,
$R^{10}$ and $R^{12}$ are linked to the atoms connected thereto to form C3-10 cycloalkyl, 3-10 membered heterocyclic, $C_{6-12}$ aryl or 5-12 membered heteroaryl;
n1-n5, m1 or x are each independently selected from 0, 1, 2, 3, 4 or 5.

In a further preferred embodiment of the present invention, the compound of general formula (XVI) is as follows:

ring F is selected from $C_{3-12}$ cycloalkyl, 3-14 membered heterocyclyl containing 1-3 heteroatoms selected from N, O or S, $C_{6-14}$ aryl or 5-14 membered heteroaryl containing 1-3 heteroatoms selected from N, O or S;
Preferably, ring F is selected from $C_{3-10}$ cycloalkyl, 5-14 membered heterocyclyl containing 1-3 heteroatoms selected from N, O or S, $C_{6-10}$ aryl or 5-14 membered heteroaryl containing 1-3 heteroatoms selected from N, O or S;
Further preferably, ring F is selected from C3-6 bridged cycloalkyl, 8-14 membered tricyclic fused heterocyclic containing 1-5 heteroatoms, 3-8 membered bridged heterocyclic containing 1-3 heteroatoms, fused heterocyclic containing 1-3 heteroatoms, bridged heterocyclic containing 1-3 heteroatoms, C6-10 aryl or 5-10 membered fused heteroaryl containing 1-3 heteroatoms;

More preferably, ring F is selected from phenyl, pyridyl, pyrimidinyl, thiazolyl, pyridazinyl, oxazolyl, morpholinyl,

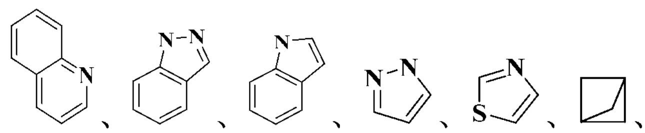

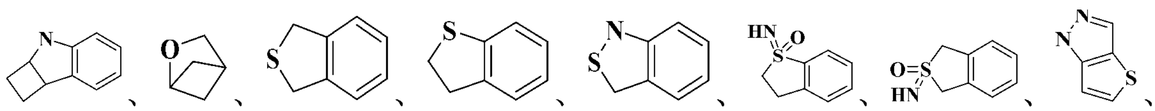

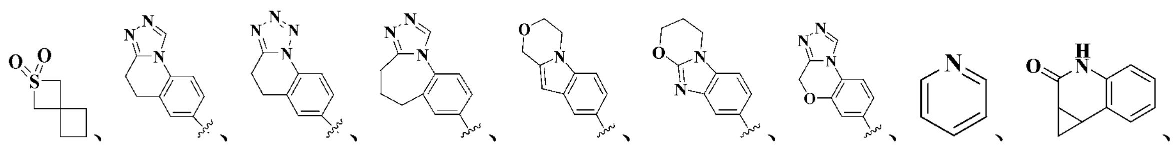

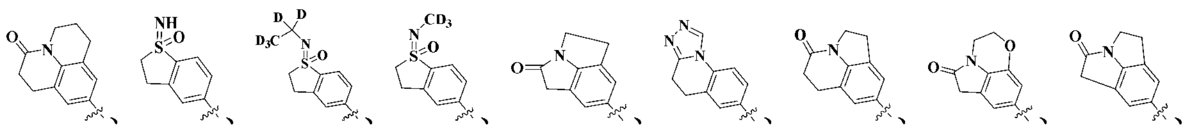

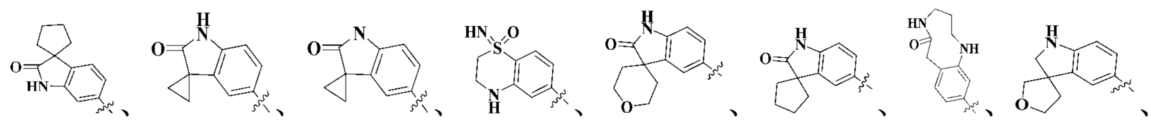

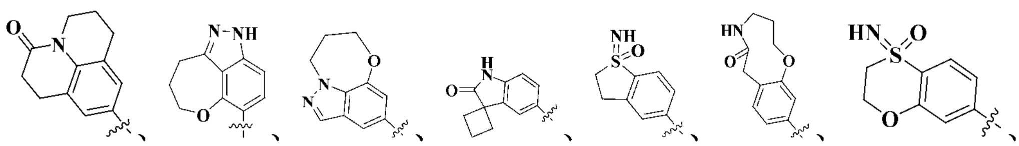

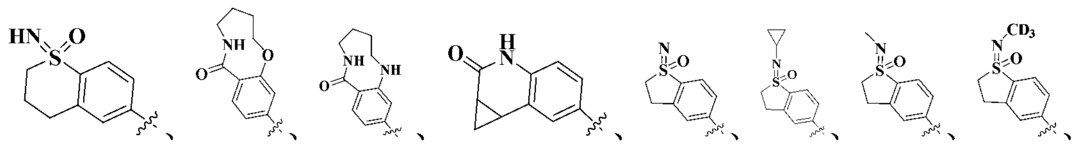

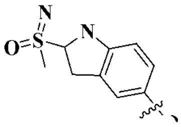、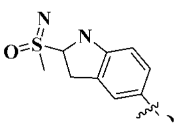、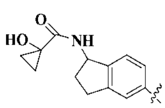、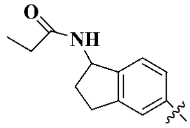、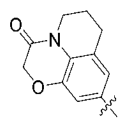、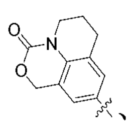、

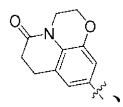、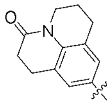、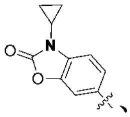、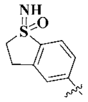、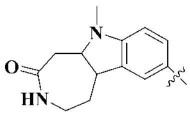、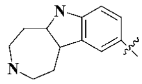、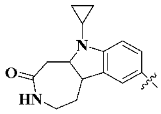、

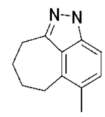、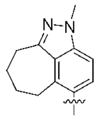、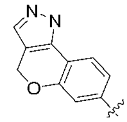、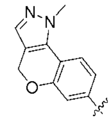、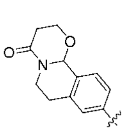、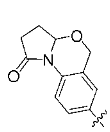、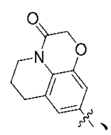、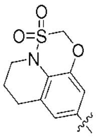、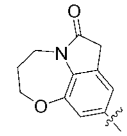、

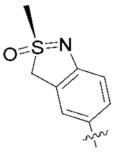、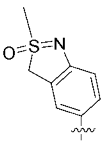、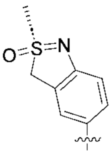、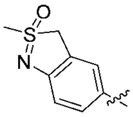、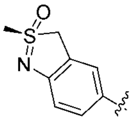、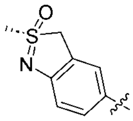、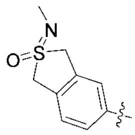、

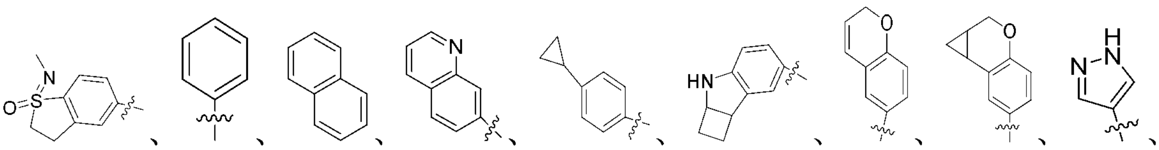
、、、、、、、、、

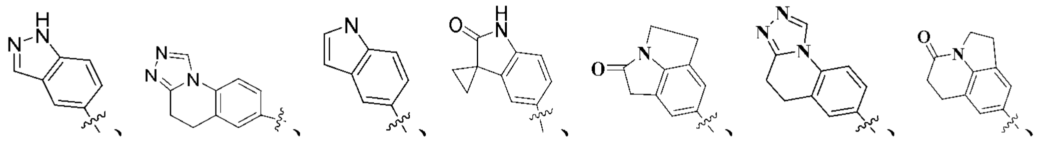
、、、、、、、

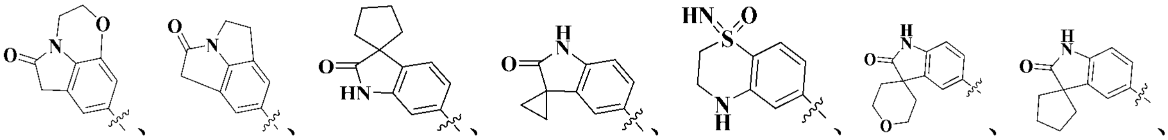
、、、、、、、

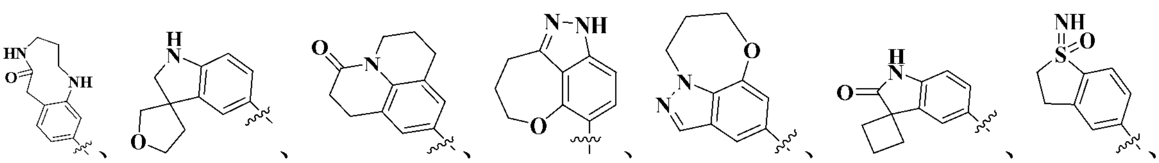
、、、、、、、

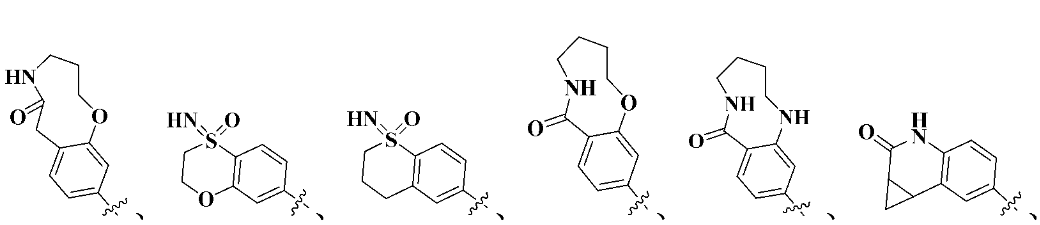
、、、、、、

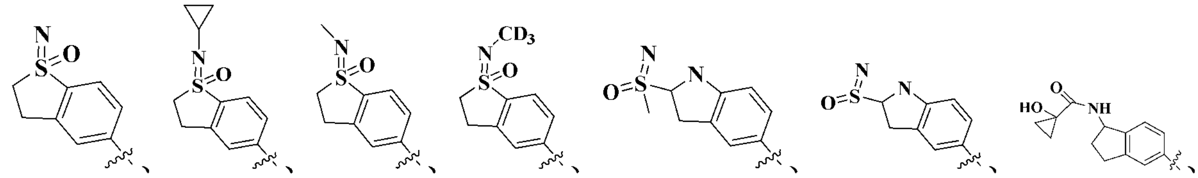

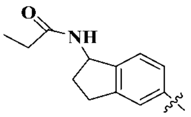、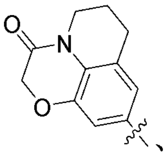、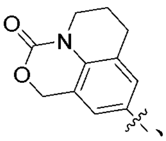、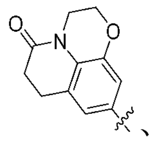、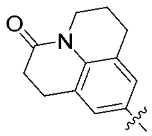、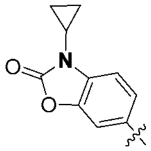、

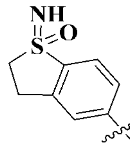、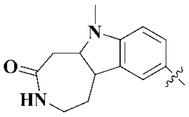、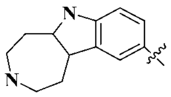、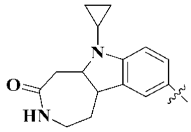、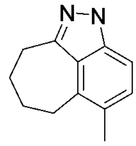、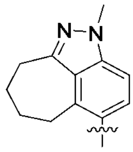、

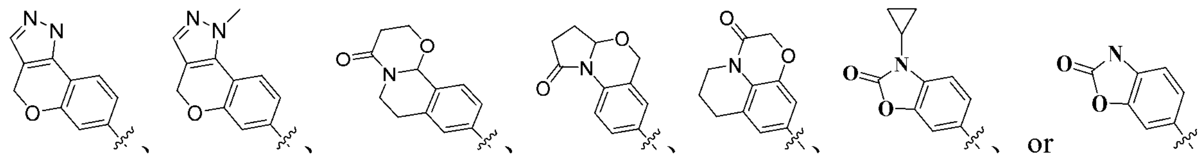

Note:

“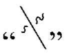”

indicates connected to $L_1$.

In a further preferred embodiment of the present invention, the compound of general formula (XVI) is as follows: ring F is selected from phenyl, pyridyl, pyrimidinyl, thiazolyl, pyridazinyl, oxazolyl, morpholinyl,

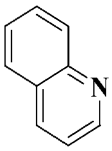、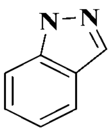、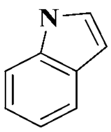、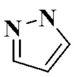、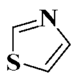、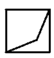、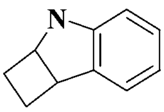、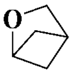、

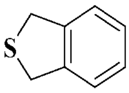、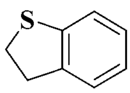、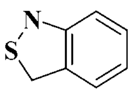、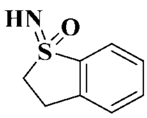、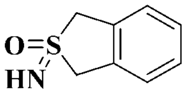、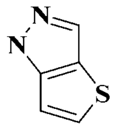、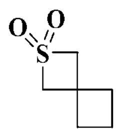、

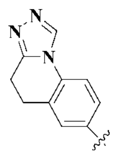、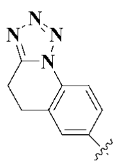、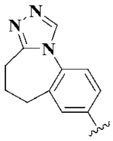、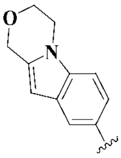、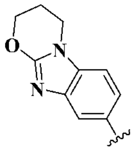、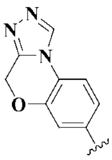、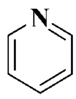、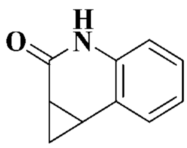、

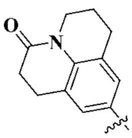, 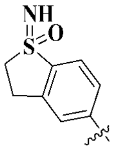, 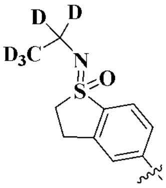, 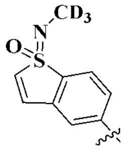, 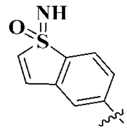, 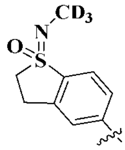, 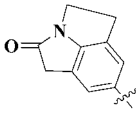,

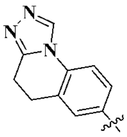, 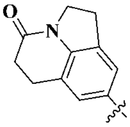, 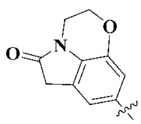, 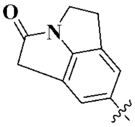, 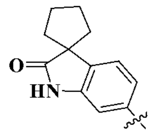, 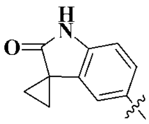, 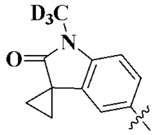,

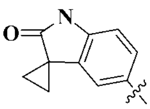, 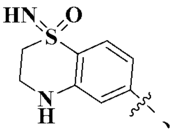, 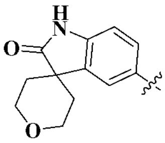, 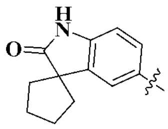, 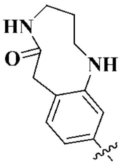, 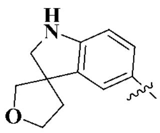,

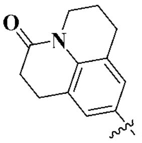, 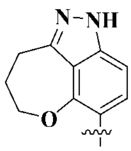, 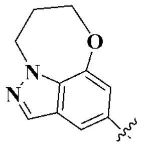, 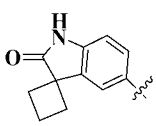, 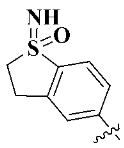, 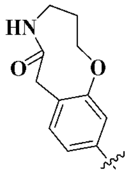, 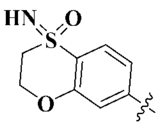,

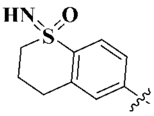, 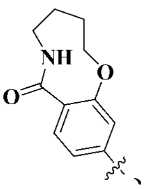, 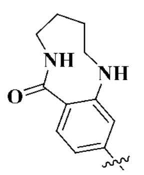, 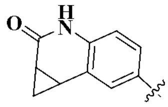, 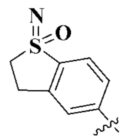, 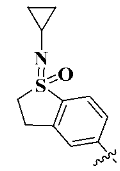, 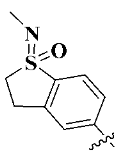,

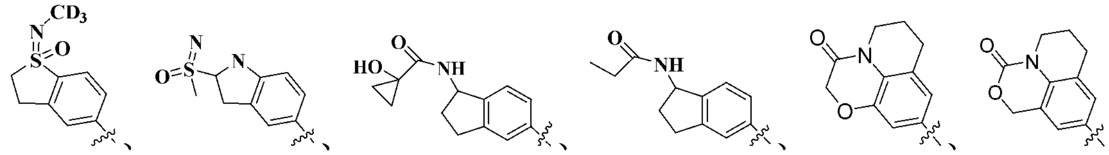
, , , , , ,

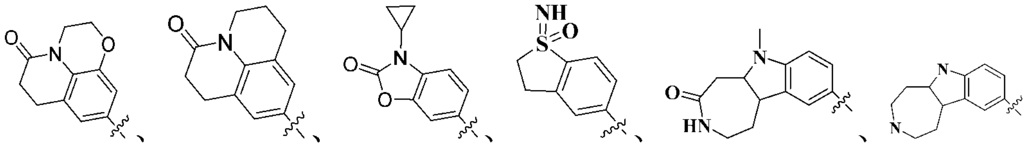
, , , , , ,

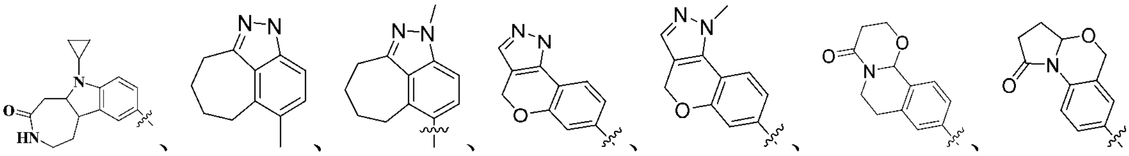
, , , , , , ,

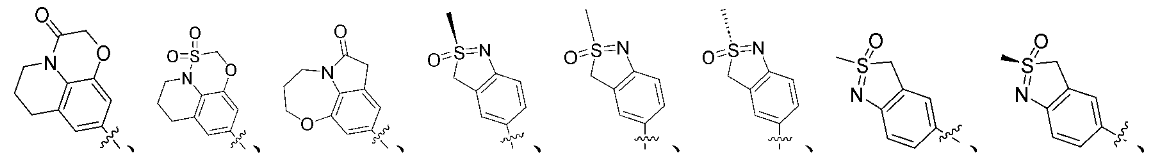
, , , , , , , ,

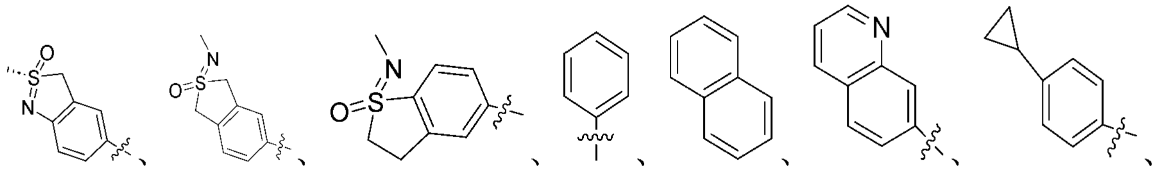
, , , , , , ,

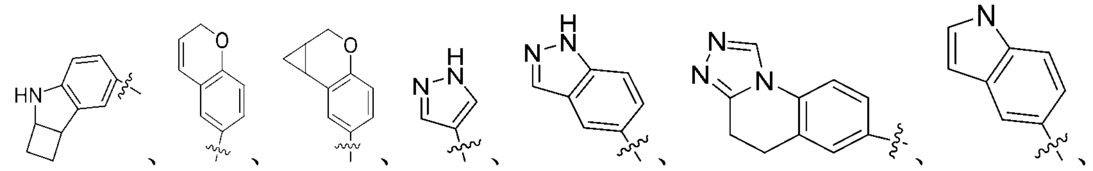
, , , , , , ,

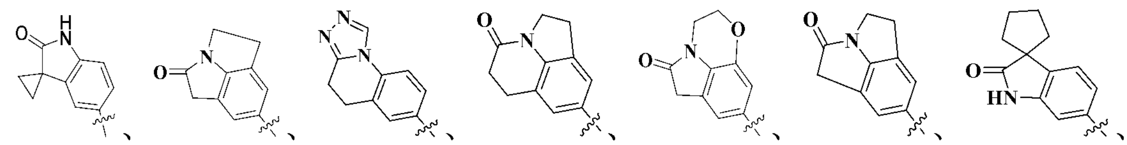
, , , , , , ,

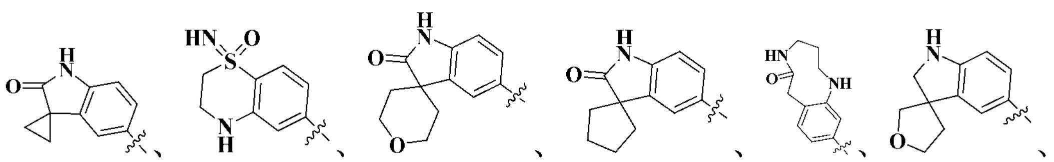
, , , , , ,

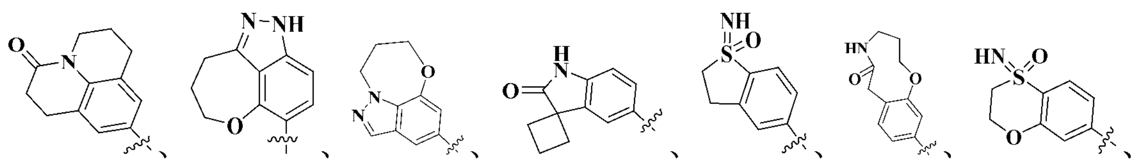
, , , , , , ,

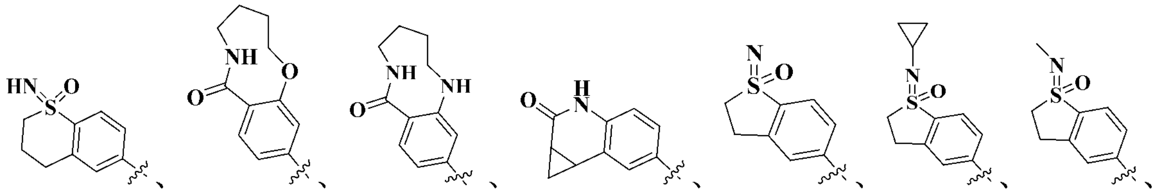
, , , , , , ,

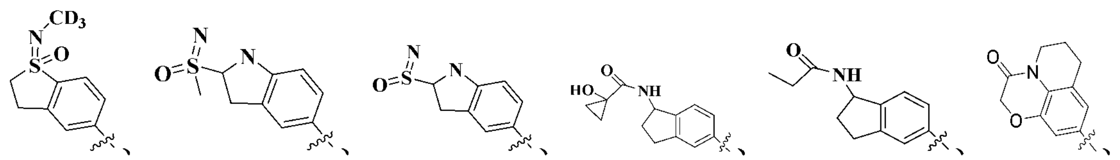
, , , , , ,

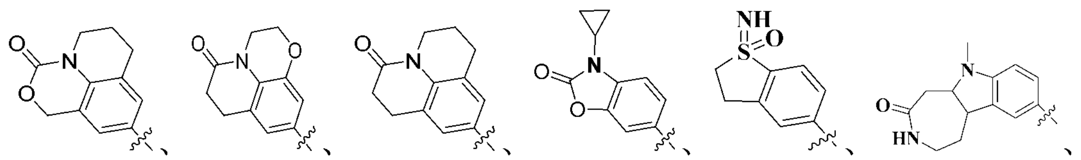
, , , , , ,

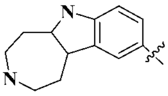、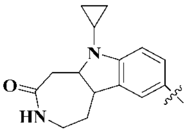、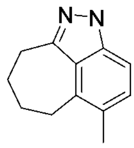、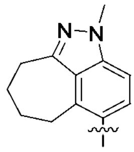、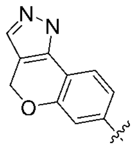、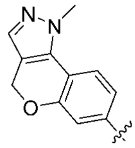、

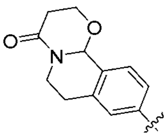、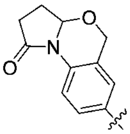、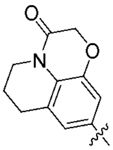、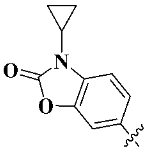、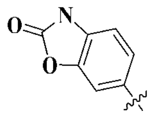、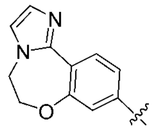、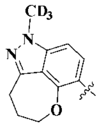、or

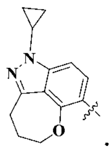.

Note:

“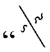”

indicates connected to $L_1$.

In a further preferred embodiment of the present invention, the compound of general formula (XVI) is as follows:

Ring B is selected from $C_{3-8}$ cycloalkyl, 3-8 membered heterocyclyl containing 1-3 N, O or S atoms, $C_{6-10}$ aryl or 5-10 membered heteroaryl containing 1-3 N, O or S atoms, which are optionally further substituted by one or more substituents selected from which are optionally further substituted with one or more substituents selected from halogen, amino, nitro, hydroxyl, cyano, oxo, thiol, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ alkoxy, $C_{1-3}$ deuterated alkoxy, $C_{1-3}$ haloalkoxy or $C_{1-3}$ deuterated haloalkoxy;

Preferably, ring B is selected from $C_{3-6}$ cycloalkyl, 3-6 membered heterocyclic containing 1-3 N, O or S atoms or 5-8 membered heteroaryl containing 1-3 N, O or S atoms, which are optionally further substituted by one or more substituents of halogen, amino, oxo, thio or $C_{1-3}$ alkyl;

Further preferably, ring B is selected from

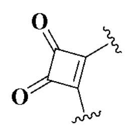、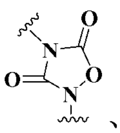、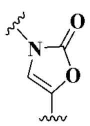、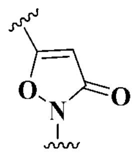、

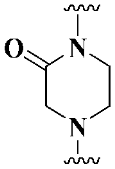、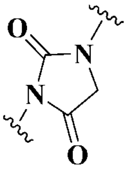、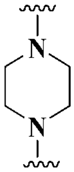、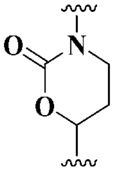、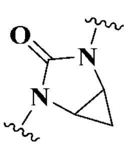、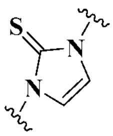、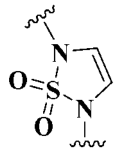、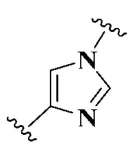、

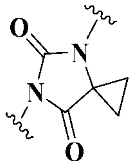 、 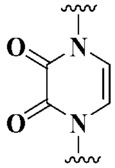 、 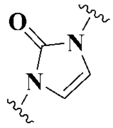 or 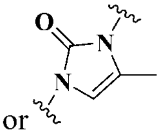 ,

which are optionally further substituted by one or more substituents selected from halogen, amino, oxo, thio or $C_{1-3}$ alkyl.

In a further preferred embodiment of the present invention, the compound of general formula (XVI) is as follows:

Ring C is selected from $C_{3-8}$ cycloalkyl, 3-8 membered heterocyclic containing 1-3 selected from N, O or S, a C6-10 aryl, or a 5-8 membered heteroaryl containing 1-3 selected from N, O or S, which are optionally further substituted by one or more substituents selected from halogen, amino, nitro, hydroxyl, cyano, oxo, thiol, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy or $C_{1-3}$ deuterated haloalkoxy;
Preferably, ring C is selected from $C_{3-6}$ cycloalkyl, 3-6 membered heterocyclic containing 1-3 selected from N, O or S, a C6-10 aryl, or a 5-6 membered heteroaryl containing 1-3 selected from N, O or S, which are optionally further substituted by one or more substituents selected from hydroxyl, thio, oxo or $C_{1-6}$ haloalkoxy;
Further preferably, ring C is selected from

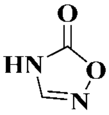 、 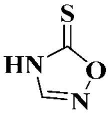 、 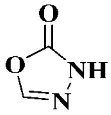 or 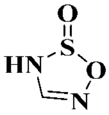 ,

which are optionally further substituted by one or more substituents selected from hydroxyl, thio, or oxo.

In a further preferred embodiment of the present invention, the compound of general formula (XVI) is as follows:

Ring E is selected from $C_{3-12}$ cycloalkyl, 3-12 membered heterocyclic containing 1-3 N, O or S atoms, $C_{6-12}$ aryl or 5-12 membered heteroaryl containing 1-3 N, O or S atoms, which are optionally further substituted by one or more substituents selected from deuterium, halogen, amino, nitro, hydroxyl, cyano, oxo, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl or 5-8 membered heteroaryl;
Preferably, ring E is selected from $C_{3-6}$ cycloalkyl, 3-10 membered heterocyclic containing 1-3 N, O or S atoms, C6-10 aryl or 5-10 membered heteroaryl containing 1-3 N, O or S atoms, which are optionally further substituted by one or more substituents selected from halogen, methoxy or deuterated methyl;
Further preferably, ring E is selected from

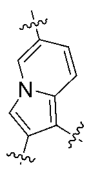 、 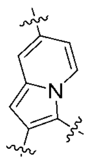 、 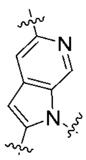 、 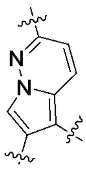 、

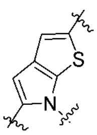 、 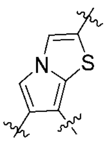 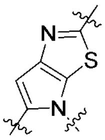 、 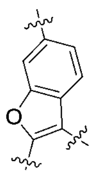 、 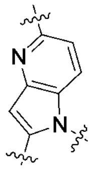 、 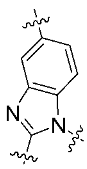 or 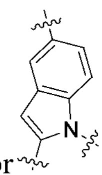 ,

which are optionally further substituted by one or more substituents selected from halogen, methoxy or deuterated methyl.

In a further preferred embodiment of the present invention, the compound of general formula (XVI) is as follows:

Ring E is

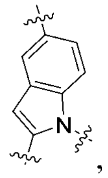
,

which is optionally further substituted by one or more substituents selected from halogen, methoxy or deuterated methyl.

In a further preferred embodiment of the present invention, the compound of general formula (XVI) is as follows:

$R^1$ is independently selected from

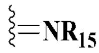
,

hydrogen, deuterium, halogen, amino, nitro, hydroxyl, cyano, oxo, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl, 5-8 membered heteroaryl, $-NS(O)R_{a1}R_{b1}$, $-(CH_2)_{n1}S(O)NR_{a2}R_{b2}$, - $P(O)R_{a3}R_{b3}$, $-NR_{a5}S(O)NR_{a4}R_{b4}$ , $-(CH_2)_{n1}O(CH_2)_{n2}R_{a5}$ , $-(CH_2)_{n1}C(O)NR_{a6}R_{b6}$ , - $(CH_2)_{n1}P(O)R_{a3}R_{b3}$,

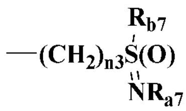
,

$-(CH2)_{n1}NR_{a8}C(O)R_{b8}$, $-(CR_{a8}R_{b8})_{n4}C(O)NR_{a9}R_{b9}$, - $(CH_2)_{n1}S(O)_2R_{b5}$, the amino, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl, 5-8 membered heteroaryl,$-NS(O)R_{a1}R_{b1}$, - $(CH_2)_{n1}S(O)NR_{a2}R_{b2}$, $-P(O)R_{a3}R_{b3}$, $-NR_{a5}S(O)NR_{a4}R_{b4}$ , $-(CH_2)_{n1}O(CH_2)_{n2}R_{a5}$ , - $(CH_2)_{n1}C(O)NR_{a6}R_{b6}$ , $-(CH_2)_{n1}P(O)R_{a3}R_{b3}$,

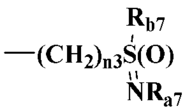
,

$-(CH_2)_{n1}NR_{a8}C(O)R_{b8}$, - $(CR_{a8}R_{b8})_{n4}C(O)NR_{a9}R_{b9}$, $-(CH_2)_{n1}S(O)_2R_{b5}$ are optionally further substituted by one or more substituents selected from

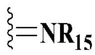
,

halogen, amino, nitro, hydroxyl, cyano, oxo, $-NS(O)R_{a1}R_{b1}$, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heteroaryl, $C_{6-10}$ aryl or 5-8 membered heteroaryl;

Preferably, $R^1$ is independently selected from

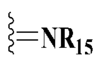
,

hydrogen, deuterium, amino, oxo, fluorine, chlorine, bromine, $C_{1-3}$ alkyl, $C_{1-3}$ deuterated alkyl, $C_{1-3}$ haloalkyl, $C_{2-4}$ alkynyl, $C_{3-8}$ bridged cycloalkyl, $C_{3-8}$ spirocycloalkyl, 3-8 membered spiro heterocyclic containing 1-3 heteroatoms, 5-8 membered heteroaryl, $-NS(O)R_{a1}R_{b1}$, $-(CH_2)_{n1}S(O)NR_{a2}R_{b2}$, $-P(O)R_{a3}R_{b3}$, - $NR_{a5}S(O)NR_{a4}Rb_4$ , $-(CH_2)_{n1}O(CH_2)_{n2}R_{a5}$ , $-(CH_2)_{n1}C(O)NR_{a6}R_{b6}$ , $-(CH_2)_{n1}P(O)R_{a3}R_{b3}$,

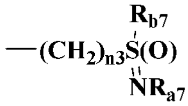, $-(CH_2)_{n1}NR_{a8}C(O)R_{b8}$, $-(CR_{a8}R_{b8})_{n4}C(O)NR_{a9}R_{b9}$, $-(CH_2)_{n1}S(O)_2R_{b5}$ or $-(CH_2)_{n5}C(O)NR_{a10}R_{b10}$, the amino, $C_{1-3}$ alkyl, $C_{1-3}$ deuterated alkyl, $C_{1-3}$ haloalkyl, $C_{2-4}$ alkynyl, $C_{3-8}$ bridged cycloalkyl, $C_{3-8}$ spirocycloalkyl, 3-8 membered spiro heterocyclic containing 1-3 heteroatoms, 5-8 membered heteroaryl, , $-NS(O)R_{a1}R_{b1}$, $-(CH_2)_{a1}S(O)NR_{a2}R_{b2}$, $-P(O)R_{a3}R_{b3}$, $-NR_{a5}S(O)NR_{a4}R_{b4}$ , $-(CH_2)_{n1}O(CH_2)_{n2}R_{a5}$ , $-(CH_2)_{n1}C(O)NR_{a6}R_{b6}$ , $-(CH_2)_{n1}P(O)R_{a3}R_{b3}$,

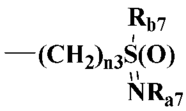,

$-(CH_2)_{a1}NR_{a8}C(O)R_{b8}$, $-(CR_{a8}R_{b8})_{n4}C(O)NR_{a9}R_{b9}$, $-(CH_2)_{n1}S(O)_2R_{b5}$ or $-(CH_2)_{n5}C(O)NR_{a10}R_{b10}$ are optionally further substituted by one or more substituents selected from

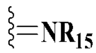,

halogen, cyano or $C_{1-3}$ alkyl.

In a further preferred embodiment of the present invention, the compound of general formula (XVI) is as follows:

Further preferably, $R^1$ is independently selected from

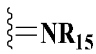,

hydrogen, deuterium, amino, oxo, cyano, fluorine, chlorine, bromine, methyl, ethyl, cyclopropyl, $-CD_3$, , $-NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$ , $-(CH2)_{n1}O(CH_2)_{n2}R_{a5}$ , $-(CH_2)_{n1}C(O)NR_{a6}R_{b6}$ , $-(CH_2)_{n1}P(O)R_{a3}R_{b3}$,

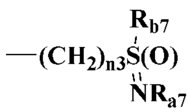,

$-(CH_2)_{n1}NR_{a8}C(O)R_{b8}$, $-(CR_{a8}R_{b8})_{n4}C(O)NR_{a9}R_{b9}$, $-(CH_2)_{n1}S(O)_2R_{b5}$ or $-(CH_2)_{n5}C(O)NR_{a10}R_{b10}$, the amino, methyl, ethyl, cyclopropyl, $-NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$ , $-(CH_2)_{n1}O(CH_2)_{n2}R_{a5}$ , $-(CH_2)_{n1}C(O)NR_{a6}R_{b6}$ , $-(CH_2)_{n1}P(O)R_{a3}R_{b3}$,

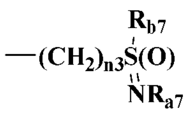, $-(CH_2)_{n1}NR_{a8}C(O)R_{b8}$, $-(CR_{a8}R_{b8})_{n4}C(O)NR_{a9}R_{b9}$, $-(CH_2)_{n1}S(O)_2R_{b5}$ or $-(CH_2)_{a5}C(O)NR_{a10}R_{b10}$ are optionally further substituted by one or more substituents selected from

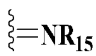,

halogen, cyano or $C_{1-3}$ alkyl.

In a further preferred embodiment of the present invention, the compound of general formula (XVI) is as follows:

$R^1$ is respectively linked to the atoms connected thereto to form $C_{3-6}$ cycloalkyl, 3-10 membered heterocyclic containing 1-3 selected from N, O or S, $C_{6-10}$ aryl, or 5-8 membered heteroaryl containing 1-3 selected from N, O or S, which are optionally further substituted by one or more substituents selected from

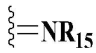,

deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl, 5-8 membered heteroaryl, $-NS(O)R_{a1}R_{b1}$, $-(CH_2)_{n1}S(O)NR_{a2}R_{b2}$, - $P(O)R_{a3}R_{b3}$, $-NR_{a5}S(O)NR_{a4}R_{b4}$, $-(CH2)_{n1}S(O)_2R_{a5}$, $-(CH_2)_{n1}O(CH_2)_{n2}R_{b5}$,

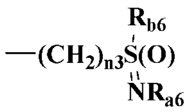, -

$(CH_2)_{n1}NR_{a8}C(O)R_{b8}$, $-(CR_{a8}R_{b8})_{na}C(O)NR_{a9}R_{b9}$, $-(CH_2)_{n1}S(O)_2R_{b5}$ or $-(CH_2)_{n5}C(O)NR_{a10}R_{b10}$;

Preferably, $R^1$ is respectively linked to the atoms connected thereto to form $C_{3-4}$ cycloalkyl, 5-7 membered heterocyclic containing 1-3 selected from N, O or S, which are optionally further substituted by one or more substituents selected from

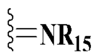,

, deuterium, halogen, $C_{1-3}$ alkyl, $C_{1-3}$ deuterated alkyl, $C_{3-6}$ cycloalkyl, amino, oxo, $-NS(O)R_{a1}R_{b1}$, - $(CH_2)_{n1}S(O)NR_{a2}R_{b2}$, $-P(O)R_{a3}R_{b3}$, $-NR_{a5}S(O)NR_{a4}R_{b4}$, $-(CH_2)_{n1}S(O)_2R_{a5}$, $-(CH_2)_{n1}O(CH_2)_{n2}R_{b5}$,

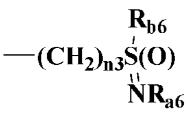,

$-(CH_2)_{n1}NR_{a8}C(O)R_{b8}$, $-(CR_{a8}R_{b8})_{na}C(O)NR_{a9}R_{b9}$, $-(CH_2)_{n1}S(O)_2R_{b5}$ or - $(CH_2)_{n5}C(O)NR_{a10}Rb_{10}$;

Further preferably, $R^1$ is respectively linked to the atoms connected thereto to form cyclopropyl, which are optionally further substituted by one or more substituents selected from

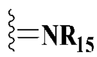,

deuterium, halogen, $C_{1-3}$ alkyl, $C_{1-3}$ deuterated alkyl, $C_{3-6}$ cycloalkyl, amino, oxo, - $NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$, $-(CH_2)_{n1}S(O)_2R_{a5}$, - $(CH_2)_{n1}O(CH_2)_{n2}R_{b5}$,

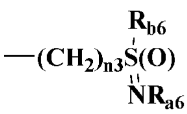,

$-(CH_2)_{n1}NR_{a8}C(O)R_{b8}$, $-(CR_{a8}R_{b8})_{n4}C(O)NR_{a9}R_{b9}$, - $(CH_2)_{n1}S(O)_2R_{b5}$ or $-(CH_2)_{n5}C(O)NR_{a10}R_{b10}$.

In a further preferred embodiment of the present invention, the compound of general formula (XVI) is as follows:

$R_{a1}$, $R_{b1}$, $R_{a2}$, $R_{b2}$, $R_{a3}$, $R_{b3}$, $R_{a4}$, $R_{b4}$, $R_{a5}$, $R_{a6}$, $R_{b6}$, $R_{a7}$, $R_{b7}$, $R_{a8}$, $R_{b8}$, $R_{a9}$, $R_{b9}$, $Ra_{10}$ or $Rb_{10}$ are each independently selected from hydrogen, deuterium, halogen, amino, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ alkoxy, $C_{1-3}$ deuterated alkoxy, $C_{1-3}$ haloalkoxy, $C_{1-3}$ deuterated haloalkoxy, $C_{3-6}$ bridged cycloalkyl, $C_{3-6}$ spirocycloalkyl, 3-8 membered heterocyclic containing $_{1-3}$

heteroatoms selected from N, O or S, $C_{6-10}$ aryl or 5-6 membered heteroaryl containing $_{1-3}$ heteroatoms selected from N, O or S, the amino $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ alkoxy, $C_{1-3}$ deuterated alkoxy, $C_{1-3}$ haloalkoxy, $C_{1-3}$ deuterated haloalkoxy, $C_{3-6}$ bridged cycloalkyl, $C_{3-6}$ spirocycloalkyl, 3-8 membered heterocyclic containing $_{1-3}$ heteroatoms selected from N, O or S, $C_{6-10}$ aryl or 5-6 membered heteroaryl containing $_{1-3}$ heteroatoms selected from N, O or S are optionally further substituted by one or more substituents of halogen or $C_{1-3}$ alkyl;

Preferably, $R_{a1}$, $R_{b1}$, $R_{a2}$, $R_{b2}$, $R_{a3}$, $R_{b3}$, $R_{a4}$, $R_{b4}$, $R_{a5}$, $R_{a6}$, $R_{b6}$, $R_{a7}$, $R_{b7}$, $R_{a8}$, $R_{b8}$, $R_{a9}$, $R_{b9}$, $Ra_{10}$ or $R_{b10}$ are each independently selected from hydrogen, deuterium, halogen, methyl, ethyl, cyano, deuterated methyl, cyclopropyl, cyclopentyl, phenyl,

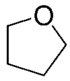、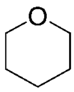、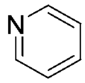、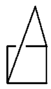

or

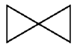,

the methyl, ethyl, deuterated methyl, cyclopropyl, cyclopentyl, phenyl, phenyl,

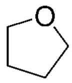、

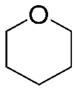、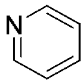、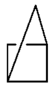 or 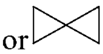

are optionally further substituted with one or more fluorine.

In a further preferred embodiment of the present invention, the compound of general formula (XVI) is as follows:

$R_{a1}$ and $R_{b1}$, $R_{a2}$ and $R_{b2}$, $R_{a3}$ and $R_{b3}$, $R_{a4}$ and $R_{b4}$, $R_{a6}$ and $R_{b6}$, $R_{a8}$ and $R_{b8}$, $R_{a9}$ and $R_{b9}$, $R_{a10}$ and $R_{b10}$ are linked with adjacent atoms to form $C_{3-8}$ cycloalkyl, 3-8 membered heterocyclic containing 1-3 heteroatoms selected from N, O or S, $C_{6-10}$ aryl, or 5-8 membered heteroaryl containing 1-3 heteroatoms selected from N, O or S, which are optionally further substituted by one or more substituents selected from halogen, amino, nitro, cyano, oxo, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl or 5-8 membered heteroaryl;

Preferably, $R_{a1}$ and $R_{b1}$, $R_{a2}$ and $R_{b2}$, $R_{a3}$ and $R_{b3}$, $R_{a4}$ and $R_{b4}$, $R_{a6}$ and $R_{b6}$, $R_{a8}$ and $R_{b8}$, $R_{a9}$ and $R_{b9}$, $R_{a10}$ and $R_{b10}$ are linked with adjacent atoms to form $C_{3-6}$ cycloalkyl, 3-6 membered heterocyclic containing 1-3 heteroatoms selected from N, O or S, which are optionally further substituted by one or more substituents selected from halogen, amino, thio or $C_{1-3}$ alkyl.

In a further preferred embodiment of the present invention, the compound of general formula (XVI) is as follows:

$R^4$, $R^5$ or $R^6$ is each independently selected from hydrogen, deuterium, halogen, amino, nitro, hydroxyl, cyano, oxo, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl, the amino, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl or 5-8 membered heteroaryl, are optionally further substituted by one or more substituents selected from Halogen, amino, nitro, hydroxyl, cyano, oxo, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl or 5-8 membered heteroaryl;

Preferably, $R^4$, $R^5$ or $R^6$ is each independently selected from fluorine, cyano, cyclopropyl or methyl, the cyclopropyl or methyl are optionally further substituted with one or more substituents selected from deuterium, deuterated methyl, methyl, cyclopropyl or oxo.

In a further preferred embodiment of the present invention, the compound of general formula (XVI) is as follows:

$R^7$, $R^8$, $R^9$, $R^{10}$, $R^{12}$, $R^{13}$, $R^{14}$ or $R^{15}$ are each independently selected from hydrogen, deuterium, halogen, amino, nitro, hydroxyl, cyano, oxo, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclyl, $C_{6-10}$ aryl, 5-8 membered heteroaryl or -$(CH_2)_{m1}OR_{c1}$, the amino, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclyl, $C_{6-10}$ aryl, 5-8 membered heteroaryl, or -$(CH_2)_{m1}OR_{c1}$ are optionally further substituted by one or more substituents selected from deuterium, halogen, amino, nitro, hydroxyl, cyano, oxo, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl or 5-8 membered heteroaryl;
Preferably, $R^7$, $R^8$, $R^9$, $R^{10}$, $R^{12}$, $R^{13}$, $R^{14}$ or $R^{15}$ are each independently selected from hydrogen, deuterium, halogen, cyano, $C_{2-4}$ alkynyl, $C_{3-6}$ cycloalkyl, $C_{1-3}$ alkyl, $C_{1-3}$ deuterated alkyl, $C_{1-3}$ haloalkyl or -$(CH_2)_{m1}OR_{c1}$, the $C_{2-4}$ alkynyl, $C_{3-6}$ cycloalkyl, $C_{1-3}$ alkyl, $C_{1-3}$ deuterated alkyl, $C_{1-3}$ haloalkyl or -$(CH_2)_{m1}OR_{c1}$ are optionally further substituted with one or more substituents selected from deuterium, deuterated methyl, methyl, cyclopropyl or oxo;
Further preferably, $R^7$, $R^8$, $R^9$, $R^{10}$, $R^{12}$, $R^{13}$, $R^{14}$ or $R^{15}$ are each independently selected from cyano, methyl, fluoro, cyclopropyl, ethynyl or -$(CH_2)_{m1}OR_{c1}$ are optionally further substituted with one or more substituents selected from deuterium, deuterated methyl, methyl, cyclopropyl or oxo.

In a further preferred embodiment of the present invention, the compound of general formula (XVI) is as follows: $R^7$, $R^8$, $R^9$, $R^{10}$, $R^{12}$, $R^{13}$, $R^{14}$ or $R^{15}$ are each independently selected from cyano, methyl, fluoro, cyclopropyl, ethynyl or -$(CH_2)_{m1}OR_{c1}$ are optionally further substituted with one or more substituents selected from deuterium, deuterated methyl, methyl, cyclopropyl or oxo.

In a further preferred embodiment of the present invention, the compound of general formula (XVI) is as follows:

$R_{c1}$ is selected from hydrogen, deuterium, halogen, amino, nitro, hydroxyl, cyano, oxo, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl, or 5-8 membered heteroaryl, the amino, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl or 5-8 membered heteroaryl are optionally further substituted by one or more substituents selected from halogen, amino, nitro, hydroxyl, cyano, oxo, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl or 5-8 membered heteroaryl;
Preferably, $R_{c1}$ is selected from fluorine, methyl, cyano, difluoromethyl, trifluoromethyl, ethynyl, methylthio, amino, cyclopropyl or oxetane, the methyl, difluoromethyl, trifluoromethyl, ethynyl, methylthio, amino, cyclopropyl or oxetane are optionally further substituted by one or more substituents of halogen, methoxy or deuterated methyl.

In a further preferred embodiment of the present invention, the compound of general formula (XVI) is as follows:

$R^7$, $R^8$, $R^9$, $R^{10}$, $R^{12}$, $R^{13}$, $R^{14}$ or $R^{15}$ are linked to the atoms connected thereto to form $C_{3-6}$ cycloalkyl, 3-8 membered heterocyclic containing 1-3 N, O or S atoms, $C_{6-10}$ aryl or 5-8 membered heteroaryl containing 1-3 N, O or S atoms;
Preferably, $R^7$, $R^8$, $R^9$, $R^{10}$, $R^{12}$, $R^{13}$, $R^{14}$ or $R^{15}$ are linked to the atoms connected thereto to form $C_{3-6}$ cycloalkyl, 3-6 membered heterocyclic containing 1-3 N, O or S atoms, $C_{6-10}$ aryl or 5-8 membered heteroaryl containing 1-3 N, O or S atoms;
Further preferably, $R^7$, $R^8$, $R^9$, $R^{10}$, $R^{12}$, $R^{13}$, $R^{14}$ or $R^{15}$ are linked to the atoms connected thereto to form cyclopropyl or

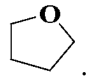

In a further preferred embodiment of the present invention, the compound of general formula (XVI) is as follows:

$R^{10}$ and $R^{12}$ are linked to the atoms connected thereto to form $C_{3-8}$ cycloalkyl, 3-8 membered heterocyclic containing 1-3 N, O or S atoms, $C_{6-10}$ aryl or 5-10 membered heteroaryl containing 1-3 N, O or S atoms;
Preferably, $R^{10}$ and $R^{12}$ are linked to the atoms connected thereto to form $C_{3-6}$ cycloalkyl, 3-6 membered heterocyclic containing 1-3 N, O or S atoms, $C_{6-10}$ aryl or 5-8 membered heteroaryl containing 1-3 N, O or S atoms;
Further preferably, $R^{10}$ and $R^{12}$ are linked to the atoms connected thereto to form

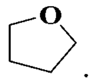

In a further preferred embodiment of the present invention, the compound of general formula (XVI) is as follows:

$R^{10}$ is linked to the atoms connected thereto and adjacent atoms to form 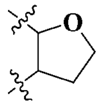

or

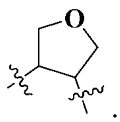

In a further preferred embodiment of the present invention, the compound of general formula (XVI) is as follows: Ring F is selected from phenyl, pyridyl, pyrimidinyl, thiazolyl, pyridazinyl, oxazolyl, morpholinyl,

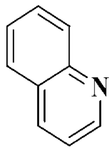、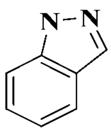、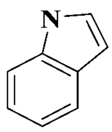、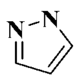、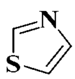、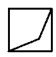、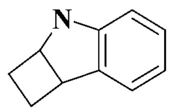、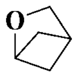、

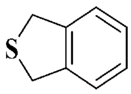、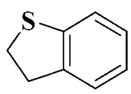、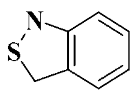、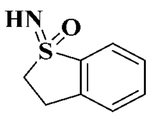、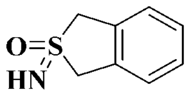、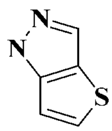、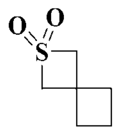、

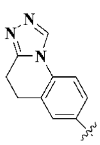、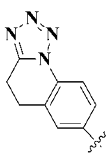、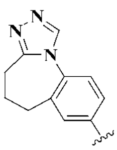、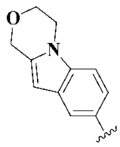、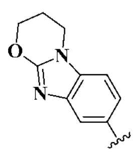、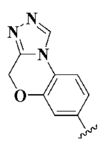、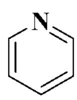、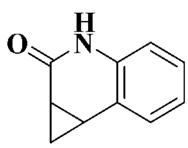、

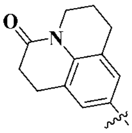、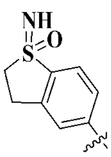、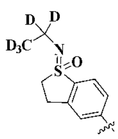、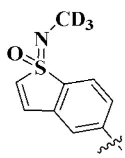、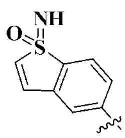、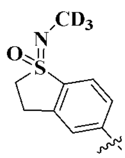、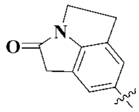、

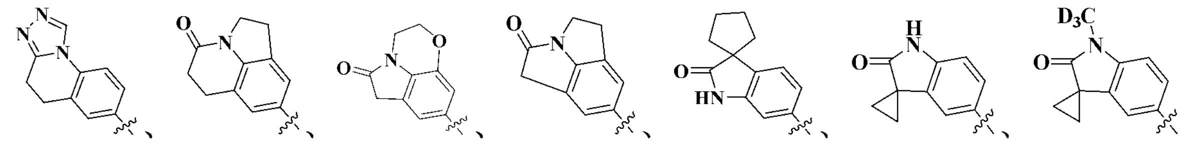, , , , , , ,

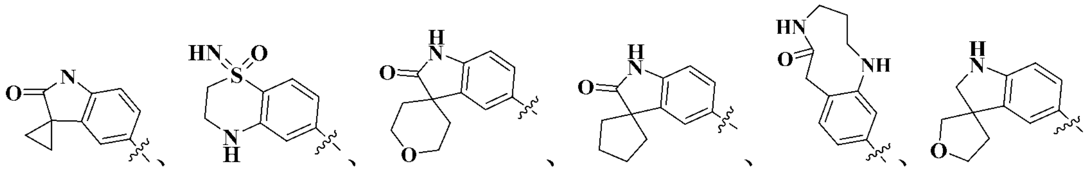, , , , , ,

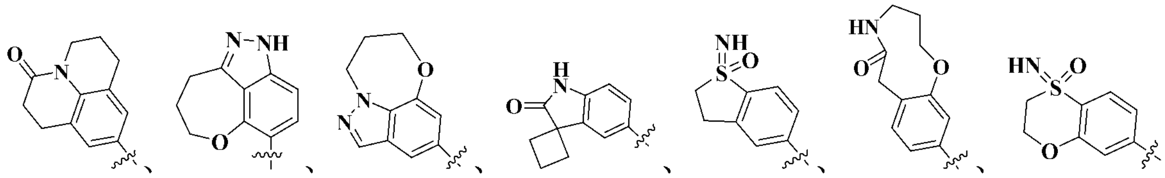, , , , , , ,

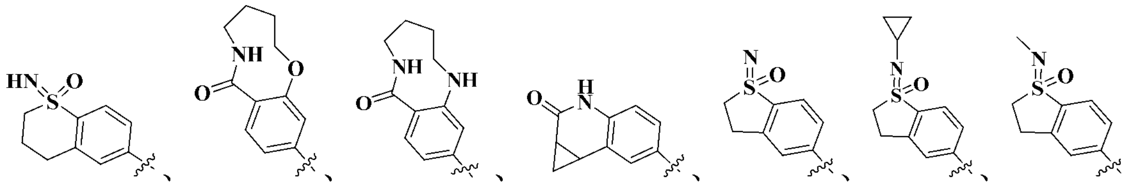, , , , , , ,

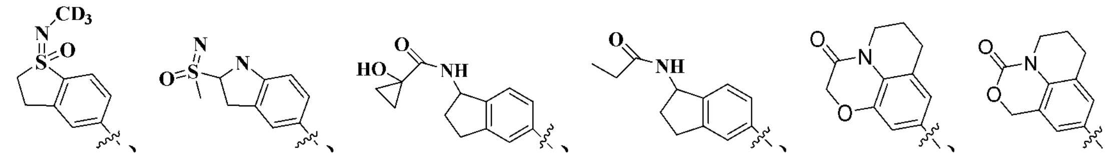, , , , , ,

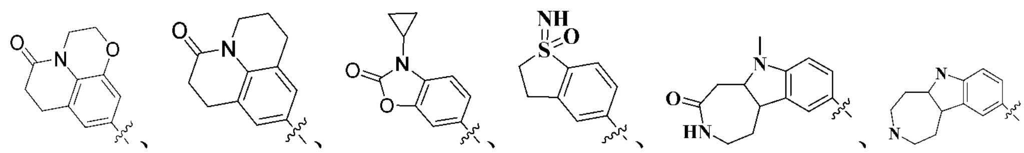, , , , , ,

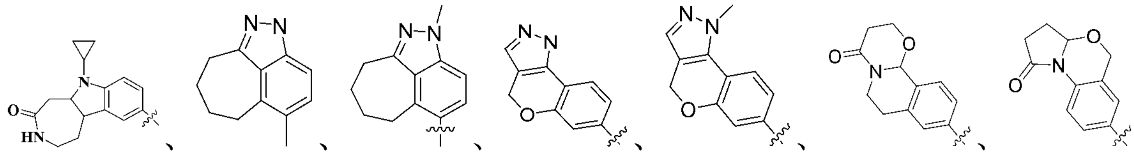, , , , , , ,

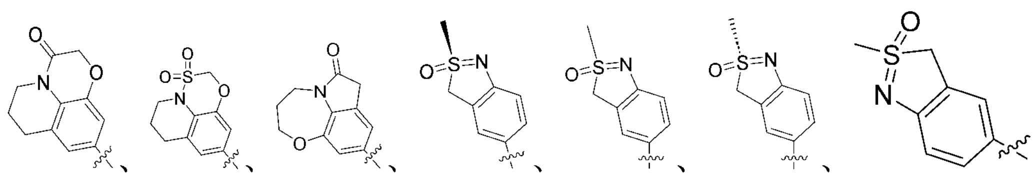, , , , , , ,

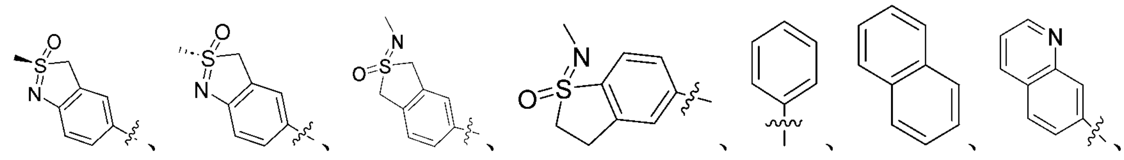, , , , , , ,

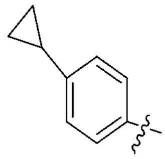、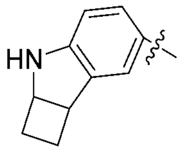、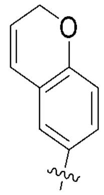、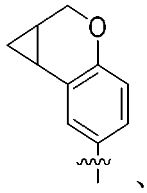、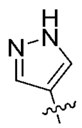、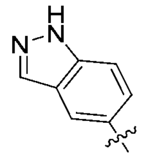、

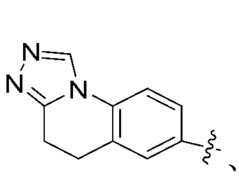、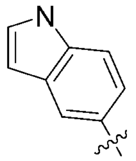、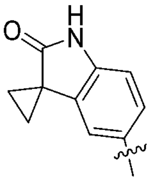、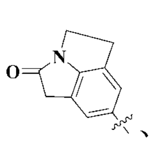、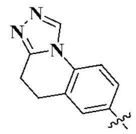、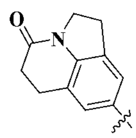、

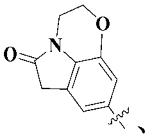、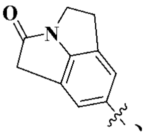、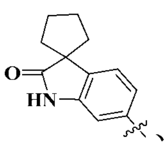、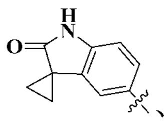、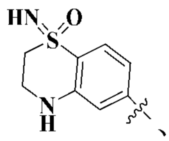、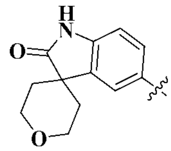、

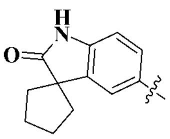、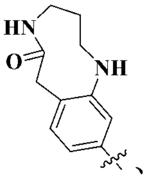、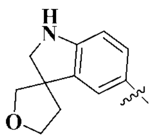、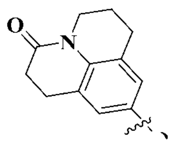、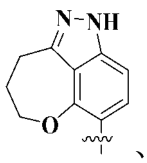、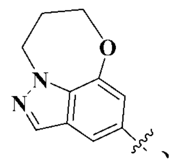、

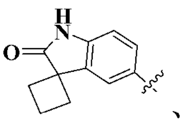、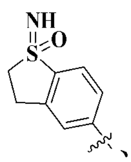、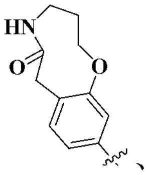、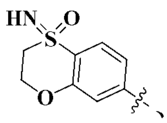、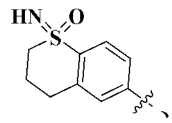、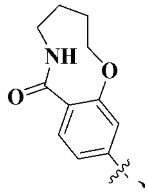、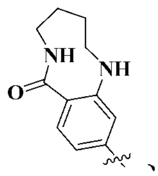、

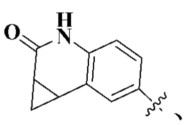、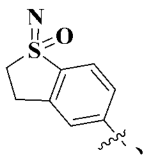、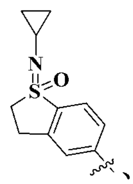、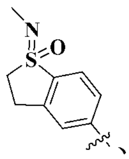、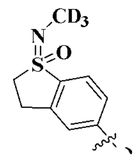、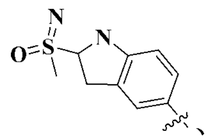、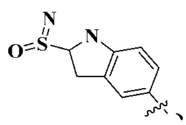、

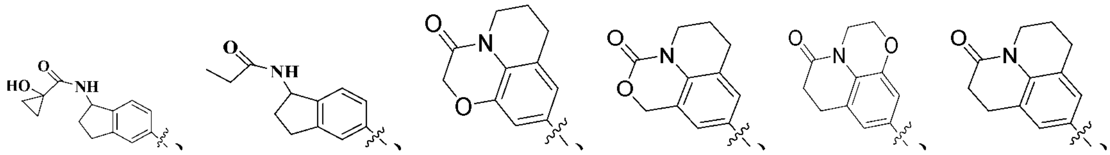
、、、、、、

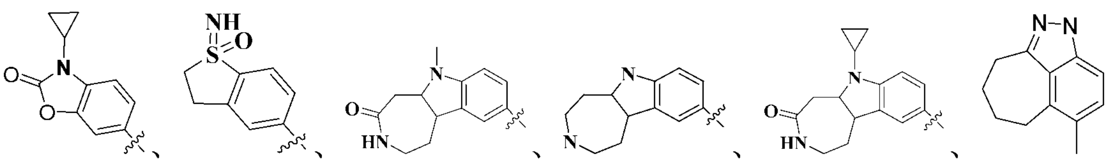
、、、、、、

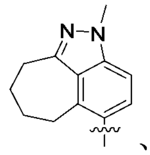, 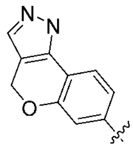, 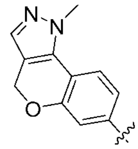, 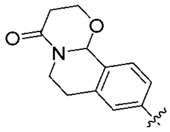, 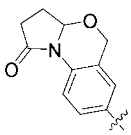, 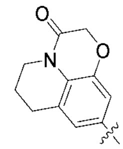, 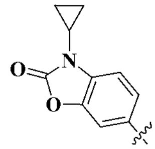

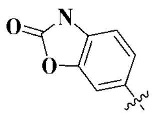 or 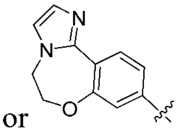.

Note:

“ 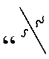 ”

indicates connected to $L_1$.

In a further preferred embodiment of the present invention, the compound of general formula (XVI) is further represented by general formula (XVI-1):

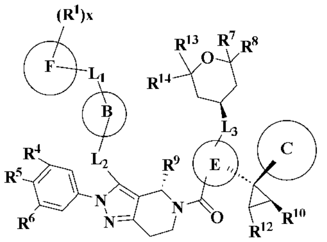

(XVI-1)

.

In a further preferred embodiment of the present invention, the compound of general formula (XVI) is further represented by general formula (XVI-A):

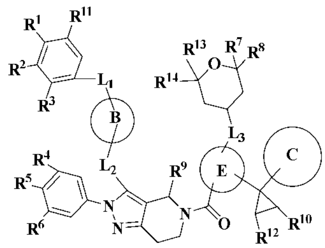

(XVI-A)

wherein,

$R^1$ and $R^2$, $R^1$ and $R^{11}$ are linked to adjacent atoms to form a $C_{3-12}$ cycloalkyl, 3-12 membered heterocyclic, $C_{6-14}$ aryl or 5-14 membered heteroaryl, which are optionally further substituted by one or more substituents selected from

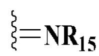,

, hydrogen, deuterium, halogen, amino, nitro, hydroxyl, cyano, oxo, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl, 5-10 membered heteroaryl, $-NS(O)R_{a1}R_{b1}$, - $(CH2)_{n1}S(O)NR_{a2}R_{b2}$, $-P(O)R_{a3}R_{b3}$, $-NR_{a5}$-$S(O)NR_{a4}R_{b4}$ , $(CH2)_{n1}S(O)2R_{a5}$, $-(CH_2)_{n1}O(CH_2)_{n2}R_{a5}$ ,

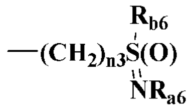,

$-(CH_2)_{n1}NR_{a8}C(O)R_{b8}$, $-(CR_{a8}R_{b8})_{n4}C(O)NR_{a9}R_{b9}$, $-(CH_2)_{n1}S(O)_2R_{b5}$ or $-(CH_2)_{n5}C(O)NR_{a10}R_{b10}$,

Alternatively,

$R^1$, $R^2$, $R^3$ or $R^{11}$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl, 5-10 membered heteroaryl, $-NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$ , $-(CH_2)_{n1}O(CH_2)_{n2}R_{a5}$ , $-(CH_2)_{n1}C(O)NR_{a6}R_{b6}$, $-(CH_2)_{n1}P(O)R_{a3}R_{b3}$,

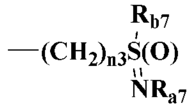,

$-(CH_2)_{n1}NR_{a8}C(O)R_{b8}$, $-(CR_{a8}R_{b8})_{n4}C(O)NR_{a9}R_{b9}$, $-(CH_2)_{n1}S(O)_2R_{b5}$ or $-(CH_2)_{n5}C(O)NR_{a10}R_{b10}$, the amino, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl, 5-10 membered heteroaryl, $-NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$ , $-(CH_2)_{n1}O(CH_2)_{n2}R_{a5}$ , $-(CH_2)_{n1}C(O)NR_{a6}R_{b6}$ , $-(CH_2)_{n1}P(O)R_{a3}R_{b3}$,

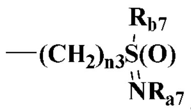, -

$(CH_2)_{n1}NR_{a8}C(O)R_{b8}$, $-(CR_{a8}R_{b8})_{n4}C(O)NR_{a9}R_{b9}$, $-(CH_2)_{n1}S(O)_2R_{b5}$ or $-(CH_2)_{a5}C(O)NR_{a10}R_{b10}$ are optionally further substituted by one or more substituents selected from halogen, amino, nitro, hydroxyl, cyano, oxo, -NS(O) Ra1Rb1, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R_{a1}$, $R_{b1}$, $R_{a2}$, $R_{b2}$, $R_{a3}$, $R_{b3}$, $R_{a4}$, $R_{b4}$, $R_{a5}$, $R_{a6}$, $R_{b6}$, $R_{a7}$, $R_{b7}$, $R_{a5}$, $R_{b8}$, $R_{a9}$, $R_{b9}$, $Ra_{10}$ or $R_{b10}$ are each independently selected from hydrogen, deuterium, halogen, amino, nitro, hydroxyl, cyano, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, the amino, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl are optionally further substituted by one or more substituents selected from halogen, amino, nitro, hydroxyl, cyano, oxo, $-NS(O)R_{a1}R_{b1}$, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

alternatively,

$R_{a1}$ and $R_{b1}$, $R_{a2}$ and $R_{b2}$, $R_{a3}$ and $R_{b3}$, $R_{a4}$ and $R_{b4}$, $R_{a6}$ and $R_{b6}$, $R_{a8}$ and $R_{b8}$, $R_{a9}$ and $R_{b9}$, $R_{a10}$ and $R_{b10}$ are linked with adjacent atoms to form $C_{3-10}$ cycloalkyl, 3-10 membered heterocyclic, C6-12 aryl or 5-12 membered heteroaryl, which are optionally further substituted by one or more substituents selected from halogen, amino, nitro, cyano, oxo, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

Ring B, ring C, ring E, $L_1$, $L_2$, $L_3$, $R^4$, $R^5$, $R^6$, $R^7$, $R^8$, $R^9$, $R^{10}$, $R^{12}$, $R^{13}$, $R^{14}$, $R^{15}$, n1, n2, n3, n4, n5, m1 and x are as defined in the general formula (XVI).

In a further preferred embodiment of the present invention, the compound of general formula (XVI-A) is as follows:

$R^1$ and $R^2$, $R^1$ and $R^{11}$ are linked to adjacent atoms to form $C_{3-6}$ cycloalkyl, 3-10 membered heterocyclic containing 1-3 selected from N, O or S, $C_{6-10}$ aryl or 5-8 membered heteroaryl containing 1-3 selected from N, O or S, which are optionally further substituted by one or more substituents selected from

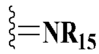,

, deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl, 5-8 membered heteroaryl, $NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$, $(CH_2)_{n1}S(O)_2R_{a5}$, $-(CH_2)_{n1}O(CH_2)_{n2}R_{a5}$,

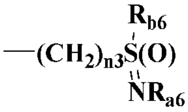,

$-(CH_2)_{n1}NR_{a8}C(O)R_{b8}$, $-(CR_{a8}R_{b8})_{n4}C(O)NR_{a9}R_{b9}$, $-(CH_2)_{n1}S(O)_2R_{b5}$ or $-(CH_2)_{n5}C(O)NR_{a10}R_{b10}$;

Preferably, $R^1$ and $R^2$, $R^1$ and $R^{11}$ are respectively linked to adjacent atoms to form 5-7 membered heterocyclic containing 1-3 selected from N, O or S, which are optionally further substituted by one or more substituents selected from

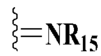,

, deuterium, halogen, $C_{1-3}$ alkyl, $C_{1-3}$ deuterated alkyl, $C_{3-6}$ cycloalkyl, amino, oxo, $NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$, $(CH_2)_{n1}S(O)_2R_{a5}$, $-(CH_2)_{n1}O(CH_2)_{n2}R_{a5}$,

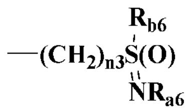,

$-(CH_2)_{n1}NR_{a8}C(O)R_{b8}$, $-(CR_{a8}R_{b8})_{n4}C(O)NR_{a9}R_{b9}$, $-(CH_2)_{n1}S(O)_2R_{b5}$ or $-(CH_2)_{n5}C(O)NR_{a10}R_{b10}$;

Further preferably, $R^1$ and $R^2$, $R^1$ and $R^{11}$ are linked to adjacent phenyl to form

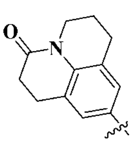、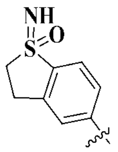、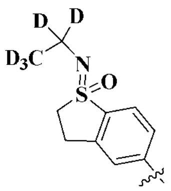、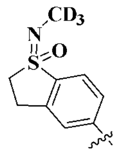、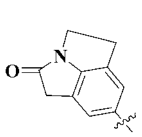、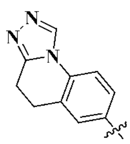、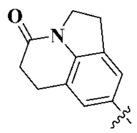、

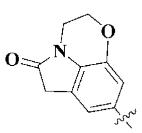、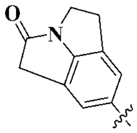、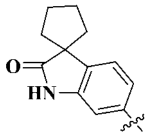、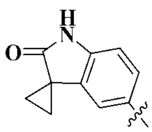、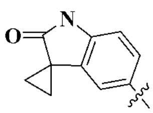、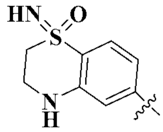、

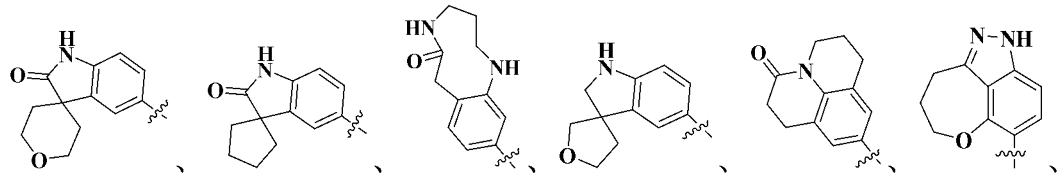

, , , , , ,

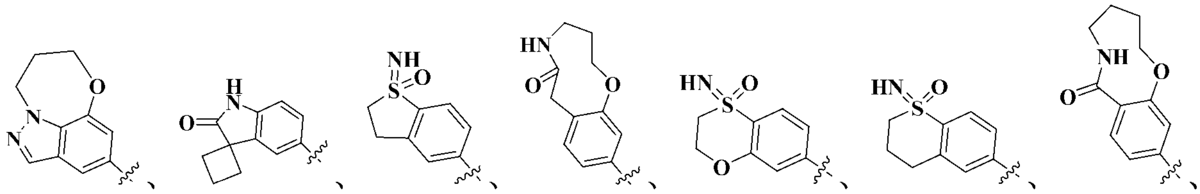

, , , , , , ,

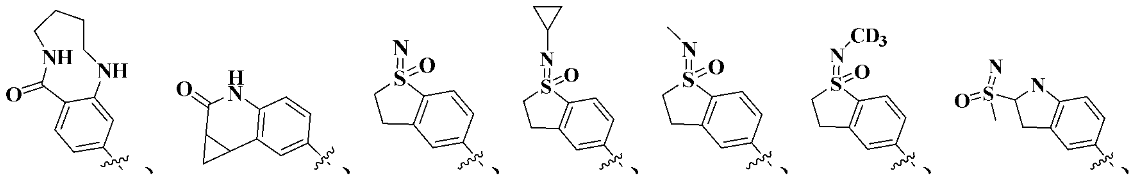

, , , , , , ,

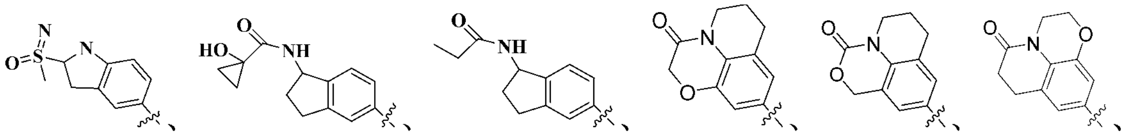

, , , , , ,

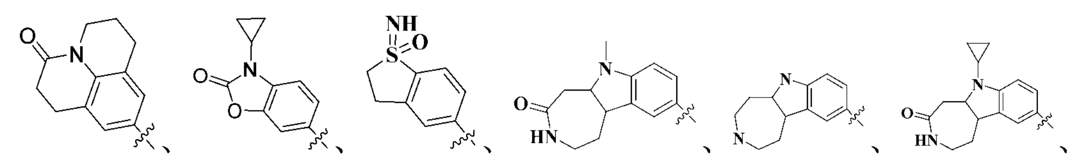

, , , , , ,

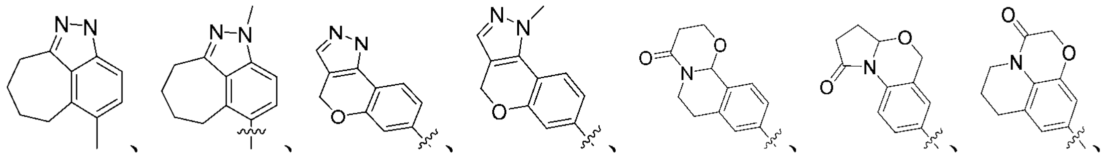

, , , , , , ,

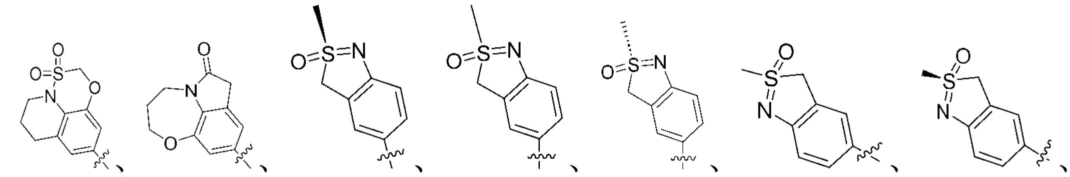

, , , , , , ,

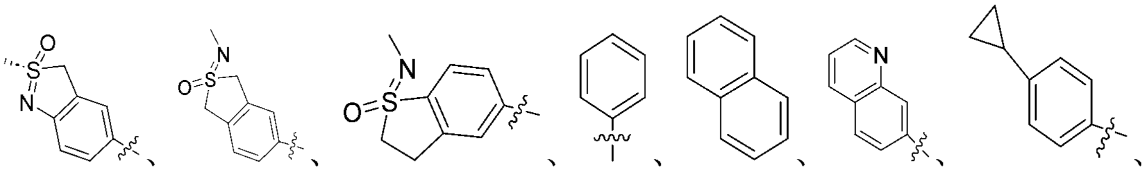

, , , , , , ,

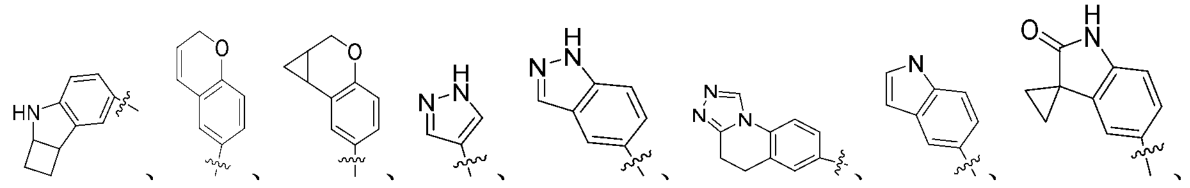
, , , , , , , ,

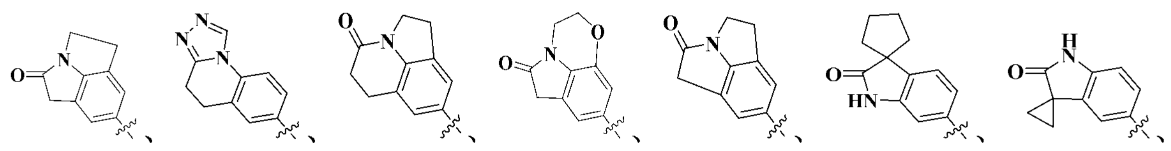
, , , , , , ,

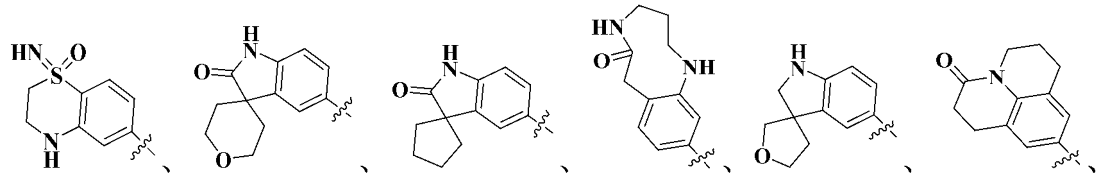
, , , , , ,

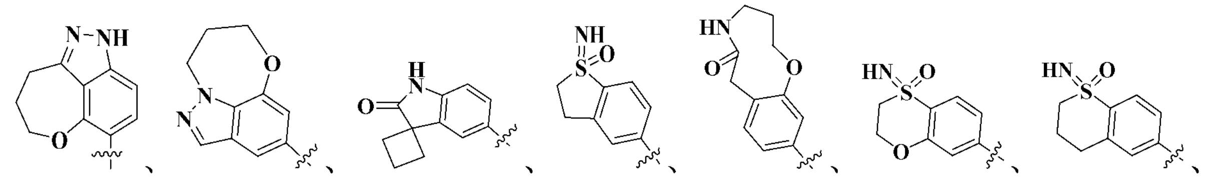
, , , , , , ,

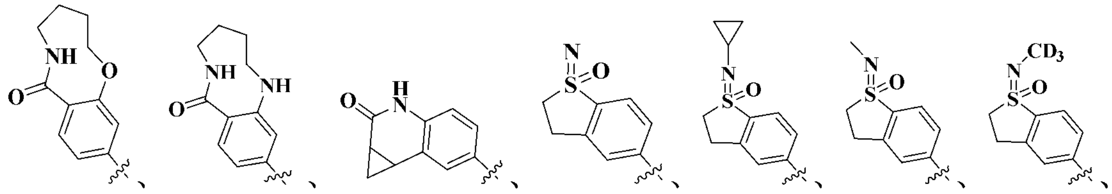
, , , , , , ,

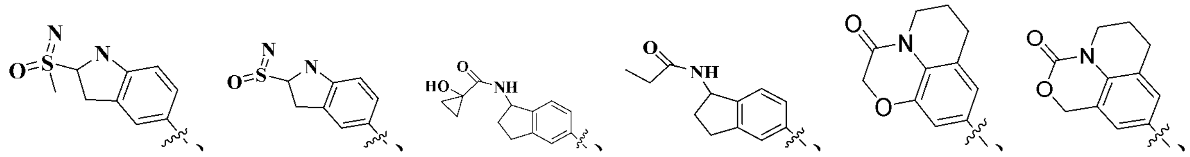
, , , , , ,

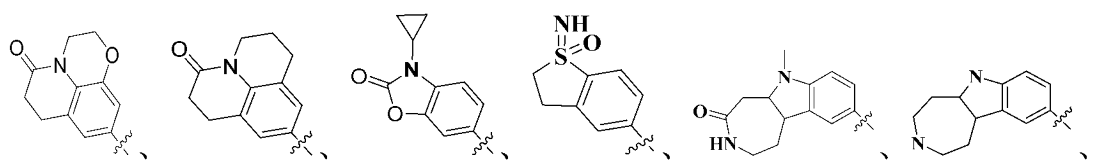
, , , , , ,

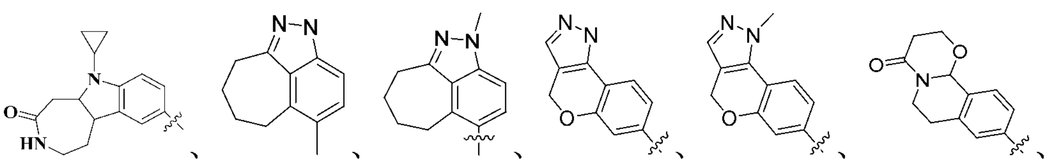
, , , , , ,

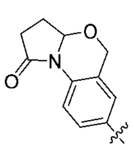, 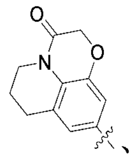, 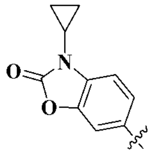 or 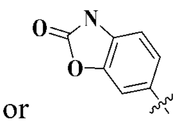,

which are optionally further substituted by one or more substituents selected from $NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$, $(CH_2)_{n1}S(O)_2R_{a5}$, $-(CH_2)_{n1}O(CH_2)_{n2}R_{b5}$,

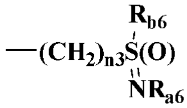, -

$(CH_2)_{n1}NR_{a8}C(O)R_{b8}$, $-(CR_{a8}R_{b8})_{n4}C(O)NR_{a9}R_{b9}$, $-(CH_2)_{n1}S(O)_2R_{b5}$ or $-(CH_2)_{n5}C(O)NR_{a10}R_{b10}$,

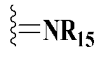,

deuterium, halogen, $C_{1-3}$ alkyl, $C_{1-3}$ deuterated alkyl, $C_{3-6}$ cycloalkyl, amino or oxo.

Note:

“ 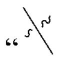 ”

indicates connected to $L_1$.

In a further preferred embodiment of the present invention, the compound of general formula (XVI-A) is as follows:

$R^1$, $R^2$, $R^3$ or $R^{11}$ are each independently selected from hydrogen, deuterium, halogen, amino, nitro, hydroxyl, cyano, oxo, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclyl, $C_{6-10}$ aryl, 5-8 membered heteroaryl, $-NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$, $-(CH_2)_{n1}O(CH_2)_{n2}R_{a5}$, $-(CH_2)_{n1}C(O)NR_{a6}R_{b6}$, $-(CH_2)_{n1}P(O)R_{a3}R_{b3}$,

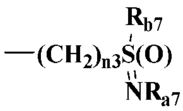,

$-(CH_2)_{n1}NR_{a8}C(O)R_{b8}$, $-(CR_{a8}R_{b8})_{n4}C(O)NR_{a9}R_{b9}$, $-(CH_2)_{n1}S(O)_2R_{b5}$ or $-(CH_2)_{n5}C(O)NR_{a10}R_{b10}$, the amino, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl, 5-8 membered heteroaryl, $-NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$, $-(CH_2)_{n1}O(CH_2)_{n2}R_{a5}$, $-(CH_2)_{n1}C(O)NR_{a6}R_{b6}$, $-(CH_2)_{n1}P(O)R_{a3}R_{b3}$,

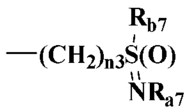, -

$(CH_2)_{n1}NR_{a8}C(O)R_{b8}$, $-(CR_{a8}R_{b8})_{n4}C(O)NR_{a9}R_{b9}$, $-(CH_2)_{n1}S(O)_2R_{b5}$ or $-(CH_2)_{a5}C(O)NR_{a10}R_{b10}$ are optionally further substituted by one or more substituents selected from halogen, amino, nitro, hydroxyl, cyano, oxo, -NS(O) Ra1Rb1, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl or 5-8 membered heteroaryl;

Preferably, $R^1$, $R^2$, $R^3$ or $R^{11}$ is each independently selected from hydrogen, deuterium, amino, oxo, fluorine, chlorine,

bromine, $C_{1-3}$ alkyl, $C_{1-3}$ deuterated alkyl, $C_{1-3}$ haloalkyl, $C_{2-4}$ alkynyl, $C_{3-8}$ bridged cycloalkyl, $C_{3-8}$ spirocycloalkyl, 3-8 membered spiro heterocyclic containing $_{1-3}$ heteroatoms, 5-8 membered heteroaryl, $-NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$ , $-(CH_2)_{n1}O(CH_2)_{n2}R_{a5}$ , $-(CH_2)_{n1}C(O)NR_{a6}R_{b6}$ , $-(CH_2)_{n1}P(O)R_{a3}R_{b3}$,

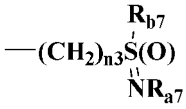,

$-(CH_2)_{n1}NR_{a8}C(O)R_{b8}$, $-(CR_{a8}R_{b8})_{n4}C(O)NR_{a9}R_{b9}$, $-(CH_2)_{n1}S(O)_2R_{b5}$ or $-(CH_2)_{n5}C(O)NR_{a10}R_{b10}$, the amino, $C_{1-3}$ alkyl, $C_{1-3}$ deuterated alkyl, $C_{1-3}$ haloalkyl, $C_{2-4}$ alkynyl, $C_{3-8}$ bridged cycloalkyl, $C_{3-8}$ spirocycloalkyl, 3-8 membered spiro heterocyclic containing 1-3 heteroatoms, 5-8 membered heteroaryl, $-NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$ , $-(CH_2)_{n1}O(CH_2)_{n2}R_{a5}$ , $-(CH_2)_{n1}C(O)NR_{a6}R_{b6}$, $-(CH_2)_{n1}P(O)R_{a3}R_{b3}$,

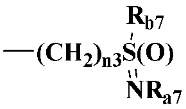,

$-(CH_2)_{n1}NR_{a8}C(O)R_{b8}$, $-(CR_{a8}R_{b8})_{n4}C(O)NR_{a9}R_{b9}$, $-(CH_2)_{n1}S(O)_2R_{b5}$ or $-(CH_2)_{n5}C(O)NR_{a10}R_{b10}$ are optionally further substituted by one or more substituents selected from halogen, cyano or $C_{1-3}$ alkyl.

In a further preferred embodiment of the present invention, the compound of general formula (XVI-A) is as follows:

$R_{a1}$, $R_{b1}$, $R_{a2}$, $R_{b2}$, $R_{a3}$, $R_{b3}$, $R_{a4}$, $R_{b4}$, $R_{a5}$, $R_{a6}$, $R_{b6}$, $R_{a7}$, $R_{b7}$, $R_{a5}$, $R_{b8}$, $R_{a9}$, $R_{b9}$, $Ra_{10}$ or $Rb_{10}$ is each independently selected from hydrogen, deuterium, halogen, amino, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ alkoxy, $C_{1-3}$ deuterated alkoxy, $C_{1-3}$ haloalkoxy, $C_{1-3}$ deuterated haloalkoxy, $C_{3-6}$ bridged cycloalkyl, $C_{3-6}$ spirocycloalkyl, 3-8 membered heterocyclic containing $_{1-3}$ heteroatoms selected from N, O or S, $C_{6-10}$ aryl or 5-6 membered heterocyclic containing 1-3 heteroatoms selected from N, O or S, the amino, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ alkoxy, $C_{1-3}$ deuterated alkoxy, $C_{1-3}$ haloalkoxy, $C_{1-3}$ deuterated haloalkoxy, $C_{3-6}$ bridged cycloalkyl, $C_{3-6}$ spirocycloalkyl, 3-8 membered heterocyclic containing $_{1-3}$ heteroatoms selected from N, O or S, $C_{6-10}$ aryl or 5-6 membered heteroaryl containing 1-3 heteroatoms selected from N, O or S are optionally further substituted by one or more substituents of halogen or $C_{1-3}$ alkyl;
Preferably, $R_{a1}$, $R_{b1}$, $R_{a2}$, $R_{b2}$, $R_{a3}$, $R_{b3}$, $R_{a4}$, $R_{b4}$, $R_{a5}$, $R_{a6}$, $R_{b6}$, $R_{a7}$, $R_{b7}$, $R_{a5}$, $R_{b8}$, $R_{a9}$, $R_{b9}$, $Ra_{10}$ or $R_{b10}$ are each independently selected from hydrogen, deuterium, halogen, methyl, ethyl, cyano, deuterated methyl, cyclopropyl, cyclopentyl, phenyl,

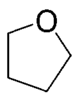、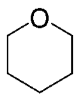、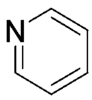、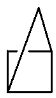 or

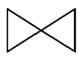,

the methyl, ethyl, deuterated methyl, cyclopropyl, cyclopentyl, phenyl, phenyl,

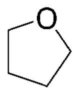、

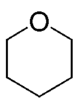、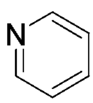、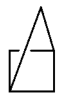 or 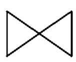

are optionally further substituted with one or more fluorine.

In a further preferred embodiment of the present invention, the compound of general formula (XVI-A) is as follows:

$R_{a1}$ and $R_{b1}$, $R_{a2}$ and $R_{b2}$, $R_{a3}$ and $R_{b3}$, $R_{a4}$ and $R_{b4}$, $R_{a6}$ and $R_{b6}$, $R_{a8}$ and $R_{b8}$, $R_{a9}$ and $R_{b9}$, $R_{a10}$ and $R_{b10}$ are linked with adjacent atoms to form $C_{3-8}$ cycloalkyl, 3-8 membered heterocyclic containing 1-3 heteroatoms selected from N, O or S, $C_{6-10}$ aryl, or 5-8 membered heteroaryl containing 1-3 heteroatoms selected from N, O or S, which are optionally further substituted by one or more substituents selected from halogen, amino, nitro, cyano, oxo, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl or 5-8 membered heteroaryl;
Preferably, $R_{a1}$ and $R_{b1}$, $R_{a2}$ and $R_{b2}$, $R_{a3}$ and $R_{b3}$, $R_{a4}$ and $R_{b4}$, $R_{a6}$ and $R_{b6}$, $R_{a8}$ and $R_{b8}$, $R_{a9}$ and $R_{b9}$, $R_{a10}$ and $R_{b10}$ are linked with adjacent atoms to form $C_{3-6}$ cycloalkyl, 3-6 membered heterocyclic containing 1-3 heteroatoms selected from N, O or S.

In a further preferred embodiment of the present invention, the compound of general formula (XVI-A) is as follows:

In the general formula (XVI-A),

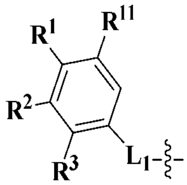

is further as shown below:

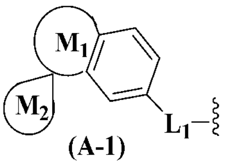
(A-1) 、

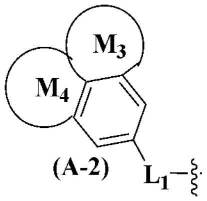
(A-2) 、

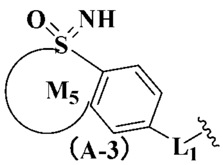
(A-3) 、

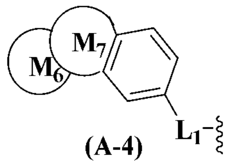
(A-4) 、

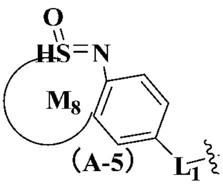
(A-5) or

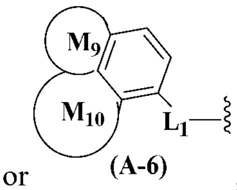
(A-6) ,

which are optionally further substituted by one or more substituents selected from halogen, methoxy, $C_{1-3}$ cycloalkyl, $C_{3-6}$ cycloalkyl or deuterated methyl;
Ring $M_1$, ring $M_2$, ring $M_3$, ring $M_4$, ring $M_5$, ring $M_6$, ring $M_7$, ring $M_8$, $M_9$ or $M_{10}$ are each independently selected from $C_{3-12}$ cycloalkyl, 3-12 membered heterocyclyl, $C_{6-12}$ aryl or 5-12 membered heteroaryl, which are optionally further substituted by one or more substituents selected from halogen, amino, nitro, hydroxyl, cyano, oxo, C1-6 alkyl, C1-6 deuterated alkyl, C1-6 haloalkyl, C1-6 deuterated haloalkyl, C1-6 hydroxyalkyl, C1-6 deuterated hydroxyalkyl, C1-6 sulfanyl, C1-6 deuterated sulfanyl, C1-6 alkoxy, C1-6 deuterated alkoxy, C1-6 haloalkoxy, C1-6 deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;
In a further preferred embodiment of the present invention, the compound of general formula (XVI-A) is as follows:

In the general formula (XVI-A),

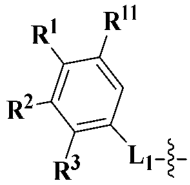

is further as shown below:

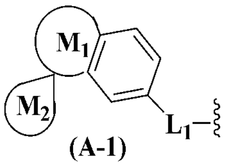 (A-1)、 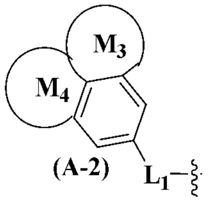 (A-2)、 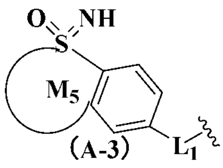 (A-3)、 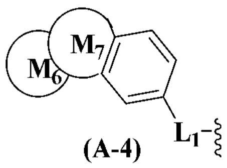 (A-4)、

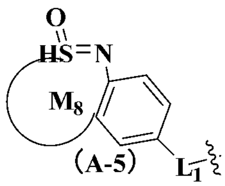 (A-5) or 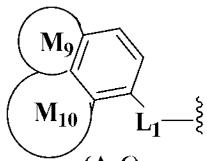 (A-6),

which are optionally further substituted by one or more substituents selected from halogen, methoxy, $C_{1-3}$ cycloalkyl, $C_{3-6}$ cycloalkyl or deuterated methyl;

Ring $M_1$, ring $M_2$, ring $M_3$, ring $M_4$, ring $M_5$, ring $M_6$, ring $M_7$, ring $M_8$, $M_9$ or $M_{10}$ are each independently selected from $C_{3-8}$ cycloalkyl, 3-8 membered heterocyclic containing 1-3 N, O or S atoms, $C_{6-10}$ aryl or 5-10 membered heteroaryl containing 1-3 N, O or S atoms, which are optionally further substituted by one or more substituents selected from halogen, amino, nitro, hydroxyl, cyano, oxo, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl or 5-8 membered heteroaryl;

Preferably, Ring $M_1$, ring $M_2$, ring $M_3$, ring $M_4$, ring $M_5$, ring $M_6$, ring $M_7$, ring $M_8$, $M_9$ or $M_{10}$ are each independently selected from $C_{3-6}$ cycloalkyl, 3-6 membered heterocyclic containing 1-3 N, O or S atoms, $C_{6-10}$ aryl or 5-8 membered heteroaryl containing 1-3 N, O or S atoms, which are optionally further substituted by one or more substituents selected from halogen, methoxy, $C_{1-3}$ cycloalkyl, $C_{3-6}$ cycloalkyl or deuterated methyl.

In a further preferred embodiment of the present invention, the compound of general formula (XVI-A) is as follows:

In the general formula (XVI-A),

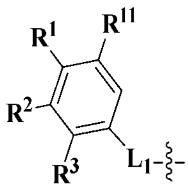

is further as shown below:

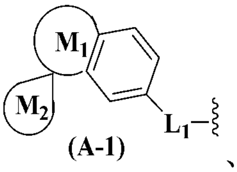 (A-1) 、 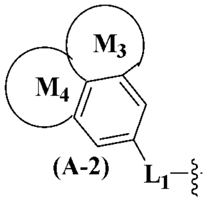 (A-2) 、 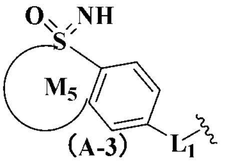 (A-3) 、 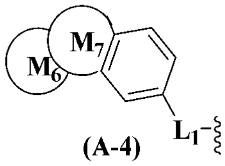 (A-4) or

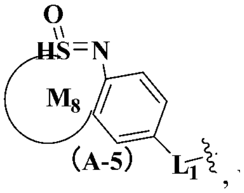 (A-5) ,

which are optionally further substituted by one or more substituents selected from halogen, methoxy, $C_{1-3}$ cycloalkyl, $C_{3-6}$ cycloalkyl or deuterated methyl.

Ring $M_1$, ring $M_2$, ring $M_3$, ring $M_4$, ring $M_5$, ring $M_6$, ring $M_7$, or ring $M_8$ are each independently selected from $C_{3-12}$ cycloalkyl, 3-12 membered heterocyclyl, $C_{6-12}$ aryl or 5-12 membered heteroaryl, which are optionally further substituted by one or more substituents selected from halogen, amino, nitro, hydroxyl, cyano, oxo, C1-6 alkyl, C1-6 deuterated alkyl, C1-6 haloalkyl, C1-6 deuterated haloalkyl, C1-6 hydroxyalkyl, C1-6 deuterated hydroxyalkyl, C1-6 sulfanyl, C1-6 deuterated sulfanyl, C1-6 alkoxy, C1-6 deuterated alkoxy, C1-6 haloalkoxy, C1-6 deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

In a further preferred embodiment of the present invention, the compound of general formula (XVI-A) is as follows: In the general formula (XVI-A),

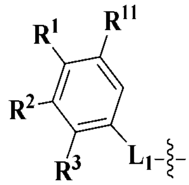

is further as shown below:

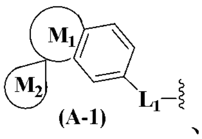 (A-1) 、 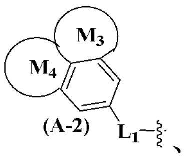 (A-2) 、 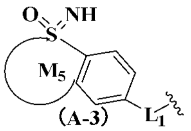 (A-3) 、 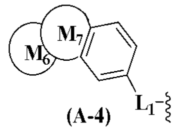 (A-4) or 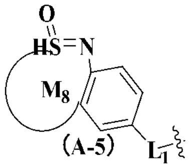 (A-5) ,

which are optionally further substituted by one or more substituents selected from halogen, methoxy, $C_{1-3}$ cycloalkyl, $C_{3-6}$ cycloalkyl or deuterated methyl.

Ring $M_1$, ring $M_2$, ring $M_3$, ring $M_4$, ring $M_5$, ring $M_6$, ring $M_7$, or ring $M_8$ are each independently selected from $C_{3-8}$ cycloalkyl, 3-8 membered heterocyclic containing 1-3 N, O or S atoms, $C_{6-10}$ aryl or 5-10 membered heteroaryl containing 1-3 N, O or S atoms, which are optionally further substituted by one or more substituents selected from halogen, amino, nitro, hydroxyl, cyano, oxo, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl or 5-8 membered heteroaryl;

Preferably, Ring $M_1$, ring $M_2$, ring $M_3$, ring $M_4$, ring $M_5$, ring $M_6$, ring $M_7$, or ring $M_8$ are each independently selected from $C_{3-6}$ cycloalkyl, 3-6 membered heterocyclic containing 1-3 N, O or S atoms, $C_{6-10}$ aryl or 5-8 membered heteroaryl containing 1-3 N, O or S atoms, which are optionally further substituted by one or more substituents selected from halogen, methoxy, $C_{1-3}$ cycloalkyl, $C_{3-6}$ cycloalkyl or deuterated methyl.

In a further preferred embodiment of the present invention, the compound of general formula (XVI-A) is as

follows:
$R^1$ and $R^2$, $R^1$ and $R^{11}$ are linked with adjacent phenyls to form

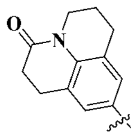、 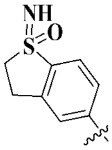、 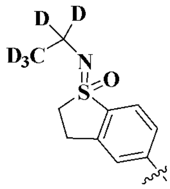、 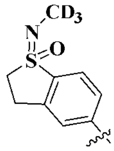、 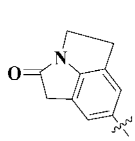、 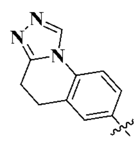、 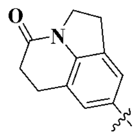、

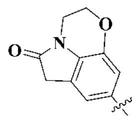、 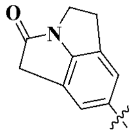、 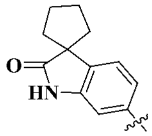、 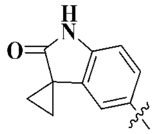、 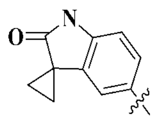、 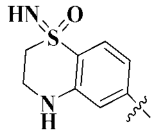、

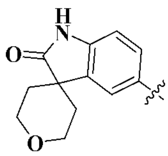、 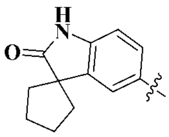、 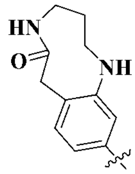、 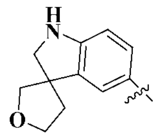、 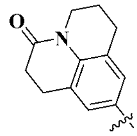、 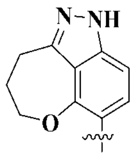、

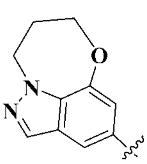、 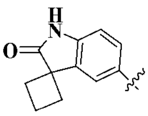、 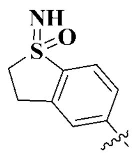、 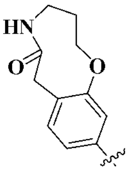、 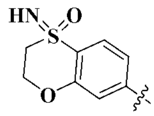、 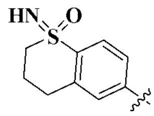、 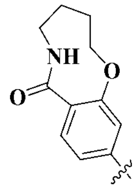、

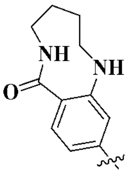、 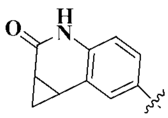、 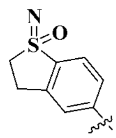、 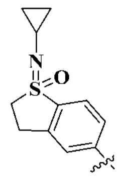、 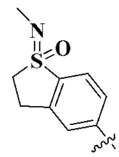、 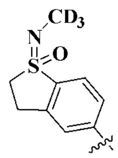、 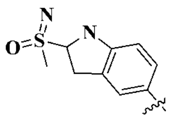、

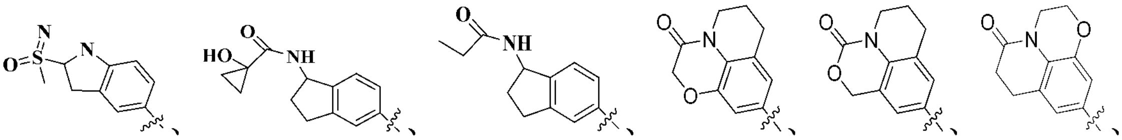
、 、 、 、 、 、

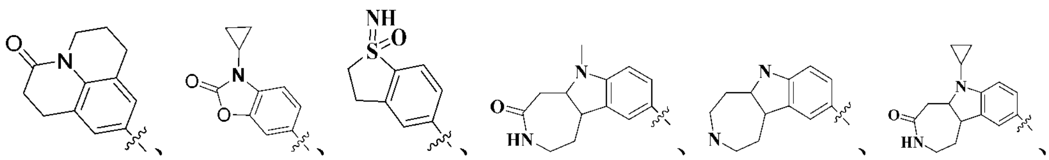
、 、 、 、 、 、

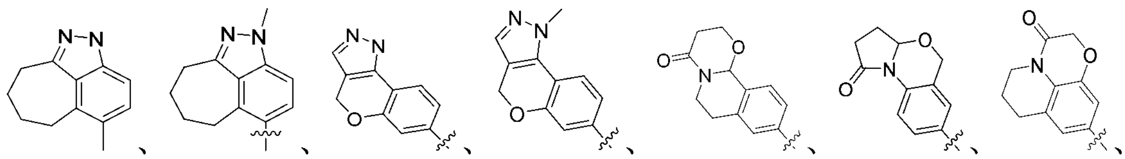
、 、 、 、 、 、 、

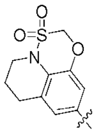, 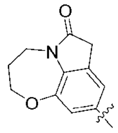, 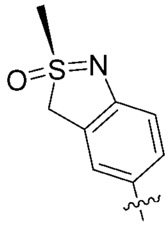, 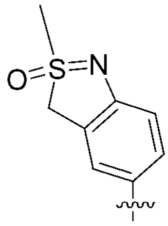, 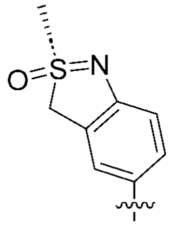, 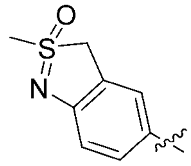,

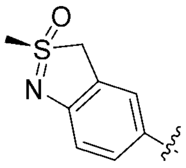, 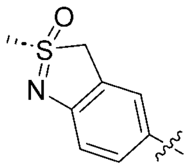, 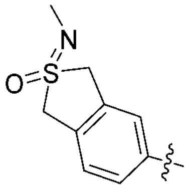, 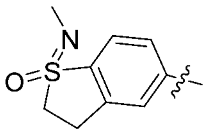, 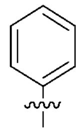, 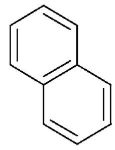,

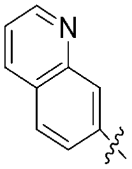, 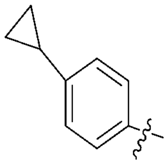, 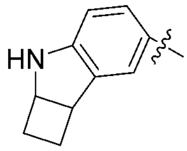, 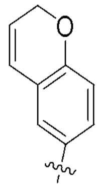, 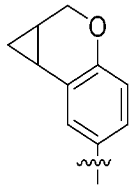, 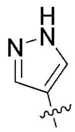, 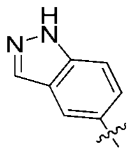,

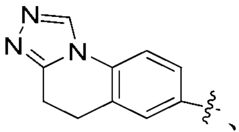, 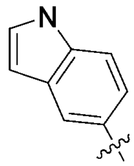, 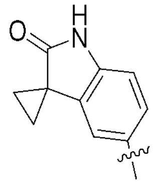, ,

which are optionally further substituted by one or more substituents selected from $-NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$, $(CH_2)_{n1}S(O)_2R_{a5}$, $-(CH_2)_{n1}O(CH_2)_{n2}R_{b5}$,

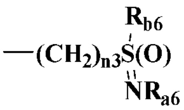, -

$(CH_2)_{n1}NR_{a8}C(O)R_{b8}$, $-(CR_{a8}R_{b8})_{n4}C(O)NR_{a9}R_{b9}$, $-(CH_2)_{n1}S(O)_2R_{b5}$ or $-(CH_2)_{n5}C(O)NR_{a10}R_{b10}$,

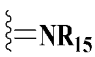,

deuterium, halogen, $C_{1-3}$ alkyl, $C_{1-3}$ deuterated alkyl, $C_{3-6}$ cycloalkyl, amino or oxo.

Note:

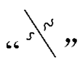

indicates connected to $L_1$.

In a further preferred embodiment of the present invention, the compound of general formula (XVI-A) is further represented by general formula (XVI-B):

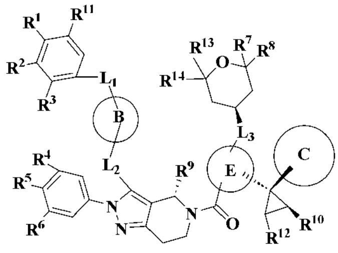

(XVI-B)

.

An object of the present invention is to provide a compound represented by general formula (II), a prodrug thereof, a stereoisomer thereof or a pharmaceutically acceptable salt thereof:

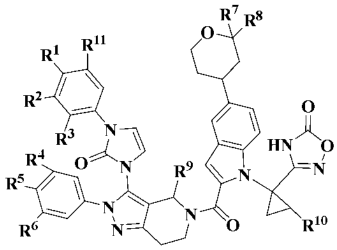

(II)

wherein,

$R^1$ and $R^2$, $R^1$ and $R^{11}$ are linked to adjacent atoms to form $C_{3-12}$ cycloalkyl, 3-12 membered heterocyclic, $C_{6-14}$ aryl or 5-14 membered heteroaryl, the $C_{3-12}$ cycloalkyl, 3-12 membered heterocyclic, $C_{6-14}$ aryl or 5-14 membered heteroaryl are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

Alternatively, $R^1$, $R^2$, $R^3$ or $R^{11}$ is selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, 5-10 membered heteroaryl, $-NS(O)R_{a1}R_{b1}$, $-(CH_2)_{n1}S(O)NR_{a2}R_{b2}$, $-P(O)R_{a3}R_{b3}$ or $-NR_{a5}S(O)NR_{a4}R_{b4}$, the amino, $C_{1-6}$ alkyl, $C_{1-6}$ deuterated alkyl, $C_{1-6}$ haloalkyl, $C_{1-6}$ hydroxyalkyl, $C_{1-6}$ Alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl are optionally further substituted by one or more substituents selected from halogen, amino, nitro, hydroxyl, cyano, oxo, $-NS(O)R_{a1}R_{b1}$, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R_{a1}$, $R_{b1}$, $R_{a2}$, $R_{b2}$, $R_{a3}$, $R_{b3}$, $R_{a4}$ or $R_{b4}$ is each independently selected from hydrogen, deuterium, halogen, amino, nitro, hydroxyl, cyano, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

alternatively, $R_{a1}$, $R_{b1}$, $R_{a2}$, $R_{b2}$, $R_{a3}$, $R_{b3}$, $R_{a4}$ or $R_{b4}$ is linked to adjacent atoms to form $C_{3-8}$ cycloalkyl, 3-8 membered heterocyclic, $C_{6-10}$ aryl or 5-10 membered heteroaryl, the $C_{3-8}$ cycloalkyl, 3-8 membered heterocyclic, $C_{6-10}$ aryl or 5-10 membered heteroaryl are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R^4$, $R^5$ or $R^6$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, the amino, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R^7$, $R^8$, $R^9$ or $R^{10}$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$

alkynyl, $C_{3-8}$ cycloalkyl, 3-8 membered heterocyclyl, $C_{6-10}$ aryl, 5-10 membered heteroaryl, or $-(CH_2)_{m1}OR_{c1}$, which are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R_{c1}$ is selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ sulfanyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, or 5-10 membered heteroaryl, the amino, $C_{1-6}$ alkyl, $C_{1-6}$ deuterated alkyl, $C_{1-6}$ haloalkyl, $C_{1-6}$ hydroxyalkyl, $C_{1-6}$ sulfanyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl are optionally further substituted with one or more substituents selected from halogen, amino, nitro, hydroxy, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

Alternatively, $R^7$, $R^8$, $R^9$ or $R^{10}$ is linked to the atoms connected thereto to form $C_{3-10}$ cycloalkyl, 3-10 membered heterocyclic, $C_{6-12}$ aryl or 5-12 membered heteroaryl;

n1 is an integer of 0-5;

m1 is an integer of 0-5.

In a further preferred embodiment of the present invention, the compound of general formula (II) is as follows: $R^1$ and $R^2$, $R^1$ and $R^{11}$ are linked to adjacent atoms to form $C_{3-8}$ cycloalkyl, 3-8 membered heterocyclic, $C_{6-10}$ aryl or 5-10 membered heteroaryl, the $C_{3-8}$ cycloalkyl, 3-8 membered heterocyclic, $C_{6-10}$ aryl or 5-10 membered heteroaryl are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl.

In a further preferred embodiment of the present invention, the compound of general formula (II) is as follows:

$R^1$ and $R^2$, $R^1$ and $R^{11}$ are linked to adjacent atoms to form $C_{3-6}$ cycloalkyl, 3-10 membered heterocyclic, $C_{6-10}$ aryl or 5-8 membered heteroaryl, which are optionally further substituted by one or more substituents selected from halogen, $C_{1-3}$ alkyl, amino or oxo;

Preferably, $R^1$ and $R^2$, $R^1$ and $R^{11}$ are linked to adjacent atoms to form 5-7 membered heterocyclic containing 1-3 selected from N, O or S, and optionally further substituted by oxo.

In a further preferred embodiment of the present invention, the compound of general formula (II) is as follows: $R^1$ and $R^2$, $R^1$ and $R^{11}$ are linked to adjacent atoms to form

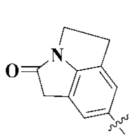、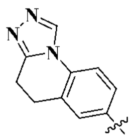、

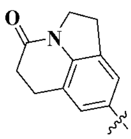、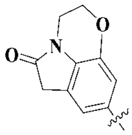、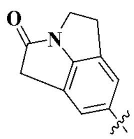、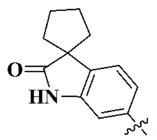、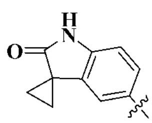、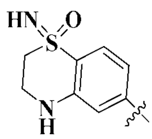、

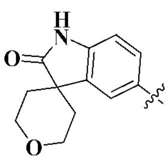、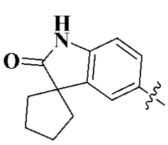、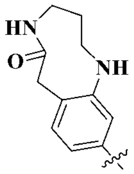、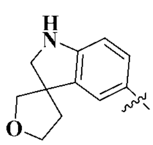、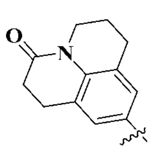、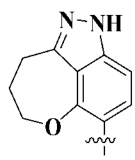、

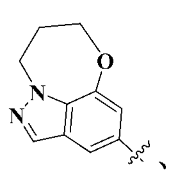, 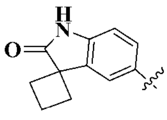, 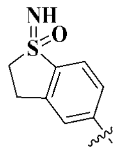, 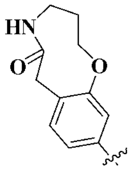, 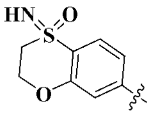, 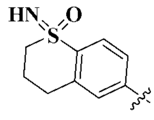, 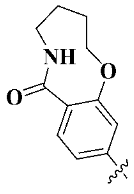,

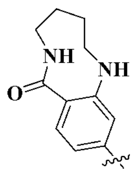 or 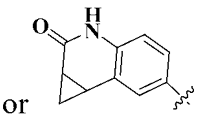,

which are optionally further substituted by one or more substituents selected from halogen, $C_{1-3}$ alkyl, $C_{1-3}$ deuterated alkyl, $C_{3-6}$ cycloalkyl, amino or oxo.

In a further preferred embodiment of the present invention, the compound of general formula (II) is as follows:

$R^1$ and $R^2$, $R^1$ and $R^{11}$ are linked to adjacent atoms to form $C_{3-6}$ cycloalkyl, 3-8 membered heterocyclic, $C_{6-10}$ aryl or 5-8 membered heteroaryl, which are optionally further substituted by one or more substituents selected from halogen, amino or oxo;

Preferably, $R^1$ and $R^2$, $R^1$ and $R^{11}$ are linked to adjacent atoms to form 5-7 membered heterocyclic containing 1-3 selected from N, O or S, and optionally further substituted by oxo.

In a further preferred embodiment of the present invention, the compound of general formula (II) is as follows:

$R^3$ is selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, C3-8 cycloalkyl, 3-8 membered heterocyclyl, C6-10 aryl, 5-10 membered heteroaryl, $-NS(O)R_{a1}R_{b1}$, $-(CH_2)_{n1}S(O)NR_{a2}R_{b2}$, -P(O) $R_{a3}R_{b3}$ or $-NR_{a5}S(O)NR_{a4}R_{b4}$, the amino, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl are optionally further substituted by one or more substituents selected from halogen, amino, nitro, hydroxyl, cyano, oxo, $-NS(O)R_{a1}R_{b1}$, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

Preferably, $R^3$ is selected from hydrogen, deuterium, halogen, amino, nitro, hydroxyl, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclyl, $C_{6-10}$ aryl, 5-8 membered heteroaryl, $-NS(O)R_{a1}R_{b1}$, $-(CH_2)_{n1}S(O)NR_{a2}R_{b2}$, -P(O) $R_{a3}R_{b3}$ or $-NR_{a5}S(O)NR_{a4}R_{b4}$, the amino, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl are optionally further substituted by one or more substituents selected from halogen, amino, nitro, hydroxyl, cyano, oxo, $-NS(O)R_{a1}R_{b1}$, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclic, $C_{6-10}$ aryl or 5-8 membered heteroaryl;

Further $R^3$ is selected from hydrogen, deuterium, halogen, amino, $C_{1-3}$ alkyl, $C_{1-3}$ deuterated alkyl, $C_{1-3}$ halogenated alkyl, $-NS(O)R_{a1}R_{b1}$, $-(CH_2)_{n1}S(O)NR_{a2}R_{b2}$, -P(O) $R_{a3}R_{b3}$ or - $NR_{a5}S(O)NR_{a4}R_{b4}$, the amino, $C_{1-3}$ alkyl, $C_{1-3}$ deuterated alkyl, $C_{1-3}$ halogenated alkyl are optionally further substituted by one or more substituents selected from $-NS(O)R_{a1}R_{b1}$, or $C_{1-3}$ alkyl.

In a further preferred embodiment of the present invention, the compound of general formula (II) is as follows:

$R^1$, $R^2$, $R^3$ or $R^{11}$ is each independently selected from hydrogen, deuterium, halogen, amino, nitro, hydroxyl, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-8}$ cycloalkyl, 3-8 membered heterocyclyl, $C_{6-10}$ aryl, 5-10 membered heteroaryl, $-NS(O)R_{a1}R_{b1}$, $-(CH_2)_{n1}S(O)NR_{a2}R_{b2}$, -P(O) $R_{a3}R_{b3}$ or $-NR_{a5}S(O)NR_{a4}R_{b4}$, the amino, $C_{1-3}$ alkyl, $C_{1-3}$ deuterated alkyl, $C_{1-3}$ haloalkyl, $C_{1-3}$ hydroxyalkyl, $C_{1-3}$ Alkoxy, $C_{1-3}$ haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl are optionally further substituted by one or more substituents selected from halogen, amino, nitro, hydroxyl, cyano, oxo, $-NS(O)R_{a1}R_{b1}$, $C_{1-3}$ alkyl, $C_{1-6}$ deuterated alkyl, $C_{1-6}$

haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-6}$ cycloalkyl, 3-6 membered heterocyclic, $C_{6-10}$ aryl or 5-8 membered heteroaryl;

Preferably, $R^1$, $R^2$, $R^3$ or $R^{11}$ is each independently selected from hydrogen, deuterium, halogen, amino, $C_{1-3}$ alkyl, $C_{1-3}$ deuterated alkyl, $C_{1-3}$ haloalkyl, $-NS(O)R_{a1}R_{b1}$, $-(CH_2)_{n1}S(O)NR_{a2}R_{b2}$, $-P(O)R_{a3}R_{b3}$ or $-NR_{a5}S(O)NR_{a4}R_{b4}$, the amino, $C_{1-3}$ alkyl, $C_{1-3}$ deuterated alkyl or $C_{1-3}$ haloalkyl are optionally further substituted by one or more substituents selected from halogen, $-NS(O)R_{a1}R_{b1}$ or $C_{1-3}$ alkyl.

In a further preferred embodiment of the present invention, the compound of general formula (II) is as follows:

$R_{a1}$, $R_{b1}$, $R_{a2}$, $R_{b2}$, $R_{a3}$, $R_{b3}$, $R_{a4}$ or $R_{b4}$ is each independently selected from hydrogen, deuterium, halogen, amino, $C_{1-3}$ alkyl, $C_{1-3}$ deuterated alkyl or $C_{1-3}$ haloalkyl;

Preferably, $R_{a1}$, $R_{b1}$, $R_{a2}$, $R_{b2}$, $R_{a3}$, $R_{b3}$, $R_{a4}$ or $R_{b4}$ is each independently selected from hydrogen, deuterium, halogen, or methyl.

In a further preferred embodiment of the present invention, the compound of general formula (II) is as follows: $R_{a1}$, $R_{b1}$, $R_{a2}$, $R_{b2}$, $R_{a3}$, $R_{b3}$, $R_{a4}$ or $R_{b4}$ is linked to adjacent atoms to form $C_{3-8}$ cycloalkyl, 3-6 membered heterocyclic, $C_{6-10}$ aryl or 5-8 membered heteroaryl.

In a further preferred embodiment of the present invention, the compound of general formula (II) is as follows: $R_{a2}$ and $R_{b2}$, $R_{a3}$, and $R_{b3}$ is linked to adjacent atoms to form $C_{3-8}$ cycloalkyl, or 3-6 membered heterocyclic containing 1-3 heteroatoms.

In a further preferred embodiment of the present invention, the compound of general formula (II) is as follows:

$R^4$, $R^5$ or $R^6$ is each independently selected from hydrogen, deuterium, halogen, amino, $C_{1-3}$ deuterated alkyl or $C_{1-3}$ halogenated alkyl;

Preferably, $R^4$, $R^5$ or $R^6$ is each independently selected from fluorine or methyl.

In a further preferred embodiment of the present invention, the compound of general formula (II) is as follows:

$R^7$, $R^8$, $R^9$ or $R^{10}$ is each independently selected from hydrogen, deuterium, halogen, $C_{1-3}$ alkyl, $C_{1-3}$ deuterated alkyl or $C_{1-3}$ haloalkyl;

Preferably, $R^7$, $R^8$, $R^9$ or $R^{10}$ is each independently selected from methyl.

In a further preferred embodiment of the present invention, the compound of general formula (II) is as follows:

$R^1$ and $R^2$, $R^1$ and $R^{11}$ are linked to adjacent atoms to form $C_{3-6}$ cycloalkyl, 3-10 membered heterocyclic, $C_{6-10}$ aryl or 5-8 membered heteroaryl, which are optionally further substituted by one or more substituents selected from halogen, $C_{1-3}$ alkyl, $C_{3-6}$ cycloalkyl, amino or oxo;

Preferably, $R^1$ and $R^2$, $R^1$ and $R^{11}$ are linked to adjacent atoms to form 5-7 membered heterocyclic containing 1-3 selected from N, O or S, optionally further substituted by $C_{3-6}$ cycloalkyl or oxo;

Further preferably, $R^1$ and $R^2$, $R^1$ and $R^{11}$ are linked to adjacent atoms to form

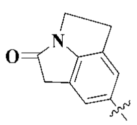 、 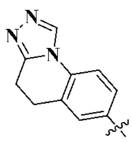 、 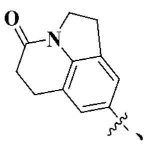 、 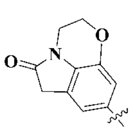 、 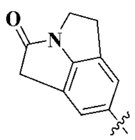 、 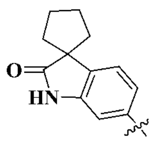 、 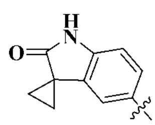 、

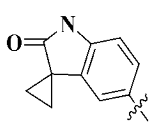 、 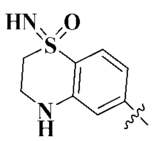 、 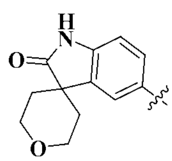 、 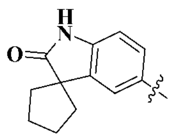 、 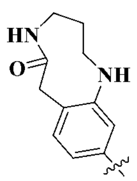 、 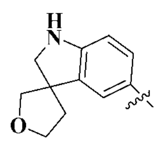 、

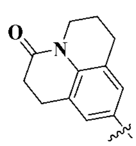 、 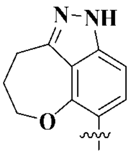 、 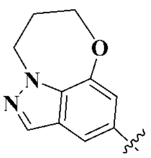 、 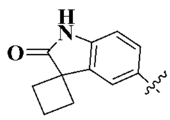 、 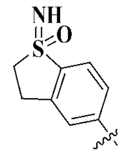 、 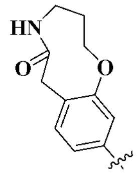 、 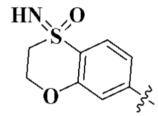 、

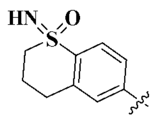 、 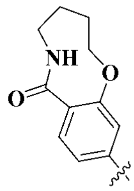 、 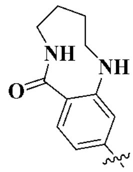 、 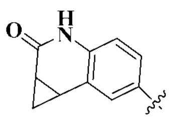 、 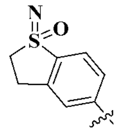 、 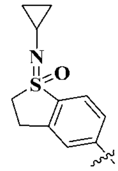 、 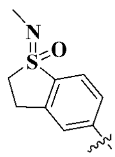 、

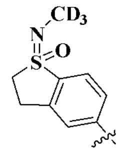 、 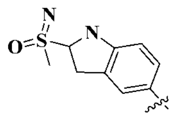 、 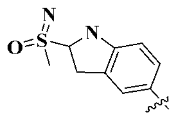 、 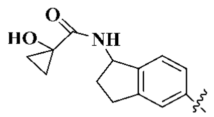 、 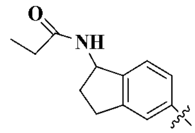 、 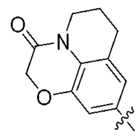 、

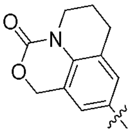 、 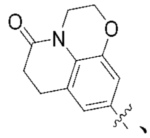 、 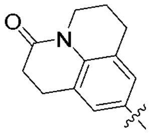 、 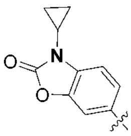 、 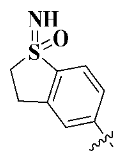 、 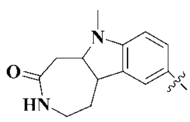 、 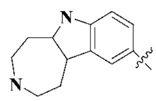 、

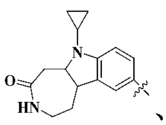 、 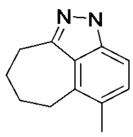 、 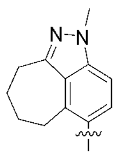 、 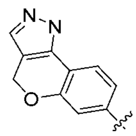 、 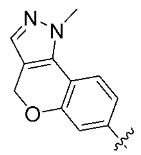 、 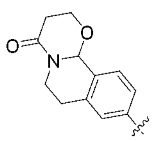 、 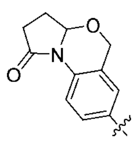 、

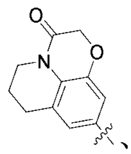 、 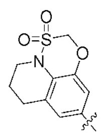 or 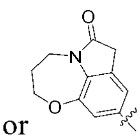 ,

which are optionally further substituted by one or more substituents selected from halogen, $C_{1-3}$ alkyl, $C_{1-3}$ deuterated alkyl, $C_{3-6}$ cycloalkyl, amino or oxo.

In a further preferred embodiment of the present invention, the compound of general formula (II) is as follows:

$R^4$, $R^5$ or $R^6$ is each independently selected from hydrogen, deuterium, halogen, amino, nitro, $C_{3-6}$ cycloalkyl, $C_{1-3}$ alkyl, $C_{1-3}$ deuterated alkyl or $C_{1-3}$ haloalkyl;

Preferably, $R^4$, $R^5$ or $R^6$ is each independently selected from fluoro, cyano, cyclopropyl or methyl.

In a further preferred embodiment of the present invention, the compound of general formula (II) is as follows:

$R^7$, $R^8$, $R^9$ or $R^{10}$ is each independently selected from hydrogen, deuterium, halogen, $C_{3-6}$ cycloalkyl, $C_{1-3}$ alkyl, $C_{1-3}$ deuterated alkyl or $C_{1-3}$ haloalkyl;

Preferably, $R^7$, $R^8$, $R^9$ or $R^{10}$ is each independently selected from methyl or cyclopropyl.

In a further preferred embodiment of the present invention, the compound of general formula (II) is as follows: $R^7$, $R^8$, $R^9$ or $R^{10}$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, which are optionally further

substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl.

In a further preferred embodiment of the present invention, the compound of general formula (II) is as follows:

$R^7$, $R^8$, $R^9$ or $R^{10}$ is respectively linked to the atoms connected thereto to form $C_{3-10}$ cycloalkyl, 3-10 membered heterocyclyl, $C_{6-12}$ aryl or 5-12 membered heteroaryl;
Preferably, $R^7$, $R^8$, $R^9$ or $R^{10}$ is respectively linked to the atoms connected thereto to form $C_{3-6}$ cycloalkyl, 3-6 membered heterocyclyl, $C_{6-10}$ aryl or 5-8 membered heteroaryl;
Further preferably, $R^7$, $R^8$, $R^9$ or $R^{10}$ is respectively linked to the atoms connected thereto to form cyclopropyl.

In a further preferred embodiment of the present invention, the compound of general formula (II) is as follows:

$R^{10}$ is linked to the atoms connected thereto and the adjacent atoms to form $C_{3-10}$ cycloalkyl, 3-10 membered heterocyclic, $C_{6-12}$ aryl or 5-12 membered heteroaryl;
Preferably, $R^{10}$ is linked to the atoms connected thereto and the adjacent atoms to form $C_{3-6}$ cycloalkyl, 3-6 membered heterocyclic, $C_{6-10}$ aryl or 5-8 membered heteroaryl;
Further preferably, $R^{10}$ is linked to the atoms connected thereto and the adjacent atoms to form

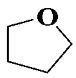
.

In a further preferred embodiment of the present invention, the compound of general formula (II) is as follows: $R^{10}$ is linked to the atoms connected thereto and the adjacent atoms to form

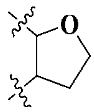

or

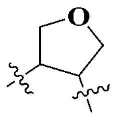
.

In a further preferred embodiment of the present invention, the compound of general formula (II) is as follows:

$R_{c1}$ is selected from hydrogen, deuterium, halogen, amino, nitro, hydroxyl, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ sulfanyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl, which are optionally further substituted with one or more substituents selected from halogen, amino, nitro, hydroxy, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl;
Preferably, $R_{c1}$ is selected from fluorine, methyl, cyano, difluoromethyl, trifluoromethyl, ethynyl, methylthio, amino, cyclopropyl or oxetane, which are optionally further substituted by halogen, methoxy or deuterated methyl.

In a further preferred embodiment of the present invention, the compound of general formula (II) is as follows:

$R^7$, $R^8$, $R^9$ or $R^{10}$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, 5-10 membered heteroaryl or $-(CH_2)_{m1}OR_{c1}$, which are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;
Preferably, $R^7$, $R^8$, $R^9$ or $R^{10}$ is each independently selected from hydrogen, deuterium, halogen, amino, nitro,

hydroxyl, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclyl, $C_{6-10}$ aryl, 5-8 membered heteroaryl or $-(CH_2)_{m1}OR_{c1}$, which are optionally further substituted by one or more substituents selected from halogen, amino, nitro, hydroxyl, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclic, $C_{6-10}$ aryl or 5-8 membered heteroaryl;

Further preferably, $R^7$, $R^8$, $R^9$ or $R^{10}$ is each independently selected from hydrogen, deuterium, halogen, cyano, $C_{2-4}$ alkynyl, $C_{3-6}$ cycloalkyl, $C_{1-3}$ alkyl, $C_{1-3}$ deuterated alkyl, $C_{1-3}$ haloalkyl or $-(CH_2)_{m1}OR_{c1}$;

More preferably, $R^7$, $R^8$, $R^9$ or $R^{10}$ is each independently selected from methyl, fluorine, cyclopropyl, ethynyl or $-(CH_2)_{m1}OR_{c1}$.

In a further preferred embodiment of the present invention, the compound of general formula (II) is further represented by general formula (II-1):

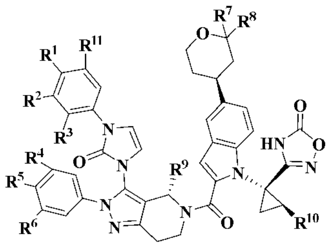

(II-1) .

An object of the present invention is to provide a compound represented by general formula (XI), a prodrug thereof, a stereoisomer thereof or a pharmaceutically acceptable salt thereof:

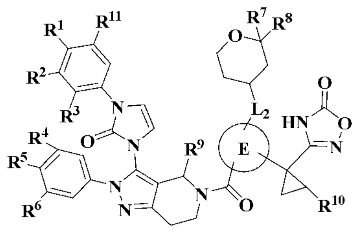

(XI)

wherein,

Ring E is selected from $C_{3-12}$ cycloalkyl, 3-12 membered heterocyclic, $C_{6-14}$ aryl or 5-14 membered heteroaryl;

$L_2$ is selected from bond, $-(CH_2)_{n4}-$, $C_{2-4}$ alkenyl, $C_{2-4}$ alkynyl;

$R^1$, $R^2$, $R^3$ or $R^{11}$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, 5-10 membered heteroaryl, $NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$ , $-(CH_2)_{n1}S(O)_2R_{a5}$, $-(CH_2)_{n1}O(CH_2)_{n2}R_{b5}$ ,

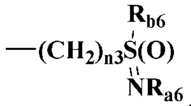

, .

$-(CH_2)_nP(O)R_{a7}R_{b7}$ or $-(CR_{a8}R_{b8})_{n4}C(O)NR_{a9}R_{b9}$, the amino, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl are optionally further substituted by one or more substituents selected from halogen, amino, nitro, hydroxyl, cyano, oxo, $-NS(O)R_{a1}R_{b1}$, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl; $R^4$, $R^5$ or $R^6$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$

alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, the amino, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R^7$, $R^8$, $R^9$ or $R^{10}$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, which are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

Alternatively, $R^7$, $R^8$, $R^9$ or $R^{10}$ is linked to the atoms connected thereto to form $C_{3-10}$ cycloalkyl, 3-10 membered heterocyclic, $C_{6-12}$ aryl or a 5-12 membered heteroaryl;

$R_{a1}$, $R_{b1}$, $R_{a2}$, $R_{b2}$, $R_{a3}$, $R_{b3}$, $R_{a4}$, $R_{b4}$, $R_{a5}$, $R_{b5}$, $R_{a6}$, $R_{b6}$, $R_{a7}$, $R_{b7}$, $R_{a8}$, $R_{b8}$, $R_{a9}$, or $R_{b9}$ is each independently selected from hydrogen, deuterium, halogen, amino, nitro, hydroxyl, cyano, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered;

Alternatively, $R_{a1}$ and $R_{b1}$, $R_{a2}$ and $R_{b2}$, $R_{a3}$ and $R_{b3}$, $R_{a4}$ and $R_{b4}$, $R_{a7}$ and $R_{b7}$, $R_{a8}$ and $R_{b8}$, $R_{a9}$ and $R_{b9}$ are linked to adjacent atoms to form $C_{3-8}$ cycloalkyl, 3-8 membered heterocyclic, $C_{6-10}$ aryl or 5-10 membered heteroaryl, the $C_{3-8}$ cycloalkyl, 3-8 membered heterocyclic, $C_{6-10}$ aryl or 5-10 membered heteroaryl are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R^4$, $R^5$ or $R^6$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, the amino, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R^7$, $R^8$, $R^9$ or $R^{10}$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, which are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R_{a1}$, $R_{b1}$, $R_{a2}$, $R_{b2}$, $R_{a3}$, $R_{b3}$, $R_{a4}$, $R_{b4}$, $R_{a5}$, $R_{b5}$, $R_{a6}$, $R_{b6}$, $R_{a7}$, $R_{b7}$, $R_{a8}$, $R_{b8}$, $R_{a9}$, or $R_{b9}$ is each independently selected from hydrogen, deuterium, halogen, amino, nitro, hydroxyl, cyano, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered;

Alternatively, $R_{a1}$ and $R_{b1}$, $R_{a2}$ and $R_{b2}$, $R_{a3}$ and $R_{b3}$, $R_{a4}$ and $R_{b4}$, $R_{a7}$ and $R_{b7}$, $R_{a8}$ and $R_{b8}$, $R_{a9}$ and $R_{b9}$ are linked to adjacent atoms to form $C_{3-8}$ cycloalkyl, 3-8 membered heterocyclic, $C_{6-10}$ aryl or 5-10 membered heteroaryl, the $C_{3-8}$ cycloalkyl, 3-8 membered heterocyclic, $C_{6-10}$ aryl or 5-10 membered heteroaryl are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

n1-n4 are integers from 0 to 5.

In a further preferred embodiment of the present invention, the general formula (XI) compound is as follows:

Ring E is selected from $C_{3-8}$ cycloalkyl, 3-10 membered heterocyclic containing 1-3 N, O or S atoms, $C_{6-10}$ aryl or 5-10 membered heteroaryl containing 1-3 N, O or S atoms;

Preferably, ring E is selected from

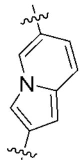 、 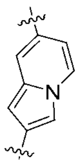 or 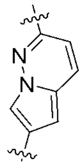 .

In a further preferred embodiment of the present invention, the general formula (XI) compound is as follows: Ring E is selected from

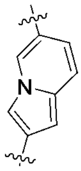 、 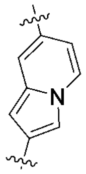 、 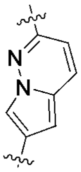 、 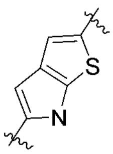 、 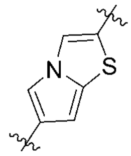 or 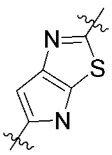 .

In a further preferred embodiment of the present invention, the general formula (XI) compound is as follows: Ring E is selected from

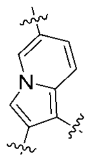 、 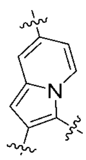 、 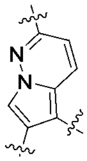 、 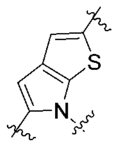 、 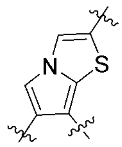 、 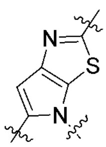 、

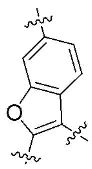 、 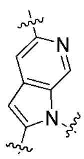 、 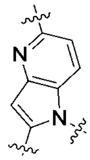 、 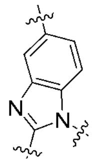 or 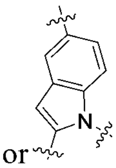 .

In a further preferred embodiment of the present invention, the general formula (XI) compound is as follows: $R^1$, $R^2$, $R^3$ or $R^{11}$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, 5-10 membered heteroaryl, $NS(O)R_{a1}R_{b1}$, - $(CH_2)_{n1}S(O)NR_{a2}R_{b2}$, $-P(O)R_{a3}R_{b3}$, $-NR_{a5}S(O)NR_{a4}R_{b4}$ , $(CH2)_{n1}S(O)2R_{a5}$, $-(CH_2)_{n1}O(CH_2)_{n2}R_{b5}$ ,

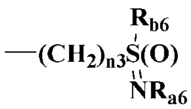 ,

the amino, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl are optionally further substituted by one or more substituents selected from halogen, amino, nitro, hydroxyl, cyano, oxo, $-NS(O)R_{a1}R_{b1}$, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl. In a further preferred embodiment of the present invention, the general formula (XI) compound is as follows:

Preferably, $R^1$, $R^2$, $R^3$ or $R^{11}$ is each independently selected from hydrogen, deuterium, halogen, amino, nitro, hydroxyl, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclyl, $C_{6-10}$ aryl, 5-10 membered heteroaryl, $NS(O)R_{a1}R_{b1}$, - $(CH_2)_{n1}S(O)NR_{a2}R_{b2}$, $-P(O)R_{a3}R_{b3}$, $-NR_{a5}S(O)NR_{a4}R_{b4}$ , $(CH2)_{n1}S(O)_2R_{a5}$, $-(CH_2)_{n1}O(CH_2)_{n2}R_{b5}$ ,

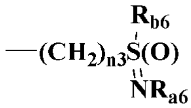,

$-(CH_2)_nP(O)R_{a7}R_{b7}$ or $-(CR_{a8}R_{b8})_{n4}C(O)NR_{a9}R_{b9}$, the amino, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-8}$ cycloalkyl, 3-8 membered heterocyclic, $c_{6-10}$ aryl or 5-10 membered heteroaryl are optionally further substituted by one or more substituents selected from halogen, amino, nitro, hydroxyl, cyano, oxo, $-NS(O)R_{a1}R_{b1}$, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclic, $C_{6-10}$ aryl or 5-10 membered heteroaryl.

In a further preferred embodiment of the present invention, the general formula (XI) compound is as follows: $R^1$, $R^2$, $R^3$ or $R^{11}$ is each independently selected from hydrogen, deuterium, halogen, amino, nitro, hydroxyl, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclyl, $C_{6-10}$ aryl, 5-10 membered heteroaryl, $NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$ , $(CH2)_{n1}S(O)2R_{a5}$, $-(CH_2)_{n1}O(CH_2)_{n2}R_{b5}$ ,

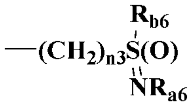,

the amino, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-8}$ cycloalkyl, 3-8 membered heterocyclic, $C_{6-10}$ aryl or 5-10 membered heteroaryl are optionally further substituted by one or more substituents selected from halogen, amino, nitro, hydroxyl, cyano, oxo, $-NS(O)R_{a1}R_{b1}$, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclic, $C_{6-10}$ aryl or 5-10 membered heteroaryl.

In a further preferred embodiment of the present invention, the general formula (XI) compound is as follows: $R^1$ and $R^2$ or $R^1$ and $R^{11}$ are linked to adjacent atoms to form $C_{3-8}$ cycloalkyl, 3-8 membered heterocyclic, $C_{6-10}$ aryl or 5-10 membered heteroaryl, the $C_{3-8}$ cycloalkyl, 3-8 membered heterocyclic, $C_{6-10}$ aryl or 5-10 membered heteroaryl are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl, 5-10 membered heteroaryl, $NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$ , $(CH_2)_{n1}S(O)_2R_{a5}$, $-(CH_2)_{n1}O(CH_2)_{n2}R_{b5}$ ,

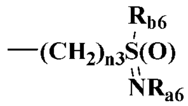.

In a further preferred embodiment of the present invention, the general formula (XI) compound is as follows: Preferably, $R^1$ and $R^2$ or $R^1$ and $R^{11}$ are linked to adjacent atoms to form $C_{3-6}$ cycloalkyl, 3-8 membered heterocyclic, $C_{6-10}$ aryl or 5-8 membered heteroaryl, are optionally further substituted by one or more substituents selected from halogen, amino, nitro, hydroxyl, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclic, $C_{6-10}$ aryl, 5-10 membered heteroaryl, $NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$ , $(CH_2)_{n1}S(O)_2R_{a5}$, $-(CH_2)_{n1}O(CH_2)_{n2}R_{b5}$ ,

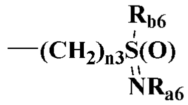.

Further preferably, $R^1$ and $R^2$ or $R^1$ and $R^{11}$ are linked to adjacent atoms to form phenyl, 5-7 membered heterocyclic containing 1-3 atoms selected from N, O or S, which are optionally further substituted by one or more substituents selected from oxo, $NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$ , $(CH_2)_{n1}S(O)_2R_{a5}$, $-(CH_2)_{n1}O(CH_2)_{n2}R_{b5}$ ,

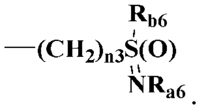.

In a further preferred embodiment of the present invention, the general formula (XI) compound is as follows:

$R^1$ and $R^2$ or $R^1$ and $R^{11}$ are linked to adjacent atoms to form $C_{3-8}$ cycloalkyl, 3-10 membered heterocyclyl, $C_{6-10}$ aryl or 5-10 membered heteroaryl, which are optionally further substituted by one or more substituents selected from halogen, amino, nitro, hydroxyl, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclic, $C_{6-10}$ aryl, 5-10 membered heteroaryl, $NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$ , $(CH_2)_{n1}S(O)_2R_{a5}$, $-(CH_2)_{n1}O(CH_2)_{n2}R_{b5}$ ,

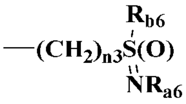,

$-(CH_2)_nP(O)R_{a7}R_{b7}$ or - $(CR_{a8}R_{b8})_{n4}C(O)NR_{a9}R_{b9}$;
Preferably, $R^1$ and $R^2$ or $R^1$ and $R^{11}$ are linked to adjacent atoms to form phenyl or 5-7 membered heterocyclic containing 1-3 atoms selected from N, O or S, which are optionally further substituted by one or more substituents selected from methyl, halogen, oxo, $NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$ , $(CH_2)_{n1}S(O)_2R_{a5}$, $-(CH_2)_{n1}O(CH_2)_{n2}R_{b5}$ ,

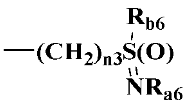,

$-(CH_2)_nP(O)R_{a7}R_{b7}$ or $-(CR_{a8}R_{b8})_{n4}C(O)NR_{a9}R_{b9}$;
Further preferably, $R^1$ and $R^2$ or $R^1$ and $R^{11}$ are linked to adjacent atoms to form

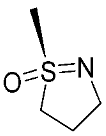、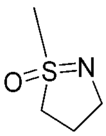、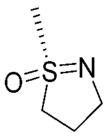、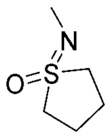、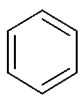、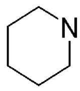、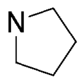、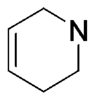、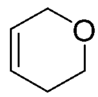、

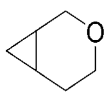、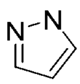 or 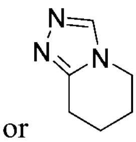,

which are optionally further substituted by one or more substituents selected from methyl, halogen, oxo, NS(O) $R_{a1}R_{b1}$, $-(CH_2)_{n1}S(O)NR_{a2}R_{b2}$, - $P(O)R_{a3}R_{b3}$, $-NR_{a5}S(O)NR_{a4}R_{b4}$ , $(CH_2)_{n1}S(O)_2R_{a5}$, $-(CH_2)_{n1}O(CH_2)_{n2}R_{b5}$ ,

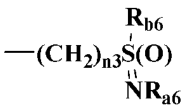, -

$(CH_2)_nP(O)R_{a7}R_{b7}$ or $-(CR_{a8}R_{b8})_{n4}C(O)NR_{a9}R_{b9}$.

In a further preferred embodiment of the present invention, the general formula (XI) compound is as follows: $R^1$ and $R^2$ or $R^1$ and $R^{11}$ are linked to adjacent atoms to form

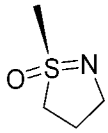、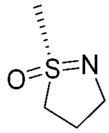、

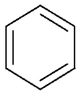、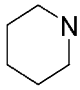、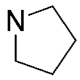、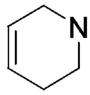、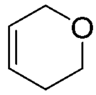 or 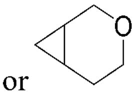.

In a further preferred embodiment of the present invention, the general formula (XI) compound is as follows:

$R^4$, $R^5$ or $R^6$ is each independently selected from hydrogen, deuterium, halogen, amino, $C_{1-3}$ alkyl, $C_{1-3}$ deuterated alkyl, $C_{3-6}$ cycloalkyl, $C_{1-3}$ haloalkyl;
Preferably, $R^4$, $R^5$ or $R^6$ is each independently selected from fluorine, methyl, cyclopropyl.

In a further preferred embodiment of the present invention, the general formula (XI) compound is as follows:

$R^7$, $R^8$, $R^9$ or $R^{10}$ is each independently selected from hydrogen, deuterium, halogen, $C_{1-3}$ alkyl, $C_{1-3}$ deuterated alkyl, $C_{1-3}$ haloalkyl;
Preferably, $R^7$, $R^8$, $R^9$ or $R^{10}$ is each independently selected from hydrogen or methyl.

In a further preferred embodiment of the present invention, the general formula (XI) compound is as follows: $R_{a1}$, $R_{b1}$, $R_{a2}$, $R_{b2}$, $R_{a3}$, $R_{b3}$, $R_{a4}$, $R_{b4}$, $R_{a5}$, $R_{b8}$, $R_{a6}$ or $R_{b6}$ is each independently selected from hydrogen, deuterium, halogen, amino, nitro, hydroxyl, cyano, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, 5-10 membered heteroaryl; or $R_{a1}$ and $R_{b1}$ or $R_{a2}$ and $R_{b2}$ or $R_{a3}$ and $R_{b3}$ or $R_{a4}$ and $R_{b4}$ are linked to adjacent atoms to form $C_{3-8}$ cycloalkyl, 3-8 membered heterocyclic, $C_{6-10}$ aryl or 5-10 membered heteroaryl, the $C_{3-8}$ cycloalkyl, 3-8 membered heterocyclic, $C_{6-10}$ aryl or 5-10 membered heteroaryl are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, C3-8 cycloalkyl, 3-8 membered heterocyclic, $C_{6-10}$ aryl or 5-10 membered heteroaryl.

In a further preferred embodiment of the present invention, the general formula (XI) compound is as follows:

$R_{a1}$, $R_{b1}$, $R_{a2}$, $R_{b2}$, $R_{a3}$, $R_{b3}$, $R_{a4}$, $R_{b4}$, $R_{a5}$, $R_{b5}$, $R_{a6}$, $R_{b6}$, $R_{a7}$, $R_{b7}$, $R_{a8}$, $R_{b8}$, $R_{a9}$, or $R_{b9}$ is each independently selected from hydrogen, deuterium, halogen, amino, cyano, $C_{1-3}$ alkyl, $C_{1-3}$ deuterated alkyl, $C_{1-3}$ haloalkyl, $C_{3-6}$ cycloalkyl, 3-6 membered heterocyclyl, $C_{6-10}$ aryl or 5-8 membered heteroaryl;
Preferably, $R_{a1}$, $R_{b1}$, $R_{a2}$, $R_{b2}$, $R_{a3}$, $R_{b3}$, $R_{a4}$, $R_{b4}$, $R_{a5}$, $R_{b5}$, $R_{a6}$, $R_{b6}$, $R_{a7}$, $R_{b7}$, $R_{a8}$, $R_{b8}$, $R_{a9}$, or $R_{b9}$ is each independently selected from hydrogen, deuterium, halogen, cyano, cyclopropyl, cyclopentyl, methyl, ethyl, phenyl or

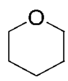.

In a further preferred embodiment of the present invention, the general formula (XI) compound is as follows:

$R_{a1}$, $R_{b1}$, $R_{a2}$, $R_{b2}$, $R_{a3}$, $R_{b3}$, $R_{a4}$, $R_{b4}$, $R_{a5}$, $R_{b5}$, $R_{a6}$ or $R_{b6}$ is each independently selected from hydrogen, deuterium, halogen, amino, $C_{1-3}$ alkyl, $C_{1-3}$ deuterated alkyl, $C_{1-3}$ haloalkyl;
Preferably, $R_{a1}$, $R_{b1}$, $R_{a2}$, $R_{b2}$, $R_{a3}$, $R_{b3}$, $R_{a4}$, $R_{b4}$, $R_{a5}$, $R_{b5}$, $R_{a6}$ or $R_{b6}$ is each independently selected from hydrogen, deuterium, halogen, and methyl.

In a further preferred embodiment of the present invention, the general formula (XI) compound is as follows: Ring E is

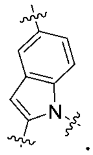.

In a further preferred embodiment of the present invention, the general formula (XI) compound is as follows:

$R^4$, $R^5$ or $R^6$ is each independently selected from hydrogen, deuterium, halogen, amino, cyano, $C_{1-3}$ alkyl, $C_{1-3}$ deuterated alkyl, C3-6 cycloalkyl or $C_{1-3}$ haloalkyl;
Preferably, $R^4$, $R^5$ or $R^6$ is each independently selected from fluoro, methyl, cyano or cyclopropyl.

In a further preferred embodiment of the present invention, the general formula (XI) compound is as follows:

$R^7$, $R^8$, $R^9$ or $R^{10}$ is linked to the atoms connected thereto to form $C_{3-10}$ cycloalkyl, 3-10 membered heterocyclic, $C_{6-12}$ aryl or 5-12 membered heteroaryl;
Preferably, $R^7$, $R^8$, $R^9$ or $R^{10}$ is linked to the atoms connected thereto to form $C_{3-6}$ cycloalkyl, 3-6 membered heterocyclic, $C_{6-10}$ aryl or 5-8 membered heteroaryl;
Further preferably, $R^7$, $R^8$, $R^9$ or $R^{10}$ is linked to the atoms connected thereto to form cyclopropyl.

In a further preferred embodiment of the present invention, the general formula (XI) compound is as follows: $R_{a1}$, $R_{b1}$, $R_{a2}$, $R_{b2}$, $R_{a3}$, $R_{b3}$, $R_{a4}$, $R_{b4}$, $R_{a5}$, $R_{b5}$, $R_{a6}$, $R_{b6}$, $R_{a7}$, $R_{b7}$, $R_{a8}$, $R_{b8}$, $R_{a9}$, or $R_{b9}$ is each independently selected from hydrogen, deuterium, halogen, cyano, cyclopropyl, cyclopentyl, methyl, ethyl, deuterated methyl, phenyl or

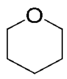.

In a further preferred embodiment of the present invention, the general formula (XI) compound is as follows:

$R_{a1}$ and $R_{b1}$ or $R_{a2}$ and $R_{b2}$ or $R_{a3}$ and $R_{b3}$ or $R_{a4}$ and $R_{b4}$ or $R_{a7}$ and $R_{b7}$ or $R_{a8}$ and $R_{b8}$ or $R_{a9}$ and $R_{b9}$ are linked with adjacent atoms to form $C_{3-6}$ cycloalkyl, 3-6 membered heterocyclic, $C_{6-10}$ aryl or 5-8 membered heteroaryl, the $C_{3-6}$ cycloalkyl, 3-6 membered heterocyclic, $C_{6-10}$ aryl or 5-8 membered heteroaryl are optionally further substituted by one or more substituents selected from halogen, amino, nitro, hydroxyl, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclic, $C_{6-10}$ aryl or 5-8 membered heteroaryl;
Preferably, $R_{a1}$ and $R_{b1}$ or $R_{a2}$ and $R_{b2}$ or $R_{a3}$ and $R_{b3}$ or $R_{a4}$ and $R_{b4}$ or $R_{a7}$ and $R_{b7}$ or $R_{a8}$ and $R_{b8}$ or $R_{a9}$ and $R_{b9}$ are linked with adjacent atoms to form cyclopropyl.

In a further preferred embodiment of the present invention, the general formula (XI) compound is as follows:

$R_1$ and $R_2$ or $R_1$ and $R_{11}$ are linked to adjacent atoms to form phenyl or 5-7 membered heterocyclic containing 1-3 atoms selected from N, O or S, which are optionally further substituted by one or more substituents selected from methyl, halogen, oxo, cyclopropyl, $NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$, $(CH_2)_{n1}S(O)_2R_{a5}$, $-(CH_2)_{n1}O(CH_2)_{n2}R_{b5}$,

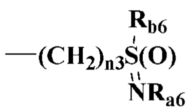,

$(CH_2)_{n1}P(O)R_{a7}R_{b7}$ or $-(CR_{a8}R_{b8})_{n4}C(O)NR_{a9}R_{b9}$.

Preferably, $R_1$ and $R_2$ or $R_1$ and $R_{11}$ are linked to adjacent atoms to form

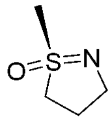 、

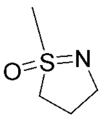 、 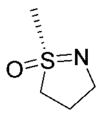 、 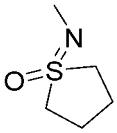 、 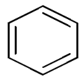 、 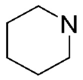 、 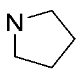 、 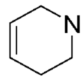 、 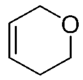 、 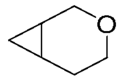 、

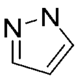 、 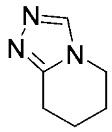 、 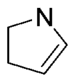 or 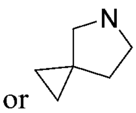 ,

which are optionally further substituted by one or more substituents selected from methyl, halogen, oxo, cyclopropyl, $NS(O)R_{a1}R_{b1}$, - $(CH_2)_{n1}S(O)NR_{a2}R_{b2}$, $-P(O)R_{a3}R_{b3}$, $-NR_{a5}S(O)NR_{a4}R_{b4}$ , $(CH_2)_{n1}S(O)_2R_{a5}$, $-(CH_2)_{n1}O(CH_2)_{n2}R_{b5}$ ,

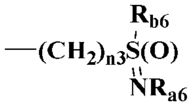 ,

$(CH_2)_{n1}P(O)R_{a7}R_{b7}$ or $-(CR_{a8}R_{b8})_{n4}C(O)NR_{a9}R_{b9}$.

In a further preferred embodiment of the present invention, the compound of general formula (XI) is further shown in general formula (XI-1):

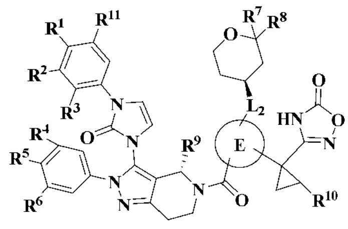

(XI-1)

.

An object of the present invention is to provide a compound represented by general formula (XV), a prodrug thereof, a stereoisomer thereof or a pharmaceutically acceptable salt thereof:

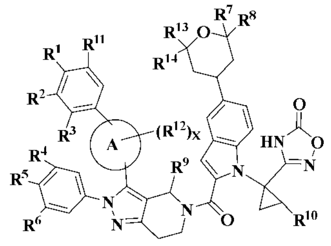

(XV)

$R^1$, $R^2$, $R^3$ or $R^{11}$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, 5-10 membered heteroaryl, $-NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$ , $-(CH_2)_{n1}O(CH_2)_{n2}R_{a5}$ , $-(CH_2)_{n1}C(O)NR_{a6}R_{b6}$, $-(CH_2)_{n1}P(O)R_{a3}R_{b3}$,

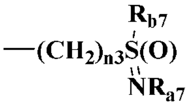,

-$(CR_{a8}R_{b8})_{n4}C(O)NR_{a9}R_{b9}$, or - $(CH_2)_{n5}C(O)NR_{a10}R_{b10}$, the amino, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl are optionally further substituted by one or more substituents selected from halogen, amino, nitro, hydroxyl, cyano, oxo, - $NS(O)R_{a1}R_{b1}$, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

Alternatively, $R^1$ and $R^2$, $R^1$ and $R^{11}$ are linked to adjacent atoms to form $C_{3-12}$ cycloalkyl, 3-12 membered heterocyclic, $C_{6-14}$ aryl or 5-14 membered heteroaryl, the $C_{3-12}$ cycloalkyl, 3-12 membered heterocyclic, $C_{6-14}$ aryl or the 5-14 membered heteroaryl are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R_{a1}$, $R_{b1}$, $R_{a2}$, $R_{b2}$, $R_{a3}$, $R_{b3}$, $R_{a4}$, $R_{b4}$, $R_{a5}$, $R_{a6}$, $R_{b6}$, $R_{a7}$, $R_{b7}$, $R_{a8}$, $R_{b8}$, $R_{a9}$, $R_{b9}$, $R_{a10}$, $R_{b10}$ is each independently selected from hydrogen, deuterium, halogen, amino, nitro, hydroxyl, cyano, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered, the amino, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered are optionally further substituted by one or more substituents selected from halogen, amino, nitro, hydroxyl, cyano, oxo, -$NS(O)R_{a1}R_{b1}$, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

Alternatively, $R_{a1}$ and $R_{b1}$ or $R_{a2}$ and $R_{b2}$ or $R_{a3}$ and $R_{b3}$ or $R_{a4}$ and $R_{b4}$ or $R_{a6}$ and $R_{b6}$ or $R_{a8}$ and $R_{b8}$ or $R_{a9}$ and $R_{b9}$ or $R_{a10}$ and $R_{b10}$ are linked with adjacent atoms to form $C_{3-8}$ cycloalkyl, 3-8 membered heterocyclic group, $C_{6-10}$ aryl or 5-10 membered heteroaryl, the $C_{3-8}$ cycloalkyl, 3-8 membered heterocyclic, $C_{6-10}$ aryl or 5-10 membered heteroaryl, which are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl.

Ring A is selected from $C_{3-12}$ cycloalkyl, 3-12 membered heterocyclyl, $C_{6-14}$ aryl or 5-14 membered heteroaryl, which are optionally further substituted by one or more substituents selected from halogen, amino, nitro, hydroxy, cyano, oxo, mercapto, thio, $C_{1-6}$ alkyl, $C_{1-6}$ deuterated alkyl, $C_{1-6}$ haloalkyl, $C_{1-6}$ hydroxyalkyl, $C_{1-6}$ alkoxy or $C_{1-6}$ haloalkoxy.

$R^4$, $R^5$ or $R^6$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, the amino, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R^7$, $R^8$, $R^9$, $R^{10}$, $R^{12}$, $R^{13}$, or $R^{14}$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, which are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

Alternatively, $R^7$, $R^8$, $R^9$, $R^{10}$, $R^{12}$, $R^{13}$, or $R^{14}$ is linked to the atoms connected thereto to form $C_{3-10}$ cycloalkyl, 3-10 membered heterocyclyl, $C_{6-12}$ aryl or 5-12 membered heteroaryl;

n1-n5 is an integer from 0 to 5;

x is an integer from 0 to 5.

In a further preferred embodiment of the present invention, the general formula (XV) compound is as follows:

$R^1$, $R^2$, $R^3$ or $R^{11}$ is each independently selected from hydrogen, deuterium, halogen, amino, nitro, hydroxyl, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-8}$ cycloalkyl, 3-8 membered heterocyclyl, $C_{6-10}$ aryl, 5-8 membered heteroaryl, $-NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$ , $-(CH_2)_{n1}O(CH_2)_{n2}R_{a5}$ , $-(CH_2)_{n1}C(O)NR_{a6}R_{b6}$, $-(CH_2)_{n1}P(O)R_{a3}R_{b3}$,

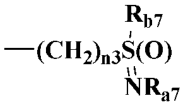,

$-(CR_{a8}R_{b8})_{n4}C(O)NR_{a9}R_{b9}$ or $-(CH_2)_{a5}C(O)NR_{a10}R_{b10}$, the amino, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-8}$ cycloalkyl, 3-8 membered heterocyclic, $C_{6-10}$ aryl or 5-10 membered heteroaryl are optionally further substituted by one or more substituents selected from halogen, amino, nitro, hydroxyl, cyano, oxo, $-NS(O)R_{a1}R_{b1}$, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclic, $C_{6-10}$ aryl or 5-8 membered heteroaryl;

Preferably, $R^1$, $R^2$, $R^3$ or $R^{11}$ is each independently selected from hydrogen, deuterium, halogen, amino, $C_{1-3}$ alkyl, $C_{1-3}$ deuterated alkyl, $C_{1-3}$ haloalkyl, $C_{2-4}$ alkynyl, $C_{3-8}$ cycloalkyl, 3-8 membered heterocyclic, 5-8 membered heteroaryl, $-NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$ , $-(CH_2)_{n1}O(CH_2)_{n2}R_{a5}$ , $-(CH_2)_{n1}C(O)NR_{a6}R_{b6}$ , $-(CH_2)_{n1}P(O)R_{a3}R_{b3}$,

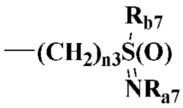,

$-(CR_{a8}R_{b8})_{n4}C(O)NR_{a9}R_{b9}$ or $-(CH_2)_{n5}C(O)NR_{a10}R_{b10}$, the amino, $C_{1-3}$ alkyl, $C_{1-3}$ deuterated alkyl, $C_{1-3}$ haloalkyl, or $C_{2-4}$ alkynyl are optionally further substituted by one or more substituents selected from halogen, $-NS(O)R_{a1}R_{b1}$, $C_{1-3}$ alkyl or $C_{3-6}$ cycloalkyl;

Further preferably, $R^1$, $R^2$, $R^3$ or $R^{11}$ is each independently selected from hydrogen, deuterium, halogen, amino, $C_{1-3}$ alkyl, $C_{1-3}$ deuterated alkyl, $C_{1-3}$ haloalkyl, $C_{2-4}$ alkynyl, $C_{3-8}$ bridged cycloalkyl, $C_{3-8}$ spirocycloalkyl, 3-8 membered spiro heterocyclic containing 1-3 heteroatoms, 5-8 membered heteroaryl, $NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$ , $-(CH_2)_{n1}O(CH_2)_{n2}R_{a5}$ , $-(CH_2)_{n1}C(O)NR_{a6}R_{b6}$ , $-(CH_2)_{n1}P(O)R_{a3}R_{b3}$,

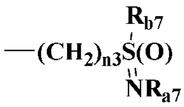,

$-(CR_{a8}R_{b8})_{n4}C(O)NR_{a9}R_{b9}$ or $-(CH_2)_{n5}C(O)NR_{a10}R_{b10}$, the amino, $C_{1-3}$ alkyl, $C_{1-3}$ deuterated alkyl, $C_{1-3}$ haloalkyl, or $C_{2-4}$ alkynyl are optionally further substituted by one or more substituents selected from halogen, $-NS(O)R_{a1}R_{b1}$, $C_{1-3}$ alkyl or $C_{3-6}$ cycloalkyl.

In a further preferred embodiment of the present invention, the general formula (XV) compound is as follows:

$R^1$ and $R^2$ or $R^1$ and $R^{11}$ are linked to adjacent atoms to form $C_{3-6}$ cycloalkyl, 3-8 membered heterocyclic, $C_{6-10}$ aryl or 5-8 membered heteroaryl, are optionally further substituted by one or more substituents selected from halogen, $C_{1-3}$ alkyl, $C_{3-6}$ cycloalkyl, amino or oxo'

Preferably, $R^1$ and $R^2$ or $R^1$ and $R^{11}$ are linked to adjacent atoms to form 5-7 membered heterocyclic containing 1-3 atoms selected from N, O or S, which is optionally further substituted by $C_{3-6}$ cycloalkyl or oxo;

Further preferably, $R^1$ and $R^2$ or $R^1$ and $R^{11}$ are linked to adjacent phenyl to form

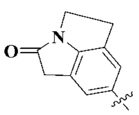、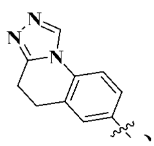、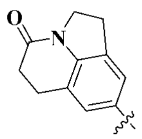、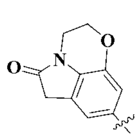、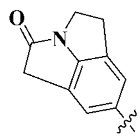、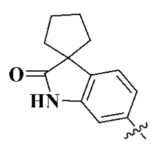、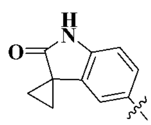、

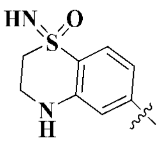、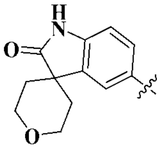、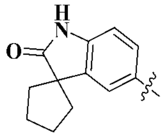、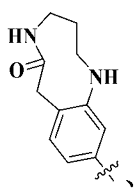、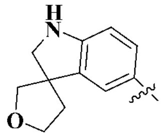、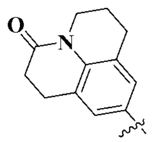、

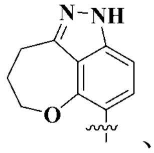、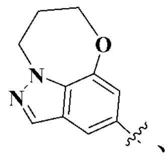、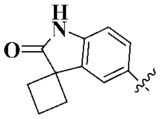、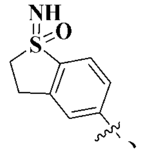、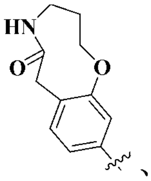、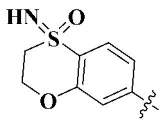、

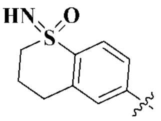、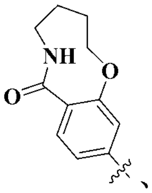、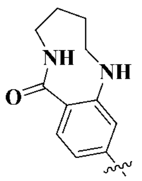、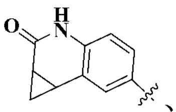、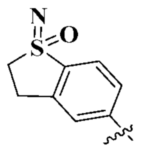、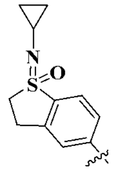、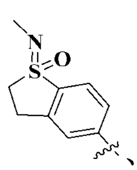、

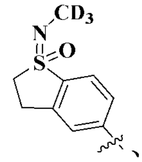、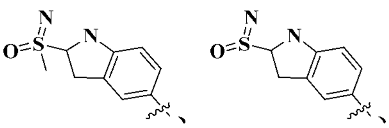、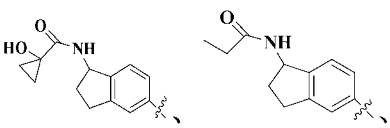、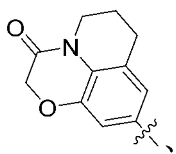、

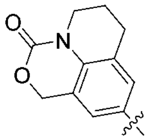、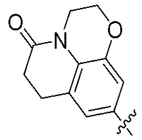、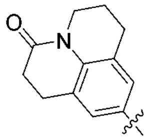、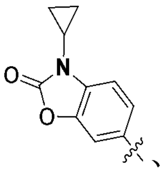、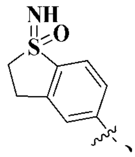、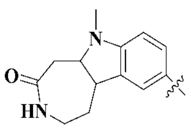、

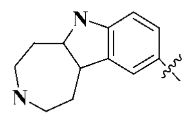、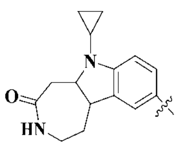、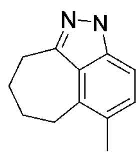、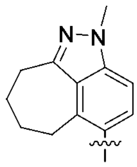、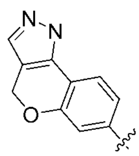、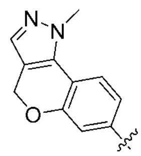、

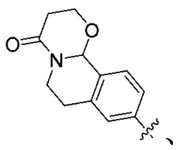、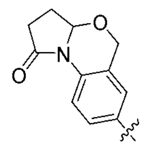、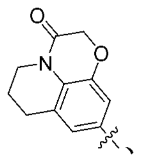、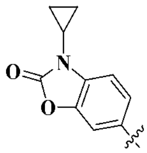 or 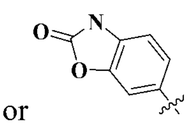,

which are optionally further substituted by one or more substituents selected from halogen, $C_{1-3}$ alkyl, $C_{1-3}$ deuterated alkyl, $C_{3-6}$ cycloalkyl, amino or oxo.

In a further preferred embodiment of the present invention, the general formula (XV) compound is as follows:

$R_{a1}$, $R_{b1}$, $R_{a2}$, $R_{b2}$, $R_{a3}$, $R_{b3}$, $R_{a4}$, $R_{b4}$, $R_{a5}$, $R_{a6}$, $R_{b6}$, $R_{a7}$, $R_{b7}$, $R_{a8}$, $R_{b8}$, $R_{a9}$, $R_{b9}$, $R_{a10}$, $R_{b10}$ is each independently selected from hydrogen, deuterium, halogen, amino, $C_{1-3}$ alkyl, $C_{1-3}$ deuterated alkyl, $C_{1-3}$ haloalkyl, $C_{3-6}$ bridged cycloalkyl, $C_{3-6}$ spirocycloalkyl, 3-8 membered heterocyclic containing 1-3 heteroatoms selected from N, O or S, $C_{6-10}$ aryl, or 5-6 membered heteroaryl containing $_{1-3}$ heteroatoms selected from N, O or S, the amino, $C_{1-3}$ alkyl, $C_{1-3}$ deuterated alkyl, $C_{1-3}$ haloalkyl, $C_{3-6}$ bridged cycloalkyl, $C_{3-6}$ spirocycloalkyl, 3-8 membered heterocyclic group

containing $_{1-3}$ heteroatoms selected from N, O or S, C6-10 aryl or 5-6 membered heteroaryl containing $_{1-3}$ heteroatoms selected from N, O or S are optionally further substituted by one or more substituents selected from halogen or $C_{1-3}$ alkyl;

Preferably, $R_{a1}$, $R_{b1}$, $R_{a2}$, $R_{b2}$, $R_{a3}$, $R_{b3}$, $R_{a4}$, $R_{b4}$, $R_{a5}$, $R_{a6}$, $R_{b6}$, $R_{a7}$, $R_{b7}$, $R_{a8}$, $R_{b8}$, $R_{a9}$, $R_{b9}$, $R_{a10}$, $R_{b10}$ is each independently selected from hydrogen, deuterium, halogen, methyl, deuterated methyl, cyclopropyl,

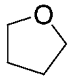 、 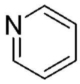 、 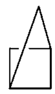 or 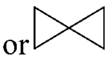,

which are optionally further substituted by fluorine.

In a further preferred embodiment of the present invention, the general formula (XV) compound is as follows:

$R_{a1}$ and $R_{b1}$ or $R_{a2}$ and $R_{b2}$ or $R_{a3}$ and $R_{b3}$ or $R_{a4}$ and $R_{b4}$ or $R_{a6}$ and $R_{b6}$ or $R_{a8}$ and $R_{b8}$ or $R_{a9}$ and $R_{b9}$ or $R_{a10}$ and $R_{b10}$ are linked with adjacent atoms to form $C_{3-8}$ cycloalkyl, 3-6 membered heterocyclic group, $C_{6-10}$ aryl or 5-8 membered heteroaryl;

Preferably, $R_{a1}$ and $R_{b1}$ or $R_{a2}$ and $R_{b2}$ or $R_{a3}$ and $R_{b3}$ or $R_{a4}$ and $R_{b4}$ or $R_{a6}$ and $R_{b6}$ or $R_{a8}$ and $R_{b8}$ or $R_{a9}$ and $R_{b9}$ or $R_{a10}$ and $R_{b10}$ are linked with adjacent atoms to form $C_{3-6}$ cycloalkyl, or 3-6 membered heterocyclic containing 1-3 heteroatoms.

In a further preferred embodiment of the present invention, the general formula (XV) compound is as follows:

Ring A is selected from $C_{3-8}$ cycloalkyl, 3-8 membered heterocyclyl, $C_{6-10}$ aryl or 5-10 membered heteroaryl, which are optionally further substituted by one or more substituents selected from halogen, amino, oxo, mercapto, thio or $C_{1-6}$ alkyl;

Preferably, ring A is selected from $C_{3-6}$ cycloalkyl, 3-6 membered heterocyclyl containing 1-3 N, O or S atoms, or 5-8 membered heteroaryl containing 1-3 N, O or S atoms; which are optionally further substituted by one or more substituents selected from halogen, amino, oxo, mercapto, thio or $C_{1-3}$ alkyl;

Further preferably, ring A is selected from

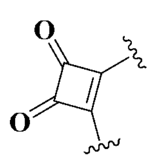 、 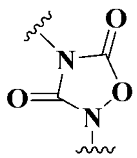 、 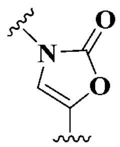

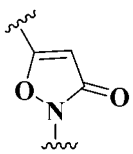 、 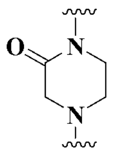 、 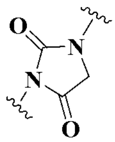 、 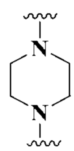 、 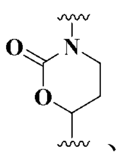 、 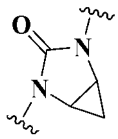 、 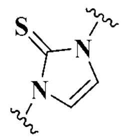 、

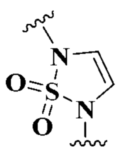 、 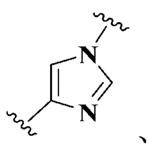 、 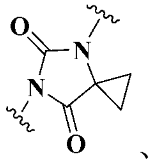 、 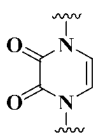 or 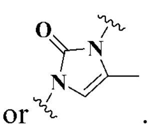 .

In a further preferred embodiment of the present invention, the general formula (XV) compound is as follows:

$R^4$, $R^5$ or $R^6$ is each independently selected from hydrogen, deuterium, halogen, amino, cyano, $C_{3-6}$ cycloalkyl, $C_{1-3}$ alkyl, $C_{1-3}$ deuterated alkyl or $C_{1-3}$ haloalkyl;

Preferably, $R^4$, $R^5$ or $R^6$ is each independently selected from fluoro, cyano, cyclopropyl or methyl.

In a further preferred embodiment of the present invention, the general formula (XV) compound is as follows:

$R^7$, $R^8$, $R^9$, $R^{10}$, $R^{12}$, $R^{13}$, or $R^{14}$ is each independently selected from hydrogen, deuterium, halogen, $C_{3-6}$ cycloalkyl, $C_{1-3}$ alkyl, $C_{1-3}$ deuterated alkyl or $C_{1-3}$ haloalkyl;
Preferably, $R^7$, $R^8$, $R^9$, $R^{10}$, $R^{12}$, $R^{13}$, or $R^{14}$ is each independently selected from methyl or cyclopropyl.

In a further preferred embodiment of the present invention, the general formula (XV) compound is as follows:

$R^7$, $R^8$, $R^9$, $R^{10}$, $R^{12}$, $R^{13}$, or $R^{14}$ is linked to the atoms connected thereto to form $C_{3-6}$ cycloalkyl, 3-6 membered heterocyclyl, $C_{6-10}$ aryl or 5-8 membered heteroaryl;
Preferably, $R^7$, $R^8$, $R^9$, $R^{10}$, $R^{12}$, $R^{13}$, or $R^{14}$ is linked to the atoms connected thereto to form cyclopropyl.

In a further preferred embodiment of the present invention, the compound of general formula (XV) is further represented by general formula (XV-1):

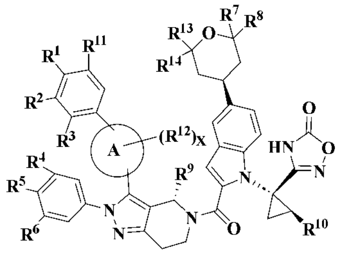

(XV-1)

.

An object of the present invention is to provide a compound represented by general formula (IV), a prodrug thereof, a stereoisomer thereof or a pharmaceutically acceptable salt thereof:

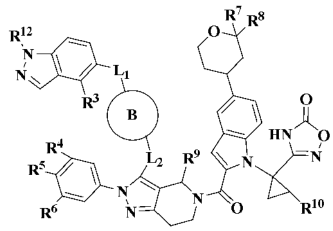

(IV)

wherein,

$L_1$ and $L_2$ are each independently selected from a bond, a $C_{2-4}$ alkenyl group, a $C_{2-4}$ alkynyl, $-(CH_2)_{n2}-$, $-(CH2)_{n3}NR_{b5}$;
ring B is selected from $C_{3-12}$ cycloalkyl, 3-12 membered heterocyclyl, $C_{6-14}$ aryl or 5-14 membered heteroaryl, which are optionally further substituted with one or more substituents selected from halogen, amino, nitro, hydroxyl, cyano, oxo, thiol, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy or $C_{1-6}$ deuterated haloalkoxy;
$R_{b5}$ is selected from hydrogen, deuterium, halogen, amino, nitro, hydroxyl, cyano, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, 5-10 membered heteroaryl;
$R^3$ is selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, 5-10 membered heteroaryl, $-NS(O)R_{a1}R_{b1}$, $-(CH_2)_{n1}S(O)NR_{a2}R_{b2}$, $-P(O)R_{a3}R_{b3}$ or $-NR_{a5}S(O)NR_{a4}R_{b4}$, the amino, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl are optionally further substituted by one or more substituents selected from halogen, amino, nitro, hydroxyl, cyano, oxo, $-NS(O)R_{a1}R_{b1}$, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;
$R_{a1}$, $R_{b1}$, $R_{a2}$, $R_{b2}$, $R_{a3}$, $R_{b3}$, $R_{a4}$ or $R_{b4}$ is each independently selected from hydrogen, deuterium, halogen, amino, nitro, hydroxyl, cyano, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy,

$C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl; alternatively, $R_{a1}$, $R_{b1}$, $R_{a2}$, $R_{b2}$, $R_{a3}$, $R_{b3}$, $R_{a4}$ or $R_{b4}$ is linked to adjacent atoms to form $C_{3-8}$ cycloalkyl, 3-8 membered heterocyclic, $C_{6-10}$ aryl or 5-10 membered heteroaryl, the $C_{3-8}$ cycloalkyl, 3-8 membered heterocyclic, $C_{6-10}$ aryl or 5-10 membered heteroaryl are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R^4$, $R^5$ or $R^6$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, the amino, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R^7$, $R^8$, $R^9$ or $R^{10}$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, which are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R^{12}$ is selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, which are optionally further substituted by one or more substituents selected from halogen, amino, nitro, hydroxy, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, or 5-10 membered heteroaryl;

n1 is an integer between 0 and 5;

n2 is an integer between 0 and 5;

n3 is an integer between 0 and 5.

In a further preferred embodiment of the present invention, the general formula (IV) compound is as follows:

$L_1$ and $L_2$ are each independently selected from a bond, $-(CH_2)_{n3}NR_{b5}$.

In a further preferred embodiment of the present invention, the general formula (IV) compound is as follows:

Ring B is selected from $C_{3-6}$ cycloalkyl, 3-6 membered heterocyclic containing 1-3 N, O or S atoms, or 5-8 membered heteroaryl containing 1-3 N, O or S atoms; optionally further substituted by one or more substituents selected from deuterium, halogen, amino, oxo, thio or $C_{1-3}$ alkyl;

Preferably, ring B is selected from

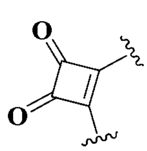 、 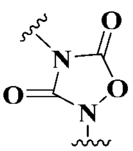 、 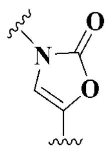 、 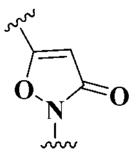 、

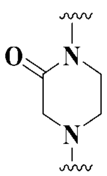 、 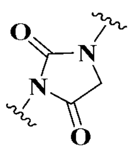 、 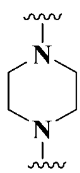 、 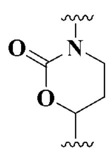 、 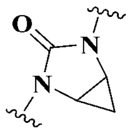 、 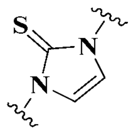 、 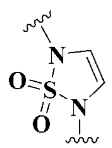 、 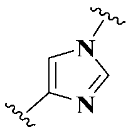 、

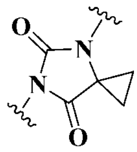 or 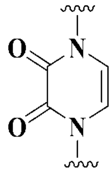 .

In a further preferred embodiment of the present invention, the general formula (IV) compound is as follows:

Ring B is selected from $C_{3-6}$ cycloalkyl, 3-6 membered heterocyclic containing 1-3 N, O or S atoms, or 5-10 membered heteroaryl containing 1-3 N, O or S atoms; optionally further substituted by one or more substituents selected from deuterium, halogen, amino, oxo, thio or $C_{1-3}$ alkyl;

Preferably, ring B is selected from

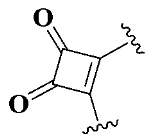 、 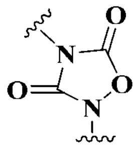 、 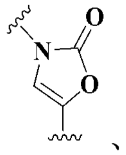 、 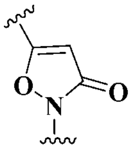 、

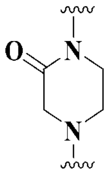 、 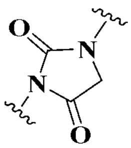 、 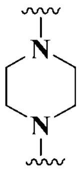 、 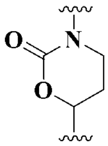 、 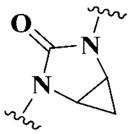 、 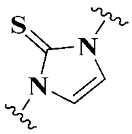 、 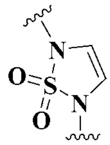 、 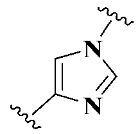 .

In a further preferred embodiment of the present invention, the general formula (IV) compound is as follows:

Ring B is selected from $C_{3-6}$ cycloalkyl, 3-6 membered heterocyclic containing 1-3 N, O or S atoms, or 5-10 membered heteroaryl containing 1-3 N, O or S atoms; optionally further substituted by one or more substituents selected from deuterium, halogen, amino, oxo, thio or $C_{1-3}$ alkyl;
Preferably, ring B is selected from

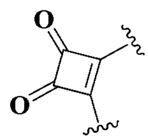 、 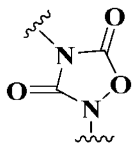 、 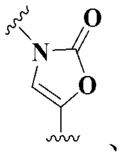 、 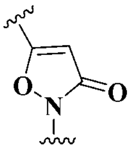 、

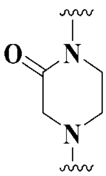 、 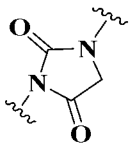 、 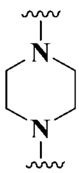 、 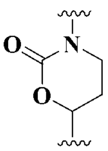 、 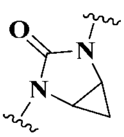 、 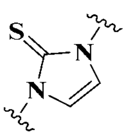 、 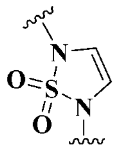 、 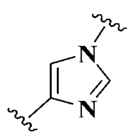 or

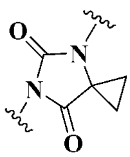 .

In a further preferred embodiment of the present invention, the general formula (IV) compound is as follows:

$R^3$ is selected from hydrogen, deuterium, halogen, amino, nitro, hydroxyl, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6

membered heterocyclyl, $C_{6-10}$ aryl, 5-8 membered heteroaryl, -NS(O)$R_{a1}R_{b1}$, -$(CH_2)_{n1}$S(O)N$R_{a2}R_{b2}$, -P(O)$R_{a3}R_{b3}$, -N$R_{a5}$S(O)N$R_{a4}R_{b4}$, the amino, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-8}$ cycloalkyl, 3-8 membered heterocyclic, $C_{6-10}$ aryl and 5-10 membered heteroaryl are optionally further substituted by one or more of halogen, amino, nitro, hydroxyl, cyano, oxo, -NS(O)$R_{a1}R_{b1}$, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclic, $C_{6-10}$ aryl and 5-8 membered heteroaryl;

Preferably, $R^3$ is selected from hydrogen, deuterium, halogen, amino, $C_{1-3}$ alkyl, $C_{1-3}$ deuterated alkyl, $C_{1-3}$ haloalkyl, -NS(O)$R_{a1}R_{b1}$, -$(CH_2)_{n1}$S(O)N$R_{a2}R_{b2}$, -P(O)$R_{a3}R_{b3}$, or - N$R_{a5}$S(O)N$R_{a4}R_{b4}$, the amino, $C_{1-3}$ alkyl, $C_{1-3}$ deuterated alkyl or $C_{1-3}$ haloalkyl are optionally further substituted by one or more substituents selected from halogen, -NS(O)$R_{a1}R_{b1}$ or $C_{1-3}$ alkyl.

In a further preferred embodiment of the present invention, the general formula (IV) compound is as follows:

$R_{a1}$, $R_{b1}$, $R_{a2}$, $R_{b2}$, $R_{a3}$, $R_{b3}$, $R_{a4}$ or $R_{b4}$ is each independently selected from hydrogen, deuterium, halogen, amino, $C_{1-3}$ alkyl, $C_{1-3}$ deuterated alkyl or $C_{1-3}$ haloalkyl;

Preferably, $R_{a1}$, $R_{b1}$, $R_{a2}$, $R_{b2}$, $R_{a3}$, $R_{b3}$, $R_{a4}$ or $R_{b4}$ is each independently selected from hydrogen, deuterium, halogen, or methyl.

In a further preferred embodiment of the present invention, the general formula (IV) compound is as follows: $R_{a1}$ and $R_{b1}$, $R_{a2}$ and $R_{b2}$, $R_{a3}$ and $R_{b3}$, $R_{a4}$ and $R_{b4}$ are linked to adjacent atoms to form $C_{3-8}$ cycloalkyl, 3-6 membered heterocyclic, $C_{6-10}$ aryl or 5-8 membered heteroaryl.

In a further preferred embodiment of the present invention, the general formula (IV) compound is as follows: $R_{a2}$ and $R_{b2}$, $R_{a3}$ and $R_{b3}$ are linked to adjacent atoms to form $C_{3-8}$ cycloalkyl group or 3-6 membered heterocyclic containing 1-3 heteroatoms.

In a further preferred embodiment of the present invention, the general formula (IV) compound is as follows:

$R^4$, $R^5$ or $R^6$ is each independently selected from hydrogen, deuterium, halogen, amino, $C_{1-3}$ alkyl, $C_{1-3}$ deuterated alkyl or $C_{1-3}$ haloalkyl;

Preferably, $R^4$, $R^5$ or $R^6$ is each independently selected from fluorine or methyl.

In a further preferred embodiment of the present invention, the general formula (IV) compound is as follows:

$R^7$, $R^8$, $R^9$ or $R^{10}$ is each independently selected from hydrogen, deuterium, halogen, $C_{1-3}$ alkyl, $C_{1-3}$ deuterated alkyl or $C_{1-3}$ haloalkyl;

Preferably, $R^7$, $R^8$, $R^9$ or $R^{10}$ is each independently selected from methyl.

In a further preferred embodiment of the present invention, the general formula (IV) compound is as follows:

$R^{12}$ is selected from $C_{1-3}$ alkyl;

Preferably, $R^{12}$ is selected from methyl.

In a further preferred embodiment of the present invention, the general formula (IV) compound is as follows: Ring B is selected from

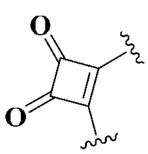 、 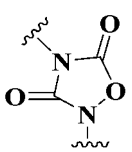 、 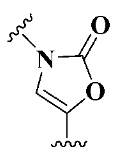 、 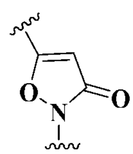 、 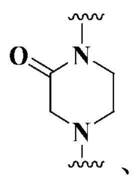 、

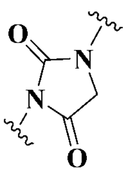 、 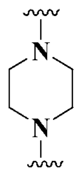 、 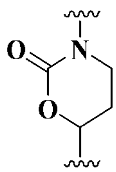 、 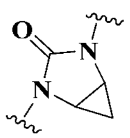 、 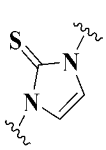 、 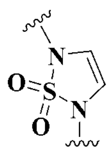 、 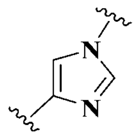 、 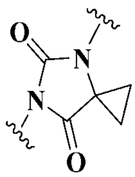 、

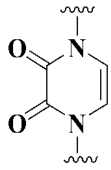 or 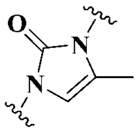 .

In a further preferred embodiment of the present invention, the general formula (IV) compound is as follows: $R^{12}$ is selected from hydrogen, deuterium, halogen, amino, nitro, hydroxyl, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl, which are optionally further substituted by one or more substituents selected from halogen, amino, nitro, hydroxyl, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl.

Preferably, $R^{12}$ is selected from $C_{1-3}$ alkyl or $C_{3-6}$ cycloalkyl;

Further preferably, $R^{12}$ is selected from methyl or cyclopropyl.

In a further preferred embodiment of the present invention, the compound of general formula (IV) is further represented by general formula (IV-1):

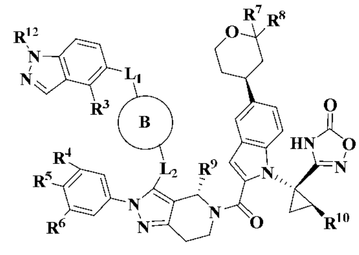

(IV-1) .

An object of the present invention is to provide a compound represented by general formula (VI), a prodrug thereof, a stereoisomer thereof or a pharmaceutically acceptable salt thereof:

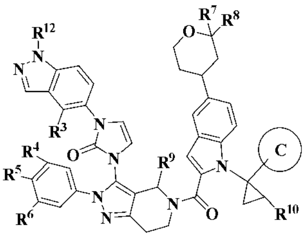

(VI)

wherein,

Ring C is selected from $C_{3-12}$ cycloalkyl, 3-12 membered heterocyclyl, $C_{6-14}$ aryl or 5-14 membered heteroaryl, which are optionally further substituted by one or more substituents selected from halogen, amino, nitro, hydroxyl, cyano, oxo, thiol, thio, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $-NR_{a6}S(O)_2R_{b6}$.

$R^{10}$ is selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, which are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aromatic or 5-10 membered heteroaryl;

Alternatively, $R^{10}$ is linked to the connected C atoms to form $C_{3-8}$ cycloalkyl, 3-8 membered heterocyclic, $C_{6-10}$ aryl or 5-10 membered heteroaryl, the $C_{3-8}$ cycloalkyl, 3-8 membered heterocyclic, $C_{6-10}$ aryl or 5-10 membered heteroaryl are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ sulfanyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or a 5-10 membered heteroaryl;

$R_{a6}$ and $R_{b6}$ are each independently selected from hydrogen, deuterium, halogen, amino, nitro, hydroxyl, cyano, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, 5-10 membered heteroaryl;

Wherein when $R^3$ is fluorine, $R_4$ and $R_6$ are methyl, $R_5$ is fluorine, $R^7$ and $R^8$ are hydrogen or methyl, $R^{12}$ is methyl, and ring C is

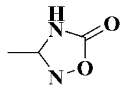,

$R^{10}$ cannot be methyl at the same time;

When $R^3$ is fluorine, $R^4$ and $R^6$ are methyl, $R^5$ is fluorine, $R^7$ and $R^8$ are hydrogen, $R^{12}$ is methyl, and ring C is

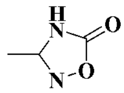,

$R^{10}$ cannot be hydroxymethyl at the same time;

When $R^3$ is fluorine, $R^4$ and $R^6$ are methyl, $R^5$ is fluorine, $R^7$ and $R^8$ are hydrogen, $R^{12}$ is methyl, and ring C is

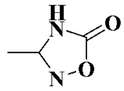,

$R^{10}$ cannot be

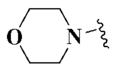

at the same time;

$R^3$ is selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, 5-10 membered heteroaryl, $-NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$, the amino, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl and 5-10 membered heteroaryl are optionally further substituted by one or more of halogen, amino, nitro, hydroxyl, cyano, oxo, $-NS(O)R_{a1}R_{b1}$, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl and 5-10 membered heteroaryl;

$R_{a1}$, $R_{b1}$, $R_{a2}$, $R_{b2}$, $R_{a3}$, $R_{b3}$, $R_{a4}$ or $R_{b4}$ is each independently selected from hydrogen, deuterium, halogen, amino, nitro, hydroxyl, cyano, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

alternatively, $R_{a1}$, $R_{b1}$, $R_{a2}$, $R_{b2}$, $R_{a3}$, $R_{b3}$, $R_{a4}$ and $R_{b4}$ are linked to adjacent atoms to form $C_{3-8}$ cycloalkyl, 3-8 membered heterocyclic, $C_{6-10}$ aryl or 5-10 membered heteroaryl, the $C_{3-8}$ cycloalkyl, 3-8 membered heterocyclic, $C_{6-10}$ aryl or 5-10 membered heteroaryl are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R^4$, $R^5$ or $R^6$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, the amino, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R^7$, $R^8$, or $R^9$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$

alkynyl, $C_{3-8}$ cycloalkyl, 3-8 membered heterocyclyl, $C_{6-10}$ aryl or 5-10 membered heteroaryl, which are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R^{12}$ is selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, which are optionally further substituted by one or more substituents selected from halogen, amino, nitro, hydroxy, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, or 5-10 membered heteroaryl;

n1 is an integer of 0 to 5.

In a further preferred embodiment of the present invention, the general formula (VI) compound is as follows: Ring C is selected from $C_{3-8}$ cycloalkyl, 3-8 membered heterocyclyl, $C_{6-10}$ aryl or 5-8 membered heteroaryl, which are optionally further substituted by one or more substituents selected from halogen, amino, nitro, hydroxyl, cyano, oxo, thiol, thio, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $-NR_{a6}S(O)_2R_{b6}$.

Preferably, ring C is selected from $C_{3-8}$ cycloalkyl, 3-8 membered heterocycli containing 1-3 atoms selected from N, O or S, and 5-8 membered heteroaryl containing 1-3 atoms selected from N, O or S, which are optionally further substituted by one or more substituents selected from hydroxyl, oxo, $-NR_{a6}S(O)_2R_{b6}$;

Further preferably, ring C is selected from

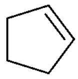 、 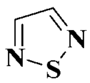 、 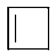 、 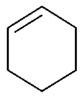 、 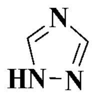

or

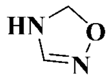 ,

which are optionally further substituted by one or more substituents selected from hydroxyl, oxo, $-NR_{a6}S(O)_2R_{b6}$.

In a further preferred embodiment of the present invention, the general formula (VI) compound is as follows:

$R^{10}$ is selected from hydrogen, deuterium, halogen $C_{1-3}$ alkyl;

Preferably, $R^{10}$ is selected from methyl.

In a further preferred embodiment of the present invention, the general formula (VI) compound is as follows:

$R^{10}$ is linked to the connected C atoms to form $C_{3-6}$ cycloalkyl;

Preferably, $R^{10}$ is linked to the connected C atoms to form $C_{3-6}$ cycloalkyl.

In a further preferred embodiment of the present invention, the general formula (VI) compound is as follows:

$R_{a6}$ and $R_{b6}$ are each independently selected from hydrogen, deuterium, halogen, amino, nitro, hydroxyl, cyano, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-3}$ alkenyl, $C_{2-3}$ alkynyl, $C_{3-8}$ cycloalkyl, 5-7 membered heterocyclic, $C_{6-10}$ aryl, 5-8 membered heteroaryl;

Preferably, $R_{a6}$ and $R_{b6}$ are each independently selected from hydrogen or methyl.

In a further preferred embodiment of the present invention, the general formula (VI) compound is as follows:

$R^3$ is selected from hydrogen, deuterium, halogen, amino, nitro, hydroxyl, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclyl, $C_{6-10}$ aryl, 5-8 membered heteroaryl, $-NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$, the amino, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-8}$ cycloalkyl, 3-8 membered heterocyclic, $C_{6-10}$ aryl and 5-10 membered heteroaryl are optionally further substituted by one or more of halogen, amino, nitro, hydroxyl, cyano, oxo, $-NS(O)R_{a1}R_{b1}$, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl,

$C_{2-4}$ alkynyl, $C_{3-6}$ cycloalkyl, 3-6 membered heterocyclic, $C_{6-10}$ aryl and 5-8 membered heteroaryl;
Preferably, $R^3$ is selected from hydrogen, deuterium, halogen, amino, $C_{1-3}$ alkyl, $C_{1-3}$ deuterated alkyl, $C_{1-3}$ haloalkyl, $-NS(O)R_{a1}R_{b1}$, $-(CH_2)_{n1}S(O)NR_{a2}R_{b2}$, $-P(O)R_{a3}R_{b3}$, or $-NR_{a5}S(O)NR_{a4}R_{b4}$, the amino, $C_{1-3}$ alkyl, $C_{1-3}$ deuterated alkyl or $C_{1-3}$ haloalkyl are optionally further substituted by one or more substituents selected from halogen, $-NS(O)R_{a1}R_{b1}$ or $C_{1-3}$ alkyl.

In a further preferred embodiment of the present invention, the general formula (VI) compound is as follows:

$R_{a1}$, $R_{b1}$, $R_{a2}$, $R_{b2}$, $R_{a3}$, $R_{b3}$, $R_{a4}$ or $R_{b4}$ is each independently selected from hydrogen, deuterium, halogen, amino, $C_{1-3}$ alkyl, $C_{1-3}$ deuterated alkyl or $C_{1-3}$ halogenated alkyl;
Preferably, $R_{a1}$, $R_{b1}$, $R_{a2}$, $R_{b2}$, $R_{a3}$, $R_{b3}$, $R_{a4}$ or $R_{b4}$ is each independently selected from hydrogen, deuterium, halogen, or methyl.

In a further preferred embodiment of the present invention, the general formula (VI) compound is as follows:

$R_{a1}$ and $R_{b1}$, $R_{a2}$ and $R_{b2}$, $R_{a3}$ and $R_{b3}$, $R_{a4}$ and $R_{b4}$, are respectively linked to adjacent atoms to form $C_{3-8}$ cycloalkyl, 3-6 membered heterocyclyl, $C_{6-10}$ aryl or 5-8 membered heteroaryl;
Preferably, $R_{a2}$ and $R_{b2}$, $R_{a3}$ and $R_{b3}$ is linked to adjacent atoms to form $C_{3-8}$ cycloalkyl, or 3-6 membered heterocyclic containing 1-3 heteroatoms.

In a further preferred embodiment of the present invention, the general formula (VI) compound is as follows:

$R^4$, $R^5$ or $R^6$ is each independently selected from hydrogen, deuterium, halogen, amino, $C_{1-3}$ deuterated alkyl or $C_{1-3}$ halogenated alkyl;
Preferably, $R^4$, $R^5$ or $R^6$ is each independently selected from fluorine or methyl.

In a further preferred embodiment of the present invention, the general formula (VI) compound is as follows:

$R^7$, $R^8$, or $R^9$ is each independently selected from hydrogen, deuterium, halogen, $C_{1-3}$ alkyl, $C_{1-3}$ deuterated alkyl or $C_{1-3}$ haloalkyl;
Preferably, $R^7$, $R^8$, or $R^9$ is each independently selected from methyl.

In a further preferred embodiment of the present invention, the general formula (VI) compound is as follows:

$R^{12}$ is selected from hydrogen, deuterium, halogen, amino, nitro, hydroxyl, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl, which are optionally further substituted by one or more substituents selected from halogen, amino, nitro, hydroxyl, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl;
Preferably, $R^{12}$ is selected from $C_{1-3}$ alkyl;
Further preferably, $R_{12}$ is selected from methyl.

In a further preferred embodiment of the present invention, the compound of general formula (VI) is further represented by general formula (VI-1):

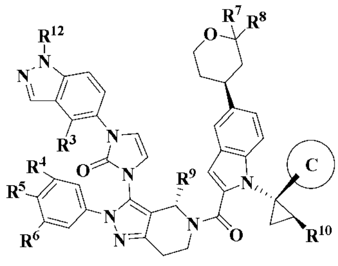

(VI-1)

.

An object of the present invention is to provide a compound represented by general formula (VII), a prodrug

thereof, a stereoisomer thereof or a pharmaceutically acceptable salt thereof:

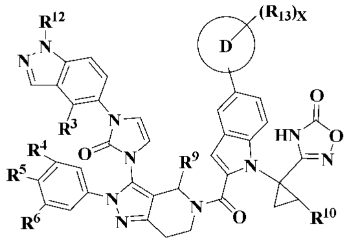

(VII)

wherein,

Ring D is selected from $C_{3-12}$ cycloalkyl, 3-12 membered heterocyclyl, $C_{6-14}$ aryl or 5-14 membered heteroaryl;

$R^{13}$ is selected from hydrogen, deuterium, halogen, amino, nitro, hydroxyl, cyano, oxo, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl, which are optionally further substituted by one or more substituents selected from deuterium, halogen, amino, nitro, hydroxyl, cyano, oxo, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

$R^3$ is selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, 5-10 membered heteroaryl, $-NS(O)R_{a1}R_{b1}$, $-(CH_2)_{n1}S(O)NR_{a2}R_{b2}$, $-P(O) R_{a3}R_{b3}$ or $-NR_{a5}S(O)NR_{a4}R_{b4}$, the amino, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl are optionally further substituted by one or more substituents selected from halogen, amino, nitro, hydroxyl, cyano, oxo, $-NS(O)R_{a1}R_{b1}$, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R_{a1}$, $R_{b1}$, $R_{a2}$, $R_{b2}$, $R_{a3}$, $R_{b3}$, $R_{a4}$ or $R_{b4}$ is each independently selected from hydrogen, deuterium, halogen, amino, nitro, hydroxyl, cyano, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

alternatively, $R_{a1}$ and $R_{p1}$, $R_{a2}$ and $R_{b2}$, $R_{a3}$ and $R_{b3}$, $R_{a4}$ and $R_{b4}$ is linked to adjacent atoms to form $C_{3-8}$ cycloalkyl, 3-8 membered heterocyclic, $C_{6-10}$ aryl or 5-10 membered heteroaryl, the $C_{3-8}$ cycloalkyl, 3-8 membered heterocyclic, $C_{6-10}$ aryl or 5-10 membered heteroaryl are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R^4$, $R^5$ or $R^6$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, the amino, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R^9$ or $R^{10}$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, which are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R^{12}$ is selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8

membered heterocyclyl, $C_{6-10}$ aryl or 5-10 membered heteroaryl, which are optionally further substituted by one or more substituents selected from halogen, amino, nitro, hydroxy, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, or 5-10 membered heteroaryl;
nl is an integer from 0 to 5;
x is an integer from 0 to 5.

Wherein when $R^3$ is hydrogen, $R^4$ and $R^6$ are methyl, $R^5$ is fluorine, and $R^9$, $R^{10}$, and $R^{12}$ are methyl, $R^{10}$ cannot be

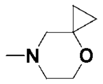 、 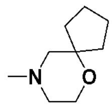 or 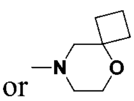

at the same time.

In a further preferred embodiment of the present invention, the general formula (VII) compound is as follows:

Ring D is selected from 5-12-membered bicyclic fused heterocyclic, 5-12-membered bicyclic spiro heterocyclic, and 5-12-membered bicyclic bridged heterocyclic;
Ring D is preferably 5-8-membered bicyclic fused heterocyclic, 5-8-membered bicyclic spiro heterocyclic, and 5-8-membered bicyclic bridged heterocyclic;
Ring D is more preferably

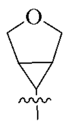 、 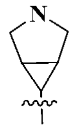 、 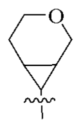 、 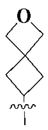 .

In a further preferred embodiment of the present invention, the general formula (VII) compound is as follows: $R^{13}$ is selected from hydrogen, deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl or 5-8 membered heteroaryl, which are optionally further substituted by one or more substituents selected from deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl or 5-8 membered heteroaryl; $R^{13}$ is selected from methyl.

In a further preferred embodiment of the present invention, the general formula (VII) compound is as follows:

Ring D is selected from $C_{3-12}$ cycloalkyl, 3-12 membered heterocyclyl, $C_{6-14}$ aryl or 5-14 membered heteroaryl;
$R^{13}$ is selected from hydrogen, deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl or 5-8 membered heteroaryl, which are optionally further substituted by one or more substituents selected from deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl or 5-8 membered heteroaryl;
$R^3$ is selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, 5-10 membered heteroaryl, $-NS(O)R_{a1}Rb_1$, $-(CH_2)_{n1}S(O)NR_{a2}R_{b2}$, $-P(O)R_{a3}R_{b3}$ or $-NR_{a5}S(O)NR_{a4}R_{b4}$, the amino, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl are optionally further substituted by one or more substituents selected from halogen, amino, nitro, hydroxyl, cyano, oxo, $-NS(O)R_{a1}R_{b1}$, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R_{a1}$, $R_{b1}$, $R_{a2}$, $R_{b2}$, $R_{a3}$, $R_{b3}$, $R_{a4}$ or $R_{b4}$ is each independently selected from hydrogen, deuterium, halogen, amino, nitro, hydroxyl, cyano, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

alternatively, $R_{a1}$ and $R_{b1}$, $R_{a2}$ and $R_{b2}$, $R_{a3}$ and $R_{b3}$, $R_{a4}$ and $R_{b4}$ is linked to adjacent atoms to form $C_{3-8}$ cycloalkyl, 3-8 membered heterocyclic, $C_{6-10}$ aryl or 5-10 membered heteroaryl, the $C_{3-8}$ cycloalkyl, 3-8 membered heterocyclic, $C_{6-10}$ aryl or 5-10 membered heteroaryl are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

alternatively, $R_{a1}$ and $R_{b1}$, $R_{a2}$ and $R_{b2}$, $R_{a3}$ and $R_{b3}$, $R_{a4}$ and $R_{b4}$ is linked to adjacent atoms to form $C_{3-8}$ cycloalkyl, 3-6 membered heterocyclyl, $C_{6-10}$ aryl or 5-8 membered heteroaryl;

$R^4$, $R^5$ or $R^6$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, the amino, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R^9$ or $R^{10}$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, which are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R^{12}$ is selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, which are optionally further substituted by one or more substituents selected from halogen, amino, nitro, hydroxy, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, or 5-10 membered heteroaryl;

n1 is an integer of 0 to 5;

x is an integer of 0 to 5.

In another embodiment of the present invention, the general formula (VII) compound is as follows:

Ring D is selected from 3-8 membered monocyclic heterocyclic, 5-12 membered bicyclic fused heterocyclic, 5-12 membered bispiro heterocyclic, 5-12 membered bicyclic bridged heterocyclic;

Preferably, ring D is selected from 5-6 membered monocyclic heterocyclic, 5-10 membered bicyclic fused heterocyclic, 5-10 membered bispiro heterocyclic, 5-8 membered bicyclic bridged heterocyclic;

More preferably, ring D is selected from

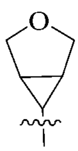 、 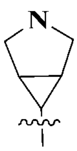 、 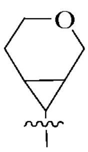 、 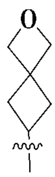 、 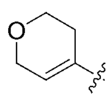

or

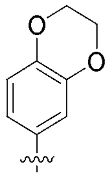 .

In another embodiment of the present invention, the general formula (VII) compound is as follows:

$R^{13}$ is selected from hydrogen, deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl or 5-8 membered heteroaryl, which are optionally further substituted by one or more substituents selected from deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl or 5-8 membered heteroaryl;
Preferably, $R_{13}$ is selected from methyl.

In another embodiment of the present invention, the general formula (VII) compound is as follows:

$R^3$ is selected from hydrogen, deuterium, halogen, amino, nitro, hydroxyl, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclyl, $C_{6-10}$ aryl, 5-8 membered heteroaryl, $-NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$, the amino, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-8}$ cycloalkyl, 3-8 membered heterocyclic, $C_{6-10}$ aryl and 5-10 membered heteroaryl are optionally further substituted by one or more of halogen, amino, nitro, hydroxyl, cyano, oxo, $-NS(O)R_{a1}R_{b1}$, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclic, $C_{6-10}$ aryl and 5-8 membered heteroaryl;
Preferably, $R^3$ is selected from hydrogen, deuterium, halogen, amino, $C_{1-3}$ alkyl, $C_{1-3}$ deuterated alkyl, $C_{1-3}$ haloalkyl, $-NS(O)R_{a1}R_{b1}$, $-(CH_2)_{n1}S(O)NR_{a2}R_{b2}$, $-P(O)R_{a3}R_{b3}$, or $-NR_{a5}S(O)NR_{a4}R_{b4}$, the amino, $C_{1-3}$ alkyl, $C_{1-3}$ deuterated alkyl or $C_{1-3}$ haloalkyl are optionally further substituted by one or more substituents selected from halogen, $-NS(O)R_{a1}R_{b1}$ or $C_{1-3}$ alkyl.

In another embodiment of the present invention, the general formula (VII) compound is as follows:

$R_{a1}$, $R_{b1}$, $R_{a2}$, $R_{b2}$, $R_{a3}$, $R_{b3}$, $R_{a4}$ or $R_{b4}$ is each independently selected from hydrogen, deuterium, halogen, amino, $C_{1-3}$ alkyl, $C_{1-3}$ deuterated alkyl or $C_{1-3}$ halogenated alkyl;
Preferably, $R_{a1}$, $R_{b1}$, $R_{a2}$, $R_{b2}$, $R_{a3}$, $R_{b3}$, $R_{a4}$ or $R_{b4}$ is each independently selected from hydrogen, deuterium, halogen, or methyl.

In another embodiment of the present invention, the general formula (VII) compound is as follows:
$R_{a2}$ and $R_{b2}$, $R_{a3}$ and $R_{b3}$ are each independently linked to adjacent atoms to form $C_{3-8}$ cycloalkyl or 3-6 membered heterocyclic containing 1-3 heteroatoms.
In another embodiment of the present invention, the general formula (VII) compound is as follows:

$R^4$, $R^7$ or $R^6$ is each independently selected from hydrogen, deuterium, halogen, amino, $C_{1-3}$ alkyl, $C_{1-3}$ deuterated alkyl or $C_{1-3}$ haloalkyl;
Preferably, $R^4$, $R^5$ or $R^6$ is each independently selected from fluoro or methyl.

In another embodiment of the present invention, the general formula (VII) compound is as follows:

$R_9$ or $R_{10}$ are each independently selected from hydrogen, deuterium, halogen, $C_{1-3}$ alkyl, $C_{1-3}$ deuterated alkyl or $C_{1-3}$ halogenated alkyl;
Preferably, $R_9$ or $R_{10}$ are each independently selected from methyl.

In another embodiment of the present invention, the general formula (VII) compound is as follows:

$R_{12}$ is selected from C1-3 alkyl;
Preferably, $R^{12}$ is selected from methyl.

In another embodiment of the present invention, the general formula (VII) compound is as follows:
Ring D is selected from

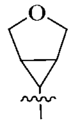 、 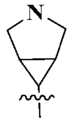 、 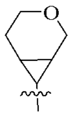 、 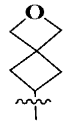 、 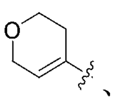 、 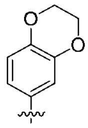 、 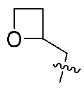 、

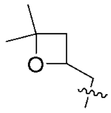 、 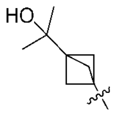 、 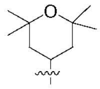 or 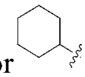 .

In another embodiment of the present invention, the general formula (VII) compound is as follows:

$R^{12}$ is selected from hydrogen, deuterium, halogen, amino, nitro, hydroxyl, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl, which are optionally further substituted by one or more substituents selected from halogen, amino, nitro, hydroxyl, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl.

Preferably, $R^{12}$ is selected from $C_{1-3}$ alkyl or $C_{3-6}$ cycloalkyl;

Further preferably, $R^{12}$ is selected from methyl or cyclopropyl.

In a further preferred embodiment of the present invention, the compound of formula (VII) is further shown in formula (VII-1):

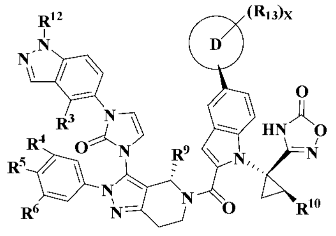

(VII-1) .

An object of the present invention is to provide a compound represented by general formula (XII), a prodrug thereof, a stereoisomer thereof or a pharmaceutically acceptable salt thereof:

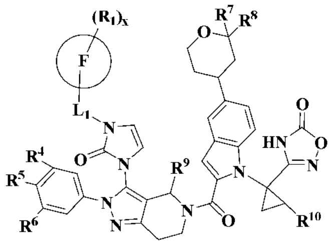

(XII)

$L_1$ is selected from a bond, C alkenylene, C alkynylene, $-(CH_2)_{n4}-$, $-(CH_2)_{n5}NR_{b5}$, wherein the C alkenylene, C alkynylene, $-(CH_2)_{n4}-$ or $-(CH_2)_{n5}NR_{b5}$, which are optionally further substituted by one or more substituents selected from halogen, amino, nitro, hydroxyl, cyano, oxo, C alkyl, C deuterated alkyl, C haloalkyl, C hydroxyalkyl, C alkoxy, C haloalkoxy, C alkenyl, C alkynyl, $C_{3-8}$ cycloalkyl, 3-8 membered heterocyclyl, $C_{6-10}$ aryl or 5-10 membered heteroaryl;

Ring F is selected from $C_{3-12}$ cycloalkyl, 3-14 membered heterocyclyl, $C_{6-14}$ aryl or 5-14 membered heteroaryl;

$R^1$ is independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $Cs_{_10}$ aryl, 5-10 membered heteroaryl, $-NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$ , $-(CH_2)_{n1}O(CH_2)_{n2}R_{a5}$, $-(CH_2)_{n1}C(O)NR_{a6}R_{b6}$ , $-(CH_2)_{n1}P(O)R_{a3}R_{b3}$,

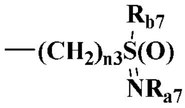,

$-(CH_2)_{n1}NR_{a8}C(O)R_{b8}$, the amino, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl are optionally further substituted by one or more substituents selected from halogen, amino, nitro, hydroxyl, cyano, oxo, $-NS(O)R_{a1}R_{b1}$, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heteroaryl, $C_{6-10}$ aryl or 5-10 membered heteroaryl;

Alternatively,

$R^1$ and the connected C atoms are linked to form $C_{3-10}$ cycloalkyl, 3-10 membered heterocyclyl, $C_{6-12}$ aryl or 5-12 membered heteroaryl;

$R^4$, $R^5$ or $R^6$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, which are optionally further substituted by one or more substituents selected from Halogen, amino, nitro, hydroxyl, cyano, oxo, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R^7$, $R^8$, $R^9$ or $R^{10}$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, which are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R_{a1}$, $R_{b1}$, $R_{a2}$, $R_{b2}$, $R_{a3}$, $R_{b3}$, $R_{a4}$, $R_{b4}$, $R_{a5}$, $R_{a6}$, $R_{b6}$, $R_{a7}$, $R_{b7}$, $R_{a8}$ or $R_{b8}$ is each independently selected from hydrogen, deuterium, halogen, amino, nitro, hydroxyl, cyano, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, the amino, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl are optionally further substituted by one or more substituents selected from halogen, amino, nitro, hydroxyl, cyano, oxo, $-NS(O)R_{a1}R_{b1}$, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

n1~n5 are integers of 0~5.

In a further preferred embodiment of the present invention, the general formula (XII) compound is as follows: Ring F is selected from $C_{3-12}$ cycloalkyl, 3-12 membered heterocyclyl, $C_{6-14}$ aryl or 5-14 membered heteroaryl.

In a further preferred embodiment of the present invention, general formula (XII) compound is as follows:

Preferably, ring F is selected from $C_{3-10}$ cycloalkyl, 5-14 membered heterocyclyl, $C_{6-10}$ aryl or 5-10 membered heteroaryl;

Further preferably, ring F is selected from $C_{3-6}$ bridged cycloalkyl, 8-14-membered tricyclic fused heterocyclic containing 1-5 heteroatoms, 3-8-membered bridged heterocyclic containing 1-3 heteroatoms, fused heterocyclic containing 1-3 heteroatoms, bridged heterocyclic containing 1-3 heteroatoms, $C_{6-10}$ aryl or 5-10-membered fused heteroaryl containing 1-3 heteroatoms;

More preferably, ring F is selected from phenyl, pyridyl, pyrimidinyl, thiazolyl, pyridazinyl, oxazolyl, morpholinyl,

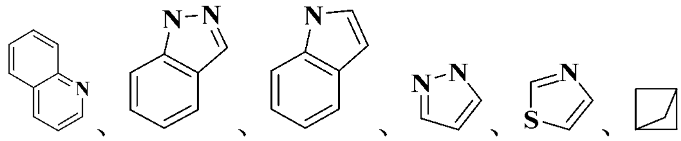
、、、、、、

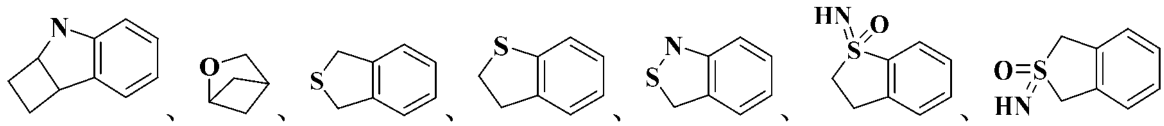
、、、、、、、

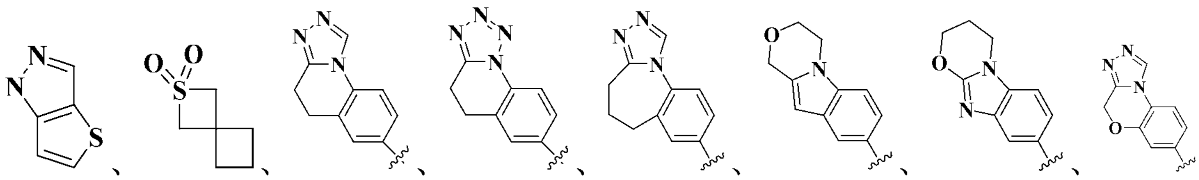
、、、、、、、

or 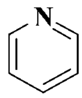.

In a further preferred embodiment of the present invention, general formula (XII) compound is as follows: Ring F is selected from phenyl, pyridyl, pyrimidinyl, thiazolyl, pyridazinyl, oxazolyl, morpholinyl,

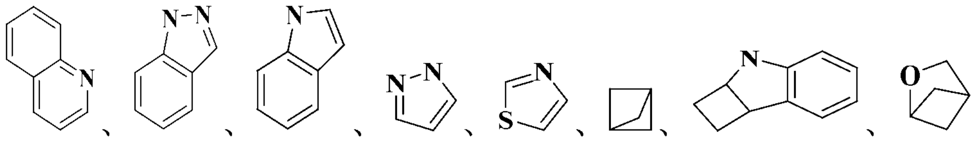
、、、、、、、、

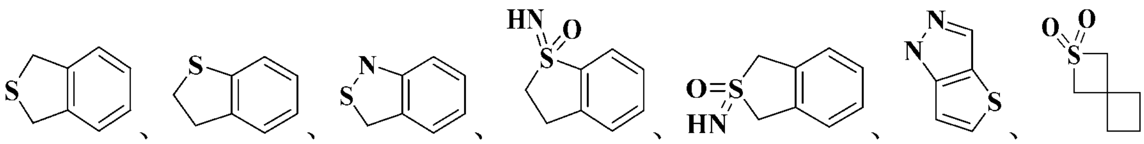
、、、、、、、

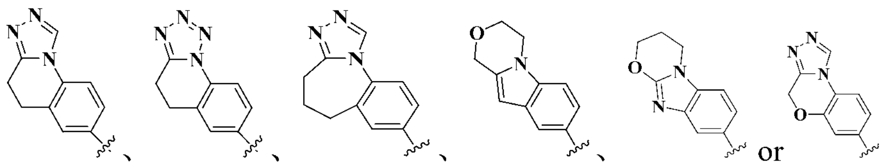
、、、、 or .

In a further preferred embodiment of the present invention, general formula (XII) compound is as follows:

Ring F is selected from $C_{3-10}$ cycloalkyl, 3-10 membered heterocyclic, $C_{6-10}$ aryl or 5-10 membered heteroaryl;
Preferably, ring F is selected from $C_{3-6}$ bridged cycloalkyl, 3-8 membered bridged heterocyclic containing 1-3 heteroatoms, fused heterocyclic containing 1-3 heteroatoms, bridged heterocyclic containing 1-3 heteroatoms, $C_{6-10}$ aryl or 5-10 membered fused heteroaryl containing 1-3 heteroatoms;
More preferably, ring F is selected from phenyl, pyridyl, pyrimidinyl, thiazolyl, pyridazinyl, oxazolyl, morpholinyl,

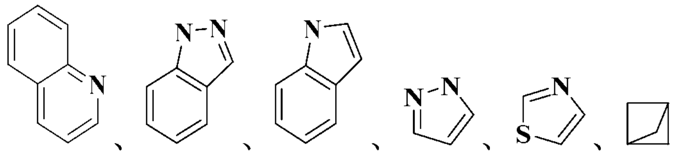
、、、、、、

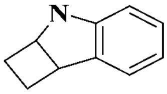、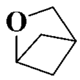、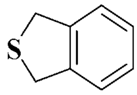、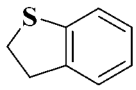、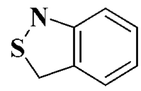、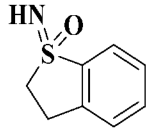 or 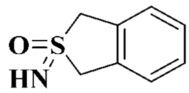.

In a further preferred embodiment of the present invention, general formula (XII) compound is as follows: Ring F is selected from phenyl, pyridyl, pyrimidinyl, thiazolyl, pyridazinyl, oxazolyl, morpholinyl,

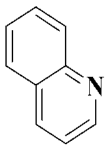、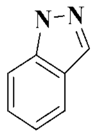、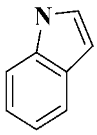、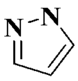、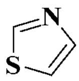、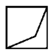、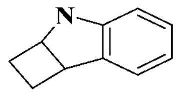、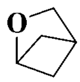、

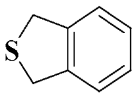、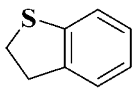、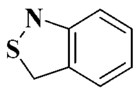、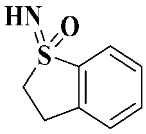、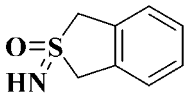、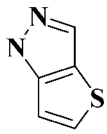 or 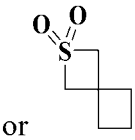.

In a further preferred embodiment of the present invention, general formula (XII) compound is as follows: Ring F is selected from phenyl, pyridyl, pyrimidinyl, thiazolyl, pyridazinyl, oxazolyl, morpholinyl,

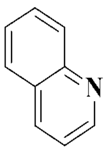、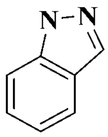、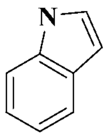、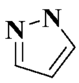、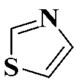、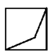、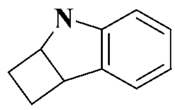、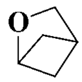、

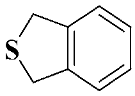、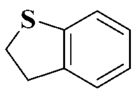、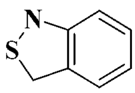、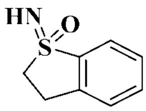、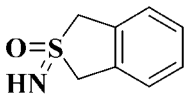、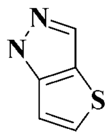、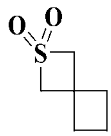、

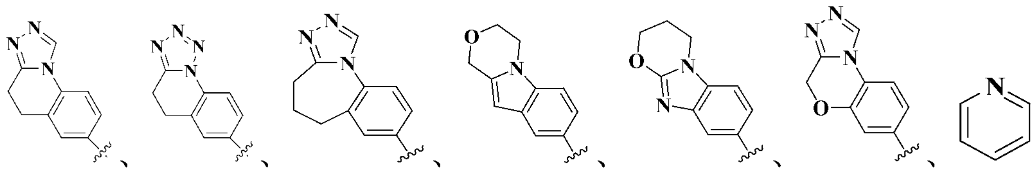 or

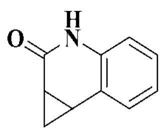.

In a further preferred embodiment of the present invention, general formula (XII) compound is as follows:

$R^1$ is independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl, 5-10 membered heteroaryl, $-NS(O)R_{a1}R_{b1}$, $-(CH_2)_{b1}S(O)NR_{a2}R_{b2}$, $-P(O)R_{a3}R_{b3}$, $-NR_{a5}S(O)NR_{a4}R_{b4}$ , $-(CH_2)_{n1}O(CH_2)_{n2}R_{a5}$ , $-(CH_2)_{n1}C(O)NR_{a6}R_{b6}$ , $-(CH_2)_{n1}P(O)R_{a3}R_{b3}$,

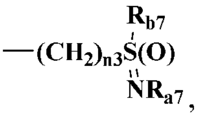

$-(CH_2)_{n1}NR_{a8}C(O)R_{b8}$, or $-(CH_2)_{n1}S(O)_2R_{b5}$, the amino, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl are optionally further substituted by one or more substituents selected from halogen, amino, nitro, hydroxyl, cyano, oxo, $-NS(O)R_{a1}R_{b1}$, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heteroaryl, $C_{6-10}$ aryl or 5-10 membered heteroaryl;

Preferably, $R^1$ is independently selected from hydrogen, deuterium, halogen, amino, nitro, hydroxyl, cyano, oxo, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl, 5-8 membered heteroaryl, $-NS(O)R_{a1}R_{b1}$, $-(CH_2)_{b1}S(O)NR_{a2}R_{b2}$, $-P(O)R_{a3}R_{b3}$, $-NR_{a5}S(O)NR_{a4}R_{b4}$, $-(CH_2)_{n1}O(CH_2)_{n2}R_{a5}$, $-(CH_2)_{n1}C(O)NR_{a6}R_{b6}$, $-(CH_2)_{n1}P(O)R_{a3}R_{b3}$,

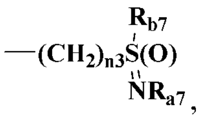

$-(CH_2)_{n1}NR_{a8}C(O)R_{b8}$, $-(CH_2)_{n1}S(O)_2R_{b5}$, the amino, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl, 5-8 membered heteroaryl are optionally further substituted by one or more substituents selected from halogen, amino, nitro, hydroxyl, cyano, oxo, $-NS(O)R_{a1}R_{b1}$, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heteroaryl, $C_{6-10}$ aryl or 5-8 membered heteroaryl;

Further preferably, $R^1$ is independently selected from amino, oxo, $C_{1-3}$ alkyl, $-NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$, $-(CH_2)_{n1}O(CH_2)_{n2}R_{a5}$, $-(CH_2)_{n1}C(O)NR_{a6}R_{b6}$, $-(CH_2)_{n1}P(O)R_{a3}R_{b3}$,

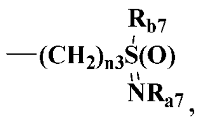

$-(CH_2)_{n1}NR_{a8}C(O)R_{b8}$, or $-(CH_2)_{n1}S(O)_2R_{b5}$, the amino and $C_{1-3}$ alkyl are optionally further substituted by cyano and $C_{1-3}$ alkyl.

In a further preferred embodiment of the present invention, general formula (XII) compound is as follows:

$R^1$ is independently selected from hydrogen, deuterium, halogen, amino, nitro, hydroxyl, cyano, oxo, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl, 5-8 membered heteroaryl, $-NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$, $-(CH_2)_{n1}O(CH_2)_{n2}R_{a5}$, $-(CH2)_{n1}C(O)NR_{a6}R_{b6}$, $-(CH_2)_{n1}P(O)R_{a3}R_{b3}$,

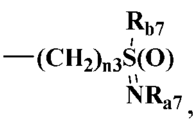

$-(CH_2)_{n1}NR_{a8}C(O)R_{b8}$, the amino, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl, 5-8 membered

heteroaryl are optionally further substituted by one or more substituents selected from halogen, amino, nitro, hydroxyl, cyano, oxo, $-NS(O)R_{a1}R_{b1}$, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heteroaryl, $C_{6-10}$ aryl or 5-8 membered heteroaryl;
Preferably, $R^1$ is independently selected from amino, oxo, $C_{1-3}$ alkyl, $-NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$, $-(CH_2)_{n1}O(CH_2)_{n2}R_{a5}$, $-(CH_2)_{n1}C(O)NR_{a6}R_{b6}$, $-(CH_2)_{n1}P(O)R_{a3}R_{b3}$,

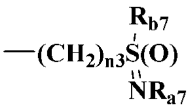,

$-(CH_2)_{n1}NR_{a8}C(O)R_{b8}$, the amino and $C_{1-3}$ alkyl are optionally further substituted by cyano and $C_{1-3}$ alkyl.

In a further preferred embodiment of the present invention, general formula (XII) compound is as follows:

$R^1$ and the connected C atoms are linked to form $C_{3-6}$ cycloalkyl, 3-8 membered heterocyclic, $C_{6-10}$ aryl or 5-10 membered heteroaryl;
Preferably, $R^1$ and the connected C atoms are further linked to form $C_{3-4}$ cycloalkyl.

In a further preferred embodiment of the present invention, general formula (XII) compound is as follows:

$R^4$, $R^5$ or $R^6$ is each independently selected from hydrogen, deuterium, halogen, amino, $C_{1-3}$ alkyl, $C_{1-3}$ deuterated alkyl or $C_{1-3}$ haloalkyl;
Preferably, $R^4$, $R^5$ or $R^6$ is each independently selected from fluoro or methyl.

In a further preferred embodiment of the present invention, general formula (XII) compound is as follows:

$R^7$, $R^8$, $R^9$ or $R^{10}$ is each independently selected from hydrogen, deuterium, halogen, $C_{1-3}$ alkyl, $C_{1-3}$ deuterated alkyl or $C_{1-3}$ halogenated alkyl;
Preferably, $R^7$, $R^8$, $R^9$ or $R^{10}$ is each independently selected from methyl.

In a further preferred embodiment of the present invention, general formula (XII) compound is as follows:

$R_{a1}$, $R_{b1}$, $R_{a2}$, $R_{b2}$, $R_{a3}$, $R_{b3}$, $R_{a4}$, $R_{b4}$, $R_{a5}$, $R_{b5}$, $R_{a6}$, $R_{b6}$, $R_{a7}$, $R_{b7}$, $R_{a8}$ or $R_{b8}$ is each independently selected from hydrogen, deuterium, halogen, amino, nitro, hydroxyl, cyano, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, the amino, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl are optionally further substituted by one or more substituents selected from halogen, amino, nitro, hydroxyl, cyano, oxo, $-NS(O)R_{a1}R_{b1}$, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;
Preferably, $R_{a1}$, $R_{b1}$, $R_{a2}$, $R_{b2}$, $R_{a3}$, $R_{b3}$, $R_{a4}$, $R_{b4}$, $R_{a5}$, $R_{b5}$, $R_{a6}$, $R_{b6}$, $R_{a7}$, $R_{b7}$, $R_{a8}$ or $R_{b8}$ is each independently selected from hydrogen, deuterium, halogen, amino, $C_{1-3}$ alkyl, $C_{1-3}$ deuterated alkyl, $C_{1-3}$ haloalkyl, 3-8 membered heterocyclic containing 1-3 atoms selected from N, O or S,
$C_{6-10}$ aryl or 5-8 membered heteroaryl containing 1-3 atoms selected from N, O or S;
Further preferably, $R_{a1}$, $R_{b1}$, $R_{a2}$, $R_{b2}$, $R_{a3}$, $R_{b3}$, $R_{a4}$, $R_{b4}$, $R_{a5}$, $R_{b5}$, $R_{a6}$, $R_{b6}$, $R_{a7}$, $R_{b7}$, $R_{a8}$ or $R_{b8}$ is each independently selected from hydrogen, deuterium, halogen, phenyl, or methyl.

In a further preferred embodiment of the present invention, general formula (XII) compound is as follows:

$R_{a1}$, $R_{b1}$, $R_{a2}$, $R_{b2}$, $R_{a3}$, $R_{b3}$, $R_{a4}$, $R_{b4}$, $R_{a5}$, $R_{a6}$, $R_{b6}$, $R_{a7}$, $R_{b7}$, $R_{a8}$ or $R_{b8}$ is each independently selected from hydrogen, deuterium, halogen, amino, $C_{1-3}$ alkyl, $C_{1-3}$ deuterated alkyl, $C_{1-3}$ haloalkyl;
Preferably, $R_{a1}$, $R_{b1}$, $R_{a2}$, $R_{b2}$, $R_{a3}$, $R_{b3}$, $R_{a4}$, $R_{b4}$, $R_{a5}$, $R_{a6}$, $R_{b6}$, $R_{a7}$, $R_{b7}$, $R_{a8}$ or $R_{b8}$ is each independently selected

from hydrogen, deuterium, halogen, and methyl.

In a further preferred embodiment of the present invention, the compound of general formula (XII) is further shown as general formula (XII-1):

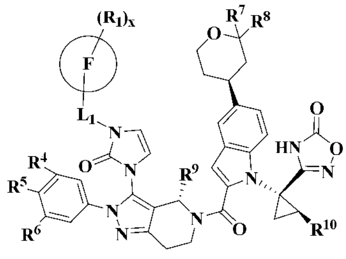

(XII-1)

.

An object of the present invention is to provide a compound represented by general formula (XVII), a prodrug thereof, a stereoisomer thereof or a pharmaceutically acceptable salt thereof:

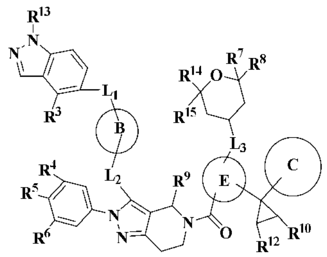

(XVII)

wherein,

$R^3$ or $R^{13}$ is each independently selected from hydrogen, deuterium, halogen, amino, nitro, hydroxyl, cyano, oxo, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl, the amino, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl, which are optionally further substituted by one or more substituents selected from deuterium, halogen, amino, nitro, hydroxyl, cyano, oxo, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

$L_1$, $L_2$ or $L_3$ is each independently selected from a bond, $C_{2-4}$ alkenylene, $C_{2-4}$ alkynylene or $-(CH_2)_{n1}-$;

Ring B is selected from $C_{3-12}$ cycloalkyl, 3-12 membered heterocyclyl, $C_{6-14}$ aryl or 5-14 membered heteroaryl, which are optionally further substituted with one or more substituents selected from deuterium, halogen, amino, nitro, hydroxyl, cyano, oxo, thiol, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy or $C_{1-6}$ deuterated haloalkoxy;

$R^4$, $R^5$ or $R^6$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, the amino, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$

deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R^7$, $R^8$, $R^9$, $R^{10}$, $R^{12}$, $R^{14}$ or $R^{15}$ is each independently selected from hydrogen, deuterium, halogen, amino, nitro, hydroxyl, cyano, oxo, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, $_{3-8}$ membered heterocyclyl, $C_{6-10}$ aryl, 5-10 membered heteroaryl, $-(CH_2)_{m1}OR_{c1}$, the amino, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl, 5-10 membered heteroaryl or $-(CH_2)_{m1}OR_{c1}$, are optionally further substituted by one or more substituents selected from deuterium, halogen, amino, nitro, hydroxyl, cyano, oxo, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

$R_{c1}$ is selected from hydrogen, deuterium, halogen, amino, nitro, hydroxyl, cyano, oxo, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl, 5-10 membered heteroaryl, the amino, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, $_{3-8}$ membered heterocyclic, $C_{6-10}$ aryl, 5-8 membered heteroaryl are optionally further substituted by one or more substituents selected from deuterium, halogen, amino, nitro, hydroxyl, cyano, oxo, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

Alternatively, $R^7$, $R^8$, $R^9$, $R^{10}$, $R^{12}$, $R^{14}$ or $R^{15}$ is linked to the atoms connected thereto to form $C_{3-10}$ cycloalkyl, 3-10 membered heterocyclyl, $C_{6-12}$ aryl or 5-12 membered heteroaryl;

Alternatively,

$R^7$ and $R^8$, $R^{14}$ and $R^{15}$, $R^{10}$ and $R^{12}$ are linked to the atoms connected thereto to form $C_{3-10}$ cycloalkyl, 3-10 membered heterocyclic, $C_{6-12}$ aryl or 5-12 membered heteroaryl, which are optionally further substituted by one or more substituents selected from deuterium, halogen, amino, nitro, hydroxyl, cyano, oxo, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

Ring E is selected from $C_{3-14}$ cycloalkyl, 3-14 membered heterocyclyl, $C_{6-14}$ aryl or 5-14 membered heteroaryl;

Alternatively, ring E is

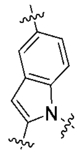

;

Ring C is selected from $C_{3-12}$ cycloalkyl, 3-12 membered heterocyclyl, $C_{6-14}$ aryl or 5-14 membered heteroaryl, which are optionally further substituted by one or more substituents selected from halogen, amino, nitro, hydroxyl, cyano, oxo, thiol, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy or $C_{1-6}$ deuterated haloalkoxy;

n1, n2 or m1 is each independently selected from 0, 1, 2, 3, 4 or 5.

In a further preferred embodiment of the present invention, the general formula (XVII) compound is as follows:

$R^3$ or $R^{13}$ is each independently selected from hydrogen, deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl or 5-8

membered heteroaryl, the amino, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl or 5-10 membered heteroaryl, which are optionally further substituted by one or more substituents selected from deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl;

Preferably, $R^3$ or $R^{13}$ is each independently selected from hydrogen, deuterium, fluorine, chlorine, bromine, methyl or cyclopropyl, the methyl or cyclopropyl is optionally further substituted by one or more substituents of halogen or $C_{1-3}$ alkyl.

In a further preferred embodiment of the present invention, the general formula (XVII) compound is as follows:

Ring B is selected from $C_{3-8}$ cycloalkyl, 3-8 membered heterocyclic containing 1-3 atoms selected from N, O or S, $C_{6-10}$ aryl or 5-10 membered heteroaryl containing 1-3 atoms selected from N, O or S, which are optionally further substituted by one or more substituents selected from halogen, amino, oxo, thiol, thio or $C_{1-3}$ alkyl;

Preferably, ring B is selected from $C_{3-6}$ cycloalkyl, 3-6 membered heterocyclic containing 1-3 N, O or S atoms or 5-8 membered heteroaryl containing 1-3 N, O or S atoms, which are optionally further substituted by one or more substituents selected from halogen, amino, oxo, thio or $C_{1-3}$ alkyl;

Further preferably, ring B is selected from

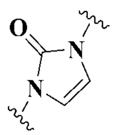 、 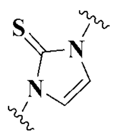 or 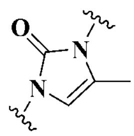 ,

which are optionally further substituted by one or more substituents selected from halogen, amino, oxo, thio or $C_{1-3}$ alkyl.

In a further preferred embodiment of the present invention, the general formula (XVII) compound is as follows:

$R^4$, $R^5$ or $R^6$ is each independently selected from hydrogen, deuterium, halogen, amino, nitro, hydroxyl, cyano, oxo, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl, the amino, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl or 5-8 membered heteroaryl are optionally further substituted by one or more substituents selected from halogen, amino, nitro, hydroxyl, cyano, oxo, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl or 5-8 membered heteroaryl;

Preferably, $R^4$, $R^5$ or $R^6$ is each independently selected from fluorine, cyano, cyclopropyl or methyl, the cyclopropyl or methyl is optionally further substituted by one or more substituents selected from halogen, amino, oxo, thio or $C_{1-3}$ alkyl.

In a further preferred embodiment of the present invention, the general formula (XVII) compound is as follows:

$R^7$, $R^8$, $R^9$, $R^{10}$, $R^{12}$, $R^{14}$ or $R^{15}$ is each independently selected from hydrogen, deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, $_{3-6}$ membered heterocyclyl, $C_{6-10}$ aryl, 5-8 membered heteroaryl, $-(CH_2)_{m1}OR_{c1}$, the amino, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl,

3-6 membered heterocyclic, $C_{6-10}$ aryl, 5-8 membered heteroaryl or $-(CH_2)_{m1}OR_{c1}$, are optionally further substituted by one or more substituents selected from deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl;

Preferably, $R^7$, $R^8$, $R^9$, $R^{10}$, $R^{12}$, $R^{14}$ or $R^{15}$ is each independently selected from hydrogen, deuterium, halogen, cyano, $C_{2-4}$ alkynyl, $C_{3-6}$ cycloalkyl, $C_{1-3}$ alkyl, $C_{1-3}$ deuterated alkyl, $C_{1-3}$ haloalkyl or $-(CH_2)_{m1}OR_{c1}$, the $C_{2-4}$ alkynyl, $C_{3-6}$ cycloalkyl, $C_{1-3}$ alkyl, $C_{1-3}$ deuterated alkyl, $C_{1-3}$ haloalkyl or $-(CH_2)_{m1}OR_{c1}$ are optionally further substituted by one or more substituents selected from deuterium, deuterated methyl, methyl, cyclopropyl or oxo;

Further preferably, $R^7$, $R^8$, $R^9$, $R^{10}$, $R^{12}$, $R^{14}$ or $R^{15}$ is each independently selected from cyano, methyl, fluorine, cyclopropyl, ethynyl or $-(CH_2)_{m1}OR_{c1}$, the methyl, cyclopropyl, ethynyl or $-(CH_2)_{m1}OR_{c1}$ are optionally further substituted by one or more substituents of deuterium, deuterated methyl, methyl, cyclopropyl or oxo.

In a further preferred embodiment of the present invention, the general formula (XVII) compound is as follows:

$R_{c1}$ is selected from hydrogen, deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclyl, $C_{6-10}$ aryl, 5-8 membered heteroaryl, the amino, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl, 5-8 membered heteroaryl are optionally further substituted by one or more substituents selected from deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl;

Preferably, $R_{c1}$ is selected from fluorine, methyl, cyano, difluoromethyl, trifluoromethyl, ethynyl, methylthio, amino, cyclopropyl or oxetane, the methyl, difluoromethyl, trifluoromethyl, ethynyl, methylthio, amino, cyclopropyl or oxetane are optionally further substituted by one or more substituents of halogen, methoxy or deuterated methyl.

In a further preferred embodiment of the present invention, the general formula (XVII) compound is as follows:

$R^7$, $R^8$, $R^9$, $R^{10}$, $R^{12}$, $R^{14}$ or $R^{15}$ is each linked to the atoms connected thereto to form $C_{3-6}$ cycloalkyl, 3-6 membered heterocyclic containing 1-3 atoms selected from N, O or S, $C_{6-10}$ aryl or 5-8 membered heteroaryl containing 1-3 atoms selected from N, O or S;

Preferably, $R^7$, $R^8$, $R^9$, $R^{10}$, $R^{12}$, $R^{14}$ or $R^{15}$ is each linked to the atoms connected thereto to form cyclopropyl.

In a further preferred embodiment of the present invention, the general formula (XVII) compound is as follows:

$R^{10}$ and $R^{12}$ are linked to the atoms connected thereto to form $C_{3-10}$ cycloalkyl, 3-10 membered heterocyclyl, $C_{6-12}$ aryl, or 5-12 membered heteroaryl;

$R^{10}$ and $R^{12}$ are linked to the atoms connected thereto to form $C_{3-6}$ cycloalkyl, 3-6 membered heterocyclic containing 1-3 atoms selected from N, O or S, $C_{6-10}$ aryl, or 5-8 membered heteroaryl containing 1-3 atoms selected from N, O or S;

Preferably, $R^{10}$ and $R^{12}$ are linked to the atoms connected thereto to form

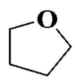.

In a further preferred embodiment of the present invention, the general formula (XVII) compound is as follows:

$R^7$ and $R^8$, $R^{14}$ and $R^{15}$, $R^{10}$ and $R^{12}$ are linked to the atoms connected thereto to form $C_{3-6}$ cycloalkyl, 3-6 membered heterocyclic, $C_{6-10}$ aryl or 5-8 membered heteroaryl, which are optionally further substituted by one or more substituents selected from deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl,

$C_{2-4}$ alkynyl, $C_{3-6}$ cycloalkyl, 3-6 membered heterocyclic, $C_{6-10}$ aryl or 5-8 membered heteroaryl;
Preferably, $R^7$ and $R^8$, $R^{14}$ and $R^{15}$, $R^{10}$ and $R^{12}$ are linked to the atoms connected thereto to form cyclopropyl,

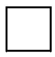 、 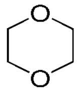 、 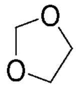 、 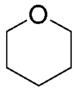 、 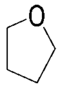 、 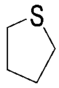 or 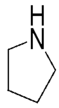 ,

which are optionally further substituted by one or more substituents selected from deuterium, fluorine, chlorine, bromine, amino, nitro, hydroxy, cyano, oxo, thio, methyl or ethyl.

In a further preferred embodiment of the present invention, the general formula (XVII) compound is as follows:

Ring E is selected from $C_{3-8}$ cycloalkyl, 3-10 membered heterocyclic containing 1-3 atoms selected from N, O or S, $C_{6-10}$ aryl or 5-10 membered heteroaryl containing 1-3 atoms selected from N, O or S;

Preferably, ring E is selected from

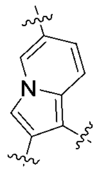 、 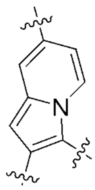 、 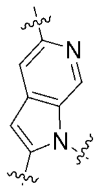 or 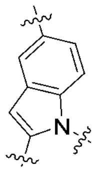 .

In a further preferred embodiment of the present invention, the general formula (XVII) compound is as follows: Ring E is

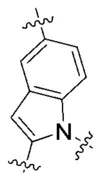 .

In a further preferred embodiment of the present invention, the general formula (XVII) compound is as follows:

Ring C is selected from $C_{3-8}$ cycloalkyl, 3-8 membered heterocyclic containing 1-3 atoms selected from N, O or S, $C_{6-10}$ aryl or 5-8 membered heteroaryl containing 1-3 atoms selected from N, O or S, which are optionally further substituted by one or more substituents selected from halogen, amino, nitro, hydroxyl, cyano, oxo, thiol, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy or $C_{1-3}$ deuterated haloalkoxy;
Preferably, ring C is selected from $C_{3-8}$ cycloalkyl, 3-8 membered heterocyclic containing 1-3 atoms selected from N, O or S, or 5-8 membered heteroaryl containing 1-3 atoms selected from N, O or S, which are optionally further substituted by one or more substituents selected from hydroxyl, thioxo, oxo or $C_{1-3}$ haloalkoxy;
Further preferably, ring C is selected from

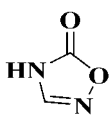 、 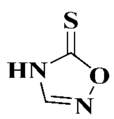 、 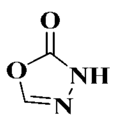 、 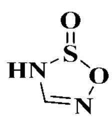 、

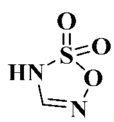 or 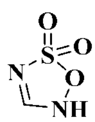 ,

which are optionally further substituted by one or more substituents selected from hydroxyl, thio or oxo.

In a further preferred embodiment of the present invention, the general formula (XVII) compound is as follows:

$R^{13}$ is selected from $C_{1-6}$ deuterated alkyl, $C_{1-6}$ deuterated halogenated alkyl, $C_{1-6}$ deuterated hydroxyalkyl, $C_{1-6}$ deuterated alkoxy, $C_{1-6}$ deuterated halogenated alkoxy, $C_{3-8}$ cycloalkyl, 3-8 membered heterocyclic group, $C_{6-10}$ aryl or 5-10 membered heteroaryl, the $C_{1-6}$ deuterated alkyl, $C_{1-6}$ deuterated halogenated alkyl, $C_{1-6}$ deuterated hydroxyalkyl, $C_{1-6}$ deuterated alkoxy, $C_{1-6}$ deuterated halogenated alkoxy, $C_{3-8}$ cycloalkyl, 3-8 membered heterocyclic, $C_{6-10}$ aryl or 5-10 membered heteroaryl are optionally further substituted by one or more substituents selected from deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic group, $C_{6-10}$ aryl or 5-10 membered heteroaryl;

Preferably, $R^{13}$ is selected from $C_{1-3}$ deuterated alkyl, $C_{1-3}$ deuterated halogenated alkyl, $C_{1-3}$ deuterated hydroxyalkyl, $C_{1-3}$ deuterated alkoxy, $C_{1-3}$ deuterated halogenated alkoxy, $C_{3-6}$ cycloalkyl, 3-6 membered heterocyclic group, $C_{6-10}$ aryl or 5-8 membered heteroaryl, the $C_{1-3}$ deuterated alkyl, $C_{1-3}$ deuterated halogenated alkyl, $C_{1-3}$ deuterated hydroxyalkyl, $C_{1-3}$ deuterated alkoxy, $C_{1-3}$ deuterated halogenated alkoxy, $C_{3-6}$ cycloalkyl, 3-6 membered heterocyclic $C_{6-10}$ aryl or 5-8 membered heteroaryl are optionally further substituted by one or more substituents selected from deuterium, halogen, amino, nitro, hydroxyl, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclic group, $C_{6-10}$ aryl or 5-8 membered heteroaryl;

Further preferably, $R_{13}$ is selected from $-CD_3$ or cyclopropyl, $-CD_3$ or cyclopropyl is optionally further substituted by one or more substituents selected from deuterium, halogen, or $C_{1-3}$ alkyl;

More preferably, $R_{13}$ is selected from cyclopropyl, optionally further substituted by one or more substituents selected from deuterium, halogen, or $C_{1-3}$ alkyl.

In a further preferred embodiment of the present invention, the compound of general formula (XVII) is further represented by general formula (XVII-1):

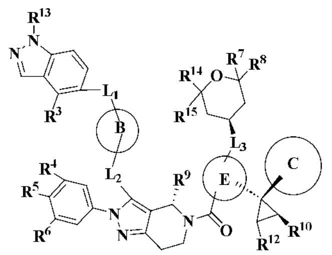

(XVII-1)

The present invention further provides a compound represented by the general formula (IN-A), (IN-B) or (IN-C), a prodrug thereof, a stereoisomer thereof or a pharmaceutically acceptable salt thereof:

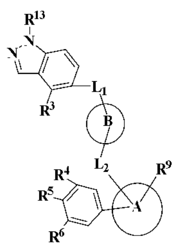

(IN-A)

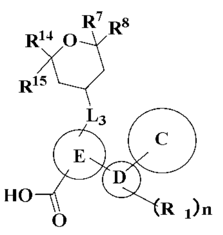

(IN-B)

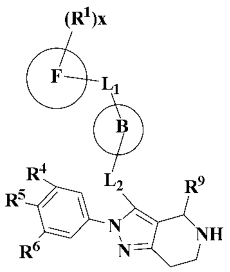

(IN-C)

wherein, in the general formula (IN-A), $R^{13}$, ring A, ring B, $L_1$, $L_2$, $R^4$, $R^5$, $R^6$, and $R^9$ are as defined in the general formula (XIX);

In the general formula (IN-B), ring C, ring D, ring E, $L_3$, $R^1$, $R^7$, $R^8$, $R^{14}$, $R^{15}$, and n are as defined in the general formula (XIX);

In the general formula (IN-C), ring F, ring B, $L_1$, $L_2$, $R^1$, $R^4$, $R^5$, $R^6$, $R^9$, and x are as defined in the general formula (XVI).

In a further preferred embodiment of the present invention, the general formula (IN-A) compound is as follows:

$R^{13}$ is selected from $C_{3-8}$ cycloalkyl, $C_{6-10}$ aryl, 5-10 membered heteroaryl; the $C_{3-8}$ cycloalkyl, $C_{6-10}$ aryl, 5-10 membered heteroaryl are optionally further substituted by one or more substituents selected from deuterium, halogen, amino, nitro, hydroxyl, cyano, oxo, $C_{1-6}$ alkyl.

In a further preferred embodiment of the present invention, the general formula (IN-B) compound is as follows: ring D is selected from $C_{3-8}$ bicyclic cycloalkyl, 3-8 membered bicyclic heterocyclic, or 5-10 membered bicyclic heteroaryl containing 1-3 atoms selected from N, O or S; preferably, ring D is selected from $C_{3-6}$ bicyclic cycloalkyl, 3-8 membered bicyclic heterocyclic containing 1-3 atoms selected from N, O or S, or 5-8 membered bicyclic heteroaryl containing 1-3 atoms selected from N, O or S; preferably, Ring D is selected from

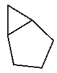 or 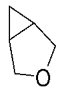.

In a further preferred embodiment of the present invention, the general formula (IN-C) compound is as follows: ring F is selected from 8-14 membered tricyclic fused heterocyclic containing 1-5 heteroatoms.

The present invention further provides a method of the general formulas and compounds as described above, comprising the following steps,

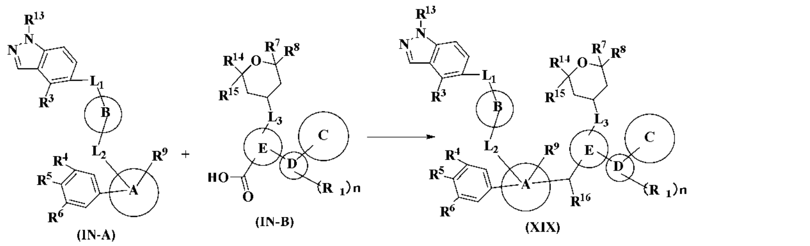

(IN-A) (IN-B) (XIX)

wherein: Ring A, ring B, ring C, ring D, ring E, $L_1$, $L_2$, $L_3$, $R^1$, $R^4$, $R^5$, $R^6$, $R^7$, $R^3$, $R^9$, $R^{13}$, $R^{14}$, $R^{15}$, $R^{16}$ are as defined in general formula (XIX);

General formula (IN-A) and general formula (IN-B) react under the action of a condensing agent and a base to obtain general formula (XIX);

The condensing agent is selected from EDC, DIC, DCC, TBTU, HATU5, HBTU, HCTU, DEPBT, PyBOP or PyAOP;

The base is selected from DIPEA, DIEA, Et3N, NMP, DBU or DABCO.

In a further preferred embodiment of the present invention, in the preparation method, the condensing agent is selected from HATU; and the base is selected from DIPEA.

In a further preferred embodiment of the present invention, the preparation method is carried out under a solvent condition, and the reaction solvent is selected from N,N-dimethylformamide, N,N-dimethylacetamide, tetrahydrofuran, acetonitrile, or dichloromethane; and preferably N,N-dimethylformamide.

The present invention further provides a method of preparing the above-mentioned general formulas and compounds, comprising the following steps:

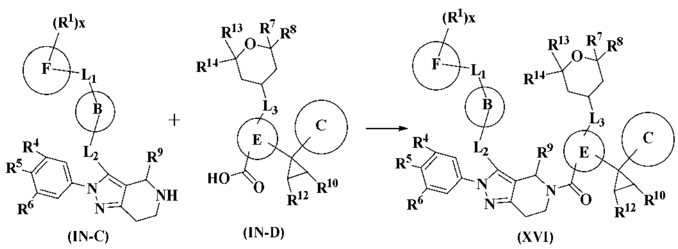

(IN-C) (IN-D) (XVI)

wherein: Ring F, ring B, ring C, ring E, $L_1$, $L_2$, $L_3$, $R^1$, $R^4$, $R^5$, $R^6$, $R^7$, $R^8$, $R^9$,$R^{10}$, $R^{12}$, $R^{13}$, $R^{14}$, and x are as defined in general formula (XVI);

General formula (IN-C) and general formula (IN-D) react under the action of a condensing agent and a base to obtain general formula (XVI);

The condensing agent is selected from EDC, DIC, DCC, TBTU, HATU, HBTU, HCTU, DEPBT, PyBOP or PyAOP; preferably HATU;

The base is selected from DIPEA, DIEA, Et3N, NMP, DBU or DABCO.

In a further preferred embodiment of the present invention, the condensing agent in the preparation method is selected from HATU; the base is selected from DIEA.

In a further preferred embodiment of the present invention, the preparation method is carried out under a solvent condition, the reaction solvent is selected from N,N-dimethylformamide, N,N-dimethylacetamide, tetrahydrofuran, acetonitrile, or dichloromethane; and preferably N,N-dimethylformamide.

The present invention further relates to a pharmaceutical composition, comprising a therapeutically effective dose of any compound of the general formula shown, a prodrug thereof, a stereoisomer thereof or a pharmaceutically acceptable salt thereof, and one or more pharmaceutically acceptable carriers, diluents, or excipients.

In certain embodiments of the present invention, the weight percentage of the compound, the prodrug thereof, the stereoisomer thereof or the pharmaceutically acceptable salt thereof in the pharmaceutical composition, calculated as free base, is 0.1% to 95%, preferably 5-70%, for example 70%, 65%, 60%, 55%, 50%, 45%, 40%, 35%, 30%, 25%, 20%, 15%, 10% or 5%.

In certain embodiments of the present invention, the pharmaceutical composition is selected from tablets, capsules, liquid preparations, or injections, preferably, further comprising a filler, optionally a disintegrant, or further comprising one or more of a glidant or a lubricant.

In certain embodiments of the present invention, the pharmaceutical composition is a rapid release preparation or a sustained release preparation.

In certain embodiments of the present invention, the pharmaceutical composition, calculated as a free base, has a unit dose of 1-1000 mg, preferably 1-500 mg, or preferably 1 mg, 2 mg, 3 mg, 5 mg, 10 mg, 20 mg, 40 mg, 50 mg, 60 mg, 80 mg, 100 mg, 200 mg, 300 mg, 400 mg, or 500 mg.

In certain embodiments of the present invention, the compound, the prodrug thereof, the stereoisomer thereof or the pharmaceutically acceptable salt thereof, can be administered by any convenient method, for example, by oral, parenteral, buccal, sublingual, nasal, rectal, intrathecal, or transdermal administration, and the pharmaceutical composition adjusted accordingly.

In certain embodiments of the present invention, the compound, the prodrug thereof, the stereoisomer thereof or the pharmaceutically acceptable salt thereof, can be formulated into a liquid or solid preparation, for example, a syrup, suspension, emulsion, tablet, capsule, powder, granule, or lozenge.

The present invention further relates to the use of any of the compounds of the general formulas shown, the prodrugs thereof, the stereoisomers thereof or the pharmaceutically acceptable salts thereof, or an application of the pharmaceutical compositions in the preparation of GLP-1 receptor agonist drugs.

The present invention further relates to the use of the compounds represented by the general formulas, the prodrugs thereof, the stereoisomers thereof or the pharmaceutically acceptable salts thereof, or the pharmaceutical compositions thereof in the preparation of a medicament for treating metabolic-related diseases, wherein the disease is selected from diabetes, obesity or non-alcoholic fatty liver disease-related diseases or other related diseases caused by diabetes, obesity or non-alcoholic fatty liver disease.

The present invention further relates to a method for preparing a medicament for treating metabolic diseases and related diseases using the compounds represented by the general formulas, the prodrugs thereof, the stereoisomers thereof or the pharmaceutically acceptable salts thereof, or the pharmaceutical compositions thereof.

The present invention also relates to a method for treating, preventing, and/or treating metabolic-related diseases, which comprises administering to a patient a therapeutically effective dose of a compound represented by the general formula, a prodrug thereof, a stereoisomer thereof or a pharmaceutically acceptable salt thereof, or a pharmaceutical composition thereof.

The present invention also provides a method for treating a disease state using the compound or pharmaceutical composition of the present invention; the disease state comprises but is not limited to a condition associated with a GLP-1 receptor modulator.

The present invention also relates to a method for treating a metabolic disease-related disease in a mammal, comprising administering to the mammal a therapeutically effective amount of a compound of the present invention or a pharmaceutically acceptable salt, ester, prodrug, solvate, hydrate or derivative thereof.

## Detailed Description of the Invention

Unless stated otherwise, the terms used in the description and claims have the following meanings.

The term "alkyl" refers to a saturated aliphatic hydrocarbon group, which is a straight or branched group containing 1 to 20 carbon atoms, preferably an alkyl group containing 1 to 8 carbon atoms, more preferably an alkyl group containing 1 to 6 carbon atoms, and most preferably an alkyl group containing 1 to 3 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, etc. More preferred are lower alkyl groups containing 1 to 6 carbon atoms, non-limiting examples of which include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. The alkyl group may be substituted or unsubstituted, and when substituted, the substituent may be substituted at any available point of attachment. The substituent is preferably one or more groups independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkyloxy, heterocycloalkyloxy, cycloalkylthio, heterocycloalkylthio, oxo, carboxyl or carboxylate groups. Methyl, ethyl, isopropyl, tert-butyl, haloalkyl, deuterated alkyl, alkoxy-substituted alkyl and hydroxy-substituted alkyl are preferred in the present invention.

The term "alkylene" refers to an alkyl group of which one hydrogen atom is further substituted. For example: "methylene" refers to $-CH_2-$, "ethylene" refers to $-(CH_2)_2-$, "propylene" means $-(CH_2)_3-$, "butylene" refers to $-(CH_2)_4-$, etc.

The term "alkenyl" refers to an alkyl group as defined above consisting of at least two carbon atoms and at least one carbon-carbon double bond, such as vinyl, 1-propenyl, 2-propenyl, 1-, 2- or 3-butenyl, etc. The alkenyl group may be substituted or unsubstituted, and when substituted, the substituent is preferably one or more groups independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkyloxy, heterocycloalkyloxy, cycloalkylthio, and heterocycloalkylthio.

The term "alkenylene" refers to an alkenyl group of which one hydrogen atom is further substituted. For example, "vinylene" refers to $-CH_2=CH_2-$, "propenylene" refers to $-CH_2-=CH_2-CH_2-$, etc. Alkenylene may be substituted or unsubstituted, and when substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkyloxy, heterocycloalkyloxy, cycloalkylthio, heterocycloalkylthio. The term "cycloalkyl" refers to a saturated or partially unsaturated monocyclic or polycyclic cyclic hydrocarbon substituent, and the cycloalkyl ring contains 3 to 20 carbon atoms, preferably 3 to 12 carbon atoms, more preferably 3 to 8 carbon atoms, and further preferably 3 to 6 carbon atoms. Non-limiting examples of monocyclic cycloalkyl groups include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc.; polycyclic cycloalkyl groups include spiro, fused, and bridged cycloalkyl groups, preferably cyclopropyl, cyclobutyl, cyclopentyl, cyclohexyl, and cycloheptyl.

The term "spirocycloalkyl" refers to a 5- to 20-membered polycyclic group where the monocycles share a carbon atom (called spiro atom), which may contain one or more double bonds, but none of the rings have a fully conjugated $\pi$ electron system. Preferably, the spirocycloalkyl is 6- to 14-membered, more preferably 7- to 10-membered. According to the number of shared spiro atoms between the rings, the spirocycloalkyl is classified into mono-spirocycloalkyl, bi-spirocycloalkyl or poly-spirocycloalkyl, and is preferably mono-spirocycloalkyl and bi-spirocycloalkyl. More preferably, the spirocycloalkyl is a 4-membered/4-membered, 4-membered/5-membered, 4-membered/6-membered, 5-membered/5-membered, or 5-membered/6-membered mono-spirocycloalkyl. Non-limiting examples of the spirocycloalkyl include:

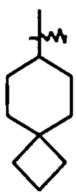 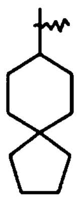 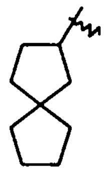 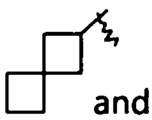 and 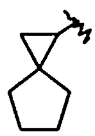 .

also included is the spirocycloalkyl in which a mono-spirocycloalkyl and a heterocycloalkyl share a spiro atom, and non-limiting examples include:

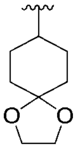 and 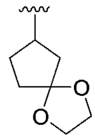.

The term "fused cycloalkyl" refers to a 5- to 20-membered all-carbon polycyclic group where each of the rings in the system shares an adjacent pair of carbon atoms with the other rings in the system, wherein one or more rings may contain one or more double bonds, but none of the rings have a fully conjugated π electron system. Preferably, the fused cycloalkyl is 6- to 14-membered, more preferably 7- to 10-membered. According to the number of constituent rings, the fused cycloalkyl may be classified into bicyclic, tricyclic, tetracyclic or polycyclic fused cycloalkyl, preferably bicyclic or tricyclic, more preferably 5-membered/5-membered or 5-membered/6-membered bicyclic alkyl. Non-limiting examples of the fused cycloalkyl include:

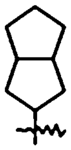 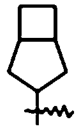 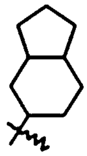 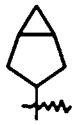 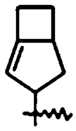 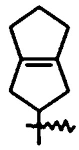 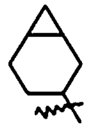 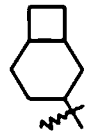 and 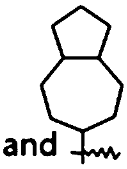.

The term "bridged cycloalkyl" refers to a 5- to 20-membered all-carbon polycyclic group where any two rings share two carbon atoms that are not directly connected, which may contain one or more double bonds, but none of the rings have a fully conjugated π electron system. Preferably, the bridged cycloalkyl is 6- to 14-membered, more preferably 7- to 10-membered. According to the number of constituent rings, the bridged cycloalkyl may be classified into bicyclic, tricyclic, tetracyclic or polycyclic bridged cycloalkyl, preferably bicyclic, tricyclic, or tetracyclic, more preferably bicyclic or tricyclic. Non-limiting examples of the bridged cycloalkyl include:

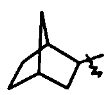 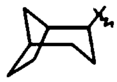 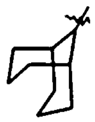 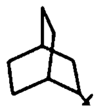 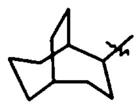

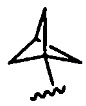 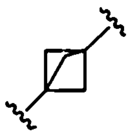 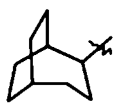 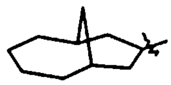 and 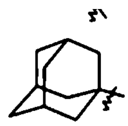

The cycloalkyl ring may be fused to an aryl, heteroaryl or heterocycloalkyl ring, wherein the ring to which the parent structure is attached is cycloalkyl, non-limiting examples of which include indanyl, tetrahydronaphthyl, benzocycloheptanyl, etc. The cycloalkyl may be optionally substituted or unsubstituted, and when substituted, the substituent is preferably one or more groups independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxy or carboxylate group.

The term "heterocyclyl" refers to a saturated or partially unsaturated monocyclic or polycyclic cyclic hydrocarbon substituent containing 3 to 20 ring atoms, wherein one or more of the ring atoms are a heteroatom selected from nitrogen, oxygen, or $S(O)_m$ (where m is an integer of 0 to 2), excluding the ring portion of -O-O-, -O-S-, or -S-S-, and the remaining ring atoms are carbon. Preferably, the heterocyclyl contains 3 to 12 ring atoms, of which 1 to 4 are heteroatoms; more preferably, the heterocyclyl contains 3 to 10 ring atoms; further preferably, the heterocyclyl contains 3 to 8 ring atoms. Non-limiting examples of monocyclic heterocyclyl include pyrrolidinyl, azetidinyl, oxetanyl, oxanyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl, etc.; preferably pyrrolidinyl, azetidinyl, oxetanyl, tetrahydrofuranyl, pyrazolidinyl, morpholinyl,

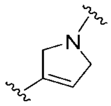
,

piperazinyl and pyranyl; more preferably pyrrolidinyl, azetidinyl, oxetanyl, oxanyl, piperidinyl,

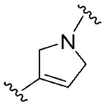
,

piperazinyl and pyranyl. Polycyclic heterocyclyl includes spiro, fused and bridged heterocyclyl; the involved spiro, fused and bridged heterocyclyl are optionally connected to other groups through a single bond, or are further connected to other cycloalkyl, heterocyclyl, aryl and heteroaryl through any two or more atoms on the ring.

The term "spiroheterocyclyl" refers to a 5- to 20-membered polycyclic heterocyclic group where the monocyclic rings share one atom (called spiro atom), wherein one or more of the ring atoms are a heteroatom selected from nitrogen, oxygen or $S(O)_m$ (where m is an integer of 0 to 2), and the remaining ring atoms are carbon. The spiroheterocyclyl may contain one or more double bonds, but none of the rings has a fully conjugated $\pi$-electron system. Preferably, the spiroheterocyclyl is 6 to 14-membered, more preferably 7 to 10-membered. According to the number of shared spiro atoms between the rings, the spiroheterocyclyl is classified into mono-spiroheterocyclyl, bi-spiroheterocyclyl or poly-spiroheterocyclyl, with mono-spiroheterocyclyl and bi-spiroheterocyclyl being preferred. More preferably, the spiroheterocyclyl is 4-membered/4-membered, 4-membered/5-membered, 4-membered/6-membered, 5-membered/5-membered, or 5-membered/6-membered mono-spiroheterocyclyl. Non-limiting examples of spiroheterocyclyl include:

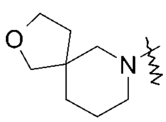
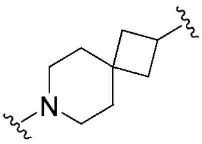
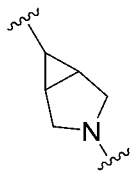
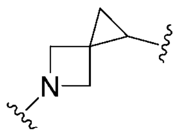
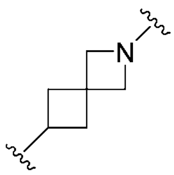
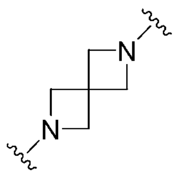
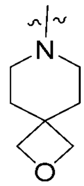

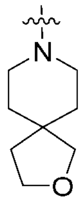
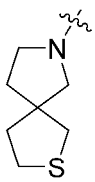
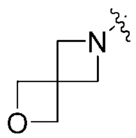
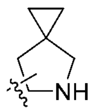
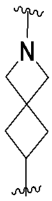
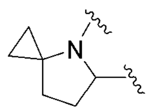
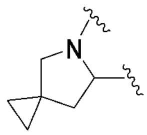
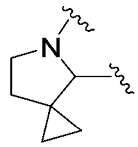
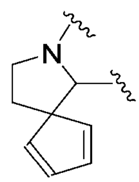
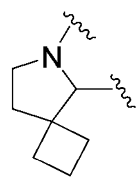

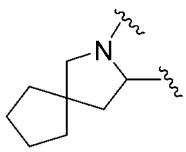
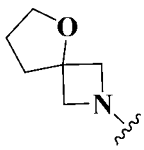
and
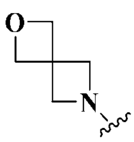
.

The term "fused heterocyclyl" refers to a 5- to 20-membered polycyclic heterocyclic group, where each of the rings in the system shares an adjacent pair of atoms with other rings in the system, and one or more rings may contain one or more double bonds, but none of the rings has a fully conjugated $\pi$ electron system, wherein one or more of the ring atoms are a heteroatom selected from nitrogen, oxygen, or $S(O)_m$ (where m is an integer of 0 to 2), and the remaining ring atoms are carbon. Preferably, the fused heterocyclyl is 6- to 14-membered, more preferably 7- to 10-membered. According to the number of constituent rings, the fused heterocyclyl may be classified into bicyclic, tricyclic, tetracyclic or polycyclic fused heterocyclyl, preferably bicyclic or tricyclic, more preferably 5-membered/5-membered or 5-membered/6-membered bicyclic fused heterocyclyl. Non-limiting examples of the fused heterocyclyl include:

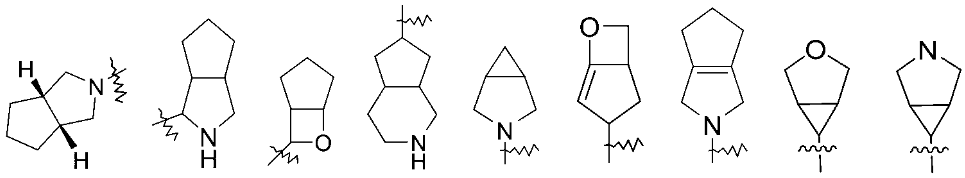

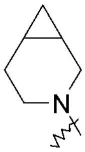 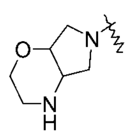 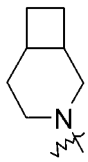 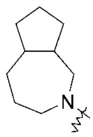 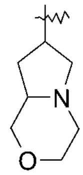 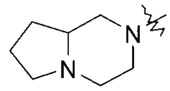 and 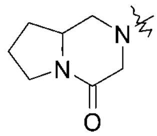 .

The term "bridged heterocyclyl" refers to a 5- to 14-membered polycyclic heterocyclic group, where any two rings share two atoms that are not directly connected, which may contain one or more double bonds, but none of the rings has a fully conjugated π electron system, wherein one or more of the ring atoms are a heteroatom selected from nitrogen, oxygen, or $S(O)_m$ (where m is an integer of 0 to 2), and the remaining ring atoms are carbon. Preferably, the bridged heterocyclyl is 6 to 14-membered, more preferably 7 to 10-membered. According to the number of constituent rings, the bridged heterocyclyl may be classified into bicyclic, tricyclic, tetracyclic or polycyclic bridged heterocyclyl, preferably bicyclic, tricyclic, or tetracyclic, more preferably bicyclic or tricyclic. Non-limiting examples of the bridged heterocyclyl include:

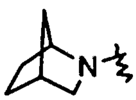 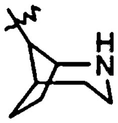 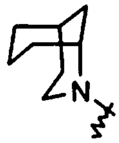 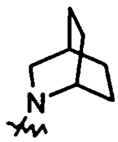

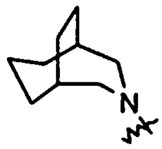 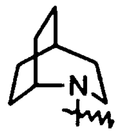 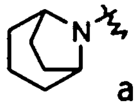 and 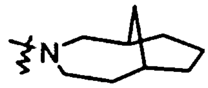 .

The heterocyclyl ring may be fused to an aryl, heteroaryl or cycloalkyl ring, wherein the ring connected to the parent structure is heterocyclyl, non-limiting examples of which include:

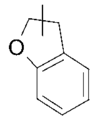 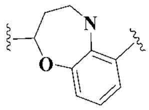 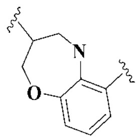 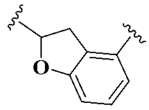 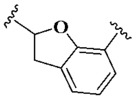 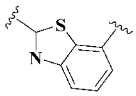 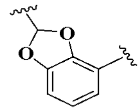 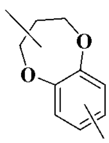

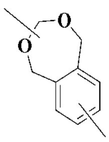 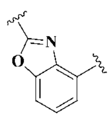 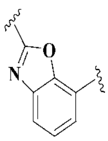 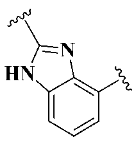 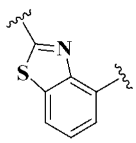 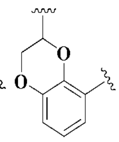 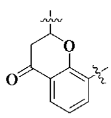 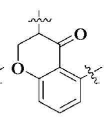 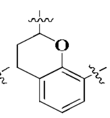 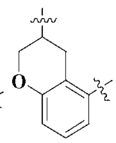

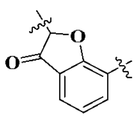 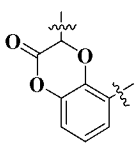 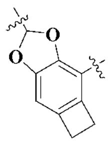 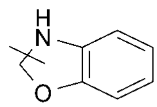 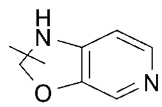 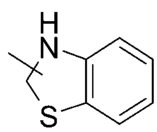

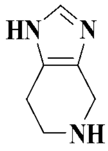 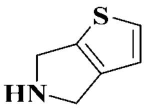 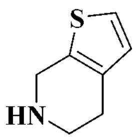 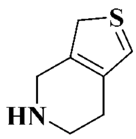 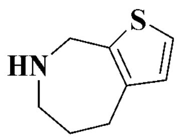 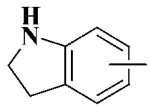 .

The heterocyclyl may be optionally substituted or unsubstituted, and when substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl or carboxylate group.

The term "aryl" refers to a 6- to 14-membered all-carbon monocyclic or fused polycyclic (i.e., rings sharing adjacent pairs of carbon atoms) group having a conjugated $\pi$ electron system. The aryl is preferably 6- to 10-membered, more preferably 6- to 8-membered, such as phenyl and naphthyl, more preferably phenyl. The aryl ring may be fused to a heteroaryl, heterocyclyl or cycloalkyl ring, wherein the ring connected to the parent structure is aryl ring, non-limiting examples of which include:

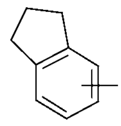 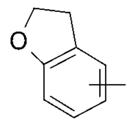 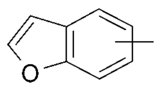 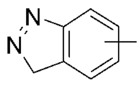 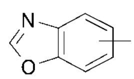 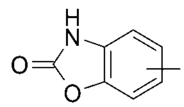 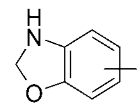

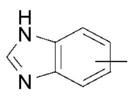 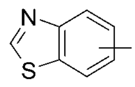 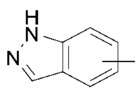 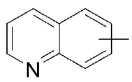 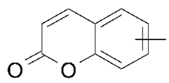 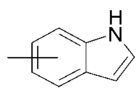 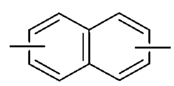

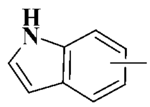 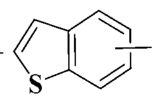 .

The aryl may be substituted or unsubstituted, and when substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or carboxylate group.

The term "heteroaryl" refers to a heteroaromatic system containing 1 to 4 heteroatoms and 5 to 14 ring atoms, wherein the heteroatom is selected from oxygen, sulfur, and nitrogen. The heteroaryl is preferably 5- to 10-membered, more preferably 5- to 8-membered, most preferably 5-membered or 6-membered, such as imidazolyl, furyl, thienyl, thiazolyl, pyrazolyl, oxazolyl, pyrrolyl, triazolyl, tetrazolyl, pyridyl, pyrimidinyl, thiadiazolyl, pyrazinyl, etc., preferably triazolyl, thienyl, imidazolyl, pyrazolyl, pyrimidinyl or thiazolyl; more preferably triazolyl, pyrrolyl, thienyl, triazolyl, and pyrimidinyl. The heteroaryl ring may be fused to an aryl, heterocyclyl or cycloalkyl ring, wherein the ring connected to the parent structure is heteroaryl ring, non-limiting examples of which include:

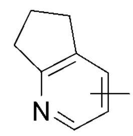 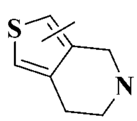 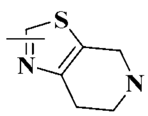 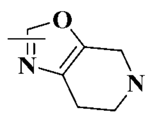 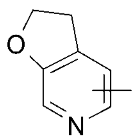 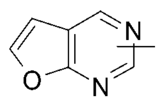 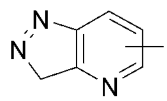

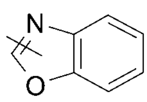 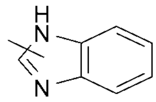 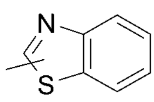 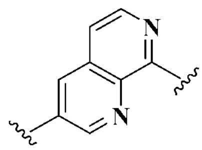 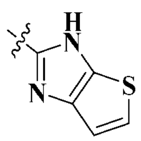 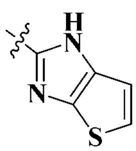 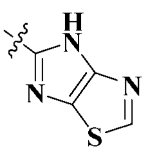

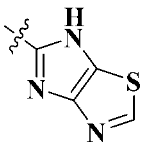 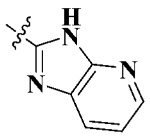 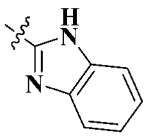 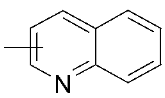 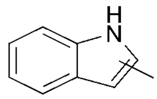 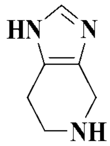 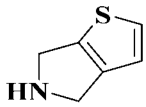 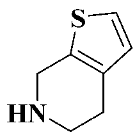

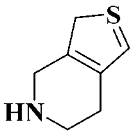 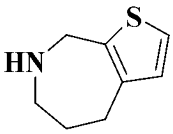 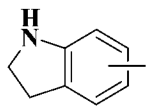 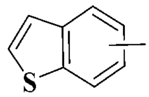 .

The hereroaryl may be substituted or unsubstituted, and when substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or carboxylate group.

The term "alkoxy" refers to -O-(alkyl) and -O-(non-substituted cycloalkyl), wherein alkyl is as defined above, preferably alkyl containing 1 to 8 carbon atoms, more preferably alkyl containing 1 to 6 carbon atoms, and most preferably alkyl containing 1 to 3 carbon atoms. Non-limiting examples of the alkoxy include: methoxy, ethoxy, propoxy, butoxy, cyclopropoxy, cyclobutoxy, cyclopentyloxy, cyclohexyloxy. The alkoxy may optionally be substituted or unsubstituted, and when substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or carboxylate group.

"Haloalkyl" refers to an alkyl substituted with one or more halogens, wherein the alkyl is as defined above.

"Haloalkoxy" refers to an alkoxy substituted with one or more halogens, wherein the alkoxy is as defined above.

"Hydroxyalkyl" refers to an alkyl substituted with hydroxyl, wherein the alkyl is as defined above.

"Alkenyl" refers to olefinyl group, as known as alkene group, preferably containing an alkyl of 2 to 8 carbon atoms, more preferably an alkyl of 2 to 6 carbon atoms, and most preferably an alkyl of 2 to 3 carbon atoms. The alkenyl may be further substituted with other related groups, such as: alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or carboxylate group.

"Alkynyl" refers to (CH=C-), preferably containing an alkyl of 2 to 8 carbon atoms, more preferably an alkyl of 2 to 6 carbon atoms, and most preferably an alkyl of 2 to 3 carbon atoms. The alkynyl may be further substituted with other related groups, such as: alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or carboxylate group.

The term "alkynylene" refers to an alkynyl of which one hydrogen atom is further substituted. For example, "ethynylene" refers to -C≡C-, "propynylene" refers to - C≡C $-CH_2-$, etc. The alkynylene (containing three or more carbon atoms) may be substituted or unsubstituted, and when substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, and heterocycloalkylthio group.

"Fused ring group" refers to a polycyclic group formed by two or more carbon rings or heterocyclic rings with a common ring edge. The fused ring group includes fused ring alkyl, fused ring heteroaryl, fused ring aryl and fused ring heteroaryl. The fused ring alkyl refers to a polycyclic group formed by a cycloalkyl and a heterocyclic group, an aryl, or a heteroaryl with a common ring edge; the fused ring heterocyclic group refers to a polycyclic group formed by a heterocyclic group and a cycloalkyl, an aryl, or a heteroaryl with a common ring edge; the fused ring aryl refers to a polycyclic group formed by an aryl and a cycloalkyl, a heterocyclic group, or a heteroaryl with a common ring edge; the fused ring heteroaryl refers to a polycyclic group formed by a heteroaryl and a cycloalkyl, a heterocyclic group, or a hetero group with a common ring edge; for example:

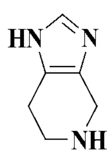 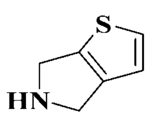 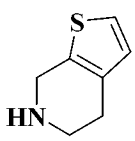 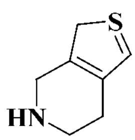 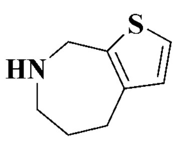 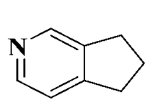 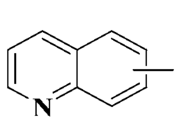 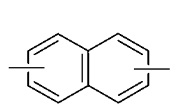

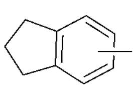 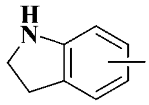 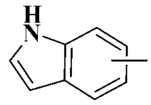 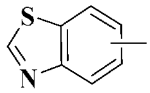 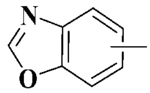 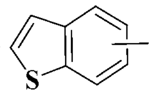 .

"Haloalkyl" refers to an alkyl group substituted with one or more halogens, wherein the alkyl group is as defined above.

"Haloalkoxy" refers to an alkoxy group substituted with one or more halogens, wherein the alkoxy group is as defined above.

"Hydroxyalkyl" refers to an alkyl group substituted with a hydroxyl group, wherein the alkyl group is as defined above.

"Hydroxy" refers to an-OH group.

"Halogen" refers to fluorine, chlorine, bromine, or iodine.

"Amino" refers to $-NH_2$.

"Cyano" refers to -CN.

"Nitro" refers to $-NO_2$.

"Carboxy" refers to -C(O)OH.

"THF" refers to tetrahydrofuran.

"EtOAc" refers to ethyl acetate.

"MeOH" refers to methanol.

"DMF" refers to N,N-dimethylformamide.

"DIPEA" refers to diisopropylethylamine.

"TFA" refers to trifluoroacetic acid.

"MeCN" refers to acetonitrile.

"DMA" refers to N,N-dimethylacetamide.

"$Et_2O$" refers to diethyl ether.

"DCE" refers to 1,2-dichloroethane.

"DIPEA" refers to N,N-diisopropylethylamine.

"NBS" refers to N-bromosuccinimide.

"NIS" refers to N-iodosuccinimide.

"Cbz-Cl" refers to benzyl chloroformate.

"$Pd_2(dba)_3$" refers to tris(dibenzylideneacetone)dipalladium.

"Dppf" refers to 1,1'-bis(diphenylphosphino)ferrocene.

"HATU" refers to 2-(7-oxybenzotriazole)-N,N,N',N'-tetramethyluronium hexafluorophosphate.

"KHMDS" refers to potassium bistrimethylsilylamide.

"LiHMDS" refers to lithium bistrimethylsilylamide.

"MeLi" refers to methyllithium.

"n-BuLi" refers to n-butyllithium.

"$NaBH(OAc)_3$" refers to sodium triacetoxyborohydride.

"X is selected from A, B, or C", "X is selected from A, B, and C", "X is A, B, or C", "X is A, B, and C", etc. are different terms expressing the same meaning, that is, X can be any one or more of A, B, and C.

The hydrogen atoms described in the present invention may all be replaced by their isotope deuterium, and any hydrogen atom in the example compounds involved in the present invention may also be replaced by a deuterium atom.

"Optional" or "optionally" means that the event or circumstance described subsequently may but need not occur, and the description includes the situations in which the event or circumstance occurs or does not occur. For example, "heterocyclic group optionally substituted with an alkyl" means that the alkyl may but do not need to be present, and the description includes cases in which the heterocyclic group is substituted with an alkyl and cases in which the heterocyclic group is not substituted with an alkyl.

"Substituted" means that one or more hydrogen atoms, preferably up to 5, more preferably 1 to 3 hydrogen atoms in a group are each independently substituted with a corresponding number of substituents. It goes without saying that the substituents are only in their possible chemical positions, and those skilled in the art can determine (either experimentally or theoretically) possible or impossible substitutions without undue effort. For example, an amino or hydroxyl with a free hydrogen may be unstable when bound to a carbon atom with an unsaturated (such as olefinic) bond.

"Pharmaceutical composition" means a mixture of one or more of the compound described herein or a physiologically/pharmaceutically acceptable salt or prodrug thereof with other chemical components, and other components such as physiologically/pharmaceutically acceptable carriers and excipients. The purpose of the pharmaceutical composition is to facilitate administration to living organisms and facilitate the absorption of the active ingredient to exert a biological activity.

"Pharmaceutically acceptable salt" refers to a salt of the compound of the present invention that is safe and effective when used in mammals and that has desirable biological activity.

**Detailed Description of Preferred Embodiments**

The present invention will be further described below with reference to examples, but these examples are not intended to limit the scope of the present invention.

Examples

The structure of the compound of the present invention was determined by nuclear magnetic resonance (NMR) or/and liquid chromatography-mass spectrometry (LC-MS). The NMR chemical shift (δ) is given in parts per million (ppm). The NMR was measured by a Bruker AVANCE-400 nuclear magnetic apparatus. The solvent for measurement was deuterated dimethyl sulfoxide (DMSO-$d_6$), deuterated methanol ($CD_3OD$) and deuterated chloroform ($CDCl_3$), and the internal standard was tetramethylsilane (TMS).
LC-MS determination was performed using an Agilent 1200 Infinity Series mass spectrometer. HPLC determination was performed using an Agilent 1200 DAD high pressure liquid chromatograph (Sunfire C18 150 x 4.6 mm column) and a Waters 2695-2996 high pressure liquid chromatograph (Gimini C18 150 x 4.6 mm column).
A Yantai Huanghai HSGF254 or Qingdao GF254 silica gel plate was used as the thin layer chromatography silica gel plate. The specification used for TLC was 0.15 mm to 0.20 mm, and the specification used for product separation and purification by thin layer chromatography was 0.4 mm to 0.5 mm. Yantai Huanghai silica gel 200- to 300-mesh silica gel was generally used as a carrier for column chromatography.
The starting materials in the examples of the present invention are known and commercially available, or can be synthesized using or according to methods known in the art.
Unless otherwise stated, all reactions of the present invention were carried out under continuous magnetic stirring in a dry nitrogen or argon atmosphere, the solvent was a dry solvent, and the reaction temperature was in degree Celsius.

## Intermediate A

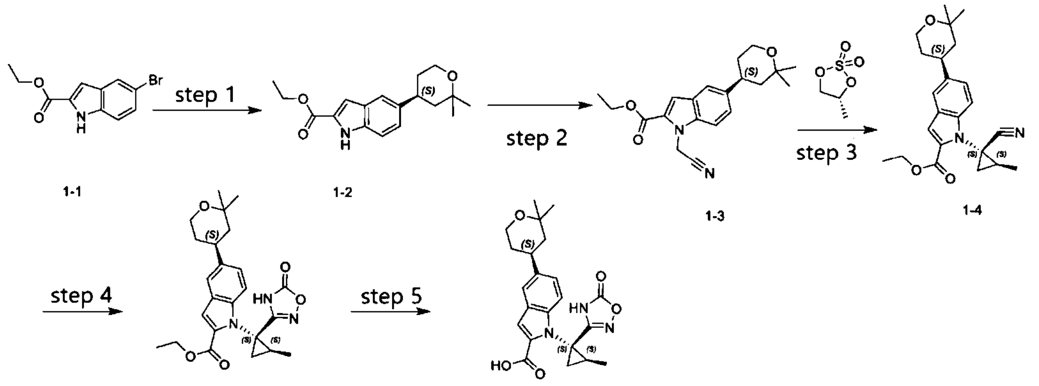

Intermediate A

Step 1: Zinc powder (4.88 g, 74.6 mmol) was suspended in DMF (20 mL), and the suspension was purged with nitrogen for protection. Chlorotrimethylsilane (1.01 g, 9.32 mmol) and 1,2-dibromoethane (1.75 g, 9.32 mmol) were added, and the resulting mixture was stirred at room temperature for 5 minutes. To the mixture was added dropwise a solution of 4-iodo-2,2-dimethyltetrahydropyran (13.43 g, 55.95 mmol) in DMF (25 mL), and the mixture was stirred at room temperature for 20 minutes. To the above solution were added palladium acetate (209 mg, 0.93 mmol) and 4-(N,N-dimethylamino) phenyl]di-tert-butylphosphine (494 mg, 1.86 mmol), ethyl 5-bromoindole-2-carboxylate (5.0 g, 18.65 mmol) were added, and nitrogen gas was introduced into the container. The mixture was stirred at an external temperature of 50°C for 1 hour, then the external temperature was cooled to 0°C, and 5 N hydrochloric acid (6 mL) was added for neutralization. 30% sodium chloride aqueous solution (50 mL) and ethyl acetate (300 mL) were added, and insoluble matter was removed with diatomaceous earth. The filtrate was extracted with ethyl acetate. The organic layer was washed with 30% sodium chloride aqueous solution, then dried over magnesium sulfate and passed through a filter, and the solvent was removed by evaporation under reduced pressure. The residue was purified by silica gel column chromatography (ethyl acetate:hexane = 1:1) to give the product, which was separated chirally to obtain the product compound (1.96 g, 35% yield).
MS m/z (ESI): 302.2[M+1].

Step 2: ethyl (S)-5-(2,2-dimethyltetrahydro-2H-pyran-4-yl)-1H-indole-2-carboxylate (1.96 g, 6.5 mmol) was dissolved in acetonitrile (30 mL) at room temperature, and potassium carbonate (2.70 g, 19.51 mmol) was added to the solution. At an external temperature of 10°C, 2-chloroacetonitrile (736 mg, 9.75 mmol) was added to the resulting solution, and the solution was stirred for 12 hours. Saturated saline (50 mL) and ethyl acetate (100 mL) were added to the reaction solution for extraction, and the aqueous layer was extracted for the second time using ethyl acetate (20 mL). The combined organic layer was washed with a saturated saline solution (50 mL), and was then concentrated under reduced pressure at an external temperature of 40°C to obtain the target product, which was used in the subsequent step without purification.

MS m/z (ESI): 341.2[M+1].

Step 3: Ethyl (S)-1-(cyanomethyl)-5-(2,2-dimethyltetrahydro-2H-pyran-4-yl)-1H-indole-2-carboxylate (2 g, 5.88 mmol) and (4R)-4-methyl-1,3,2-dioxathiolane 2,2-dioxide (2.43 g, 17.6 mmol) were dissolved in tetrahydrofuran (15 mL), and the mixture was cooled to 0°C. In a nitrogen atmosphere, a solution of potassium bis(trimethylsilyl)amide in THF (23.5 mL, 23.5 mmol, 1 M) was slowly added dropwise. The reaction mixture was stirred at 0°C for 2.5 hours, then formic acid (3 mL) was added, and extraction was performed using a mixture of ethyl acetate (40 mL). The organic layer was washed with water three times, with a sodium bicarbonate saturated aqueous solution twice, and with brine once, and then dried over sodium sulfate. After filtration, the filtrate was concentrated under reduced pressure, and the residue was purified by silica gel column chromatography (ethyl acetate:hexane=1:4) to obtain the target product (1.20 g, yield 54%).

MS m/z (ESI): 381.2[M+1].

Step 4: To a solution of ethyl 1-((1S,2S)-1-cyano-2-methylcyclopropyl)-5-((S)-2,2-dimethyltetrahydro-2H-pyran-4-yl)-1H-indole-2-carboxylate (1.20 g, 3.15 mmol) in dimethyl sulfoxide (DMSO) (10 mL) was added 50% hydroxylamine aqueous solution (2.08 g, 31.5 mmol), and the mixture was stirred at room temperature for 17 hours. Ethyl acetate (50 mL) was added, and the mixture was washed with water (50 mL) and brine (50 mL), and then dried over magnesium sulfate. After the mixture was filtered, the filtrate was concentrated under reduced pressure, and the resulting residue was dissolved in DMSO (10 mL). Then, carbonyldiimidazole (1.02 g, 6.31 mmol) and 1,8-diazabicycloundec-7-ene (1.20, 7.88 mmol) were added, and the resulting mixture was stirred at room temperature for 0.5 hours. Formic acid was added to the mixture, which was then purified by reverse phase chromatography (acetonitrile/water, 0.1% formic acid) to obtain the target product (0.96 g, yield 69%).

MS m/z (ESI): 440.2[M+1].

Step 5: To a solution of ethyl 5-((S)-2,2-dimethyltetrahydro-2H-pyran-4-yl)-1-((1S,2S)-2-methyl-1-(5-carbonyl-4,5-dihydro-1,2,4-oxadiazol-3-yl)cyclopropyl)-1H-indole-2-carboxylate (0.96 g, 2.18 mmol) in DMSO (8 mL) was added 2 M sodium hydroxide aqueous solution (3.28 mL, 6.55 mmol), and the mixture was stirred at room temperature for 1.5 hours. Formic acid was added to the mixture, which was then purified by reverse phase chromatography (acetonitrile/-water, 0.1% formic acid) to obtain the target product (0.65 g, yield 72%).

MS m/z (ESI): 412.2[M+1].

## Intermediate B

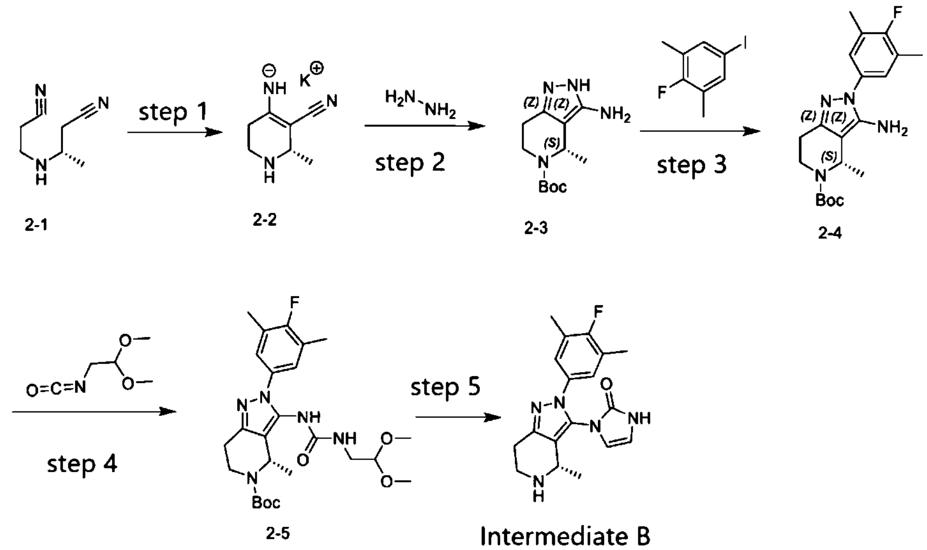


Step 1: To a solution of raw material 2-1 (2.4 g, 17.9 mmol) in tetrahydrofuran (THF) (20 mL) was added a 1 M solution of potassium tert-butoxide in THF (17.9 mL), and the mixture was stirred at room temperature for 1 hour. The reaction mixture was filtered, and washed with THF (50 mL), and the filtrate was dried under reduced pressure to obtain

intermediate 2-2 (2.7 g, yield 88%).

MS m/z (ESI): 138.1[M+1].

Step 2: To a solution of hydrazine hydrochloride (1.06 g, 15.4 mmol) and intermediate 2-2 (2.7 g, 15.4 mmol) in ethanol (30 mL) was added 2N hydrochloric acid (11.5 mL, 23.1 mmol), and the mixture was stirred at 50°C for 1 hour. After the reaction mixture was cooled to 0°C, 5 M sodium hydroxide aqueous solution (4.62 mL, 23.1 mmol) and di-tert-butyl dicarbonate (3.36 g, 15.4 mmol) were added, and the mixture was stirred at 0°C for 1 hour. Water was added to the reaction mixture, and extraction was performed using ethyl acetate. Then, the organic layer was washed with brine and dried over anhydrous magnesium sulfate. After filtration, the filtrate was concentrated under reduced pressure, and the residue was purified by silica gel column chromatography (dichloromethane:methanol = 20:1) to obtain intermediate 2-3 (2.5 g, yield 65%).

MS m/z (ESI): 253.1[M+1].

Step 3: Intermediate 2-3 (2.5 g, 9.91 mmol), 2-fluoro-5-iodo-1,3-dimethylbenzene (2.48 g, 9.91 mmol), and L-proline (342.23 mg, 2.97 mmol) were dissolved in dimethyl sulfoxide (30 mL). Under nitrogen gas protection, cuprous iodide (754.82 mg, 3.96 mmol) and potassium carbonate (1.10 g, 7.93 mmol) were added. The mixture was stirred and reacted at 120°C for 19 hours. The reaction was quenched by adding saturated saline solution (150 mL) to the mixture, and extraction was performed using ethyl acetate (50 mL × 3). The organic phases were combined, and were washed with saturated saline (50 mL × 5), dried over anhydrous sodium sulfate, and concentrated under reduced pressure successively. The residue was purified by flash silica gel chromatography (elution with petroleum ether:ethyl acetate=100:0 to 50:50) to obtain intermediate 2-4 (2.9 g, yield 78%).

MS m/z (ESI): 375.2[M+1].

Step 4: To a solution of intermediate 2-4 (2.9 g, 7.4 mmol) in pyridine (10 mL) was added 2-isocyanato-1,1-dimethoxyethane (2.03 g, 15.49 mmol), and the mixture was stirred at room temperature. After 3 hours, diethylamine (1.13 g, 15.49 mmol) was added, and the mixture was stirred at room temperature for 5 minutes. Then water (50 mL) was added, and the resulting mixture was stirred at room temperature for 20 minutes. The reaction mixture, which had become a suspension, was filtered. The resulting solid was washed with water (20 mL), and then dried under reduced pressure to obtain intermediate 2-5 (3.6 g, yield 92%).

MS m/z (ESI): 506.3[M+1].

Step 5: To (Intermediate 2-5 (3.6 g, 7.12 mmol) was added formic acid (40 mL), and the mixture was stirred at room temperature for 21 hours. The reaction mixture was concentrated under reduced pressure, toluene was added, and the solvent was removed by evaporation under reduced pressure. Dichloromethane (10 mL) was added to the residue to dissolve the residue. Then hydrogen chloride (4 M di-alkane solution, 10 mL) was added at room temperature, and stirring was performed at room temperature for 2 hours. The reaction mixture was concentrated under reduced pressure. Toluene was added, and the solvent was removed by evaporation under reduced pressure to obtain a crude product (3.6 g).

MS m/z (ESI): 342.2[M+1].

## Intermediates C and D

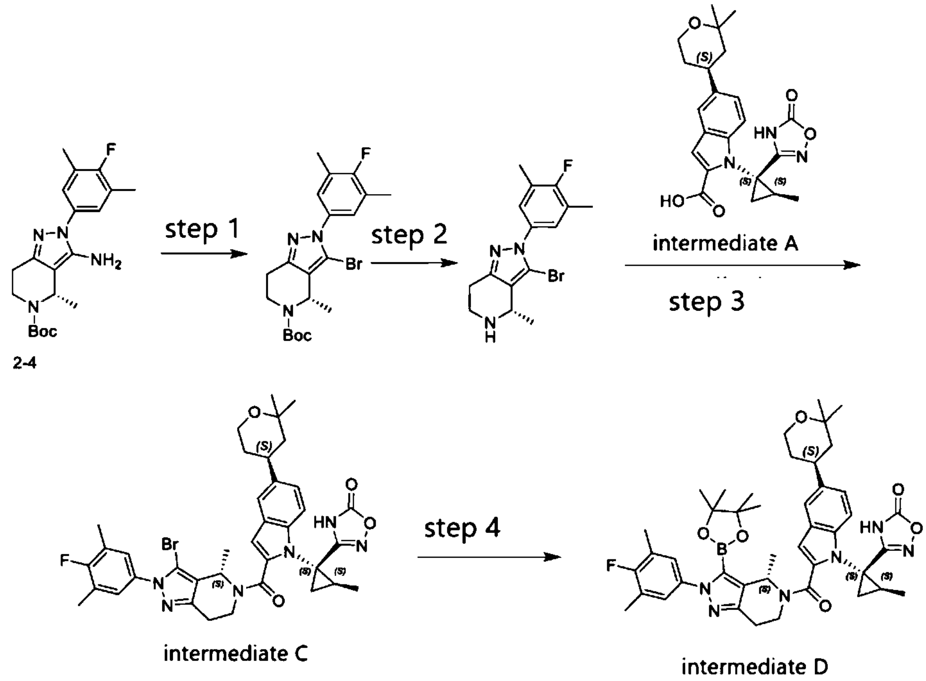


Step 1: Tert-butyl (S)-3-amino-2-(4-fluoro-3,5-dimethylphenyl)-4-methyl-2,4,6,7-tetrahydro-5H-pyrazolo[4,3-c]pyridine-5-carboxylate (2.5 g, 6.68 mmol) was dissolved in bromoform (8 mL), and isoamyl nitrite (2.07 g, 20.03 mmol) was added under nitrogen gas protection. The mixture was stirred at 60°C for 2 hours. The mixture was concentrated under reduced pressure, and saturated brine (30 mL) was added to quench the reaction. The reaction was extracted with ethyl acetate (50 mL), and was washed with saturated brine (50 mL × 2), dried over anhydrous sodium sulfate, and concentrated under reduced pressure successively. The residue was purified by flash silica gel chromatography (elution with petroleum ether:ethyl acetate = 100:0 to 50:50) to give the target product tert-butyl (S)-3-bromo-2-(4-fluoro-3,5-dimethylphenyl)-4-methyl-2,4,6,7-tetrahydro-5H-pyrazolo[4,3-c]pyridine-5-carboxylate (2.1 g, yield 72%).

MS m/z (ESI): 438.1, 440.1[M+1].

Step 2: Tert-butyl (S)-3-bromo-2-(4-fluoro-3,5-dimethylphenyl)-4-methyl-2,4,6,7-tetrahydro-5H-pyrazolo[4,3-c]pyridine-5-carboxylate (2.1 g, 4.79 mmol) was added to hydrogen chloride (4M di-alkane solution, 20 mL) at room temperature and stirred at room temperature for 2 hours. The resulting crude product (S)-3-bromo-2-(4-fluoro-3,5-dimethylphenyl)-4-methyl-4,5,6,7-tetrahydro-2H-pyrazolo[4,3-c]pyridine (1.7 g) was concentrated under reduced pressure, and was used directly in the next step.

MS m/z (ESI): 338.0, 340.0[M+1].

Step 3: (S)-3-bromo-2-(4-fluoro-3,5-dimethylphenyl)-4-methyl-4,5,6,7-tetrahydro-2H-pyrazolo[4,3-c]pyridine hydrochloride (1.7 g, 4.54 mmol), intermediate A (526.84 mg, 4.54 mmol) and diisopropylethylamine (1.76 g, 13.61 mmol, 2.37 mL) were dissolved in N,N'-dimethylformamide (15 mL), and 2-(7-azobenzotriazole)-N,N,N',N'-tetramethyluronium hexafluorophosphate (2.59 g, 6.81 mmol) was added under nitrogen gas protection. The mixture was stirred at 25°C for 12 hours. The crude reaction solution was separated by preparative high performance liquid chromatography to give the product 3-((1S,2S)-1-(2-((S)-3-bromo-2-(4-fluoro-3,5-dimethylphenyl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indol-1-yl)-2-methylcyclopropyl)-1,2,4-oxadiazol-5(4H)-one (2 g, yield 60%).

MS m/z (ESI): 731.2, 733.2[M+1].

Step 4: 3-((1S,2S)-1-(2-((S)-3-bromo-2-(4-fluoro-3,5-dimethylphenyl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indol-1-yl)-2-methylcyclopropyl)-1,2,4-oxadiazol-5(4H)-one (1 g, 1.37 mmol), bis-pinacol borate (0.52 g, 2.05 mmol) and [1,1'-bis(diphenylphosphino)ferrocene] dichloropalladium dichloromethane complex (100 mg, 137 μmol) were dissolved in 1'4-dioxane (30 mL). Potassium acetate (335 mg, 3.42 mmol) was added under nitrogen gas protection. The mixture was stirred at 90°C for 12 hours. The mixture was concentrated under reduced pressure. The crude product was added with saturated brine (50 mL) to quench the reaction, and was extracted with ethyl acetate (50 mL × 2). The organic phases were combined, and washed with saturated brine (50 mL × 2), dried over anhydrous sodium sulfate, and concentrated under reduced pressure successively. The crude reaction solution was separated by preparative high performance liquid chromatography to obtain the

product (0.6 g, yield 56%).

MS m/z (ESI): 779.4[M+1].

## Intermediate E

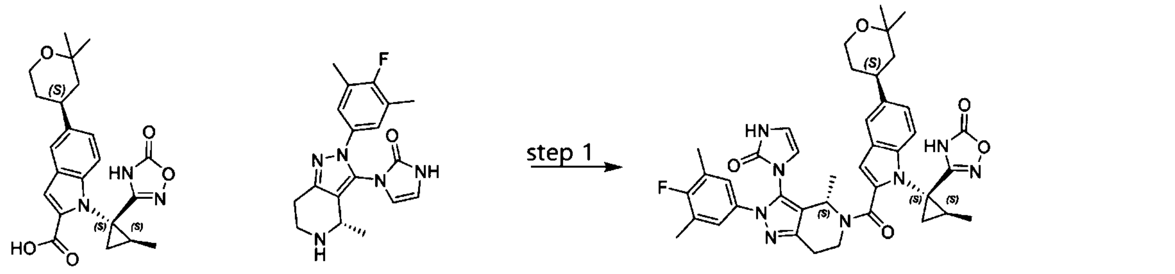


intermediate A intermediate B intermediate E

Step 1: Step 3 for intermediate C and intermediate D was followed to obtain the product.

MS m/z (ESI): 735.3[M+1].

## Intermediate F

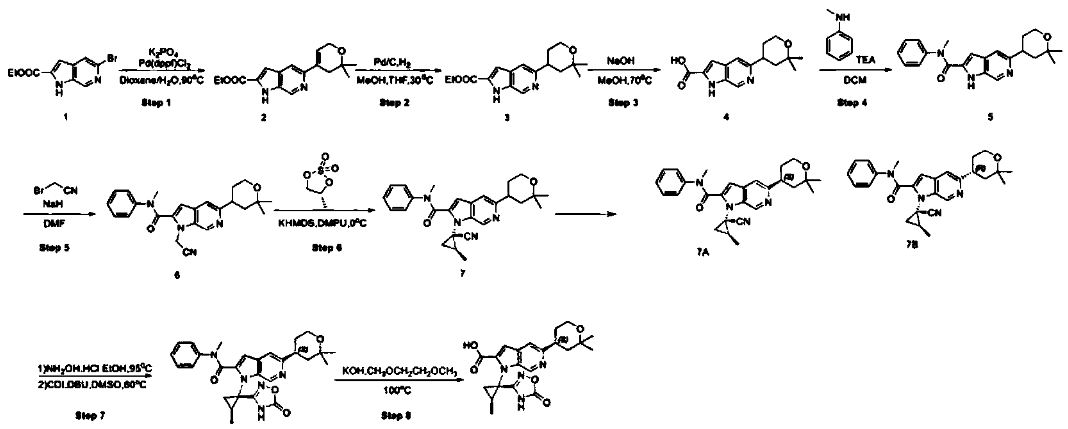


Intermediate F

Step 1: Ethyl 5-bromo-1H-pyrrolo[2,3-c]pyridine-2-carboxylate (5.0 g, 18.58 mmol, 1.0 eq.), 2-(6,6-dimethyl-5,6-dihydro-2H-pyran-3-yl)-4,4,5,5-tetramethyl-1,3,2-dioxaborolane (8.6 g, 22.30 mmol, 1.2 eq.), $K_3PO_4$ (11.8 g, 55.75 mmol, 3.0 eq.), Pd(dppf)$Cl_2$ (1.52 g, 1.86 mmol, 0.1 eq.) were added to a 500 ml three-necked flask successively, and then Dioxane (100 mL) and $H_2O$ (20 mL) were added. After replacement with nitrogen gas three times, reaction was carried out under reflux for 12 hours. After LCMS determination showed that the raw materials were reacted completely, the reaction solution was cooled to room temperature, and 100 mL of $H_2O$ was added to the system for extraction and layering. The aqueous phase was extracted with ethyl acetate (100 mL×3), the organic phases were combined, washed with saturated brine (100 mL×2), dried over anhydrous sodium sulfate, filtered, and concentrated. Purification was performed using silica gel column chromatography to obtain product 2 (4.18 g, 74.90% yield) as a yellow oily compound.

MS m/z (ESI): 301.1[M+1].

Step 2: Product 2 (4.0 g, 13.32 mmol, 1.0 eq.) was dissolved in a mixed solution of anhydrous methanol (20 mL) and THF (20 mL), and Pd/C (0.4 g, 10% w/w) was added to the system. After being replaced with nitrogen gas three times, the system was further replaced with hydrogen gas three times, and catalytic hydrogenation reaction was carried out at 30°C for 10 hours using a hydrogen gas balloon. After LCMS showed that the raw materials were completely reacted, Pd/C was filtered off with diatomaceous earth, and the filtrate was concentrated to dryness at 45°C to obtain product 3 (4 g, 99.32% yield) as a light yellow oil.

MS m/z (ESI): 303.1[M+1].

Step 3: Product 3 (4.0 g, 13.23 mmol, 1.0 eq.) and sodium hydroxide solution (14.6 ml, 29.11 mmol, 2 N, 2.2 eq.) were dissolved in methanol (40 mL), and the system was reacted at 70°C for 3 hours. The reaction solution was cooled to room temperature. The aqueous phase was washed with ethyl acetate (50 mL x 3), then adjusted to pH 5 with hydrochloric acid (3 N), and then extracted with ethyl acetate (50 mL x 3), washed with saturated brine (20 mL), dried over anhydrous

sodium sulfate, filtered, and concentrated to obtain product 4 (2.72 g, 74.98% yield) as an off-white solid.

MS m/z (ESI): 275.1[M+1].

Step 4: Product 4 (2.0 g, 7.29 mmol) was added to a 100 mL single-mouth bottle, and then 20 mL toluene was added. Under nitrogen gas protection, oxalyl chloride (1.85 g, 14.58 mmol, 2.0 eq.) and 2 drops of dry DMF were added slowly at room temperature. After the addition, reaction was carried out at 100°C for 2 hours. After the reaction solution was concentrated to dryness, dry dichloromethane (100 ml) was added, and N-methylaniline (1.56 g, 14.58 mmol, 2.0 eq.) was slowly added to the reaction system. After the addition, triethylamine (1.48 g, 14.58 mmol, 2.0 eq.) was added. After the addition, reaction was carried out at 300C for 8 hours. After LCMS monitoring showed that the reaction was complete, the reaction system was added with $H_2O$ (50 ml), and was extracted with dichloromethane (100 mL x 3). The organic phases were combined, washed with saturated brine (30 mL), dried over anhydrous sodium sulfate, filtered, and concentrated. The resulting residue was purified by silica gel column chromatography to obtain the target product (1.58 g, 59.63% yield) as a yellow oil.

MS m/z (ESI): 364.2[M+1].

Step 5: Product 5 (1.5 g, 4.13 mmol, 1.0 eq.) was dissolved in 15 ml of dry DMF. After cooling to 0°C in an ice bath under nitrogen gas protection, NaH (495 mg 12.38 mmol, 3.0 eq.) was added slowly. After the addition, reaction under stirring was carried out at 0°C for 0.5 hours. Bromoacetonitrile (2.48 g, 20.65 mmol, 5.0 eq.) was added slowly. After the addition, reaction was carried out at 30°C for 8 hours. The reaction system was added with ethyl acetate and water, and was extracted with ethyl acetate (50 mL x 3). The organic phases were combined, washed with saturated brine (30 mL), dried over anhydrous sodium sulfate, filtered, and concentrated. The resulting residue was purified by silica gel column chromatography to obtain the target product (997 mg, 60% yield) as a yellow foamy solid.

MS m/z (ESI): 403.2[M+1].

Step 6: Product 6 (900 mg, 2.24 mmol, 1.0 eq.) and (R)-4-methyl-1,3,2-dioxathiacyclohexane 2,2-dioxide (1.08 g, 7.84 mmol, 3.5 eq.) were dissolved in DMPU (20 mL). After cooling to 0°C in an ice bath, KHMDS (22.4 mmol, 10.0 eq., 1M in THF) was slowly added dropwise. After the addition, reaction was carried out under heat preservation for 5 hours. The reaction system was added with ethyl acetate and water, and was extracted with ethyl acetate (50 mL x 3). The organic phases were combined, washed with saturated brine (30 mL), dried over anhydrous sodium sulfate, filtered, and concentrated. The resulting residue was purified by silica gel column chromatography to obtain the target product (496 mg, 50% yield) as a yellow foamy solid.

MS m/z (ESI): 443.2[M+1].

Chiral resolution was performed on 7 to obtain 7A and 7B

MS m/z (ESI): 443.2[M+1].

Step 7: Product 7A (450 mg, 1.02 mmol, 1.0 eq.), hydroxylamine hydrochloride (354 mg, 5.10 mmol, 5.0 eq.), $K_2CO_3$ (775 mg, 5.61 mmol, 5.5 eq.) and anhydrous ethanol (25 mL) were added to a 100 mL single-necked bottle successively, and reaction was carried out at 95°C for 5 hours. After the reaction solution was concentrated to dryness, dry THF (30 ml) was added. After stirring to dissolution, insoluble matter was filtered off, and N,N'-carbonyldiimidazole (364 mg, 2.24 mmol, 2.2 eq.) and 1, 8-diazabicyclo[5.4.0]undec-7-ene (466 mg, 3.06 mmol, 3.0 eq.) were added to the filtrate. The reaction mixture was stirred at 50°C for three hours. After the reaction solution was concentrated to dryness, the resulting residue was purified by silica gel column chromatography to obtain product 8 (417 mg, 81.6% yield) as a light yellow solid.

MS m/z (ESI): 502.2[M+1].

Step 8: Product 8 (400 mg, 0.80 mmol, 1.0 eq.) was dissolved in ethylene glycol dimethyl ether (20 mL), and KOH (404 mg, 7.2 mmol, 9.0 eq.) was added. The system was reacted at 100°C for 5 hours. Afte the reaction solution was concentrated to dryness, the resulting residue was purified by silica gel column chromatography to obtain the target product (200 mg, 60.60% yield) as a yellow solid.

MS m/z (ESI): 413.2[M+1].

## Intermediate G

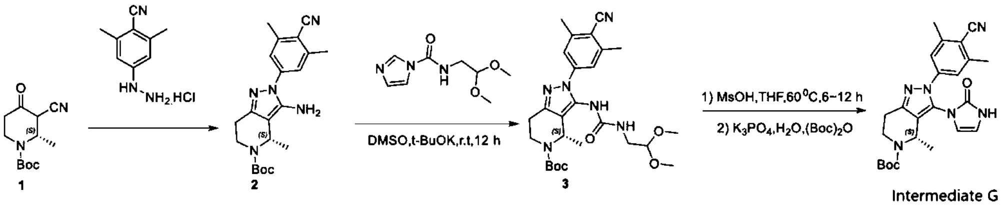


1 2 3 Intermediate G

Step 1: Tert-butyl (2S)-3-cyano-2-methyl-4-carbonylpiperidine-1-carboxylate (2 g, 8.4 mmol), toluene (20 mL, 10v), 4-hydrazino-2,6-dimethylbenzonitrile hydrochloride (1.65 g, 8.4 mmol), and pyridine hydrochloride (485 mg, 4.2

mmol) were added to a bottle. After the addition, the mixture was heated to 96°C under nitrogen gas protection, and was stirred overnight. After the reaction is completed, the reaction was added with water and extracted with ethyl acetate (50mL*3). The organic phase was dried over $Na_2SO_4$, and the residue was purified by silica gel column chromatography (PE:EA=1:1) to obtain the product (1.32 g, yield 41.3%).

MS m/z (ESI): 382.2[M+1].

Step 2: Compound 2 (1.22 g, 3.2 mmol), dimethyl sulfoxide (30 ml, 15v), N-(2,2-dimethoxyethyl)-1H-imidazole-1-carboxamide (766 mg, 3.8 mmol), and potassium tert-butoxide (1.29 g, 11.6 mmol) were added to a bottle. After replacement with nitrogen gas, the mixture was stirred at room temperature overnight. After LC-MS determination showed that the reaction was completed, the reaction solution was added with water and extracted ethyl acetate (80 mL*3). The organic phase was dried over $Na_2SO_4$, and the residue was purified by silica gel column chromatography (PE:EA=3:1) to obtain the product (1.08 g, yield 65.8%).

MS m/z (ESI): 513.2[M+1].

Step 3: Compound 3 (500 mg, 0.98 mmol), tetrahydrofuran (15 ml, 30 v), MsOH (470.4 mg, 4.90 mmol) were added to a bottle. After the addition, the bottle was replaced with nitrogen gas and was heated to 60°C to react for 6 hours. LC-MS determination showed no compound 3. The temperature was decreased to room temperature, and potassium phosphate (1.67 g, 7.84 mmol) was added to the reaction solution. After the reaction system was determined to be alkaline, Boc anhydride (320 mg, 1.47 mmol) was added. Stirring was performed at room temperature for 2 hours. The reaction solution was added with water and extracted with ethyl acetate (80 mL*3). The organic phase was dried over $Na_2SO_4$, and the residue was purified by silica gel column chromatography (PE:EA=3:1) to obtain the product (380 mg, yield 86.5%).

MS m/z (ESI): 449.2[M+1].

**Example 1**

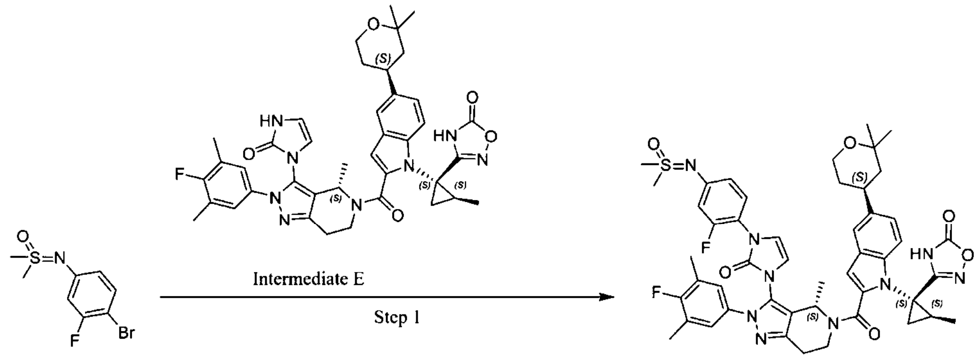


Step 1: To a suspension of intermediate E (20 mg, 0.027 mmol), ((4-bromo-3-fluorophenyl)imino)dimethyl-6-thione (14.5 mg, 0.054 mmol), (1*S*,2*S*)-1-*N*,2-*N*-dimethylcyclohexane-1,2-diamine (1.9 mg, 0.013 mmol) and potassium carbonate (11.3 mg, 0.082 mmol) in N-methylpyrrolidone (0.5 mL) was added copper iodide (I) (1.0 mg, 0.0054 mmol) at room temperature, and the mixture was stirred at 130°C under a nitrogen atmosphere for 3 hours. The reaction mixture was purified by reverse phase silica gel chromatography (acetonitrile/water, 0.1% formic acid) to obtain a target compound (10 mg, yield 40%).

MS m/z (ESI): 920.3[M+1].

**Example 2**

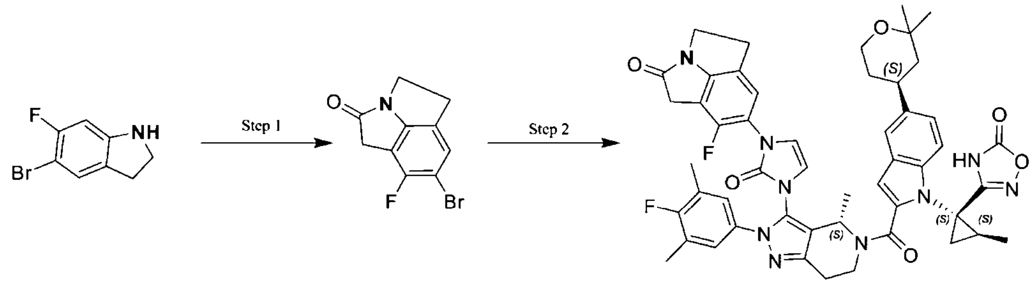


Step 1: From 5-bromo-6-fluoroindoline as a starting material, a target product was obtained by referring to *Journal of Organic Chemistry,* 1992, vol. 57, #8, p. 2508-2511.
MS m/z (ESI): 256.0, 258.0[M+1]
Step 2: Referring to Example 1, a target product was obtained.
MS m/z (ESI): 910.4[M+1].

**Example 3**

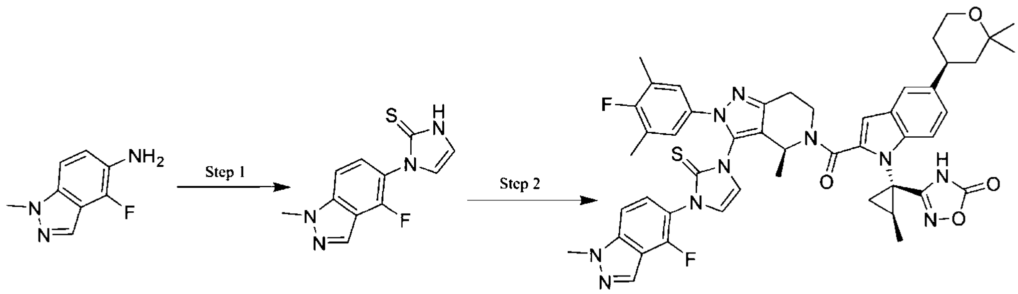


Step 1: From 4-fluoro-1-methyl-1*H*-indazol-5-amine as a starting material, a target product was obtained by referring to *New Journal of Chemistry,* 2014, vol. 38, #9, p. 4071-4082.
MS m/z (ESI): 249.1[M+1].
Step 2: From intermediate C as a starting material, a target product was obtained by referring to Example 1.
MS m/z (ESI): 899.4[M+1].

**Example 4**

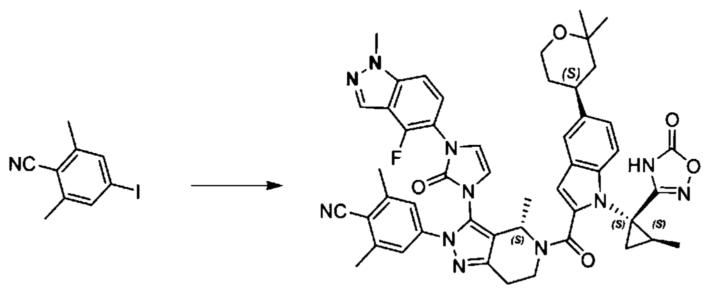

Step 1: Referring to step 3 for intermediate B, 4-iodo-2,6-dimethylbenzonitrile was used instead of 2-fluoro-5-iodo-1,3-dimethylbenzene to synthesize an intermediate, and then a target product was synthesized by referring to intermediate E and Example 1 (5-bromo-4-fluoro-1-methyl-1*H*-indazole was used instead of ((4-bromo-3-fluorophenyl) imino)dimethyl-6-thione).
MS m/z (ESI): 890.4[M+1].

**Example 5**

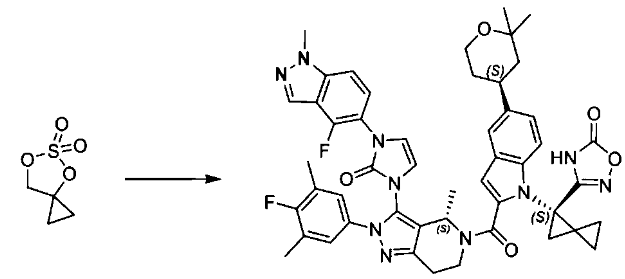

Step 1: Referring to step 3 for intermediate A, 4,6-dioxa-5-thiospiro[2.4]heptane 5,5-dioxide was used instead of (4*R*)-4-methyl-1,3,2-dioxathiolane 2,2-dioxide to synthesize an intermediate, and then a target product was synthesized by referring to intermediate E and Example 1 (5-bromo-4-fluoro-1-methyl-1*H*-indazole was used instead of ((4-bromo-3-fluorophenyl)imino)dimethyl-6-thione).

MS m/z (ESI): 895.4[M+1].

**Example 6**

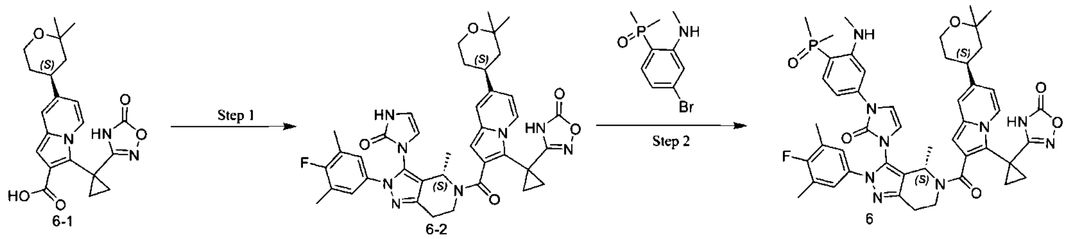


Step 1: From 6-1 as a starting material, 6-2 was obtained by referring to intermediate C and step 3 for intermediate D.

MS m/z (ESI): 721.3[M+1].

Step 2: Referring to Example 1, target product 6 was obtained from (4-bromo-2-(methylamino)phenyl)dimethylphosphine oxide.

MS m/z (ESI): 902.4[M+1].

**Example 7**

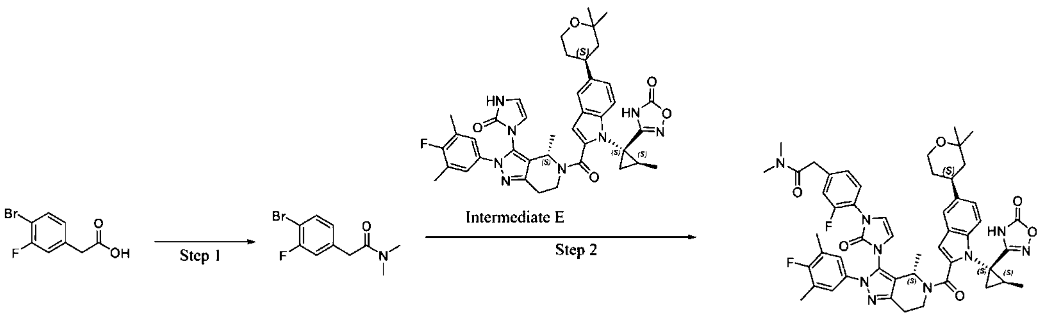


Step 1: 2-(4-Bromo-3-fluorophenyl)-*N,N*-dimethylacetamide

At room temperature, 2-(4-bromo-3-fluorophenyl)acetic acid (2.33 g, 10 mmol) was dissolved in *N,N*-dimethylformamide (30 mL), and then dimethylamine hydrochloride (0.98 g, 12 mmol) and triethylamine (4.05 g, 40 mmol) were added, followed by 2-(7-azobenzotriazole)-*N,N,N',N'*-tetramethyluronium hexafluorophosphate (5.7 g, 15 mmol). The mixture was stirred at room temperature overnight, until LCMS indicated that the reaction was completed. The reaction solution was diluted with ethyl acetate (60 mL), and then washed with saturated brine (15 mL × 4). The organic phase was dried over anhydrous sodium sulfate, filtered, and spun dry. The residue was separated by flash column chromatography (PE: EA = 1: 1) to obtain a target product as a white solid (1.30 g, yield 50%).

MS m/z (ESI): 260.0, 262.0[M+H]+.

Step 2: From 2-(4-bromo-3-fluorophenyl)-*N,N*-dimethylacetamide and intermediate E as starting materials, a target product was obtained by referring to Example 1.

MS m/z (ESI): 914.4[M+H]+.

**Example 8**

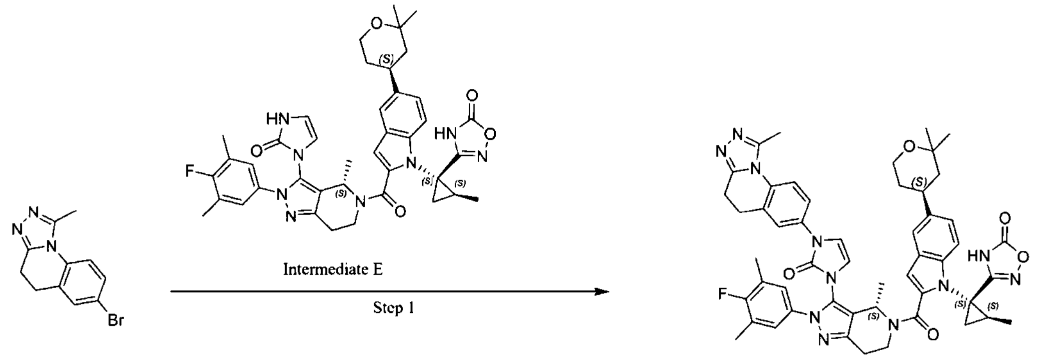


Step 1: From 7-bromo-1-methyl-4,5-dihydro-[1,2,4]triazolo[4,3-a]quinoline and intermediate E as starting materials, a target product was obtained by referring to Example 1.

MS m/z (ESI): 918.4[M+H]+.

**Example 9**

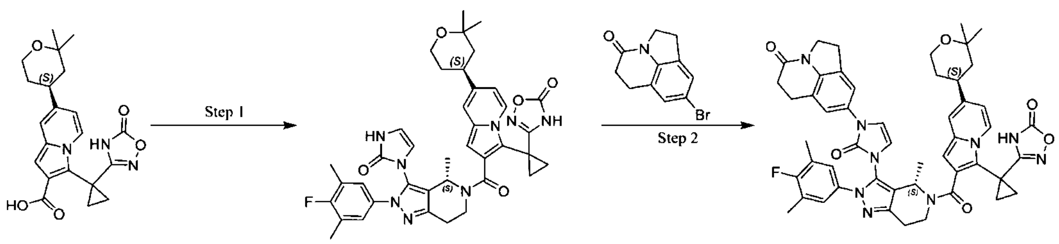


Step 1: A product was obtained by referring to intermediate C and step 3 for intermediate D.

MS m/z (ESI): 721.3[M+1].

Step 2: From 8-bromo-5,6-dihydro-1*H*-pyrrolo[3,2,1-ij]quinolin-4(2*H*)-one as a starting material, a target product was obtained by referring to Example 1.

MS m/z (ESI): 892.4[M+1].

**Example 10**

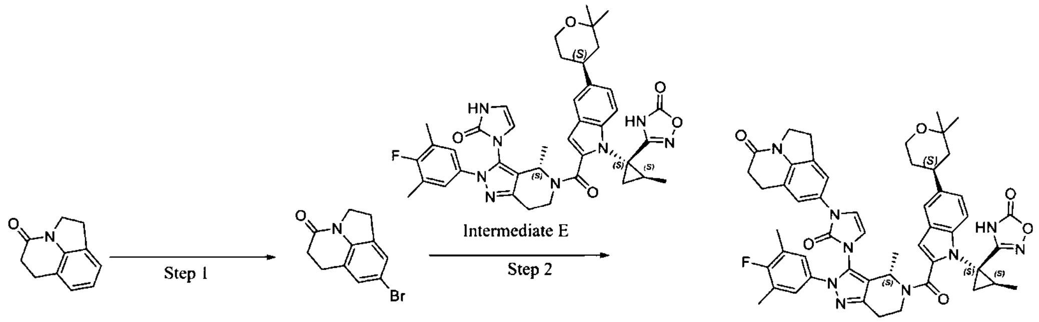


Step 1: At 0°C, 1,2,5,6-tetrahydro-4*H*-pyrrolo[3,2,1-ij]quinolin-4-one (1.73 g, 10 mmol) was dissolved in *N,N*-dimethylformamide (12 mL), and then *N*-bromosuccinimide (1.96 g, 11 mmol) in *N,N*-dimethylformamide (20 mL) was added dropwise. The mixture was stirred at room temperature for 2 hours. The reaction solution was quenched with water (60 mL), and then extracted with ethyl acetate (25 mL × 3). The organic phases were merged, washed with saturated brine (25 mL × 2), dried over anhydrous sodium sulfate, filtered, and spun dry. The crude product was crystallized with acetone/water to obtain a target product as a yellow solid (1.89 g, yield 75%).

MS m/z (ESI): 252.1, 254.1[M+H]+.

Step 2: From 8-bromo-1,2,5,6-tetrahydro-4*H*-pyrrolo[3,2,1-i]quinolin-4-one and intermediate E as starting materials, a target product was obtained by referring to Example 1.

MS m/z (ESI): 906.4[M+H]+.

**Example 11**

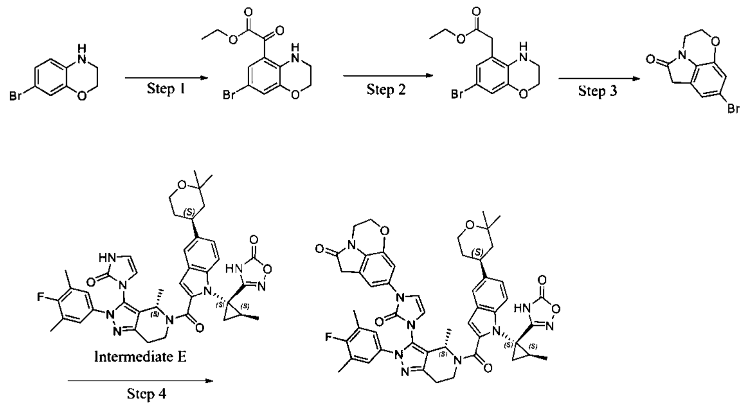


Step 1: In an ice bath, ethyl oxalyl chloride (1.64 g, 12 mmol) was dissolved in dichloromethane (20 mL), and then aluminum chloride (8.0 g, 60 mmol) was added. The mixture was stirred in an ice bath for 30 minutes, and then 7-bromo-3,4-dihydro-2*H*-benzo[b][1,4]oxazine (2.14 g, 10 mmol) was added. The mixture was stirred at room temperature for 2 hours, until LCMS indicated that a target product was generated. The reaction solution was quenched with a 10% aqueous sodium hydroxide solution and filtered, and the filter residue was washed with dichloromethane. The organic phase was washed with a saturated sodium bicarbonate solution (25 mL × 2) and saturated brine (25 mL × 2) in sequence. The organic phase was dried over anhydrous sodium sulfate, filtered, and spun dry. The residue was separated by flash column chromatography to obtain a target product (0.31 g, yield 10%).

MS m/z (ESI): 314.1, 316.1[M+H]+

Step 2: At room temperature, ethyl 2-(7-bromo-3,4-dihydro-2*H*-benzo[b][1,4]oxazin-5-yl)-2-carbonyl acetate (0.31 g, 1 mmol) was dissolved in trifluoroacetic acid (5 mL), and then triethylsilyl hydrochloride (0.23 g, 2 mmol) was added. The mixture was heated to 60°C for reaction for 2 hours, until LCMS indicated that the reaction was complete. The reaction system was cooled to room temperature and spun dry. The residue was diluted with ethyl acetate (20 mL), and then washed with a saturated sodium bicarbonate solution (25 mL × 2) and saturated brine (25 mL × 2) in sequence. The organic phase was dried over anhydrous sodium sulfate, filtered, and spun dry. The residue was directly used for the next step.

MS m/z (ESI): 300.1, 302.1[M+H]+.

Step 3: At room temperature, ethyl 2-(7-bromo-3,4-dihydro-2*H*-benzo[b][1,4]oxazin-5-yl) acetate (0.30 g, 1

mmol) was dissolved in ethanol (5 mL), and then acetic acid (0.2 mL) was added. The mixture was heated to 80°C for reaction for 2 hours. The reaction solution was cooled to room temperature, until LCMS indicated that a target product was generated. The reaction solution was spun dry, and the residue was dissolved in ethyl acetate (20 mL). The resulting solution was then washed with a saturated sodium bicarbonate solution (25 mL × 2) and saturated brine (25 mL × 2) in sequence. The organic phase was dried over anhydrous sodium sulfate, filtered, and spun dry. The residue was separated by flash column chromatography to obtain target product 11-3 (0.05 g, yield 20%).

MS m/z (ESI): 254.1, 256.1[M+H]+.

Step 4: From 8-bromo-2,3-dihydro-[1,4]oxaazino[2,3,4-hi]indol-5(6*H*)-one and intermediate E as starting materials, target product 11 was obtained by referring to Example 1.

MS m/z (ESI): 908.4[M+H]+.

**Example 12**

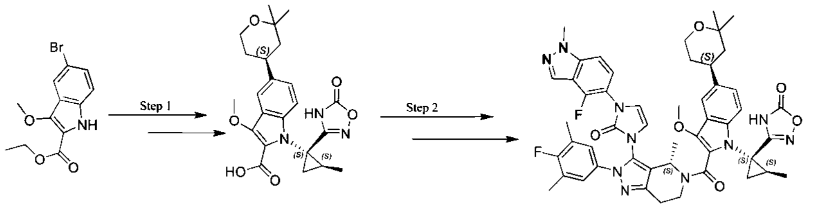


Step 1: From ethyl 5-bromo-3-methoxyindole-2-carboxylate as a starting material, a target product was obtained by referring to intermediate A.

MS m/z (ESI): 442.2[M+1].

Step 2: A product was obtained by referring to intermediate C and step 3 for intermediate D, and then a target product was obtained from 5-bromo-4-fluoro-1-methyl-1*H*-indazole as a starting material by referring to Example 1.

MS m/z (ESI): 913.4[M+1].

**Example 13**

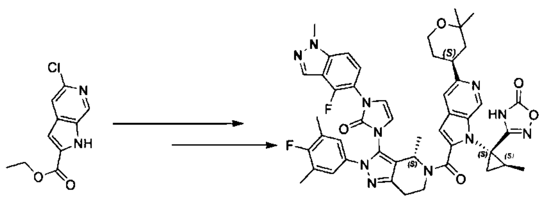

Referring to Example 12, a target product was obtained from ethyl 5-chloro-1*H*-pyrrolo[2.3-c]pyridine-2-carboxylic acid as a starting material.

MS m/z (ESI): 884.4[M+1].

**Example 14**

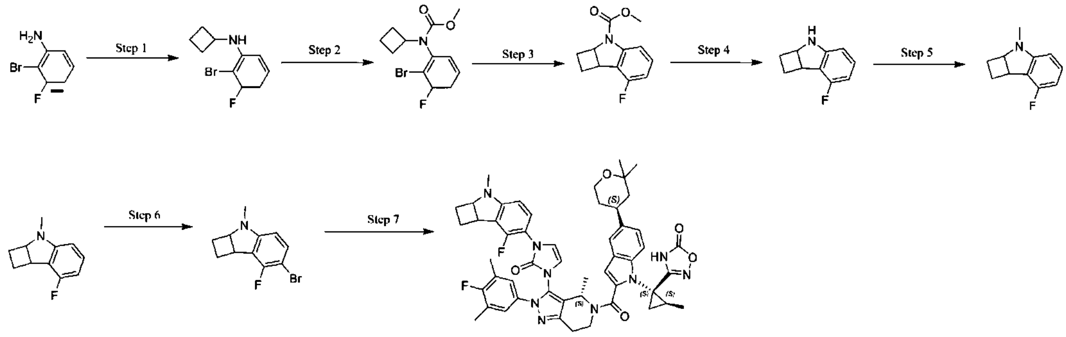


Step 1: From 2-bromo-3-fluoroaniline as a starting material, a target product was obtained by referring to MERCK & CO INC-WO2020/96871, 2020, A1.

MS m/z (ESI): 244.0[M+1].

Step 2: From 2-bromo-*N*-cyclobutyl-3-fluoroaniline as a starting material, a target product was obtained by referring to MERCK & CO INC-WO2020/96871, 2020, A1.

MS m/z (ESI): 302.0[M+1].

Step 3: From methyl (2-bromo-3-fluorophenyl)(cyclobutyl)carbamate as a starting material, a target product was obtained by referring to Bioorganic and Medicinal Chemistry Letters, 2021, vol. 47.

MS m/z (ESI): 222.0[M+1].

Step 4: From methyl 7-fluoro-1,2,2a,7b-tetrahydro-3*H*-cyclobutadiene[b]indole-3-carboxylate as a starting material, a target product was obtained by referring to Tetrahedron, 2015, vol. 71, #37, art. no. 26568, p. 6499-6505.

MS m/z (ESI): 164.0[M+1].

Step 5: From 7-fluoro-2,2a,3,7b-tetrahydro-1*H*-cyclobutadiene[b]indole as a starting material, a target product was obtained by referring to European Journal of Medicinal Chemistry, 2020, vol. 190.

MS m/z (ESI): 178.1[M+1].

Step 6: From 7-fluoro-3-methyl-2,2a,3,7b-tetrahydro-1*H*-cyclobutadiene[b]indole as a starting material, a target product was obtained by referring to Chemistry A European Journal, 2022, vol. 28, #2, art. no. E202103135.

MS m/z (ESI): 256.0[M+1].

Step 7: From 6-bromo-7-fluoro-3-methyl-2,2a,3,7b-tetrahydro-1*H*-cyclobutadiene[b]indole and intermediate E as starting materials, a target product was obtained by referring to Example 1.

MS m/z (ESI): 910.4[M+1].

**Example 15**

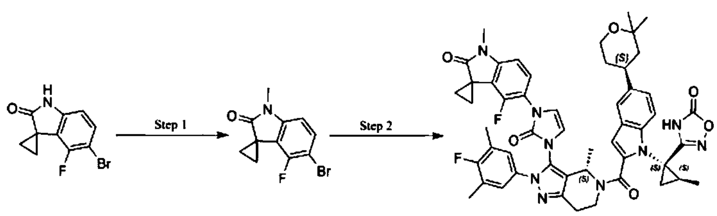


Step 1: From 5'-bromo-4'-fluorospiro[cyclopropane-1,3'-dihydroindole]-2'-one as a starting material, a target product was obtained by referring to European Journal of Medicinal Chemistry, 2020, vol. 190.

MS m/z (ESI): 269.9[M+1].

Step 2: From intermediate E as a starting material, a target product was obtained by referring to Example 1.

MS m/z (ESI): 924.3[M+1].

**Example 16**

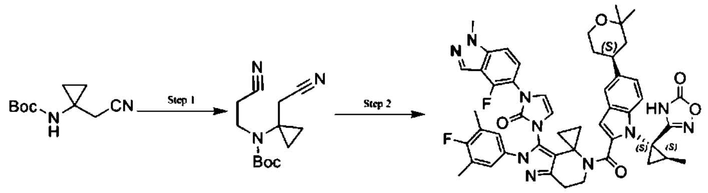


Step 1: *tert*-Butyl (1-(cyanomethyl)cyclopropyl)carbamate (5 g, 25.48 mmol) and 3-bromopropionitrile (3.41 g, 25.48 mmol) were dissolved in acetonitrile (100 mL), and potassium carbonate (10.56 g, 76.43 mmol) was added under nitrogen protection. The mixture was stirred at 25°C for reaction for 12 hours. The reaction solution was evaporated to dryness. Saturated brine (130 mL) was added to the crude product, and the crude product was extracted with ethyl acetate (80 mL × 2). The organic phases were merged, washed with saturated brine (30 mL × 2), dried over anhydrous sodium sulfate, and concentrated under reduced pressure to obtain *tert*-butyl (2-cyanoethyl)(1-(cyanomethyl)cyclopropyl)carbamate (6 g, yield: 94.46%).

MS m/z (ESI): 250.2[M+1].

Step 2: From *tert*-butyl (1-(cyanomethyl)cyclopropyl)carbamate as a starting material, a target product was synthesized by referring to intermediate B and intermediate E and Example 1.

MS m/z (ESI): 895.4[M+1].

## Example 17

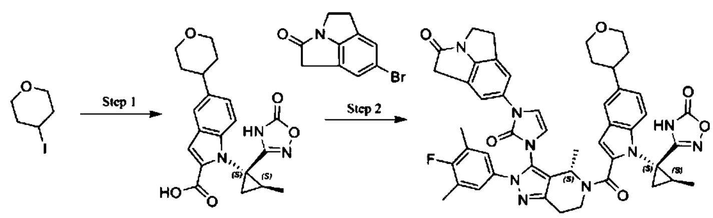


Step 1: From 4-iodotetrahydro-2*H*-pyran as a starting material, 1-((1*S*,2*S*)-2-methyl-1-(5-carbonyl-4,5-dihydro-1,2,4-oxadiazol-3-yl)cyclopropyl)-5-(tetrahydro-2*H*-pyran-4-yl)-1*H*-indole-2-carboxylic acid was synthesized by referring to intermediate A.

MS m/z (ESI): 384.2[M+1].

Step 2: From 7-bromo-4,5-dihydropyrrolo[3,2,1-hi]indol-2(1*H*)-one as a starting material, a target compound was obtained by referring to Example 1.

MS m/z (ESI): 864.4[M+1].

## Example 18

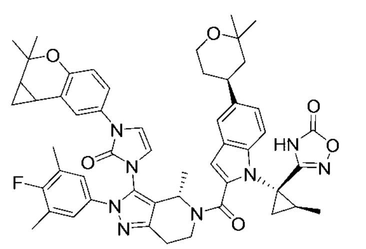

From 6-bromo-2,2-dimethyl-1,1a,2,7b-tetrahydrocyclopropene[c]chromene as a starting material, a target product was obtained by referring to Example 1.

MS m/z (ESI): 907.4[M+1].

**Example 19**

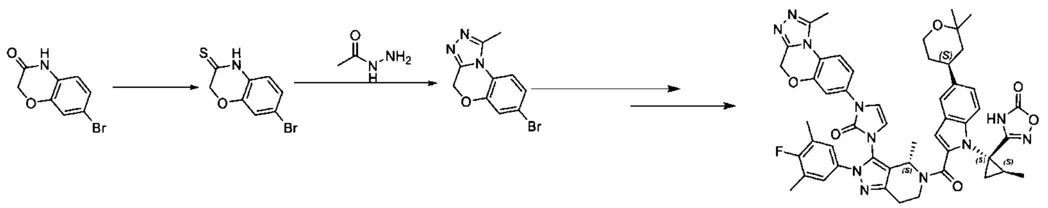

Step 1: A mixture of 7-bromo-2*H*-benzo[b][1,4]oxazine-3(4*H*)-one (3 g, 13.16 mmol), Lawesson's reagent (3.19 g, 7.89 mmol), and toluene (100 mL) was stirred at 110°C for 1 hour, cooled, and directly concentrated with silica gel added. The mixture was separated by column chromatography (PE/EtOAc = 10: 1) to obtain a light yellow solid (600 mg, yield: 18%).
MS m/z (ESI): 244.0 246.0[M+1].
Step 2: A mixture of 7-bromo-2*H*-benzo[b][1,4]oxazine-3(4*H*)-thione (300 mg, 1.23 mmol), acetic hydrazide (109 mg, 1.47 mmol), and *n*-butanol (10 mL) was stirred at 120°C for 24 hours, cooled, and directly concentrated with silica gel added, and separated by column chromatography (DCM/MeOH = 10: 1) to obtain a target product (190 mg, yield: 58%). MS m/z (ESI): 266.0, 268.0[M+1]
Step 3: From 7-bromo-1-methyl-4*H*-benzo[b][1,2,4]triazolo[4,3-d][1,4]oxazine as a starting material, a target product was obtained by referring to Example 1.
MS m/z (ESI): 920.4[M+1].

**Example 20**

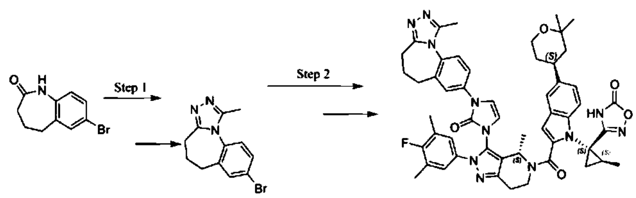


Step 1: From 7-bromo-1,3,4,5-tetrahydro-2*H*-1-benzazepin-2-one as a starting material, 8-bromo-1-methyl-5,6-dihydro-4*H*-benzo[f][1,2,4]triazolo[4,3-a]azepine was obtained by referring to step 1 and step 2 in Example 19.
MS m/z (ESI): 278.0, 280.0[M+1].
Step 2: From 8-bromo-1-methyl-5,6-dihydro-4*H*-benzo[f][1,2,4]triazolo[4,3-a]azepine as a starting material, a target product was obtained by referring to Example 1.
MS m/z (ESI): 932.4[M+1].

**Example 21**

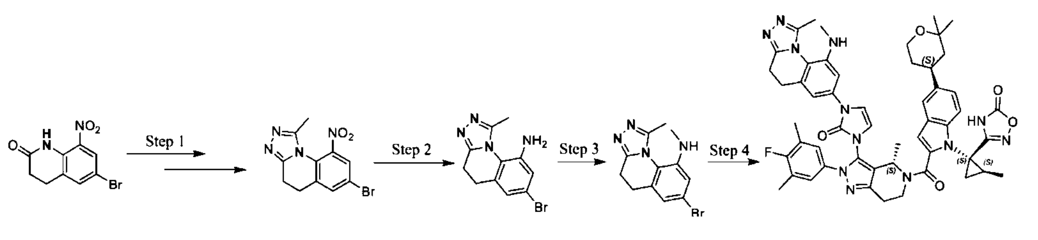


Step 1: From 6-bromo-8-nitro-3,4-dihydroquinolin-2(1*H*)-one as a starting material, a target product was obtained by referring to Example 1.
MS m/z (ESI): 309.0, 311.0[M+1].

Step 2: Sodium dithionite (9.03 g, 51.92 mmol) was added to a solution of 7-bromo-1-methyl-9-nitro-4,5-dihydro-[1,2,4]triazolo[4,3-a]quinoline (2 g, 6.49 mmol) in ethanol (20 mL) and water (15 mL). The mixture was stirred at room temperature for 12 hours and filtered. The filter cake was washed with EtOH. Water was added to the filtrate, and the filtrate was extracted with EtOAc. The organic phases were merged and washed with saturated brine, dried over anhydrous sodium sulfate, and filtered, and the filtrate was concentrated to dryness under reduced pressure to obtain 21-3 (1.5 g, yield: 83.1%)

MS m/z (ESI): 279.0, 281.0[M+1].

Step 3: NaH (130 mg, 3.24 mmol, 60% purity) was added to a solution of 7-bromo-1-methyl-4,5-dihydro-[1,2,4]triazolo[4,3-a]quinolin-9-amine (600 mg, 2.16 mmol) in DMF (10 mL). The mixture was stirred at room temperature for 30 minutes, and then iodomethane (457 mg, 3.24 mmol) was added dropwise. The mixture was stirred at room temperature for 1 hour, and water was added thereto. The solution was extracted three times with DCM. The organic phase was washed with saturated brine, dried over anhydrous sodium sulfate, and filtered, and the filtrate was concentrated to dryness under reduced pressure and separated by column chromatography to obtain light yellow oily matter 21-4 (300 mg, yield: 47.6%)

MS m/z (ESI): 293.0 295.0[M+1].

Step 4: From 7-bromo-N,1-dimethyl-4,5-dihydro-[1,2,4]triazolo[4,3-a]quinolin-9-amine as a starting material, a target product was obtained by referring to Example 1.

MS m/z (ESI): 947.4[M+1].

**Example 22**

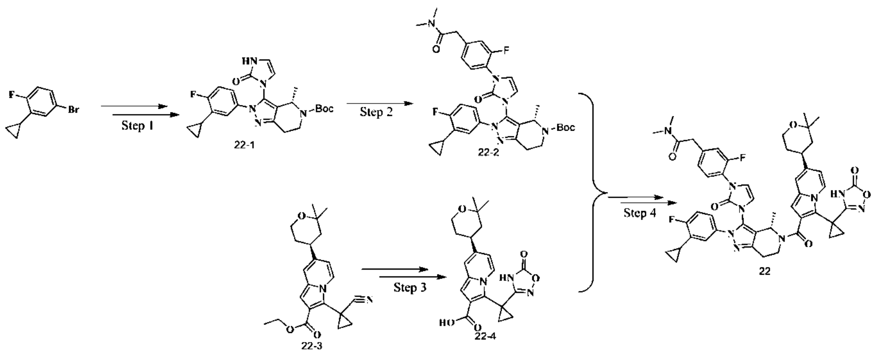


Step 1: From 4-bromo-2-cyclopropyl-1-fluorobenzene as a starting material, product 22-1 was obtained by referring to the synthesis of intermediate B in step 3 to step 5.

MS m/z (ESI): 454.2[M+1].

Step 2: From 22-1 and 2-(4-bromo-3-fluorophenyl)-N,N-dimethylacetamide as starting materials, product 22-2 was obtained by referring to the synthesis steps in Example 1.

MS m/z (ESI): 633.3[M+1].

Step 3: From 22-3 as a starting material, product 22-4 was obtained by referring to the synthesis steps of intermediate A in step 4 to step 5.

MS m/z (ESI): 398.2[M+1].

Step 4:

From 22-2 and 22-4 as starting materials, product 22 was obtained by referring to the synthesis of intermediate C in step 2 and step 3.

MS m/z (ESI): 912.4[M+1].

**Example 23**

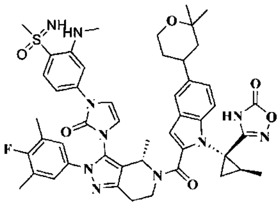

23

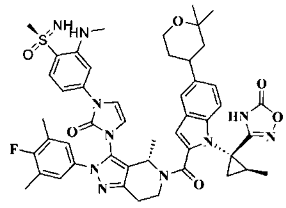

23-a

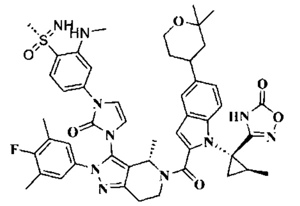

23-b

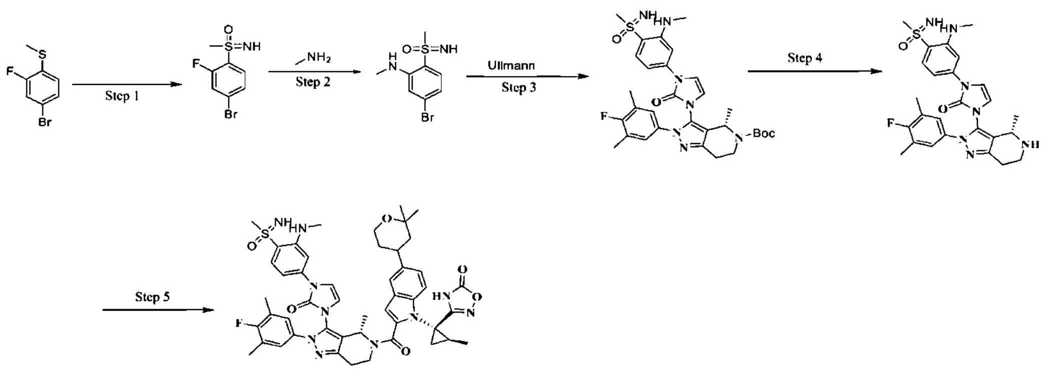


Step 1: To a solution of 4-bromo-2-fluoro-1-methylsulfanyl-benzene (2 g, 9.05 mmol) in methanol (100 mL), ammonium carbamate (2.82 g, 36.18 mmol) and [di(acetoxy)iodo]benzene (8.74 g, 27.14 mmol) were added in sequence, and then the mixture was stirred at room temperature for half an hour. Water was added, and the mixture was extracted three times with DCM. The organic phase was washed with saturated brine, dried over anhydrous sodium sulfate, and filtered, and the filtrate was concentrated to dryness under reduced pressure and separated by column chromatography (DCM/MeOH = 10: 1) to obtain light yellow oily matter (2 g, yield: 87.7%).

MS m/z (ESI): 252.0, 254.0M+H]+.

Step 2: A mixture of (4-bromo-2-fluorophenyl)(imino)(methyl)-16-thiocanone (400 mg, 1.59 mmol), methylamine hydrochloride (214 mg, 3.17 mmol), potassium carbonate (657 mg, 4.76 mmol), and DMF (10 mL) was stirred at 120°C for 3 hours. Water was added, and the mixture was extracted three times with DCM. The organic phase was washed with saturated brine, dried over anhydrous sodium sulfate, and filtered, and the filtrate was concentrated to dryness under reduced pressure and separated by column chromatography (DCM/MeOH = 10: 1) to obtain light yellow oily matter (200 mg, yield: 47.9%).

MS m/z (ESI): 263.0, 265.0[M+H]+

Step 3: From (4-bromo-2-(methylamino)phenyl)(imino)(methyl)-16-thiocanone as a starting material, a product was obtained by referring to Example 1.

MS m/z (ESI): 624.3[M+H]+

Step 4: TFA (1 mL) was added dropwise to a solution of *tert*-butyl (4*S*)-2-(4-fluoro-3,5-dimethylphenyl)-4-methyl-3-(3-(3-(methylamino)-4-(*S*-methylsulfonimide)phenyl)-2-carbonyl-2,3-dihydro-1*H*-imidazol-1-yl)-2,4,6,7-tetrahydro-5H-pyrazolo[4,3-c]pyridine-5-carboxylate (60 mg, 96.19 μmol) in DCM (3 mL), and stirred at room temperature for 0.5 hours. The mixture was spun dry, adjusted to pH 10 with saturated $NaHCO_3$ added, and extracted with DCM three times. The organic phase was washed with saturated brine, dried over anhydrous sodium sulfate, and filtered, and the filtrate was concentrated to dryness under reduced pressure to obtain light yellow oily matter (50 mg, crude product).

MS m/z (ESI): 524.2[M+H]+

Step 5: To a solution of 1-((*S*)-2-(4-fluoro-3,5-dimethylphenyl)-4-methyl-4,5,6,7-tetrahydro-2*H*-pyrazolo[4,3-c]pyridin-3-yl)-3-(3-(methylamino)-4-(S-methylsulfonimidoyl)phenyl)-1,3-dihydro-2*H*-imidazol-2-one (23.57 mg, 57.29 μmol) and intermediate A (30 mg, 57.29 μmol) in DMF (3 mL) were added HATU (43.23 mg, 114.59 μmol) and DIEA (22 mg, 171.88 μmol), and were stirred at room temperature for 16 hours. Water was added, and the mixture was extracted three times with DCM. The organic phase was washed with saturated brine, dried over anhydrous sodium sulfate, and filtered, and the filtrate was concentrated to dryness under reduced pressure to obtain light yellow oily matter, which was separated by preparative chromatography (acidic) to obtain target product 23 (9 mg, yield: 16.7%).

MS m/z (ESI): 917.4[M+H]+

Target products 23-a and 23-b were obtained by chiral separation.

MS m/z (ESI): 917.4[M+H]+.

**Example 24**

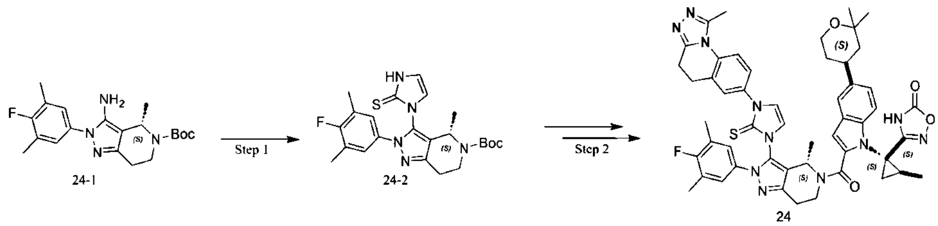


24-1 24-2 24

Step 1: From 24-1 as a starting material, product 24-2 was obtained by referring to the synthesis steps in step 1 of Example 3.

MS m/z (ESI): 458.2[M+1].

Step 2: From 24-2 as a starting material, 7-bromo-1-methyl-4,5-dihydro-[1,2,4]triazolo[4,3-a]quinoline was used instead of the ((4-bromo-3-fluorophenyl)imino)dimethyl-6-thione in Example 1 to obtain title product 24 by referring to intermediate E and the synthesis steps in Example 1,.

MS m/z (ESI): 934.4[M+1].

**Example 25**

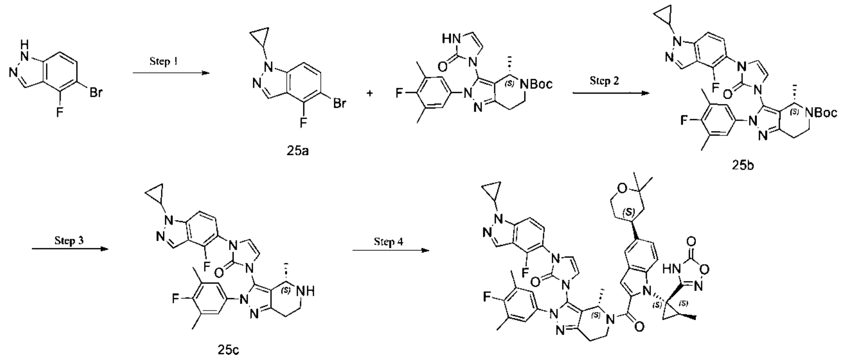


25a 25b 25c

Step 1: 5-Bromo-4-fluoro-1*H*-indazole (200 mg, 0.93 mmol), cyclopropylboronic acid (160 mg, 1.86 mmol), copper acetate (169 mg, 0.93 mmol), 2,2'-bipyridine (146 mg, 0.93 mmol), and sodium carbonate (197 mg, 1.86 mmol) were dispersed in acetonitrile (6 mL). The reaction solution was heated to 709°C, and was stirred for reaction for 4 hours. The reaction solution was cooled to room temperature and concentrated under reduced pressure. The residue was purified by silica gel column chromatography to obtain target product 25a (204 mg, yield 86%).

MS m/z (ESI): 255.0, 257.0[M+1].

Step 2: 25a (52 mg, 0.20 mmol), tert-butyl 4(S)-2-(4-fluoro-3,5-dimethylphenyl)-4-methyl-3-(2-oxo-1*H*-imidazol-3-yl)-6,7-dihydro-4*H*-pyrazolo[4,3-c]pyridine-5-carboxylate (60 mg, 0.14 mmol), (1*S*,2*S*)-1-*N*,2-*N*-dimethylcyclohexane-1,2-diamine (19 mg, 0.14 mmol), potassium carbonate (19 mg, 0.14 mmol), and cuprous iodide (26 mg, 0.14 mmol) were dispersed in *N*-methylpyrrolidone (2 mL). The reaction solution was heated to 130°C and stirred for reaction for 4 hours. The reaction solution was cooled to room temperature, and saturated brine (5 mL) was added thereto to quench the reaction. The reaction solution was extracted with ethyl acetate (5 mL × 3). The organic phases were merged, washed with saturated brine (20 mL × 5), dried over anhydrous sodium sulfate, and concentrated under reduced pressure. The residue was purified by flash silica gel chromatography to obtain target product 25b (55 mg, yield: 62%).

MS m/z (ESI): 616.3[M+1].

Step 3: 25b (55 mg, 89.33 μmol) and trifluoroacetic acid (0.5 mL) were dissolved in dichloromethane (2 mL) and stirred at room temperature for 2 hours. The reaction solution was concentrated under reduced pressure to obtain target product 25c (42 mg, yield: 91%).

MS m/z (ESI): 516.2[M+1].

Step 4: 25c (42 mg, 81.5 μmol), intermediate A (51 mg, 0.122 mmol), HATU (46 mg, 0.122 mmol), and DIEA (53 mg, 0.407 mmol) were dissolved in DMF (3 mL) and stirred at room temperature for 3 hours. A saturated ammonium

chloride solution was added to the reaction solution to quench the reaction, and the reaction solution was extracted with ethyl acetate (5 mL × 3). The organic phases were merged, washed with saturated sodium chloride solution (10 mL), dried over anhydrous sodium sulfate, and filtered. The filtrate was concentrated under reduced pressure and the resulting residue was purified by silica gel column chromatography to obtain a target product (62 mg, yield: 84%).

MS m/z (ESI): 909.4[M+1].

$^1$H NMR (400 MHz, DMSO) δ 11.74 (s, 1H), 8.27 (s, 1H), 7.67 (d, 1H), 7.56-7.47 (m, 2H), 7.40 (d, 1H), 7.26 (d, 1H), 7.18 (d, 2H), 7.07 (d, 1H), 6.95 (s, 2H), 5.58 (d, 1H), 4.38 (d, 1H), 3.85 (s, 1H), 3.71 (d, 2 H), 3.62 (d, 1H), 3.18 (t, 1H), 3.09-2.98 (m, 1H), 2.89 (d, 1H), 2.26 (s, 6H), 1.67 (d, 2H), 1.57 (d, 1H), 1.48 (d, 2H), 1.43 (s, 1H), 1.32 (s, 1H), 1.27 (s, 4H), 1.20-1.11 (m, 12H).

**Example 26**

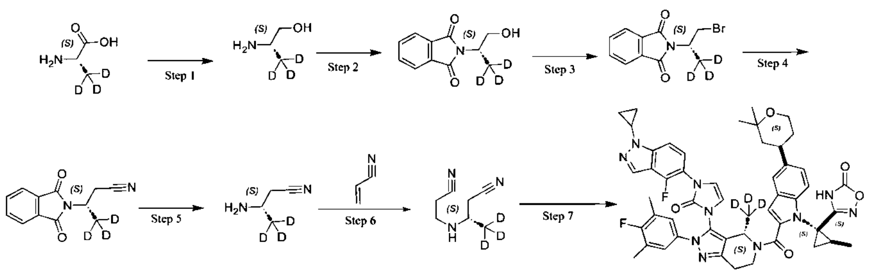


Step 1: L-alanine-3,3,3-d3 (10 g, 108.56 mmol) was dissolved in tetrahydrofuran (200 mL) in a 500 mL reaction bottle, then lithium aluminum hydride (4.12 g, 108.56 mmol) was added in batches at 0°C, and then the reaction solution was stirred at 0°C for 2 hours under nitrogen protection. The reaction was stopped, and an aqueous sodium hydroxide solution (1 M, 15 mL) was slowly added to quench the reaction. The mixture was filtered and concentrated to obtain a target product (8 g, yellow oily matter) in a yield of 94.3%.

Step 2: (*S*)-2-Aminopropane-3,3,3-d3-1-ol (8 g, 102.39 mmol), phthalic anhydride (15.17 g, 102.39 mmol), and triethylamine (10.36 g, 102.39 mmol) were dissolved in toluene (200 mL) in a 500 mL reaction bottle, and then the reaction solution was added into a water separator and stirred at 110°C for 12 hours under nitrogen protection. The reaction was stopped, and the reaction solution was concentrated and then recrystallized from ether to obtain a target product (17 g, yellow oily matter) in a yield of 79.7%.

MS m/z (ESI): 209.0[M+1].

Step 3: (*S*)-2-(1-Hydroxypropane-2-yl-3,3,3-d3)isoindoline-1,3-dione (17 g, 81.64 mmol) was dissolved in dichloromethane (200 mL) in a 500 mL reaction bottle, phosphorus tribromide (22.1 g, 81.64 mmol) was added at 25°C, and then the reaction solution was stirred at 25°C for 1 hour under nitrogen protection. The reaction was stopped, and an aqueous solution (100 mL) was added to quench the reaction. The reaction system was extracted with dichloromethane (100 mL × 2). The organic phases were merged, and washed with saturated sodium chloride (100 mL), dried over anhydrous sodium sulfate, and filtered and concentrated to obtain a target product (19 g, yellow solid) in a yield of 85.8%.

MS m/z (ESI): 271.0[M+1].

Step 4: (*S*)-2-(1-Bromopropan-2-yl-3,3,3-d3)isoindoline-1,3-dione (19 g, 70.08 mmol) was dissolved in DMSO (200 mL) in a 500 mL reaction bottle, and sodium cyanide (3.43 g, 70.08 mmol) and potassium iodide (1.16 g, 7.01 mmol) were added at 25°C, and then the reaction solution was stirred under nitrogen protection at 25°C for 12 hours. The reaction was stopped, and an aqueous solution (200 mL) was added to quench the reaction. The reaction system was extracted with dichloromethane (200 mL × 2), and the organic phases were merged, washed with saturated sodium chloride (200 mL), dried over anhydrous sodium sulfate, and filtered and concentrated, and then the resulting residue was purified by silica gel column chromatography with an eluent system of petroleum ether and ethyl acetate to obtain a target product (11 g, yellow solid) in a yield of 72.2%.

MS m/z (ESI): 218.0[M+1].

Step 5: (*S*)-3-(1,3-Dicarbonylisoindolin-2-yl)butyronitrile-4,4,4-d3 (11 g, 50.63 mmol) was dissolved in ethanol (100 mL) in a 250 mL reaction bottle, hydrazine hydrate (2.53 g, 50.63 mmol) was added at 25°C, and then the reaction solution was stirred at 50°C for 2 hours under nitrogen protection. The reaction was stopped, and the reaction system was cooled to room temperature, until a white solid precipitated. After filtering, the filtrate was concentrated and then 20 mL of ethanol was added for treatment, until a solid precipitated. After filtering again, the filtrate was concentrated to obtain a target product (4 g, yellow oily matter) in a yield of 90.6%.

Step 6: From (*S*)-3-aminobutyronitrile-4,4,4-d3 as a starting material, a target product was obtained by referring to the synthesis in step 1 of Example 516.

MS m/z (ESI): 141.0[M+1].

Step 7: From (*S*)-3-((2-cyanoethyl)amino)butyronitrile-4,4,4-d3 as a starting material, a target product was obtained by referring to the synthesis of intermediate B, intermediate E, and Example 1.

MS m/z (ESI): 912.4[M+1].

**Example 27**

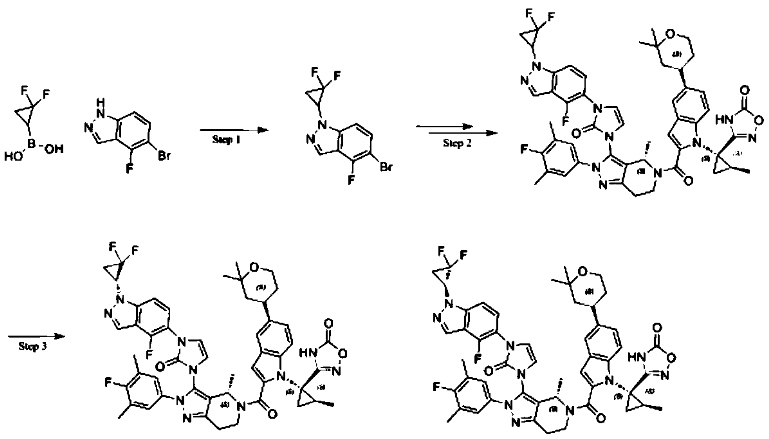


Step 1: In a 100 mL reaction bottle, 5-bromo-4-fluoro-1H-indazole (1 g, 4.65 mmol), (2,2-difluorocyclopropyl) boric acid (1.13 g, 9.30 mmol), copper acetate monohydrate (928.37 mg, 4.65 mmol), sodium carbonate (985.81 mg, 9.30 mmol), and 2,2'-bipyridine (726.37 mg, 4.65 mmol) were dissolved in 1,2-dichloroethane (25.0 mL), and the reaction solution was stirred at 25°C for 4 hours under nitrogen protection. The reaction was stopped, and a saturated aqueous ammonium chloride solution (60 mL) was added to quench the reaction. The reaction system was extracted with dichloromethane (60 mL × 2), the organic phases were merged and washed with saturated sodium chloride (60 mL × 1), dried over anhydrous sodium sulfate, and filtered, and the resulting residue was purified by silica gel column chromatography with an eluent system of petroleum ether and ethyl acetate to obtain a target product (700 mg, yellow solid) in a yield of 51.7%.
MS m/z (ESI): 291.0, 293.0[M+1]

Step 2: From 5-bromo-1-(2,2-difluorocyclopropyl)-4-fluoro-1H-indazole as a starting material, the title product was obtained by referring to the synthesis steps in Example 1.

MS m/z (ESI): 945.3[M+1].

Step 3: After chiral separation, 27-1 and 27-2 were obtained.

MS m/z (ESI): 945.3[M+1].

**Example 28**

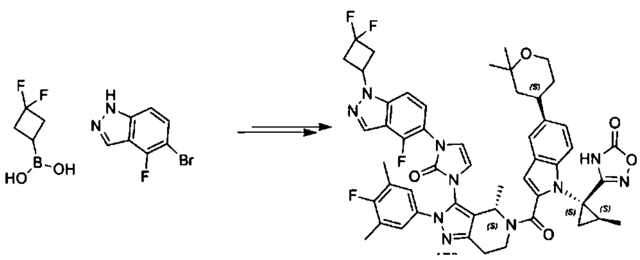

From 5-bromo-4-fluoro-1*H*-indazole and (3,3-difluorocyclobutyl)boric acid as starting materials, a target product was obtained by referring to the synthesis steps in Example 27.

MS m/z (ESI): 959.3[M+1].

**Example 29**

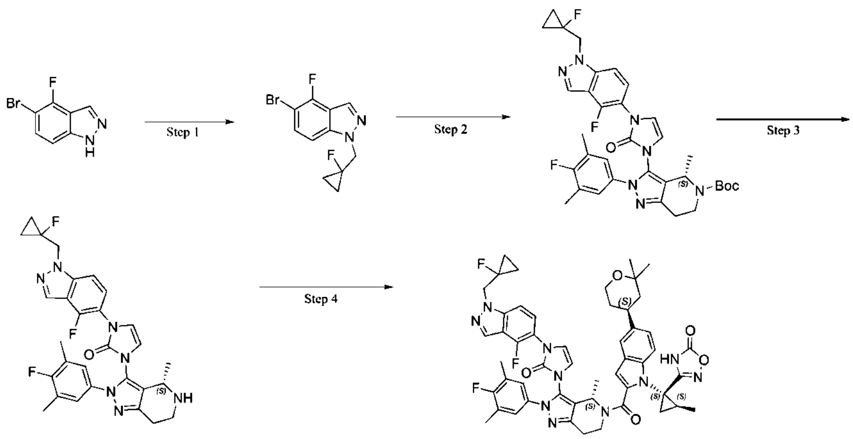


Step 1: In an ice bath, 5-bromo-4-fluoro-1*H*-indazole (0.43 g, 2 mmol) was dissolved in tetrahydrofuran (6 mL), the atmosphere was replaced with nitrogen, and then triphenylphosphine (0.79 g, 3 mmol) and diisopropyl azodicarboxylate (0.61 g, 3 mmol) were added in sequence. The mixture was stirred in an ice bath for 30 minutes. Finally, (1-fluorocyclopropyl)methanol was added and stirred at room temperature for 2 hours, until LCMS indicated that the reaction was completed. The reaction solution was quenched with a saturated ammonium chloride solution (10 mL), and then extracted with ethyl acetate (15 mL × 2). The organic phases were merged, dried over anhydrous sodium sulfate, filtered, and spun dry. The residue was separated by flash column chromatography to obtain a target product (0.29 g, yield 50%).
MS m/z (ESI): 287.0, 289.0[M+1].
Step 2: *tert*-Butyl (4*S*)-2-(4-fluoro-3,5-methyl-phenyl)-4-methyl-3-(2-carbonyl-1*H*-imidazol-3-yl)-6,7-dihydro-4*H*-pyrazolo[4,3-c]pyridine-5-carboxylate (50 mg, 0.11 mmol), 5-bromo-4-fluoro-1-((1-fluorocyclopropyl) methyl)-1*H*-indazole (37 mg, 0.13 mmol), cuprous iodide (11 mg, 0.06 mmol), potassium carbonate (47 mg, 0.33 mmol), and (1S,2S)-*N,N*-dimethyl-1,2-diaminocyclohexane (8 mg, 0.06 mmol) were dissolved in *N*-methylpyrrolidone (2 mL) at room temperature. The atmosphere was replaced with nitrogen. The solution was heated to 130°C for reaction for 5 hours, and cooled to room temperature, until LCMS indicated the reaction was completed. The reaction solution was diluted with ethyl acetate (15 mL) and filtered, and the filter residue was washed with ethyl acetate (10 mL). The organic phases were merged, washed with saturated brine (15 mL × 2), dried over anhydrous sodium sulfate, filtered, and spun dry. The residue was purified by thin layer preparative chromatography (dichloromethane: methanol = 20: 1) to obtain a target product as yellow oily matter (0.043 g, yield 60%).
MS m/z (ESI): 648.2[M+1].
Step 3: At room temperature, tert-butyl (*S*)-3-(3-(4-fluoro-1-((1-fluorocyclopropyl)methyl)-1*H*-indazol-5-yl)-2-carbonyl-2,3-dihydro-1H-imidazol-1-yl)-2-(4-fluoro-3,5-dimethylphenyl)-4-methyl-2,4,6,7-tetrahydro-5*H*-pyrazolo[4,3-c] pyridin-5-ylcarboxylate (43 mg, 0.07 mmol) was dissolved in dichloromethane (2 mL), and then a hydrochloric acid/-dioxane solution (4.0 M, 1 mL) was added and stirred at room temperature for 1 hour, until LCMS indicated that the reaction was completed. The mixture was spun dry and the residue was used directly in the next step (40 mg, crude product, yellow oily matter).
MS m/z (ESI): 548.2[M+1].
Step 4: At room temperature, (*S*)-1-(4-fluoro-1-((1-fluorocyclopropyl)methyl)-1H-indazol-5-yl)-3-(2-(4-fluoro-3,5-dimethylphenyl)-4-methyl-4,5,6,7-tetrahydro-2*H*-pyrazolo[4,3-c]pyridin-3-yl)-1,3-dihydro-2*H*-imidazol-2-one hydrochloride (40 mg, 0.07 mmol) was dissolved in *N,N*-dimethylformamide (3 mL), and then *N,N*-diisopropylethylamine (0.5 mL), 5-((*S*)-2,2-dimethyltetrahydro-2*H*-pyran-4-yl)-1-((1*S*,2*S*)-2-methyl-1-(5-carbonyl-4,5-dihydro-1,2,4-oxadiazol-3-yl)cyclopropyl)-1*H*-indole-2-carboxylic acid (29 mg, 0.07 mmol), and 2-(7-azobenzotriazole)-*N,N,N',N'*-tetramethyluronium hexafluorophosphate (38 mg, 0.1 mmol) were added in sequence, and stirred at room temperature for 14 hours, until LCMS indicated that the reaction was completed. The reaction solution was diluted with ethyl acetate (15 mL), and then washed with saturated brine (15 mL × 2). The organic phase was dried over anhydrous sodium sulfate, filtered, and spun dry. The residue was purified by reverse phase preparative chromatography and lyophilized to obtain a target compound (10 mg, yield 15.6%).
MS m/z (ESI): 941.3[M+1].

**Example 30**

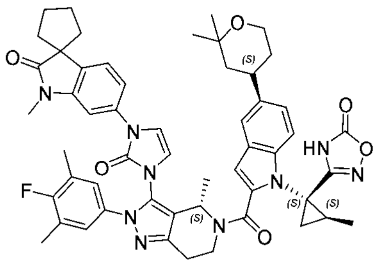

From 6-bromospiro[cyclopenta-1,3'-dihydroindole]-2'-one as a starting material, a product was obtained by referring to step 1 in Example 15 and step 2 to step 4 in Example 25.
MS m/z (ESI): 934.4[M+1].

**Example 31**

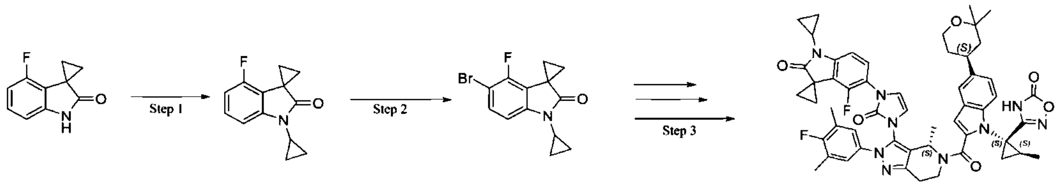


Step 1: At room temperature, 4'-fluorospiro[cyclopropane-1,3'-dihydroindole]-2'-one (0.35 g, 2 mmol), 2-(2-pyridyl)pyridine (0.47 g, 3 mmol), cyclopropylboronic acid (0.52 g, 6 mmol), copper acetate (0.54 g, 3 mmol), and sodium carbonate (0.64 g, 6 mmol) were dissolved in 1,2-dichloroethane (10 mL). The atmosphere was replaced with oxygen, and the solution was heated to 80°C for reaction for 14 hours, cooled to room temperature until LCMS indicated that the reaction was completed, filtered, and spun dry, and the residue was separated by flash column chromatography (PE: EA = 10: 1) to obtain a target product (0.15 g, yield: 35%).
MS m/z (ESI): 218.1[M+1].
Step 2: At room temperature, 1'-cyclopropyl-4-fluorospiro[cyclopropane-1,3'-dihydroindole]-2'-one (0.15 g, 0.7 mmol) was dissolved in *N,N*-dimethylformamide (5 mL), and then *N*-bromosuccinimide (0.12 g, 0.7 mmol) was added. The mixture was stirred at room temperature for 1 hour, until LCMS indicated that the reaction was completed. The reaction solution was diluted with ethyl acetate (20 mL), and then washed with saturated brine (15 mL × 2). The organic phase was dried over anhydrous sodium sulfate, filtered, and spun dry. The residue was separated by flash column chromatography (PE: EA = 10: 1) to obtain a target product (0.10 g, yield: 50%).
MS m/z (ESI): 296.0, 298.0[M+1].
Step 3: From 5'-bromo-1'-cyclopropyl-4'-fluorospiro[cyclopropane-1,3'-dihydroindole]-2'-one as a starting material, a target product was obtained by referring to step 2, step 3, and step 4 in Example 29.
MS m/z (ESI): 950.3[M+1].

**Example 32**

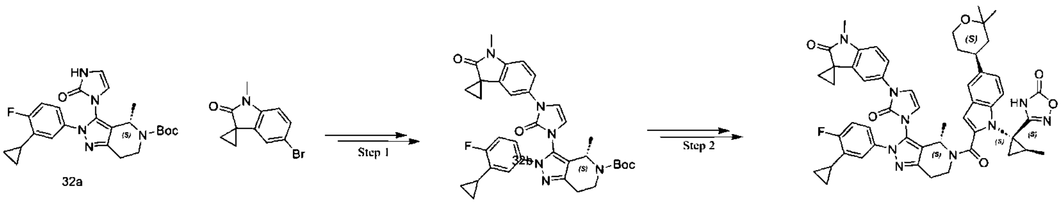


32a 32b 32

Step 1: From 32a and 5-bromo-1'-methylspiro[cyclopropane-1,3'-dihydroindole]-2'-one as a starting material, 32b was obtained by referring to the synthetic steps in Example 1.

MS m/z (ESI): 625.2[M+1].

Step 2: From 32b as a starting material, product 32 was obtained by referring to the synthesis of intermediate C in step 2 to step 3.

MS m/z (ESI): 918.4[M+1].

**Example 33**

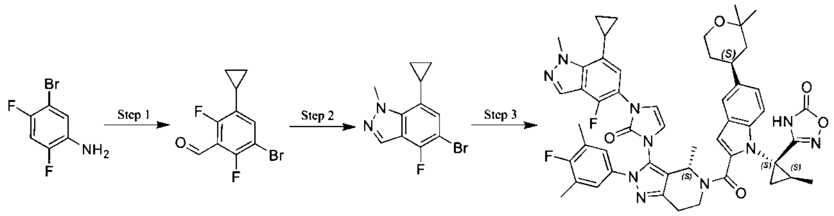


Step 1: From 5-bromo-2,4-difluoroaniline as a starting material, a target product was obtained by referring to WO2021/76890, 2021, A1, Page/Page column 163.

MS m/z (ESI): 261.0, 263.0[M+1].

Step 2: From 3-bromo-5-cyclopropyl-2,6-difluorobenzaldehyde as a starting material, a target product was obtained by referring to [Organic Letters, 2007, vol. 9, #3, p. 525-528].

MS m/z (ESI): 269.0, 271.0[M+1].

Step 3: From 5-bromo-7-cyclopropyl-4-fluoro-1-methyl-1*H*-indazole as a starting material, a target product was obtained by referring to Example 1.

MS m/z (ESI): 923.4[M+1].

**Example 34**

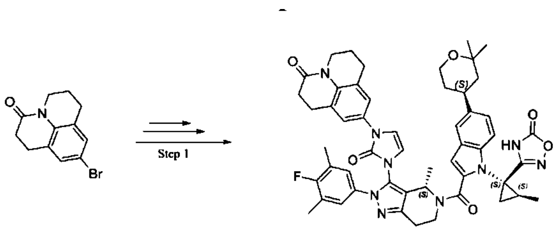


Step 1: From 9-bromo-2,3,6,7-tetrahydro-1*H*,5*H*-pyrido[3,2,1-i]quinolin-5-one as a starting material, a target product was obtained by referring to step 2, step 3, and step 4 in Example 29.

MS m/z (ESI): 920.3[M+1].

$^1$H NMR (400 MHz, DMSO-$d_6$) δ 11.73 (s, 1H), 7.51-7.45 (m, 1H), 7.41-7.31 (m, 3H), 7.27-7.03 (m, 4H), 6.94-6.90 (m , 1H), 6.87-6.70 (m, 1H), 5.62-5.50 (m, 1H), 4.43-4.30 (m, 1H), 3.73-3.57 (m, 5H), 3.24-3.11 (m, 1 H), 3.06-3.00 (m, 1H), 2.89-2.70 (m, 5H), 2.59-2.52 (m, 2H), *2.22 (br,* 6H), 1.85-1.75 (m, 2H), 1.73-1.58 ( m, 3H), 1.55-1.48 (m, 2H), 1.39-1.34 (m, 2H), 1.27 (s, 6H), 1.18-1.11 (m, 6H),

**Example 35**

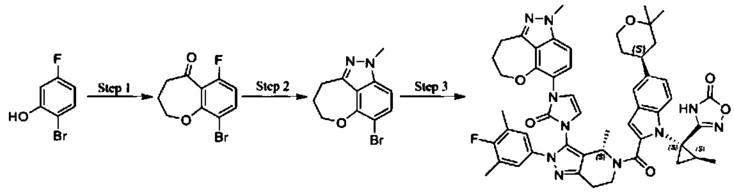


Step 1: From 2-bromo-5-fluorophenol as a starting material, a target product was obtained by referring to Bioorganic and Medicinal Chemistry Letters, 2004, vol. 14, #10, p. 2451-2457.

MS m/z (ESI): 259.0, 261.0[M+1].

Step 2: From 9-bromo-6-fluoro-3,4-dihydrobenzo[b]oxepin-5(2*H*)-one as a starting material, a target product was obtained by referring to step 2 in Example 33.

MS m/z (ESI): 267.0, 269.0[M+1].

Step 3: From 5-bromo-2-methyl-2,7,8,9-tetrahydrooxepin[4,3,2-cd]indazole as a starting material, a target product was obtained by referring to Example 1.

$^1H$ NMR (400 MHz, DMSO) δ [12.30 (s) and 11.77 (s), total 1 H], 7.53 (s, 1H), 7.41-7.32 (m, 1H), 7.27-7.11 (m, 5H), 7.00-6.60 (m, 3H), 5.79-5.18 (m, 1H), 4.66-4.14 (m, 2H), 4.04-3.93 (m, 3H), 3.75-3.59 (m, 3H), 3.23-2.81 (m , 5H), 2.26-2.07 (m, 8H), 1.83-1.40 (m, 10H), 1.33-1.02 (m, 10H).

MS m/z (ESI): 921.4[M+1].

**Example 36**

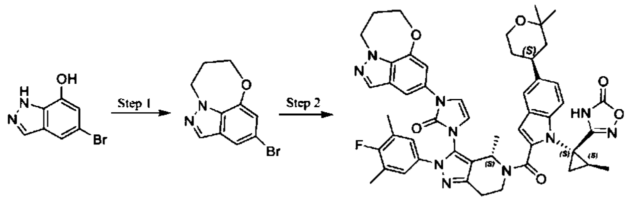


Step 1: From 5-bromo-1*H*-indazole-7-ol as a starting material, a target product was obtained by referring to NANJING SANHOME INVESTMENT GROUP CO LTD-EP3290419, 2018, A1, Paragraphs 0592; 0593; 0594.

MS m/z (ESI): 253.0, 255.0[M+1].

Step 2: From 9-bromo-3,4-dihydro-2*H*-[1,4]oxazepino[2,3,4-hi]indazole as a starting material, a target product was obtained by referring to Example 1.

MS m/z (ESI): 907.4[M+1].

**Example 37**

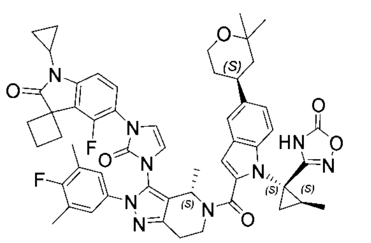

Referring to the synthesis method in Example 31, a target product was obtained from 4'-fluorospiro[cyclobutane-1,3'-dihydroindole]-2'-one.

MS m/z (ESI): 964.4[M+1].

**Example 38**

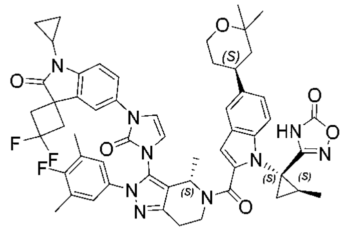

From 5'-bromo-3,3-difluorospiro[cyclobutane-1,3'-dihydroindole]-2'-one as a starting material, a target product was obtained by referring to Example 31.

MS m/z (ESI): 982.4[M+1].

**Example 39**

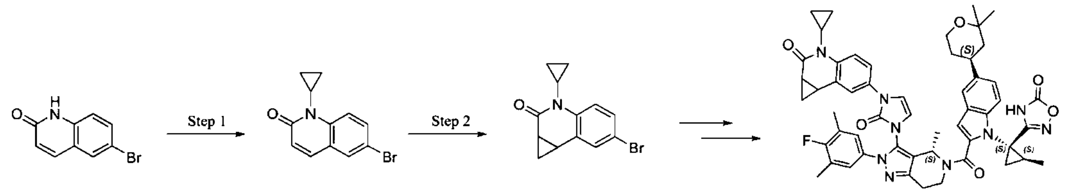


Step 1: A target product was obtained by referring to step 1 in Example 31.

MS m/z (ESI): 264.0, 266.0[M+1].

Step 2: Trimethylsulfoxide iodide (94 mg, 0.43 mmol) and NaH (20 mg, 0.84 mmol) were dispersed in DMSO (5 mL) and stirred at room temperature for 30 minutes. Subsequently, 6-bromo-1-cyclopropylquinolin-2(1*H*)-one (66 mg, 0.25 mmol) was added thereto, and the temperature of the reaction solution was raised to 80°C, and stirred for reaction for 18 hours. A saturated ammonium chloride solution was added to the reaction solution to quench the reaction, and the reaction solution was extracted with ethyl acetate (10 mL × 3). The organic phases were merged, and washed with a saturated sodium chloride solution (30 mL), dried over anhydrous sodium sulfate, and filtered. The filtrate was concentrated under reduced pressure and the resulting residue was purified by silica gel column chromatography to obtain the title product 6-bromo-3-cyclopropyl-1,1a,3,7b-tetrahydro-2*H*-cyclopropen[c]quinolin-2-one (42 mg, yield: 60%).
MS m/z (ESI): 278.0, 280.0[M+1]

Step 3: From 6-bromo-3-cyclopropyl-1,1a,3,7b-tetrahydro-2*H*-cyclopropene[c]quinolin-2-one as a starting material, a target product was obtained by referring to Example 25.

MS m/z (ESI): 932.4[M+1].

**Example 40**

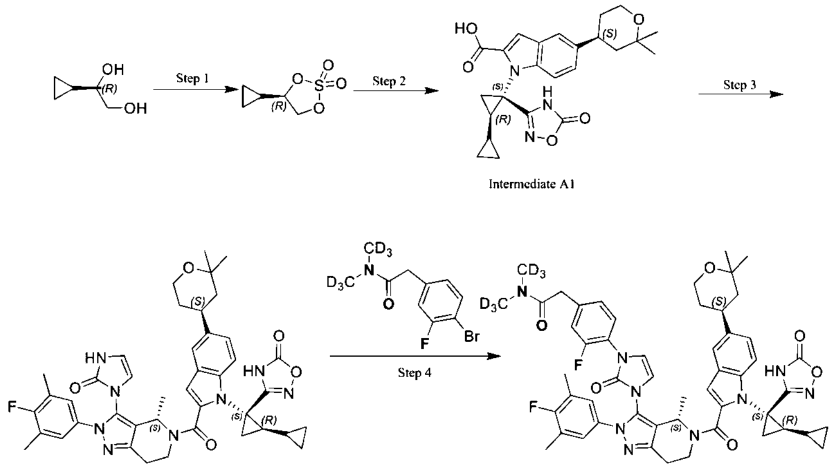

Step 1: (1*R*)-1-Cyclopropylethane-1,2-diol (1 g, 9.79 mmol) was dissolved in tetrahydrofuran (20 mL), and thionyl chloride (1.40 g, 11.75 mmol, 853.39 μL) was added under nitrogen protection and ice-water bath cooling. The mixture was stirred at 60°C for reaction for 1 hour. The reaction solution was evaporated to dryness to obtain a crude product. The crude product was dissolved in acetonitrile (15 mL) and water (15 mL). Sodium periodate (4.19 g, 19.58 mmol) and ruthenium trichloride (101.55 mg, 489.57 μmol) were added under ice-water bath cooling and nitrogen protection. The mixture was stirred at 25°C for reaction for 3 hours. The reaction solution was quenched with saturated brine (30 mL), the organic phase was separated, and the aqueous phase was extracted with dichloromethane (30 mL × 2). The organic phases were merged, and washed with saturated brine (30 mL × 2), dried over anhydrous sodium sulfate, and concentrated under reduced pressure to obtain a crude product (R)-4-cyclopropyl-1,3,2-dioxathiapiran 2,2-dioxide (1.3 g). The crude product was used directly in the next step.

MS m/z (ESI): 165.0[M+1].

Step 2: From (*R*)-4-cyclopropyl-1,3,2-dioxathiolane 2,2-dioxide as a starting material, intermediate A1 was synthesized by referring to step 3 to step 5 for intermediate A.

MS m/z (ESI): 438.2[M+1].

Step 3: From intermediate A1 as a starting material, intermediate E1 was synthesized by referring to intermediate E.

MS m/z (ESI): 761.4[M+1].

Step 4: From deuterated dimethylamine hydrochloride as a starting material, a target product was synthesized by referring to Example 7.

MS m/z (ESI): 946.5[M+1].

**Example 41**

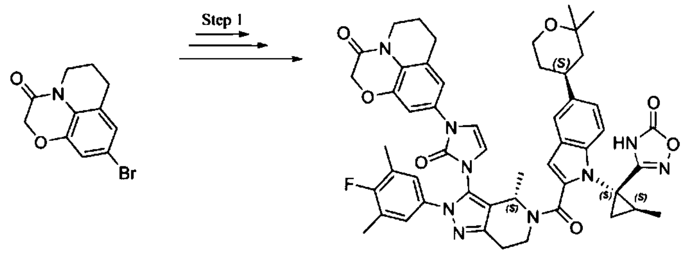


Step 1: From 9-bromo-6,7-dihydro-5*H*-[1,4]oxaazino[2,3,4-ij]quinolin-3(2*H*)-one as a starting material, a target compound was obtained by referring to Example 34.

$^1$H NMR (400 MHz, DMSO-$d_6$) δ [12.29 (s) and 11.74 (s), total 1H], 7.53-7.50 (m, 1H), 7.41-7.34 (m, 1H), 7.30-7.25 (m, 2H), 7.19-7.07 (m, 4H), 6.97-6.72 (m, 2H), [5.73-5.56 (m) and 5.33-5.19 (m), total 1H], 4.68-4.64 (m, 2H), 4.40-4.35 (m, 1H), 3.78-3.58 (m, 5H), 3.21-3.14 (m, 1H), 3.05-3.00 (m, 1H), 2.98- 2.65 (m, 3H), 2.22 (s, 6H), 1.95-1.90 (m, 2H), 1.80-1.49 (m, 7H), 1.38-1.27 (m, 7H), 1.18-1.14 (m, 5H).

MS m/z (ESI): 922.3[M+H]+.

**Example 42**

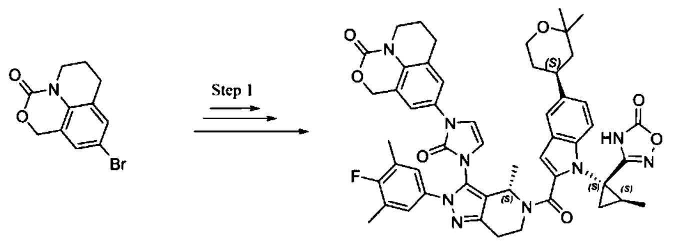


Step 1: From 9-bromo-6,7-dihydro-1*H*,3*H*,5*H*-[1,3]oxaazino[5,4,3-ij]quinolin-3-one as a starting material, a target compound was obtained by referring to Example 34.

MS m/z (ESI): 922.3[M+H]+.

**Example 43**

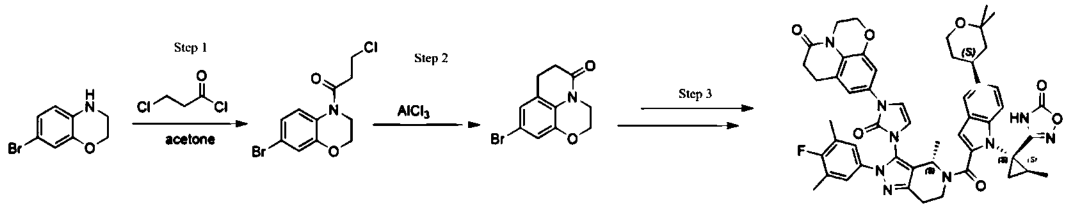


Step 1: 7-Bromo-3,4-dihydro-2*H*-benzo[b][1,4]oxazine (500 mg, 2.34 mmol) was dissolved in acetone (20 mL), and 3-chloropropionyl chloride (444.87 mg, 3.50 mmol) was added thereto. The reaction system was stirred in an oil bath at 60°C for 4 hours. After the reaction was completed, the reaction system was cooled to room temperature, and the excess solvent was concentrated. Water (10 mL) was added for dilution, and the reaction system was extracted with ethyl acetate (20 mL × 2), washed with saturated brine (20 mL × 2), dried over anhydrous sodium sulfate, and filtered and concentrated to obtain a crude product 1-(7-bromo-2,3-dihydro-4*H*-benzo[b][1,4]oxazin-4-yl)-3-chloropropane-1-one (700 mg, crude). The crude product was not further purified and was directly used in the next step.

MS m/z (ESI): 304.0, 306.0[M+H].

Step 2: 1-(7-Bromo-2,3-dihydro-4*H*-benzo[b][1,4]oxazin-4-yl)-3-chloropropane-1-one (700 mg, 2.30 mmol) was mixed with anhydrous aluminum chloride (459.70 mg, 3.45 mmol) in a one-necked bottle, and the mixture was placed in an oil bath at 120°C for 4 hours for melting stirring. After the reaction was completed, the reaction system was cooled to room temperature, diluted with water added (10 mL), extracted with dichloromethane (20 mL × 2), washed with saturated brine (20 mL × 2), dried over anhydrous sodium sulfate, and filtered and concentrated to obtain a crude product. The crude product was purified by column (petroleum ether: ethyl acetate = 5: 1, UV = 254 nm) to obtain 9-bromo-2,3,6,7-tetrahydro-5*H*-[1,4]oxaazino[2,3,4-ij]quinolin-5-one (300 mg, 1.12 mmol, 48.69% yield).

MS m/z (ESI): 267.9, 269.9[M+H]+.

Step 3: From 9-bromo-2,3,6,7-tetrahydro-5*H*-[1,4]oxaazino[2,3,4-i]quinolin-5-one as a starting material, a target compound was finally obtained by referring to Example 34.

$^1$H NMR (400 MHz, DMSO) δ [12.29 (s) and 11.74 (s), total 1H], 7.53 (s, 1H), 7.40 (d, 1H), 7.287.25 (m, 2H), 7.17-7.08 (m, 4H), 6.97-6.93 (m, 2H), 5.56-5.54 (q, 1H), 4.39-4.36 (m, 1H), 4.24-4.22 (m, 2H), 3.87-3.82 (m, 2H) ,3.73-3.70 (m, 2H), 3.62 (t, 1H), 3.18 (m, 1H), 3.06-2.98 (m, 1H), 2.95-2.86 (m, 3H), 2.60 (t, 2H), 2.22 (s, 6H), 1.78-1.49 (m, 7H), 1.36 (d, 2H), 1.27 (s, 3H), 1.24 (s, 2H), 1.18-1.13 (m, 5H).

MS m/z (ESI): 922.3[M+H]+.

**Example 44**

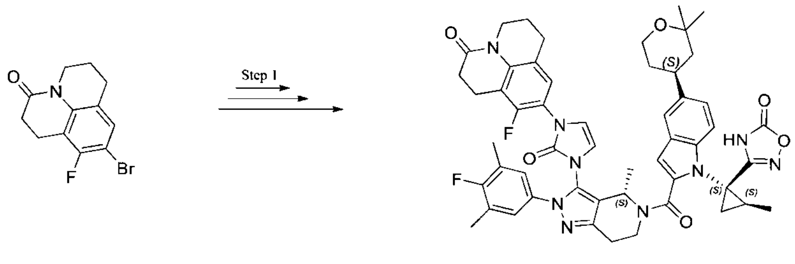


Step 1: From 9-bromo-8-fluoro-2,3,6,7-tetrahydro-1*H*,5*H* pyrido[3,2,1-ij]quinolin-5-one as a starting material, a target compound was obtained by referring to Example 34.

MS m/z (ESI): 938.3[M+H]+.

**Example 45**

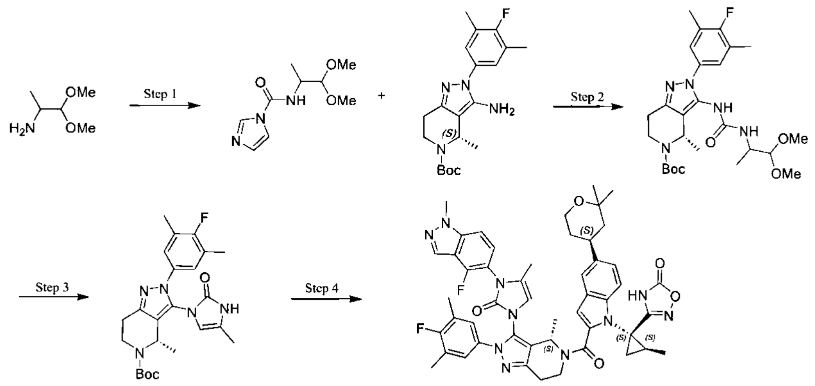


Step 1: 1,1-Dimethoxy-2-propylamine (500 mg, 4.20 mmol) and carbonyldiimidazole (748 mg, 4.62 mmol) were dissolved in THF (15 mL) and stirred at room temperature for reaction for 4 hours. A saturated ammonium chloride solution was added to the reaction solution for quenching. The reaction solution was extracted with ethyl acetate (20 mL × 3), and the organic phases were merged, washed with a saturated sodium chloride solution (30 mL), dried over anhydrous sodium sulfate, and filtered, and the filtrate was concentrated under reduced pressure. The resulting residue was purified by silica gel column chromatography to obtain a title product *N*-(1,1-dimethoxypropan-2-yl)-1/*H*-imidazole-1-carboxamide (742 mg, yield: 82.9%)

MS m/z (ESI): 214.1[M+1].

Step 2: *N*-(1,1-Dimethoxypropan-2-yl)-1*H*-imidazole-1-carboxamide (300 mg, 1.41 mmol), intermediate 2-4 (527 mg, 1.41 mmol), and potassium tert-butoxide (475 mg, 4.23 mmol) were dissolved in DMA (10 mL) and stirred at room temperature for reaction for 4 hours. A saturated ammonium chloride solution was added to the reaction solution for quenching. The reaction solution was extracted with ethyl acetate (20 mL × 3), and the organic phases were merged, washed with a saturated sodium chloride solution (30 mL), dried over anhydrous sodium sulfate, and filtered, and the filtrate was concentrated under reduced pressure. The resulting residue was purified by silica gel column chromatography to obtain a product tert-butyl (4S)-3-(3-(1,1-dimethoxypropan-2-yl)ureido)-2-(4-fluoro-3,5-dimethylphenyl)-4-methyl-2,4,6,7-tetrahydro-5*H*-pyrazolo[4,3-c]pyridine-5-carboxylate (512 mg, yield: 70.0%).
MS m/z (ESI): 520.3[M+1]

Step 3: *tert*-Butyl (4*S*)-3-(3-(1,1-dimethoxypropan-2-yl)ureido)-2-(4-fluoro-3,5-dimethylphenyl)-4-methyl-2,4,6,7-tetrahydro-5*H*-pyrazolo[4,3-c]pyridine-5-carboxylate (512 mg, 0.985 mmol) was dissolved in a mixed solvent of methanesulfonic acid (1 mL) and DCM (10 mL), and stirred at room temperature for reaction for 2 hours. A saturated potassium phosphate solution (10 mL) and di-*tert*-butyl dicarbonate (430 mg, 1.97 mmol) were added to the reaction solution, and stirred at room temperature for reaction for 1 hour. The reaction system was extracted with ethyl acetate (20 mL × 3), and the organic phases were merged, washed with a saturated sodium chloride solution (30 mL), dried over anhydrous sodium sulfate, and filtered, and the filtrate was concentrated under reduced pressure. The resulting residue was purified by silica gel column chromatography to obtain a title product *tert-butyl* ((*S*)-2-(4-fluoro-3,5-dimethyl-phenyl)-4-methyl-3-(4-methyl-2-oxo-2,3-dihydro-1*H*-imidazol-1-yl)-2,4,6,7-tetrahydro-5*H*-pyrazolo[4,3-c]pyridine-5-carboxylate (344 mg, yield: 76.6%).

MS m/z (ESI): 456.2[M+1].

Step 4: A target product was obtained by referring to the synthesis method in step 3 to step 5 of Example 23.

MS m/z (ESI): 897.4[M+1].

**Example 46**

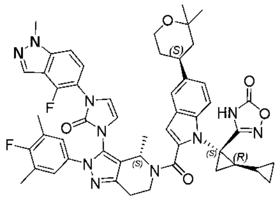

From 5-bromo-4-fluoro-1-methyl-1*H*-indazole and intermediate E1 as starting materials, a target compound was synthesized by referring to Example 1.

MS m/z (ESI): 909.4[M+1].

**Example 47**

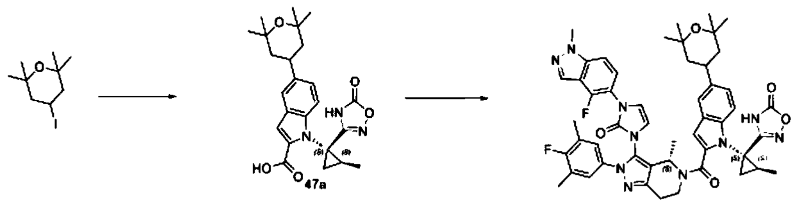

Step 1: Product 47a was obtained from 4-iodo-2,2,6,6-tetramethyltetrahydro-2*H*-pyran by referring to intermediate A.

MS m/z (ESI): 440.2[M+1].

Step 2: From 47a as a starting material, a product was obtained by referring to the synthesis method in step 5 of Example 23.

MS m/z (ESI): 911.4[M+1].

**Example 48**

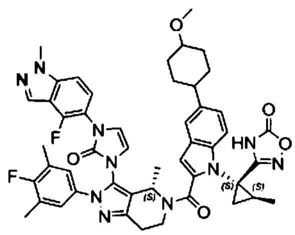

From 1-iodo-4-methoxycyclohexane as a starting material, a product was obtained by referring to the synthesis method in Example 47.

MS m/z (ESI): 883.4[M+1].

**Example 49**

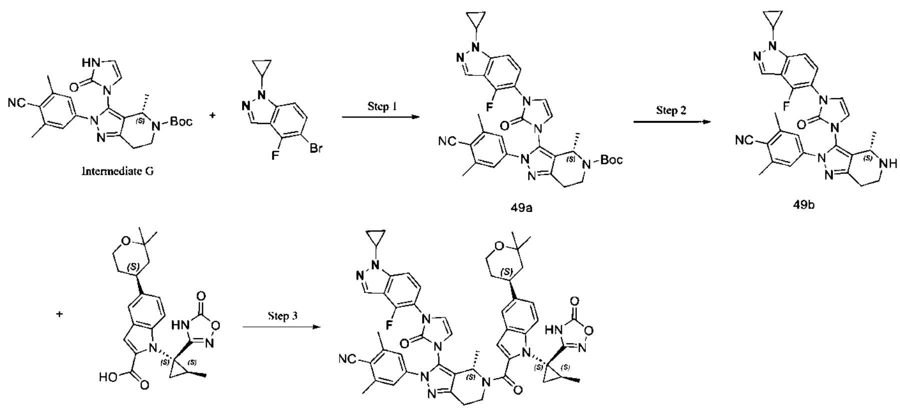


Step 1: Intermediate G (90 mg, 200.66 μmol), 5-bromo-1-cyclopropyl-4-fluoro-1*H*-indazole (56.30 mg, 220.73 μmol), CuI (38.22 mg, 200.66 μmol), potassium carbonate (83.20 mg, 601.98 μmol), and (1*S*,2*S*)-*N*,*N*'-dimethyl-1,2-cyclohexanediamine (28.54 mg, 200.66 μmol) were dispersed in NMP (6 mL), and the atmosphere was replaced with

nitrogen for protection. The reaction solution was heated to 130°C, and stirred for reaction for 3 hours.

The reaction solution was cooled to room temperature, and a saturated ammonium chloride solution was added to the reaction solution for quenching. The reaction system was extracted twice with ethyl acetate. The organic phases were merged, washed once with water and once with a saturated sodium chloride solution, dried over anhydrous sodium sulfate, and filtered, and the filtrate was concentrated under reduced pressure to obtain a yellow oily crude product. The crude product was purified by silica gel column chromatography (eluent: ethyl acetate: petroleum ether = 50%, v/v) to obtain 49a (92 mg, yield: 74%).

MS m/z (ESI): 623.3[M+1].

Step 2: 49a (92 mg, 147.75 μmol) was dissolved in a mixed solvent of HCl (4M in dioxane) (2 mL)/DCM (4 mL) and stirred at room temperature for reaction for 2 h. The reaction solution was concentrated under reduced pressure to obtain product 49b (74 mg, yield: 90%), which was used directly in the next step without purification.

MS m/z (ESI): 523.2[M+1].

Step 3: 49b (74 mg, 132.37 μmol), 5-((*S*)-2,2-dimethyltetrahydro-2*H*-pyran-4-yl)-1-((1*S*,2*S*)-2-methyl-1-(5-oxo-4,5-dihydro-1,2,4-oxadiazol-3-yl)cyclopropyl)-1*H*-indole-2-carboxylic acid (54.46 mg, 132.37 μmol), HATU (74.91 mg, 198.56 μmol), and DIEA (102.65 mg, 794.22 μmol, 138.34 μL) were dissolved in DMF (4 mL) and stirred at room temperature for 18 hours. The reaction solution was filtered through celite and sent for preparative HPLC purification to obtain a target product (81 mg, yield: 67%).

MS m/z (ESI): 916.4[M+1].

H-NMR (400 MHz, DMSO) δ 11.73 (s, 1H), 8.28 (s, 1H), 7.68 (d, 1H), 7.53 (s, 2H), 7.40 (d, 1H), 7.33 (s, 2H), 7.26 (d, 2H), 7.17 (s, 1H), 7.06 (s, 1H), 6.95 (s, 1H), 5.61 (d, 1H), 4.40 (d, 1H), 3.85 (brs, 1H) ), 3.71 (d, 3H), 3.63 (d, 1H), 3.03 (t, 1H), 2.93 (d, 1H), 1.84-1.47 (m, 8H), 1.43 (d, 3H), 1.38-1.22 ( m, 7H), 1.22-1.07 (m, 10H).

**Example 50**

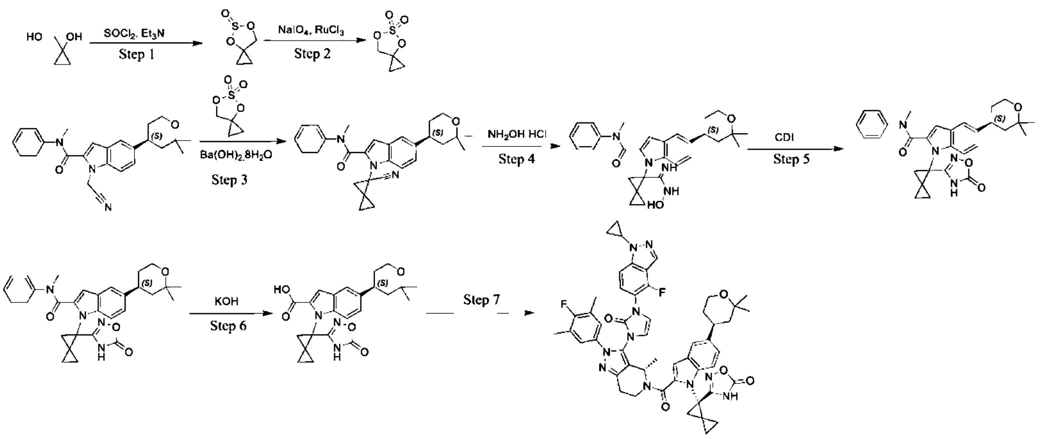


Step 1: To a mixture of 1-(hydroxymethyl)cyclopropane-1-ol (4.5 g, 51.08 mmol), triethylamine (15.51 g, 153.23 mmol, 21.37 mL), and carbon tetrachloride (100 mL) at 0°C, thionyl chloride (9.11 g, 76.61 mmol, 5.56 mL) was added dropwise, and stirred at 0°C for 1.5 hours. Water was added, and the reaction system was extracted with DCM three times. The organic phase was washed with saturated brine, dried over anhydrous sodium sulfate, and filtered, and the filtrate was concentrated under reduced pressure to dryness to obtain light yellow oily matter 4,6-dioxa-5-thiaspiro[2.4]heptane 5-oxide (6 g, crude product)

Step 2: To a solution of 4,6-dioxa-5-thiaspiro[2.4]heptane 5-oxide (6 g, 44.72 mmol) in carbon tetrachloride (50 mL), acetonitrile (50 mL), and water (60 mL) at 0°C, sodium periodate (14.35 g, 67.09 mmol) and hydrated ruthenium trichloride (504.15 mg, 2.24 mmol) were added, and stirred at room temperature for 1.5 hours. Water was added, and the reaction system was extracted with DCM three times. The organic phase was washed with saturated brine, dried over anhydrous sodium sulfate, and filtered, and the filtrate was concentrated to dryness under reduced pressure. The filtrate was separated by column chromatography (PE/EA = 4: 1) to obtain colorless oily matter 4,6-dioxa-5-thiaspiro[2.4]heptane 5,5-oxide (4 g, yield: 59.6%).

$^1H$ NMR (400 MHz, $CDCl_3$) δ 4.73 (s, 2H), 1.55-1.45 (m, 2H), 1.02-0.96 (m, 2H).

Step 3: (*S*)-1-(Cyanomethyl)-5-(2,2-dimethyltetrahydro-2*H*-pyran-4-yl)-*N*-methyl-*N*-phenyl-1*H*-indole-2-carboxamide (200 mg, 0.5 mmol) and barium hydroxide octahydrate (786 mg, 0.25 mmol) were added to a reaction bottle, and a solution of 4,6-dioxa-5-thiaspiro[2.4]heptane 5,5-dioxide (374 mg, 0.25 mmol) in *N,N*-dimethylpropylene urea (8

mL) was added under stirring at room temperature. After the addition was completed, the system was stirred at room temperature for 1.5 hours and then heated to 120°C for reaction for 1.5 hours. The heating was stopped, and a sample was taken for dilution with acetonitrile, and filtered and then sent for LCMS analysis. A signal peak of the target product was visible. The system was diluted with an appropriate amount of acetonitrile and filtered. The system was directly sent for preparative liquid separation. After freeze-drying, a white solid 1-(1-cyanospiro[2.2]pentan-1-yl)-5-((*S*)-2,2-dimethyltetrahydro-2*H*-pyran-4-yl)-*N*-methyl-*N*-phenyl-1*H*-indole-2-carboxamide (21 mg, yield: 9.2%) was obtained.

MS m/z (ESI): 454.2[M+1].

Step 4: A mixture of 1-(1-cyanospiro[2.2]pentan-1-yl)-5-((*S*)-2,2-dimethyltetrahydro-2*H*-pyran-4-yl)-*N*-methyl-*N*-phenyl-1*H*-indole-2-carboxamide (80 mg, 176.38 μmol), hydroxylamine hydrochloride (28.00 mg, 402.94 μmol), triethylamine (57.11 mg, 564.41 μmol), and ethanol (5 mL) was stirred at 85°C for reaction for 12 hours, until a white solid precipitated. LCMS showed that the reaction was complete and MS of a target product appeared. The reaction system was cooled, and water was added thereto. The reaction system was extracted with EtOAc, and the organic phase was washed with saturated brine, dried over anhydrous sodium sulfate, and filtered to obtain light yellow oily matter 5-((*S*)-2,2-dimethyltetrahydro-2*H*-pyran-4-yl)-1-(1-(*N*-hydroxyaminomethylimidoyl)spiro[2.2]pentan-1-yl)-*N*-methyl-*N*-phenyl-1*H*-indole-2-carboxamide (80 mg, crude product).

MS m/z (ESI): 487.3[M+1].

Step 5: At room temperature, 5-((*S*)-2,2-dimethyltetrahydro-2*H*-pyran-4-yl)-1-(1-(*N*-hydroxyaminocarboximidoyl)spiro[2.2]pentan-1-yl)-*N*-methyl-*N*-phenyl-1*H*-indole-2-carboxamide (80 mg, 164.40 μmol) was dissolved in THF (3 mL), and then 1,1'-carbonyldiimidazole (53.32 mg, 328.81 μmol) and 1,8-diazabicyclo[5.4.0]undec-7-ene (75.09 mg, 493.21 μmol) were added in sequence. The atmosphere was replaced with nitrogen, and the mixture was heated to 55°C for reaction for 2 hours, until LCMS indicated that the reaction was completed. The reaction system was cooled to room temperature and spun dry, and the residue was dissolved in ethyl acetate, then washed with a saturated sodium bicarbonate solution and saturated brine in sequence. The organic phase was dried over anhydrous sodium sulfate, filtered, spun dry, and separated by column chromatography (DCM/MeOH = 10: 1) to obtain a white solid 5-((*S*)-2,2-dimethyltetrahydro-2*H*-pyran-4-yl)-*N*-methyl-1-(1-(5-carbonyl-4,5-dihydro-1,2,4-oxadiazol-3-yl)spiro[2.2]pentan-1-yl)-*N*-phenyl-1*H*-indole-2-carboxamide (80 mg, yield: 94.9%).

MS m/z (ESI): 513.2[M+1].

Step 6: A mixture of 5-((*S*)-2,2-dimethyltetrahydro-2*H*-pyran-4-yl)-*N*-methyl-1-(1-(5-carbonyl-4,5-dihydro-1,2,4-oxadiazol-3-yl)spiro[2.2]pentan-1-yl)-*N*-phenyl-1*H*-indole-2-carboxamide (80 mg, 156.07 μmol), potassium hydroxide (87.57 mg, 1.56 mmol), and polyethylene glycol (2 mL) was stirred at 165°C for 3 hours. The reaction system was cooled, and water was added thereto, and dilute hydrochloric acid was added to adjust to pH = 5. The reaction system was extracted with DCM, washed with saturated brine, dried over anhydrous sodium sulfate, filtered, concentrated, and subjected to preparative chromatography acidic separation to obtain a white solid 5-((*S*)-2,2-dimethyltetrahydro-2H-pyran-4-yl)-1-(1-(5-carbonyl-4,5-dihydro-1,2,4-oxadiazol-3-yl)spiro[2.2]pentan-1-yl)-1*H*-indole-2-carboxylic acid (18 mg, yield: 27.2%).

MS m/z (ESI): 424.2[M+1].

Step 7: To a solution of 5-((*S*)-2,2-dimethyltetrahydro-2*H*-pyran-4-yl)-1-(1-(5-carbonyl-4,5-dihydro-1,2,4-oxadiazol-3-yl)spiro[2.2]pentan-1-yl)-1*H*-indole-2-carboxylic acid (18 mg, 42.51 μmol), and 1-(1-cyclopropyl-4-fluoro-indazol-5-yl)-3-[(4*S*)-2-(4-fluoro-3,5-dimethyl-phenyl)-4-methyl-4,5,6,7-tetrahydropyrazolo[4,3-c]pyridin-3-yl]imidazol-2-one (26.30 mg, 51.01 μmol) in DMF (2 mL) were added HATU (32.07 mg, 85.01 μmol) and DIEA (16.48 mg, 127.52 μmol, 22.21 μL). The mixture was stirred at room temperature for 16 hours, until LCMS showed that the reaction was completed, and MS of a target product appeared. Water was added and the reaction system was extracted with DCM three times. The organic phase was washed with saturated brine, dried over anhydrous sodium sulfate, and filtered, and the filtrate was concentrated under reduced pressure to obtain light yellow oily matter, which was separated by preparative chromatography (acidic) to obtain a target product (13 mg, yield: 33.2%).

MS m/z (ESI): 921.4[M+1].

**Example 51**

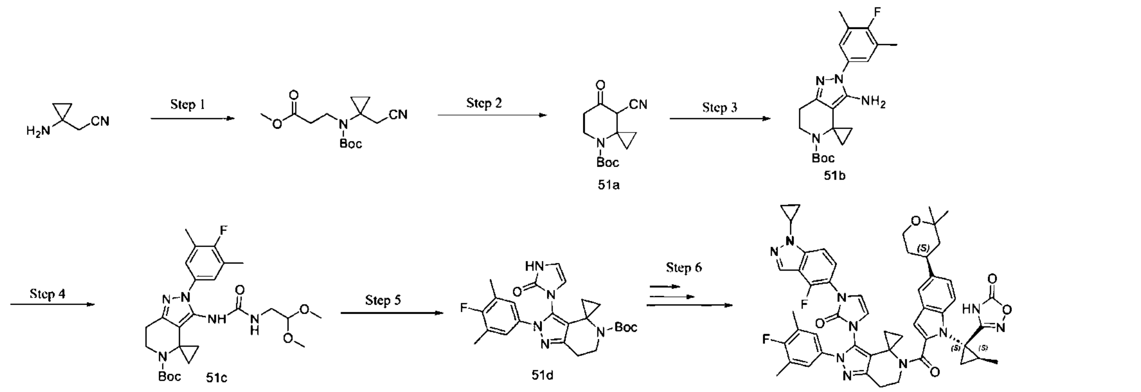


Step 1: At room temperature, 2-(1-aminocyclopropyl)acetonitrile (9.6 g, 0.1 mol) was dissolved in ethanol (100 mL), and then ethyl acrylate (12 g, 0.12 mol) and triethylamine (15.2 g, 0.15 mol) were added in sequence. The mixture was heated to 70°C and reacted for three hours. The mixture was then cooled to room temperature. N-methylpiperazine (3 g, 0.03 mol) and di-*tert*-butyl dicarbonate (26.2 g, 0.12 mol) were added to the reaction system, and stirred at room temperature overnight. After the reaction was completed, the solvent was removed under reduced pressure. The residue was dissolved in ethyl acetate (100 mL), and then washed with saturated brine (100 mL × 3). The organic phase was dried over anhydrous sodium sulfate, filtered, and spun dry to obtain a crude product, which was directly used in the next step.

MS m/z (ESI): 283.1[M+H]+.

Step 2: At room temperature, methyl 3-((*tert*-butoxycarbonyl)(1-(cyanomethyl)cyclopropyl)amino)propanoate (28.2 g, 0.1 mol) was dissolved in tetrahydrofuran (100 mL), and then potassium tert-butoxide (11.2 g, 0.1 mol) was added and stirred at room temperature for 2 hours. Dilute hydrochloric acid (2N, 80 mL) was then added to the reaction system and stirred at room temperature for 30 minutes, and then water (200 mL) was added to the reaction system and the reaction system was then extracted with ethyl acetate (100 mL × 3). The organic phases were merged and dried over anhydrous sodium sulfate, filtered, and spun dry. The crude product was separated by flash column chromatography to obtain target compound 51a (7.5 g, yield 30%).

MS m/z (ESI): 251.1[M+H]+.

Step 3: Di-*tert*-butyl-1-(4-fluoro-3,5-dimethylphenyl)hydrazine-1,2-dicarboxylate (2.58 g, 7.28 mmol) was dissolved in *N*-methylpyrrolidone (20 mL) at room temperature, and then methanesulfonic acid (1.40 g, 14.58 mmol) was added. The mixture was heated to 80°C and reacted for 12 hours, and then cooled to room temperature, and then the reactant was added into toluene (20 mL). The pH was then adjusted to 9 with an aqueous potassium carbonate solution. The reaction system was separated, and the organic phase was collected and dried over anhydrous sodium sulfate and filtered. 51a (1.82 g, 7.28 mmol) and pyridine hydrochloride (0.08 g, 0.73 mmol) were then added to the organic phase, and heated to 90°C for reaction for 1 hour. The reaction system was cooled to room temperature and the reaction solution was then poured into water (40 mL). The pH of the solution was then adjusted to 9 with an aqueous sodium hydroxide solution, and the solution was extracted with ethyl acetate (50 mL × 3). The organic phases were merged and dried over anhydrous sodium sulfate, filtered, and spun dry. The crude product was separated by flash column chromatography to obtain a target compound 51b (1.69 g, yield 60%).

MS m/z (ESI): 387.2[M+H]+.

Step 4: At room temperature, *N*-(2,2-dimethoxyethyl)imidazole-1-carboxamide (1.29 g, 6.47 mmol) and 51b (2.27 g, 5.88 mol) were dissolved in *N,N*-dimethylacetamide (30 mL), and potassium *tert*-butoxide (1.98 g, 17.65 mmol) was added at room temperature for reaction for 4 hours, and then the reaction solution was slowly poured into water (80 mL), and then extracted with ethyl acetate (50 mL × 3). The organic phases were merged, then washed with saturated brine (50 mL × 3), dried over anhydrous sodium sulfate, filtered, and spun dry. The residue was separated by flash column chromatography to obtain target compound 51c (1.22 g, yield 40%).

MS m/z (ESI): 518.3[M+H]+.

Step 5: At room temperature, 51c (20 mL) was added into methanesulfonic acid (0.18 g, 1.88 mmol), and heated to 60°C for reaction for 2 hours. The reaction system was cooled to room temperature, and then the pH value was adjusted to 9 with an aqueous potassium phosphate solution. Di-tert-butyl dicarbonate (0.51 g, 2.35 mmol) was then added and stirred at room temperature for 2 hours, until LCMS indicated that the reaction was completed. The reaction solution was then poured into water (40 mL), and then extracted with ethyl acetate (30 mL × 3). The organic phases were merged, washed with saturated brine (30 mL × 2), dried over anhydrous sodium sulfate, filtered, and spun dry. The crude product was separated by flash column chromatography to obtain target product 51d (0.53 g, yield 50%).

MS m/z (ESI): 454.2[M+H]+.

Step 6: From 51d and 5-bromo-1-cyclopropyl-4-fluoro-1/-indazole as starting materials, a target compound was

obtained by referring to step 2, step 3, and step 4 in Example 29.

MS m/z (ESI): 921.4[M+H]+.

**Example 52**

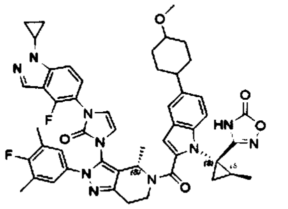

From 25c as a starting material, a product was obtained by referring to the synthesis method in Example 48.

MS m/z (ESI): 909.4[M+1].

**Example 53**

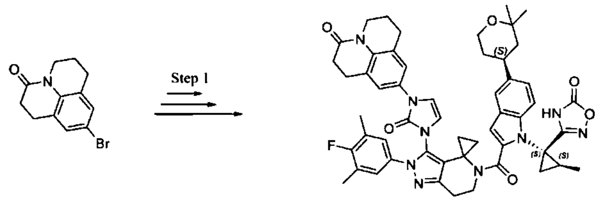


Step 1: From 9-bromo-2,3,6,7-tetrahydro-1*H*,5*H*-pyrido[3,2,1-ij]quinolin-5-one as a starting material, a target compound was obtained by referring to step 2, step 3, and step 4 of Example 29.
MS m/z (ESI): 932.3[M+H]+

**Example 54**

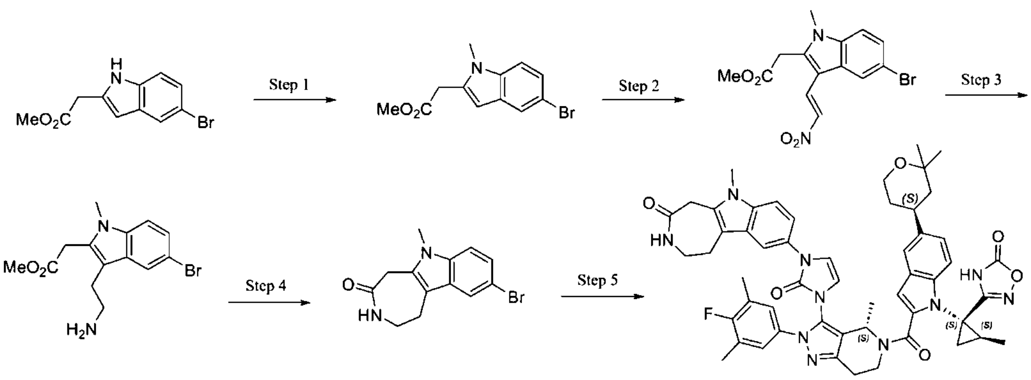


Step 1: Methyl 2-(5-bromo-1*H*-indol-2-yl)acetate (200 mg, 0.75 mmol), sodium hydride (36 mg, 1.49 mmol), and iodomethane (127 mg, 0.90 mmol) were dispersed in DMF (10 mL) and stirred at room temperature for 4 hours. A saturated ammonium chloride solution was added to the reaction solution for quenching. The reaction system was extracted with ethyl acetate (10 mL × 3), and the organic phases were merged, washed with saturated sodium chloride solution (30 mL), dried over anhydrous sodium sulfate, and filtered, and the filtrate was concentrated under reduced pressure. The resulting residue was purified by silica gel column chromatography to obtain a title product, 2-(5-bromo-1-methyl-1*H*-indol-2-yl) acetic acid methyl ester (174 mg, yield: 82.7%).

MS m/z (ESI): 282.0, 284.0[M+1].

Step 2: Methyl 2-(5-bromo-1-methyl-1*H*-indol-2-yl)acetate (174 mg, 0.62 mmol) and (E)-*N,N*-dimethyl-2-nitroethylene-1-amine (86 mg, 0.74 mmol) were dissolved in a mixed solvent of DCM (10 mL) and trifluoroacetic acid (5 mL), and stirred at room temperature for 2 hours. The reaction solution was concentrated under reduced pressure, the crude product was dissolved in dichloromethane, washed with a saturated sodium bicarbonate solution, dried over anhydrous sodium sulfate, and filtered, and the filtrate was concentrated under reduced pressure. The resulting residue was purified by silica gel column chromatography to obtain a product methyl (E)-methyl-2-(5-bromo-1-methyl-3-(2-nitrovinyl)-1*H*-indol-2-yl)acetate (186 mg, yield: 85.4%).

MS m/z (ESI): 353.0, 355.0[M+1].

Step 3: (E)-Methyl 2-(5-bromo-1-methyl-3-(2-nitrovinyl)-1*H*-indol-2-yl)acetate (186 mg, 0.53 mmol) and palladium carbon (19 mg, 10% wt.) were dispersed in ethanol (5 mL) and stirred at room temperature under a hydrogen balloon pressure for reaction for 5 hours. The reaction solution was filtered through celite, the filtrate was concentrated under reduced pressure, and the resulting residue was purified by silica gel column chromatography to obtain a product methyl 2-(3-(2-aminoethyl)-5-bromo-1-methyl-1*H*-indol-2-yl)acetate (151 mg, yield: 88.2%).
MS m/z (ESI): 325.0, 327.0[M+1]

Step 4: Methyl 2-(3-(2-aminoethyl)-5-bromo-1-methyl-1*H*-indol-2-yl)acetate (151 mg, 0.46 mmol) and *p*-toluenesulfonic acid (268 mg, 1.39 mmol) were dissolved in toluene (5 mL) and stirred at 80°C for 12 hours. The reaction solution was cooled to room temperature, and a saturated sodium bicarbonate solution was added for quenching. The reaction system was extracted with ethyl acetate (10 mL × 3), and the organic phases were merged, washed with a saturated sodium chloride solution (30 mL), dried over anhydrous sodium sulfate, and filtered, and the filtrate was concentrated under reduced pressure. The residue was purified by silica gel column chromatography to obtain a product 9-bromo-6-methyl-2,3,5,6-tetrahydroazepine[4,5-b]indol-4(1*H*)-one (92 mg, yield: 67.6%).

MS m/z (ESI): 293.0, 295.0[M+1].

Step 5: Referring to the synthesis method in step 3 to step 5 of Example 23, a product 3-((1*S*,2*S*)-1-(5-((S)-2,2-dimethyltetrahydro-2*H*-pyran-4-yl)-2-((*S*)-2-(4-fluoro-3,5-dimethylphenyl)-4-methyl-3-(3-(6-methyl-4-oxo-1,2,3,4,5,6-hexahydroazepine[4,5-b]indol-9-yl)-2-oxo-2,3-dihydro-1*H*-imidazol-1-yl)-4,5,6,7-tetrahydro-2*H*-pyrazolo[4,3-c]pyridine-5-carbonyl)-1*H*-indol-1-yl)-2-methylcyclopropyl)-1,2,4-oxadiazol-5(4*H*)-one was obtained.
MS m/z (ESI): 947.4[M+1]

**Example 55**

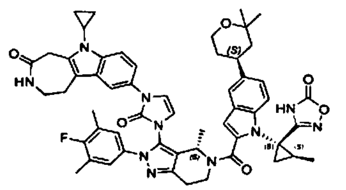

Referring to the synthetic method in Reference Example 54, a target product was obtained.

MS m/z (ESI): 973.4[M+1].

**Example 56**

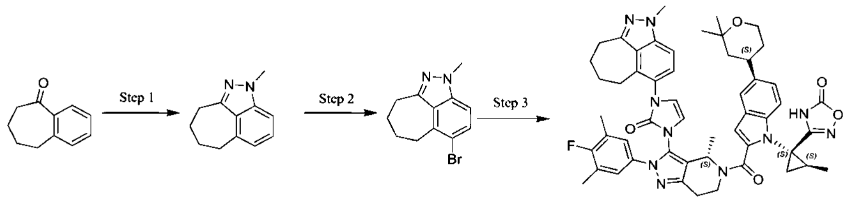


Step 1: Methylhydrazine hydrochloride (2.97 g, 24.97 mmol) was dissolved in *N,N*-dimethylformamide (30 mL), and potassium carbonate (1.73 g, 12.48 mmol) was added under nitrogen protection. The mixture was stirred at 25°C for reaction for 15 minutes. Then 6,7,8,9-tetrahydrobenzo[7]cycloalken-5-one (1 g, 6.24 mmol) and acetic acid (374.83 mg, 6.24 mmol), and 2,2,6,6-tetramethylpiperidinoxide (3.90 g, 24.97 mmol) and copper acetate (283.43 mg, 1.56 mmol) were

added in sequence. The mixture was stirred at 140°C for 48 hours. Most of the solvent was removed from the mixture under reduced pressure, saturated brine (100 mL) was added, and the mixture was extracted with ethyl acetate (50 mL × 2). The organic phases were merged, washed with saturated brine (50 mL × 2), dried over anhydrous sodium sulfate, and concentrated under reduced pressure. The residue was purified by flash silica gel chromatography (eluted with petroleum ether: ethyl acetate = 3: 1) to obtain a target product (0.26 g, yield: 22.36%).

MS m/z (ESI): 187.1[M+1].

Step 2: 2-Methyl-6,7,8,9-tetrahydro-2*H*-cycloheptatrien[cd]indazole (0.26 g, 1.40 mmol) was dissolved in dichloromethane (15 mL), and *N*-bromosuccinimide (NBS) (273.30 mg, 1.54 mmol) was added under nitrogen protection. The mixture was stirred at room temperature for 2 hours. Saturated brine (50 mL) was added to the mixture to quench the reaction. The organic phase was separated, washed with saturated brine (30 mL × 2), dried over anhydrous sodium sulfate, and concentrated under reduced pressure. The residue was purified by flash silica gel chromatography (eluted with petroleum ether: ethyl acetate = 100: 0 to 60: 40) to obtain a target product (0.35 g, yield: 94.56%).

MS m/z (ESI): 265.0, 267.0[M+1].

Step 3: Referring to Example 1, a target product was obtained.

MS m/z (ESI): 919.4[M+1].

**Example 57**

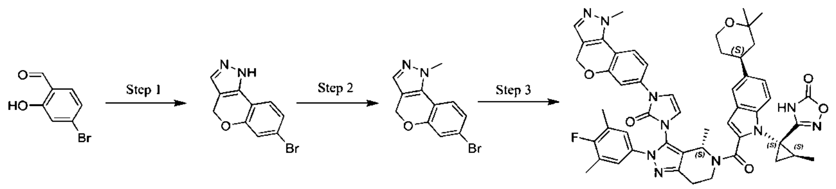


Step 1: From 4-bromo-2-hydroxybenzaldehyde as a starting material, a target product was synthesized by referring to Tetrahedron Letters, 2018, vol. 59, #15, p. 1501-1505.

MS m/z (ESI): 251.0, 253.0[M+1].

Step 2: 7-Bromo-1,4-dihydrochromeno[4,3-c]pyrazole (150 mg, 597.42 μmol) was dissolved in tetrahydrofuran (10 mL), and sodium hydroxide (28.67 mg, 716.91 μmol, 60% w/w) was added under ice-water bath cooling and nitrogen protection. The mixture was stirred at 0°C for 15 minutes. Iodomethane (127.20 mg, 896.13 μmol) was added. The mixture was stirred at 0°C for 1 hour. Saturated brine (50 mL) was added to the mixture to quench the reaction, and the mixture was extracted with ethyl acetate (50 mL). The organic phases were merged, washed with saturated brine (50 mL × 2), dried over anhydrous sodium sulfate, and concentrated under reduced pressure. The residue was purified by flash silica gel chromatography (eluted with petroleum ether: ethyl acetate = 100: 0 to 70: 30) to obtain a desired product (0.12 g, yield: 75.77%).

MS m/z (ESI): 265.0, 267.0[M+1].

Step 3: Referring to Example 1, a target product was obtained.

MS m/z (ESI): 919.4[M+1].

**Example 58**

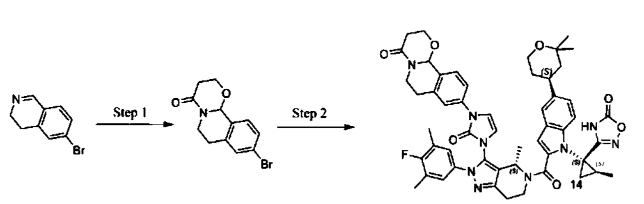


Step 1: From 6-bromo-3,4-dihydroisoquinoline as a starting material, a target product was synthesized by referring to Journal of Organic Chemistry, 2014, vol. 79, #3, p. 1368-1376.

MS m/z (ESI): 282.0, 284.0[M+1].

Step 2: Referring to Example 1, a target product was obtained.

MS m/z (ESI): 936.4[M+1].

**Example 59**

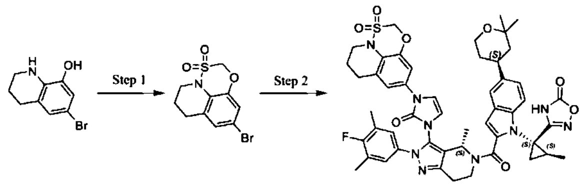


Step 1: From 6-bromo-1,2,3,4-tetrahydroquinolin-8-ol as a starting material, a target product was synthesized by referring to European Journal of Medicinal Chemistry, 2015, vol. 90, p. 788-796.
MS m/z (ESI): 304.0, 306.0[M+1].
Step 2: Referring to Example 1, a target product was obtained.
MS m/z (ESI): 958.4[M+1].

**Example 60**

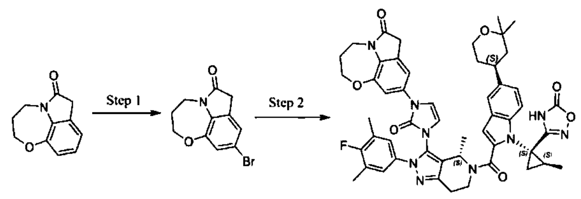


Step 1: Referring to step 2 of Example 56, a target compound was synthesized.
MS m/z (ESI): 268.0, 270.0[M+1].
Step 2: Referring to Example 1, a target compound was synthesized.
MS m/z (ESI): 922.4[M+1].

**Example 61**

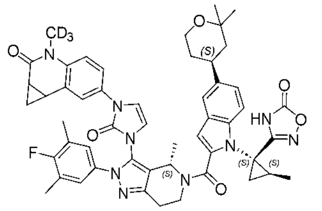

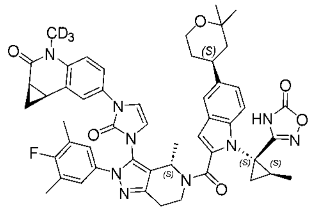

61-1

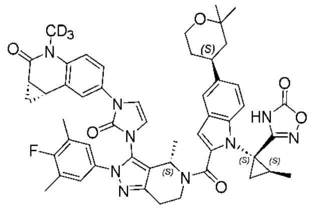

61-2

Referring to the synthesis method in Example 39, a product was obtained.
MS m/z (ESI): 909.4[M+1].
Example 61 was split to obtain 61-1 and 61-2.
MS m/z (ESI): 909.4[M+1].

**Example 62**

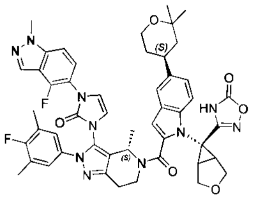
62

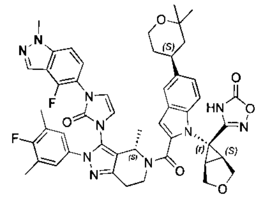
62-1

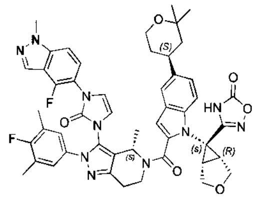
62-2

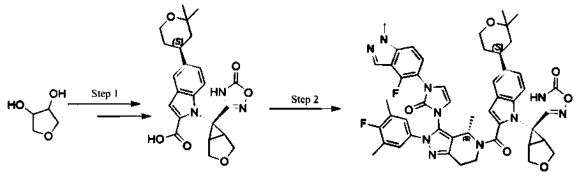


Step 1: From 3,4-dihydroxytetrahydrofuran as a starting material, a target compound was obtained by referring to Example 40.

MS m/z (ESI): 440.2[M+1].

Step 2: From 5-((*S*)-2,2-dimethyltetrahydro-2*H*-pyran-4-yl)-1-(6-(5-carbonyl-4,5-dihydro-1,2,4-oxadiazol-3-yl)-3-oxabicyclo[3.1.0]hexane-6-yl)-1*H*-indole-2-carboxylic acid as a starting material, a target product was obtained by referring to step 2, step 3, and step 4 of Example 29.

MS m/z (ESI): 911.4[M+1].

Step 3: 62 was split into 62-1 and 62-2.

MS m/z (ESI): 911.4[M+1].

**Example 63**

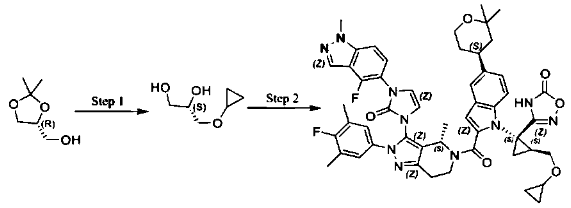


Step 1: (*R*)-(2,2-dimethyl-1,3-dioxolan-4-yl)methanol (1 g, 7.57 mmol) and cyclopropane bromide (1.01 g, 8.32 mmol) were dissolved in *N*,*N*-dimethylformamide (15 mL), and potassium carbonate (3.14 g, 22.70 mmol) was added under nitrogen protection. The mixture was stirred at 25°C for 12 hours. The reaction solution was quenched with saturated brine (50 mL), the organic phase was separated, and the aqueous phase was extracted with dichloromethane (30 mL × 2). The organic phases were merged, washed with saturated brine (30 mL × 5), dried over anhydrous sodium sulfate, and concentrated under reduced pressure to obtain a crude product. The crude product was dissolved in MeOH (15 mL), and *p*-toluenesulfonic acid monohydrate (0.144 g, 0.757 mmol) was added under nitrogen protection. The mixture was stirred at 25°C for 3 hours. The mixture was concentrated under reduced pressure. The residue was purified by flash silica gel chromatography (eluted with petroleum ether: ethyl acetate = 100: 0 to 10: 90) to obtain a desired product (0.3 g, yield: 30.00%).

MS m/z (ESI): 133.1[M+1].

Step 2: Referring to Example 40, a target product was obtained.

MS m/z (ESI): 939.4[M+1].

**Example 64**

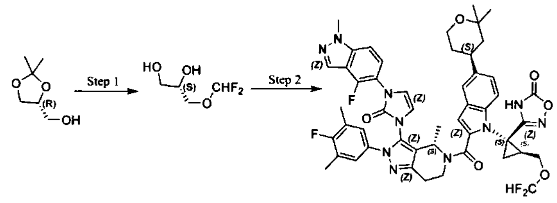


From (*R*)-(2,2-dimethyl-1,3-dioxolan-4-yl)methanol and difluoroiodomethane as starting materials, a target product was obtained by referring to Example 63.
MS m/z (ESI): 949.4[M+1].

**Example** 65

3-(4-(2-((*S*)-3-(3-(1-Cyclopropyl-4-fluoro-1*H*-indazol-5-yl)-2-carbonyl-2,3-dihydro-1*H*-imidazol-1-yl)-2-(4-fluoro-3,5-dimethylphenyl)-4-methyl-4,5,6,7-tetrahydro-2*H*-pyrazolo[4,3-c]pyridine-5-carbonyl)-5-((*S*)-2,2-dimethyltetrahydro-2H-ylpyran-4-yl)-1*H*-indol-1-yl)tetrahydro-2H-pyran-4-yl)-1,2,4-oxadiazol-5(4H)-one

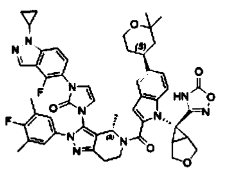
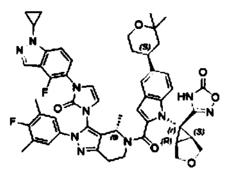
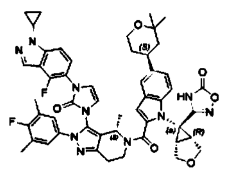

From 5-bromo-1-cyclopropyl-4-fluoro-1*H*-indazole as a starting material, a target product was obtained by referring to step 2, step 3, and step 4 of Example 29.
MS m/z (ESI): 937.4[M+1].
65 was split into 65-1 and 65-2.
MS m/z (ESI): 937.4[M+1].

**Example 65-1**

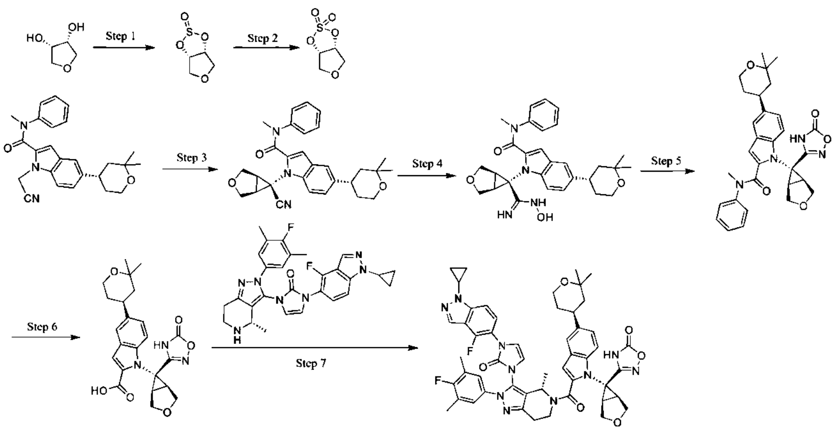


3-((3*R*,4*r*,5*S*)-4-(2-((*S*)-3-(3-(1-Cyclopropyl-4-fluoro-1*H*-indazol-5-yl)-2-carbonyl-2,3-dihydro-1*H*-imidazol-1-yl)-2-(4-fluoro-3,5-dimethylphenyl)-4-methyl-4,5,6,7-tetrahydro-2*H*-pyrazolo[4,3-c]pyridine-5-carbonyl)-5-((*S*)-2,2-dimethyltetrahydro-2*H*-pyran-4-yl)-1*H*-indol-1-yl)tetrahydro-2*H*-pyran-4-yl)-1,2,4-oxadiazol-5(4H)-one

Step 1: (3*R*,4*S*)-Tetrahydrofuran-3,4-diol (8 g, 76.85 mmol) and triethylamine (31.10 g, 307.38 mmol, 42.87 mL) were dissolved in dichloromethane (150 mL), and thionyl chloride (10.97 g, 92.22 mmol, 6.70 mL) was added under ice-water bath cooling and nitrogen protection. The mixture was stirred at 0°C for 1 hour. The reaction solution was washed

with saturated brine (130 mL × 2), and the organic phase was separated, dried over anhydrous sodium sulfate, and concentrated under reduced pressure to obtain a crude product (11.5 g). The crude product was used directly in the next step.

Step 2: (3a*R*,6a*S*)-Tetrahydrofurano[3,4-d][1,3,2]dioxathiazole 2-oxide (11.5 g, 76.59 mmol) was dissolved in dichloromethane (50 mL), acetonitrile (50 mL), and water (50 mL), and sodium periodate (32.76 g, 153.18 mmol) and ruthenium trichloride (794.34 mg, 3.83 mmol) were added under ice-water bath cooling and nitrogen protection. The mixture was stirred at 0°C for 2 hours. The mixture was filtered, the solid was washed with dichloromethane (50 mL), and the liquid phase was added to saturated brine (50 mL), and extracted with dichloromethane (50 mL × 2), and the organic phases were merged, washed with saturated brine (50 mL × 2), dried over anhydrous sodium sulfate, and concentrated under reduced pressure. The residue was purified by flash silica gel chromatography (eluted with petroleum ether: ethyl acetate = 100: 0 to 60: 40) to obtain a target product (3aR,6aS)-tetrahydrofuro[3,4-d][1,3,2]dioxathiazole 2,2-dioxide (4.8 g, 28.89 mmol, yield: 37.72%).

$^1$H NMR (400 MHz, $CDCl_3$) 5.455.33 (m, 2H), 4.42-4.28 (m, 2H), 3.81-3.68 (m, 2H).

Step 3: (*S*)-1-(Cyanomethyl)-5-(2,2-dimethyltetrahydro-2*H*-pyran-4-yl)-*N*-methyl-*N*-phenyl-1*H*-indole-2-carboxamide (0.65 g, 1.62 mmol) and (3a*R*,6a*S*)-tetrahydrofuro[3,4-d][1,3,2]dioxathiazole 2,2-dioxide (806.97 mg, 4.86 mmol) were dissolved in 1,3-dimethyl-3,4,5,6-tetrahydro-2-pyrimidinone (6 mL) and tetrahydrofuran (6 mL). Lithium diisopropylamide (2 M, 4.86 mL) was added at -40°C under nitrogen protection. The mixture was stirred at -40°C for 1 hour. Saturated brine (50 mL) was added to the mixture to quench the reaction, and the mixture was extracted with ethyl acetate (50 mL). The organic phases were merged, washed with saturated brine (50 mL), dried over anhydrous sodium sulfate, and concentrated under reduced pressure. The residue was purified by flash silica gel chromatography (eluted with petroleum ether: ethyl acetate = 100: 0 to 40: 60) to obtain a target product (0.4 g, 851.84 μmol, yield: 52.62%).

MS m/z (ESI): 470.2[M+1].

Step 4: 1-((1*R*,5*S*,6*R*)-6-Cyano-3-oxabicyclo[3.1.0]hexan-6-yl)-5-((S)-2,2-dimethyltetrahydro-2*H*-pyran-4-yl)-*N*-methyl-*N*-phenyl-1*H*-indole-2-carboxamide (0.51 g, 1.09 mmol) and triethylamine (329.70 mg, 3.26 mmol, 454.45 μL) were dissolved in ethanol (10 mL), and hydroxylamine hydrochloride (150.95 mg, 2.17 mmol) was added under nitrogen protection. The mixture was stirred at 80°C for 12 hours. The reaction solution was evaporated to dryness and quenched with saturated brine (30 mL). The aqueous phase was extracted with ethyl acetate (30 mL × 2). The organic phase was separated, washed with saturated brine (30 mL × 2), dried over anhydrous sodium sulfate, and concentrated under reduced pressure to obtain a crude product (0.5 g). The crude product was used directly in the next step.

MS m/z (ESI): 503.3[M+1].

Step 5: 5-((*S*)-2,2-Dimethyltetrahydro-2H-pyran-4-yl)-1-((1*R*,5*S*,6*r*)-6-(*N*-hydroxyaminomethyliminoyl)-3-oxabicyclo[3.1.0]hexan-6-yl)-*N*-methyl-*N*-phenyl-1*H*-indole-2-carboxamide (0.5 g, 994.82 μmol) and 1,8-diazabicyclo[5.4.0]undec-7-ene (378.62 mg, 2.49 mmol, 371.20 μL) were dissolved in tetrahydrofuran (10 mL), and N,N-carbonyldiimidazole (322.62 mg, 1.99 mmol) was added under nitrogen protection. The mixture was stirred at 55°C for reaction for 2 hours. The reaction solution was cooled to room temperature, saturated brine (50 mL) was added to quench the reaction, and stirring was continued for 0.5 hours. The mixture was separated, the aqueous phase was extracted with ethyl acetate (50 mL), and the organic phases were merged, washed with saturated brine (50 mL), dried over anhydrous sodium sulfate, and concentrated under reduced pressure. The residue was purified by flash silica gel chromatography (eluted with dichloromethane: methanol = 100: 0 to 95: 5) to obtain a target product (0.33 g, 624.29 μmol, yield: 62.75%).

$^1$H NMR (400 MHz, $CDCl_3$) δ 10.95 (s, 1H), 7.62 (d, J= 8.5 Hz, 1H), 7.44-7.28 (m, 3H), 7.25-7.08 (m, 4H), 5.92 (s, 1H), 4.77-4.64 (m, 1H), 4.31-4.18 (m, 1H), 4.08-3.99 (m, 2H), 3.89-3.68 (m, 2H), 3.54 (s, 3H), 3.00-2.85 ( m, 1H), 2.70-2.54 (m, 2H), 1.76-1.48 (m, 4H), 1.29 (s, 3H), 1.24 (s, 3H).

MS m/z (ESI): 529.2[M+1].

Step 6: 5-((*S*)-2,2-Dimethyltetrahydro-2*H*-pyran-4-yl)-*N*-methyl-1-((1*R*,5*S*,6*r*)-6-(5-carbonyl-4,5-dihydro-1,2,4-oxadiazol-3-yl)-3-oxabicyclo[3.1.0]hexane-6-yl)-*N*-phenyl-1*H*-indole-2-carboxamide (60 mg, 113.51 μmol) was dissolved in polyethylene glycol 400 (1 mL), and potassium hydroxide (63.68 mg, 1.14 mmol) was added under nitrogen protection. The mixture was stirred at 160°C for reaction for 1 hour. The reaction solution was cooled and quenched with an aqueous 1N hydrochloric acid solution (1.5 mL). The aqueous phase was extracted with dichloromethane (30 mL × 2). The organic phases were merged, washed with saturated brine (30 mL × 2), dried over anhydrous sodium sulfate, and concentrated under reduced pressure to obtain a crude product. The crude product was used directly in the next step.

MS m/z (ESI): 440.2[M+1].

Step 7: 5-((*S*)-2,2-Dimethyltetrahydro-2*H*-pyran-4-yl)-1-((1*R*,5*S*,6*r*)-6-(5-carbonyl-4,5-dihydro-1,2,4-oxadiazol-3-yl)-3-oxabicyclo[3.1.0]hexan-6-yl)-1*H*-indole-2-carboxylic acid (19 mg, 43.23 μmol), (*S*)-1-(1-cyclopropyl-4-fluoro-1*H*-indazol-5-yl)-3-(2-( 4-fluoro-3,5-dimethylphenyl)-4-methyl-4,5,6,7-tetrahydro-2*H*-pyrazolo[4,3-*c*]pyridin-3-yl)-1,3-dihydro-2H-imidazol-2-one (17.83 mg, 34.59 μmol), and N,N-diisopropylethylamine (22.35 mg, 172.94 μmol, 30.12 μL) were dissolved in *N*,*N*-dimethylformamide (1.5 mL), and 2-(7-azabenzotriazole)-*N*,*N*,*N'*,*N'*-tetramethyluronium hexafluorophosphate (24.66 mg, 64.85 μmol) was added under nitrogen protection. The mixture was stirred at 15°C for 12 hours. The crude reaction solution was separated by preparative high-performance liquid chromatography to obtain

a product (4 mg, yield: 9.68%).

$^1$H NMR (400 MHz, DMSO) δ [12.17 (s) and 11.53 (s), total 1H], 7.73-7.49 (m, 1H), 7.487.35 (m, 1H), 7.27-7.18 (m, 1H) , 7.18-6.98 (m, 4H), 6.87-6.60 (m, 3H), [5.80-5.39 (m), 5.43-4.99 (m), total 1H], 4.43-3.90 (m, 5H), 3.90-3.47 (m, 5H), 3.15-2.61 (m, 7H), 2.29-2.06 (m, 8H), 1.71-1.26 (m, 7H), 1.21 (s, 3H), 1.12 (s, 3H).

MS m/z (ESI): 937.4[M+1].

**Example 66**

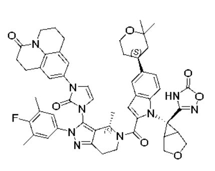 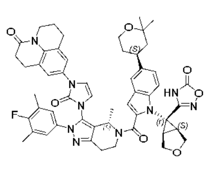 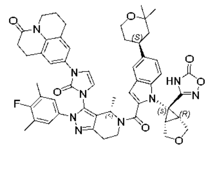

From 9-bromo-2,3,6,7-tetrahydro-1*H*,5*H*-pyrido[3,2,1-ij]quinolin-5-one as a starting material, a target product was obtained by referring step 2, step 3, and step 4 of Example 29.

MS m/z (ESI): 948.4[M+1].

**Example 67**

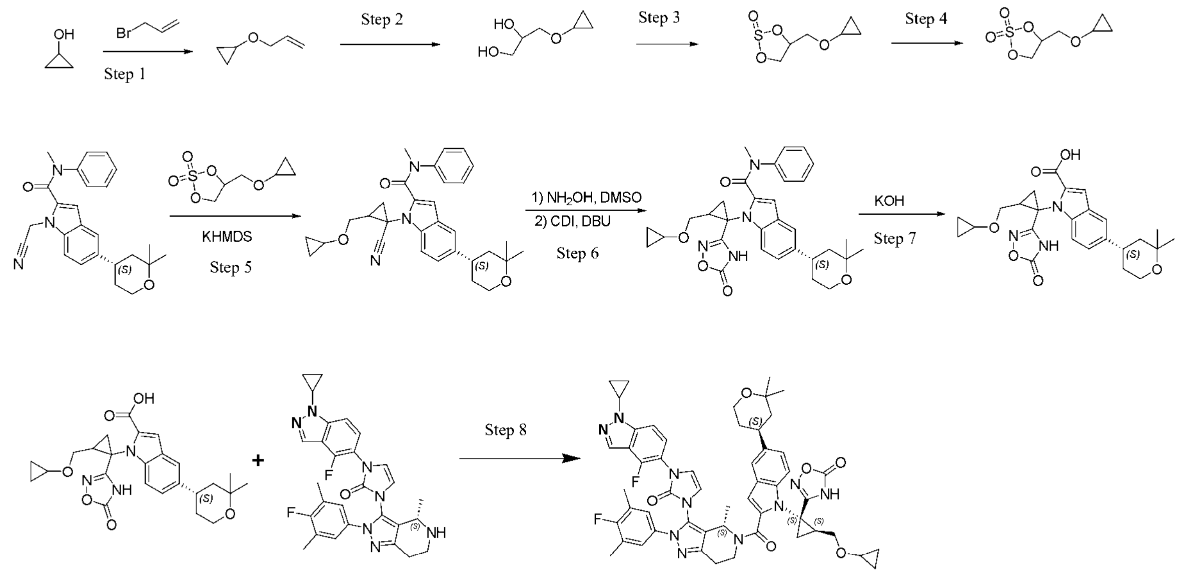


Step 1: 2-propanol (2g, 34.43mmol) and tetrahydrofuran (40mL) were added at 0-5°C. Under nitrogen protection, sodium hydrogen (2.76g, 68.88mmol, 60%) was added in batches. Stirring was performed for 35min in an ice water bath. Then, 3-bromoprop-1-ene (8.32g, 68.88mmol) was injected. Stirring was performed for 10min. Then, the ice water bath was removed and the temperature was naturally decreased to room temperature. Then, heating was performed for refluxing overnight. After the temperature of the reaction solution was decreased to room temperature, the reaction solution was poured into icy saturated ammonium chloride aqueous solution (40mL) for quenching, and was layered. Liquid separation was performed to obtain an organic phase, which was directly used for the next reaction step.

Step 2: water (55mL), potassium osmate dihydrate (1.26g, 3.44mmol), potassium ferrocyanide (33.6g, 10.32mmol) and potassium carbonate (14g, 10.32mmol) were added to the organic phase in the previous step. Stirring at room temperature overnight was performed. Sodium sulfite (5.3g, 4.30 mmol) was added to the reaction solution. Extraction with ethyl acetate, washing with a small amount of saturated ammonium chloride aqueous solution, washing with saturated saline solution, drying and concentration were performed to obtain an oily crude product. Column chromatography was performed to obtain a yellow oily substance 3-cyclopropoxypropane-1,2-diol (1.07g, yield: 23%).

MS m/z (ESI): 133.1[M+1].

Step 3: the temperature was controlled in the ice water bath. 3-cyclopropoxypropane-1,2-diol (876mg, 6.61mmol), carbon tetrachloride (20mL), triethylamine (2.01g, 19.82mmol) were added to a round necked flask. Thionyl chloride (1.18g, 9.91mmol) was added dropwise. Stirring was performed for reaction for 3.5h in the ice water bath. After TLC showed that the reaction was completed, water (15mL) was added to the reaction solution. Extraction with dichloromethane, washing with water, washing with saturated saline solution, drying and concentration were performed to obtain a black oily substance 4-(cyclopropoxymethyl)-1,3,2-dioxathiolane pentacyclo 2-oxide (1.22g, crude product). The crude product was directly used for the next reaction step.

Step 4: 4-(cyclopropoxymethyl)-1,3,2-dioxathiolane pentacyclo 2-oxide (1.18g, 6.61mmol), carbon tetrachloride (10mL), acetonitrile (10mL), water (10mL), sodium periodate (2.12g, 9.08mmol) and ruthenium trichloride hydrate (75mg, 302μmol) were added to a round necked flask in the ice water bath. Stirring was performed for 30min. Then, the temperature is naturally decreased room temperature for reaction overnight. After TLC showed that the reaction was completed, water (25mL) was added to the reaction solution. Extraction with dichloromethane, washing with water, washing with saturated saline solution, drying and concentration were performed to obtain an oily substance. Column chromatography was performed to obtain a colorless oily substance 4-(cyclopropoxymethyl)-1,3,2-dioxathiolane pentacyclo 2,2-dioxide (673mg, yield: 52.3%).

Step 5: (S)-1-(cyanomethyl)-5-(2,2-dimethyltetrahydro-2H-pyran-4-yl)-N-methyl-N-phenyl-1H-indol-2-carboxamide (130mg, 0.324mmol), N, N-dimethylacryloylurea (3mL) and 4-(cyclopropoxymethyl)-1,3,2-dioxathiolane pentacyclo-2,2-dioxide (188mg, 0.972mmol) were added to a three necked flask. Under nitrogen protection. potassium bis(trimethylsilyl)amide (1.3mmol, 1.3mL, 1M in THF) was added dropwise at 0°C. Stirring was performed for reaction for 3h at 0°C. After LCMS showed that the reaction was completed, formic acid was added to the reaction solution for quenching. Water was added. Extraction with ethyl acetate was performed. The organic phases were combined. Then, washing with water twice, washing with saturated NaHCO3 aqueous solution once, washing with saturated saline solution, drying and concentration were performed to obtain an oily substance. Column chromatography was performed to obtain 1-(1-cyano-2-(cyclopropoxymethyl)cyclopropyl)-5-(S)-2,2-dimethyltetrahydro-2H-pyran-4-yl)-N-methyl-N-phenyl-1H-indol-2-carboxamide (127mg, yield: 78.8%).

MS m/z (ESI): 498.3 [M+1].

Step 6: 1-(1-cyano-2-(cyclopropoxymethyl)cyclopropyl)-5-(S)-2,2-dimethyltetrahydro-2H-pyran-4-yl)-N-methyl-N-phenyl-1H-indol-2-carboxamide (127mg, 255.21μmol), ethanol (5mL), hydroxylamine hydrochloride (35.47mg, 510.42μmol) and triethylamine (77.47mg, 765.63μmol) were added to a round necked flask. Under nitrogen protection, heating to 90°C was performed for reaction for 2h. After LCMS showed that the reaction was completed, ethyl acetate (50mL) was added. Washing with water (10mL*2), washing with saturated saline solution (10mL*2), drying and concentration were performed to obtain an oily substance. The above oily substance was dissolved in THF (10mL). N,N'-carbonyldiimidazole (82.77mg, 510.42μmol) and 1,8-diazabicyclo[5.4.0]undec-7-ene (97.13mg, 638.03μmol) were added. Heating to 55°C was performed for reaction for 4h. After LCMS showed that the reaction was completed, ethyl acetate (30mL) was added. Washing with water (10mL*2), washing with saturated saline solution (10 mL*2), drying and concentration were performed to obtain an oily substance. Column chromatography was performed to obtain an oily substance 1-(2-(cyclopropoxymethyl)-1-(5-carbonyl-4,5-dihydro-1,2,4-oxadiazol-3-yl)cyclopropyl)-5-((S)-2,2-dimethyltetrahydro-2H-pyran-4-yl)-N-methyl-N-phenyl-1H-indol-2-carboxamide (90mg, yield: 63.3%).

MS m/z (ESI):557.3 [M+1].

Step 7: 1-(2-(cyclopropoxymethyl)-1-(5-carbonyl-4,5-dihydro-1,2,4-oxadiazol-3-yl)cyclopropyl)-5-((S)-2,2-dimethyltetrahydro-2H-pyran-4-yl)-N-methyl-N-phenyl-1H-indol-2-carboxamide (90mg, 161.68μmol), polyethylene glycol 400 (6mL) and potassium hydroxide (181.08mg, 3.23mmol) were added to a round necked flask. Heating externally to 165°C was performed for reaction for 3h. After LCMS showed that the reaction was completed, the temperature of the reaction solution was decreased to room temperature. Water (10mL) was added. Hydrochloric acid (6N) was added dropwise. Extraction with ethyl acetate was performed. Organic phases were combined. Then, washing with water, drying, concentration and reverse phase preparation were performed to obtain 1-(2-(cyclopropoxymethyl)-1-(5-carbonyl-4,5-dihydro-1,2,4-oxadiazol-3-yl)cyclopropyl)-5-((S)-2,2-dimethyltetrahydro-2H-pyran-4-yl)-1H-indol-2-carboxylic acid (12mg, yield: 15.9%).

MS m/z (ESI): 468.2[M+1].

Step 8: 2-(7-azobenzotriazole)-N,N,N',N'-tetramethylurea hexafluorophosphate (19.37mg, 51.34μmol) and N,N-diisopropylethylamine (9.95mg, 77.00μmol) were added to DMF (3mL) solution of (S)-1-(1-cyclopropyl-4-fluoro-1H-indazol-5-yl)-3-(2-(4-fluoro-3,5-dimethylphenyl)-4-methyl-4,5,6,7-tetrahydro-2H-pyrazolo[4,3-c]pyridin-3-yl)-1,3-dihydro-2H-imidazol-2-one (15.88mg, 30.80μmol), 1-(2-(cyclopropoxymethyl)-1-(5-carbonyl-4,5-dihydro-1,2,4-oxadiazol-3-yl)cyclopropyl)-5-(S)-2,2-dimethyltetrahydro-2H-pyran-4-yl)-1H-indol-2-carboxylic acid (12.00mg, 25.67μmol). Stirring was performed for 12h at room temperature. After LCMS showed that the reaction was completed and there was MS of a target product, water was added. Extraction with DCM was performed three times. The organic phases was washed with saturated saline solution. Drying with anhydrous sodium sulfate and filtration were performed. The filtrate was concentrated under reduced pressure to dryness. Preparative chromatography (acidic) separation was performed to

obtain 3-((1S,2S)-2-(cyclopropoxymethyl)-1-(2-(S)-3-(3-(1-cyclopropyl-4-fluoro-1H-indazol-5-yl)-2-carbonyl-2,3-dihydro-1H-imidazol-1-yl)-2-(4-fluoro-3,5-dimethylphenyl)-4-methyl-4,5,6,7-tetrahydro-2H-pyrazolo[4,3-c]pyridin-5-carbonyl)-5-((S)-2,2-dimethyltetrahydro-2H-pyran-4-yl)-1H-indol-1-yl)cyclopropyl)-1,2,4-oxadiazol-5(4H)-ketone (6.4mg, yield: 25.8%).

$^1$H NMR (400 MHz, DMSO) δ [12.27 (s) and 11.84 (s), total 1 H], 8.28-8.16(m, 1H), 7.76-7.60 (m, 1H), 7.65-7.35 (m, 3H), 7.28-7.09 (m, 4H), 6.96-6.92 (m, 1H), 6.76-6.54 (m, 1H), 5.78-5.55 (m, 1H), 5.40-5.20 (m, 1H), 4.81-4.38 (m, 1H), 4.00-3.63 (m, 4H), 3.19-2.67 (m, 4H), 2.26 (s, 6H),2.03-1.95 (m, 1H), 1.85-1.46 (m, 6H), 1.32-1.14 (m, 14H), 0.49-0.34 (m, 4H).

MS m/z (ESI): 965.4[M+1].

### Example 68

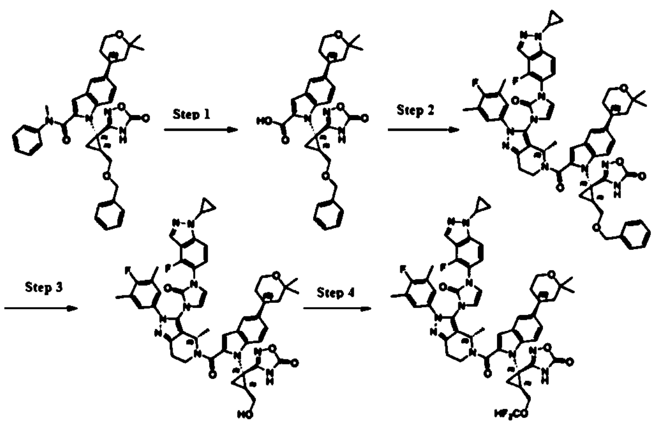


Step 1: a mixture of 1-((1S, 2S)-2-(benzyloxy) methyl)-1-(5-carbonyl-4,5-dihydro-1,2,4-oxadiazol-3-yl)cyclopropyl)-5-((S)-2,2-dimethyltetrahydro-2H-pyran-4-yl)-N-methyl-N-phenyl-1H-indol-2-carboxamide (350mg, 576.88μmol), KOH (323.69mg, 5.77mmol) and polyethylene glycol (4mL) was stirred for 3h at 165°C. The temperature was decreased to room temperature. After LCMS showed that the reaction was completed, the reaction solution was neutralized with dilute hydrochloric acid (1N). The pH was regulated to 5-6, and a solid precipitated. Filtration was performed. The solid was sequentially washed with water and petroleum ether, and dried to obtain a brown solid (290mg, crude product), which was directly used for the next step.

MS m/z (ESI):518.2 [M+1].

Step 2: HATU (145.78mg, 386.42μmol) and DIEA (74.91mg, 579.63μmol, 100.96μL) were added to DMF (5ml) solution of 1-(1-cyclopropyl-4-fluoroindol-5-yl)-3-[(4S)-2-(4-fluoro-3,5-dimethyl-phenyl)-4-methyl-4,5,6,7-tetrahydropyrazolo[4,3-c] pyridin-3-yl]imidazol-2-one (79.69mg, 154.57μmol) and 1-((1S,2S)-2-((benzyloxy) methyl)-1-(5-carbonyl-4,5-dihydro-1,2,4-oxadiazol-3-yl) cyclopropyl)-5-(S)-2,2-dimethyltetrahydro-2H-pyran-4-yl)-1H-indol-2-carboxylic acid (100mg, 193.21μmol) in DMF (5 mL). Stirring was performed for 12h at room temperature. Water was added. Extraction with DCM was performed three times. The organic phase was washed with saturated saline solution. Drying with anhydrous sodium sulfate and filtration were performed. The filtrate was concentrated under reduced pressure to obtain a light yellow oily substance. Separation with column chromatography (DCM/MeOH=10:1) was performed to obtain a white solid (120mg, yield: 61.2%).

MS m/z (ESI): 1015.4[M+1].

Step 3: 3-((1S, 2S)-2-(benzyloxy)methyl)-1-(2-(S)-3-(3-(1-cyclopropyl-4-fluoro-1H-indazol-5-yl)-2-carbonyl-2,3-dihydro-1H-imidazol-1-yl)-2-(4-fluoro-3,5-dimethylphenyl)-4-methyl-4,5,6,7-tetrahydro-2H-pyrazolo[4,3-c]pyridin-5-carbonyl)-5-((S)-2,2-dimethyltetrahydro-2H-pyran-4-yl)-1H-indol-1-yl)cyclopropyl)-1,2,4-oxadiazol-5(4H)-one (120mg, 0.12mmol) was dissolved in trifluoroacetic acid (2mL). The reaction solution was heated to 80°C. Stirring was performed for reaction for 3h. The reaction solution was cooled to room temperature. The reaction solution was concentrated under reduced pressure. The residue was dissolved in dichloromethane solution. Washing with saturated sodium bicarbonate solution was performed once. Drying with anhydrous sodium sulfate and filtration were performed. The filtrate was concentrated under reduced pressure to obtain a yellow oily product (80mg, crude product).

MS m/z (ESI): 925.4[M+1].

Step 4: potassium acetate (21.22mg, 216.22μmol) was added to DCM (1mL) and water (1mL) solution of 3-((1S, 2S)-1-(2-(S)-3-(3-(1-cyclopropyl-4-fluoro-1H-indazol-5-yl)-2-carbonyl-2,3-dihydro-1H-imidazol-1-yl)-2-(4-fluoro-3,5-dimethylphenyl)-4-methyl-4,5,6,7-tetrahydro-2H-pyrazolo[4,3-c]pyridin-5-carbonyl)-5-(S)-2,2-dimethyltetrahydro-2H-pyran-4-yl)-1H-indol-1-yl)-2-(hydroxymethyl)cyclopropyl)-1,2,4-oxadiazol-5(4H)-one (50mg, 54.05μmol) and [bromo(difluoro)methyl]-trimethyl-silane (43.05μmol, 216.22μmol). Stirring was performed for 1h at room temperature. Water was added. Extraction with DCM was performed three times. The organic phase was washed with saturated saline solution. Drying with anhydrous sodium sulfate and filtration were performed. The filtrate was concentrated under reduced pressure to dryness. Separation with preparative chromatography (acidic) was performed to obtain a target product (0.43mg, yield: 0.8%).

MS m/z (ESI): 975.4[M+1].

**Example 69**

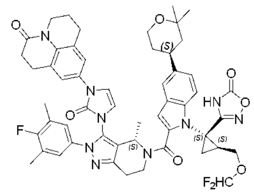

Using 9-bromo-2,3,6,7-tetrahydro-1H, 5H-pyrido[3,2,1-ij]quinolin-5-one was used as a starting material, and referring to Example 64, a target product was obtained.

MS m/z (ESI): 986.4[M+1].

**Example 70**

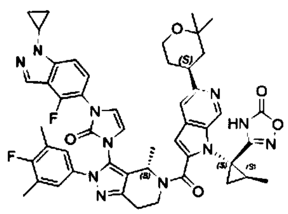

Referring to the synthesis method in Example 13, a target compound was obtained.

MS m/z (ESI): 910.4[M+1].

**Example 71**

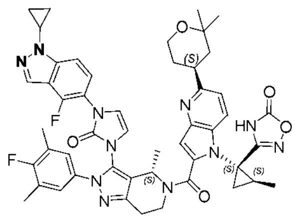

Referring to the synthesis method in Example 13, a target compound was obtained.

MS m/z (ESI): 910.4[M+1].

**Example 72**

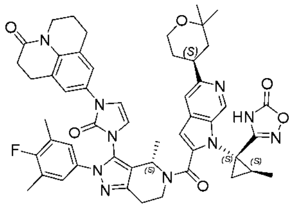

Referring to the synthesis method in Example 13, a target compound was obtained.
MS m/z (ESI): 921.4[M+1].

**Example 73**

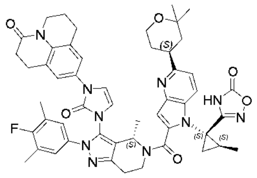

Referring to the synthesis method in Example 13, a target compound was obtained.
MS m/z (ESI): 921.4[M+1].

**Example 74**

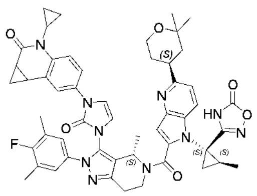

Referring to the synthesis method in Example 39, a target product was obtained.
MS m/z (ESI): 933.4[M+1].

**Example 75**

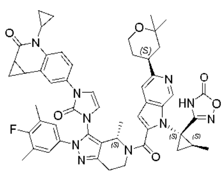

Referring to the synthesis method in Example 39, a target product was obtained.
MS m/z (ESI): 933.4[M+1].

**Example 76**

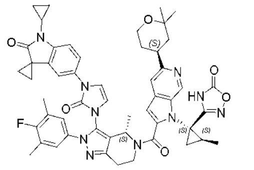

Referring to the synthesis method in Example 31, a target product was obtained.
MS m/z (ESI): 933.4[M+1].

**Example 77**

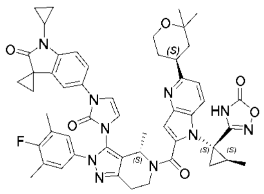

Referring to the synthesis method in Example 31, a target product was obtained.
MS m/z (ESI): 933.4[M+1].

**Example 78**

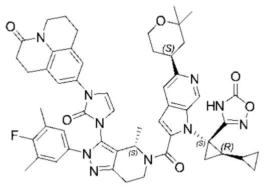

Using 9-bromo-2,3,6,7-tetrahydro-1H, 5H-pyridino[3,2,1-ij]quinolin-5-one as a raw material, and referring to Example 46, a target compound was obtained.
MS m/z (ESI): 947.3[M+H]+.

**Example 79**

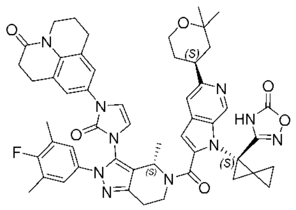

Using 9-bromo-2,3,6,7-tetrahydro-1H, 5H-pyridino[3,2,1-ij]quinolin-5-one as a raw material, and referring to Example 50, a target compound was obtained.
MS m/z (ESI):933.3[M+H]+.

**Example 80**

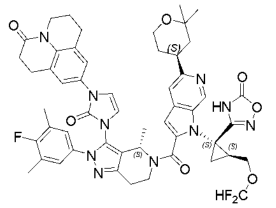

Using 9-bromo-2,3,6,7-tetrahydro-1H, 5H-pyridino[3,2,1-ij]quinolin-5-one as a raw material, and referring to Example 64, a target compound was obtained.
MS m/z (ESI):987.3[M+H]+.

### Example 81

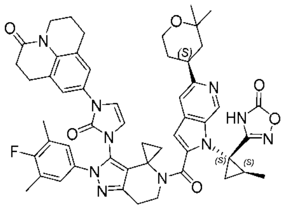

Using 9-bromo-2,3,6,7-tetrahydro-1H, 5H-pyridino[3,2,1-ij]quinolin-5-one as a raw material, and referring to Example 51, a target compound was obtained.
MS m/z (ESI):933.4[M+H]+.

### Example 82

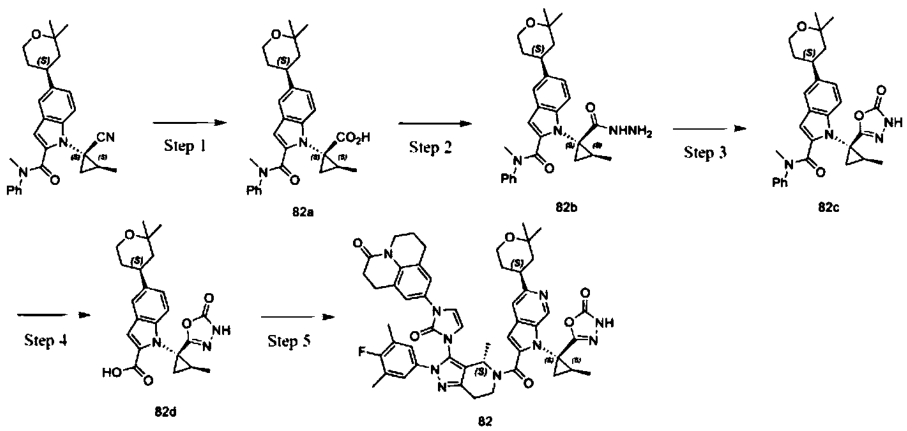


82a 82b 82c 82d 82

Step 1: 1-((1S, 2S)-1-cyano-2-methylcyclopropyl)-5-((S)-2,2-dimethyltetrahydro-2H-pyran-4-yl)-N-methyl-N-phenyl-1H-indol-2-carboxamide (300mg, 0.68mmol) and sodium hydroxide (136mg, 3.41mmol) were dissolved in a mixed solvent of ethanol (5mL) and water (5mL. Stirring was performed for reaction for 6h at 100°C. Hydrochloric acid was added to the reaction solution to regulate the pH to 2. Extraction with dichloromethane (10mL*3) was performed. The organic phases were combined. Drying with anhydrous sodium sulfate and filtration were performed. The filtrate was concentrated under reduced pressure. The obtained residue was purified by silica gel column chromatography to obtain a target product (235mg, yield: 75%).
MS m/z (ESI): 461.2[M+1].
Step 2: 82a (235mg, 0.51mmol), HATU (291mg, 0.77mmol), hydrazine hydrate (33mg, 1.0215mmol) and DIEA (196mg, 1.53mmol) were dissolved in DMF (10mL). Stirring was performed for reaction for 3h at room temperature. Saturated ammonium chloride solution was added to the reaction solution for quenching. Extraction with ethyl acetate (10mL*3) was performed. The organic phases were combined. Washing with saturated sodium chloride solution (30mL),

drying with anhydrous sodium sulfate and filtration were performed. The filtrate was concentrated under reduced pressure. The obtained residue was purified by silica gel column chromatography to obtain a target product (184mg, yield: 72%).

MS m/z (ESI): 475.3[M+1].

Step 3: 82b (184mg, 0.39mmol) and dichlorosulfoxide (51mg, 0.43mmol) were dissolved in toluene (5mL). Stirring was performed for reaction for 1h at 80°C. Saturated sodium bicarbonate solution was added to the reaction solution for quenching. Extraction with ethyl acetate (10mL*3) was performed. The organic phases were combined. Washing with saturated sodium chloride solution (30mL), drying with anhydrous sodium sulfate and filtration were performed. The filtrate was concentrated under reduced pressure. The obtained residue was purified by silica gel column chromatography to obtain a target product 82c (131 mg, yield: 68%).

MS m/z (ESI): 501.2[M+1].

Step 4: referring to the synthesis method of Intermediate A in step 5, a target product 82d was obtained.

MS m/z (ESI): 412.2[M+1].

Step 5: referring to the synthesis method in Example 34, a target product was obtained.

MS m/z (ESI): 921.4[M+1].

**Example 83**

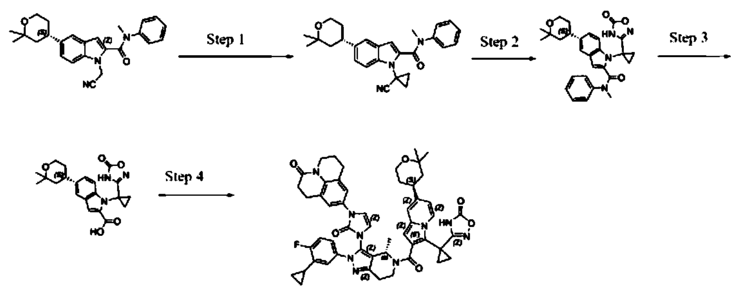


Step 1: under an ice bath, (S)-1-(cyanomethyl)-5-(2,2-dimethyltetrahydro-2H-pyran-4-yl)-N-methyl-N-phenyl-1H-indol-2-carboxamide (0.80g, 2mmol) was dissolved in N,N-dimethylformamide (12mL). Replacement with nitrogen gas was performed. Then, sodium hydrogen (0.24g, 6mmol, 60% in mineral oil) was added. Stirring was performed for 30min under the ice bath. Then, 1,2-dibromoethane (0.56g, 3mmol) was added. Stirring was performed for 2h at room temperature. After LCMS showed that the reaction was completed, the reaction solution was quenched with saturated ammonium chloride solution (20mL). Then, extraction with ethyl acetate (15mL*3) was performed. The organic phases were combined. The organic phase was washed with saturated saline solution (15mL*2), dried with anhydrous sodium sulfate, filtered, and spin-dried. The residue was separated by Flash column chromatography (PE: EA=10: 1-3:1 for elution) to obtain a white solid target product (0.34g, yield: 40%).

MS m/z (ESI):428.3[M+H]+.

Step 2: at room temperature, (S)-1-(1-cyanocyclopropyl)-5-(2,2-dimethyltetrahydro-2H-pyran-4-yl)-N-methyl-N-phenyl-1H-indol-2-carboxamide (0.34g, 0.8mmol) was dissolved in ethanol (5mL). Then, triethylamine (0.24g, 2.4mmol) and hydroxylamine hydrochloride (0.11g, 1.6mmol) were sequentially added. Replacement with nitrogen gas was performed. Heating to 90°C was performed for reaction for 3h. After LCMS showed that the reaction was completed, the temperature was decreased to room temperature. Spin-drying was performed. The residue was dissolved in ethyl acetate (15mL). Then, washing sequentially with saturated sodium bicarbonate solution (10mL*3) and saturated saline solution (15 mL*3) were performed. The organic phase was dried and with anhydrous sodium sulfate, filtered and spin-dried. Then, the residue is dissolved in tetrahydrofuran (5mL). Then, N, N-dicarbonyl imidazole (0.23g, 1.6mmol) and 1,8-diazabicyclo[5.4.0]undec-7-ene (0.36g, 2.4mmol) were added. Replacement with nitrogen gas was performed. Heating to 55°C for reaction for 2h was performed. After LCMS showed that the reaction was completed, the temperature was decreased to room temperature. Spin-drying was performed. The residue was dissolved in ethyl acetate (30mL), and then washed sequentially with saturated sodium bicarbonate solution (15mL*2) and saturated saline solution (15mL*2). The organic phase was dried with anhydrous sodium sulfate, filtered, spin-dried. The residue was separated by Flash column chromatography to obtain a white solid target compound (0.25g, yield: 65%).

MS m/z (ESI): 487.3[M+H]+.

Step 3: at room temperature, (S)-5-(2,2-dimethyltetrahydro-2H-pyran-4-yl)-N-methyl-1-(1-(5-carbonyl-4,5-dihydro-1,2,4-oxadiazol-3-yl)cyclopropyl)-N-phenyl-1H-indol-2-carboxamide (0.25g, 0.52mmol) was dissolved in PEG-400 (5mL). Then, potassium hydroxide (0.29g, 5.2mmol) was added. Replacement with nitrogen gas was performed. Heating to 165°C for reaction for 3h was performed. The temperature was decreased to room temperature. After

LCMS showed that the reaction was completed, the reaction solution was neutralized with dilute hydrochloric acid (6N). The pH was regulated to 5-6. A solid precipitated. Filtration was performed. The solid was washed sequentially with water and petroleum ether and dried in air. The obtained solid was directly used for the next step (0.15g, yield: 75%).

MS m/z (ESI):398.2[M+H]+.

Step 4:

Referring to step 4 in Example 29, a target product was obtained.

MS m/z (ESI):918.4[M+H]+.

**Example 84**

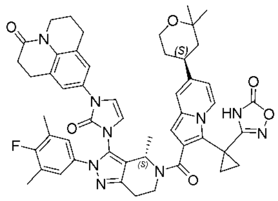

Using 9-bromo-2,3,6,7-tetrahydro-1H, 5H-pyridino[3,2,1-ij]quinolin-5-one as a raw material, and referring to Example 83, a target compound was obtained.

MS m/z (ESI):906.4[M+H]+.

Example 85

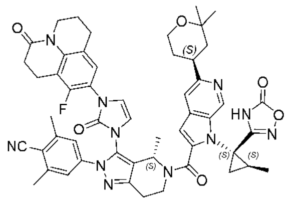

Referring to the synthesis method in Example 44, a target compound was obtained.

MS m/z (ESI): 946.4[M+1].

**Example 86**

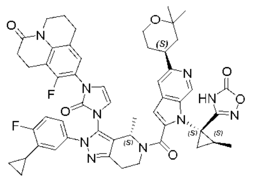

Referring to the synthesis method in Example 44, a target compound was obtained.

MS m/z (ESI): 951.4[M+1].

**Example 87**

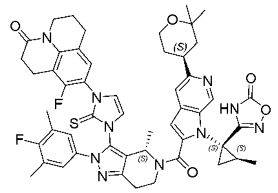

Referring to the synthesis method in Example 3, a target compound was obtained.
MS m/z (ESI): 955.4[M+1].

## Example 88

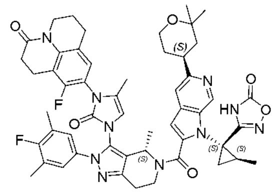

Referring to the synthesis method in Example 45, a target compound was obtained.
MS m/z (ESI): 953.4[M+1].

## Example 89

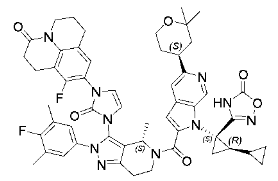

Referring to the synthesis method in Example 46, a target compound was obtained.
MS m/z (ESI): 965.4[M+1].

## Example 90

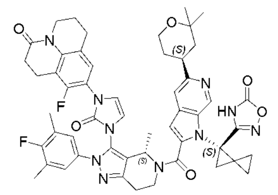

Referring to the synthesis method in Example 79, a target compound was obtained.
MS m/z (ESI): 951.4[M+1].

## Example 91

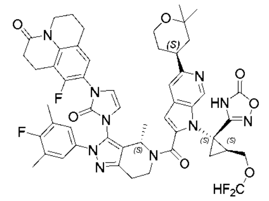

Referring to the synthesis method in Example 64, a target compound was obtained.
MS m/z (ESI): 1005.4[M+1].

**Example 92**

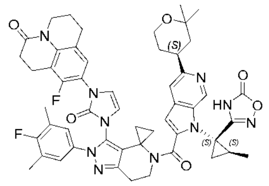

Referring to the synthesis method in Example 16, a target compound was obtained.
MS m/z (ESI): 951.4[M+1].

**Example 93**

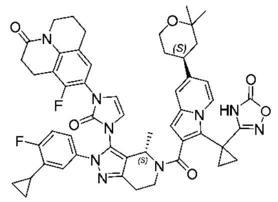

Referring to the synthesis method in Example 44, a target compound was obtained.
MS m/z (ESI): 936.4[M+1].

**Example 94**

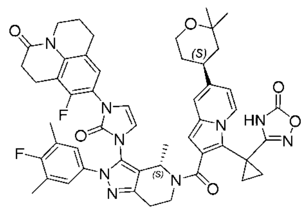

Referring to the synthesis method in Example 44, a target compound was obtained.
MS m/z (ESI): 924.4[M+1].

**Example 95**

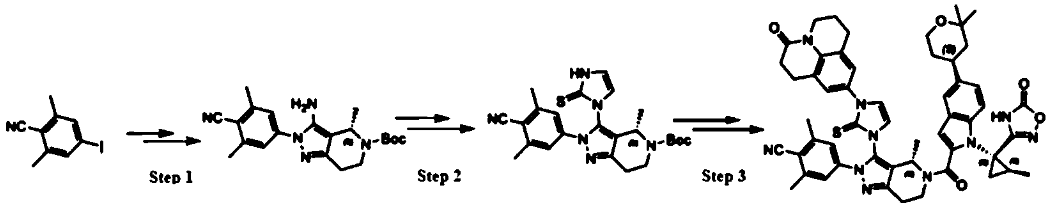


Step 1:

Using 4-iodo-2,6-dimethylbenzonitrile as a raw material, and referring to the synthesis method of Intermediate B in step 3, a target product was obtained.

MS m/z (ESI): 382.2[M+1].

Step 2:

Using (S)-3-amino-2-(4-cyano-3,5-dimethylphenyl)-4-methyl-2,4,6,7-tetrahydro-5H-pyrazolo[4,3-c]pyridin-5-carboxylic acid tert-butyl ester as a raw material, and referring to the synthesis method in step 1 in Example 3, a target product was obtained.

MS m/z (ESI): 465.2[M+1].

Step 3:

Using (S)-2-(4-cyano-3,5-dimethylphenyl)-4-methyl-3-(2-thioxy-2,3-dihydro-1H-imidazol-1-yl)-2,4,6,7-tetrahydro-5H-pyrazolo[4,3-c]pyridin-5-carboxylic acid tert-butyl ester as a raw material, referring to the synthesis method in step 2 to step 4 in Example 25, and replacing 5-bromo-1-cyclopropyl-4-fluoroindazole with 9-bromo-2,3,6,7-tetrahydro-1H, 5H-pyridino[3,2-1-ij]quinolin-5-one, a target product was obtained.

MS m/z (ESI): 943.4[M+1].

## Example 96

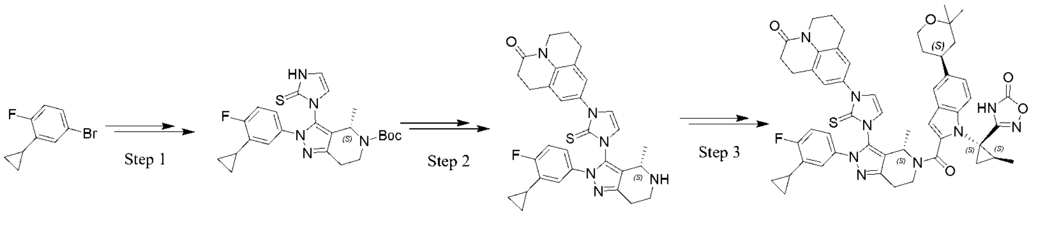


Step 1: using 4-bromo-2-cyclopropyl-1-fluorophenyl as a raw material, and referring to the synthesis method in step 1 and step 2 in Example 95, a target product was obtained.

MS m/z (ESI): 470.1[M+1].

Step 2: using (S)-2-(3-cyclopropyl-4-fluorophenyl)-4-methyl-3-(2-thioxy-2,3-dihydro-1H-imidazol-1-yl)-2,4,6,7-tetrahydro-5H-pyrazolo [4,3-c]pyridin-5-carboxylic acid tert-butyl ester as a raw material, referring to the synthesis method in step 2 to step 3 in Example 25, and replacing 5-bromo-1-cyclopropyl-4-fluoroindazole with 9-bromo-2,3,6,7-tetrahydro-1H, 5H-pyridino[3,2-1-ij]quinolin-5-one, a target product was obtained.

MS m/z (ESI): 555.2[M+1].

Step 3: using (S)-9-(3-(2-(3-cyclopropyl-4-fluorophenyl)-4-methyl-4,5,6,7-tetrahydro-2H-pyrazolo[4,3-c]pyridin-3-yl)-2-thioxy-2,3-dihydro-1H-imidazol-1-yl)-2,3,6,7-tetrahydro-1H, 5H-pyrido [3,2,1-ij] quinolin-5-one as a raw material, and referring to the synthesis method in step 4 in Example 25, a target product was obtained.

MS m/z (ESI): 948.4[M+1].

## Example 97

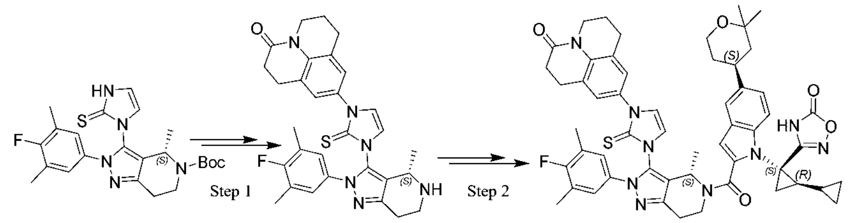


Step 1: using (S)-2-(4-fluoro-3,5-dimethylphenyl)-4-methyl-3-(2-thioxy-2,3-dihydro-1H-imidazol-1-yl)-2,4,6,7-tetrahydro-5H-pyrazolo[4,3-c]pyridin-5-carboxylic acid tert-butyl ester as a raw material, referring to the synthesis method in step 2 and step 3 in Example 25, and replacing 5-bromo-1-cyclopropyl-4-fluoroindazole with 9-bromo-2,3,6,7-tetrahydro-1H, 5H-pyridino[3,2-1-ij]quinolin-5-one, a target product was obtained.

MS m/z (ESI): 543.2[M+1].

Step 2: using (S)-9-(3-(2-(4-fluoro-3,5-dimethylphenyl)-4-methyl-4,5,6,7-tetrahydro-2H-pyrazolo[4.3-c]pyridin-3-yl)-2-thioxy-2,3-dihydro-1H-imidazol-1-yl)-2,3,6,7-tetrahydro-1H, 5H-pyrido[3,2,1-ij]quinolin-5-one and 5-((S)-2,2-dimethyltetrahydro-2H-pyran-4-yl)-1-(1R, 2S)-2-(5-carbonyl-4,5-dihydro-1,2,4-oxadiazol-3-yl)-[1,1'-bis(cyclopropane)]-2-yl)-1H-indol-2-carboxylic acid as raw materials, and referring to the synthesis method in step 4 in Example 25, a target product was obtained.

MS m/z (ESI): 962.4[M+1].

**Example 98**

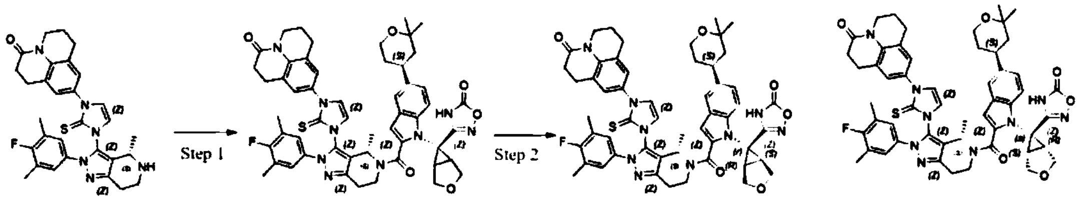


Step 1: using (S)-9-(3-(2-(4-fluoro-3,5-dimethylphenyl)-4-methyl-4,5,6,7-tetrahydro-2H-pyrazolo[4,3-c]pyridin-3-yl)-2-thioxy-2,3-dihydro-1H-imidazol-1-yl)-2,3,6,7-tetrahydro-1H, 5H-pyrido[3,2,1-ij]quinolin-5-one and 5-((S)-2,2-dimethyltetrahydro-2H-pyran-4-yl)-1-(6-(5-carbonyl-4,5-dihydro-1,2,4-oxadiazol-3-yl)-3-oxabicyclo[3.1.0] hexan-6-yl)-1H-indol-2-carboxylic acid as raw materials, and referring to the synthesis method in step 4 in Example 25, a target product was obtained.

MS m/z (ESI): 964.3 [M+1].

Step 2: chiral separation was performed on Compound 98 to obtain title product 98-1 and 98-2.

MS m/z (ESI): 964.3 [M+1].

**Example 99**

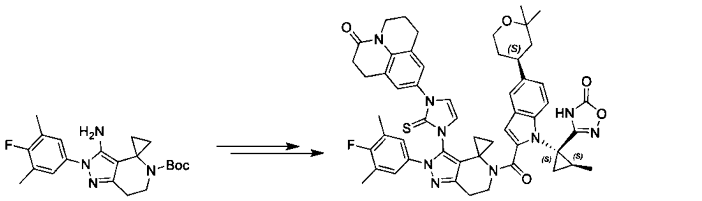

Using tert-butyl 3'-amino-2'-(4-fluoro-3,5-dimethylphenyl)-6',7'-dihydrospiro [cyclopropane-1,4'-pyrazolo[4,3-c] pyridin]-5'(2'H)-carboxylate as a raw material, and referring to the synthesis method in step 2 and step 3 in Example 95, a target product was obtained.

MS m/z (ESI): 948.4 [M+1].

**Example 100**

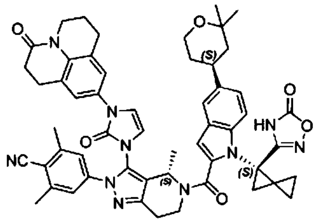

Referring to the synthesis method in Example 403, a target product was obtained.
MS m/z (ESI):939.4[M+1].

**Example 101**

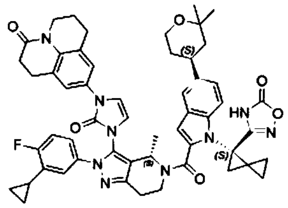

Referring to the synthesis method in Example 22, a target product was obtained.
MS m/z (ESI):944.4[M+1].

**Example 102**

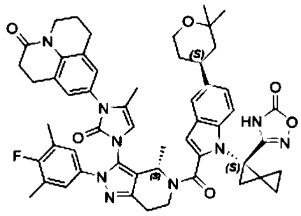

Referring to the synthesis method in Example 406, a target product was obtained.
MS m/z (ESI): 946.4[M+1].

**Example 103**

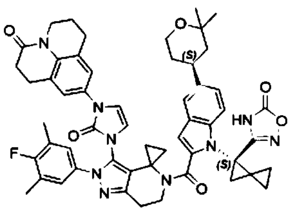

Referring to the synthesis method in Example 51, a target compound was obtained.

MS m/z (ESI):944.4[M+1].

**Example 104**

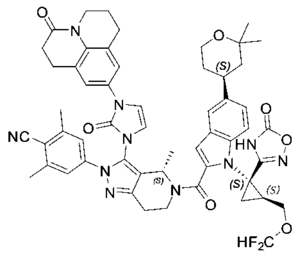

Referring to the synthesis method in Example 403, a target compound was obtained.
MS m/z (ESI): 993.4[M+1].

**Example 105**

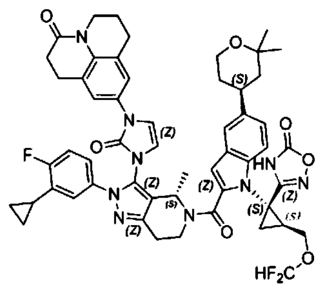

Referring to the synthesis method in Example 22, a target product was obtained.
MS m/z (ESI): 998.4[M+1].

**Example 106**

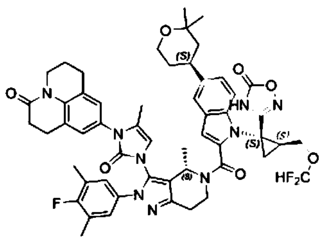

Referring to the synthesis method in Example 406, a target product was obtained.
MS m/z (ESI):1000.4 [M+1].

**Example 107**

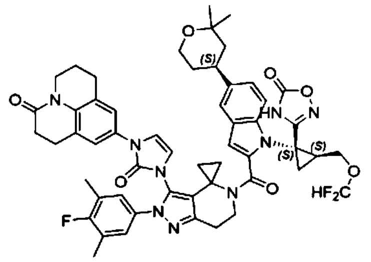

Referring to the synthesis method in Example 51, a target compound was obtained.
MS m/z (ESI): 998.4[M+1].

**Example 108**

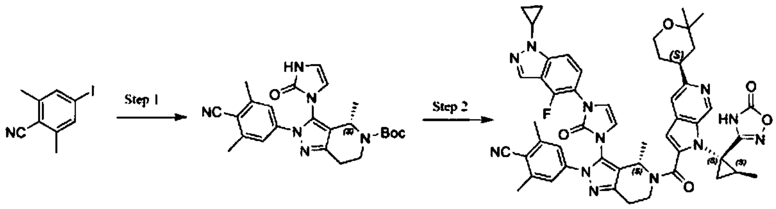


Step 1: using 4-iodo-2,6-dimethylbenzonitrile as a raw material, and referring to the synthesis method of Intermediate B, a target product was obtained.
MS m/z (ESI): 449.2[M+1].
Step 2: referring to the synthesis method in Example 25, a target product was obtained.
MS m/z (ESI): 917.4[M+1].

**Example 109**

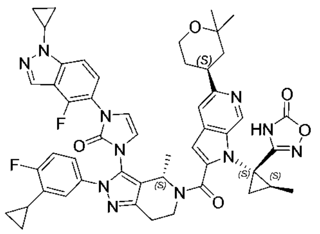

Using 2-cyclopropyl-1-fluoro-4-iodobenzene as a raw material, and referring to the synthesis method in Example 108, a target product was obtained.
MS m/z (ESI): 922.4[M+1].

**Example 110**

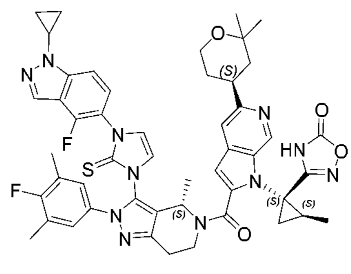

1-cyclopropyl-4-fluoro-1H-indazol-5-amine was used as the starting material, and the target product was synthesized following Example 3.

MS m/z (ESI): 926.4[M+1].

**Example 111**

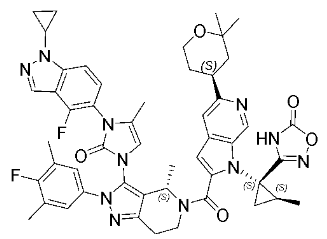

5-bromo-1-cyclopropyl-4-fluoro-1H-indazol was used as the starting material, and the target product was synthesized following Example 45.

MS m/z (ESI): 924.4[M+1].

**Example 112**

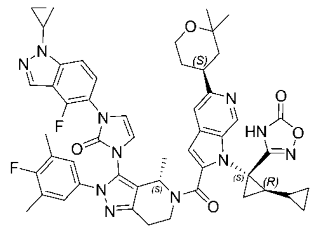

5-bromo-1-cyclopropyl-4-fluoro-1H-indazol was used as the starting material, and the target product was synthesized following Example 40.

MS m/z (ESI): 936.4[M+1].

**Example 113**

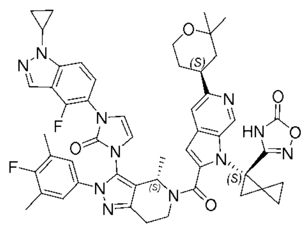

5-bromo-1-cyclopropyl-4-fluoro-1H-indazol was used as the starting material, and the target product was synthesized following Example 5.

MS m/z (ESI): 922.4[M+1].

**Example 114**

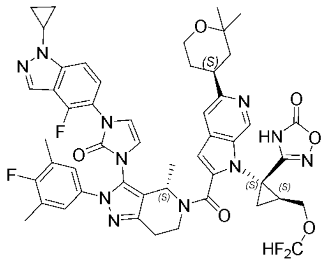

5-bromo-1-cyclopropyl-4-fluoro-1H-indazol was used as the starting material, and the target product was synthesized following Example 64.

MS m/z (ESI): 976.4[M+1].

**Example 115**

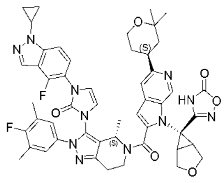 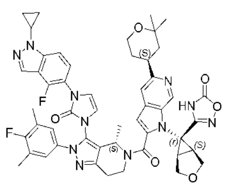 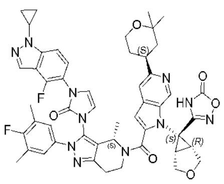

5-bromo-1-cyclopropyl-4-fluoro-1H-indazol was used as the starting material, and the target product was synthesized following Example 62, and chirally split to obtan 115-1 and 115-2.

MS m/z (ESI): 938.4[M+1].

**Example 116**

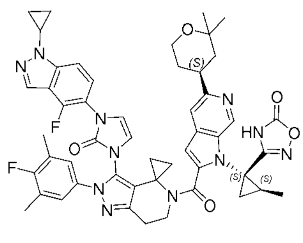

The target product was synthesized following Example 110.

MS m/z (ESI): 922.4[M+1].

**Example 117**

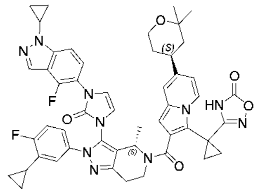

The target product was synthesized following Examples 109 and 6.

MS m/z (ESI): 907.4[M+1].

**Example 118**

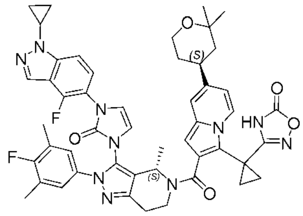

5-bromo-1-cyclopropyl-4-fluoro-1H-indazol was used as the starting material, and the target product was synthesized following Example 6.

MS m/z (ESI): 895.4[M+1].

**Example 119**

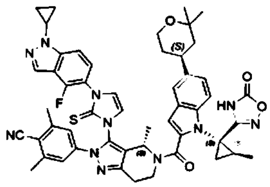

5-bromo-1-cyclopropyl-4-fluoro-1H-indazol was used as the starting material, and the target product was ultimately obtained following Example 4.

MS m/z (ESI): 932.4[M+1].

**Example 120**

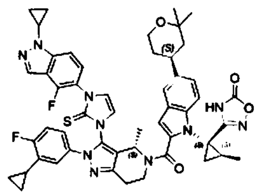

5-bromo-1-cyclopropyl-4-fluoro-1H-indazol was used as the starting material, and the target product was ultimately obtained following Example 4.

MS m/z (ESI): 937.3[M+1].

**Example 121**

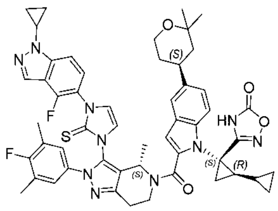

5-bromo-1-cyclopropyl-4-fluoro-1H-indazol was used as the starting material, and the target product was ultimately obtained following Example 46.

MS m/z (ESI): 951.4[M+1].

**Example 122**

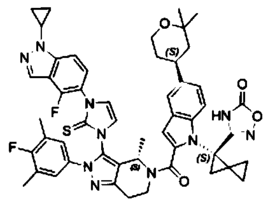

5-bromo-1-cyclopropyl-4-fluoro-1H-indazol was used as the starting material, and the target product was ultimately obtained following Example 46.

MS m/z (ESI): 937.4[M+1].

**Example 123**

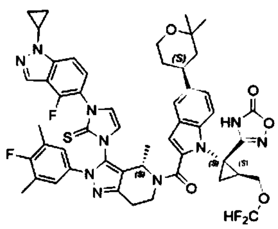

5-bromo-1-cyclopropyl-4-fluoro-1H-indazol was used as the starting material, and the target product was ultimately obtained following Example 64.

MS m/z (ESI): 991.4[M+1].

**Example 124**

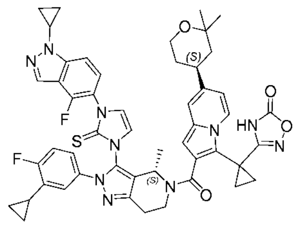

5-bromo-1-cyclopropyl-4-fluoro-1H-indazol was used as the starting material, and the target product was ultimately obtained following Example 47.

MS m/z (ESI): 923.3[M+1].

**Example 125**

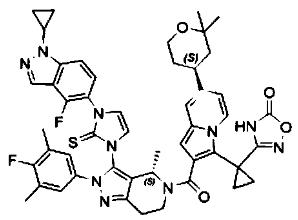

5-bromo-1-cyclopropyl-4-fluoro-1H-indazol was used as the starting material, and the target product was ultimately obtained following Example 47.

MS m/z (ESI): 911.3[M+1].

**Example 126**

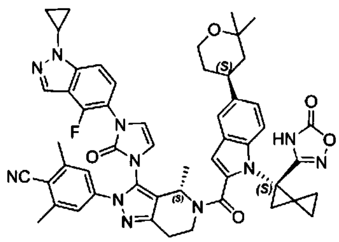

5-bromo-1-cyclopropyl-4-fluoro-1H-indazol, tert-butyl(S)-2-(4-cyano-3,5-dimethylphenyl)-4-methyl-3-(2-carbonyl-2,3-dihydro-1H-imidazol-1-yl)-2,4,6,7-tetrahydro-SH-pyrazolo[4,3-c]pyridine-5-carboxylate, and 5-((S)-2,2-dimethyltetrahydro-2H-pyran-4-yl)-1-((S)-1-(5-carbonyl-4,5-dihydro-1,2,4-oxadiazol-3-yl)spiro[2.2]pentan-1-yl)-1H-indol-2-carboxylic acid were used as the starting materials, and the product was obtained following Example 29.

MS m/z (ESI): 928.4[M+1].

**Example 127**

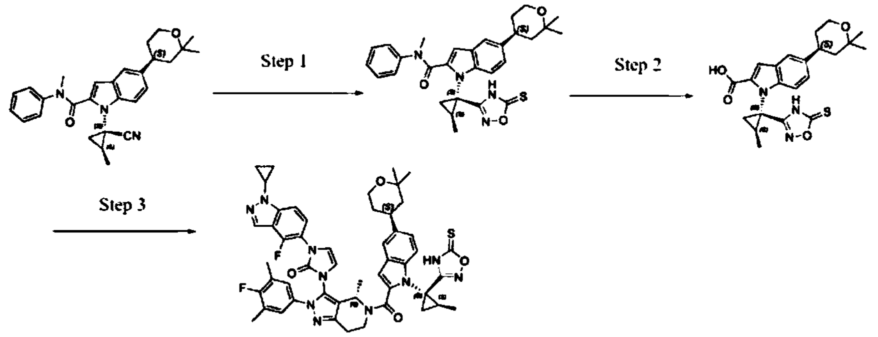


Step 1: At room temperature, 1-((1S,2S)-1-cyano-2-methylcyclopropyl)-5-((S)-2,2-dimethyltetrahydro-2H-pyran-4-yl)-N-methyl-N-phenyl-1H-indol-2-carboxamide (0.44 g, 1 mmol) was dissolved in ethanol (6 mL). Then triethylamine (0.30 g, 3 mmol) and hydroxylamine hydrochloride (0.14 g, 2 mmol) were added sequentially, and nitrogen gas was replaced. The mixture was heated to 90°C and reacted for 3 hours. When LCMS indicated the end of the reaction, the reaction product was cooled to room temperature and spin dried. The residue was dissolved in ethyl acetate (15 mL), and then washed sequentially with saturated sodium bicarbonate solution (10 mL×3) and saturated saline solution (15 mL×3). The organic phase was dried with anhydrous sodium sulfate, filtered, and spin dried. The residue was dissolved in tetrahydrofuran (5 mL). Then N, N-thiocarbonyl diimidazole (0.36 g, 2 mmol) and 1,8-diazabicyclo[5.4.0]undec-7-ene (0.45 g, 3 mmol) were added sequentially, and nitrogen gas was replaced. The mixture was heated to 55°C and reacted for 2 hours. When LCMS indicated the end of the reaction, the reaction product was cooled to room temperature and spin dried. The residue was dissolved in ethyl acetate (30 mL), and then washed sequentially with saturated sodium bicarbonate solution (15 mL×2) and saturated saline solution (15 mL×2). The organic phase was dried with anhydrous sodium sulfate, filtered, and spin dried. The residue was separated by Flash column chromatography to obtain the target compound as a white solid (0.28 g, yield 55%).

MS m/z (ESI): 517.2[M+H]+.

Step 2: At room temperature, 5-((S)-2,2-dimethyltetrahydro-2H-pyran-4-yl)-N-methyl-1-((1S,2S)-2-methyl-1-(5-thio-4,5-dihydro-1,2,4-oxadiazol-3-yl)cyclopropyl)-N-phenyl-1H-indol-2-carboxamide (0.28 g, 0.55 mmol) was dissolved in PEG-400 (6 mL). Then potassium hydroxide (0.31 g, 5.5 mmol) was added, and nitrogen gas was replaced. The mixture was heated to 165°C, reacted for 3 hours, and was cooled to room temperature. When LCMS indicated the end of the reaction, the reaction solution was neutralized with dilute hydrochloric acid (6 N) and adjusted to pH 5-6. A solid precipitated was filtered, washed sequentially with water and petroleum ether, air dried, and directly used for the next step (0.16 g, yield 70%).

MS m/z (ESI):428.2[M+H]+.

Step 3: 5-((S)-2,2-dimethyltetrahydro-2H-pyran-4-yl)-1-((1S,2S)-2-methyl-1-(5-thio-4,5-dihydro1,2,4-oxadiazol-3-yl)cyclopropyl)-1H-indol-2-carboxylic acid was used as the starting material, and the target compound was obtained following Step 4 of Example 25.

MS m/z (ESI):925.4[M+H]+.

**Example 128**

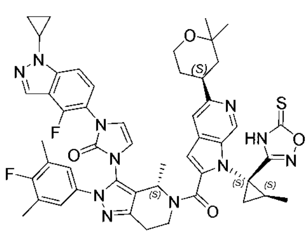

Ethyl5-chloro-1H-pyrrolo[2,3-c]pyridine-2-carboxylate was used as the starting material, and the target compound was obtained following Examples 13 and 448.

MS m/z (ESI):926.4[M+H]+.

**Example 129**

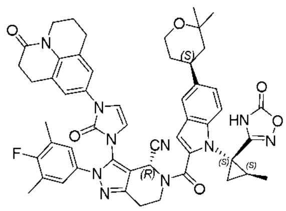

The target compound was obtained following the synthesis method of Example 407. MS m/z (ESI):931.4[M+H]+.

**Example 130**

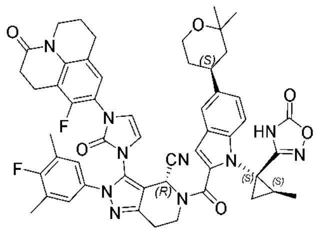

The target compound was obtained following the synthesis method of Example 407. MS m/z (ESI):949.4[M+H]+.

**Example 131**

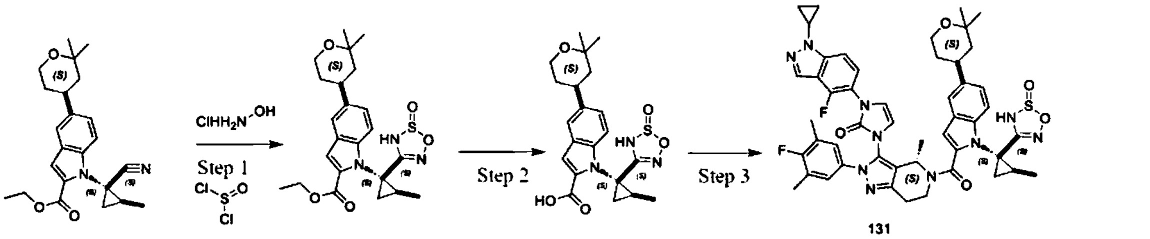


131

Step 1: 1-((1S,2S)-1-cyano-2-methylcyclopropyl)-5-((S)-2,2-dimethyltetrahydro-2H-pyran-4-yl)-1H-indol-2-ethyl carboxylate was used as the starting material, and the target product was obtained following the synthesis method of intermediate A in Step 4 and replacing the carbonyl diimidazole with thionyl chloride.

MS m/z (ESI): 460.1[M+1].

Step 2: 5-((S)-2,2-dimethyltetrahydro-2H-pyran-4-yl)-1-((1S,2S)-2-methyl-1-(2-hydroxy-3H-1,2,3,5-thiadiazole-4-yl)cyclopropyl)-1H-indol-2-ethyl carboxylate was used as the starting material, and the target product was obtained following the synthesis method of intermediate A in Step 5.

MS m/z (ESI): 432.1[M+1].

Step 3: 5-((S)-2,2-dimethyltetrahydro-2H-pyran-4-yl)-1-((1S,2S)-2-methyl-1-(2-hydroxy-3H-1,2,3,5-thiadiazole-4-yl)cyclopropyl)-1H-indol-2-carboxylic acid and (S)-1-(1-cyclopropyl-4-fluoro-1H-indazol-5-yl)-3-(2-(4-fluoro-3,5-dimethylphenyl)-4-methyl-4,5,6,7-tetrahydro-2H-pyrazolo[4,3-c]pyridine-3-yl)-1,3-dihydro-2H-imidazol-2-one were used as the starting materials, and the target was obtained following the synthesis method in Step 4 of Example 25.

MS m/z (ESI): 929.3[M+1].

**Example 132**

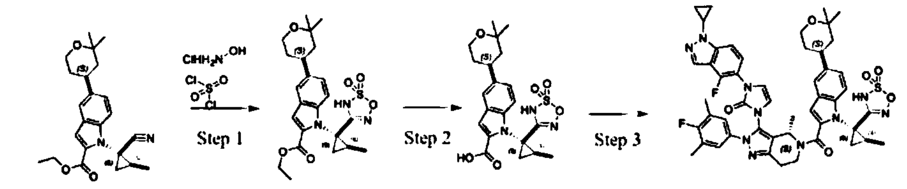


Step 1: 1-((1S,2S)-1-cyano-2-methylcyclopropyl)-5-((S)-2,2-dimethyltetrahydro-2H-pyran-4-yl)-1H-indol-2-ethyl carboxylate was used as the starting material, and the target product was obtained following the synthesis method of intermediate A in Step 4 and replacing the carbonyl diimidazole with sulfonyl chloride.

MS m/z (ESI): 476.1[M+1].

Step 2: 5-((S)-2,2-dimethyltetrahydro-2H-pyran-4-yl)-1-((1S,2S)-1-(2,2-dihydroxy-3H-1,2,3,5-thiadiazole-4-yl)-2-methylcyclopropyl)-1H-indol-2-ethyl carboxylate was used as the starting material, and the target product was obtained following the synthesis method of intermediate A in Step 5.

MS m/z (ESI): 448.1[M+1].

Step 3: 5-((S)-2,2-dimethyltetrahydro-2H-pyran-4-yl)-1-((1S,2S)-1-(2,2-dihydroxy-3H-1,2,3,5-thiadiazole-4-yl)-2-methylcyclopropyl)-1H-indol-2-carboxylic acid and (S)-1-(1-cyclopropyl-4-fluoro-1H-indazol-5-yl)-3-(2-(4-fluoro-3,5-dimethylphenyl)-4-methyl-4,5,6,7-tetrahydro-2H-pyrazolo[4,3-c]pyridine-3-yl)-1,3-dihydro-2H-imidazol-2-one were used as the starting materials, and the target product was obtained following the synthesis method in Step 4 of Example 25.

MS m/z (ESI): 945.3[M+1].

**Example 133**

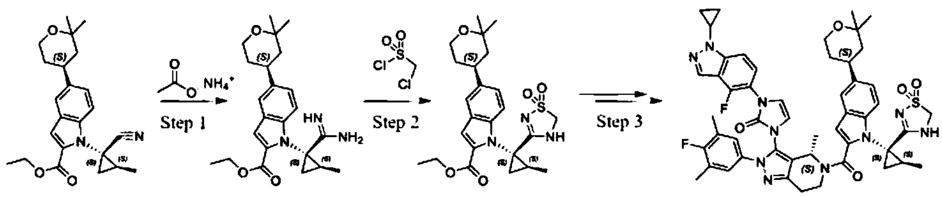


Step 1: 1-((1S,2S)-1-cyano-2-methylcyclopropyl)-5-((S)-2,2-dimethyltetrahydro-2H-pyran-4-yl)-1H-indol-2-ethyl carboxylate (1 g, 2.63 mmol) and ammonium acetate (202.7 mg, 2.63 mmol) were dissolved in 10 mL DMF and refluxed for reaction at 100°C for 12 hours. The reaction solution was concentrated under reduced pressure, and the residue was purified by silica gel column chromatography (eluents: dichloromethane and methanol in a ratio of 10:1) to obtain the target product (260 mg, yellow solid) with a yield of 24.8%.

MS m/z (ESI): 398.2 [M+1].

Step 2: 1-((1S,2S)-1-aminocarboximidoyl-2-methylcyclopropyl)-5-((S)-2,2-dimethyltetrahydro-2H-pyran4-yl)-1H-indol-2-ethyl carboxylate (260 mg, 654.06 μmol) and triethylamine (198.56 mg, 1.96 mmol) were dissolved in 5 mL DCM. Then a solution of chloromethanesulfonyl chloride (97.44 g, 654.06 μmol) in dichloromethane was slowly added, and the reaction solution was stirred at room temperature for 10 hours. The reaction solution was concentrated under reduced pressure, and the residue was purified by silica gel column chromatography (eluents: dichloromethane and methanol in a ratio of 10:1) to obtain the target product (110 mg, yellow solid) with a yield of 35.5%.

MS m/z (ESI): 474.2 [M+1].

Step 3: 5-((S)-2,2-dimethyltetrahydro-2H-pyran-4-yl)-1-((1S,2S)-1-(1,1-dihydroxy-4,5-dihydro-1,2,4-thiadiazole-3-yl)-2-methylcyclopropyl)-1H-indol-2-ethyl carboxylate was used as the starting material, and the target product was obtained following the synthesis method of Example 25.

MS m/z (ESI): 943.3[M+1].

**Example 134**

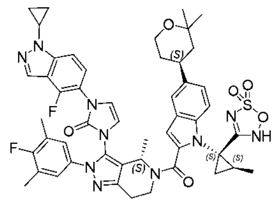

1-((1S,2S)-1-cyano-2-methylcyclopropyl)-5-((S)-2,2-dimethyltetrahydro-2H-pyran-4-yl)-1H-indol-2-ethyl carboxylate was used as the starting material, and the target product was obtained following the synthesis method of Example 132.

MS m/z (ESI): 945.3[M+1].

**Example 135**

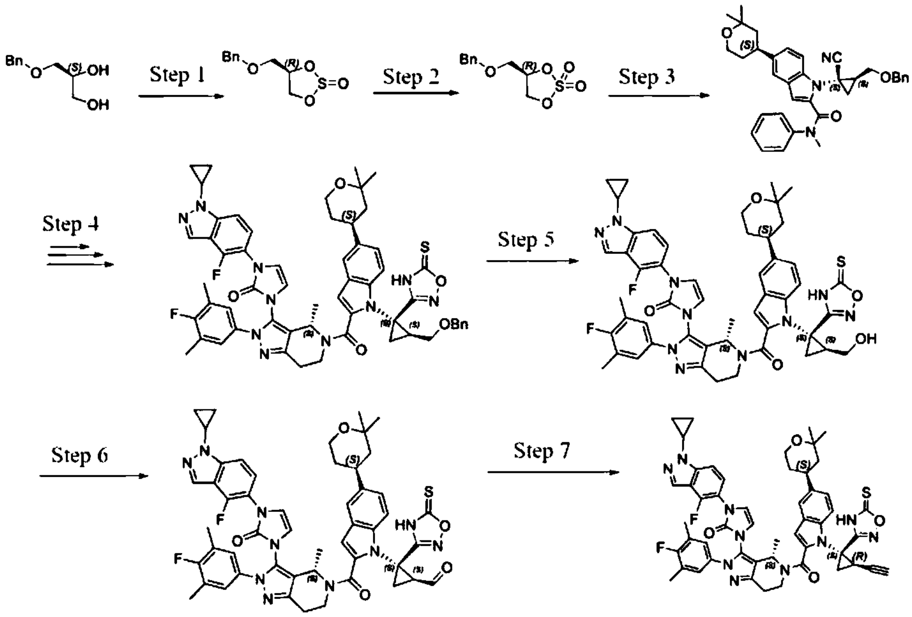


Step 1: In an ice bath, (2S)-3-benzyloxypropane-1,2-diol (5 g, 27.44 mmol) was dissolved in dichloromethane (100 mL). Then triethylamine (11.11 g, 109.76 mmol, 15.31 mL) was added, and thionyl chloride (4.90 g, 41.16 mmol, 2.99 mL) was slowly added dropwise. The mixture was stirred for one hour in the ice bath. When LCMS indicated complete reaction, the reaction solution was quenched with water (150 mL), and then extracted with dichloromethane (100 mL×3). The organic phases were mixed, washed with saturated saline solution (100 mL×2), dried with anhydrous sodium sulfate, filtered, and spin dried to obtain the target product (6 g, crude).

Step 2: In an ice bath, (4R)-4-((benzyloxy)methyl)-1,3,2-dithiolan 2-oxide (6 g, 26.29 mmol) was dissolved in acetonitrile (90 mL) and water (10 mL). Then sodium periodate (8.43 g, 39.43 mmol) and ruthenium chloride hydrate (296.29 mg, 1.31 mmol) were added, and stirred at room temperature for 3 hours. When LCMS indicated the end of the reaction, the reaction solution was quenched with water (150 mL), and then extracted with dichloromethane (100 mL×3). The organic phases were mixed, washed with saturated saline solution (100 mL×2), dried with anhydrous sodium sulfate, filtered, and spin dried. The crude product was separated by Flash column chromatography (PE/EA=4:1) to obtain the target compound (5 g, yield 55%).

Step 3: In an ice bath, 1-(cyanomethyl)-5-[(4S)-2,2-dimethyltetrahydro-pyran-4-yl]-N-methyl-N-phenyl-indol-2-carboxamide (0.5 g, 1.25 mmol) was dissolved in DMPU (15 mL). Then (R)-4-((benzyloxy)methyl)-1,3,2-dithiolan 2,2-dioxide (1.06 g, 4.36 mmol) was added, and nitrogen gas was replaced. Then potassium hexamethyldisilazide (1.0 M, 5.6 mL) was slowly added dropwise. After the dropwise addition, the reaction solution was stirred in the ice bath for 2 hours. When LCMS indicated the end of the reaction, the reaction solution was quenched with saturated ammonium chloride solution (20 mL). Then saturated saline solution (20 mL) was added, and the reaction solution was extracted with ethyl acetate (30 mL×3), The organic phases were mixed, dried with anhydrous sodium sulfate, filtered, and spin dried. The residue was separated by Flash column chromatography to obtain the target compound as a light yellow solid (0.48 g, yield

70.4%).

Step 4: The target product was obtained following Steps 1, 2, and 3 of Example 127.

MS m/z (ESI): 1031.4[M+H]+.

Step 5: At room temperature, 1-((S)-5-(1-((1S,2S)-2-((benzyloxy)methyl)-1-(5-thio-4,5-dihydro-1,2,4-oxadiazol-3-yl)cyclopropyl)-5-((S)-2,2-dimethyltetrahydro-2H-pyran-4-yl)-1H-indol-2-carbonyl)-2-(4-fluoro-3,5-dimethylphenyl)-4-methyl-4,5,6,7-tetrahydro-2H-pyrazolo[4,3-c]pyridine-3-yl)-3-(1-cyclopropyl-4-fluoro-1H-indazol-5-yl)-1,3-dihydro-2H-imidazol-2-one (0.20 g, 0.19 mmol) was dissolved in dichloromethane (5 mL). Then boron trichloride (1.0 M, 1 mL) was added dropwise and stirred at room temperature for 1 hour. When LCMS indicated the end of the reaction, the reaction solution was quenched with saturated sodium bicarbonate solution (10 mL), and the reaction solution was extracted with dichloromethane (15 mL×3). The organic phases were mixed, dried with anhydrous sodium sulfate, filtered, and spin dried. The residue was purified by reversed phase preparative chromatography to obtain the target product (0.10 g, yield 55%).

MS m/z (ESI): 941.4[M+H]+.

Step 6: At room temperature, 1-(1-cyclopropyl-4-fluoro-1H-indazol-5-yl)-3-((S)-5-(5-((S)-2,2-dimethyltetrahydro-2H-pyran-4-yl)-1-((1S,2S)-2-(methylol)-1-(5-thio-4,5-dihydro-1,2,4-oxadiazol-3-yl)cyclopropyl)-1H-indol-2-carbonyl)-2-(4-fluoro-3,5-dimethylphenyl)-4-methyl-4,5,6,7-tetrahydro-2H-pyrazolo[4,3-c]pyridine-3-yl)-1,3-dihydro-2H-imidazol-2-one (0.10 g, 0.11 mmol) was dissolved in dichloromethane (5 mL). Then Dess-Martin (0.09 g, 0.22 mmol) was added and stirred at room temperature for 14 hours. When LCMS indicated the end of the reaction, the reaction solution was diluted with dichloromethane (20 mL), and then washed sequentially with saturated sodium bicarbonate solution (15 mL×2) and saturated saline solution (15 mL×2). The organic phase was dried with anhydrous sodium sulfate, filtered, and spin dried. The residue was separated by Flash column chromatography to obtain the target compound (0.06 g, yield 60%).

MS m/z (ESI): 939.4[M+H]+.

Step 7: At room temperature, (1S,2S)-2-(2-((S)-3-(3-(1-cyclopropyl-4-fluoro-1H-indazol-5-yl)-2-carbonyl-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indol-1-yl)-2-(5-thio-4,5-dihydro-1,2,4-oxadiazol-3-yl)cyclopropane-1-formaldehyde (0.06 g, 0.06 mmol) was dissolved in methanol (3 mL). Then 1-diazo-1-dimethoxyphosphorylpropane-2-one (0.02 g, 0.1 mmol) and potassium carbonate (0.02 g, 0.14 mmol) were added and stirred at room temperature for 5 hours. When LCMS indicated the end of the reaction, the reaction solution was quenched with saturated saline solution (20 mL), and then extracted with ethyl acetate (15 mL×3). The organic phases were mixed, dried with anhydrous sodium sulfate, filtered, and spin dried. The residue was purified by reversed phase preparative chromatography to obtain the target compound (0.02 g, yield 35.7%).

MS m/z (ESI): 935.4[M+H]+.

**Example 136**

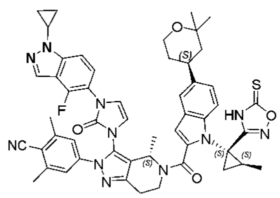

Intermediate G was used as the starting material, and the target product was obtained following Examples 25 and 127.

MS m/z (ESI): 932.4[M+H]+.

**Example 137**

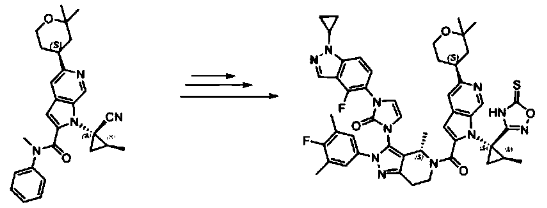

1-((1S,2S)-1-cyano-2-methylcyclopropyl)-5-((S)-2,2-dimethyltetrahydro-2H-pyran-4-yl)-N-methyl-N-phenyl-1H-pyrrolo[2,3-c]pyridine-2-carboxamide was used as the starting material, and the target product was obtained following Example 127.

MS m/z (ESI): 926.2[M+H]+.

**Example 138**

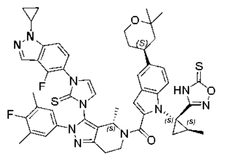

The target product was obtained following Examples 3 and 127.

MS m/z (ESI): 941.4[M+H]+.

**Example 139**

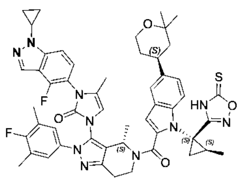

The target product was obtained following Examples 45 and 127.

MS m/z (ESI): 939.4[M+H]+.

**Example 140**

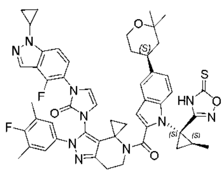

The target product was obtained following Examples 51 and 127.

MS m/z (ESI): 937.4[M+H]+.

**Example 141**

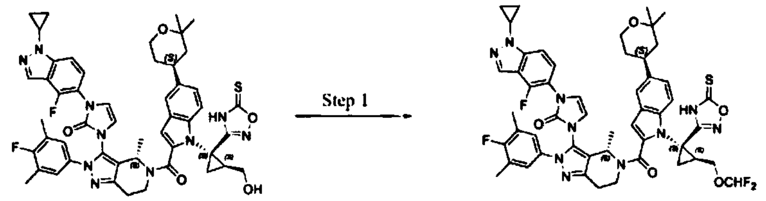


Step 1: 1-(1-cyclopropyl-4-fluoro-1H-indazol-5-yl)-3-((S)-5-(5-((S)-2,2-dimethyltetrahydro-2H-pyran-4-yl)-1-((1S,2S)-2-(methylol)-1-(5-thio-4,5-dihydro-1,2,4-oxadiazol-3-yl)cyclopropyl)-1H-indol-2-carbonyl)-2-(4-fluoro-3,5-dimethylphenyl)-4-methyl-4,5,6,7-tetrahydro-2H-pyrazolo[4,3-c]pyridine-3-yl)-1,3-dihydro-2H-imidazol-2-one (0.05 g, 0.053 mmol) was dissolved in dichloromethane (2 mL). Then potassium acetate (0.02 g, 0.212 mmol) was added, and (bromodifluoromethyl) trimethylsilane (0.022 g, 0.11 mmol) was added dropwise. The mixture was stirred at room temperature for 12 hours. When LCMS indicated the end of the reaction, the reaction solution was quenched with water (5 mL), and then extracted with dichloromethane (10 mL×3). The organic phases were mixed, washed with saturated saline solution (10 mL×2), dried with anhydrous sodium sulfate, and filtered. The residue was purified by reversed phase preparative chromatography to obtain the target compound (0.01 g, yield 19%).

MS m/z (ESI): 991.4[M+H]+.

**Example 142**

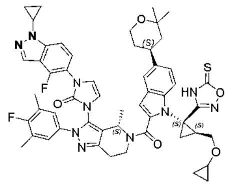

The target product was obtained following Examples 63 and 135.

MS m/z (ESI): 981.4[M+H]+.

**Example 143**

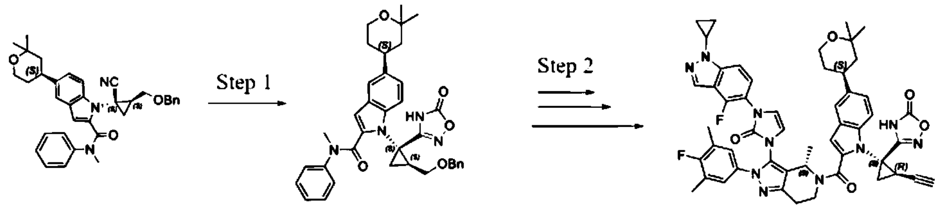


Step 1: 1-((1S,2S)-2-((benzyloxy)methyl)-1-(5-carbonyl-4,5-dihydro-1,2,4-oxadiazol-3-yl)cyclopropyl)-5-((S)-2,2-dimethyltetrahydro-2H-pyran-4-yl)-N-methyl-N-phenyl-1H-indol-2-carboxamide

At room temperature, 1-((1S,2S)-2-((benzyloxy)methyl)-1-cyanocyclopropyl)-5-((S)-2,2-dimethyltetrahydro-2H-pyran4-yl)-N-methyl-N-phenyl-1H-indol-2-carboxamide (0.55 g, 1 mmol) was dissolved in ethanol (8 mL). Then triethylamine (0.30 g, 3 mmol) and hydroxylamine hydrochloride (0.14 g, 2 mmol) were added sequentially, and nitrogen gas was replaced. The mixture was heated to 90°C and reacted for 3 hours. When LCMS indicated the end of the reaction, the reaction product was cooled to room temperature and spin dried. The residue was dissolved in ethyl acetate (15 mL), and then washed sequentially with saturated sodium bicarbonate solution (10 mL×3) and saturated saline solution (15 mL×3). The organic phase was dried with anhydrous sodium sulfate, filtered, and spin dried. The residue was dissolved in tetrahydrofuran (5 mL). Then N, N-carbonyl diimidazole (0.32 g, 2 mmol) and 1,8-diazabicyclo[5.4.0]undec-7-ene (0.45 g, 3 mmol) were added sequentially, and nitrogen gas was replaced. The mixture was heated to 55°C and reacted for 2 hours. When LCMS indicated the end of the reaction, the reaction product was cooled to room temperature and spin dried. The

residue was dissolved in ethyl acetate (30 mL), and then washed sequentially with saturated sodium bicarbonate solution (15 mL×2) and saturated saline solution (15 mL×2). The organic phase was dried with anhydrous sodium sulfate, filtered, and spin dried. The residue was separated by Flash column chromatography to obtain the target compound as a white solid (0.42 g, yield 70%).

MS m/z (ESI): 607.3[M+H]+.

Step 2: 3-((1S,2R)-1-(2-((S)-3-(3-(1-cyclopropyl-4-fluoro-1H-indazol-5-yl)-2-carbonyl-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indol-1-yl)-2-ethynylcyclopropyl)-1,2,4-oxadiazol-5(4H)-one

1-((1S,2S)-2-((benzyloxy)methyl)-1-(5-carbonyl-4,5-dihydro-1,2,4-oxadiazol-3-yl)cyclopropyl)-5-((S)-2,2-dimethyltetrahydro-2H-pyran-4-yl)-N-methyl-N-phenyl-1H-indol-2-carboxamide was used as the starting material, and the target compound was obtained following Step 2 of Example 127, Step 3 of Example 25, and Steps 5, 6, and 7 of Example 135.

$^1$H NMR (400 MHz, DMSO-$d_6$) δ [12.49 (s) and 11.96 (s), total 1H], 8.29 (s, 1H), 7.68-7.60 (m, 1H), 7.54-7.27 (m, 4H), 7.20-7.08 (m, 3H), 6.98-6.74 (m, 2H), [5.77-5.60 (m) and 5.33-5.17 (m), total 1H], [4.82-4.69 (m) and 4.51-4.35 (m), total 1H], 3.88-3.78 (m, 1H), 3.72-3.47 (m, 3H), 3.22- 3.16 (m, 1H), 3.17-3.01 (m, 1H), 2.91-2.76 (m, 2H), 2.33-2.28 (m, 1H), 2.26 (s, 6H), 2.01-1.95 (m, 30 1H),1.67-1.49 (m, 6H), 1.38-1.27 (m, 5H), 1.18-1.13 (m, 7H).

MS m/z (ESI): 919.4[M+H]+.

**Example 144**

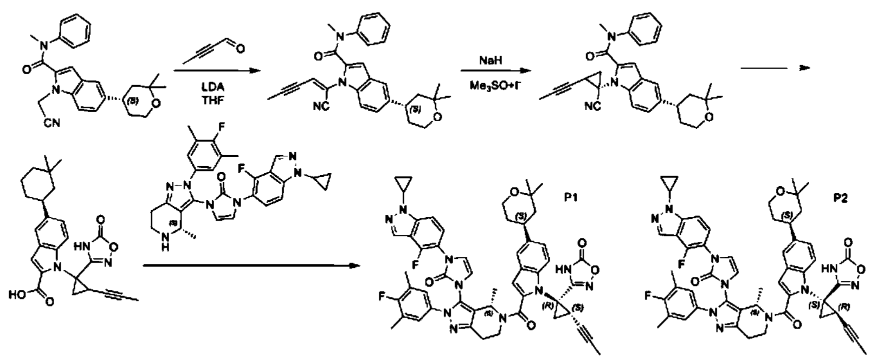


Step 1: (S)-1-(1-cyanopentyl-1-alkenyl-3-alkynyl-1-yl)-5-(2,2-dimethyltetrahydro-2H-pyran-4-yl)-N-methyl-N-phenyl-1H-indole-2-carboxamide.

(S)-1-(cyanomethyl)-5-(2,2-dimethyltetrahydro-2H-pyran-4-yl)-N-methyl-N-phenyl-1H-indole-2-carboxamide (1.8 g, 4.48 mmol) and butyl-2-alkynal (915.56 mg, 13.45 mmol) were dissolved in anhydrous tetrahydrofuran (18 mL), and lithium diisopropylamide (2 M, 4.48 mL) was added under nitrogen protection at -40°C. The mixture was stirred at -40°C for 2 h. The reaction solution was slowly raised to room temperature. LCMS shows that there was a target product generated. The crude reaction solution was directly used in the next step.

MS m/z (ESI): 452.2 [M+1].

Step 2: 1-(1-cyano-2-(propyl-1-alkynyl-1-yl)cyclopropyl)-5-((S)-2,2-dimethyltetrahydro-2H-pyran-4-yl)-N-methyl-N-phenyl-1H-indole-2-carboxamide

Trimethylsulfoxonium iodide (1.46 g, 6.64 mmol) was dissolved in dimethyl sulfoxide (8 mL), and sodium hydrogen (265.72 mg, 6.64 mmol, w/w 60%) was added under nitrogen protection. The mixture was stirred at 18°C for 3 h. Then, the reaction solution was added to the crude solution obtained in Step 1 at 0°C. The mixture was stirred overnight at room temperature. The mixture was quenched with saturated brine (50 mL), extracted with ethyl acetate (30 mL×2), merged with organic phases, sequentially washed with saturated brine (50 mL×4), dried with anhydrous sodium sulfate, and concentrated under reduced pressure. The residue was rapidly purified by silica gel chromatography (eluting with petroleum ether: ethyl acetate = 100:0 to 40:60) to obtain a crude product. The crude product was subjected to high-performance liquid chromatography separation to obtain a product ((0.26 g, 558.44 μmol, yield: 12.61%),), and the product

was white solid.

MS m/z (ESI): 466.2 [M+1].

Step 3: 5-((S)-3,3-dimethylcyclohexyl)-1-(1-(5-carbonyl-4,5-dihydro-1,2,4-oxadiazol-3-yl)-2-(propyl-1-alkynyl-1-yl)cyclopropyl)-1H-indole-2-carboxylic acid

A target compound was synthesized with reference to Step 4 and Step 5 of the intermediate A.

MS m/z(ESI): 434.2 [M+1].

Step 4: 3-((1S,2R)-1-(2-((S)-3-(3-(1-cyclopropyl-4-fluoro-1H-indazole-5-yl)-2-carbonyl-2,3-dihydro-1H-imidazole-1-yl)-2-(4-fluoro-3,5-dimethylphenyl)-4-methyl-4,5,6,7-tetrahydro-2H-pyrazolo[4,3-c]pyridin-5-carbonyl)-5-((S)-2,2-dimethyltetrahydro-2H-pyrano-4-yl)-1H-indole-1-yl)-2-(propyl-1-alkynyl-1-yl)cyclopropyl)-1,2,4-oxadiazol-5(4H)-one

A crude product was synthesized with reference to Step 3 of the intermediate C and the intermediate D, and the crude product was subjected to high-performance liquid chromatography separation to obtain P1, P2, and P2 as the target compounds, which is the Example 144.

$^1$H NMR (400 MHz, DMSO) 8, [12.42 (s) and 11.92 (s), total 1H], 8.28 (s, 1H), 7.77-7.59 (m, 1H), 7.58-7.23 (m, 4H), 7.23-7.02 (m, 3H), 7.02-6.85 (m, 2H), [5.83-5.53 (m), 5.38-5.05 (m), total 1H], [4.88-4.61 (m), 4.56-4.23 (m) , total 1H], 3.93-3.78 (m, 1H), 3.78-3.41 (m, 3H), 3.23-2.96 (m, 2H), 2.96-2.64 (m, 2H), 2.26 (s, 6H), 2.04-1.87 (m, 1H), 1.87-1.40 (m, 9H), 1.40-0.96 (m, 12H).

MS m/z (ESI): 933.4 [M+1].

**Example 145**

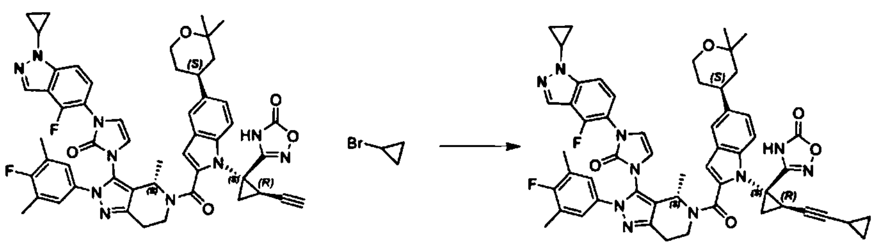

3-((1S,2R)-1-(2-((S)-3-(3-(1-cyclopropyl-4-fluoro-1H-indazole-5-yl)-2-carbonyl-2,3-dihydro-1H-imidazole-1-yl)-2-(4-fluoro-3,5-dimethylphenyl)-4-methyl-4,5,6,7-tetrahydro-2H-pyrazole[4,3-c]pyridin-5-carbonyl)-5-((S)-2,2-dimethyltetrahydro-2H-pyran-4-yl)-1H-indole-1-yl)-2-ethynylcyclopropyl)-1,2,4-oxadiazol-5(4H)-one (100 mg, 108.81 umol), [μ2-N2,N2'-bis(3-formyl-4-oxide sodium benzylene sulfonate)benzene-1,3-dicarbonylhydrazide]bis[hydrate copper (II)] monohydrate (7.79 mg, 10.88 umol), potassium carbonate (30.07 mg, 217.63 umol), and cyclopropyl bromide (26.33 mg, 217.63 umol) were dissolved in ethanol (5 mL), and the reaction solution was stirred for 10 h under the nitrogen protection at 80°C. The reaction solution was quenched with water (5 mL), extracted with ethyl acetate (5 mL×2), merged with organic phase, washed with saturated sodium chloride (5 mL), dried with anhydrous sodium sulfate, filtered and concentrated, and then the residue was purified by silica gel column chromatography with petroleum ether and ethyl acetate in the eluent system to obtain a target product (10 mg), yield: 9.58%.

$^1$H NMR (400 MHz, DMSO) 3 [12.36 (s) and 11.93 (s), total 1H], 8.28 (s, 1H), 7.73-7.56 (m, 1H), 7.56-7.23 (m, 4H), 7.22-6.97 (m, 3H), 6.98-6.67 (m, 2H), [5.84-5.53 (m), 5.35-5.05 (m), total 1H], 4.87-4.23 (m, 1H), 3.92-3.43 (m, 4H), 3.21-2.64 (m, 4H), 2.26 (s, 6H), 2.09-1.43 (m, 8H), 1.38-0.99 (m, 12H), 0.85-0.69 (m, 2H), 0.60-0.37 (m, 2H).

MS m/z (ESI): 959.4 [M+1].

Example 146

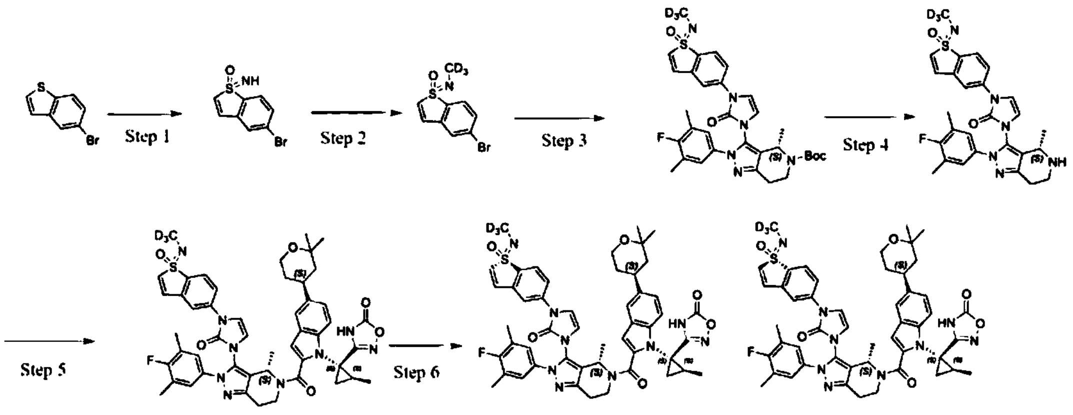


146-1 146-2

Step 1: 5-bromobenzo[b]thiophene (453 mg, 2.13 mmol) and ammonium carbamate (664.10 mg, 8.51 mmol) were dissolved in methanol (15 mL), and iodophenylacetic acid (2.05 g, 6.38 mmol) was added under nitrogen protection. The mixture was stirred at 25°C for 12 h. The mixture was evaporated and dried under reduced pressure, quenched with saturated brine (100 mL), extracted with ethyl acetate (30 mL×2), merged with organic phase, sequentially washed with saturated brine (50mL×2), dried with anhydrous sodium sulfate, and concentrated under reduced pressure. The residue was rapidly purified by silica gel chromatography (eluting with petroleum ether: ethyl acetate = 100:0 to 0:100) to obtain a target product (410 mg, white solid) with a yield: 79.0%.

MS m/z (ESI): 243.9, 245.9 [M+1].

Step 2: 5-bromo-1-imino-1H-1l4-benzo[b]thiophene 1-oxide (410 mg, 1.68 mmol) was dissolved in tetrahydrofuran (10 mL), and sodium hydrogen (100.77 mg, 2.52 mmol, 60%w/w) was added under nitrogen protection. The mixture was stirred at 25°C for 0.5 h. Methyl iodide (486.94 mg, 3.36 mmol) was added under nitrogen protection. The mixture was stirred at 25°C for 12 h. The reaction solution was quenched with saturated salt water (50 mL), the mixture was separated, the organic phase was dried with anhydrous sodium sulfate, and the mixture was dried by distillation under reduced pressure to obtain a crude product; and the crude product was rapidly purified with a silica gel column (petroleum ether: ethyl acetate = 1:1) to obtain a target product (260 mg, white solid), yield: 59.2%.

MS m/z (ESI): 261.0,263.0 [M+1].

Step 3: 5-bromo-1-((methyl-d3)imino)-1H-1l4-benzo[b]thiophene 1-oxide was used as the raw material to obtain the target product with reference to the Step 2 in the Example 25.

MS m/z (ESI): 622.2 [M+1].

Step 4: (4S)-2-(4-fluoro-3,5-dimethylphenyl)-4-methyl-3-(3-(1-((methyl-d3)imino)-1-hydroxy-1H-1l4-benzo[b]thiophene-5-yl)-2-carbonyl-2,3-dihydro-1H-imidazole-1-yl)-2,4,6,7-tetrahydro-5H-pyrazolo[4,3-c]pyridin-5-carboxylate tert-butylester was used as the raw material to obtain the target product with reference to Step 3 in the Example 25.

MS m/z (ESI): 522.2 [M+1].

Step 5: (4S)-2-(4-fluoro-3,5-dimethylphenyl)-4-methyl-3-(3-(1-((methyl-d3)imino)-1-hydroxy-1H-1l4-benzo[b]thiophene-5-yl)-2-carbonyl-2,3-dihydro-1H-imidazol-1-yl)-2,4,6,7-tetrahydro-5H-pyrazolo[4,3-c]pyridin-5-carboxylic acid tert-butylester was used as the raw material to obtain the target product with reference to Steps 3-4 in the Example 25.

MS m/z (ESI): 915.3 [M+1].

Step 6: After chiral split, 146-1 and 146-2 were obtained.

MS m/z (ESI): 915.3 [M+1].

**Example 147**

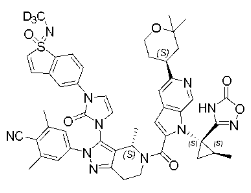

5-bromobenzo[b]thiophene and the intermediate G were used as the initial raw materials to obtain a target product with reference to the Example 146.

MS m/z (ESI): 923.4 [M+1].

**Example 148**

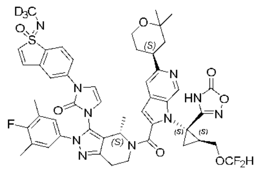

5-bromobenzo[b]thiophene was used as the initial raw material to obtain a target product with reference to the Example 135 and the Example 146.

MS m/z (ESI): 982.4 [M+1].

**Example 149**

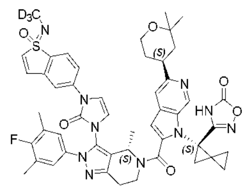

5-bromobenzo[b]thiophene was used as the initial raw material to obtain a target product with reference to the Example 135 and the Example 146.

MS m/z (ESI): 928.4 [M+1].

**Example 150**

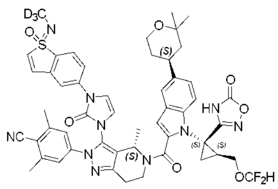

5-bromobenzo[b]thiophene and the intermediate G were used as the initial raw materials to obtain a target product with reference to the Example 146 and the Example 135.

MS m/z (ESI): 988.4 [M+1].

**Example 151**

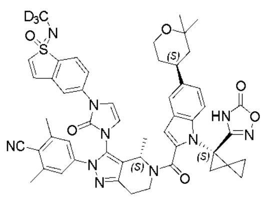

5-bromobenzo[b]thiophene and the intermediate G were used as the initial raw materials to obtain a target product with reference to the Example 146 and the Example 135.

MS m/z (ESI): 934.4 [M+1].

**Example 152**

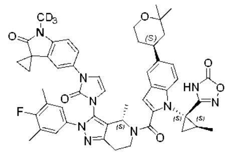

5'-bromo-spiro[cyclopropane-1,3'-dihydroindole]-2'-one was used as the initial raw material to obtain a target product with reference to the Example 146.

MS m/z (ESI): 909.4 [M+1].

**Example 153**

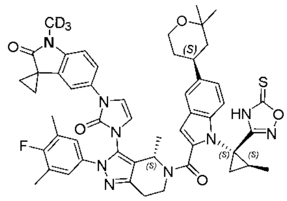

5'-bromo-spiro[cyclopropane-1,3'-dihydroindole]-2'-one was used as the initial raw material to obtain a target product with reference to the Example 146 and the intermediate F.

MS m/z (ESI): 925.4 [M+1].

**Example 154**

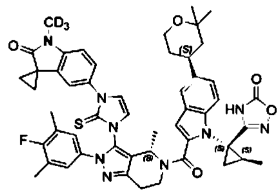

5'-bromo-1'-(methyl-d3)spiro[cyclopropane-1,3'-dihydroindole]-2'-one was used as the initial raw material to obtain a target compound with reference to the Example 3 and the Example 127. MS m/z (ESI): 925.4 [M+1].

**Example 155**

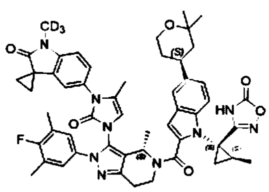

5'-bromo-1'-(methyl-d3)spiro[cyclopropane-1,3'-dihydroindole]-2'-one was used as the raw material to obtain a target product with reference to the Example 45 and the Example 127. MS m/z (ESI): 923.4 [M+1].

**Example 156**

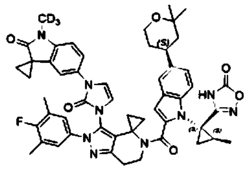

5'-bromo-1'-(methyl-d3)spiro[cyclopropane-1,3'-dihydroindole]-2'-one was used as the raw material to obtain a target product with reference to the Example 51 and the Example 127. MS m/z (ESI): 921.4 [M+1].

**Example 157**

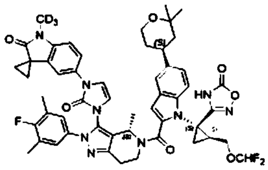

5'-bromo-1'-(methyl-d3)spiro[cyclopropane-1,3'-dihydroindole]-2'-one was used as the raw material to synthesize a target compound with reference to the Example 141.
MS m/z (ESI): 975.4 [M+1].

**Example 158**

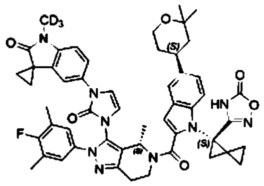

5'-bromo-1'-(methyl-d3)spiro[cyclopropane-1,3'-dihydroindole]-2'-one was used as the raw material to synthesize a target compound with reference to the Example 79.
MS m/z (ESI): 921.4 [M+1].

**Example 159**

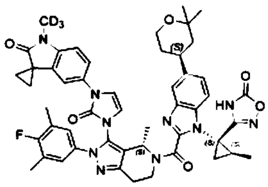

5'-bromo-1'-(methyl-d3)spiro[cyclopropane-1,3'-dihydroindole]-2'-one was used as the raw material to synthesize a target compound with reference to an Example 364.

MS m/z (ESI): 910.4 [M+1].

**Example 160**

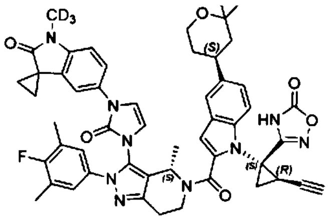

5'-bromo-1'-(methyl-d3)spiro[cyclopropane-1,3'-dihydroindole]-2'-one was used as the raw material to synthesize a target compound with reference to the Example 143.

MS m/z (ESI): 919.4 [M+1].

**Example 161**

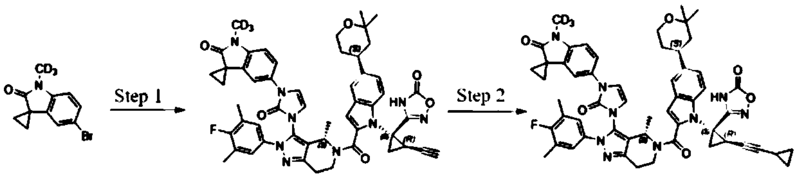


Step 1: 5'-bromo-1'-(methyl-d3)spiro[cyclopropane-1,3'-dihydroindole]-2'-one was used as the raw material to obtain a target product with reference to the Example 135.

MS m/z (ESI): 919.4 [M+1].

Step 2: The target product was obtained with reference to the Example 145.

MS m/z (ESI):959.4 [M+1].

**Example 162**

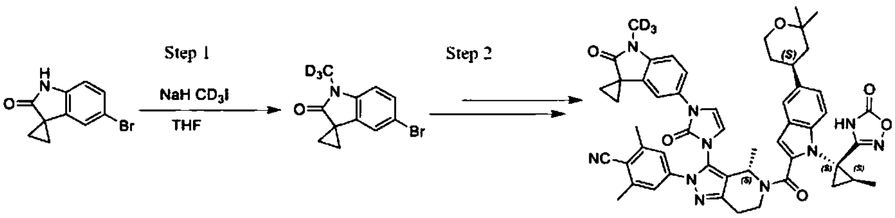


Step 1: 5'-bromo-spiro[cyclopropane-1,3'-dihydroindole]-2'-one (200 mg, 840.05 μmol) was dissolved in anhydrous tetrahydrofuran (10 mL), sodium hydrogen (67.20 mg, 1.68 mmol, 60% purity) was added at room temperature of 20°C, and stirred for 0.5 h at room temperature, and then deuteroiodemethane (243.54 mg, 1.68 mmol) was added dropwise. The reaction system was stirred at room temperature of 20°C for 3.5 h. After the reaction was completed, a saturated ammonium chloride solution (10 mL) was added dropwise to quench, the mixture was extracted with ethyl acetate (20 mL x2), washed with saturated saline (20 mLx2), dried with anhydrous sodium sulfate, and filtered and concentrated to obtain a crude product 5'-bromo-1'-(methyl-d3)spiro[cyclopropane-1,3'-dihydroindole]-2'-one (210 mg, crude). The crude product was used directly in the next step of the reaction and not further purified.

MS m/z (ESI): 255.0, 257.0 [M+1].

Step 2: 5'-bromo-1'-(methyl-d3)spiro[cyclopropane-1,3'-dihydroindole]-2'-one was used as the raw material to

obtain a target product with reference to the Example 147.

$^1$H NMR (400 MHz, DMSO) δ [11.97 (s) and 11.74 (s), total 1H], 7.50 (d, 2H), 7.38 (t, 2H), 7.29-7.24 (m, 4H), 7.16 (d, 1H), 6.96 (s, 1H), 6.76 (s, 1H), 5.59-5.57 (q, 1H), 4.41-4.38 (m, 1H), 3.70-3.60(m, 5H), 3.50-3.46(m, 1H), 3.25-3.17(m, 1H), 3.03-2.89(m, 3H), 2.47 (s, 6H), 1.78-1.49(m, 7H), 1.36 (d, 2H), 1.27 (s, 3H), 1.24 (s, 2H), 1.18-1.13 (m, 5H)

MS m/z (ESI):917.4 [M+1].

**Example 163**

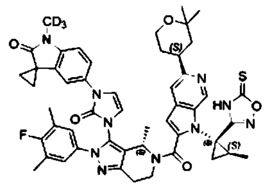

5'-bromo-1'-(methyl-d3)spiro[cyclopropane-1,3'-dihydroindole]-2'-one was used as the raw material to obtain a target product with reference to the Example 137.

MS m/z (ESI): 926.4 [M+1].

**Example 164**

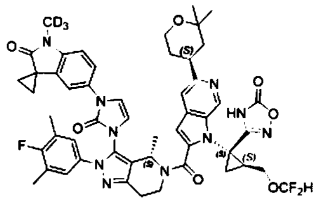

5'-bromo-1'-(methyl-d3)spiro[cyclopropane-1,3'-dihydroindole]-2'-one was used as the raw material to obtain a target product with reference to the Example 148.

MS m/z (ESI): 976.4 [M+1].

**Example 165**

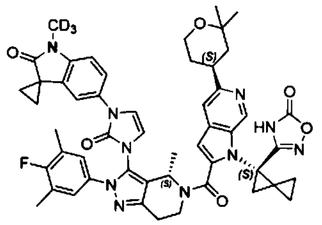

5'-bromo-1'-(methyl-d3)spiro[cyclopropane-1,3'-dihydroindole]-2'-one was used as the raw material to obtain a target product with reference to the Example 149.

MS m/z (ESI): 922.4 [M+1].

**Example 166**

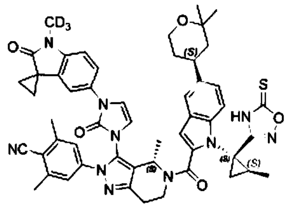

5'-bromo-1'-(methyl-d3)spiro[cyclopropane-1,3'-dihydroindole]-2'-one was used as the raw material to obtain a target product with reference to the Example 136.

MS m/z (ESI): 932.4 [M+1].

**Example 167**

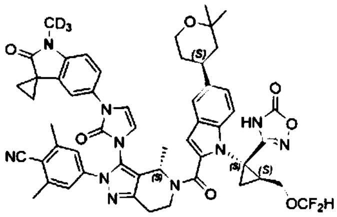

5'-bromo-1'-(methyl-d3)spiro[cyclopropane-1,3'-dihydroindole]-2'-one was used as the raw material to obtain a target product with reference to the Example 150.

MS m/z (ESI): 982.4 [M+1].

**Example 168**

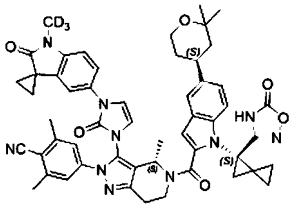

5'-bromo-1'-(methyl-d3)spiro[cyclopropane-1,3'-dihydroindole]-2'-one was used as the raw material to obtain a target product with reference to the Example 151.

MS m/z (ESI): 928.4 [M+1].

**Example 169**

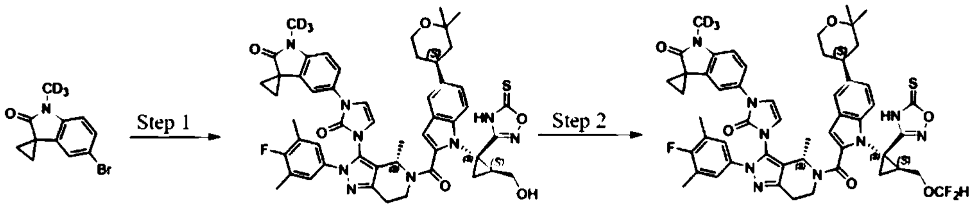


Step 1: 5'-bromo-1'-(methyl-d3)spiro[cyclopropane-1,3'-dihydroindole]-2'-one was used as the raw material to obtain a target product with reference to the Example 135.

MS m/z (ESI):941.4 [M+1].

Step 2: A target product was obtained with reference to the Example 141.
MS m/z (ESI):991.4 [M+1].

**Example 170**

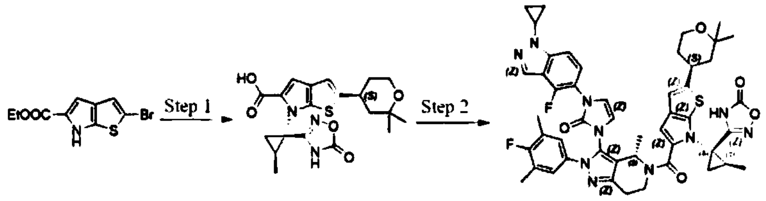


Step 1: Ethyl 2-bromo-6H-thiophene[2,3-b]pyrrole-5-carboxylate was used as the raw material to obtain the target product with reference to the intermediate F.
MS m/z (ESI):418.1 [M+1].
Step 2: A target product was obtained with reference to the Example 25.
MS m/z (ESI):915.4 [M+1].

**Example 171**

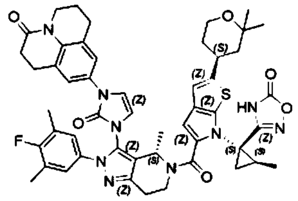

9-bromo-2,3,6,7-tetrahydro-1H,5H-pyridino[3,2,1-ij]quinoline-5-one was used as raw material to obtain a target product with reference to the Example 170.
MS m/z (ESI): 926.4 [M+1].

**Example 172**

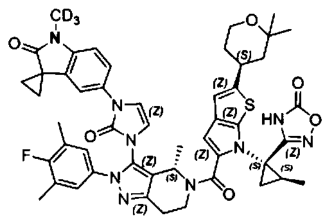

5'-bromo-1'-(methyl-d3)spiro[cyclopropane-1,3'-dihydroindole]-2'-one was used as the raw material to obtain a target product with reference to the Example 170.
MS m/z (ESI):915.4 [M+1].

**Example 173**

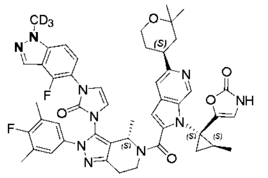

A target product was obtained with reference to a synthesis method in an Example 370.
MS m/z (ESI): 886.4 [M+1]

**Example 174**

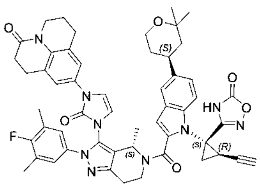

A target product was obtained with reference to a synthesis method in the Example 143.
$^1$H NMR (400 MHz, DMSO-$d_6$) δ [12.47 (s) and 11.94 (s), total 1H], 7.54-7.51 (m, 1H), 7.437.09 (m, 7H), 6.98-6.70 (m, 2H), [5.72-5.58 (m) and 5.32-5.14 (m), total 1H], [4.81-4.67 (m) and 4.49-4.35 (m), total 1H], 3.75-3.47 (m, 5H),3.21-3.01 (m, 2H), 2.99-2.73 (m, 6H), 2.56-2.51 (m, 2H), 2.28-2.15 (m, 7H), 1.96-1.92 (m, 1H), 1.90-1.78 (m, 2H), 1.73-1.41 (m, 6H), 1.28-1.14(m, 8H).
MS m/z (ESI): 930.4[M+1],

**Example 175**

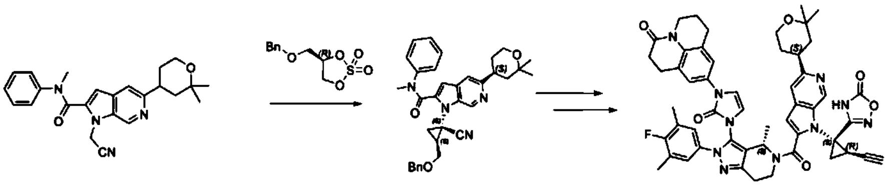

Step 1: 1-(cyanomethyl)-5-(2,2-dimethyltetrahydro-2H-pyrano-4-yl)-N-methyl-N-phenyl-1H-pyrrolo[2,3-c]pyridine-2-carboxamide and (R)-4-((benzyloxy)methyl)-1,3,2-dioxathipentanecyclo2,2-dioxide were used as the raw materials, and a target compound was obtained by splitting with reference to Step 6 of the intermediate F.
MS m/z (ESI): 549.2 [M+1].
Step 2: 1-((1 S,2S)-2-((benzyloxy)methyl)-1-cyanocyclopropyl)-5-((S)-2,2-dimethyltetrahydro-2H-pyrano-4-yl)-N-methyl-N-phenyl-1H-pyrrolo[2,3-c]pyridin-2-carboxamide and tert-butyl(S)-2-(4-fluoro-3,5-dimethylphenyl)-4-methyl-3-(2-carbonyl-3-(3-carbonyl-2,3,6,7-tetrahydro-1H,5H-pyridino[ 3,2,1-ij]quinoline-9-yl)-2,3-dihydro-1H-imidazole-1-yl)-2,4,6,7-tetrahydro-5H-pyrazolo[4,3-c]pyridin-5-carboxylate were used as the raw materials to obtain the target compound with reference to the Example 143.
MS m/z(ESI):931.4 [M+1].

**Example 176**

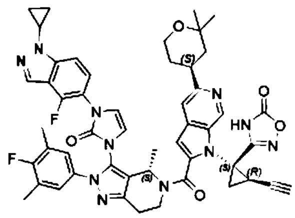

Tert-butyl(S)-3-(3-(1-cyclopropyl-4-fluoro-1H-indazole-5-yl)-2-carbonyl-2,3-dihydro-1H-imidazole-1-yl)-2-(4-fluoro-3,5-dimethylbenzene)-4-methyl-2,4,6,7-tetrahydro-5H-pyrazole[4,3-c]pyridin-5-carboxylate was used as the raw material to obtain a target product with reference to the Example 175.

MS m/z (ESI): 920.4[M+1].

**Example 177**

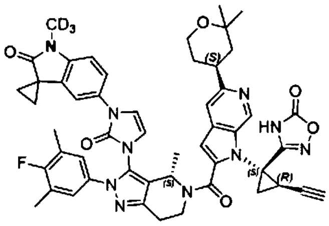

Tert-butyl(S)-2-(4-fluoro-3,5-dimethylphenyl)-4-methyl-3-(3-(1'-(methyl-d3)-2'-carbonylspiron[cyclopropane-1,3'-dihydroindole]-5'-yl)-2-carbonyl-2,3-dihydro-1H-imidazole-1-yl)-2,4,6,7-tetrahydro-5H-pyrazolo[4,3-c]pyridine-5-carboxylate was used as the raw material to obtain a target product with reference to the Example 176.

MS m/z (ESI) : 920.4[M+1].

**Example 178**

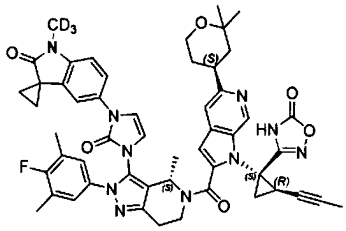

Tert-butyl(S)-2-(4-fluoro-3,5-dimethylphenyl)-4-methyl-3-(3-(1'-(methyl-d3)-2'-carbonylspiron[cyclopropane-1,3'-dihydroindole]-5'-yl)-2-carbonyl-2,3-dihydro-1H-imidazole-1-yl)-2,4,6,7-tetrahydro-5H-pyrazolo[4,3-c]pyridine-5-carboxylate was used as the raw material to obtain a target product with reference to an Example 465.

MS m/z (ESI): 934.4[M+1].

**Example 179**

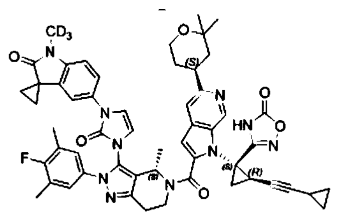

Tert-butyl(S)-2-(4-fluoro-3,5-dimethylphenyl)-4-methyl-3-(3-(1'-(methyl-d3)-2'-carbonylspiro[cyclopropane-1,3'-dihydroindole]-5'-yl)-2-carbonyl-2,3-dihydro-1H-imidazole-1-yl)-2,4,6,7-tetrahydro-5H-pyrazolo[4,3-c]pyridine-5-carboxylate was used as the raw material to obtain a target product with reference to an Example 471.
MS m/z (ESI): 960.4 [M+1].

**Example 180**

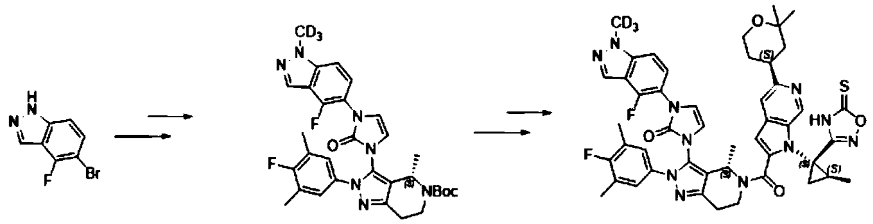

Step 1: 5-bromo-4-fluoro-1H-indazole was used as the raw material to obtain a target product with reference to Step 2-3 in the Example 145.
MS m/z (ESI): 593.2 [M+1].
Step 2: 5-((S)-2,2-dimethyltetrahydro-2H-pyrano-4-yl)-1-((1S,2S)-2-methyl-1-(5-thio-4,5-dihydro-1,2,4-oxadiazol-3-yl)cyclopropyl)-1H-pyrrolo[2,3-c]pyridin-2-carboxylic acid and tert-butyl(S)-3-(3-(4-fluoro-1-(methyl-d3)-1H-indazole-5-yl)-2-carbonyl-2,3-dihydro-1H-imidazole-1-yl)-2-(4-fluoro-3,5-dimethylphenyl)-4-methyl-2,4,6,7-tetrahydro-5H-pyrazolo[4,3-c]pyridin-5-carboxylate were used as raw materials to obtain the target product with reference to Steps 3-4 in the Example 25.
MS m/z (ESI): 903.4 [M+1].

**Example 181**

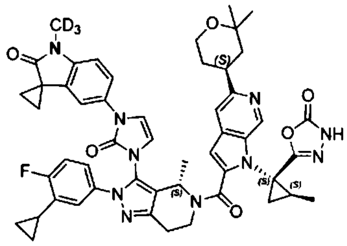

-butyl(S)-2-(3-cyclopropyl-4-fluorophenyl)-4-methyl-3-(3-(1'-(methyl-d3)-2'-carbonylspiro[cyclopropane-1,3'-dihydroindole]-5'-yl)-2-carbonyl-2,3-dihydro-1H-imidazole-1-yl)-2,4,6,7-tetrahydro-5H-pyrazolo[4,3-c]pyridin-5-carboxylate was used as the raw material to obtain a target product with reference to a synthesis method in an Example 478.
MS m/z (ESI): 922.4 [M+1].

**Example 182**

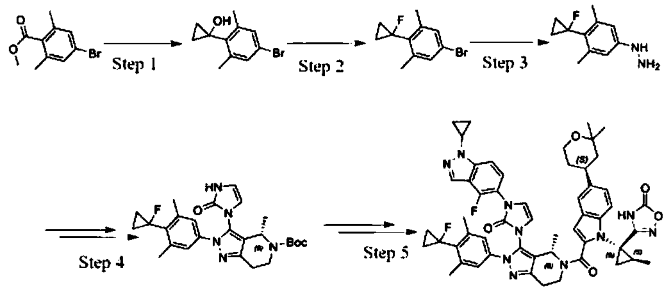


Step 1: 4-bromo-2,6-dimethylbenzoate (3 g, 12.34 mmol) and titanium (IV) isopropionate (5.26 g, 18.51 mmol) were dissolved in tetrahydrofuran (30 mL) in a 100 mL flask, then ethylmagnesium bromide (37.02 mL, 37.02 mmol, 1 M) was slowly added at 80°C, and the reaction solution was stirred at 80°C for 1 h. The reaction was stopped, saturated ammonium chloride aqueous solution (30 mL) was added to quench, the mixture was extracted with ethyl acetate (30 mL×2), merged with organic phase, washed with saturated sodium chloride (30 mL), dried with anhydrous sodium sulfate, and filtered; and the residue was purified by silica gel column chromatography with petroleum ether and ethyl acetate in the eluent system to obtain a target product (1.1 g, yellow solid), yield: 37.0%.
MS m/z (ESI): 241.0 [M+1].
Step 2: 1-(4-bromo-2,6-dimethylphenyl)cyclopropane-1-ol (1.1 g, 4.56 mmol) was dissolved in dichloromethane (10 mL), and then DAST (1.10 g, 6.84 mmol) was added at 0°C. The reaction solution was stirred at 25°C for 2 h. The reaction was stopped, water (10 mL) was added to quench, the mixture was extracted with dichloromethane (10 mL×2), merged with the organic phase, washed with saturated sodium chloride (10 mL), dried with anhydrous sodium sulfate, filtered, and concentrated under reduced pressure; and the residue was purified by silica gel column chromatography with petroleum ether and ethyl acetate in the eluent system to obtain the target product (800 mg, yellow solid), yield: 72.1%.
MS m/z (ESI): 243.0 [M+1].
Step 3: 5-bromo-2-(1-fluorocyclopropyl)-1,3-dimethylbenzene (800 mg, 3.29 mmol), cuprous iodide (125.34 mg, 658.11 μmol), sodium phosphate (1.08 g, 16.58 mmol), 85% hydrazine hydrate (1 mL) were dissolved in PEG-400 (10 mL) in a 50 mL flask, and the reaction solution was stirred at 120°C for 6 h under nitrogen protection. The reaction was stopped, saturated ammonium chloride aqueous solution (30 mL) was added to quench, the mixture was extracted with ethyl acetate (30 mL×2), merged with the organic phase, washed with saturated sodium chloride (30 mL), dried with anhydrous sodium sulfate, and filtered; and the residue was purified by silica gel column chromatography with petroleum ether and ethyl acetate in the eluent system to obtain the target product (400 mg, yellow solid), yield: 62.5%.
MS m/z (ESI): 195.1 [M+1].
Step 4: (4-(1-fluorocyclopropyl)-3,5-dimethylphenyl)hydrazine was used as the raw material to obtain the target product with reference to the synthesis method of the intermediate G.
MS m/z (ESI): 482.3 [M+1].
Step 5: (S)-2-(4-(1-fluorocyclopropyl)-3,5-dimethylbenzene)-4-methyl-3-(2-carbonyl-2,3-dihydro-1H-imidazole-1-yl)-2,4,6,7-tetrahydro-5H-pyrazolo[4,3-c]pyridine-5-tert-butyl carboxylate was used as the raw material to obtain the target product with reference to the synthesis method in the Example 25.
MS m/z (ESI): 949.4 [M+1].

**Example 183**

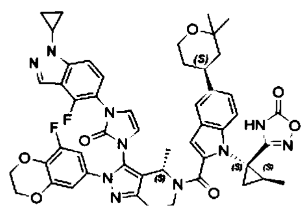

(8-fluoro-2,3-dihydrobenzo[b][1,4]dioxin-6-yl)hydrazine was used as the initial raw material to obtain a target product with reference to the intermediate G and the Example 136.
MS m/z (ESI): 939.4 $[M+H]^+$.

**Example 184**

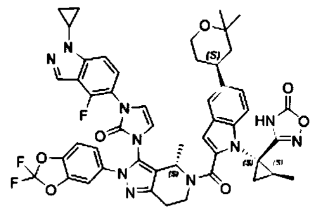

(2,2-difluorobenzo[d][1,3]dioxazole-5-yl)hydrazine was used as the initial raw material to obtain 3-((1S,2S)-1-(2-((S)-3-(3-(1-cyclopropyl-4-fluoro-1H-indazole-5-yl)-2-carbonyl-2,3-dihydro-1H-imidazole-1-yl)-2-(2,2-difluorobenzo[d][1,3]dioxazole-5-yl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indole-1-yl)-2-methylcyclopropyl)-1,2,4-oxadiazole-5(4H)-one with reference to intermediate G and the Example 136.

MS m/z (ESI): 943.3 $[M+H]^+$.

**Example 185**

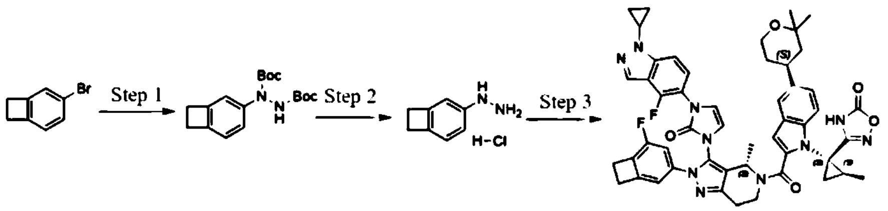


Step 1: 3-bromodicyclo[4.2.0]oct-1(6),2,4-triene (1 g, 5.46 mmol) was dissolved in tetrahydrofuran (20 mL), and cooled in a dry ice ethanol bath under nitrogen protection, and n-butyllithium (2.5 M, 2.40 mL) was added. The mixture was stirred at -78°C for 0.5 h. Then, a tetrahydrofuran solution (4 mL) of di-tert-butyl azodicarboxylate (1.26 g, 5.46 mmol) was added under the cooling of a dry ice ethanol bath. The reaction solution was slowly raised to room temperature and stirred for 12 h. The mixture was quenched with saturated brine (50 mL), extracted with ethyl acetate (50 mL), merged with the organic phases, sequentially washed with saturated brine (50 mL×2), dried with anhydrous sodium sulfate, and concentrated under reduced pressure. The residue was rapidly purified by silica gel chromatography (eluting with petroleum ether: ethyl acetate = 100:0 to 80:20) to obtain a target product which is a light brown solid (1.5 g, 4.49 mmol, yield: 82.11%).

1H NMR (400 MHz, CDC13) 5 7.24-7.14 (m, 1H), 7.14-7.02 (m, 1H), 7.02-6.93 (m, 1H), 6.81-6.59 (m, 1H), 3.13 (s, 4H), 1.49 (s, 18H).
MS m/z (ESI):335.2 [M+H]

Step 2: Di-tert-butyl1-(bicyclo[4.2.0]oct-1(6),2,4-trien-3-yl)hydrazine-1,2-dicarboxylate (1 g, 6 3 mmol) was dissolved in NMP (8 mL), methanesulfonic acid (0.65 g, 6.6 mmol) was added, and the mixture was stirred at an external temperature of 80°C for 7 h. After cooling the reaction solution to room temperature, toluene (15 mL), potassium carbonate (0.46 g) and water (6 g) were added, and the reaction solution was stirred at room temperature for 10 min. After the aquifer was removed, the organic phase was used directly in the next step.
MS m/z (ESI):135.1 [M+H]

Step 3: 5-bromo-1-cyclopropyl-4-fluoro-1H-indazole was used as the raw material to obtain the target product with reference to the Steps 1-2 in an Example 515.
MS m/z (ESI): 907.4 [M+H]

**Example 186**

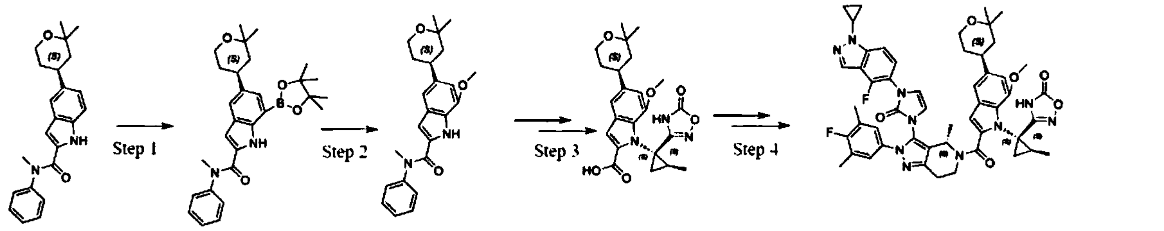


Step 1: 5-[(4S)-2,2-dimethyltetrahydropyran-4-yl]-N-methyl-N-phenyl-1H-indole-2-carboxamide (1 g, 2.76 mmol), and 4,4,5,5-tetramethyl-2-(4,4,5,5-tetramethyl-1,3,2-dioxaboran-2-yl)-1,3,2-dioxaboran-pentacyclo(1.05 g, 4.14 mmol) were dissolved in THF (10 mL) in a 50 mL flask, 4,4'-Di-tert-butyl-2,2'-dipyridyl (74.05 mg, 275.89 μmol) and Di-μ-methylcarbonylbis(1,5-cyclooctadiene)diiridium(I) (180.65 mg, 275.89 μmol) were added, then the reaction solution was stirred at 60°C for 1 h under nitrogen protection. The reaction was stopped, an aqueous solution (10 mL) was added to quench, the mixture was extracted with dichloromethane (10 mL×2), merged with organic phase, washed with saturated sodium chloride (10 mL), dried with anhydrous sodium sulfate, and filtered; and the residue was purified by silica gel column chromatography with petroleum ether and ethyl acetate in the eluent system to obtain the target product (900 mg, yellow solid), yield: 66.7%.

MS m/z (ESI): 489.3 [M+1].

Step 2: (S)-5-(2,2-dimethyltetrahydro-2H-pyran-4-yl)-N-methyl-N-phenyl-7-(4,4,5,5-tetramethyl-1,3,2-dioxaboran-2-yl)-1H-indole-2-carboxamide (900 mg, 1.84 mmol), DMAP (450.22 mg, 3.69 mmol), and copper acetate (183.94 mg, 921.33 μmol) were dissolved in MeOH in a 50 mL flask (5 mL) and DCM (25 mL), and added to a 4A molecular sieve (5g), and then the reaction solution was stirred at 25°C for 1 h under nitrogen protection. The reaction was stopped, an aqueous solution (10 mL) was added to quench, the mixture was extracted with dichloromethane (10 mL×2), merged with the organic phase, washed with saturated sodium chloride (10 mL), dried with anhydrous sodium sulfate, and filtered; and the residue was purified by silica gel column chromatography with petroleum ether and ethyl acetate in the eluent system to obtain the target product (500 mg, yellow solid), yield: 69.1%.

MS m/z (ESI): 393.2 [M+1].

Step 3: (S)-5-(2,2-dimethyltetrahydro-2H-pyran-4-yl)-7-methoxy-N-methyl-N-phenyl-1H-indole-2-carboxamide was used as the raw material to obtain the target product with reference to the synthesis method in Steps 5-8 of the intermediate F.

MS m/z (ESI): 442.2 [M+1].

Step 4: 5-((S)-2,2-dimethyltetrahydro-2H-pyran-4-yl)-7-methoxy-1-((1S,2S)-2-methyl-1-(5-carbonyl-4,5-dihydro-1,2,4-oxadiazol-3-yl)cyclopropyl)-1H-indole-2-carboxylic acid was used as the raw material to obtain the target product with reference to the synthesis method in the Step 4 of the Example 25.

MS m/z (ESI): 939.4 [M+1].

**Example 187**

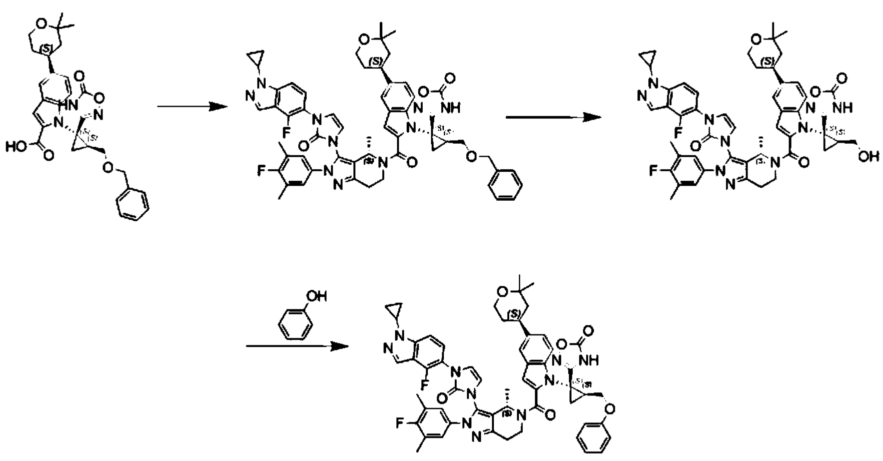

Step 1: A target compound was obtained with reference to step 4 of Example 25.

MS m/z (ESI): 1015.4[M+1].

Step 2:

3-((1S,2S)-2-((benzyloxy)methyl)-1-(2-((S)-3-(3-(1-cyclopropyl-4-fluoro-1H-indazol-5-yl)-2-carbonyl-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indol-1-yl)cyclopropyl)-1,2,4-oxadiazol-5(4H)-one (0.51 g, 0.5 mmol) was dissolved in trifluoroacetic acid (20 mL) at room temperature. The mixture was heated to 80°C, reacted for 14 hours, and spin-dried. The residue was dissolved in ethyl acetate (30 mL), then washed with a saturated sodium bicarbonate solution (20 mL×2), and saturated brine (20 mL×2) in sequence. The organic phase was dried over anhydrous sodium sulfate, filtered, and spin-dried. The residue was separated by Flash column chromatography (dichloromethane: methanol=95:5 for elution) to obtain a target compound as a white solid (0.26 g, yield of 55%).
MS m/z (ESI): 925.4[M+H]+.
Step 3: A target product was obtained from raw materials with reference to the literature [Chemical Communications, 2016, vol. 52, no.8, pp. 1665-1668]
MS m/z (ESI): 1001.4[M+1].

**Example 188**

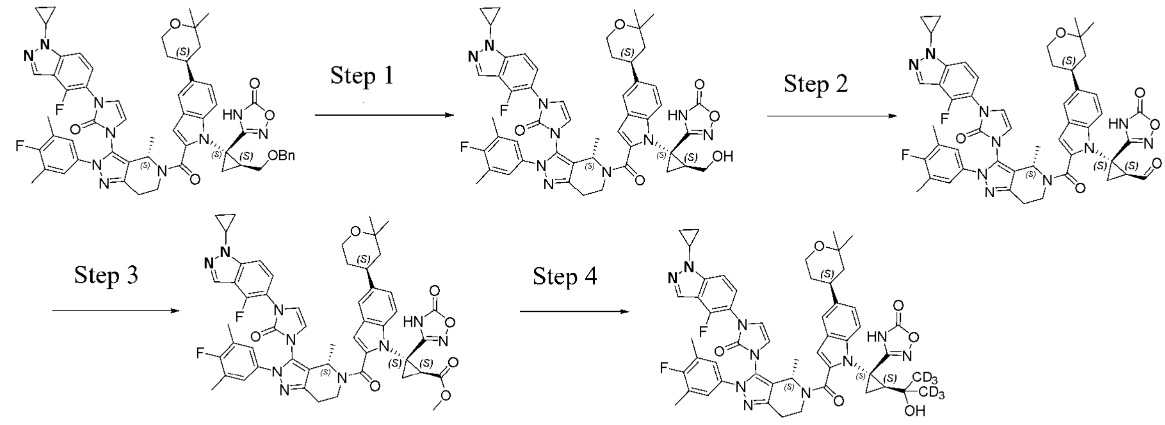


Step 1: 3-((1S,2S)-2-((benzyloxy)methyl)-1-(2-((S)-3-(3-(1-cyclopropyl-4-fluoro-1H-indazol-5-yl)-2-carbonyl-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indol-1-yl)cyclopropyl)-1,2,4-oxadiazol-5(4H)-one (0.51 g, 0.5 mmol) was dissolved in trifluoroacetic acid (20 mL) at room temperature. The mixture was heated to 80°C, and reacted for 14 hours. LCMS indicated that the reaction was complete. The mixture was spin-dried. The residue was dissolved in ethyl acetate (30 mL), and then washed with a saturated sodium bicarbonate solution (20 mL×2), and saturated brine (20 mL×2) in sequence. The organic phase was dried over anhydrous sodium sulfate, filtered, and spin-dried. The residue was separated by Flash column chromatography (dichloromethane: methanol=95:5 for elution) to obtain a target compound as a white solid (0.26 g, yield of 55%).
MS m/z (ESI): 925.4[M+H]+.
Step 2: 3-((1S,2S)-1-(2-((S)-3-(3-(1-cyclopropyl-4-fluoro-1H-indazol-5-yl)-2-carbonyl-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indol-1-yl)-2-(hydroxymethyl)cyclopropyl)-1,2,4-oxadiazol-5(4H)-one (0.26 g, 0.28 mmol) was dissolved in dichloromethane (10 mL) at room temperature, and then a Dess-Martin reagent (0.24 g, 0.56 mmol) was added. The mixture was stirred at room temperature for 2 hours. LCMS indicated that the reaction was complete. The reaction solution was diluted with dichloromethane (20 mL), and then washed with a saturated sodium bicarbonate solution (20 mL×2) and saturated brine (20 mL×2) in sequence. The organic phase was dried over anhydrous sodium sulfate, filtered, and spin-dried. The residue was used directly in a next step (0.25 g, crude)
MS m/z (ESI): 923.4[M+H]+.
Step 3: (1S,2S)-2-(2-((S)-3-(3-(1-cyclopropyl-4-fluoro-1H-indazol-5-yl)-2-carbonyl-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indol-1-yl)-2-(5-carbonyl-4,5-dihydro-1,2,4-oxadiazol-3-yl)cyclopropane-1-carbaldehyde (0.25 g, 0.27 mmol) was dissolved in tert-butyl alcohol (10 mL) at room temperature. Then, an aqueous solution (1 mL) of 2-methylbut-2-ene (0.38 g, 5.4 mmol), an aqueous solution (1 mL) of sodium dihydrogen phosphate (0.12 g, 1.03

mmol) and an aqueous solution (1 mL) of sodium chlorite (0.098 g, 1.08 mmol) were added in sequence. The mixture was stirred at room temperature for one hour. LCMS indicated that the reaction was complete. The reaction solution was diluted with ethyl acetate (30 mL). The reaction solution was washed with saturated brine (20 mL×2). The organic phase was dried over anhydrous sodium sulfate, filtered, and spin-dried. After that, the obtained crude carboxylic acid product was dissolved in methanol (10 mL) and cooled to 0°C, and then a trimethylsilyldiazomethane solution (2.0 M/ether, 0.34 mL, 0.68 mmol) was added dropwise. The mixture was stirred under ice bath for half an hour. LCMS indicated that the reaction was complete. The mixture was spin-dried. The residue was separated by Flash column chromatography to obtain a target compound (0.19 g, yield of 71%).

MS m/z (ESI): 953.4[M+H]+.

Step 4: Methyl(1S,2S)-2-(2-((S)-3-(3-(1-cyclopropyl-4-fluoro-1H-indazol-5-yl)-2-carbonyl-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indol-1-yl)-2-(5-carbonyl-4,5-dihydro-1,2,4-oxadiazol-3-yl)cyclopropane-1-carboxylate (0.19 g, 0.2 mmol) was dissolved in tetrahydrofuran (5 mL) at -78°C, nitrogen was replaced, and then (methyl-d3) magnesium bromide (1.0/tetrahydrofuran, 1 mL, 1 mmol) was added dropwise. The mixture was reacted at -78°C for one hour. LCMS indicated that the reaction was complete. The reaction solution was quenched with a saturated ammonium chloride solution (10 mL), and then washed with saturated brine (20mL × 3). The organic phase was dried over anhydrous sodium sulfate, filtered, and spin-dried. The residue was separated by Flash column chromatography to obtain a target compound (0.086 g, yield of 45%).

MS m/z (ESI): 959.2[M+H]+.

**Example 189**

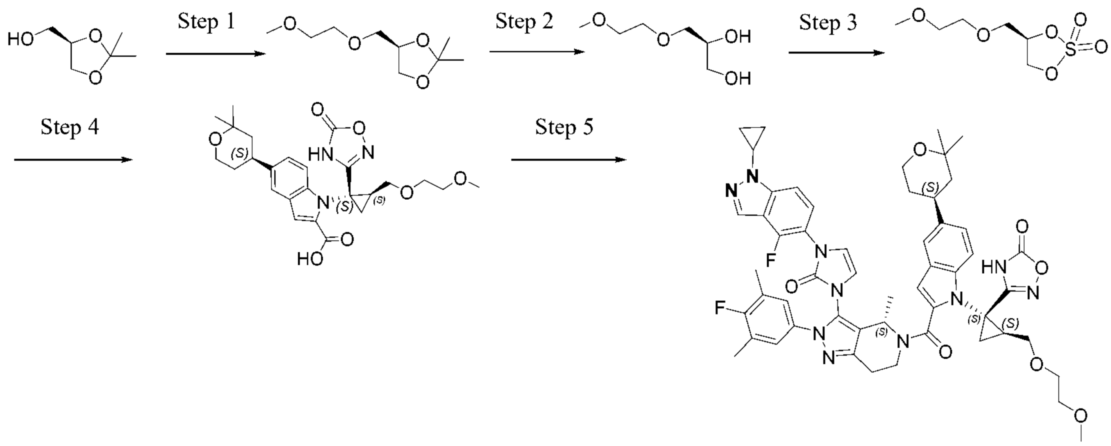


Step 1: (R)-(2,2-dimethyl-1,3-dioxolane-4-yl)methanol (1.38 g, 10.44 mmol), 1-bromo-2-methoxy-ethane (2.90 g, 20.88 mmol) and tetrabutylammonium iodide (385.70 mg, 1.04 mmol) were dissolved in toluene (10 mL) at room temperature, and then an aqueous solution (10 mL) of sodium hydroxide (4.18 g, 104.42 mmol) was added. The mixture was heated to 80°C and reacted for 14 hours. The mixture was cooled to room temperature. TLC indicated that new spots were generated. The reaction solution was extracted with ethyl acetate (20 mL×3). The organic phases were combined, dried over anhydrous sodium sulfate, filtered, and spin-dried. The residue was separated by Flash column chromatography (PE:EA=10:1-2:1 for elution) to obtain a target product as a colorless oily substance (1.40 g, yield of 70.5%).

MS m/z (ESI): 191.1[M+H]+.

Step 2: (R)-4-((2-methoxyethoxy)methyl)-2,2-dimethyl-1,3-dioxolane (1.4 g, 7.36 mmol) was dissolved in acetone (15 mL) at room temperature, and then dilute hydrochloric acid (1.0 M, 15 mmol, 15 mL) was added. The mixture was stirred at room temperature for 2 hours. TLC indicated that the reaction was complete. The acetone was removed by rotary evaporation and the aqueous phase was lyophilized to obtain a target compound as a colorless oily substance (0.80 g, yield of 72%). MS m/z (ESI): 151.0[M+H]+.

Step 3: A target product was obtained by using (S)-3-(2-methoxyethoxy)propane-1,2-diol as a raw material, with reference to steps 1 and 2 of Example 135.

MS m/z (ESI): 213.0[M+H]+.

Step 4: A target product was obtained by using (R)-4-((2-methoxyethoxy)methyl)-1,3,2-dioxathiapiprolin 2,2-dioxide as a raw material, with reference to steps 6, 7, 8 and 9 of intermediate F.

MS m/z (ESI): 486.2[M+H]+.

Step 5: A target product was obtained by using 5-((S)-2,2-dimethyltetrahydro-2H-pyran-4-yl)-1-((1S,2S)-2-((2-methoxyethoxy)methyl)-1-(5-carbonyl-4,5-dihydro-1,2,4-oxadiazol-3-yl)cyclopropyl)-1H-indole-2-carboxylic acid as a raw material, with reference to step 4 of Example 25.

$^1$H NMR (400 MHz, DMSO-$d_6$) δ 12.11 (s, 1H), 8.28 (s, 1H), 7.68-7.66 (m, 1H), 7.53-7.48 (m,2H), 7.21-7.07 (m, 5H), 6.98-6.74 (m, 2H), [5.54-5.43 (m) and 5.22-5.11 (m), total 1H], [4.60-4.48(m) and 4.37-4.25 (m), total 1H],3.93-3.80 (m, 2H), 3.73-3.68 (m, 2H), 3.60-3.48 (m, 6H), 3.28 (s,3H), 3.16-3.09 (m, 1H), 2.94-2.72 (m, 2H), 2.30-2.22 (m, 7H), 2.03-1.94 (m, 1H), 1.76-1.49 (m, 6H), 1.38-1.27 (m, 5H), 1.19-1.13 (m, 7H).

MS m/z (ESI): 983.2[M+H]+.

**Example 190**

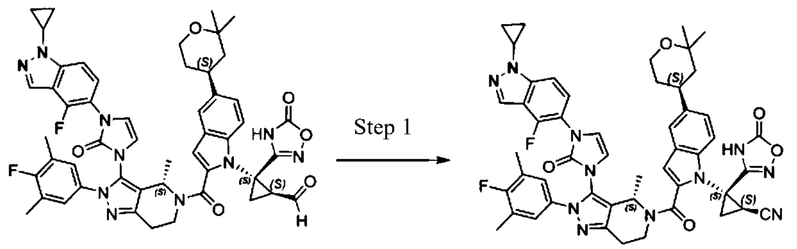


Step 1: (1S,2S)-2-(2-((S)-3-(3-(1-cyclopropyl-4-fluoro-1H-indazol-5-yl)-2-carbonyl-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indol-1-yl)-2-(5-carbonyl-4,5-dihydro-1,2,4-oxadiazol-3-yl)cyclopropane-1-carbaldehyde (0.1 g, 108.35 μmol) and hydroxylamine hydrochloride (9.79 mg, 140.85 μmol) were dissolved in acetonitrile (3 mL). Under nitrogen protection and ice-water bath cooling, iodosodium (16.24 mg, 108.35 μmol) was added. The mixture was stirred for reaction at 90°C for 1 hour. The reaction solution was evaporated to dryness and was separated by preparative HPLC to obtain a product (23 mg, 25.00 μmol, yield: 23.08%).
MS m/z (ESI): 920.4[M+H]

**Example 191**

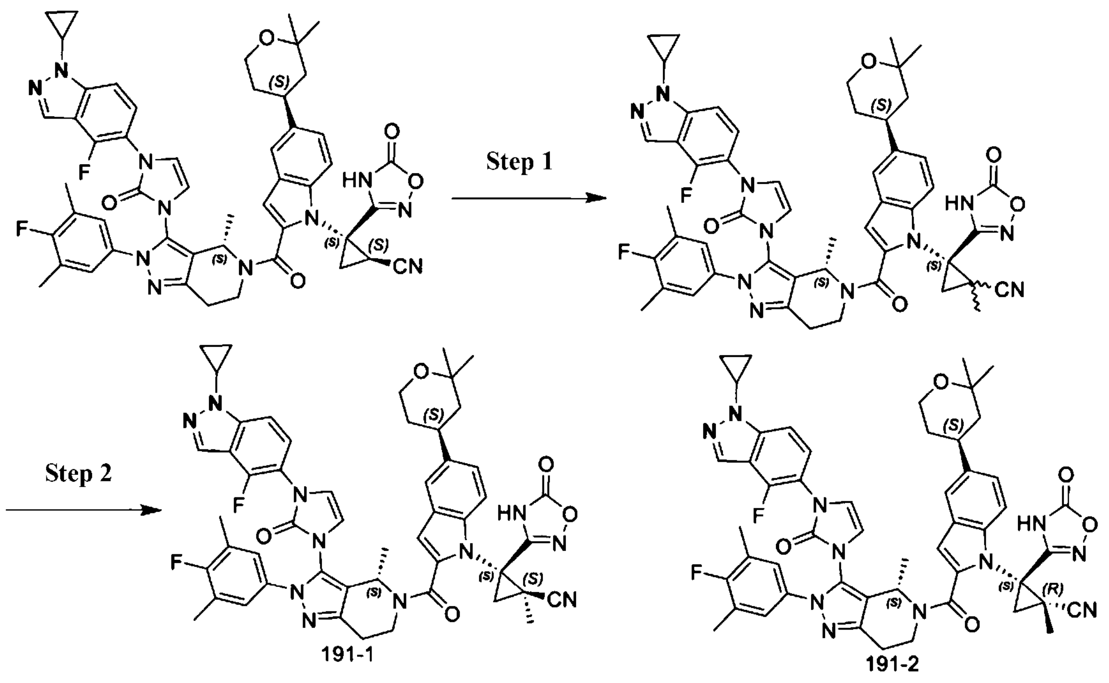


191-1 191-2

Step 1: (0.1 g, 108.70 μmol) and iodomethane (20.06 mg, 141.31 μmol, 8.80 μL) were dissolved in acetonitrile (3

mL). Potassium carbonate (45.07 mg, 326.10 μmol) was added under nitrogen protection. The mixture was stirred for reaction at 80°C for 3 hours. The reaction solution was evaporated to dryness to obtain a product (15 mg, yield: 14.77%).
MS m/z (ESI): 934.4[M+H]

Step 2: Chiral separation was performed to obtain 191-1 and 191-2.
MS m/z (ESI): 934.4[M+H]

**Example 192**

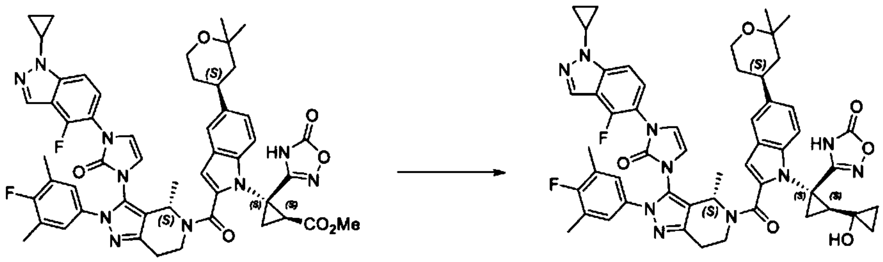

(1S,2S)-2-(2-((S)-3-(3-(1-cyclopropyl-4-fluoro-1H- indazol-5-yl)-2-oxo -2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-5-((S-2,2-dimethyltetrahydro-2H-pyran-4-yl)-1H-indol-1-yl)-2-(5-oxo-4,5-dihydro-1,2,4-oxadiazol-3-yl)cyclopropane-1-carboxylic acid methyl ester (200 mg, 0.210 mmol) and tetraisopropyl ester (179 mg, 0.630 mmol) were dissolved in THF (10 mL). At 0°C, ethylmagnesium bromide (0.53 mL, 0.525 mmol, 1 M in THF) was added to the reaction solution and stirred for reaction. After 1 hour, a saturated ammonium chloride solution was added to the reaction solution to quench, and the reaction solution was extracted with ethyl acetate twice. The organic phases were combined, dried over anhydrous sodium sulfate, and filtered. The filtrate was concentrated under reduced pressure to obtain a yellow oily crude product. The crude product was purified by silica gel column chromatography to obtain a target product (104 mg, yield: 52%).
MS m/z (ESI): 951.4[M+1]

**Example 193**

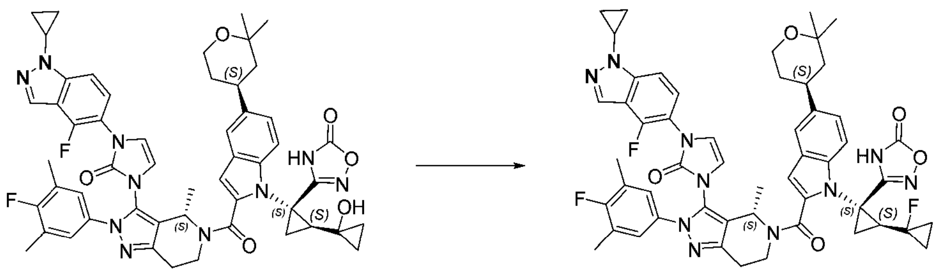

3-((1S,2S)-2-(2-((S)-3-(3-(1-cyclopropyl-4-fluoro-1H-indazol-5-yl)-2-oxo-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indol-1-yl)-1'-hydroxy-[1,1'-bis(cyclopropane)]-2-yl)-1,2,4-oxadiazol-5(4H)-one (100 mg, 0.105 mmol) and BAST (1 mL) were dissolved in dichloromethane (5 mL). The mixture was stirred for reaction at 0°C for 1 hour. A saturated sodium bicarbonate solution was added to the reaction solution to quench, and extracted twice with ethyl acetate. The organic phases were combined, dried over anhydrous sodium sulfate, and filtered. The filtrate was concentrated under reduced pressure to obtain a yellow oily crude product. The crude product was purified by silica gel column chromatography to obtain a target product (36 mg, yield: 36%).
MS m/z (ESI): 953.4[M+1]

**Example 194**

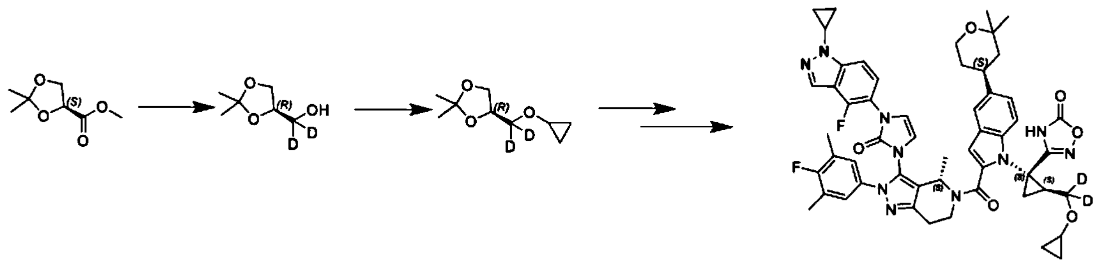

Sodium borodeuteride (3.94 g, 93.75) was added to a methanol solution (50 mL) of (S)-(-)-2,2- dimethyl-1,3-dioxolane-4-carboxylic acid methyl ester (5 g, 31.25 mmol). The mixture was reacted at room temperature for 12 hours. TLC indicated that new spots were generated. The reaction solution was quenched with saturated ammonium chloride added and extracted with ethyl acetate (50 mL×3). The organic phases were combined, dried over anhydrous sodium sulfate, filtered, and spin-dried to obtain a target product as a colorless oily substance (1.40 g, crude product).

MS m/z (ESI): 175.1 [M+1].

Step 2: A target product was obtained with reference to step 1 of Example 189.

MS m/z (ESI): 175.1 [M+1].

Step 3: A target product was obtained with reference to Example 189.

$^1$H NMR (400 MHz, DMSO) δ[12.25 (s) and 11.82 (s), total 1 H], 8.26-8.16 (m, 1H), 7.81 - 7.60 (m, 1H), 7.60-7.33 (m, 3H), 7.28-7.09 (m, 4H), 6.95-6.92 (m, 1H), 6.75 (s, 1H), 5.75-5.58(m, 1H), 5.2-4.65(m, 1H), 4.42-4.39 (m, 1H), 3.88-3.83 (m, 1H), 3.72-3.48 (m, 3H), 3.17-2.65 (m, 4H), 2.40-2.26 (m, 6H), 2.07-1.77 (m, 2H), 1.76-1.42 (m, 6H), 1.35-1.10 (m, 11H), 0.49-0.31(m, 4H).

MS m/z (ESI): 967.4[M+1].

**Example 195**

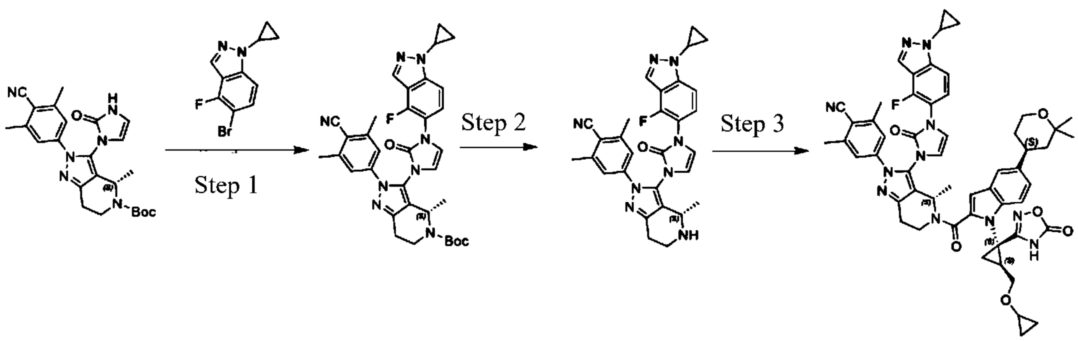


Step 1: A mixture of intermediate G (1.5 g, 3.34 mmol), 5-bromo-1-cyclopropyl-4-fluoro-1H-indazole (1.28 g, 5.02 mmol), CuI (509.55 mg, 2.68 mmol), (1S,2S)-N1,N1-dimethylcyclohexane-1,2-diamine (475.70 mg, 3.34 mmol), potassium carbonate (1.38 g, 10.03 mmol) and NMP (20 mL) was stirred at 130°C for 4 hours under nitrogen protection. LCMS showed that the reaction was complete and there was MS of a target product. Water and EtOAc were added for extraction. The organic phase was washed with saturated brine, dried over anhydrous sodium sulfate, and separated by column chromatography (DCM/MeOH=10:1) to obtain the target product (2 g, yield of 96%).

MS m/z (ESI): 623.3 [M+1].

Step 2: HCl (4 M, 15 mL) was added dropwise to a DCM solution (30 mL) of (S)-4-(3-(3-(1-cyclopropyl-4-fluoro-1H-indazol-5-yl)-2-carbonyl-2,3-dihydro-1H-imidazol-1-yl)-4-methyl-4,5,6,7-tetrahydro-2H-pyrazolo[4,3-c]pyridin-2-yl)-2,6-dimethylbenzonitrile (2 g, 3.21 mmol). The mixture was stirred at room temperature for 0.5 hours. LCMS showed that the reaction was complete and there was MS of a target product. The mixture was spin-dried to obtain a light yellow solid (1.6 g, crude product).

MS m/z (ESI): 523.2[M+1].

HATU (242.09 mg, 641.69 μmol) and DIEA (165.86 mg, 1.28 mmol) were added to a DMF solution (5 mL) of (S)-4-(3-(3-(1-cyclopropyl-4-fluoro-1H-indazol-5-yl)-2-carbonyl-2,3-dihydro-1H-imidazol-1-yl)-4-methyl-4,5,6,7-tetrahydro-2H-pyrazolo[4,3-c]pyridin-2-yl)-2,6-dimethylbenzonitrile (215.24 mg, 385.02 μmol) and 1-(2-(cyclopropyloxymethyl)-1-(5-carbonyl-4,5-dihydro-1,2,4-oxadiazol-3-yl)cyclopropyl)-5-((S)-2,2-dimethyltetrahydro-2H-pyran-4-yl)-1H-indole-2-carboxylic acid (150.00 mg, 320.85 μmol). The mixture was stirred at room temperature for 12 hours. Water and

DCM were added to perform extraction three times. The organic phase was washed with saturated brine, dried over anhydrous sodium sulfate, and filtered. The filtrate was concentrated under reduced pressure, and separated by preparative chromatography (acidic) to obtain a target product (188 mg, yield: 60.2%).

MS m/z (ESI): 972.4[M+1].

$^1$H NMR (400 MHz, DMSO) δ [12.20 (s) and 11.75 (s), total 1 H],8.22-8.15 (m, 1H), 7.70-7.60(m, 1H), 7.54-7.40(m, 1H), 7.31-7.24 (m, 3H), 7.20-6.84 (m, 3H), 6.83-6.34 (m, 2H), 5.60-5.22 (m, 1H), 4.72 (d, *J* =13.1 Hz, 1H) , 3.83-3.56 (m, 4H), 2.98-2.92 (m, 1H), 2.67-2.60 (m, 2H), 2.39 (s, 3H), 2.03-1.81 (m, 3H), 1.69-1.29 (m, 6H), 1.26-1.02 (m, 16H), 0.84-0.74 (m, 1H), 0.33-0.25 (m, 4H).

**Example 196**

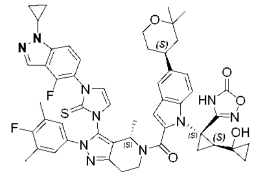

A target product was obtained with reference to the synthesis method of Example 192.
MS m/z (ESI): 967.4[M+1]

**Example 197**

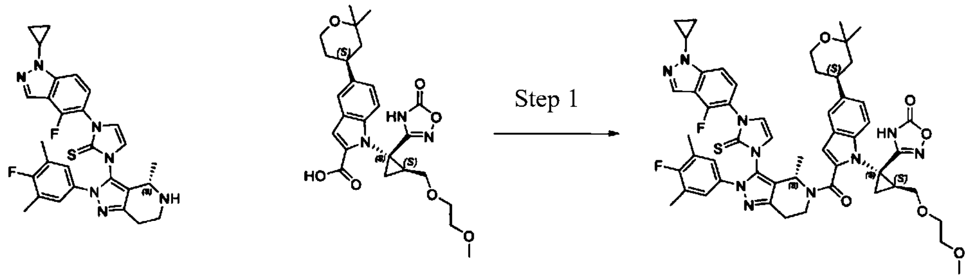


Step 1: A target compound was obtained with reference to step 4 of Example 25.

MS m/z (ESI): 999.4 [M+H]+.

**Example 198**

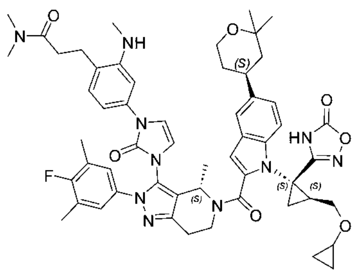

A titled product was obtained with reference to Examples 25, 63 and 135.

MS m/z (ESI): 995.5[M+H]+.

**Example 199**

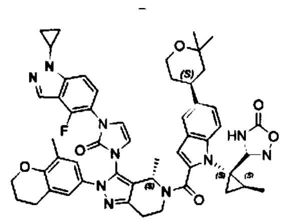

A target product was obtained by using (8-methylchroman-6-yl)hydrazine as an initial raw material, with reference to intermediate G and Example 136.

MS m/z (ESI): 933.4[M+H]+.

**Example 200**

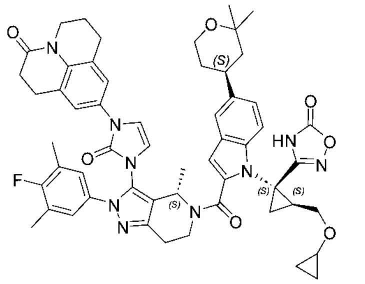

A titled product was obtained with reference to Examples 25, 63 and 135.

$^1$H NMR (400 MHz, DMSO-$d_6$) $\delta$ [12.27 (s) and 11.83 (s), total 1H], 7.77-7.32 (m, 3H), 7.28-7.20 (m, 2H), 7.17-7.05 (m , 3H),6.96-6.89 (m, 1H), 6.76-6.70 (m, 1H), [5.72-5.57 (m) and 5.24-5.14(m), total 1H], [4.79-4.63 (m) and 4.46 -4.38 (m), total 1H], 3.97-3.59 (m, 5H), 3.47-3.37 (m, 1H), 3.26-3.00 (m, 3H), 2.90-2.65 (m, 5H), 2.62-2.53 (m, 2H), 2.43-2.29 (m, 1H), 2.22(s, 6H),2.08-1.93(m, 1H), 1.90-1.75 (m, 3H), 1.73-1.50 (m, 5H), 1.42-1.38 (m, 1H), 1.27-1.18 (m, 8H), 0.48-0.21 (m, 4H).

MS m/z (ESI): 976.4[M+H]+.

**Example 201**

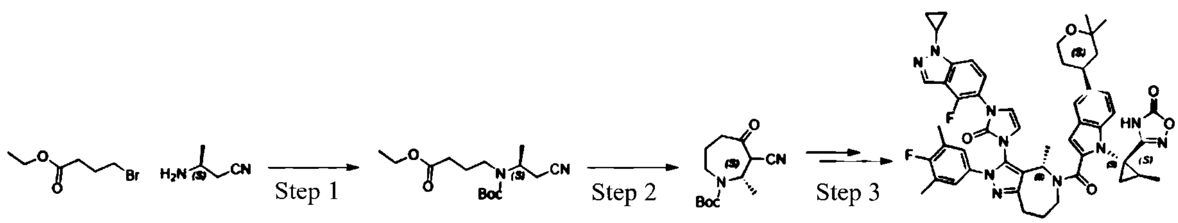


Step 1: In a 50 mL reaction bottle, 4-bromobutyric acid ethyl ester (1 g, 5.13 mmol) and (S)-3-aminobutyronitrile (431.27 mg, 5.13 mmol) were dissolved in THF (10 mL), triethylamine (5.19 g, 51.27 mmol) was added at 25°C, and then the reaction liquid was stirred at 60°C for 10 hours under nitrogen protection. The reaction liquid was cooled to room temperature, Di-tert-butyl dicarbonate (1 g, 5.13 mmol) was added, and stirring was continued for 2 hours. The reaction was stopped. An aqueous solution (10 mL) was added to quench the reaction. The mixture was extract with dichloromethane (10 mL×2). The organic phases were combined, washed with saturated sodium chloride (10 mL), dried over anhydrous sodium sulfate, and filtered. The residue was purified by silica gel column chromatography with an eluent system of petroleum ether and ethyl acetate to obtain a target product (500 mg, yellow liquid), with a yield of 32.6%.

MS m/z (ESI): 299.2 [M+1].

Step 2: In a 50 mL reaction bottle, (S)-4-((1-cyanopropane-2-yl)amino)butyric acid ethyl ester (500 mg, 1.68 mmol) and potassium tert-butoxide (188.04 mg, 1.68 mmol) were dissolved in tetrahydrofuran (5 mL), and then the reaction solution was stirred at 25°C for 2 hours under nitrogen protection. Then, 2N hydrochloric acid (1 mL, 2.0 mmol) was added to the reaction solution. The reaction was stopped. An aqueous solution (10 mL) was added to quench the reaction. The mixture was extract with ethyl acetate (10 mL×2). The organic phases were combined, washed with saturated sodium chloride (10 mL), dried over anhydrous sodium sulfate, and filtered. The residue was purified by silica gel column chromatography with an eluent system of petroleum ether and ethyl acetate to obtain a target product (300 mg, yellow solid), with a yield of 70.9%.

MS m/z (ESI): 253.2 [M+1].

Step 3: A target product was obtained by using (2S)-3-cyano-2-methyl-4-carbonylazepane-1-carboxylic acid tert-butyl ester as a raw material, with reference to the synthesis method of intermediate G and the synthesis method of Example 25.

MS m/z (ESI): 923.4[M+1].

**Example 202**

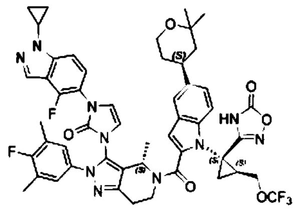

A target product was obtained by using 3-((1S,2S)-1-(2-((S)-3-(3-(1-cyclopropyl-4-fluoro-1H-indazol-5-yl)-2-carbonyl-2,3-dihydro-1H-imidazol-1-yl)-2-(4-fluoro-3,5-dimethylphenyl)-4-methyl-4,5,6,7-tetrahydro -2H-pyrazolo[4,3-c]pyridine-5-carbonyl)-5-((S)-2,2-dimethyltetrahydro-2H-pyran-4-yl)-1H-indol-1-yl)-2-(hydroxymethyl)cyclopropyl)-1,2,4-oxadiazol-5(4H)-one as a raw material, with reference to the literature [Journal of Organic Chemistry, 1964, 29, 11-15].

MS m/z (ESI): 993.4 [M+1].

**Example 203**

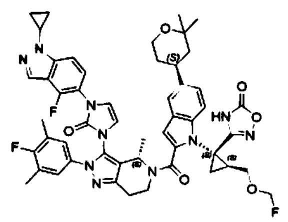

A target product was obtained by using 3-((1S,2S)-1-(2-((S)-3-(3-(1-cyclopropyl-4-fluoro-1H-indazol-5-yl)-2-carbonyl-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indol-1-yl)-2-(hydroxymethyl)cyclopropyl)-1,2,4-oxadiazol-5(4H)-one as a raw material, with reference to the literature [Journal of Organic Chemistry, 2017, 82, 16, 8604 - 8610].

MS m/z (ESI): 957.4 [M+1].

**Example 204**

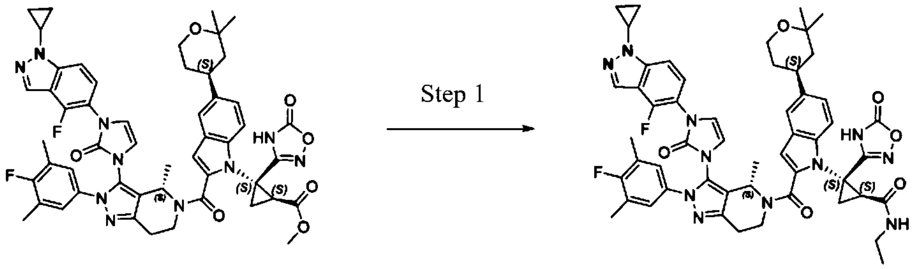


Methyl(1S,2S)-2-(2-((S)-3-(3-(1-cyclopropyl-4-fluoro-1H-indazol-5-yl)-2-carbonyl-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indol-1-yl)-2-(5-carbonyl-4,5-dihydro-1,2,4-oxadiazol-3-yl)cyclopropane-1-carboxylate (0.095 g, 0.1 mmol) was dissolved in tetrahydrofuran (10 mL) at room temperature. Then, ethylamine hydrochloride (0.016 g, 0.2 mmol) and triethylamine (0.03 g, 0.3 mmol) were added. The mixture was stirred at room temperature overnight. LCMS indicated the reaction was complete. The reaction solution was diluted with ethyl acetate (30 mL), then washed with a saturated sodium bicarbonate solution (20 mL×2) and saturated brine (20 mL×2) in sequence. The organic phase was dried over anhydrous sodium sulfate, filtered, and spin-dried. The residue was purified by reverse phase preparative chromatography to obtain a target compound (0.029 g, yield of 30%).

MS m/z (ESI): 966.4[M+H]+.

**Example 205**

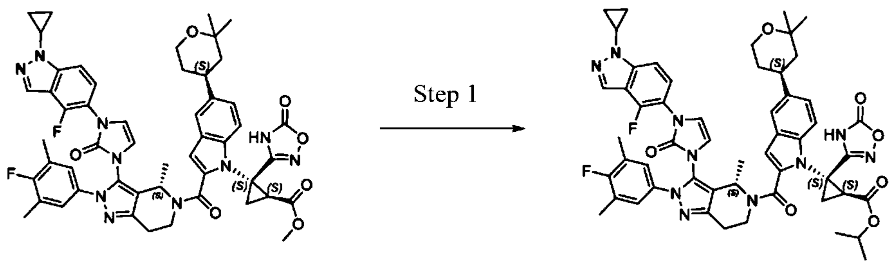


Methyl(1S,2S)-2-(2-((S)-3-(3-(1-cyclopropyl-4-fluoro-1H-indazol-5-yl)-2-carbonyl-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indol-1-yl)-2-(5-carbonyl-4,5-dihydro-1,2,4-oxadiazol-3-yl)cyclopropane-1-carboxylate (0.095 g, 0.1 mmol) was dissolved in isopropanol (10 mL) at room temperature. Then, concentrated sulfuric acid (0.015 g, 0.15 mmol) was added dropwise. Nitrogen was replaced. The mixture was heated to reflux for 14 hours, LCMS indicated that the reaction was complete. The mixture was cooled to room temperature, and spin-dried to remove isopropanol. The residue was dissolved in ethyl acetate (10 mL), and then washed with a saturated sodium bicarbonate solution (20 mL×2) and saturated brine (20 mL×2) in sequence. The organic phase was dried over anhydrous sodium sulfate, filtered, and spin-dried. The residue was purified by reverse phase preparative chromatography to obtain a target compound (0.02 g, yield of 20%).

MS m/z (ESI): 981.4[M+H]+.

**Example 206**

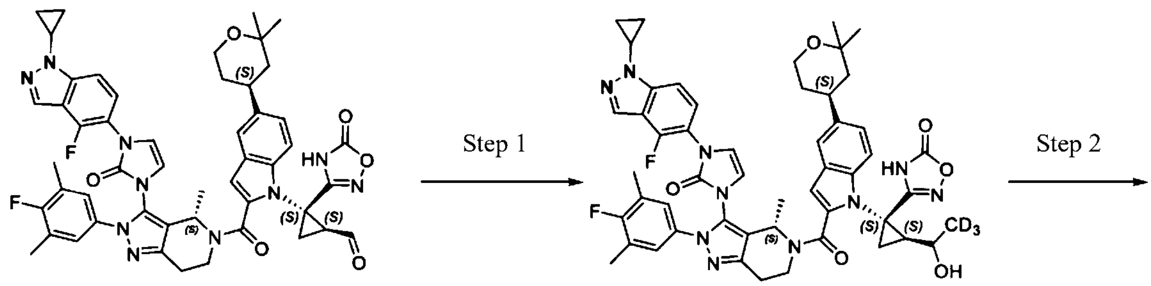


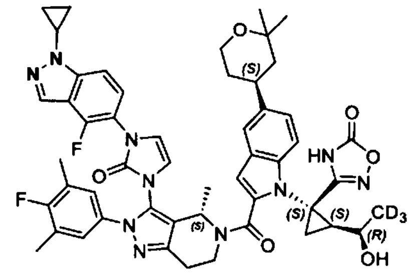
206a

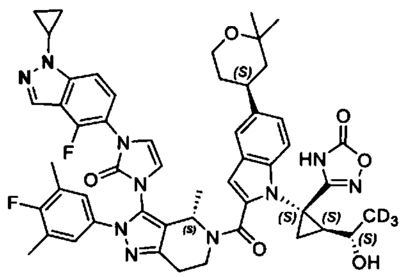
206 b

Step 1: (1S,2S)-2-(2-((S)-3-(3-(1-cyclopropyl-4-fluoro-1H-indazol-5-yl)-2-carbonyl-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indol-1-yl)-2-(5-carbonyl-4,5-dihydro-1,2,4-oxadiazol-3-yl)cyclopropane-1-carbaldehyde (0.092 g, 0.1 mmol) was dissolved in tetrahydrofuran (10 mL) at -78°C, nitrogen was replaced, and then (methyl-d3) magnesium bromide (1.0 M, 0.3 mmol, 0.3 mL) was added dropwise. The mixture was stirred at -78°C for 2 hours. LCMS indicated that the reaction was complete. The reaction solution was quenched with a saturated ammonium chloride solution (50 mL), and then extracted with ethyl acetate (20 mL×3). The organic phases are combined, dried over anhydrous sodium sulfate, filtered, and spin-dried. The residue was purified by reverse phase preparative chromatography to obtain a target compound (0.047 g, yield of 50%).

MS m/z (ESI): 942.4[M+H]+.

Step 2:

Compound 206 was split to obtain compound 206a and compound 206b.

MS m/z (ESI): 942.4[M+H]+.

**Example 207**

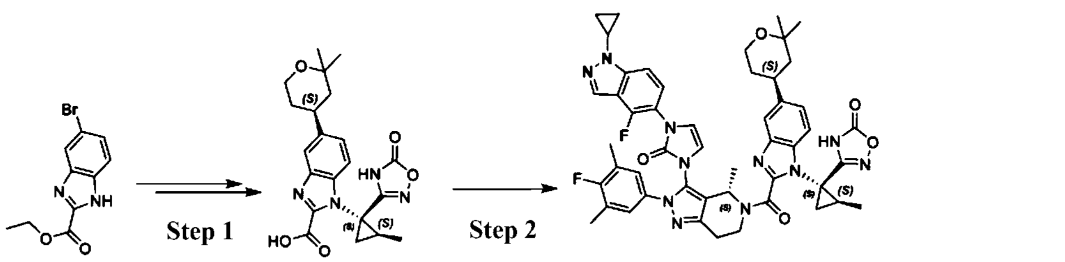


Step 1: A target product was obtained by using 5-bromo-1H-benzo[d]imidazole-2-carboxylic acid ethyl ester as a raw material, with reference to the synthesis method of intermediate F.

MS m/z (ESI): 413.2[M+1].

Step 2: A target product was obtained by using (S)-1-(1-cyclopropyl-4-fluoro-1H-indazol-5-yl)-3-(2-(4-fluoro-3,5-dimethylphenyl)-4-methyl-4,5,6,7-tetrahydro-2H-pyrazolo[4,3-c]pyridin-3-yl)-1,3-dihydro-2H-imidazole-2-one and 5-((S)-2,2-dimethyltetrahydro-2H-pyran-4-yl)-1-((1S,2S)-2-methyl-1-(5-carbonyl-4,5-dihydro-1,2,4-oxadiazol-3-yl)cyclopropyl)-1H-benzo[d]imidazole-2-carboxylic acid as raw materials, with reference to the synthesis method of step 4 of

Example 25.

MS m/z (ESI): 910.4[M+1].

**Example 208**

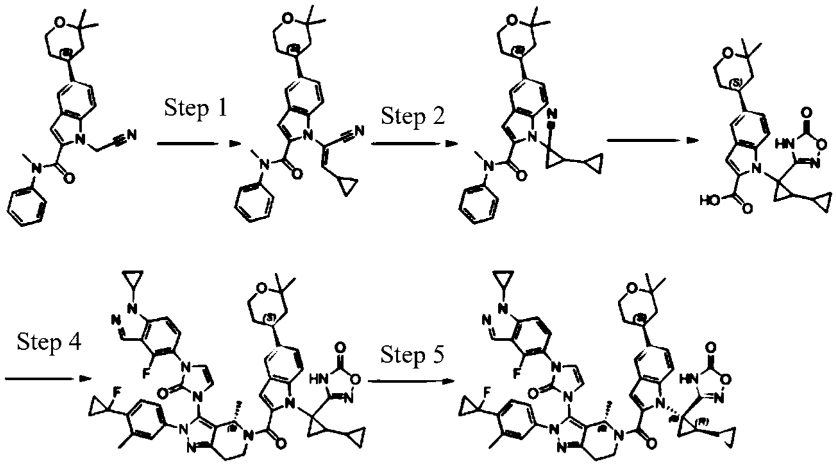


Step 1: (S)-1-(cyanomethyl)-5-(2,2-dimethyltetrahydro-2H-pyran-4-yl)-N-methyl-N-phenyl-1H-indole-2-carboxamide (300 mg, 0.75), cyclopropanecarboxaldehyde (262 mg, 3.74 mmol) and potassium hydroxide (3.0 eq., 126 mg, 2.24 mmol) were added to a reaction bottle, and tetrahydrofuran (6 mL) was added. The mixture was stirred for reaction at room temperature for 2 h. An appropriate amount of ammonium chloride (10 mL) was added to the system for quenching, and ethyl acetate (15 mL) was added for extraction. The organic phases were combined, dried and concentrated to obtain a light yellow solid crude product (320 mg), which was directly used for a next reaction.
MS m/z (ESI): 454.2[M+H]

Step 2: Potassium tert-butoxide (1.1 eq., 6.2 mg) and trimethyl sulfoxide iodide (1.1 eq., 12.1 mg) were added to the reaction bottle, and dimethyl sulfoxide (0.2 mL) was added. After the mixture was heated to 50°C and reacted for 1 h, a dimethyl sulfoxide solution (0.2 mL) of (S,E)-1-(1-cyano-2-cyclopropylethylene)-5-(2,2-dimethyltetrahydro-2H-pyran-4-yl)-N-methyl-N-phenyl-1H-indole-2-carboxamide (0.05 mmol, 1.0 eq. 23 mg) was added. The system was kept at 50°C and reacted for 3 h. The reaction solution was cooled. Saturated brine (15 mL) was added, and then the reaction solution was extracted with acetate (10 mL). Saturated brine (15 mL×3) was separated by preparative TLC to obtain a target product (7 mg, yield: 29.5%).
MS m/z (ESI): 468.3[M+H]

Step 3: A target product was synthesized with reference to steps 7 and 8 of intermediate F.
MS m/z (ESI): 438.2[M+H]

Step 4: A target product was synthesized with reference to Example 182.
MS m/z (ESI): 961.4[M+H]

Step 5: Chiral separation is performed to obtain a target product.
MS m/z (ESI): 961.4[M+H]

**Example 209**

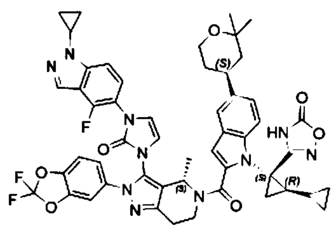

A target product was synthesized by using 5-bromo-2,2 -difluorobenzo[d][1,3]dioxazole as a raw material, with

reference to Example 208.
MS m/z (ESI): 969.4[M+H]

**Example 210**

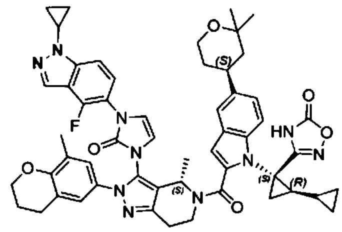

A target product was synthesized by using 6-bromo-8-methylchromane as a raw material, with reference to Example 208.
MS m/z (ESI): 959.4[M+H]

**Example 211**

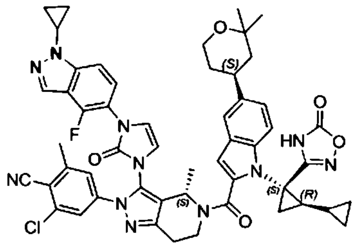

A target product was synthesized by using 4-bromo-2-chloro-6-methylbenzonitrile as a raw material, with reference to Example 208.
MS m/z (ESI): 962.4[M+H]

**Example 212**

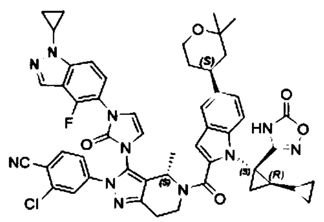

A target product was synthesized by using 4-bromo-2-chlorobenzonitrile as a raw material, with reference to Example 208.
MS m/z (ESI): 948.3[M+H]

**Example 213**

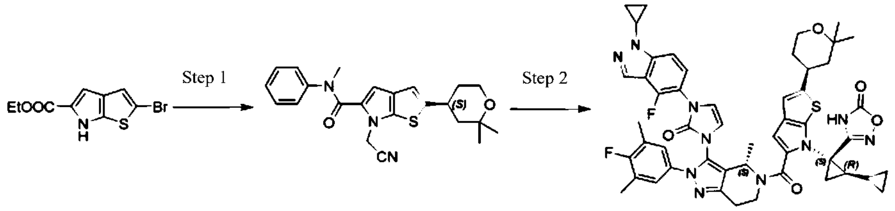


Step 1: A target product was obtained by using ethyl 2-bromo-6H-thieno[2,3-b]pyrrole-5-carboxylate as a raw material, with reference to intermediate F.

MS m/z (ESI): 408.2[M+1].

Step 2: A target product was synthesized with reference to Example 208.
MS m/z (ESI): 941.4[M+H]

**Example 214**

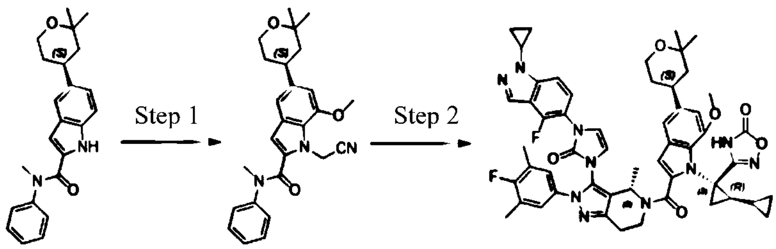


Step 1: A target product was synthesized by using (S)-5-(2,2-dimethyltetrahydro-2H- pyran-4-yl)-N-methyl-N-phenyl-1H-indole-2-carboxamide as a raw material, with reference to Example 186.

MS m/z (ESI): 432.2[M+1].

Step 2: A target product was synthesized with reference to Example 208.
MS m/z (ESI): 965.4[M+H]

**Example 215**

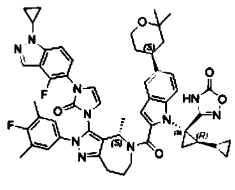

A target product was synthesized by using a raw material with reference to Examples 201 and 208.
MS m/z (ESI): 949.4[M+H]

**Example 216**

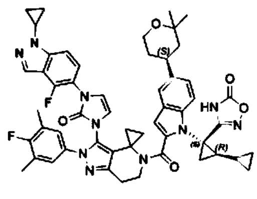

A target product was synthesized by using a raw material with reference to Examples 51 and 208. MS m/z (ESI): 947.4[M+H]

**Example 217**

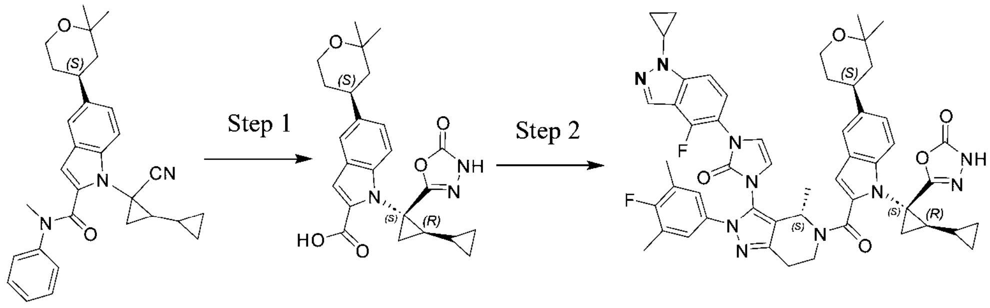


Step 1: A target product was synthesized by using 1-(2-cyano-[1,1'-bi(cyclopropane)]-2-yl)-5-((S)-2,2-dimethyltetrahydro-2H-pyran-4-yl)-N-methyl-N-phenyl-1H-indole-2-carboxamide as a raw material, with reference to steps 1 to 4 of Example 237.

MS m/z (ESI): 438.2[M+1].

Step 2: A target product was synthesized by using (4-fluoro-3,5-dimethylphenyl)hydrazine hydrochloride as a raw material, with reference to intermediate G and Example 208.

MS m/z (ESI): 935.4[M+H]

**Example 218**

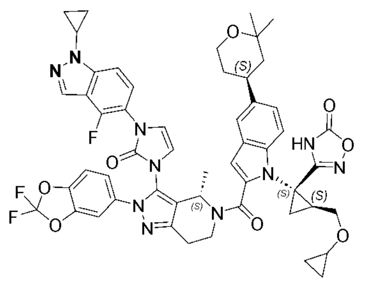

A titled product was obtained with reference to Example 184.

MS m/z (ESI): 999.4[M+1].

**Example 219**

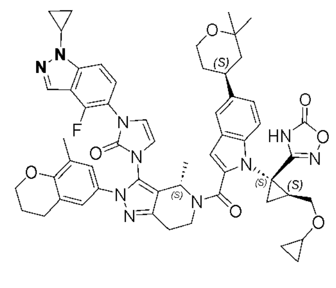

A titled product was obtained with reference to Example 199.

MS m/z (ESI): 989.4[M+1].

**Example 220**

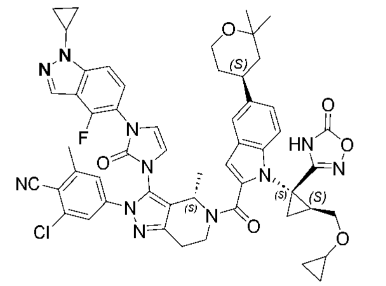

A titled product was obtained with reference to Example 484.
MS m/z (ESI): 992.4[M+1].

**Example 221**

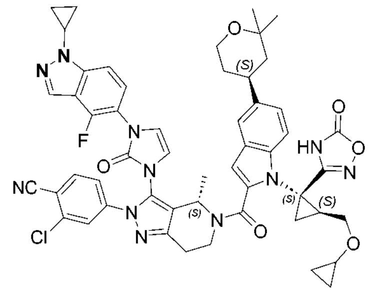

A titled product was obtained with reference to Example 485.
MS m/z (ESI): 978.4[M+1].

**Example 222**

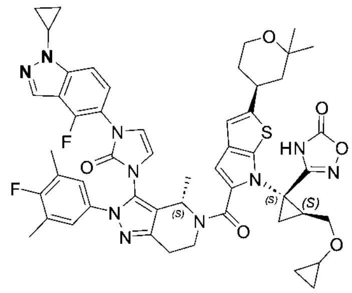

A titled product was obtained with reference to Example 213.
MS m/z (ESI): 971.4[M+1].

**Example 223**

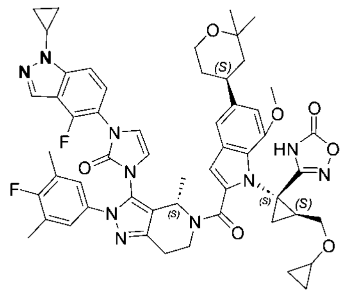

A titled product was obtained with reference to Example 186.

MS m/z (ESI): 995.4[M+1].

**Example 224**

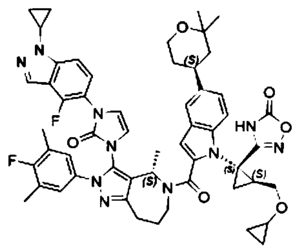

A titled product was obtained with reference to Example 215.

MS m/z (ESI): 979.4[M+1].

**Example 225**

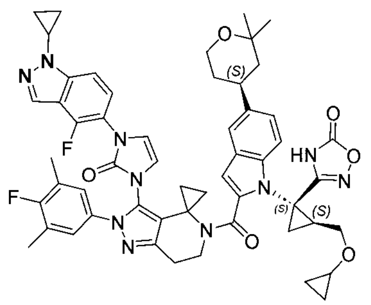

A target compounds was obtained with reference to the synthetic manner of Example 51.

MS m/z (ESI): 977.4[M+1].

**Example 226**

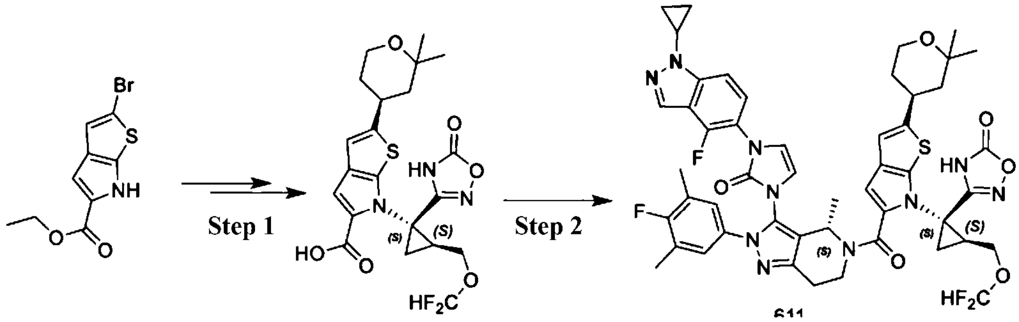


611

Step 1: A target product was obtained by using 2-bromo-6H-thieno[2,3-b]pyrrole-5-carboxylic acid ethyl ester as a raw material, with reference to the synthesis method of intermediate F, and replacing (R)-4-methyl-1,3,2-dioxathiolane 2,2-dioxide with (R)-4-((difluoromethoxy)methyl)-1,3,2-dioxathiolane 2,2-dioxide.

MS m/z (ESI): 484.1[M+1].

Step 2: A target product was obtained by using (S)-1-(1-cyclopropyl-4-fluoro-1H-indazol-5-yl)-3-(2-(4-fluoro-3,5-dimethylphenyl)-4-methyl-4,5,6,7-tetrahydro-2H-pyrazolo[4,3-c]pyridin-3-yl)-1,3-dihydro-2H-imidazole-2-one and 6-((1S,2S)-2-((difluoromethoxy)methyl)-1-(5-carbonyl-4,5-dihydro-1,2,4-oxadiazol-3-yl)cyclopropyl)-2-((S)-2,2-dimethyltetrahydro-2H-pyran-4-yl)-6H-thieno[2,3-b]pyrrole-5-carboxylic acid as raw materials, with reference to the synthesis method of step 4 of Example 25.

MS m/z (ESI): 981.3[M+1].

**Example 227**

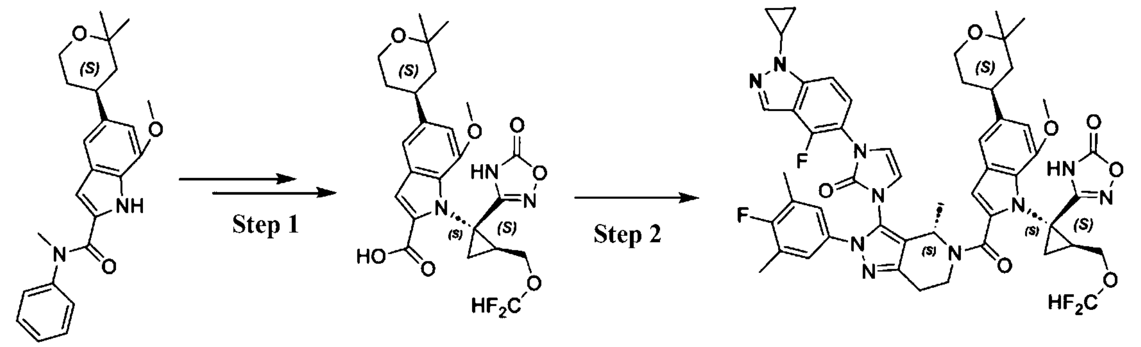


Step 1: A target product was obtained by using (S)-5-(2,2-dimethyltetrahydro-2H-pyran-4-yl)-7-methoxy-N-methyl-N-phenyl-1H-indole-2-carboxamide as a raw material, with reference to the synthesis method of intermediate F, and replacing (R)-4-methyl-1,3,2-dioxathiolane 2,2-dioxide with (R)-4-((difluoromethoxy)methyl)-1,3,2-dioxathiolane 2,2-dioxide.

MS m/z (ESI): 508.1[M+1].

Step 2: A target product was obtained by using (S)-1-(1-cyclopropyl-4-fluoro-1H-indazol-5-yl)-3-(2-(4-fluoro-3,5-dimethylphenyl)-4-methyl-4,5,6,7-tetrahydro-2H-pyrazolo[4,3-c]pyridin-3-yl)-1,3-dihydro-2H-imidazole-2-one and 1-((1S,2S)-2-((difluoromethoxy)methyl)-1-(5-carbonyl-4,5-dihydro-1,2,4- oxadiazol-3-yl)cyclopropyl)-5-((S)-2,2-dimethyltetrahydro-2H-pyran-4-yl)-7-methoxy-1H-indole-2-carboxylic acid as raw materials, with reference to the synthesis method of step 4 of Example 25.

MS m/z (ESI): 1005.4[M+1].

**Example 228**

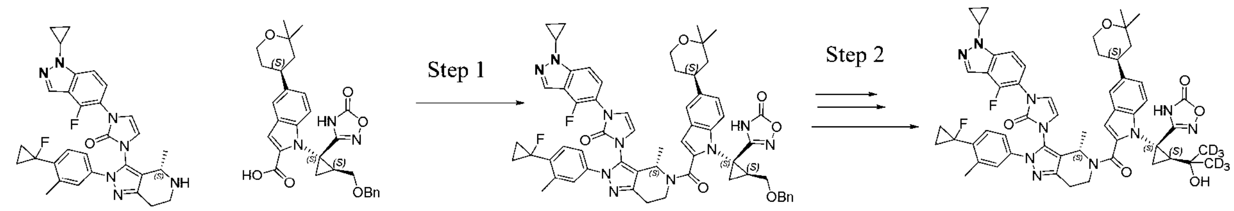


Step 1: A target compound was obtained by using (S)-1-(1-cyclopropyl-4-fluoro-1H-indazol-5-yl)-3-(2-(4-(1-fluorocyclopropyl)-3-methylphenyl)-4-methyl-4,5,6,7-tetrahydro-2H-pyrazolo[4,3-c]pyridin-3-yl)-1,3-dihydro-2H-imidazol-2-one and 1-((1S,2S)-2-((benzyloxy)methyl)-1-(5-carbonyl-4,5-dihydro-1,2,4-oxadiazol-3-yl)cyclopropyl)-5-((S)-2,2-dimethyltetrahydro-2H-pyran-4-yl)-1H-indole-2-carboxylic acid as raw materials, with reference to step 4 of Example 25.

MS m/z (ESI): 1041.2[M+H]+.

Step 2: A target compound was obtained by using 3-((1S,2S)-2-((benzyloxy)methyl)-1-(2-((S)-3-(3-(1-cyclopropyl-4-fluoro-1H-indazol-5-yl)-2-carbonyl-2,3-dihydro- 1H-imidazol-1-yl)-2-(4-(1-fluorocyclopropyl)-3-methylphenyl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indol-1-yl)cyclopropyl)-1,2,4-oxadiazol-5(4H)-one as a raw material, with reference to steps 1, 2, 3 and 4 of Example 188.

MS m/z (ESI): 985.5[M+H]+.

## Example 229

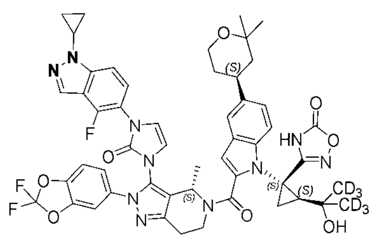

A target compound was obtained by using (2,2-difluorobenzo[d][1,3]dioxazol-5-yl)hydrazine as a raw material, with reference to intermediate G and Example 228.

MS m/z (ESI): 993.4[M+H]+.

## Example 230

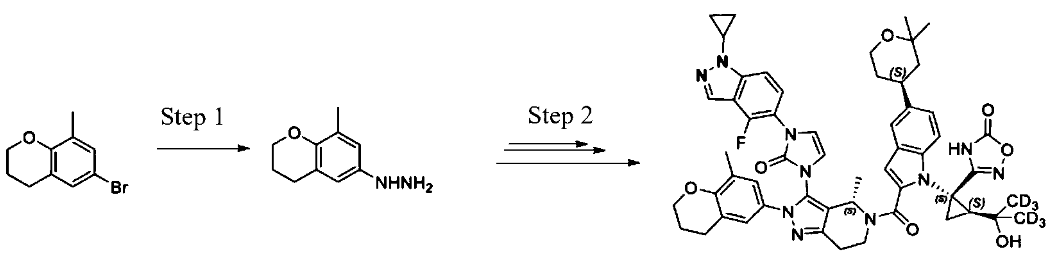


Step 1: A target compound was obtained by using 6-bromo-8-methylchromane as a raw material, with reference to step 3 of Example 208.

MS m/z (ESI): 179.1[M+H]+.

Step 2: A target compound was obtained by using (8-methylchroman-6-yl)hydrazine as a raw material, with reference to intermediate G and Example 228.

MS m/z (ESI): 983.5[M+H]+.

**Example 231**

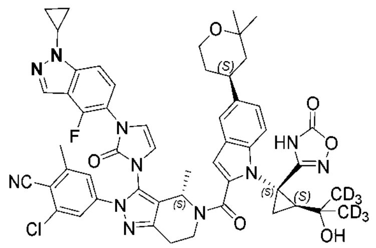

A target compound was obtained by using 4-bromo-2-chloro-6-methylbenzonitrile as a raw material, with reference to steps 3 to 5 of Example 182, and Example 228.

MS m/z (ESI): 986.4[M+H]+.

**Example 232**

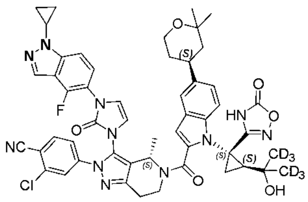

A target compound was obtained by using 4-bromo-2-chlorobenzonitrile as a raw material, with reference to steps 3 to 5 of Example 182, and Example 228.

MS m/z (ESI): 972.4[M+H]+.

**Example 233**

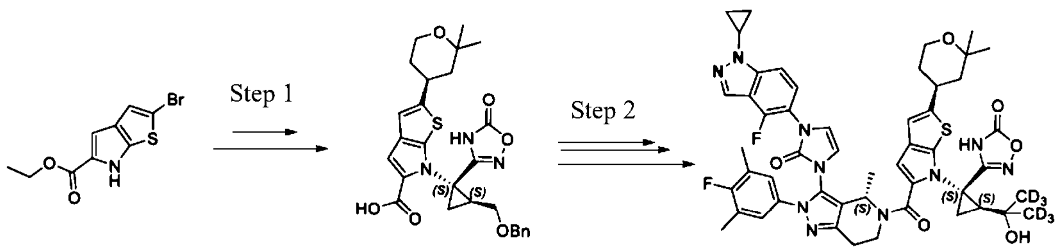


Step 1: A target compound was obtained by using ethyl 2-bromo-6H-thieno[2,3-b]pyrrole-5-carboxylate as a raw material, with reference to steps 1 and 2 of Example 135, and the synthesis step of intermediate F.

MS m/z (ESI): 524.2[M+H]+.

A target compound was obtained by using 6-((1S,2S)-2-((benzyloxy)methyl)-1-(5-carbonyl-4,5-dihydro-1,2,4-oxadiazol-3-yl)cyclopropyl)-2-((S)-2,2-dimethyltetrahydro-2H-pyran-4-yl)-6H-thieno[2,3-b]pyrrole-5-carboxylic acid as a raw material, with reference to Example 228.

MS m/z (ESI): 965.2[M+H]+.

**Example 234**

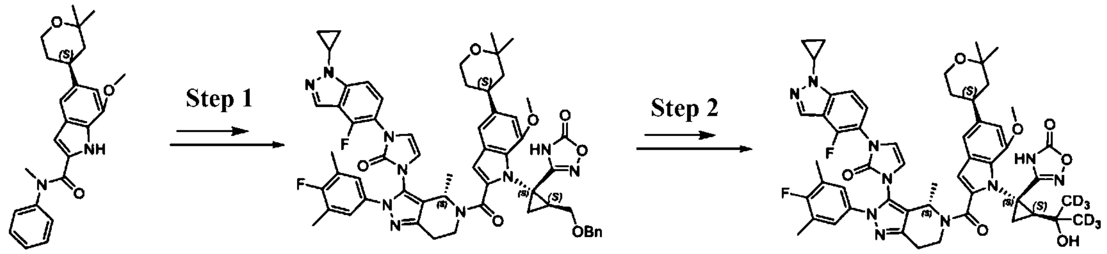


Step 1: A target compound was obtained by using (S)-5-(2,2-dimethyltetrahydro-2H-pyran-4-yl)-7-methoxy-N-methyl-N-phenyl-1H-indole-2-carboxamide as a raw material, with reference to steps 1 and 2 of Example 135, and the synthesis step of intermediate F.

MS m/z (ESI): 1045.2[M+H]+.

Step 2: A target compound was obtained by using 3-((1S,2S)-2-((benzyloxy)methyl)-1-(2-((S)-3-(3-(1-cyclopropyl-4-fluoro-1H-indazol-5-yl)-2-carbonyl-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-5-((S)-2,2-dimethyltetrahydro-2H-pyran-4-yl)-7-methoxy-1H-indol-1-yl)cyclopropyl)-1,2,4-oxadiazol-5(4H)-one as a raw material, with reference to Example 228.

MS m/z (ESI): 989.4[M+H]+.

## Example 235

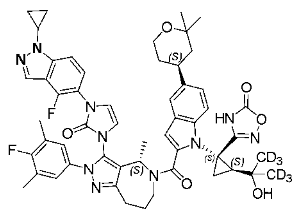

A target product was obtained with reference to Examples 201 and 228.

MS m/z (ESI): 973.4[M+H]+.

## Example 236

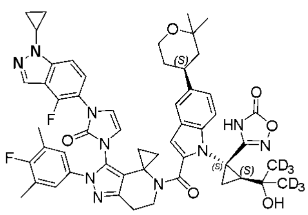

A target product was obtained with reference to Examples 51 and 228.

MS m/z (ESI): 971.4[M+H]+.

## Example 237

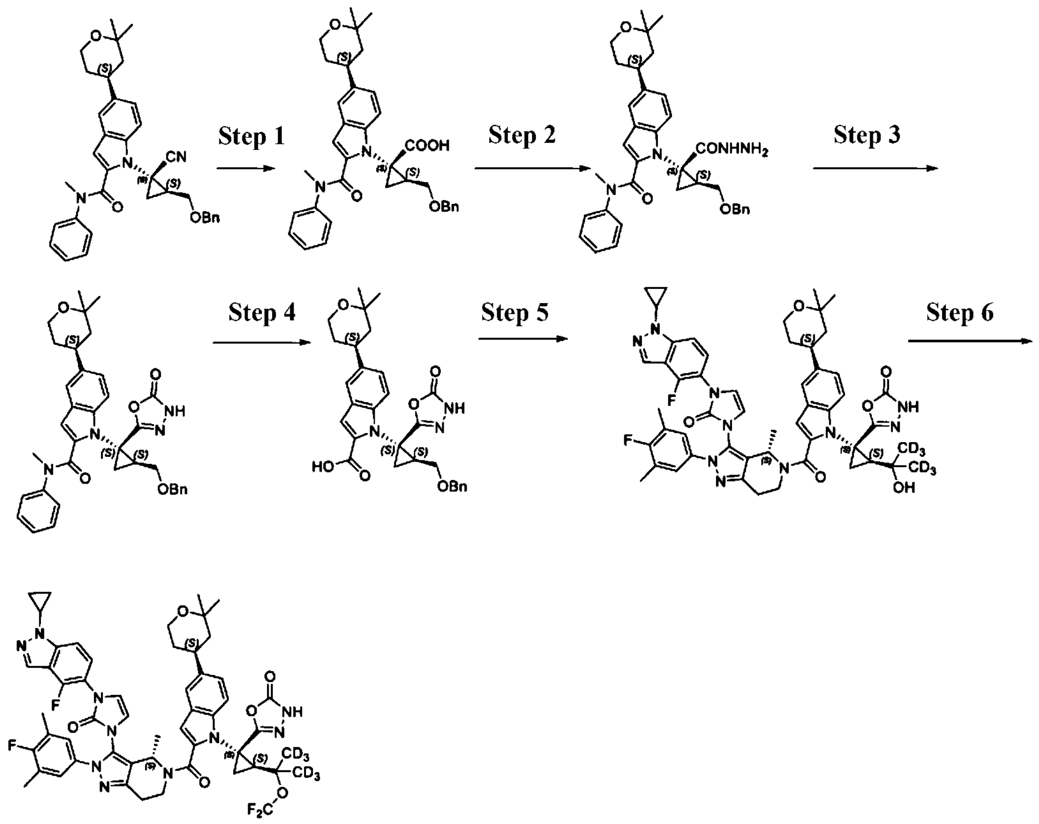


Step 1 : 1-((1S,2S)-2-((benzyloxy)methyl)-1-cyanocyclopropyl)-5-((S)-2,2-dimethyltetrahydro-2H-pyran-4-yl)-N-methyl-N-phenyl-1H-indole-2-carboxamide (0.30 g, 0.55 mmol) was dissolved in a mixed solvent of concentrated sulfuric acid (2 mL) and HCl (2 M in EtOH, 6 mL). The reaction solution was heated to 80°C, and stirred for reaction. After 18 hours, the heating was stopped, and the reaction solution was cooled to room temperature. LCMS indicated that the reaction was complete. The reaction solution was slowly added dropwise to ice water (20 mL) for quenching, and extracted with ethyl acetate (20 mL×3). The organic phases were combined, washed once with water (20 mL×1), washed once with a saturated sodium bicarbonate solution (20 mL×1), dried over anhydrous sodium sulfate, and filtered. The filtrate was concentrated under reduced pressure to obtain a colorless oily crude product. The crude product was purified by silica gel column chromatography (eluent: acetate: petroleum ether=50%, v/v) to obtain a mixture of amide and ester (0.15 g).

The mixture of ester and amide (0.15 g) was dissolved in a mixed solvent of ethanol (3 mL) and water (3 mL), and then lithium hydroxide (0.10 g, 4.2 mmol) was added. The reaction solution was heated to 85°C and stirred for reaction. After 32 hours, the heating was stopped, and the reaction solution was cooled to room temperature. LCMS indicated that the reaction was complete, and dilute hydrochloric acid (1 M) was added to the solution to adjust so that pH=1. The reaction solution was extracted with acetate (20 mL×3). The organic phases were combined, washed once with water (20 mL×1), dried over anhydrous sodium sulfate, and filtered. The filtrate was concentrated under reduced pressure to obtain a colorless oily crude product (130 mg), which was directly used for a next reaction without purification.

MS m/z (ESI): 567.2[M+H]+.

Step 2: (1S,2S)-2-((benzyloxy)methyl)-1-(5-((S)-2,2-dimethyltetrahydro-2H-pyran-4-yl)-2-(methyl(phenyl)carbamoyl)-1H-indol-1-yl)cyclopropane-1-carboxylic acid (0.13 g, 0.23 mmol) was dissolved in a mixed solvent of dichloromethane (3 mL) and N,N-dimethylformamide (0.1 mL). Oxalyl chloride (0.058 g, 0.46 mmol) was added to the reaction solution, and the mixture was stirred for reaction at room temperature for one hour. The reaction was completed by LCMS detection, and the mixture was spin-dried to obtain a yellow oily crude product (0.13 g), which was directly used for a next reaction without purification. The crude acyl chloride product (0.13 mg, 0.22 mmol) obtained above and triethylamine (0.067 g, 0.66 mmol) were dissolved in a mixed solvent of water, 50% hydrazine (1 mL) and tetrahydrofuran (2 mL), and stirred for reaction at room temperature for one hour. The reaction was completed by LCMS detection, and then the reaction was quenched with a saturated ammonium chloride solution (5 mL). Then, the reaction solution was extracted with dichloromethane (20mL×3). The organic phases were combined, dried over anhydrous sodium sulfate, filtered, and spin-dried to obtain a yellow oily crude product. The crude product was purified by preparative TLC (developing agent: methanol: dichloromethane
=10%, v/v) to obtain a target product (0.13 g, yield of 97%).

MS m/z (ESI): 581.3 [M+H]+.

Step 3: 1-((1S,2S)-2-((benzyloxy)methyl)-1-(hydrazinocarbonyl)cyclopropyl)-5-((S)-2,2-dimethyltetrahydro-2H-pyran-4-yl)-N-methyl-N-phenyl-1H-indole-2-carboxamide (0.13 g, 0.22 mmol), N,N'-dicarbonylimidazole (0.07 g, 0.44 mmol), and 1,8-diazacyclo[5.4.0]undec-7-ene (0.17 g, 1.1 mmol) were dissolved in tetrahydrofuran (3 mL), nitrogen was replaced to protect the reaction, and the solution was heated to 55 °C and stirred for reaction for 2 hours. LCMS indicated that the reaction was completed. The residue was spin-dried and dissolved in acetate (20 mL), and then washed with saturated brine (20 mL×3). The organic phase was dried over anhydrous sodium sulfate, filtered, and spin-dried. The crude product was purified by preparative TLC (developing agent: methanol: ethyl acetate=10%, v/v) to obtain a target product (0.093 g, yield of 70%).

MS m/z (ESI): 607.3 [M+H]+.

Step 4: A target compound is obtained with reference to step 8 of intermediate F.

MS m/z (ESI): 518.3 [M+H]+.

Step 5: A target compound is obtained by using 1-((1S,2S)-2-((benzyloxy)methyl)-1-(5-carbonyl-4,5-dihydro-1,3,4-oxadiazol-2-yl)cyclopropyl)-5-((S)-2,2-dimethyltetrahydro-2H-pyran-4-yl)-1H-indole-2-carboxylic acid as a raw material, with reference to steps 1, 2, 3 and 4 of Example 188.

MS m/z (ESI): 959.4[M+H]+.

Step 6: A target compound is obtained by using 5-((1S,2S)-1-(2-((S)-3-(3-(1-cyclopropyl-4-fluoro-1H-indazol-5-yl)-2-carbonyl-2,3-dihydro-1H-imidazol-1-yl)-2-(4-fluoro-3,5-dimethylphenyl)-4-methyl-4,5,6,7-tetrahydro-2H-pyrazolo [4,3-c]pyridine-5-carbonyl)-5-((S)-2,2-dimethyltetrahydro-2H-pyran-4-yl)-1H-indol-1-yl)-2-(2-hydroxypropane-2-yl-1,1,1,3,3,3-d6)cyclopropyl)-1,3,4-oxadiazol-2(3H)-one as a raw material, with reference to step 1 of Example 141.

MS m/z (ESI): 1008.4[M+H]+.

**Example 238**

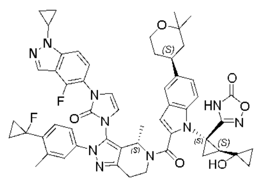

A target product was obtained with reference to the synthesis method of Example 192.
MS m/z (ESI): 977.4[M+1]

**Example 239**

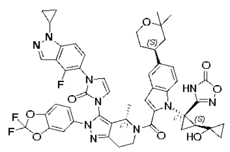

A target product was obtained with reference to the synthesis method of Example 192.
MS m/z (ESI): 985.4[M+1]

**Example 240**

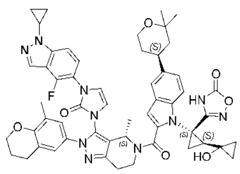

A target product was obtained with reference to the synthesis method of Example 192. MS m/z (ESI): 975.4[M+1]

**Example 241**

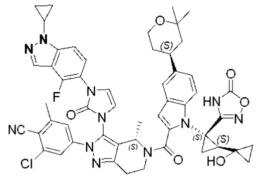

A target product was obtained with reference to the synthesis method of Example 192. MS m/z (ESI): 978.4[M+1]

**Example 242**

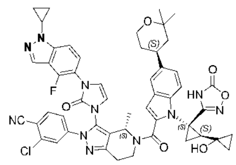

A target product was obtained with reference to the synthesis method of Example 192. MS m/z (ESI): 964.3[M+1]

**Example 243**

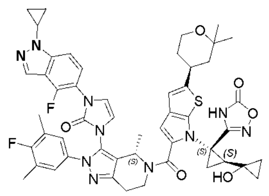

A target product was obtained with reference to the synthesis method of Example 192. MS m/z (ESI): 957.4[M+1]

**Example 244**

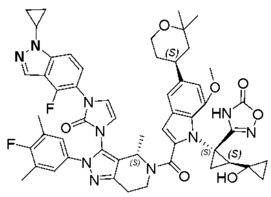

A target product was obtained with reference to the synthesis method of Example 192. MS m/z (ESI): 981.4[M+1]

**Example 245**

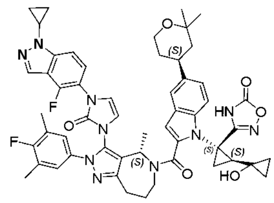

A target product was obtained with reference to the synthesis method of Example 192.
MS m/z (ESI): 965.4[M+1]

**Example 246**

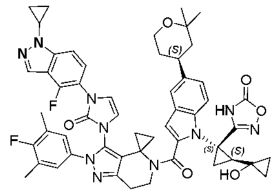

A target product was obtained with reference to the synthesis method of Example 192.
MS m/z (ESI): 963.4[M+1]

**Example 247**

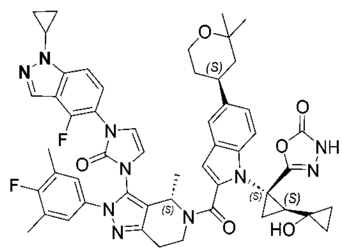

A target product was obtained with reference to the synthesis method of Example 192.
MS m/z (ESI): 951.4[M+1]

**Example 248**

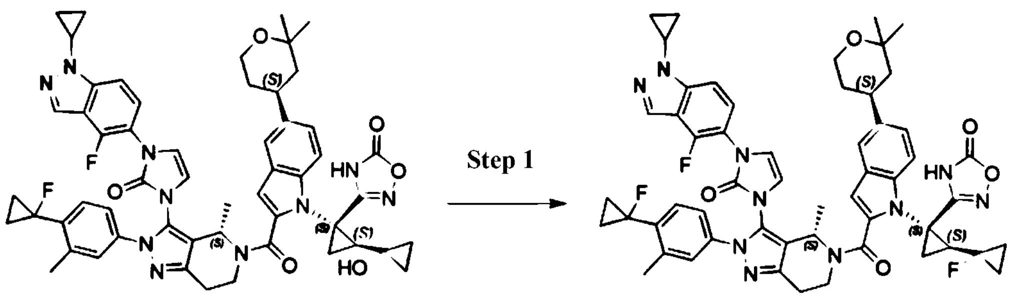

Step 1: Step 1: 3-((1S,2S)-2-(2-((S)-3-(3-(1-cyclopropyl-4-fluoro-1H-indazol-5-yl)-2-carbonyl-2,3-dihydro-1H-imidazol-1-yl)-2-(4-(1-fluorocyclopropyl)-3-methylphenyl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indol-1-yl)-1'-hydroxy-[1,1'-bi(cyclopropane)]-2-yl)-1,2,4-oxadiazol-5(4H)-one (0.44 g, 0.456 mmol) was dissolved in dichloromethane (10 mL), and then DAST (0.11 g, 0.684 mmol) was added at 0°C. The reaction solution was stirred at 25°C for 2 hours. The reaction was stopped, and quenched with water (10 mL). The reaction solution was extracted with dichloromethane (10 mL×2). The organic phases were combined, washed with saturated sodium chloride (10 mL), dried over anhydrous sodium sulfate, filtered, and concentrated under reduced pressure. The residue was purified by silica gel column chromatography with an eluent system of petroleum ether and ethyl acetate to obtain a target product (0.21 g), with a yield of 47.6%.

MS m/z (ESI): 979.4[M+1].

**Example 249**

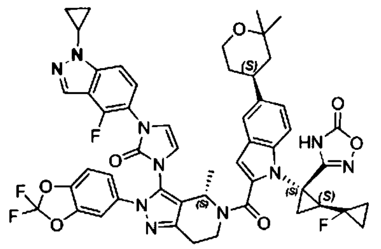

A target product was synthesized by using the final product of Example 239 as a raw material, with reference to Example 248.
MS m/z (ESI): 987.4[M+H]

**Example 250**

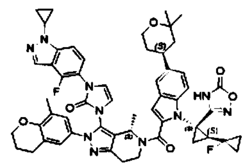

A target product was synthesized by using the final product of Example 240 as a raw material, with reference to Example 248.
MS m/z (ESI): 977.4[M+H]

**Example 251**

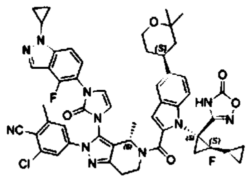

A target product was synthesized by using the final product of Example 241 as a raw material, with reference to Example 248.
MS m/z (ESI): 980.4[M+H]

**Example 252**

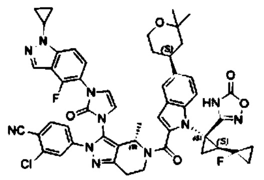

A target product was synthesized by using the final product of Example 242 as a raw material, with reference to Example 248.
MS m/z (ESI): 966.3[M+H]

**Example 253**

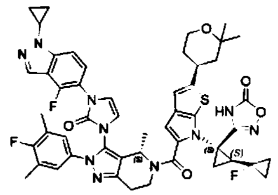

A target product was synthesized by using the final product of Example 243 as a raw material, with reference to Example 248.
MS m/z (ESI): 959.4[M+H]

**Example 254**

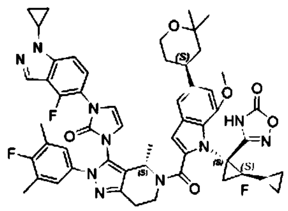

A target product was synthesized by using the final product of Example 244 as a raw material, with reference to Example 248.
MS m/z (ESI): 983.4[M+H]

**Example 255**

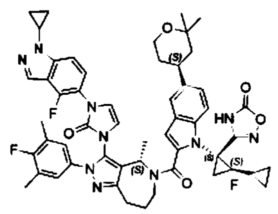

A target product was synthesized by using the final product of Example 245 as a raw material, with reference to Example 248.
MS m/z (ESI): 967.4[M+H]

**Example 256**

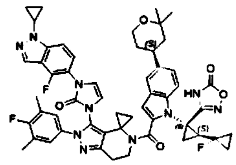

A target product was synthesized by using the final product of Example 246 as a raw material, with reference to Example 248.
MS m/z (ESI): 965.4[M+H]

**Example 257**

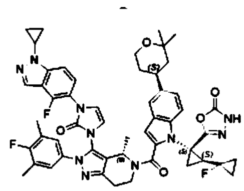

A target product was synthesized by using the final product of Example 247 as a raw material, with reference to Example 248.
MS m/z (ESI): 953.4[M+H]

**Example 258**

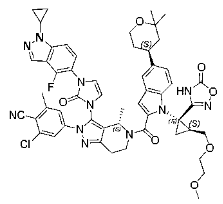

A target compound was obtained by using 4-bromo-2-chloro-6-methylbenzonitrile as a raw material, with reference to steps 3 to 5 of Example 182, and Example 189.
MS m/z (ESI): 1010.3[M+H]+.

**Example 259**

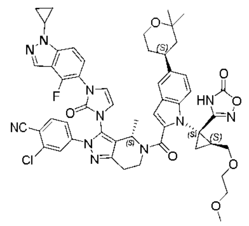

A target compound was obtained by using 4-bromo-2-chlorobenzonitrile as a raw material, with reference to steps 3 to 5 of Example 182, and Example 189.

MS m/z (ESI): 996.3[M+H]+.

**Example 260**

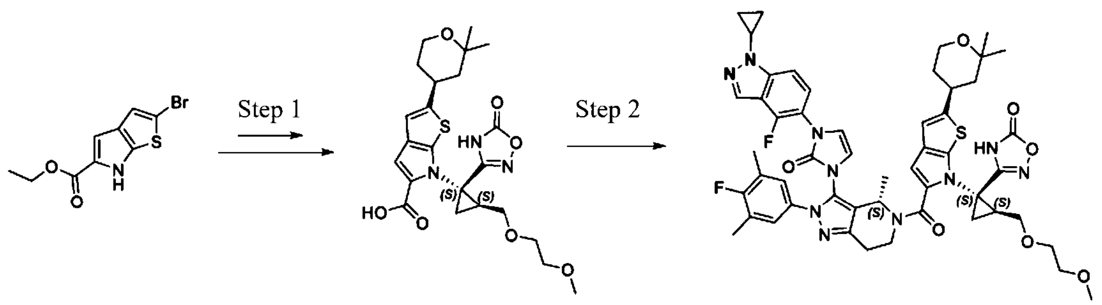


Step 1: A target compound was obtained by using ethyl 2-bromo-6H-thieno[2,3-b]pyrrole-5-carboxylate as a raw material, with reference to steps 1, 2 and 3 of Example 189, and the synthesis step of intermediate F.

MS m/z (ESI): 492.2[M+H]+.

A target compound was obtained by using (S)-1-(1-cyclopropyl-4-fluoro-1H-indazol-5-yl)-3-(2-(4-fluoro-3,5-dimethylphenyl)-4-methyl-4,5,6,7-tetrahydro-2H-pyrazolo[4,3-c]pyridin-3-yl)-1,3-dihydro-2H-imidazole-2-one and 2-((S)-2,2-dimethyltetrahydro-2H-pyran-4-yl)-6-((1S,2S)-2-((2- methoxyethoxy)methyl)-1-(5-carbonyl-4,5-dihydro-1,2,4-oxadiazol-3-yl)cyclopropyl)-6H-thieno[2,3-b]pyrrole-5-carboxylic acid as raw materials, with reference to step 4 of Example 25.

MS m/z (ESI): 989.3[M+H]+.

**Example 261**

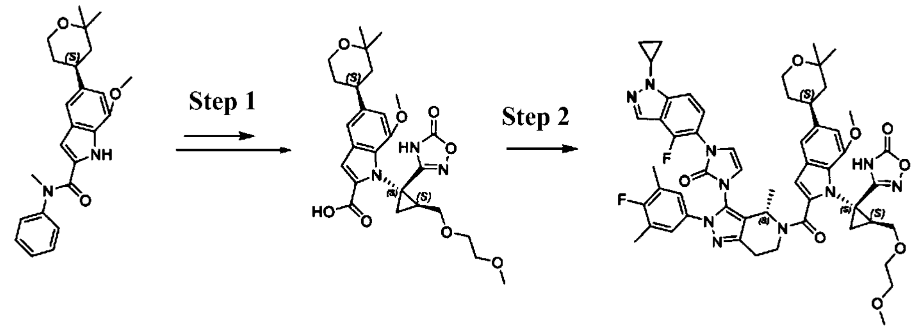


Step 1: A target compound was obtained by using (S)-5-(2,2-dimethyltetrahydro-2H-pyran-4-yl)-7-methoxy-N-methyl-N-phenyl-1H-indole-2-carboxamide as a raw material, with reference to steps 1, 2 and 3 of Example 189, and the synthesis step of intermediate F.

MS m/z (ESI): 516.2[M+H]+.

Step 2: A target compound was obtained by using (S)-1-(1-cyclopropyl-4-fluoro-1H-indazol-5-yl)-3-(2-(4-fluoro-3,5-dimethylphenyl)-4-methyl-4,5,6,7-tetrahydro-2H-pyrazolo[4,3-c]pyridin-3-yl)-1,3-dihydro-2H-imidazole-2-one and 5-((S)-2,2-dimethyltetrahydro-2H-pyran-4-yl)-7-methoxy-1-((1S,2S)-2-((2- methoxyethoxy)methyl)-1-(5-carbonyl-4,5-dihydro-1,2,4-oxadiazol-3-yl)cyclopropyl)-1H-indole-2-carboxylic acid as raw materials, with reference to Example 25.

MS m/z (ESI): 1013.4[M+H]+.

**Example 262**

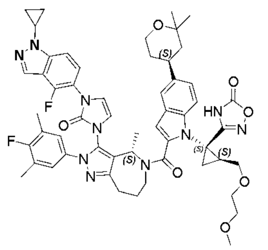

A target product was obtained with reference to Examples 201 and 189.
MS m/z (ESI): 997.4[M+H]+.

**Example 263**

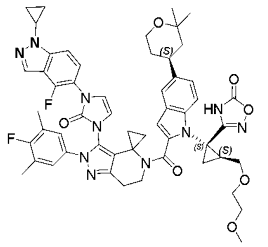

A target product was obtained with reference to Examples 51 and 189.
MS m/z (ESI): 995.4[M+H]+.

**Example 264**

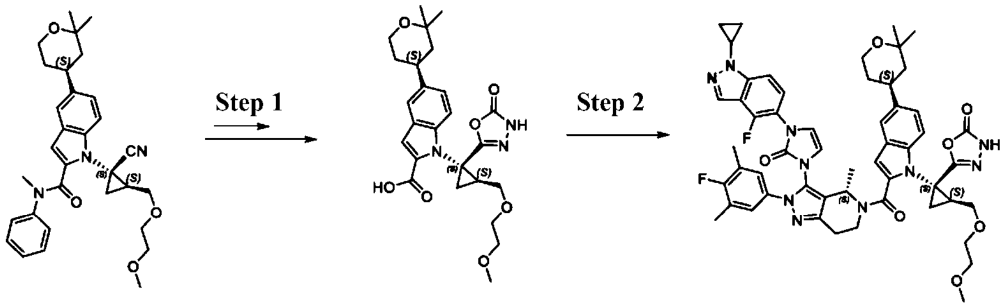


Step 1: A target compound was obtained by using 1-((1S,2S)-1-cyano-2-((2 - methoxyethoxy)methyl)cyclopropyl)-5-((S)-2,2-dimethyltetrahydro-2H-pyran-4-yl)-N-methyl-N-phenyl-1H-indole-2-carboxamide as a raw material, with reference to steps 1, 2, 3 and 4 of Example 237.

MS m/z (ESI): 486.2[M+H]+.

Step 2: A target compound was obtained by using (S)-1-(1-cyclopropyl-4-fluoro-1H-indazol-5-yl)-3-(2-(4-fluoro-3,5-dimethylphenyl)-4-methyl-4,5,6,7-tetrahydro-2H-pyrazolo[4,3-c]pyridin-3-yl)-1,3-dihydro-2H-imidazole-2-one and 5-((S)-2,2-dimethyltetrahydro-2H-pyran-4-yl)-1-((1S,2S)-2-((2- methoxyethoxy)methyl)-1-(5-carbonyl-4,5-dihydro-1,3,4-oxadiazol-2-yl)cyclopropyl)-1H-indole-2-carboxylic acid as raw materials, with reference to step 4 of Example 25.

MS m/z (ESI): 983.4[M+H]+

**Example 265**

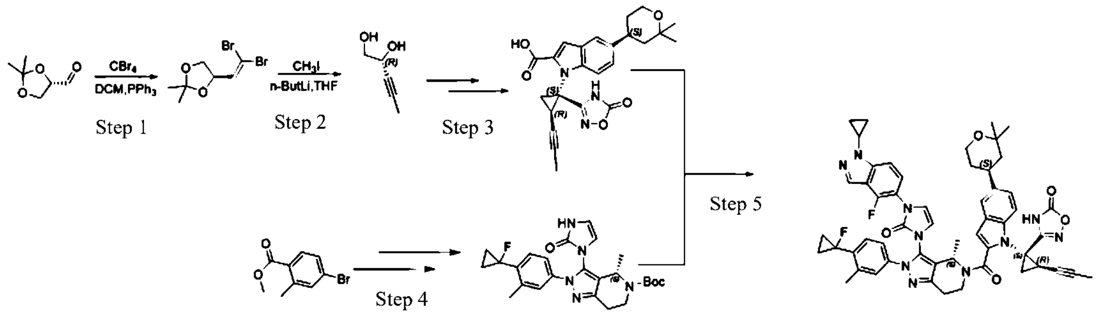


Step 1: Triphenylphosphine (48.6 g, 185 mmol) was dissolved in dichloromethane (150 mL) at room temperature, carbon tetrabromide (30.6 g, 92.3 mmol) was added at 0°C, and they reacted at room temperature for 30 minutes. A dichloromethane solution (10 mL) of (S)-2,2-dimethyl-1,3-dioxolane-4-carboxaldehyde (6 g, 46.1 mmol) was added at 0°C, and they reacted at room temperature for 3 hours. 250 mL of n-hexane was added at 0°C, and the mixture was stirred for 1 h. The mixture was filtered and spin-dried, and the residue was separated by Flash column chromatography to obtain a target product (12 g, yield of 91%).

MS m/z (ESI): 286.9 , 284.9 [M+H]+.

Step 2: n-butyllithium (2.5 M, 12.4 mL, 31.0 mmol) was added to a THF solution (700 mL) of (R)-4-(2,2-dibromovinyl)-2,2-dimethyl-1,3-dioxolane (4.0 g, 13.99 mmol) at -78°C. The mixture was stirred at -78°C for 1 h, and slowly heated to room temperature. Iodomethane (1.31 mL, 21.04 mmol) was added at room temperature, and the mixture was stirred at room temperature for 4 h. 30 mL of water was added, and the reaction solution was extracted with ethyl acetate (50 mL×3). The organic phases were combined, dried with anhydrous sodium sulfate, filtered, and spin-dried. 30 mL of methanol and p-toluenesulfonic acid (133 mg, 0.7 mmol) were added. The mixture was stirred at room temperature for 18 h, and spin-dried. The residue was separated by Flash column chromatography to obtain a target product (700 mg, yield of 50%).

Step 3: A target product was obtained by using (R)-pent-3-yne-1,2-diol as a raw material, with reference to the synthesis method of intermediate F.

MS m/z (ESI): 436.2[M+H]+.

Step 4: A target product was obtained by using methyl 4-bromo-2-methylbenzoate as a raw material, with reference to steps 1 to 4 of Example 182.

MS m/z (ESI): 468.2[M+H]+.

Step 5: A target product was obtained by using 5-((S)-2,2-dimethyltetrahydro-2H-pyran-4-yl)-1-((1S,2R)-1-(5-carbonyl-4,5-dihydro-1,2,4-oxadiazol-3-yl)-2-(prop-1-yne-1-yl)cyclopropyl)-1H-indole-2-carboxylic acid and tert-butyl(S)-2-(4-(1- fluorocyclopropyl)-3-methylphenyl)-4-methyl-3-(2-carbonyl-2,3-dihydro-1H-imidazol-1-yl)-2,4,6,7-tetrahydro-5H-pyrazolo[4,3-c]pyridine-5-carboxylate as raw materials, with reference to the synthesis method of Example 25.

MS m/z (ESI): 959.4[M+H]+.

**Example 266**

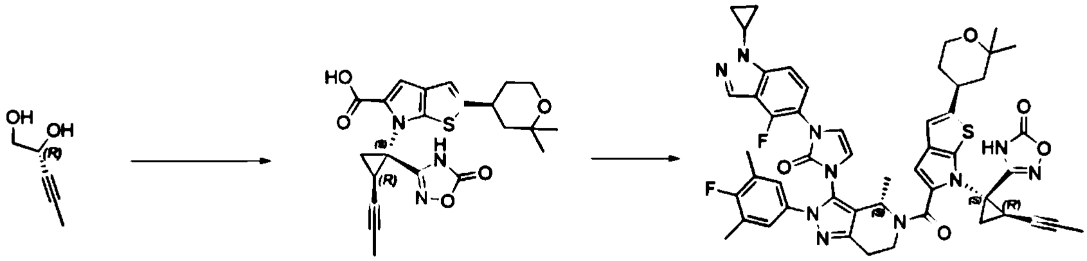

Step 1: A product was obtained by using (R)-pent-3-yne-1,2-diol and 2-bromo-6H-thieno[2,3-b]pyrrole-5-carboxylic acid ethyl ester as raw materials, with reference to the synthesis method of intermediate F.
MS m/z (ESI): 442.1[M+H]+.
Step 2: A product was obtained by using 2-((S)-2,2-dimethyltetrahydro-2H-pyran-4-yl)-6-((1S,2R)-1-(5-carbonyl-4,5-dihydro-1,2,4-oxadiazol-3-yl)-2-(prop-1-yne-1-yl)cyclopropyl)-6H-thieno[2,3-b]pyrrole-5-carboxylic acid as a raw material, with reference to the synthesis method of Example 25.
MS m/z (ESI): 939.4[M+H]+.

**Example 267**

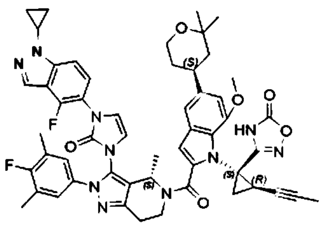

A product was obtained by using (R)-pent-3-yne-1,2-diol as a raw material, with reference to the synthesis method of Example 214.
MS m/z (ESI): 963.4[M+H]+.

**Example 268**

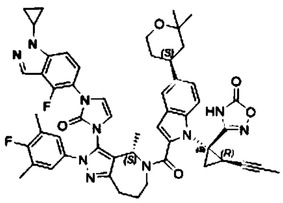

A product was obtained by using (R)-pent-3-yne-1,2-diol as a raw material, with reference to the synthesis method of Example 215.
MS m/z (ESI): 947.4[M+H]+.

**Example 269**

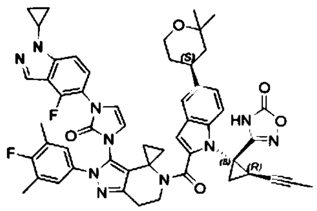

A product was obtained by using (R)-pent-3-yne-1,2-diol as a raw material, with reference to the synthesis method of Example 216.

MS m/z (ESI): 945.4[M+H]+.

**Example 270**

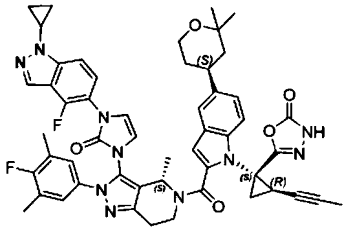

A product was obtained by using (R)-pent-3-yne-1,2-diol as a raw material, with reference to the synthesis method of Example 217.

MS m/z (ESI): 933.4[M+H]+.

**Example 271**

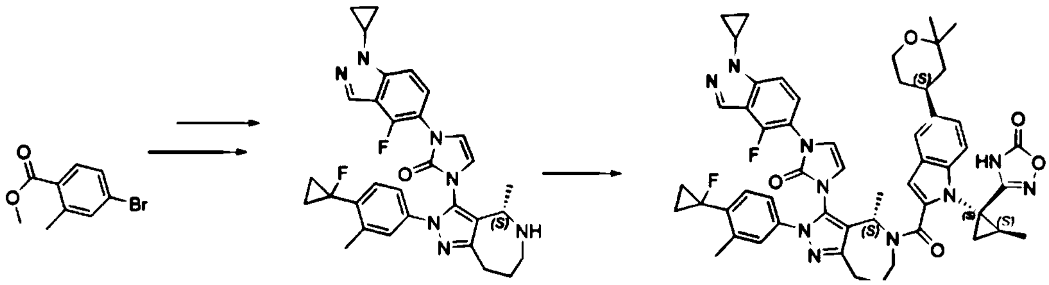

Step 1: A target product was obtained by using methyl 4-bromo-2-methylbenzoate as a raw material, with reference to the synthesis methods of Examples 208 and 215.

MS m/z (ESI): 556.3[M+H]+.

Step 2: A target product was obtained by using (S)-1-(1-cyclopropyl-4-fluoro-1H-indazol-5-yl)-3-(2-(4-(1-fluorocyclopropyl)-3-methylbenzene)-4-methyl-2,4,5,6,7,8-hexahydropyrazolo[4,3-c]azepin-3-yl)-1,3-dihydro-2H-imidazole-2-one as a raw material, with reference to the synthesis method of Example 25.

MS m/z (ESI): 949.4[M+H]+.

**Example 272**

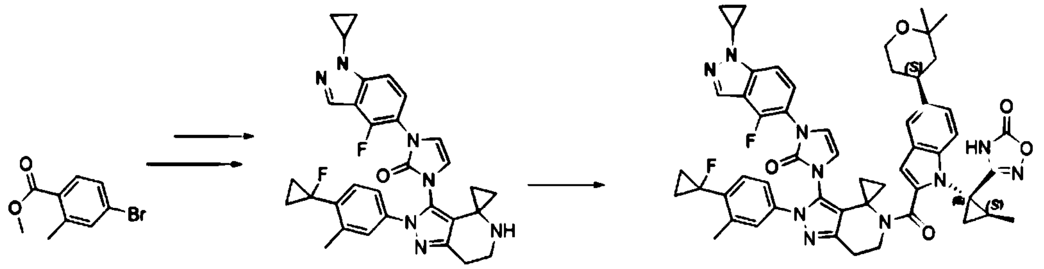

Step 1: A target product was obtained by using methyl 4-bromo-2-methylbenzoate as a raw material, with reference to the synthesis methods of Examples 208 and 206.

MS m/z (ESI): 554.2[M+H]+.

Step 2: A target product was obtained by using 1-(1-cyclopropyl-4-fluoro-1H-indazol-5-yl)-3-(2'-(4-(1- fluorocyclopropyl)-3-methylbenzene)-2',5',6',7'-tetrahydrospiro[cyclopropane-1,4'-pyrazolo[4,3-c]pyridine]-3'-yl)-1,3-dihydro-2H-imidazole-2-one as a raw material, with reference to the synthesis method of Example 25.

MS m/z (ESI): 947.4[M+H]+.

**Example 273**

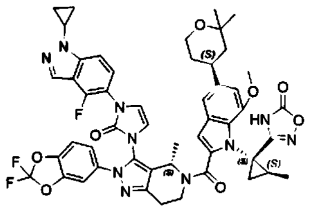

A target product was obtained by using (2,2-difluorobenzo[d][1,3]dioxazol-5-yl)hydrazine as a raw material, with reference to the synthesis method of Example 186.

MS m/z (ESI): 973.3[M+1].

**Example 274**

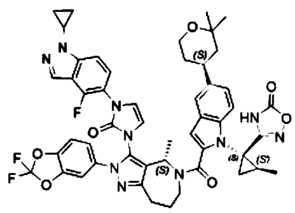

A target product was obtained by using (2,2-difluorobenzo[d][1,3]dioxazol-5-yl)hydrazine as a raw material, with reference to the synthesis method of Example 201.

MS m/z (ESI): 957.3[M+1].

**Example 275**

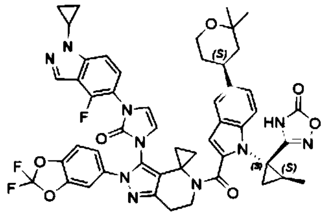

A target product was obtained by using (2,2-difluorobenzo[d][1,3]dioxazol-5-yl)hydrazine as a raw material, with reference to the synthesis method of Example 51.

MS m/z (ESI): 955.3[M+1].

**Example 276**

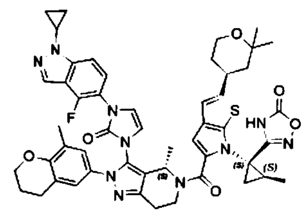

A target product was obtained by using (8-methylchroman-6-y1)hydrazine as a raw material, with reference to the synthesis method of Example 226.

MS m/z (ESI): 939.3[M+1].

**Example 277**

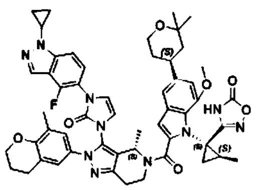

A target product was obtained by using (8-methylchroman-6-yl)hydrazine as a raw material, with reference to the synthesis method of Example 186.

MS m/z (ESI): 963.4[M+1].

**Example 278**

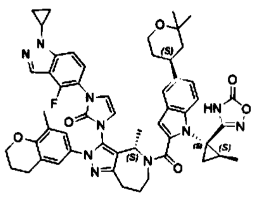

A target product was obtained by using (8-methylchroman-6-yl)hydrazine as a raw material, with reference to the synthesis method of Example 201.

MS m/z (ESI): 947.4[M+1].

**Example 279**

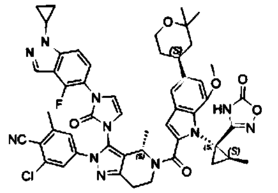

A titled product was obtained with reference to Example 485.
MS m/z (ESI): 966.4[M+1].

**Example 280**

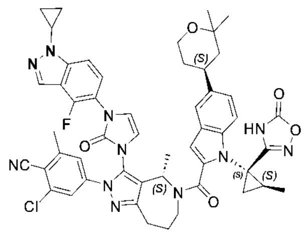

A titled product was obtained with reference to Example 215.
MS m/z (ESI): 950.4[M+1].

**Example 281**

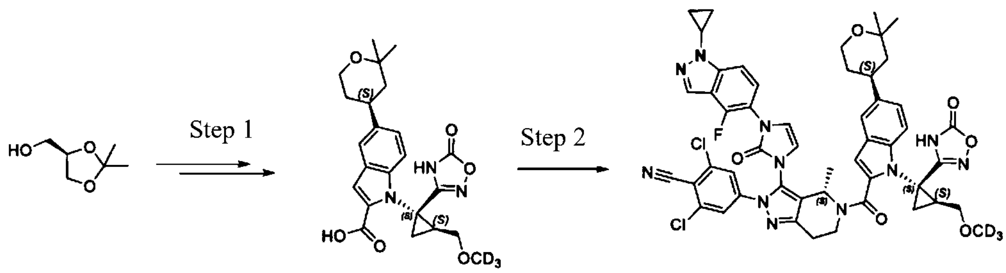


Step 1: A target product was obtained by using (R)-(2,2-dimethyl-1,3-dioxolane-4-yl)methanol as a raw material, with reference to the synthesis method of Example 189, and replacing 1-bromo-2-methoxy-ethane with deuterated iodomethane.
MS m/z (ESI): 445.2[M+1].
Step 2: A target product was obtained by using (S)-2,6-dichloro-4-(3-(3-(1-cyclopropyl-4-fluoro-1H-indazol-5-yl)-2-carbonyl-2,3-dihydro-1H-imidazol-1-yl)-4-methyl-4,5,6,7-tetrahydro-2H-pyrazolo[4,3-c]pyridin-2-yl)benzonitrile and 5-((S)-2,2-dimethyltetrahydro-2H-pyran-4-yl)-1-((1S,2S)-2-((methoxy-d3)methyl)-1-(5-carbonyl-4,5-dihydro-1,2,4-oxadiazol-3-yl)cyclopropyl)-1H-indole-2-carboxylic acid as raw materials, with reference to the synthesis method of step 4 of Example 25.
MS m/z (ESI): 989.3[M+1].

**Example 282**

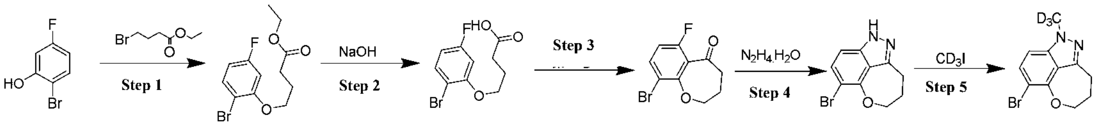


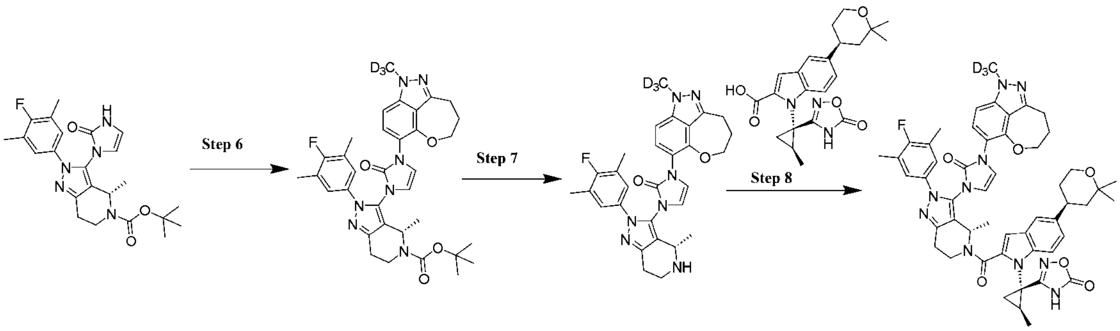


Step 1: Ethyl 4-(2-bromo-5-fluorophenoxy)butyrate

A mixture of 2-bromo-5-fluorophenol (5 g, 26.18 mmol), ethyl 4-bromobutyrate (10.21 g, 52.36 mmol), DMF (50 mL), and potassium carbonate (10.85 g, 78.54 mmol) was stirred at 100 °C for 1 hour. LCMS showed that the reaction was complete and there was MS of a target product. The reaction was stopped, and water was added to quench the reaction. The mixture was extracted with dichloromethane (100 mL×2). The organic phases were combined, washed with saturated sodium chloride (50 mL), dried over anhydrous sodium sulfate, filtered and concentrated, and separated by column chromatography (PE/EA=4: 1) to obtain a colorless oily substance (7 g, yield: 87.6%).

MS m/z (ESI): 305.0 307.0 [M+1].

Step 2: 4-(2-bromo-5-fluorophenoxy)butyric acid Sodium hydroxide (1.97 g, 49.16 mmol) was added to a mixture of a THF solution (30 mL) of ethyl 4-(2-bromo-5-fluorophenoxy)butyrate (5 g, 16.39 mmol), water (30 mL), and methanol (30 mL), and the mixture was stirred at room temperature for 2 hours. LCMS showed that the reaction was complete and there was MS of a target product. Dilute hydrochloric acid was added to adjust so that pH= 3. Extraction was performed three times with DCM. The organic phase was washed with saturated brine, and dried over anhydrous sodium sulfate. The filtrate was concentrated under reduced pressure to obtain a white solid (4 g, crude product).

MS m/z (ESI): 277.0 279.0 [M+1].

Step 3: 9-Bromo-6-fluoro-3,4-dihydrobenzo[b]oxepin-5(2H)-one

A mixture of 4-(2-bromo-5-fluorophenoxy)butyric acid (2.6 g, 9.38 mmol), polyphosphoric acid (3.17 g, 9.38 mmol, 20 mL) and toluene (60 mL) was stirred at 120°C for 12 hours, and water was added to quench the reaction. Extraction was performed with dichloromethane (50 mL×2). The organic phases were combined, washed with saturated sodium chloride (50 mL), dried over anhydrous sodium sulfate, filtered and concentrated, separated by column chromatography and concentrated to obtain a titled product (500 mg, yield: 20.6%).

MS m/z (ESI): 259.0 261.0 [M+1].

Step 4: 5-bromo- 2,7,8,9 -tetrahydrooxepin[4,3,2-cd]indazole

A mixture of 9-bromo-6-fluoro-3,4-dihydrobenzo[b]oxepin-5(2H)-one (500 mg, 1.93 mmol), hydrazine hydrate (727.60 mg, 19.30 mmol, 85% purity) and 1,4-dioxane (10 mL) was stirred at 110 °C for 12 hours. LCMS showed that the reaction was complete and there was a target product. Water was added to quench the reaction, and extraction was performed with dichloromethane (50 mL×2). The organic phases were combined, washed with saturated sodium chloride (50 mL), dried over anhydrous sodium sulfate, filtered and concentrated, separated by preparative chromatography (PE/EA=1:1) and concentrated to obtain a titled product (400 mg, yield: 81.9%).

MS m/z (ESI): 253.0 255.0[M+1].

Step 5: Sodium hydrogen sulfide (23.70 mg, 592.66 μmol, 60% purity) was added to a DMF solution (5 mL) of 5-bromo-2,7,8,9-tetrahydrooxepin [4,3,2-cd] indazole (100 mg, 395.11 μmol). The mixture was stirred at room temperature for 0.5 hour, and deuterated iodomethane (85.94 mg, 592.66 μmol) was added dropwise. Then, the mixture was stirred at room temperature for 0.5 hour. LCMS showed that the reaction was complete and there was MS of a target product. Water was added to quench the reaction, and extraction was performed with dichloromethane (50 mL×2). The organic phases were combined, washed with saturated sodium chloride (50 mL), dried over anhydrous sodium sulfate, filtered and concentrated, separated by preparative chromatography (PE/EtOAc=1:1), and concentrated to obtain the product of 5-bromo-2-(methyl-d3)-2,7,8,9-tetrahydrooxepin[4,3,2-cd]indazole (60 mg, yield: 56.2%).

MS m/z (ESI): 270.0 272.0 [M+1].

Step 6: A mixture of 5-bromo-2-(methyl-d3)-2,7,8,9-tetrahydrooxepin[4,3,2-cd]indazole (64.25 mg, 237.83 μmol), tert-butyl(S)-2-(4-fluoro-3,5-dimethylphenyl)-4-methyl-3-(2-carbonyl-2,3-dihydro-1H-imidazol-1-yl)-2,4,6,7-tetrahydro-5H-pyrazolo[4,3-c]pyridine-5-carboxylate (70 mg, 158.55 μmol), cuprous iodide (24.16 mg, 126.84 μmol), (1S,2S)-N,N'-dimethyl-1,2-cyclohexanediamine (22.55 mg, 158.55 μmol), potassium carbonate (65.64 mg, The mixture of 475.65 μmol) and NMP (3 mL) was stirred at 130°C for 16 hours under N2 protection. LCMS showed that the reaction was complete and there was MS of a target product. Water and EtOAc were added for extraction. The organic phase was washed with saturated brine, dried over anhydrous sodium sulfate, and separated by column chromatography (DCM/MeOH=10:1) to obtain tert-butyl(S)-2-(4-fluoro-3,5-dimethylbenzene)-4-methyl-3-(3-(2-(methyl-d3)-2,7,8,9-tetrahydrooxaheptano[4,3,2-cd]indazol -5-yl)-2-carbonyl-2,3-dihydro-1H-imidazole-1-yl)-2,4,6,7-tetrahydro-5H-pyrazolo [4,3-c]pyridine-5-carboxylate (40 mg, yield: 40.0%).

MS m/z (ESI): 631.1[M+1].

Step 7: TFA (7.23 mg, 63.42 μmol, 1 mL) was added dropwise to a DCM solution (2 mL) of tert-butyl(S)-2-(4-fluoro-3,5-dimethylbenzene)-4-methyl-3-(3-(2-(methyl-d3)-2,7,8,9-tetrahydrooxaheptano[4,3,2-cd]indazol -5-yl)-2-carbonyl-2,3-dihydro-1H-imidazol-1-yl)-2,4,6,7-tetrahydro-5H-pyrazolo[4,3-c]pyridine-5-carboxylate (40 mg, 63.42 μmol). The mixture was stirred at room temperature for 0.5 h. LCMS showed that the reaction was complete and there was MS of a target product. The mixture was spin-dried, saturated NaHCO3 was added to adjust so that pH=10, and extraction was performed with DCM three times. The organic phase was washed with saturated brine, dried over anhydrous sodium sulfate, and filtered. The filtrate was concentrated under reduced pressure to obtain a light yellow oily substance of (S)-1-(2-(4-fluoro-3,5-dimethylbenzene)-4-methyl-4,5,6,7-tetrahydro-2H-pyrazolo[4,3-c]pyridin-3-yl)-3-(2-(methyl-d3)-2,7,8,9-tetrahydrooxepino[4,3,2-cd]indazol-5-yl)-1,3-dihydro-2H-imidazol-2-one (30 mg, crude product).

MS m/z (ESI): 531.3 [M+1].

Step 8: HATU (21.33 mg, 56.54 μmol) and DIEA (10.96 mg, 84.81 μmol, 14.77 μL) were added to a DMF solution (2 mL) of (S)-1-(2-(4-fluoro-3,5-dimethylphenyl)-4-methyl-4,5,6,7-tetrahydro-2H-pyrazolo[4,3-c]pyridin-3-yl)-3-(2-(methyl-d3)-2,7,8,9-tetrahydrooxepino[4,3,2-cd]indazol-5-yl)-1,3-dihydro-2H-imidazol-2-one (15 mg, 28.27 μmol), and 5-((S)-2,2-dimethyltetrahydro-2H-pyran-4-yl)-1-((1S,2S)-2-methyl-1-(5-carbonyl-4,5-dihydro-1,2,4-oxadiazol-3-yl)cyclopropyl)-1H-indole-2-carboxylic acid (17.45 mg, 42.40 μmol). The mixture was stirred at room temperature for 16 hours. LCMS showed that the reaction was complete and there was MS of a target product. Water was added, and extraction was performed three times with DCM. The organic phase was washed with saturated brine, dried over anhydrous sodium sulfate, and filtered. The filtrate was concentrated under reduced pressure to obtain a light yellow oily substance, which was separated by preparative chromatography (acidic) to obtain 3-((1S,2S)-1-(5-((S)-2,2-dimethyltetrahydro-2H-pyran-4-yl)-2-((S)-2-(4-fluoro-3,5-dimethylphenyl)-4-methyl-3-(3-(2-(methyl-d3)-2,7,8,9-tetrahydrooxaheptano[4,3,2-cd]indazol-5-yl)-2-carbonyl-2,3-dihydro-1H-imidazol-1-yl)-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one (5.8 mg, yield: 22.1%).

$^1$H NMR (400 MHz, DMSO) δ [12.30(s) and 11.77 (s), total 1 H], 7.54 (s, 1H), 7.43-7.31 (m, 1H), 7.28-7.05 (m, 5H), 6.99-6.60 (m, 3H), 5.76-5.20 (m, 1H), 4.62-4.16 (m, 2H), 3.80-3.57 (m,3H), 3.22-2.76 (m, 5H), 2.25-2.07 (m, 8H), 1.81-1.41 (m, 10H), 1.35-1.15 (m, 10H).

MS m/z (ESI): 924.4[M+1].

**Example 283**

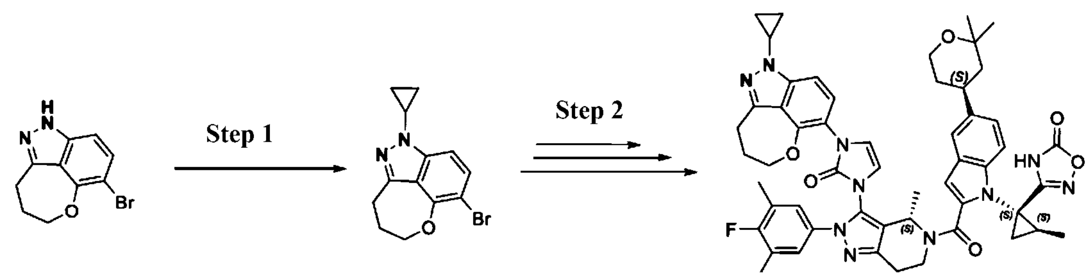


Step 1: 5-bromo-2,7,8,9-tetrahydrooxepin[4,3,2-cd] indazole (2.53 g, 10 mmol) was dissolved in 1,2-dichloroethane (30 mL) at room temperature, and then cyclopropylboronic acid (1.29 g, 15 mmol), acetic acid ketone (0.91 g, 5 mmol), 2,2-bipyridine (0.78 g, 5 mmol) and sodium carbonate (3.18 g, 30 mmol) were added. Oxygen was replaced, and the mixture was heated to 80°C, and reacted for 16 hours. LCMS indicated that the reaction was complete. The mixture

was cooled to room temperature. The reaction solution was filtered with diatomaceous earth, and then washed with dichloromethane. The organic phases were combined, and then the organic phases were spin-dried. The residue was purified by flash column chromatography (petroleum ether: ethyl acetate: 1:1) to obtain a target compound as a white solid (2.05 g, yield of 70%).

MS m/z (ESI): 293.1 , 295.1 [M+H]+.

Step 2: A target compound was obtained by using 5-bromo-2-cyclopropyl-2,7,8,9-tetrahydrooxepin[4,3,2-cd] indazole as a raw material, with reference to steps 6, 7 and 8 of Example 282.

MS m/z (ESI): 947.4[M+H]+.

## Example 284

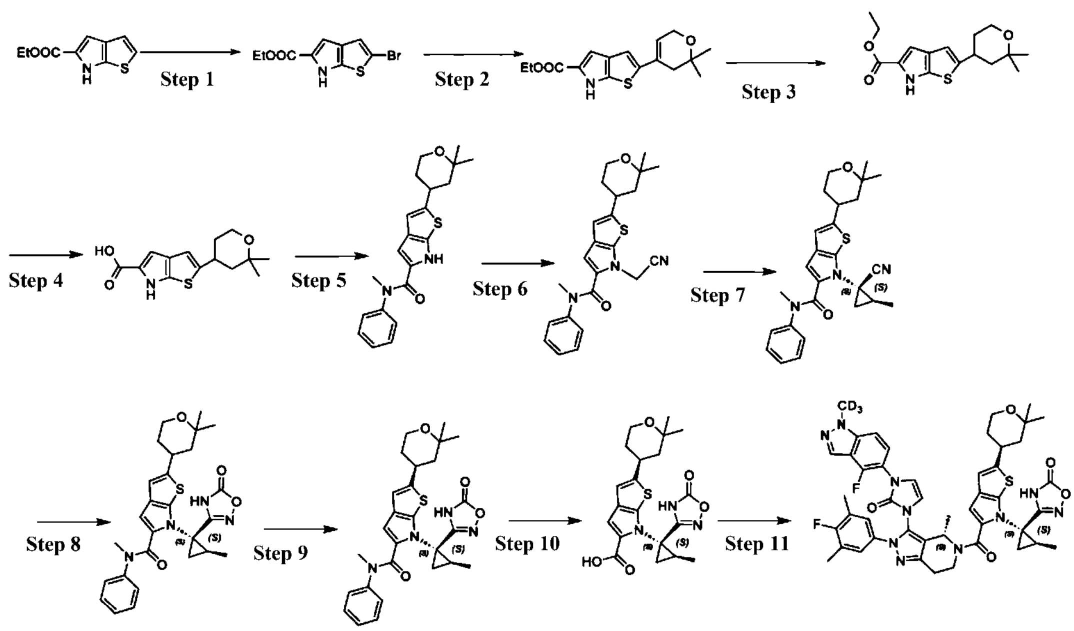


Step 1: 6H-thieno[2,3-b]pyrrole-5-carboxylic acid ethyl ester (9 g, 46.10 mmol) was dissolved in CHCl3 (100 mL), NBS (8.20 g, 46.10 mmol) was added in three batches at 0°C, and the reaction solution was stirred for 0.5 hours. It was monitored by LCMS that the raw material disappeared. 500 mL of water and DCM (300 mL X3) were added for extraction. The organic phase was dried over anhydrous sodium sulfate, and concentrated in vacuum. The crude product was separated by column chromatography (PE:EtOAc=96:4) to obtain 2-bromo-6H-thieno[2,3-b]pyrrole-5-carboxylic acid ethyl ester (9 g, yellow solid), with a yield of 71.22%.

MS m/z (ESI): 273.9 , 275.9 [M+1].

Step 2: 2-bromo-6H-thieno[2,3-b]pyrrole-5-carboxylic acid ethyl ester (9 g, 32.83 mmol) and 2-(6,6-dimethyl-2,5-dihydropyran-4-yl)-4,4,5-5-tetramethyl-1,3,2-dioxaborane (10.16 g, 42.68 mmol) were dissolved in H2O (20 mL) and 1'4-Dioxane (100 mL), and Pd(dppf)Cl2 (2.40 g, 3.28 mmol) and K3PO4 (13.94 g, 65.66 mmol) were added at room temperature. Nitrogen was replaced three times, and the reaction solution was stirred at 90°C for 8 hours. It was monitored by LCMS that the raw material disappeared.

500 mL of water and EtOAC (300 mL×3) were added to the reaction solution for extraction. The organic phase was dried over anhydrous sodium sulfate and concentrated in vacuum. The crude product was purified by column chromatography (PE:EA=90:10) to obtain a target product (4.1 g, yellow solid), with a yield of 40.89%.

MS m/z (ESI): 306.1 [M+1].

Step 3: 2-(6,6 - dimethyl-2,5-dihydropyran-4-yl)-6H-thieno[2,3-b]pyrrole-5-carboxylic acid ethyl ester (4.1 g, 13.43 mmol) was dissolved in TFA (40 mL), and Et3SiH (1.56 g, 13.43 mmol) was added at room temperature. The reaction was stirred for 10 min. It was monitored by LCMS that the raw material disappeared. Ice cubes were added to the reaction solution, and the pH was adjusted to 7-8 with saturated sodium bicarbonate solution. EtOAc (200 mLx3) was added for extraction, and the organic phase was dried over anhydrous sodium sulfate and concentrated in vacuum. The crude product was purified by column chromatography to obtain a target product (2.8 g, yellow solid), with a yield of 67.82%.

MS m/z (ESI): 308.1[M+1].

Step 4: Ethyl 2-[2,2-dimethyltetrahydropyran-4-yl]-6H-thieno[2,3-b]pyrrole-5-carboxylate (2.8 g, 9.11 mmol) was

dissolved in MeOH (20 mL) and H2O (10 mL), NaOH (801.49 mg, 20.04 mmol) was added at room temperature, and the reaction solution was stirred at 70°C for 3 hours. It was monitored by LCMS that the raw material disappeared. After the reaction solution was cooled to room temperature, 200 mL of EtOAc was added to extract the organic phase. Water was added to hydrochloric acid so that the pH was adjusted to 5 to 6, and EtOAc (200 mL×3) was added to extract the organic phase. The organic phase was dried over anhydrous sodium sulfate and concentrated in vacuum. The crude product was purified by column chromatography (DCM:MeOH=96:4) to obtain the product of 2-[2,2-dimethyltetrahydropyran-4-yl]-6H-thieno[2,3-b]pyrrole-5-carboxylic acid (2.2 g, yellow solid), with a yield of 86.46%.

MS m/z (ESI): 280.0[M+1].

Step 5: Step 5: 2-[2,2-dimethyltetrahydropyran-4-yl]-6H-thieno[2,3-b]pyrrole-5-carboxylic acid (2.2 g, 7.88 mmol) was dissolved in Tol. (20 mL), and nitrogen was replaced three times. DMF (57.56 mg, 787.53 μmol, 60.98 μL) and oxalyl chloride (2.00 g, 15.75 mmol) were added at room temperature, and the reaction solution was stirred at 100°C for 2 hours. The reaction solution was directly concentrated in vacuum, the mixture was drained and dissolved in DCM (20 mL). Triethylamine (1.59 g, 15.75 mmol, 2.20 mL) and N-methylaniline (1.69 g, 15.75 mmol) were added in sequence. Nitrogen was replaced three times to react at 30°C for 8 hours. The reaction solution was concentrated in vacuum, and the crude product was purified by column chromatography (PE:EA=80:20) to obtain a target product (1.9 g, yellow solid), with a yield of 65.47%.

MS m/z (ESI): 369.1[M+1].

Step 6: 2-[2,2-dimethyltetrahydropyran-4-yl]-N-methyl-N-phenyl-6H-thieno[2,3-b]pyrrole-5-carboxamide (1.9 g, 5.16 mmol) was dissolved in DMF (15 mL), nitrogen was replaced three times, and NaH (371.21 mg, 15.47 mmol) was slowly added at 0°C. The mixture was stirred for 0.5 hours, and then 2-bromoacetonitrile (1.86 g, 15.47 mmol) was added, and the reaction solution was stirred at 30°C for 8 hours. It was monitored by LCMS that a small amount of raw material remained and the product was generated. H2O (300 mL) and EtOAc (200 mL×3) were added to the reaction solution for extraction. The organic phase was dried over anhydrous sodium sulfate and concentrated in vacuum. The crude product was purified by column chromatography (PE:EtOAc=74:26) to obtain a target product (2 g, yellow solid), with a yield of 95.18%.

MS m/z (ESI): 408.1[M+1].

Step 7: 6-(cyanomethyl)-2-[2,2-dimethyltetrahydropyran-4-yl]-N-methyl-N-phenylthieno[2,3-b]pyrrole-5-carboxamide and (4R)-4-methyl-1,3,2-dioxathiacyclohexane 2,2-dioxide (2.37 g, 17.18 mmol) were dissolved in DMPU (20 mL). The mixture was cooled to 0°C in an ice bath, and KHMDS (9.79 g, 49.08 mmol) was slowly added dropwise. The mixture reacted at 0°C for 5 hours after adding dropwise. It was monitored by LCMS that the raw material disappeared. Formic acid was added so that the pH was adjusted to acidic. The mixture was stirred for 5 min, and then 300 mL of water and EtOAc (300 mL×3) were added for extraction. The organic phase was dried over anhydrous sodium sulfate and concentrated in vacuum. The crude product was purified by column chromatography (PE:EA=55:45) to obtain a target product (450 mg, yellow solid), with a yield of 20.49%.

MS m/z (ESI): 448.2[M+1].

Step 8: (6R)-6-[(1S,2S)-1-Cyano-2-methyl-cyclopropyl]-2-[2,2-dimethyltetrahydropyran-4-yl]-N-methyl-N-phenyl-thieno[2,3-b]pyrrole-5-carboxamide (450 mg, 1.01 mmol), K2CO3 (764.25 mg, 5.53 mmol) and hydroxylamine hydrochloride (349.32 mg, 5.03 mmol) were dissolved in THF (20 mL), and the mixture was stirred at 90°C for 2 h. It was monitored by LCMS that the intermediate was formed and the raw material disappeared. The reaction solution was concentrated to dryness, anhydrous EtOH (20 mL) was added to dissolve, and then insoluble matter was filtered off. DBU (459.17 mg, 3.02 mmol, 450.17 μL) and CDI (318.40 mg, 2.21 mmol) were added to the filtrate and dissolved in 1 ml THF (20 mL), and the reaction solution was stirred for 3 hours. It was monitored by LCMS that the product was formed and the intermediate disappeared. The reaction solution was directly mixed with silica gel and spin-dried. The crude product was purified by column chromatography (DCM:MeOH=98:2) to obtain a target product (390 mg, yellow solid), with a yield of 76.57%.

MS m/z (ESI): 507.2[M+1].

Step 9: 2-[2,2-dimethyltetrahydropyran-4-yl]-N-methyl-6-[(1S,2S)-2-methyl-1-(5-oxo-4H-1,2,4-oxadiazol-3-yl)cyclopropyl]-N-phenylthieno[2,3-b]pyrrole-5-carboxamide was subjected to chiral separation to obtain the titled product of 2-((S)-2,2-dimethyltetrahydro-2H-pyran-4-yl)-N-methyl-6-((1S,2S)-2-methyl-1-(5-carbonyl-4,5-dihydro-1,2,4-oxadiazol-3-yl)cyclopropyl)-N-phenyl-6H-thieno[2,3-b]pyrrole-5-carboxamide.

MS m/z (ESI): 507.2[M+1].

Step 10: 2-[2,2-dimethyltetrahydropyran-4-yl]-N-methyl-6-[(1S,2S)-2-methyl-1-(5-oxo-4H-1,2,4-oxadiazol-3-yl)cyclopropyl]-N-phenylthieno[2,3-b]pyrrole-5-carboxamide (390 mg, 769.81 μmol) was dissolved in ethylene glycol dimethyl ether (10 mL), KOH (345.53 mg, 6.16 mmol) was added at room temperature, nitrogen was replaced three times, and the reaction solution was stirred at 100 °C for 8 hours. It was monitored by LCMS that the raw material disappeared and the product was generated. H2O (200 mL) and EtOAc (100 mL×3) were added to the reaction solution to extract the organic phase. Hydrochloric acid was added to the aqueous phase so that the pH was adjusted to about 5 to 6. EA (100 mL×3) was added to extract the organic phase, which was dried over anhydrous sodium sulfate, and concentrated in

vacuum. The crude product was purified by reverse phase column (H2O:ACN=55:45) to obtain a target product (168 mg, yellow solid) with a yield of 52.27%.

MS m/z (ESI): 418.1[M+1].

Step 11: A target product was obtained by using (S)-1-(4-fluoro-1-(methyl-d3)-1H-indazol-5-yl)-3-(2-(4-fluoro-3,5-dimethylphenyl)-4-methyl-4,5,6,7-tetrahydro-2H-pyrazolo[4,3-c]pyridin-3-yl)-1,3-dihydro-2H-imidazole-2-one and 2-((S)-2,2-dimethyltetrahydro-2H-pyran-4-yl)-6-((1S,2S)-2-methyl-1-(5-carbonyl-4,5-dihydro-1,2,4-oxadiazol-3-yl)cyclopropyl)-6H-thieno[2,3-b]pyrrole-5-carboxylic acid as raw materials, with reference to the synthesis method of step 4 of Example 25.

MS m/z (ESI): 892.3[M+1].

**Example 285**

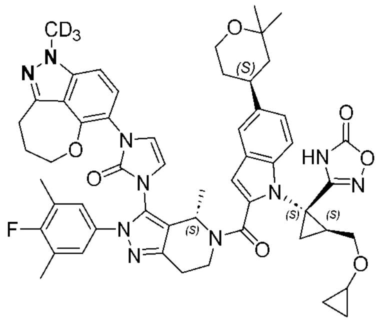

The titled product of 3-((1S,2S)-2-(cyclopropyloxymethyl)-1-(5-((S)-2,2-dimethyltetrahydro-2H-pyran-4-yl)-2-((S)-2-(4-fluoro-3,5-dimethylphenyl)-4-methyl-3-(3-(2-(methyl-d3)-2,7,8,9-tetrahydrooxahepta[4,3,2-cd]indazol-5-yl)-2-carbonyl-2,3-dibydro-1H-imidazol-1-yl)-4,5,6,7-tetrahydro-2H-pyrazolo[4,3-c]pyridine-5-carbonyl)-1H-indol-1-yl) cyclopropyl)-1,2,4-oxadiazol-5(4H)-one was obtained with reference to the synthesis method of Example 282.

MS m/z (ESI): 980.5 [M+1].

$^1$H NMR (400 MHz, DMSO) δ11.59 (s, 1H), 7.81-7.64 (m, 1H), 7.53-7.35 (m, 1H), 7.29 - 7.05 (m, 5H), 6.93-6.59 (m , 3H), 5.69-5.59 (m, 1H), 5.34-5.31(m, 1H), 4.41 (s, 2H),3.93- 3.82 (m,1H), 3.75 -3.41 (m, 3H), 3.11 -2.89 (m, 2H), 2.77 -2.64 (m, 1H), 2.33-2.19 (m, 6H), 2.11-1.90 (m,2H), 1.73-1.43 (m, 6H), 1.30-1.18 (m, 14H), 0.47-0.33 (m, 4H).

**Example 286**

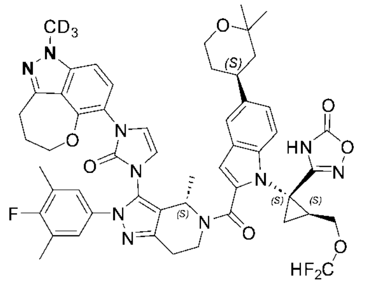

A target product was obtained with reference to the synthesis method of Example 282.

MS m/z (ESI): 990.4[M+1].

**Example 287**

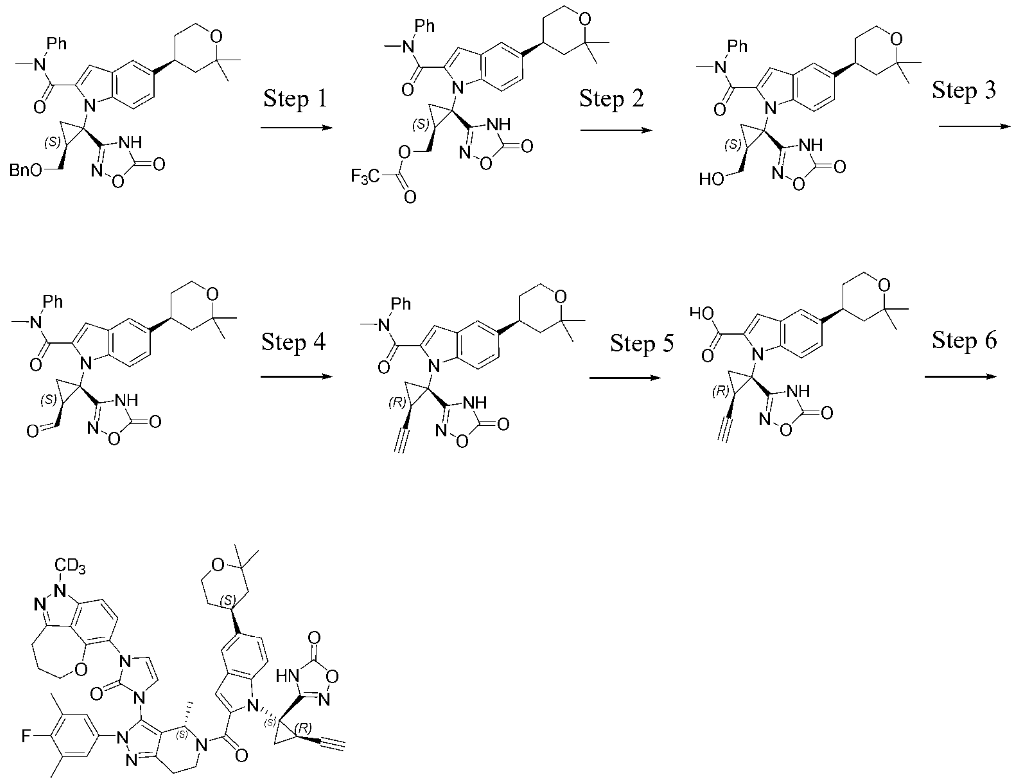


Step 1 : ((1S,2S)-2-(5-((S)-2,2-dimethyltetrahydro-2H-pyran-4-yl)-2-(methyl(phenyl)carbamoyl)-1H-indol-1-yl)-2-(5-oxo-4,5-dihydro-1,2,4-oxadiazol-3-yl)cyclopropyl)methyl 2,2,2-trifluoroacetate

1-((1S,2S)-2-((benzyloxy)methyl)-1-(5-oxo-4,5-dihydro-1,2,4-oxadiazol-3-yl)cyclopropyl)-5-((S)-2,2-dimethyltetrahydro-2H-pyran-4-yl)-N-methyl-N-phenyl-1H-indole-2-carboxamide (2.44 g, 4.02 mmol) was dissolved in trifluoroacetic acid (9 mL), and the reaction solution was heated to 80°C, and stirred for reaction for 3 hours. The reaction solution was cooled to room temperature, and concentrated under reduced pressure. The residue was dissolved in dichloromethane, washed once with a saturated sodium bicarbonate solution, dried over anhydrous sodium sulfate, and filtered. The filtrate was concentrated under reduced pressure to obtain 2.31 g of a yellow oily product (yield: 93.8%).
MS m/z (ESI): 613.2[M+1]

Step 2: 5-((S)-2,2-dimethyltetrahydro-2H-pyran-4-yl)-1-((1S,2S)-2-(hydroxymethyl)-1-(5-oxo-4,5-dihydro-1,2,4-oxadiazol-3-yl)cyclopropyl)-N-methyl-N-phenyl-1H-indole-2-carboxamide

((1S,2S)-2-(5-((S)-2,2-dimethyltetrahydro-2H-pyran-4-yl)-2-(methyl(phenyl)carbamoyl)-1H-indol-1-yl)-2-(5-oxo-4,5-dihydro-1,2,4-oxadiazol-3-yl)cyclopropyl)methyl 2,2,2-trifluoroacetate (2.31 g, 3.77 mmol), and potassium carbonate (1.56 g, 11.31 mmol) were dispersed in MeOH (15 mL), and the mixture was stirred for reaction at room temperature for 1 hour. The reaction solution was filtered through diatomaceous earth, and the filtrate was concentrated under reduced pressure to obtain a yellow oily crude product, which was purified by silica gel column chromatography (eluent: methanol: ethyl acetate=5%, v/v) to obtain 1.81 g of the titled product (yield: 92.9%).
MS m/z (ESI): 517.2[M+1]

Step 3: 5-((S)-2,2-dimethyltetrahydro-2H-pyran-4-yl)-1-((1S,2S)-2-formyl-1-(5-oxo-4,5-dihydro-1,2,4-oxadiazol-3-yl)cyclopropyl)-N-methyl-N-phenyl-1H-indole-2-carboxamide

((S)-2,2-dimethyltetrahydro-2H-pyran-4-yl)-1-((1S,2S)-2-(hydroxymethyl)-1-(5-oxo-4,5-dihydro-1,2,4-oxadiazol-3-yl)cyclopropyl)-N-methyl-N-phenyl-1H-indole-2-carboxamide (1.81 g, 3.50 mmol) and Dess-martin Periodinane (2.97 g, 7.00 mmol) were dissolved in DCM (30 mL). The mixture was stirred for reaction at room temperature for 1 hour. A saturated sodium sulfite solution was added to the reaction solution to quench the reaction, and the reaction solution was extracted with dichloromethane (30 mL×2). The organic phases were combined, dried over anhydrous sodium sulfate, and filtered. The filtrate was concentrated under reduced pressure to obtain a colorless oily crude product. The crude product was purified by silica gel column chromatography (eluent: methanol: ethyl acetate=5%, v/v) to obtain 1.42 g of the

titled product (yield: 78.8%).
MS m/z (ESI): 515.2[M+1]

Step 4: 5-((S)-2,2-dimethyltetrahydro-2H-pyran-4-yl)-1-((1S,2R)-2-ethynyl-1-(5-oxo-4,5-dihydro-1,2,4-oxadiazol-3-yl) cyclopropyl-N-methyl-N-phenyl-1H-indole-2-carboxamide

5-((S)-2,2-dimethyltetrahydro-2H-pyran-4-yl)-1-((1S,2S)-2-formyl-1-(5-oxo-4,5-dihydro-1,2,4-oxadiazol-3-yl) cyclopropyl-N-methyl-N-phenyl-1H-indole-2-carboxamide (1.42 g, 2.76 mmol), potassium carbonate (1.14 g, 8.28 mmol), and dimethyl(1-diazo-2-oxopropyl)phosphonate (1.59 g, 8.28 mmol) were dispersed in methanol (20 mL), and the mixture was stirred for reaction at room temperature for 2 hours. Saturated ammonium chloride was added to the reaction solution to quench, and the reaction solution was extracted with acetate (20 mL×2). The organic phases were combined, dried over anhydrous sodium sulfate, and filtered. The filtrate was concentrated under reduced pressure to obtain a colorless oily crude product. The crude product was purified by silica gel column chromatography (eluent: acetate) to obtain 964 mg of the titled product (yield: 74.2%).
MS m/z (ESI): 511.2[M+1]

Step 5: 5-((S)-2,2-dimethyltetrahydro-2H-pyran-4-yl)-1-((1S,2R)-2-ethynyl-1-(5-oxo-4,5-dihydro-1,2,4-oxadiazol-3-yl)cyclopropyl)-N-methyl-N-phenyl-1H-indole-2-carboxamide (964 mg, 1.89 mmol) and potassium hydroxide (1.06 g, 18.90 mmol) were dissolved in DME (20 mL), and the mixture was stirred for reaction at 100°C for 2 hours. The reaction solution was cooled to room temperature, and diluted hydrochloric acid (1 M) was added to adjust so that pH=1. The reaction solution was extracted with dichloromethane (20 mL×5). The organic phases were combined, dried over anhydrous sodium sulfate, and the concentrated solution under reduced pressure was filtered to obtain a colorless oily crude product. The product was purified by preparative HPLC to obtain 355 mg of the titled product, 5-((S)-2,2-dimethyltetrahydro-2H-pyran-4-yl)-1-((1S,2R)-2-ethynyl-1-(5-oxo-4,5-dihydro-1,2,4-oxadiazol-3-yl)cyclopropane-1H-indole-2-carboxylic acid (yield: 44.6%).
MS m/z (ESI): 422.2[M+1]

Step 6: 3-((1S,2R)-1-(5-((S)-2,2-dimethyltetrahydro-2H-pyran-4-yl)-2-((S)-2-(4-fluoro-3,5-dimethylphenyl)-4-methyl-3-(3-(2-(methyl-d3)-2,7,8,9-tetrahydrooxaheptano[4,3,2-cd]indazol-5-yl)-2-carbonyl-2,3-dihydro-1H-imidazol-1-yl)-4,5,6,7-tetrahydro-2H-pyrazolo[4,3-c]pyridine-5-carbonyl)-1H-indol-1-yl)-2-ethynylcyclopropyl)-1,2,4-oxadiazol-5(4H)-one

The titled product was obtained with reference to the synthesis method of Example 282.

$^1$H NMR (400 MHz, DMSO-d6) δ [12.46 (s) and 11.94 (s), total 1H], 7.54-7.45 (m, 1H), 7.41-7.32 (m, 1H), 7.29-7.06 (m, 5H), 6.96-6.63 (m, 3H), [5.78-5.60 (m) and 5.33-5.16 (m), total 1H], [4.81-4.70 (m) and 4.21-4.14 (m), total 1H] , 4.40-4.32 (m, 2H), 3.72-3.58 (m, 3H), 3.13-2.96 (m, 4H), 2.89-2.67 (m, 2H), 2.33-2.14(m, 8H), 2.12-1.96(m, 2H), 1.67-1.47 (m, 6H), 1.30-1.18(m, 8H).MS m/z (ESI): 934.4[M +1].

**Example 288**

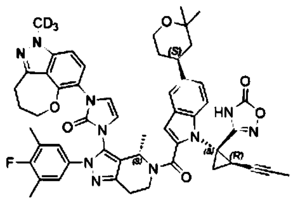

A target product was obtained with reference to the synthesis method of Example 282.

MS m/z (ESI): 948.4 [M+1].

**Example 289**

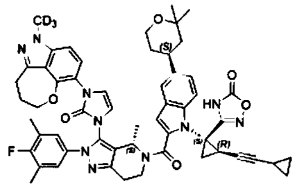

A target product was obtained with reference to the synthesis method of Example 282.

MS m/z (ESI): 974.5[M+1].

**Example 290**

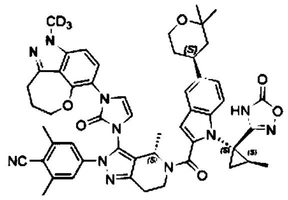

A target product was obtained by using tert-butyl(S)-2-(4-cyano-3,5-dimethylbenzene)-4-methyl-3-(2-carbonyl-2,3-dihydro-1H-imidazol-1-yl)-2,4,6,7-tetrahydro-5H-pyrazolo[4,3-c]pyridine-5-carboxylate as a raw material, with reference to the synthesis method of Example 282.

MS m/z (ESI): 931.4[M+1].

**Example 291**

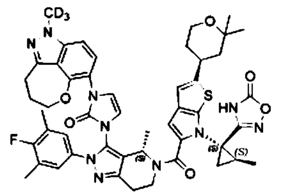

A target product was obtained with reference to the synthesis method of Example 282.

MS m/z (ESI): 930.4[M+1].

**Example 292**

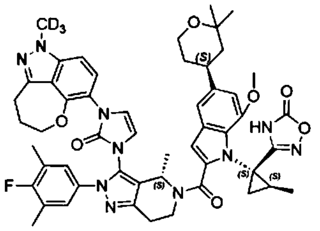

A target product was obtained with reference to the synthesis method of Example 282.

MS m/z (ESI): 954.4 [M+1].

**Example 293**

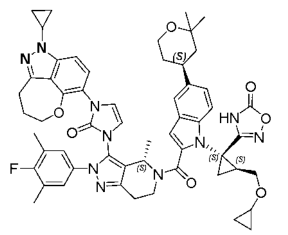

A target compounds was obtained with reference to Example 285.

MS m/z (ESI): 1003.2[M+H]+.

**Example 294**

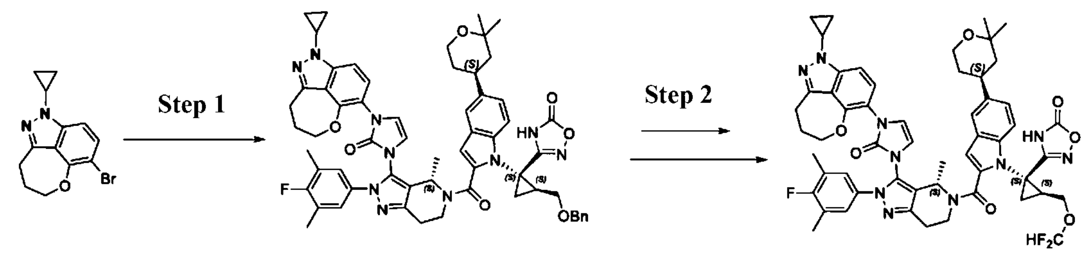


Step 1: A target compound was obtained by using 5-bromo-2-cyclopropyl-2,7,8,9-tetrahydrooxepin[4,3,2-cd] indazole as a raw material, with reference to steps 6, 7 and 8 of Example 282.

MS m/z (ESI): 1053.5[M+H]+.

Step 2: A target compound was obtained by using 3-((1S,2S)-2-((benzyloxy)methyl)-1-(2-((S)-3-(3-(2-cyclopropyl-2,7,8,9-tetrahydrooxahepta[4,3,2-cd]indazol -5-yl)-2-carbonyl-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indol-1-yl)cyclopropyl)-1,2,4-oxadiazol-5(4H)-one as a raw material, with reference to step 1 of Example 188 and step 1 of Example 141.

MS m/z (ESI): 1013.4[M+H]+.

**Example 295**

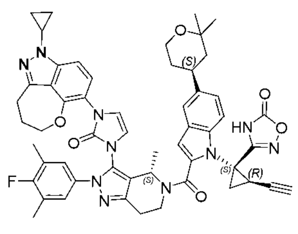

A target compounds was obtained with reference to Example 287.

MS m/z (ESI): 957.4[M+H]+.

**Example 296**

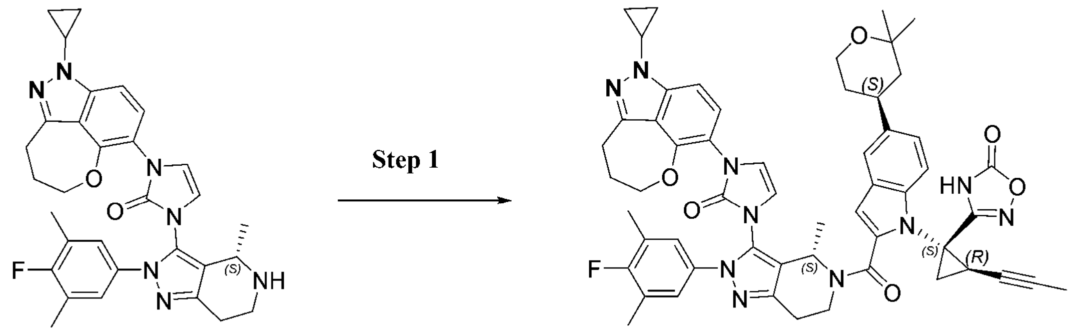


A target compounds was obtained with reference to step 8 of Example 282.

MS m/z (ESI): 971.4[M+H]+.

## Example 297

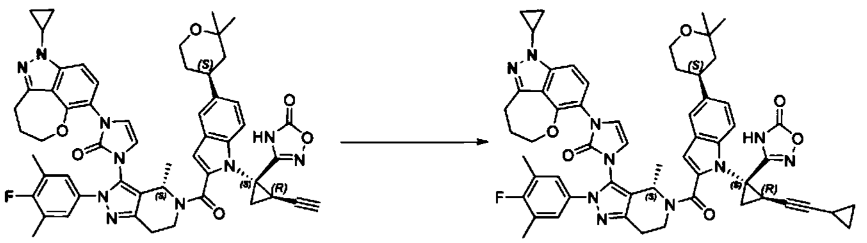

A target compounds was obtained with reference to step 1 of Example 145.

MS m/z (ESI): 997.4[M+H]+.

## Example 298

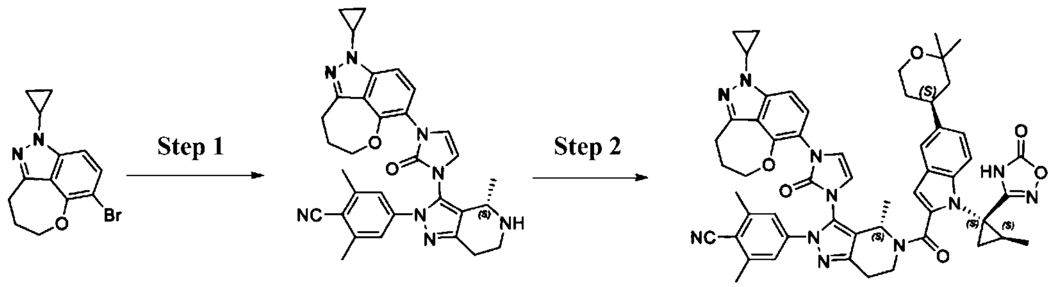


Step 1: A target compound was obtained by using 5-bromo-2-cyclopropyl-2,7,8,9-tetrahydrooxepin[4,3,2-cd]indazole as a raw material, with reference to steps 6, 7 and 8 of Example 282.

MS m/z (ESI): 561.2[M+H]+.

Step 2: A target compounds was obtained with reference to step 8 of Example 282.

MS m/z (ESI): 954.4[M+H]+.

## Example 299

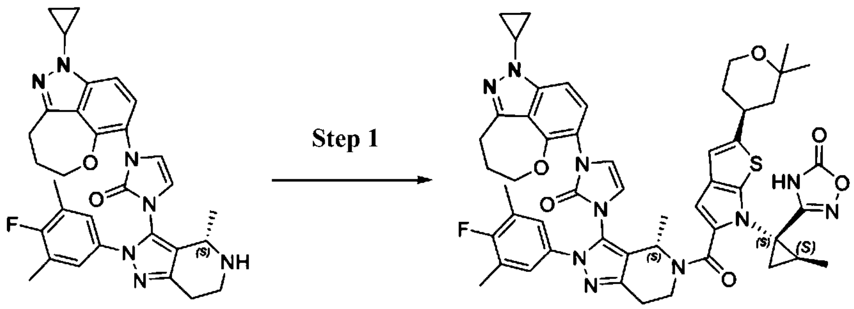


A target compounds was obtained with reference to step 8 of Example 282.

MS m/z (ESI): 953.4[M+H]+.

**Example 300**

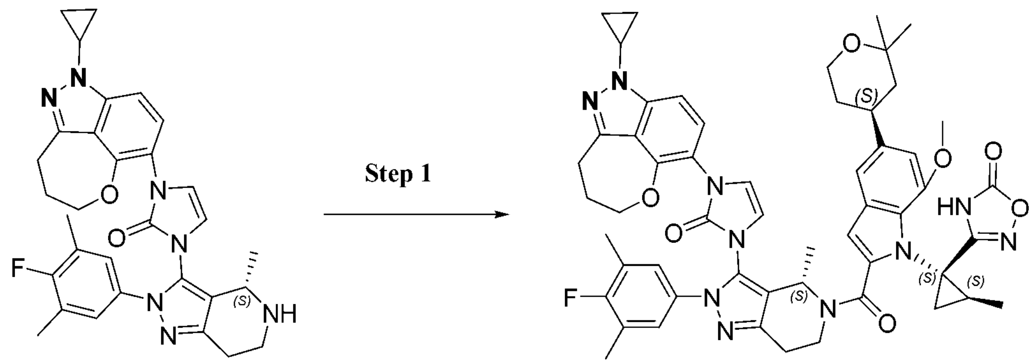


A target compounds was obtained with reference to step 8 of Example 282.

MS m/z (ESI): 977.4[M+H]+.

**Example 301**

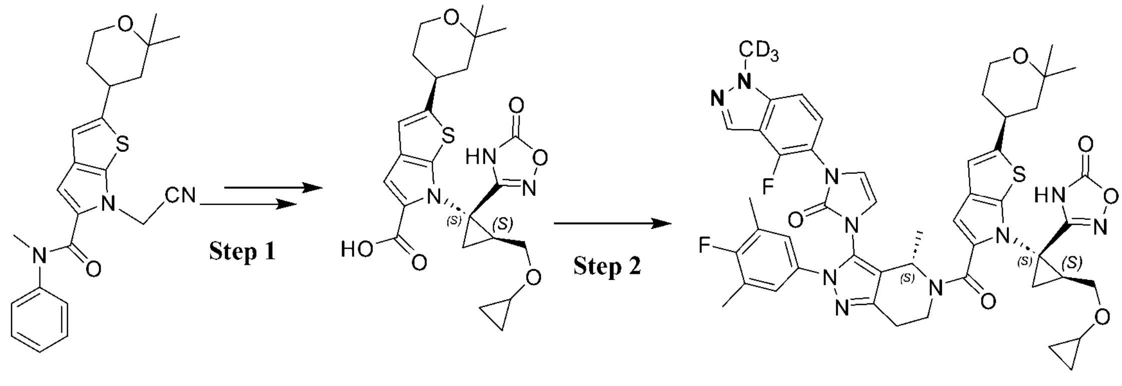


Step 1: The titled product of 6-((1S,2S)-2-(cyclopropyloxymethyl)-1-(5-carbonyl-4,5-dihydro-1,2,4-oxadiazol-3-yl)cyclopropyl)-2-((S)-2,2-dimethyltetrahydro-2H-pyran-4-yl)-6H-thieno[2,3-b]pyrrole-5-carboxylic acid was obtained by using 6-(cyanomethyl)-2-[2,2-dimethyltetrahydropyran-4-yl]-N-methyl-N-phenylthieno[2,3-b]pyrrole-5-carboxamide as a raw material, with reference to the synthesis method of steps 6 to 10 of Example 284, and replacing (R)-4-methyl-1,3,2-dioxathiacyclohexane 2,2-dioxide with (R)-4-(cyclopropyloxymethyl)-1,3,2-dioxathiolane 2,2-dioxide.

MS m/z (ESI): 474.1[M+1].

Step 2: A target product was obtained by using (S)-1-(4-fluoro-1-(methyl-d3)-1H-indazol-5-yl)-3-(2-(4-fluoro-3,5-dimethylphenyl)-4-methyl-4,5,6,7-tetrahydro-2H-pyrazolo[4,3-c]pyridin-3-yl)-1,3-dihydro-2H-imidazole-2-one and 6-((1S,2S)-2-(cyclopropyloxymethyl)-1-(5-carbonyl-4,5-dihydro-1,2,4-oxadiazol-3-yl)cyclopropyl)-2-((S)-2,2-dimethyltetrahydro-2H-pyran-4-yl)-6H-thieno[2,3-b]pyrrole-5-carboxylic acid as raw materials, with reference to the synthesis method of step 4 of Example 25.

MS m/z (ESI): 948.3[M+1].

**Example 302**

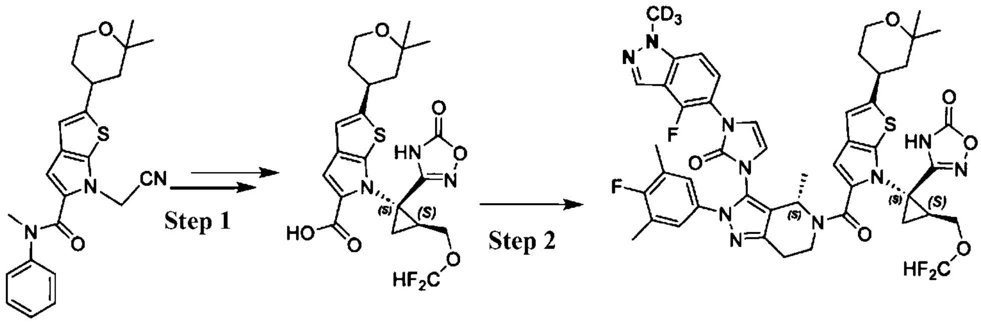


Step 1: A target product was obtained by using 6-(cyanomethyl)-2-[2,2-dimethyltetrahydropyran-4-yl]-N-methyl-N-phenylthieno[2,3-b]pyrrole-5-carboxamide as a raw material, with reference to the synthesis method of steps 6 to 10 of Example 284, and replacing (R)-4-methyl-1,3,2-dioxathiolane 2,2-dioxide with (R)-4-((difluoromethoxy)methyl)-1,3,2-dioxathiolane 2,2-dioxide.

MS m/z (ESI): 484.1[M+1].

Step 2: A target product was obtained by using (S)-1-(4-fluoro-1-(methyl-d3)-1H-indazol-5-yl)-3-(2-(4-fluoro-3,5-dimethylphenyl)-4-methyl-4,5,6,7-tetrahydro-2H-pyrazolo[4,3-c]pyridin-3-yl)-1,3-dihydro-2H-imidazole-2-one and 6-((1S,2S)-2-((difluoromethoxy)methyl)-1-(5-carbonyl-4,5-dihydro-1,2,4-oxadiazol-3-yl)cyclopropyl)-2-((S)-2,2-dimethyltetrahydro-2H-pyran-4-yl)-6H-thieno[2,3-b]pyrrole-5-carboxylic acid as raw materials, with reference to the synthesis method of step 4 of Example 25.

MS m/z (ESI): 958.3[M+1].

**Example 303**

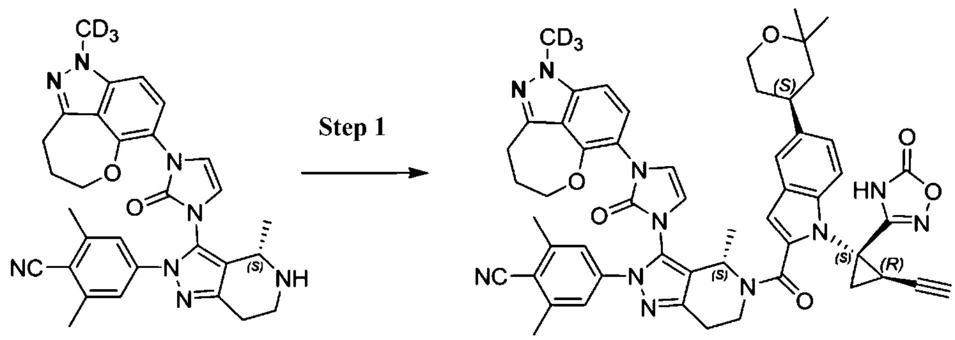


Step 1: A target compound was synthesized by using (S)-2,6-dimethyl-4-(4-methyl-3-(3-(2-(methyl-d3)-2,7,8,9-tetrahydrooxaheptano[4,3,2-cd]indazol-5-yl)-2-carbonyl-2,3-dihydro-1H-imidazol-1-yl)-4,5,6,7-tetrahydro-2H-pyrazolo [4,3-c]pyridin-2-yl)benzonitrile as a raw material, with reference to Example 143.
MS m/z (ESI): 941.4[M+H]

**Example 304**

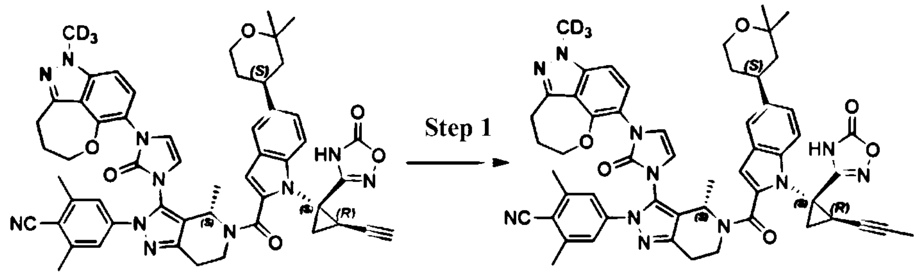


Step 1: A target compound was synthesized by using 4-((S)-5-(5-((S)-2,2-dimethyltetrahydro-2H-pyran-4-yl)-1-((1S,2R)-2-ethynyl-1-(5-carbonyl-4,5-dihydro-1,2,4-oxadiazol-3-yl)cyclopropyl)-1H-indole-2-carbonyl)-4-methyl-3-(3-(2-(methyl-d3)-2,7,8,9-tetrahydrooxahepta[4,3,2-ed]indazol-5-yl)-2-carbonyl-2,3-dihydro-1H-imidazol-1-yl)-4,5,6,7-tetrahydro-2H-pyrazolo[4,3-c]pyridin-2-yl)-2,6-dimethylbenzonitrile as a raw material, with reference to Example 144.
MS m/z (ESI): 955.4[M+H]

**Example 305**

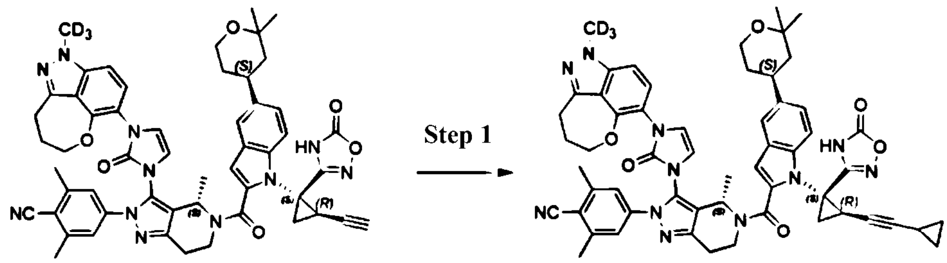


Step 1: A target compound was synthesized by using 4-((S)-5-(5-((S)-2,2-dimethyltetrahydro-2H-pyran-4-yl)-1-((1S,2R)-2-ethynyl-1-(5-carbonyl-4,5-dihydro-1,2,4-oxadiazol-3-yl)cyclopropyl)-1H-indole-2-carbonyl)-4-methyl-3-(3-(2-(methyl-d3)-2,7,8,9-tetrahydrooxahepta[4,3,2-ed]indazol-5-yl)-2-carbonyl-2,3-dihydro-1H-imidazol-1-yl)-4,5,6,7-tetrahydro-2H-pyrazolo[4,3-c]pyridin-2-yl)-2,6-dimethylbenzonitrile as a raw material, with reference to Example 145.
MS m/z (ESI): 981.5[M+H]

**Example 306**

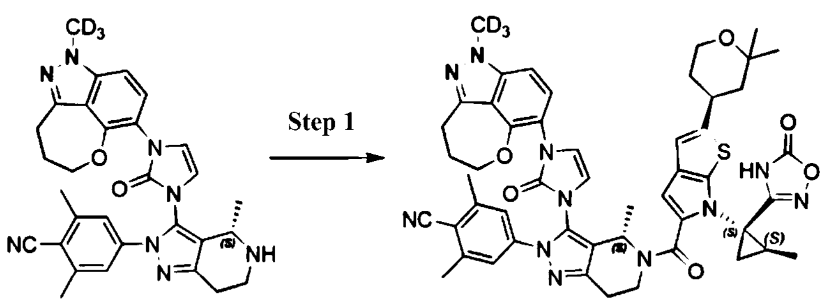


Step 1: A target compound was synthesized by using (S)-2,6-dimethyl-4-(4-methyl-3-(3-(2-(methyl-d3)-2,7,8,9-tetrahydrooxaheptano[4,3,2-cd]indazol-5-yl)-2-carbonyl-2,3-dihydro-1H-imidazol-1-yl)-4,5,6,7-tetrahydro-2H-pyrazolo[4,3-c]pyridin-2-yl)benzonitrile as a raw material, with reference to Example 170.
MS m/z (ESI): 937.4[M+H]

**Example 307**

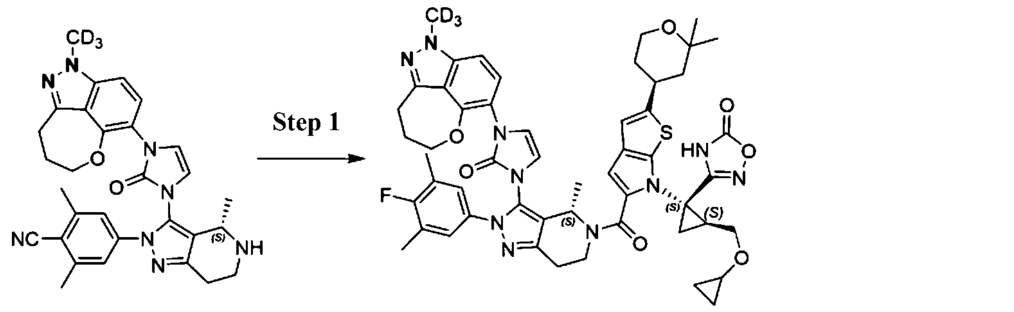


Step 1: A target compound was synthesized by using (S)-2,6-dimethyl-4-(4-methyl-3-(3-(2-(methyl-d3)-2,7,8,9-tetrahydrooxaheptano[4,3,2-cd]indazol-5-yl)-2-carbonyl-2,3-dihydro-1H-imidazol-1-yl)-4,5,6,7-tetrahydro-2H-pyrazolo[4,3-c]pyridin-2-yl)benzonitrile as a raw material, with reference to Example 222.
MS m/z (ESI): 986.4[M+H]

**Example 308**

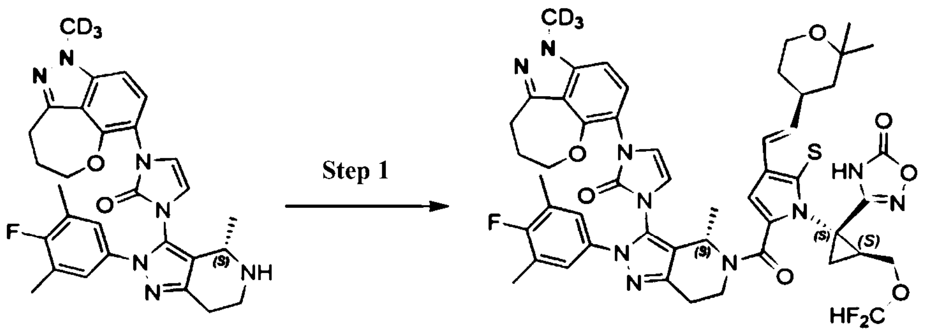


Step 1: A target compound was synthesized by using (S)-1-(2-(4-fluoro-3,5-dimethylphenyl)-4-methyl-4,5,6,7-tetrahydro-2H-pyrazolo[4,3-c]pyridin-3-yl)-3-(2-(methyl-d3)-2,7,8,9-tetrahydrooxaheptano[4,3,2-cd]indazol-5-yl)-1,3-dihydro-2H-imidazole-2-one as a raw material, with reference to Example 226.
MS m/z (ESI): 996.4[M+H]

**Example 309**

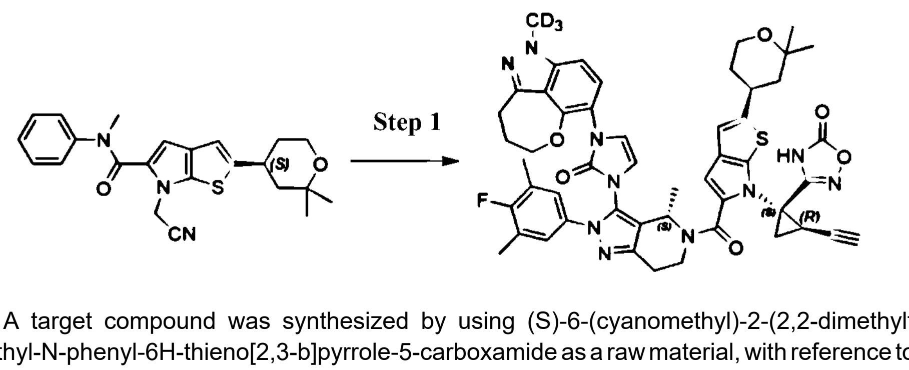


Step 1: A target compound was synthesized by using (S)-6-(cyanomethyl)-2-(2,2-dimethyltetrahydro-2H-pyran-4-yl)-N-methyl-N-phenyl-6H-thieno[2,3-b]pyrrole-5-carboxamide as a raw material, with reference to Example 135.
MS m/z (ESI): 940.4[M+H]

**Example 310**

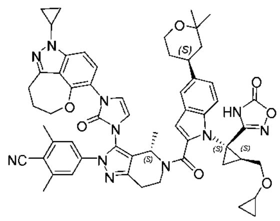

A target product was obtained with reference to the synthesis method of Example 293. MS m/z (ESI): 1010.5[M+1]

**Example 311**

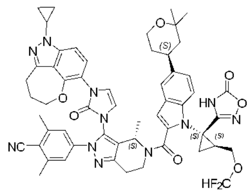

A target product was obtained with reference to the synthesis method of Example 294. MS m/z (ESI): 1020.4[M+1]

**Example 312**

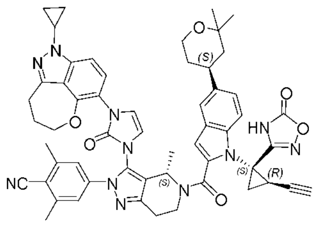

A titled product was obtained with reference to the synthesis method of Example 287. MS m/z (ESI): 964.4[M+1]

**Example 313**

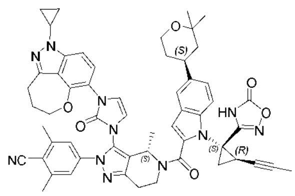

A target product was obtained with reference to the synthesis method of Example 296. MS m/z (ESI): 978.4[M+1]

**Example 314**

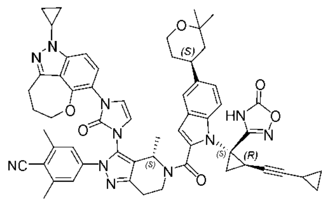

A target product was obtained with reference to the synthesis method of Example 297. MS m/z (ESI): 1004.5[M+1]

**Example 315**

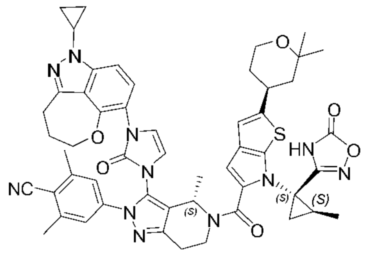

A target product was obtained with reference to the synthesis method of Example 284. MS m/z (ESI): 960.4[M+1]

**Example 316**

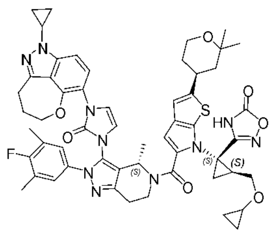

A target product was obtained with reference to the synthesis method of Example 301. MS m/z (ESI): 1009.4[M+1]

**Example 317**

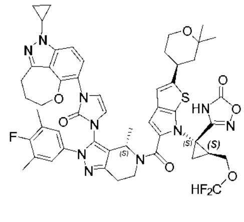

A target product was obtained with reference to the synthesis method of Example 302.
MS m/z (ESI): 1019.4[M+1]

**Example 318**

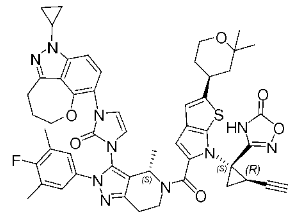

A target product was obtained with reference to the synthesis method of Example 460.
MS m/z (ESI): 963.4[M+1]

**Example 319**

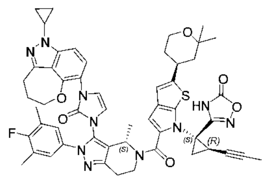

A target product was obtained with reference to the synthesis method of Example 466. MS m/z (ESI): 977.4[M+1]

Example 320

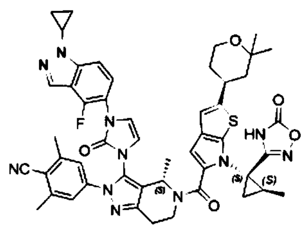

A target product was obtained by using 5-bromo-1-cyclopropyl-4-fluoro-1H-indazole as a raw material, with reference to the synthesis method of Example 282.
MS m/z (ESI): 922.3[M+1].

Example 321

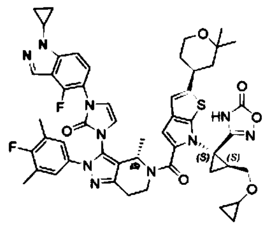

A target product was obtained by using 5-bromo-1-cyclopropyl-4-fluoro-1H-indazole as a raw material, with reference to the synthesis method of Example 67.
MS m/z (ESI): 971.4[M+1].

Example 322

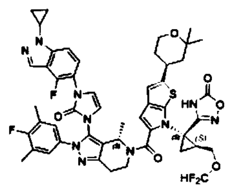

A target product was obtained by using 5-bromo-1-cyclopropyl-4-fluoro-1H-indazole as a raw material, with reference to the synthesis method of Example 286.
MS m/z (ESI): 981.3[M+1].

Example 323

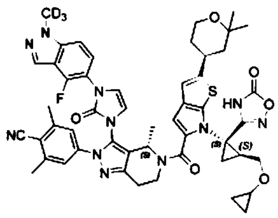

A target product was obtained by using 5-bromo-4-fluoro-1-(methyl-d3)-1H-indazole as a raw material, with reference to the synthesis method of Example 67.
MS m/z (ESI): 955.3[M+1].

Example 324

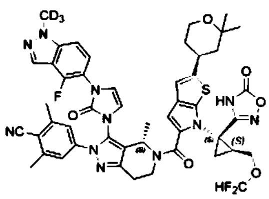

A target product was obtained by using 5-bromo-4-fluoro-1-(methyl-d3)-1H-indazole as a raw material, with reference to the synthesis method of Example 286.
MS m/z (ESI): 965.3[M+1].

Example 325

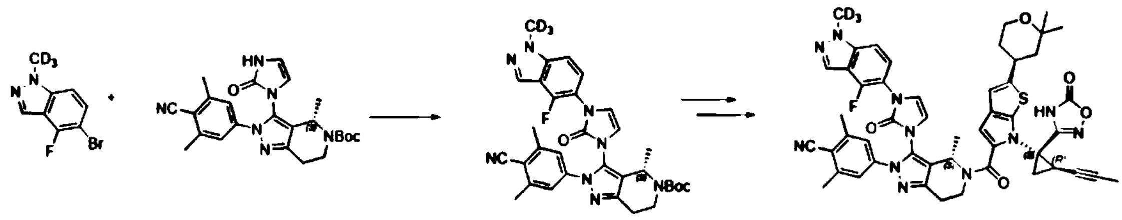

Step 1: A product was obtained by using 5-bromo-4-fluoro-1-(methyl-d3)-1H-indazole and tert-butyl(S)-2-(4-cyano-3,5-dimethylphenyl)-4-methyl-3-(2-carbonyl-2,3-dihydro-1H-imidazol-1-yl)-2,4,6,7-tetrahydro-5H-pyrazolo[4,3-c]pyridine-5-carboxylate as raw materials, with reference to step 2 of Example 25.

MS m/z (ESI): 600.2[M+1].

Step 2: A product was obtained by using tert-butyl(S)-2-(4-cyano-3,5-dimethylphenyl)-3-(3-(4-fluoro-1-(methyl-d3)-1H-indazol-5-yl)-2-carbonyl-2,3-dihydro-1H-imidazol-1-yl)-4-methyl-2,4,6,7-tetrahydro-5H-pyrazolo[4,3-c]pyridine-5-carboxylate and 2-((S)-2,2-dimethyltetrahydro-2H-pyran-4-yl)-6-((1S,2R)-1-(5-carbonyl-4,5-dihydro-1,2,4-oxadiazol-3-yl)-2-(prop-1-yne-1-yl)cyclopropyl)-6H-thieno[2,3-b]pyrrole-5-carboxylic acid as raw materials, with reference to steps 3 and 4 of Example 25.

MS m/z (ESI): 923.3[M+1].

**Example 326**

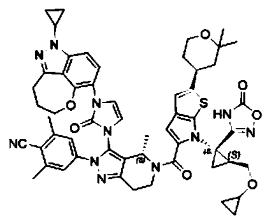

A product was obtained by using tert-butyl(S)-2-(4-cyano-3,5-dimethylphenyl)-3-(3-(2-cyclopropyl-2,7,8,9-tetrahydrooxaheptano[4,3,2-cd]indazol-5-yl)-2-carbonyl-2,3-dihydro-1H-imidazol-1-yl)-4-methyl-2,4,6,7-tetrahydro-5H-pyrazolo[4,3-c]pyridine-5-carboxylate and 6-((1S,2S)-2-(cyclopropyloxymethyl)-1-(5-carbonyl-4,5-dihydro-1,2,4-oxadiazol-3-yl)cyclopropyl)-2-((S)-2,2-dimethyltetrahydro-2H-pyran-4-yl)-6H-thieno[2,3-b]pyrrole-5-carboxylic acid as raw materials, with reference to steps 3 and 4 of Example 25.

MS m/z (ESI): 1016.4[M+1].

**Example 327**

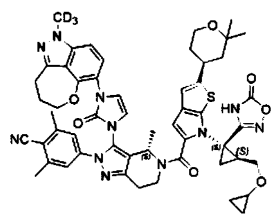

A product was obtained by using tert-butyl(S)-2-(4-cyano-3,5-dimethylphenyl)-4-methyl-3-(3-(2-(methyl-d3)-2,7,8,9-tetrahydrooxaheptano[4,3,2-cd]indazol-5-yl)-2-carbonyl-2,3-dihydro-1H-imidazol-1-yl)-2,4,6,7-tetrahydro-5H-pyrazolo[4,3-c]pyridine-5-carboxylate and 6-((1S,2S)-2-(cyclopropyloxymethyl)-1-(5-carbonyl-4,5-dihydro-1,2,4-oxadiazol-3-yl)cyclopropyl)-2-((S)-2,2-dimethyltetrahydro-2H-pyran-4-yl)-6H-thieno[2,3-b]pyrrole-5-carboxylic acid as raw materials, with reference to steps 3 and 4 of Example 25.

MS m/z (ESI): 993.4[M+1].

**Example 328**

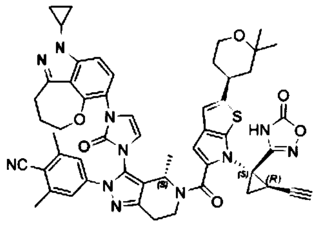

A product was obtained by using tert-butyl(S)-2-(4-cyano-3,5-dimethylphenyl)-3-(3-(2-cyclopropyl-2,7,8,9-tetrahydrooxahepta[4,3,2-cd]indazol-5-yl)-2-carbonyl-2,3-dihydro-1H-imidazol-1-yl)-4-methyl-2,4,6,7-tetrahydro-5H-pyrazolo[4,3-c]pyridine-5-carboxylate and 2-((S)-2,2-dimethyltetrahydro-2H-pyran-4-yl)-6-((1S,2R)-2-ethynyl-1-(5-carbonyl-4,5-dihydro-1,2,4-oxadiazol-3-yl)cyclopropyl)-6H-thieno[2,3-b]pyrrole-5-carboxylic acid as raw materials, with reference to steps 3 and 4 of Example 25.

MS m/z (ESI): 970.3[M+1].

**Example 329**

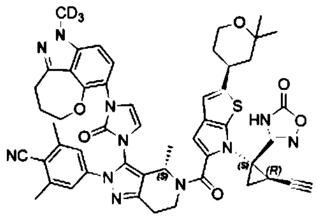

A product was obtained by using tert-butyl(S)-2-(4-cyano-3,5-dimethylphenyl)-4-methyl-3-(3-(2-(methyl-d3)-2,7,8,9-tetrahydrooxaheptano[4,3,2-cd]indazol-5-yl)-2-carbonyl-2,3-dihydro-1H-imidazol-1-yl)-2,4,6,7-tetrahydro-5H-pyrazolo[4,3-c]pyridine-5-carboxylate and 2-((S)-2,2-dimethyltetrahydro-2H-pyran-4-yl)-6-((1S,2R)-2-ethynyl-1-(5-carbonyl-4,5-dihydro-1,2,4-oxadiazol-3-yl)cyclopropyl)-6H-thieno[2,3-b]pyrrole-5-carboxylic acid as raw materials, with reference to steps 3 and 4 of Example 25.

MS m/z (ESI): 947.3[M+1].

**Example 330**

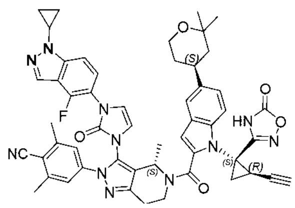

A product was obtained by using tert-butyl(S)-2-(4-cyano-3,5-dimethylphenyl)-3-(3-(1-cyclopropyl-4-fluoro-1H-indazol-5-yl)-2-carbonyl-2,3-dihydro-1H-imidazol-1-yl)-4-methyl-2,4,6,7-tetrahydro-5H-pyrazolo[4,3-c]pyridine-5-carboxylate and 5-((S)-2,2-dimethyltetrahydro-2H-pyran-4-yl)-1-((1S,2R)-2-ethynyl-1-(5-carbonyl-4,5-dihydro-1,2,4-oxadiazol-3-yl)cyclopropyl)-1H-indole-2-carboxylic acid as raw materials, with reference to steps 3 and 4 of Example 25.

MS m/z (ESI): 926.3[M+1].

**Example 331**

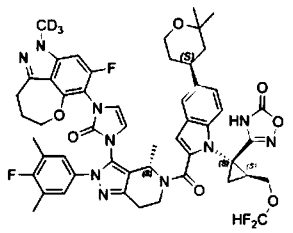

A product was obtained by using 2-bromo-3,5-difluorophenol as a raw material, with reference to the synthesis method of Examples 282.
MS m/z (ESI): 1008.4[M+1].

**Example 332**

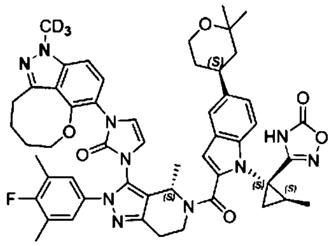

A product was obtained by using 5-bromovalerate ethyl ester as a raw material, with reference to the synthesis method of Examples 282.
MS m/z (ESI): 938.4[M+1].

**Example 333**

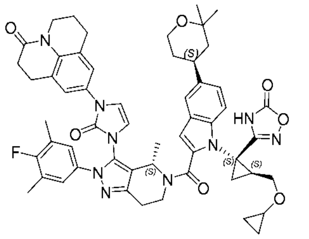

A product was obtained by using tert-butyl(S)-2-(4-fluoro-3,5-dimethylphenyl)-4-methyl-3-(2-carbonyl-3-(3-carbonyl-2,3,6,7-tetrahydro-1H,5H-pyrido[3,2,1-ij]quinolin-9-yl)-2,3-dihydro-1H-imidazol-1-yl)-2,4,6,7-tetrahydro-5H-pyrazolo[4,3-c]pyridine-5-carboxylate and 1-((1S,2S)-2-(cyclopropyloxymethyl)-1-(5-carbonyl-4,5-dihydro-1,2,4-oxadiazol-3-yl)cyclopropyl)-5-((S)-2,2-dimethyltetrahydro-2H-pyran-4-yl)-1H-indole-2-carboxylic acid as raw materials, with reference to steps 3 and 4 of Example 25.
MS m/z (ESI): 976.4[M+1].

**Example 334**

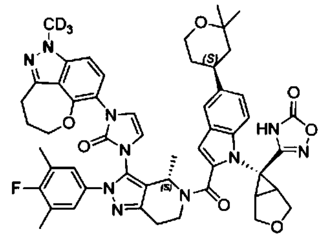

A product was obtained by using tert-butyl(S)-2-(4-fluoro-3,5-dimethylphenyl)-4-methyl-3-(3-(2-(methyl-d3)-2,7,8,9-tetrahydrooxaheptano[4,3,2-cd]indazol-5-yl)-2-carbonyl-2,3-dihydro-1H-imidazol-1-yl)-2,4,6,7-tetrahydro-5H-pyrazolo[4,3-c]pyridine-5-carboxylate and 5-((S)-2,2-dimethyltetrahydro-2H-pyran-4-yl)-1-(6-(5-carbonyl-4,5-dihydro-1,2,4-oxadiazol-3-yl)-3-oxabicyclo [3.1.0]hexan-6-yl)-1H-indole-2-carboxylic acid as raw materials, with reference to steps 3 and 4 of Example 25.

MS m/z (ESI): 952.4[M+1].

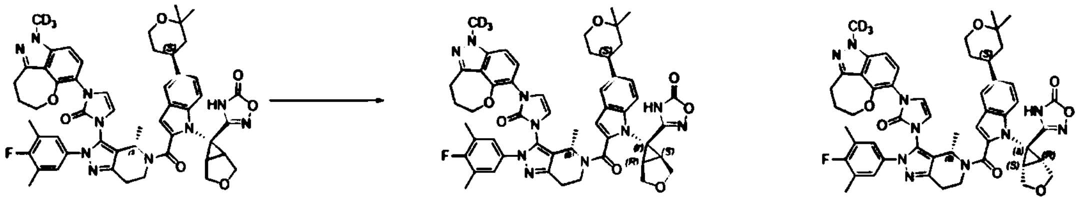

Example 334 was split to obtain 334-1 and 334-2.

MS m/z (ESI): 952.4[M+1].

Example 335

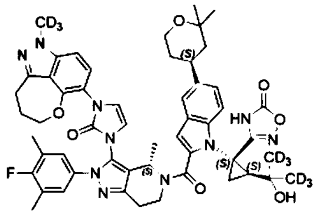

A product was obtained by using tert-butyl(S)-2-(4-fluoro-3,5-dimethylphenyl)-4-methyl-3-(3-(2-(methyl-d3)-2,7,8,9-tetrahydrooxepino[4,3,2-cd]indazol-5-yl)-2-carbonyl-2,3-dihydro-1H-imidazol-1-yl)-2,4,6,7-tetrahydro-5H-pyrazolo[4,3-c]pyridine-5-carboxylate and 5-((S)-2,2-dimethyltetrahydro-2H-pyran-4-yl)-1-((1S,2S)-2-(2-hydroxypropane-2-yl-1,1,1,3,3,3-d6)-1-(5-carbonyl-4,5-dihydro-1,2,4-oxadiazol-3-yl)cyclopropyl)-1H-indole-2-carboxylic acid as raw materials, with reference to steps 3 and 4 of Example 25.

MS m/z (ESI): 974.4[M+1].

**Example 336**

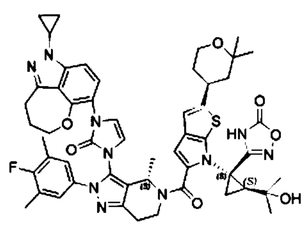

A product was obtained by using tert-butyl(S)-2-(4-fluoro-3,5-dimethylphenyl)-4-methyl-3-(3-(2-(methyl-d3)-2,7,8,9-tetrahydrooxepin[4,3,2-cd]indazol-5-yl)-2-carbonyl-2,3-dihydro-1H-imidazol-1-yl)-2,4,6,7-tetrahydro-5H-pyrazolo[4,3-c]pyridine-5-carboxylate and 2-((S)-2,2-dimethyltetrahydro-2H-pyran-4-yl)-6-((1S,2S)-2-(2-hydroxypropane-2-yl)-1-(5-carbonyl-4,5-dihydro-1,2,4-oxadiazol-3-yl)cyclopropyl)-6H-thieno[2,3-b]pyrrole-5-carboxylic acid as raw materials, with reference to steps 3 and 4 of Example 25.

MS m/z (ESI): 997.4[M+1].

**Example 337**

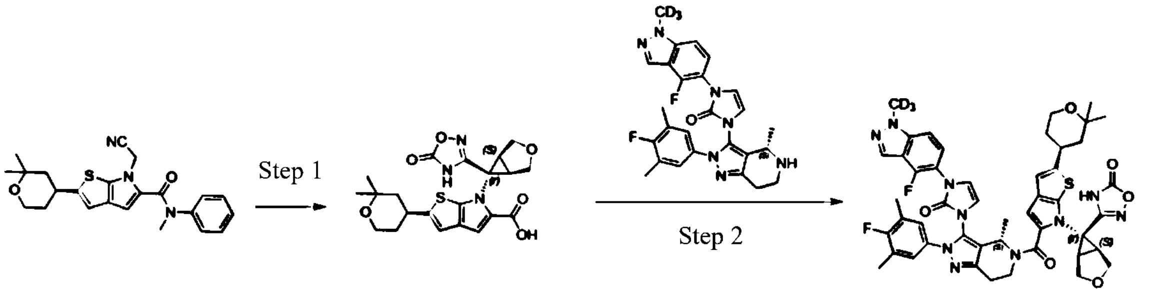


Step 1: A target compound was synthesized by using (3aR,6aS)-tetrahydrofurano[3,4-d][1,3,2]dioxathiazole 2,2-dioxide and (S)-6-(cyanomethyl)-2-(2,2-dimethyltetrahydro-2H-pyran-4-yl)-N-methyl-N-phenyl-6H-thieno[2,3-b]pyrrole-5-carboxamide as a raw material, with reference to steps 3 to 6 of Example 65.

MS m/z (ESI): 446.1[M+1].

Step 2: A target compound was synthesized by using 2-((S)-2,2-dimethyltetrahydro-2H-pyran-4-yl)-6-((1R,5S,6r)-6-(5-carbonyl-4,5-dihydro-1,2,4-oxadiazol-3-yl)-3-oxabicyclo [3.1.0]hexane-6-yl)-6H-thieno[2,3-b]pyrrole-5-carboxylic acid and (S)-1-(4-fluoro-1-(methyl-d3)-1H-indazol-5-yl)-3-(2-(4-fluoro-3,5-dimethylphenyl)-4-methyl-4,5,6,7-tetrahydro-2H-pyrazolo[4,3-c]pyridin-3-yl)-1,3-dihydro-2H-imidazole-2-one as raw materials, with reference to step 7 of Example 65.

MS m/z (ESI): 920.4[M+1].

**Example 338**

3-((3R,4r,5 S)-4-(5-((S)-2,2-dimethyltetrahydro-2H-pyran-4-yl)-2-((S)-3-(3-(4-fluoro-1-(methyl-d3)-1H-indazol-5-yl)-2-carbonyl-2,3-dihydro-1H-imidazol-1-yl)-2-(4-fluoro-3,5-dimethylbenzene)-4-methyl-4,5,6,7-tetrahydro-2H-pyrazolo [4,3-c]pyridine-5-carbonyl)-1H-indol-1-yl)tetrahydro-2H-pyran-4-yl)-1,2,4-oxadiazol-5(4H)-one

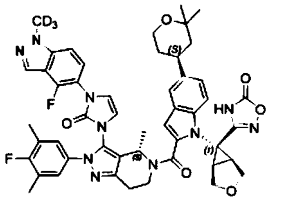

The product was obtained with reference to the synthesis method of Example 65-1.

$^1$H NMR (400 MHz, DMSO) δ [12.25 (s) and 11.59 (s), total 1 H], 8.32 (s, 1H), 7.65 (d, *J*=8.8Hz, 2H), 7.50-7.44 (m, 2H), 7.29 (d, *J*=8.6 Hz, 1H), 7.19 (d, *J*=6.2 Hz, 2H), 7.09 (s, 1H), 6.97-6.87(m, 2H), 5.68 (s, 1H), 4.27-4.07(m, 3H), 3.83-3.63 (m, 5H), 3.13-2.80 (m, 4H), 2.26 (s, 6H), 1.68- 1.42(m, 8H), 1.28(s, 3H), 1.19(s, 3H).

MS m/z (ESI): 914.3 [M+1].

**Example 515**

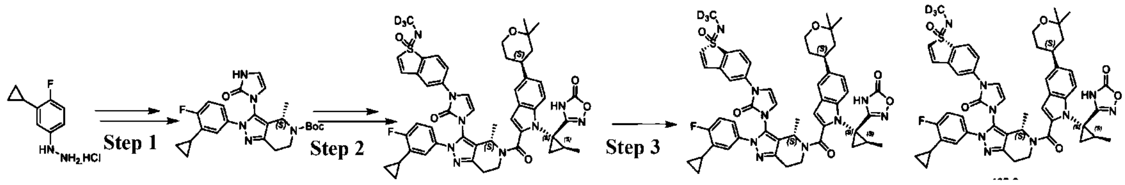


Step 1: A target product was obtained by using (3-cyclopropyl-4-fluorophenyl)hydrazine hydrochloride as a raw material, with reference to the synthesis method of intermediate G.

MS m/z (ESI): 454.2 [M+1].

Step 2: A target product was obtained by using 5-bromo-1-((methyl-d3)imino)-1H-114-benzo[b]thiophene 1-oxide and (S)-2-(3-cyclopropyl-4-fluorophenyl)-4-methyl-3-(2-carbonyl-2,3-dihydro-1H-imidazol-1-yl)-2,4,6,7-tetrahydro-5H-pyrazolo[4,3-c]pyridine-5-carboxylic acid tert-butyl ester as raw materials, with reference to the synthesis method of steps 2 to 4 of Example 175.

MS m/z (ESI): 927.3 [M+1].

Step 3: Chiral separation was performed to obtain 515-1 and 515-2.

MS m/z (ESI): 927.3 [M+1].

**Example 516**

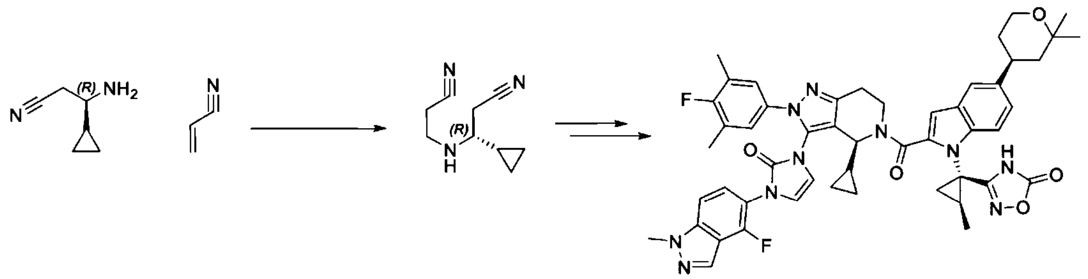

Step 1: In a 250 mL reaction bottle, (R)-3-amino-3-cyclopropylpropionitrile (10.0 g, 90.78 mmol) was dissolved in ethanol (50.0 mL), and triethylamine (11.02 g, 108.93 mmol) and acrylonitrile (5.78 g, 108.93 mmol) were then added. The reaction solution was stirred at 70°C for 3 hours. The reaction was stopped. The reaction solution was cooled to room temperature, and concentrated to obtain the titled product of (R)-3-((2-cyanoethyl)amino)-3-cyclopropylpropionitrile (21.0 g), with a yield of 87.8%.

MS m/z (ESI): 164.1[M+1].

Step 2: A product was obtained by using (R)-3-((2-cyanoethyl)amino)-3-cyclopropylpropionitrile as a raw material, with reference to Example 1.

MS m/z (ESI): 909.3[M+1].

The following examples can be prepared with reference to Example 1.

| Example | Structural formula of compound | MS m/z (ESI) | Example | Structural formula of compound | MS m/z (ESI) |
|---|---|---|---|---|---|
| 339 | 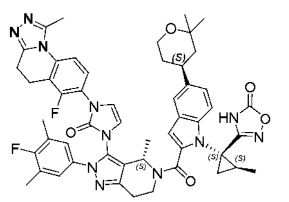 Using 7-bromo-6-fluoro-1-methyl-4,5-dihydro-[1,2,4] triazolo[4,3-a]quinoline as a raw material | 936.4[M+1] | 340 | 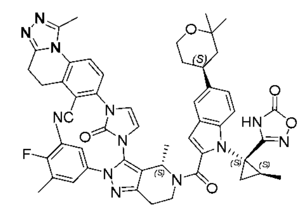 With reference to Example 339 for raw material | 943.4[M+1] |

(continued)

| Example | Structural formula of compound | MS m/z (ESI) | Example | Structural formula of compound | MS m/z (ESI) |
|---|---|---|---|---|---|
| 341 | 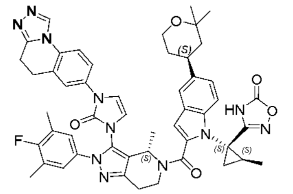 With reference to Example 339 for raw material | 904.4[M+1] | 342 | 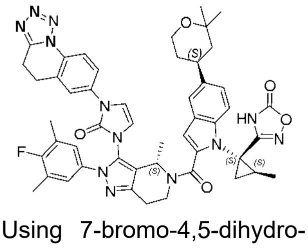 Using 7-bromo-4,5-dihydrotetrazolo[1,5-a]quinoline as a raw material | 905.4[M+1] |
| 343 | 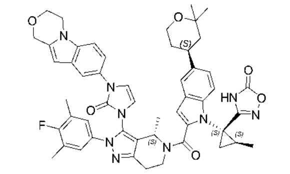 Using 8-bromo-3,4-dihydro-1H-[1,4]oxazonyl[4,3-a]indole as a raw material | 906.4[M+1] | 344 | 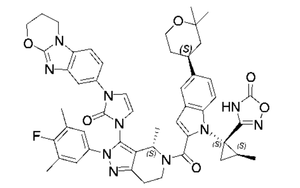 Using 8-bromo-3,4-dihydro-2H-benzo[4,5]imidazo [2,1-b][1,3]oxazine as a raw material | 907.4[M+1] |
| 345 | 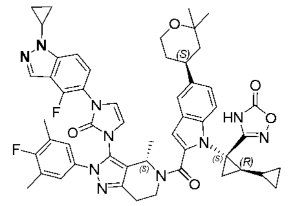 Using 5-bromo-4-fluoro-1-cyclopropyl-lH-indazole and intermediate El as raw materials | 935.4[M+1] | 346 | 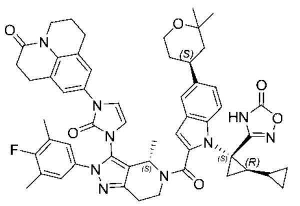 Using 9-bromo-2,3,6,7-tetrahydro-1H,5H-pyrido[3,2,1-ij] quinolin-5-one and intermediate E 1 | 946.4[M+1] |

The following examples can be prepared with reference to Example 25.

| Ex. | Structural formula of compound | MS m/z (ESI) | Ex. | Structural formula of compound | MS m/z (ESI) |
|---|---|---|---|---|---|
| 347 | 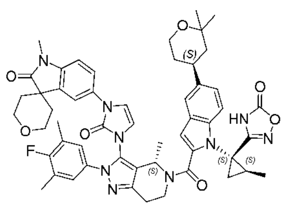 Using 5-bromo-1-methyl-2,3,5,6-tetrahydrospiroindoline-3,4 -pyran-2-one as a raw material | 950.4 [M+1] | 348 | 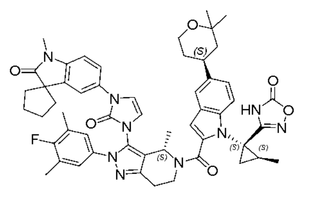 Using 5-bromo-1-methylspirocyclopentane-1,3-indolin-2-one as a raw material | 934.4 [M+1] |

(continued)

| Ex. | Structural formula of compound | MS m/z (ESI) | Ex. | Structural formula of compound | MS m/z (ESI) |
|---|---|---|---|---|---|
| 349 | 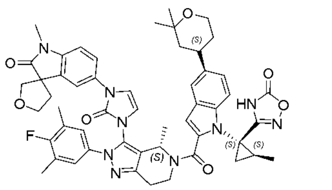 Using 5-bromo-1-methyl-4,5-dihydro-2H-spirofuran-3,3-indolin-2-one as a raw material | 936.4[ M+1] | 351 | 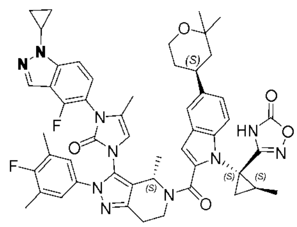 | 923.4[ M+1] |
| 350 | 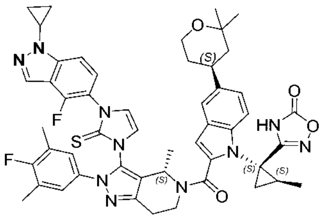 Using 5-bromo-1-cyclopropyl-4-fluoro-1H- indazole and (S)-2-(4-fluoro-3,5-dimethylphenyl)-4-methyl-3-(2-thio-2,3-dihydro-1H-imidazol-1-yl)-2,4,6,7-tetrahydro-5H-pyrazolo[4,3-c]pyridine-5-carboxylic acid tert-butyl ester as raw materials | 925.3 [M+1] | 352 | 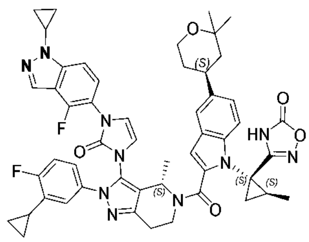 Using 5-bromo-1-cyclopropyl-4-fluoro-1H-indazole and (S)-2-(3-cyclopropyl-4-fluorophenyl)-4-methyl-3-(2-carbonyl-2,3-dihydro-1H-imidazol-1-yl)-2,4,6,7-tetrahydro-5H-pyrazolo[4,3-c]pyridine-5-carboxylic acid tert-butyl ester as raw materials | 921.3 [M+1] |
| 353 | 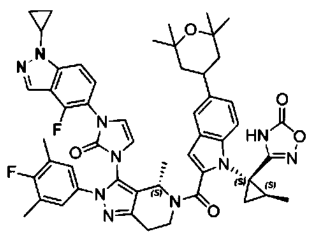 Using 1-((1S,2S)-2-methyl-1-(5-carbonyl-4,5-dihydro-1,2,4-oxadiazol-3-yl)cyclopropyl)-5-(2,2,6,6-tetramethyltetrahydro-2H-pyran-4-yl)-1H-indole-2-carboxylic acid and 175c as raw materials | 937.4[ M+1] | 354 | 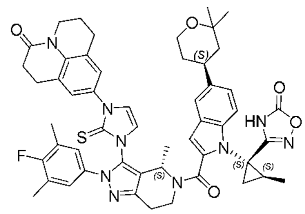 Using 9-bromo-2,3,6,7-tetrahydro-1H,5H-pyrido[3,2,1-ij]quinolin-5-one and (S)-2-(4-fluoro-3,5-dimethylphenyl)-4-methyl-3-(2-thioxo-2,3-dihydro-1H-imidazol-1-yl)-2,4,6,7-tetrahydro-5H-pyrazolo[4,3-c]pyridine-5-carboxylic acid tert-butyl ester as raw materials | 936.4 [M+1] |

(continued)

| Ex. | Structural formula of compound | MS m/z (ESI) | Ex. | Structural formula of compound | MS m/z (ESI) |
|---|---|---|---|---|---|
| 355 | 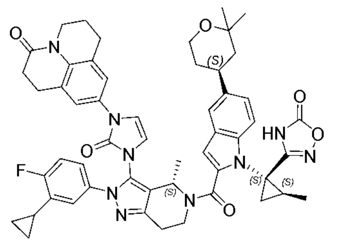 Using 9-Bromo-2,3,6,7-tetrahydro-1H,5H-pyrido[3,2,1-ij]quinolin-5-one and (S)-2-(3-cyclopropyl-4-fluorophenyl)-4-methyl-3-(2-carbonyl-2,3-dihydro-1H-imidazol-1-yl)-2,4,6,7-tetrahydro-5H-pyrazolo[4,3-c]pyridine-5-carboxylic acid tert-butyl ester as raw materials | 932.4 [M+1] | 356 | 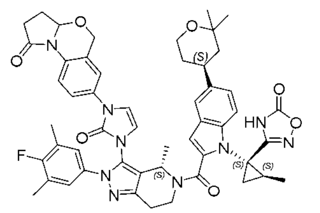 Using 7-Bromo-3,3a-dihydro-5H-benzo[d]pyrrolo[2,1-b][1,3]oxazin-1(2H)-one and (S)-1-(2-(4-fluoro-3,5-dimethylphenyl)-4-methyl-4,5,6,7-tetrahydro-2H-pyrazolo[4,3-c]pyridin-3-yl)-1,3-dihydro-2H-imidazol-2-one as raw materials | |
| 357 | 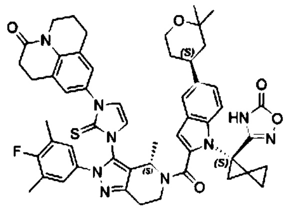 Using (S)-9-(3-(2-(4-fluoro-3,5-dimethylphenyl)-4-methyl-4,5,6,7-tetrahydro-2H-pyrazolo[4,3-c]pyridin-3-yl)-2-sulfoxy-2,3-dihydro-1H-imidazol-1-yl)-2,3,6,7-tetrahydro-1H,5H-pyrido[3,2,1-ij]quinolin-5-one and 5-((S)-2,2-dimethyltetrahydro-2H-pyran-4-yl)-1-((S)-1-(5-carbonyl-4,5-dihydro-1,2,4-oxadiazol-3-yl)spiro[2.2]pentan-1-yl)-1H-indole-2-carboxylic acid as raw materials | 948.4[ M+1] | 358 | 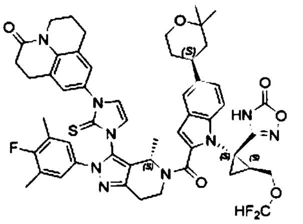 Using (S)-9-(3-(2-(4-fluoro-3,5-dimethylphenyl)-4-methyl-4,5,6,7-tetrahydro-2H-pyrazolo[4,3-c]pyridin-3-yl)-2-sulfoxy-2,3-dihydro-1H-imidazol-1-yl)-2,3,6,7-tetrahydro-1H,5H-pyrido[3,2,1-ij]quinolin-5-one and 1-((1S,2S)-2-((difluoromethoxy)methyl)-1-(5-carbonyl-4,5-dihydro-1,2,4-oxadiazol-3-yl)cyclopropyl)-5 -((S)-2,2-dimethyltetrahydro-2H-pyran-4-yl)-1H-indole-2-carboxylic acid as raw materials | 1002.3 [M+1] |

(continued)

| Ex. | Structural formula of compound | MS m/z (ESI) | Ex. | Structural formula of compound | MS m/z (ESI) |
|---|---|---|---|---|---|
| 359 | 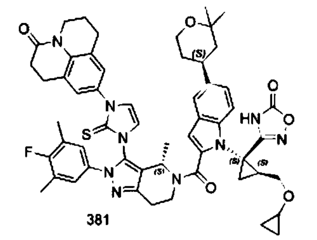 381 Using (S)-9-(3-(2-(4-fluoro-3,5-dimethylphenyl)-4-methyl-4,5,6,7-tetrahydro-2H-pyrazolo[4,3-c]pyridin-3-yl)-2-sulfoxy-2,3-dihydro-1H-imidazol-1-yl)-2,3,6,7-tetrahydro-1H,5H-pyrido[3,2,1-ij]quinolin-5-one and 1-((1S,2S)-2-(cyclopropyloxymethyl)-1-(5-carbonyl-4,5-dihydro-1,2,4-oxadiazol-3-yl)cyclopropyl)-5-((S)-2,2-dimethyltetrahydro-2H-pyran-4-yl)-1H-indole-2-carboxvlic acid as raw materials | 992.4 [M+1] | 360 | 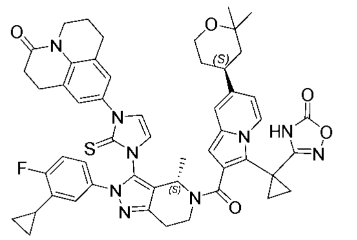 Using (S)-9-(3-(2-(3-cyclopropyl-4-fluorophenyl)-4-methyl-4,5,6,7-tetrahydro-2H-pyrazolo[4,3-c]pyridin-3-yl)-2-thioxo-2,3-dihydro-1H-imidazol-1-yl)-2,3,6,7-tetrahydro-1H,5H-pyrido[3,2,1-ij]quinolin-5-one and (S)-7-(2,2-dimethyltetrahydro-2H-pyran-4-yl)-3-(1-(5-carbonyl-4,5-dihydro-1,2,4-oxadiazol-3-yl)cyclopropyl)indolizine-2-carboxylic acid as raw materials | 934.3 [M+1] |
| 361 | 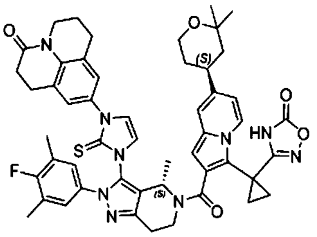 Using (S)-9-(3-(2-(4-fluoro-3,5-dimethylphenyl)-4-methyl-4,5,6,7-tetrahydro-2H-pyrazolo[4,3-c]pyridin-3-yl)-2-sulfoxy-2,3-dihydro-1H-imidazol-1-yl)- 2,3,6,7-tetrahydro-1H,SH-pyrido[3,2,1-ij]quinolin-5-one and (S)-7-(2,2-dimethyltetrahydro-2H-pyran-4-yl)-3-(1-(5-carbonyl-4,5-dihydro-1,2,4-oxadiazol-3-yl)cyclopropyl)indolizine-2-carboxylic acid as raw materials | 922.3 [M+1] | 363 | 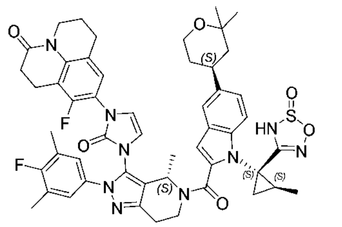 Using 5-((S)-2,2-dimethyltetrahydro-2H-pyran-4-yl)-1-((1S,2S)-2-methyl-1-(2-hydroxy-3H-1,2,3,5-oxathiadiazol-4-yl)cyclopropyl)- 1H-indole-2-carboxylic acid and (5)-8-fluoro-9-3-(2-(4-fluoro-3,5-dimethylphenyl)-4-methyl-4,5,6,7-tetrahydro-2H-pyrazolo[4,3-c]pyridin-3-yl)-2-carbonyl-2,3-dihydro-1H-imidazol-1-yl)-2,3,6,7-tetrahydro-1H,5H-pyrido[3,2,1-ij]quinolin-5-one as raw materials | 958.3[ M+1] |
| 362 | 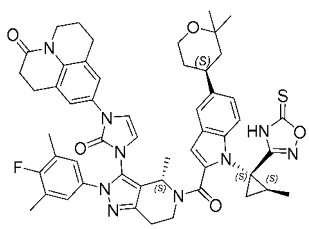 Using 9-bromo-2,3,6,7-tetrahydro-1H,5H-pyrido[3,2,1-ij]quinolin-5-one as raw material | 936.4[ M+H] + | 364 | 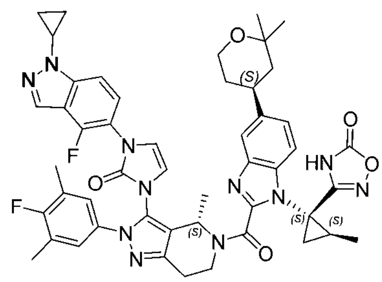 Using methyl 5-bromo-1H-benzo[d]imidazole-2-carboxylate as a raw material, with reference to Example intermediate F | 910.4[ M+H] + |

(continued)

| Ex. | Structural formula of compound | MS m/z (ESI) | Ex. | Structural formula of compound | MS m/z (ESI) |
|---|---|---|---|---|---|
| 365 | Using 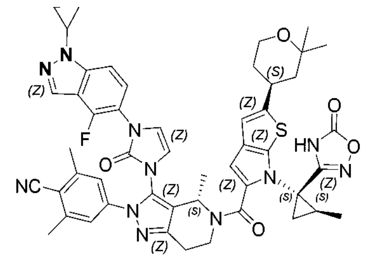 Using intermediate G as a raw material | 922.4[ M+1] | 366 | 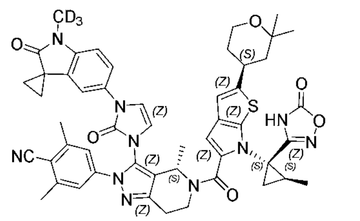 Using intermediate G as a raw material | 922.4[ M+1] |
| 367 | 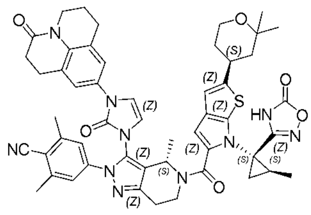 Using intermediate G as a raw material | 933.4[ M+1] | 368 | 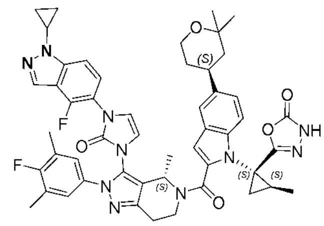 | 909.4[ M+1] |
| 369 | 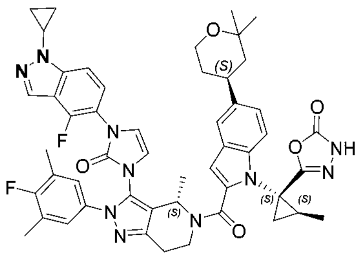 | 909.4[ M+1] | 370 | 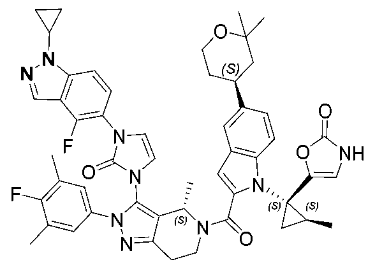 | 908.4[ M+1] |
| 371 | 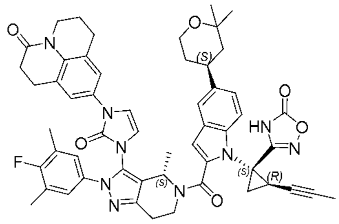 Using tert-butyl(S)-2-(4-fluoro-3,5-dimethylphenyl)-4-methyl-3-(2-carbonyl-3-(3-carbonyl-2,3,6,7-tetrahydro-1H,5H-pyrido[3,2,1-ij]quinolin-9-yl)-2,3-dihydro-1H-imidazol-1-yl)-2,4,6,7-tetrahydro-5H-pyrazolo[4,3-c]pyridine-5-carboxylate and 5-((S)-2,2-dimethyltetrahydro-2H-pyran-4-yl)-1-((1S,2R)-1-(5-carbonyl-4,5-dihydro-1,2,4-oxadiazol-3-yl)-2-(prop-1-yne-1-yl)cyclopropyl)-1H-indole-2-carboxylic acid as raw materials | 944.4[ M+1] | 375 | 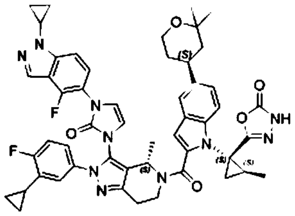 Using 5-bromo-1-cyclopropyl-4-fluoro-1H-indazole , tert-butyl(S)-2-(3-cyclopropyl-4-fluorophenyl)-4-methyl-3-(2-carbonyl-2,3-dihydro-1H-imidazol-1-yl)-2,4,6,7-tetrahydro-5H-pyrazolo[4,3-c]pyridine-5-carboxylate and 5-((S)-2,2-dimethyltetrahydro-2H-pyran-4-yl)-1-((1S,2S)-2-methyl-1-(5-carbonyl-4,5-dihydro-1,3,4-oxadiazol-2-yl)cyclopropyl)-1H-indole-2-carboxylic acid as raw materials | 921.4[ M+1] |

(continued)

| Ex. | Structural formula of compound | MS m/z (ESI) | Ex. | Structural formula of compound | MS m/z (ESI) |
|---|---|---|---|---|---|
| 373 | 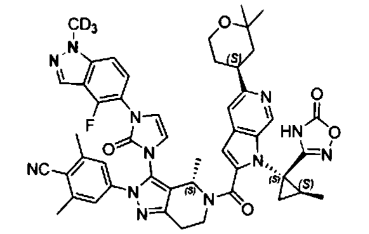 Using 5-bromo-4-fluoro-1-(methyl-d3)-1H-indazole, intermediate G and intermediate F as raw materials | 894.4[ M+1] | 374 | 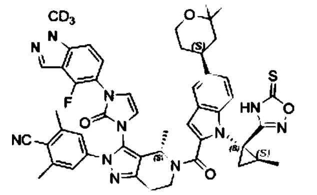 Using 5-((S)-2,2-dimethyltetrahydro-2H-pyran-4-yl)-1-((1S,2S)-2-methyl-1-(5-thio-4,5-dihydro-1,2,4-oxadiazol-3-yl)cyclopropyl)-1H-indole-2-carboxylic acid as a raw material, with reference to Example 373 | 909.4[ M+1] |
| 372 | 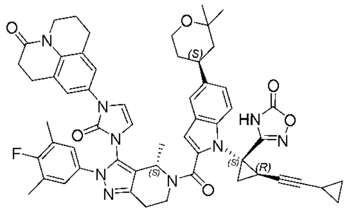 Using 1-((1S,2R)-2-(cyclopropylethynyl)-1-(5-carbonyl-4,5-dihydro-1,2,4-oxadiazol-3-yl)cyclopropyl)-5-((S)-2,2-dimethyltetrahydro-2H-pyran-4-yl)-1H-indole-2-carboxylic acid as a raw material | 970.4[ M+1] | 376 | 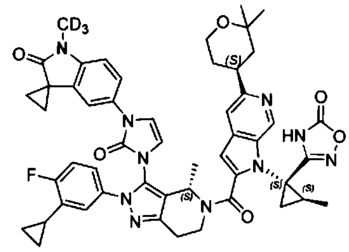 using intermediate F as a raw material, with reference to the synthesis method of Example 375 material | 922.4[ M+1] |
| 377 | 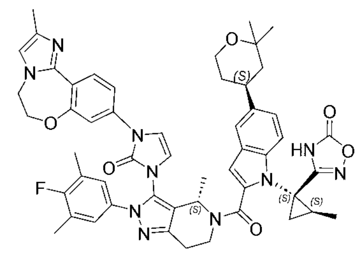 Using 9-bromo-2-methyl-5,6-dihydrobenzo[f]imidazo[1,2-d][1,4]oxazepine as a raw material | 933.4[ M+1] | 378 | | 965.4[ M+1] |

(continued)

| Ex. | Structural formula of compound | MS m/z (ESI) | Ex. | Structural formula of compound | MS m/z (ESI) |
|---|---|---|---|---|---|
| 379 | 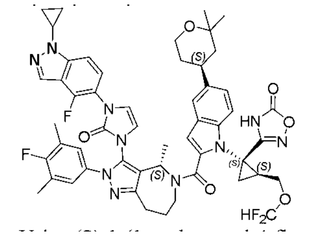 Using (S)-1-(1-cyclopropyl-4-fluoro-1H-indazol-5-yl)-3-(2-(4-fluoro-3,5-dimethylphenyl)-4-methyl-2,4,5,6,7,8-hexahydropyrazolo [4,3-c] azepin-3-yl)-1,3-dihydro-2H-imidazol-2-one and 1-((1S,2S)-2-((difluoromethoxy) methyl)-1-(5-carbonyl-4,5-dihydro-1,2,4-oxadiazol-3-yl)cyclopropyl)-5-((S)-2,2-dimethyltetrahydro-2H-pyran-4-yl)-1H-indole-2-carboxylic acid as raw materials | 989.4[ M+1] | 380 | 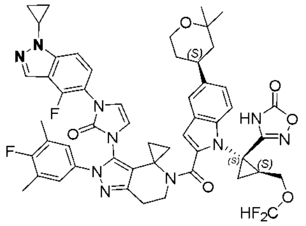 Using 1-(1-cyclopropyl-4-fluoro-1H-indazol-5-yl)-3-(2'-(4-fluoro-3,5-dimethylphenyl)-2',5',6',7'-tetrahydrospiro [cyclopropane-1,4'-pyrazolo[4,3-c]pyridine]-3'-yl)-1,3-dihydro-2H-imidazol-2-one and 1-((1S,2S)-2-((difluoromethoxy) methyl)-1-(5-carbonyl-4,5-dihydro-1,2,4-oxadiazol-3-yl)cyclopropyl)-5-((S)-2,2-dimethyltetrahydro-2H-pyran-4-yl)-1H-indole-2-carboxylic acid as raw materials | 987.4[ M+1] |
| 381 | 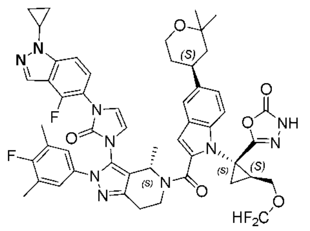 Using (S)-1-(1-cyclopropyl-4-fluoro-1H-indazol-5-yl)-3-(2-(4-fluoro-3,5-dimethylphenyl)-4-methyl-4,5,6,7-tetrahydro-2H-pyrazolo[4,3-c]pyridin-3-yl)-1,3-dihydro-2H-imidazol-2-one and 1-((1S,2S)-2-((difluoromethoxy) methyl)-1-(5-carbonyl-4,5-dihydro-1,3,4-oxadiazol-2-yl)cyclopropyl)-5-((S)-2,2-dimethyltetrahydro-2H-pyran-4-yl)-1H-indole-2-carboxylic acid as raw materials | 975.4[ M+1] | 382 | 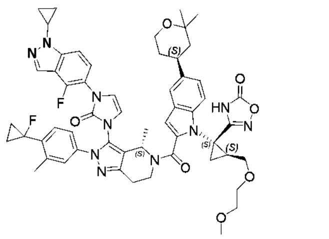 Using (S)-1-(1-cyclopropyl-4-fluoro-1H-indazol-5-yl)-3-(2-(4-(1-fluorocyclopropyl)-3-methylphenyl)-4-methyl-4,5,6,7-tetrahydro-2H-pyrazolo[4,3-c]pyridin-3-yl)-1,3-dihydro-2H-imidazol-2-one and 5-((S)-2,2-dimethyltetrahydro-2H-pyran-4-yl)-1-((1S,2S)-2-((2-methoxyethoxy)methyl)-1-(5-carbonyl-4,5-dihydro-1,2,4-oxadiazol-3-yl)cyclopropyl)-1H-indole-2-carboxylic acid as raw materials | 1009.4 [M+H] + |

(continued)

| Ex. | Structural formula of compound | MS m/z (ESI) | Ex. | Structural formula of compound | MS m/z (ESI) |
|---|---|---|---|---|---|
| 383 | 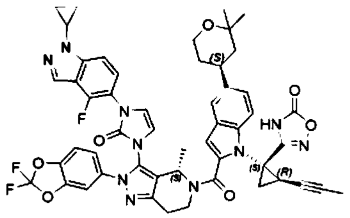 Using 5-((S)-2,2-dimethyltetrahydro-2H-pyran-4-yl)-1-((1S,2R)-1-(5-carbonyl-4,5-dihydro-1,2,4-oxadiazol-3-yl)-2-(prop-1-yne-1-yl)cyclopropyl)-1H-indole-2-carboxylic acid and (S)-1-(1-cyclopropyl-4-fluoro-1H-indazol-5-yl)-3-(2-(2,2-difluorobenzo [d][1,3] dioxazol-5-yl)-4-methyl-4,5,6,7-tetrahydro-2H- | 967.3[ M+H] + | 384 | 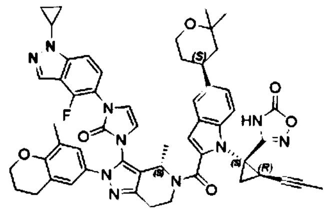 Using 5-((S)-2,2-dimethyltetrahydro-2H-pyran-4-yl)-1-((1S,2R)-1-(5-carbonyl-4,5-dihydro-1,2,4-oxadiazol-3-yl)-2-(prop-1-yne-1-yl)cyclopropyl)-1H-indole-2-carboxylic acid and 1-(1-cyclopropyl-4-fluoro-1H-indazol-5-yl)-3-(2-(8-methylchroman-6-yl)-4,5,6,7-tetrahydro-2H-pyrazolo[4,3-c]pyridin-3-yl)-1,3-dihydro-2H-imidazol-2-one as raw materials | 957.4[ M+H] + |
| | pyrazolo[4,3-c]pyridin-3-yl)-1,3-dihydro-2H-imidazol-2-one as raw materials | | | | |
| 385 | 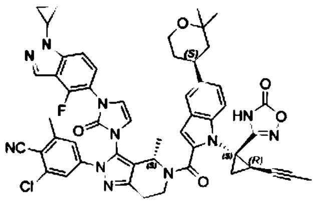 Using 5-((S)-2,2-dimethyltetrahydro-2H-pyran-4-yl)-1-((1S,2R)-1-(5-carbonyl-4,5-dihydro-1,2,4-oxadiazol-3-yl)-2-(prop-1-yne-1-yl)cyclopropyl)-1H-indole-2-carboxylic acid and (S)-2-chloro-4-(3-(3-(1-cyclopropyl-4-fluoro-1H-indazol-5-yl)-2-carbonyl-2,3-dihydro-1H-imidazol-1-yl)-4-methyl-4,5,6,7-tetrahydro-2H-pyrazolo[4,3-c]pyridin-2-yl)-6-methvlbenzonitrile as raw materials | 960.3[ M+H] + | 386 | 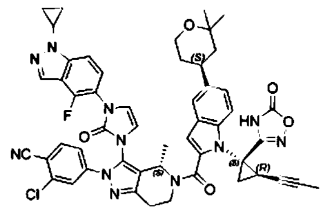 Using 5-((S)-2,2-dimethyltetrahydro-2H-pyran-4-yl)-1-((1S,2R)-1-(5-carbonyl-4,5-dihydro-1,2,4-oxadiazol-3-yl)-2-(prop-1-yne-1-yl)cyclopropyl)-1H-indole-2-carboxylic acid and (S)-2-chloro-4-(3-(3-(1-cyclopropyl-4-fluoro-1H-indazol-5-yl)-2-carbonyl-2,3-dihydro-1H-imidazol-1-yl)-4-methyl-4,5,6,7-tetrahydro-2H-pyrazolo[4,3-c]pyridin-2-yl)benzonitrile as raw materials | 946.3[ M+H] + |

(continued)

| Ex. | Structural formula of compound | MS m/z (ESI) | Ex. | Structural formula of compound | MS m/z (ESI) |
|---|---|---|---|---|---|
| 387 | 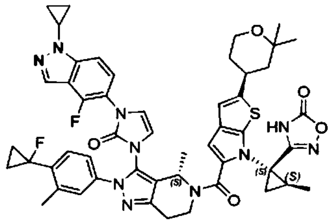<br>Using 2-((S)-2,2-dimethyltetrahydro-2H-pyran-4-yl)-6-((1S,2S)-2-methyl-1-(5-carbonyl-4,5-dihydro-1,2,4-oxadiazol-3-yl)cyclopropyl)-6H-thieno[2,3-b]pyrrole-5-carboxylic acid and (S)-1-(1-cyclopropyl-4-fluoro-1H-indazol-5-yl)-3-(2-(4-(1-fluorocyclopropyl)-3-methylphenyl)-4-methyl-4,5,6,7-tetrahydro-2H-pyrazolo[4,3-c]pyridin-3-yl)-1,3-dihydro-2H-imidazol-2-one as raw materials | 941.3[ M+H] + | 388 | 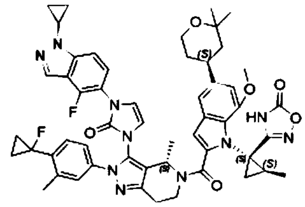<br>Using 5-((S)-2,2-dimethyltetrahydro-2H-pyran-4-yl)-7-methoxy-1-((1S,2S)-2-methyl-1-(5-carbonyl-4,5-dihydro-1,2,4-oxadiazol-3-yl)cyclopropyl)-1H-indole-2-carboxylic acid and (S)-1-(1-cyclopropyl-4-fluoro-1H-indazol-5-yl)-3-(2-(4-(1-fluorocyclopropyl)-3-methylphenyl)-4-methyl-4,5,6,7-tetrahydro-2H-pyrazolo[4,3-c]pyridin-3-yl)-1,3-dihydro-2H-imidazol-2-one as raw materials | 965.4[ M+H] + |
| 389 | 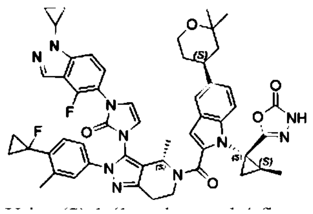<br>Using (S)-1-(1-cyclopropyl-4-fluoro-1H-indazol-5-yl)-3-(2-(4-(1-fluorocyclopropyl)-3-methylphenyl)-4-methyl-4,5,6,7-tetrahydro-2H-pyrazolo[4,3-c]pyridin-3-yl)-1,3-dihydro-2H-imidazol-2-one and 5-((S)-2,2-dimethyltetrahydro-2H-pyran-4-yl)-1-((1S,2S)-2-methyl-1-(5-carbonyl-4,5-dihydro-1,3,4-oxadiazol-2-yl)cyclopropyl)-1H-indole-2-carboxylic acid as raw materials | 935.4[ M+H] + | 395 | 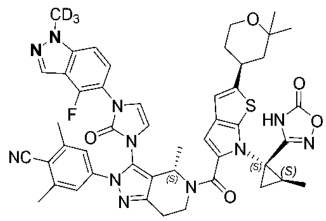<br>Using (S)-4-(3-(3-(4-fluoro-1-(methyl-d3)-1H-indazol-5-yl)-2-carbonyl-2,3-dihydro-1H-imidazol-1-yl)- 4-methyl-4,5,6,7-tetrahydro-2H-pyrazolo[4,3-c]pyridin-2-yl)-2,6-dimethylbenzonitrile and 2-((S)-2,2-dimethyltetrahydro-2H-pyran-4-yl)-6-((1S,2S)-2-methyl-1-(5-carbonyl-4,5-dihydro-1,2,4-oxadiazol-3-yl)cyclopropyl)-6H-thieno[2,3-b]pyrrole-5-carboxylic acid as raw materials | 899.3[ M+1] |
| 390 | 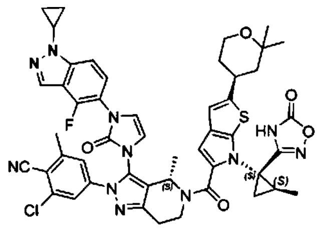 | 942.3[ M+1] | 391 | 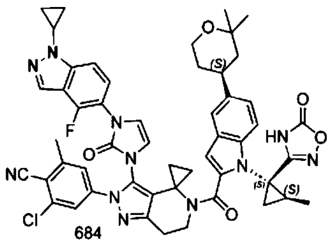 | 948.3[ M+1] |

(continued)

| Ex. | Structural formula of compound | MS m/z (ESI) | Ex. | Structural formula of compound | MS m/z (ESI) |
|---|---|---|---|---|---|
| 394 | 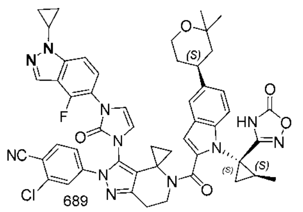 689 Using 8-cyano-7-carbonyl-4-azaspiro [2.5]octane-4-carboxylic acid tert-butyl ester and 4-bromo-2-chlorobenzonitrile as raw materials, with reference to intermediate G and Example 25 | 934.3[ M+1] | 393 | 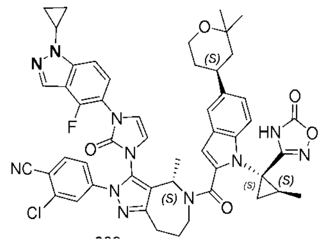 Using (2S)-3-cyano-2-methyl-4-carbonylazepane-1-carboxylic acid tert-butyl ester and 4-bromo-2-chlorobenzonitrile as raw materials, with reference to intermediate G and Example 25 | 936.3[ M+1] |
| 396 | 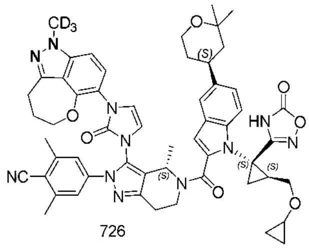 726 Using (S)-2,6-dimethyl-4-(4-methyl-3-(3-(2-(methyl-d3)-2,7,8,9-tetrahydrooxaheptano [4,3,2-cd] indazol-5-yl)-2-carbonyl-2,3-dihydro-1H-imidazol-1-yl)-4,5,6,7-tetrahydro-2H-pyrazolo [4,3-c]pyridin-2-yl)benzonitrile and 1-((1S,2S)-2-(cyclopropyloxymethyl)-1-(5-carbonyl-4,5-dihydro-1,2,4-oxadiazol-3-yl)cyclopropyl)-5-((S)-2,2-dimethyltetrahydro-2H-pyran-4-yl)-1H-indole-2-carboxvlic acid as raw materials | 987.4[ M+1] | 397 | 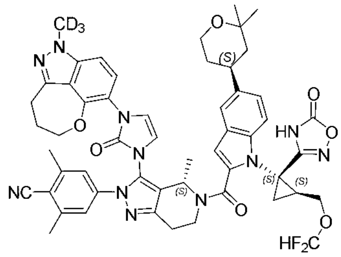 Using (S)-2,6-dimethyl-4-(4-methyl-3-(3-(2-(methyl-d3)-2,7,8,9-tetrahydrooxaheptano[4,3,2-cd]indazol-5-yl)-2-carbonyl-2,3-dihydro-1H-imidazol-1-yl)-4,5,6,7-tetrahydro-2H-pyrazolo[4,3-c]pyridin-2-yl)benzonitrile and 1-((1S,2S)-2-((difluoromethoxy)methyl)-1-(5-carbonyl-4,5-dihydro-1,2,4-oxadiazol-3-yl)cyclopropyl)-5-((S)-2,2-dimethyltetrahydro-2H-pyran-4-yl)-1H-indole-2-carboxylic acid as raw materials | 997.4[ M+1] |

(continued)

| Ex. | Structural formula of compound | MS m/z (ESI) | Ex. | Structural formula of compound | MS m/z (ESI) |
|---|---|---|---|---|---|
| 398 | 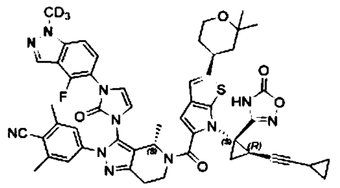 Using tert-butyl(S)-2-(4-cyano-3,5-dimethylphenyl)-3-(3-(4-fluoro-1-(methyl-d3)-1H-indazol-5-yl)-2-carbonyl-2,3-dihydro-1H-imidazol-1-yl)-4-methyl-2,4,6,7-tetrahydro-5H-pyrazolo[4,3-c]pyridine-5-carboxylate and 6-((1S,2R)-2-(cyclopropylethynyl)-1-(5-carbonyl-4,5-dihydro-1,2,4-oxadiazol-3-yl)cyclopropyl)-2-((S)-2,2-dimethyltetrahydro-2H-pyran-4-yl)-6H-thieno[2,3-b]pyrrole-5-carboxylic acid as raw materials | 949.3[ M+1] | 399 | 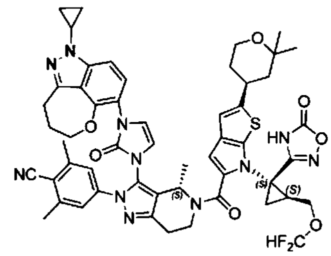 Using tert-butyl(S)-2-(4-cyano-3,5-dimethylphenyl)-3-(3-(2-cyclopropyl-2,7,8,9-tetrahydrooxepin[4,3,2-cd]indazol-5-yl)-2-carbonyl-2,3-dihydro-1H-imidazol-1-yl)-4-methyl-2,4,6,7-tetrahydro-5H-pyrazolo[4,3-c]pyridine-5-carboxylate and 6-((1S,2S)-2-((difluoromethoxy)methyl)-1-(5-carbonyl-4,5-dihydro-1,2,4-oxadiazol-3-yl)cyclopropyl)-2-((S)-2,2-dimethyltetrahydro-2H-pyran-4-yl)-6H-thieno[2,3-b]pyrrole-5-carboxylic acid as raw materials | 1026.3 [M+1] |
| 400 | 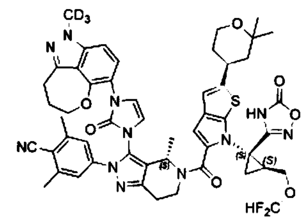 Using tert-butyl(S)-2-(4-cyano-3,5-dimethylphenyl)-4-methyl-3-(3-(2-(methyl-d3)-2,7,8,9- | 1003.3 [M+1] | 392 | 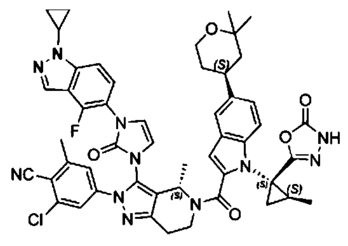 | 936.3[ M+1] |
| | tetrahydrooxaheptano[4,3,2-cd]indazol-5-yl)-2-carbonyl-2,3-dihydro-1H-imidazol-1-yl)-2,4,6,7-tetrahydro-5H-pyrazolo[4,3-c]pyridine-5-carboxylate and 6-((1 S,2S)-2-((difluoromethoxy) methyl)-1-(5-carbonyl-4,5-dihydro-1,2,4-oxadiazol-3-yl)cyclopropyl)-2-((S)-2,2-dimethyltetrahydro-2H-pyran-4-yl)-6H-thieno[2,3-b]pyrrole-5-carboxylic acid as raw materials | | | | |

The following examples can be prepared with reference to Example 29.

| Ex. | Structural formula of compound | MS m/z (ESI) | Ex. | Structural formula of compound | MS m/z (ESI) |
|---|---|---|---|---|---|
| 401 | 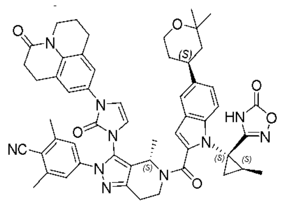<br>Using 9-bromo-2,3,6,7-tetrahydro-1H,5H-pyrido[3,2,1-ij]quinolin-5-one as a raw material | 927.4[M +1] | 402 | 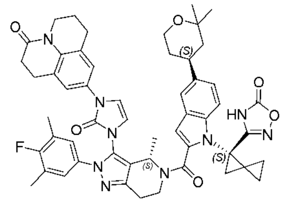<br>Using 9-bromo-2,3,6,7-tetrahydro-1H,5H-pyrido[3,2,1-ij] quinolin-5-one as a raw material | 932.4[M +1] |
| 403 | 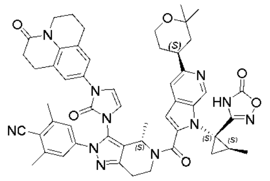<br>Using 9-Bromo-2,3,6,7-tetrahydro-1H,5H-pyrido[3,2,1-ij]quinolin-5-one and tert-butyl(S)-2-(4-cyano-3,5-dimethylphenyl)-4-methyl-3-(2-carbonyl-2,3-dihydro-1H-imidazol-1-yl)-2,4,6,7-tetrahydro-5H-pyrazolo[4,3-c]pyridine-5-carboxylate as raw materials | 928.3[M +H]+ | 404 | 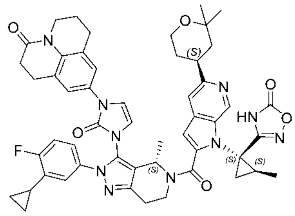<br>Using the raw materials of Example 403 as raw materials | 933.3[M +H]+ |
| 405 | 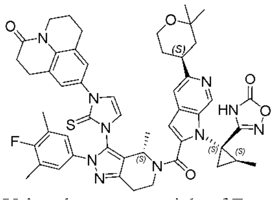<br>Using the raw materials of Example 403 as raw materials | 937.3[M +H]+ | 406 | 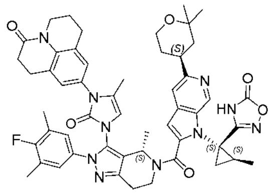<br>Using the raw materials of Example 403 as raw materials | 935.3[M +H]+ |
| 407 | 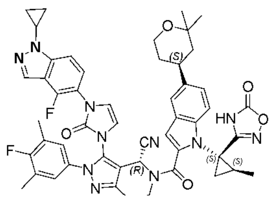<br>Using (R)-2-(4-fluoro-3,5-dimethylphenyl)-3-(2-carbonyl-2,3-dihydro-1H-imidazol-1-yl)-4,5,6,7-tetrahydro-2H-pyrazolo[4,3-c]pyridine-4-carbonitrile as a raw material | 920.4[M +1] | | | |

The following examples can be prepared with reference to Example 34.

| Ex. | Structural formula of compound | MS m/z (ESI) | Ex. | Structural formula of compound | MS m/z (ESI) |
|---|---|---|---|---|---|
| 408 | 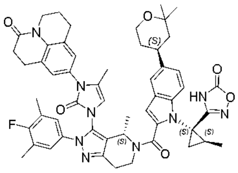 | 934.4 [M+1] | 409 | 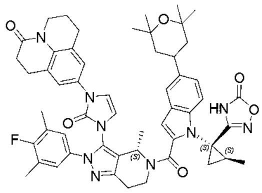 Using 1-((1S,2S)-2-methyl-1-(5-carbonyl-4,5-dihydro-1,2,4-oxadiazol-3-yl)cyclopropyl)-5-(2,2,6,6-tetramethyltetrahydro-2H-pyran-4-yl)-1H-indole-2-carboxylic acid as a raw material | 948.4 [M+1] |

The following examples can be prepared with reference to Example 62.

| Ex. | Structural formula of compound | MS m/z (ESI) | Ex. | Structural formula of compound | MS m/z (ESI) |
|---|---|---|---|---|---|
| 410 | 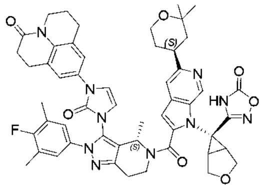 Using 9-bromo-2,3,6,7-tetrahydro-1H,5H-pyrido[3,2,1-ij]quinolin-5-one as a raw material | 949.4 [M+H]+ | 411 | 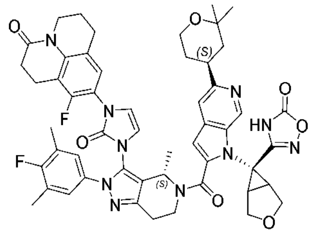 | 967.4 [M+1] |
| 412 | 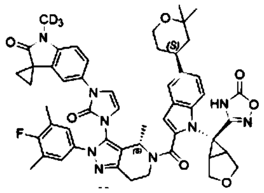 Using 5'-bromo-1'-(methyl-d3)spiro[cyclopropane-1,3'-dihydroindole]-2'-one as a raw material | 937.4 [M+1] | 413 | 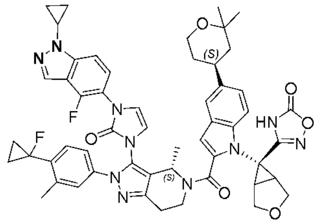 | 963.4 [M+1] |
| 414 | 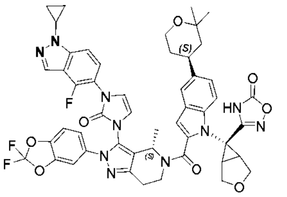 | 971.3 [M+1] | 415 | 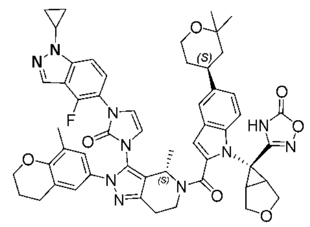 | 961.4 [M+1] |
| 416 | 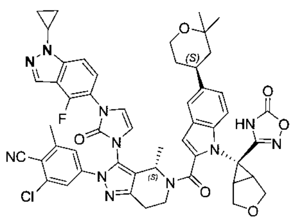 | 964.3 [M+1] | 417 | 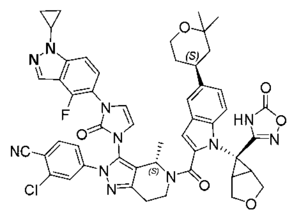 | 950.3 [M+1] |

(continued)

| Ex. | Structural formula of compound | MS m/z (ESI) | Ex. | Structural formula of compound | MS m/z (ESI) |
|---|---|---|---|---|---|
| 418 | 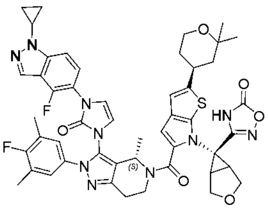 | 943.3 [M+1] | 419 | 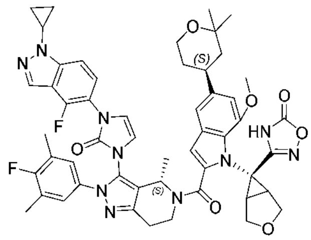 | 967.4 [M+1] |
| 420 | 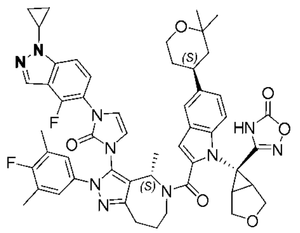 | 951.4 [M+1] | 421 | 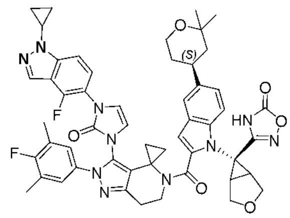 | 949.4 [M+1] |
| 422 | 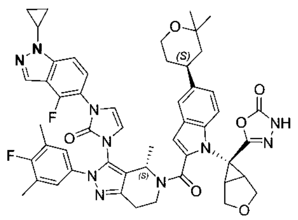 | 937.4 [M+1] | | | |

The following examples can be prepared with reference to Example 63.

| Ex. | Structural formula of compound | MS m/z (ESI) | Ex. | Structural formula of compound | MS m/z (ESI) |
|---|---|---|---|---|---|
| 423 | 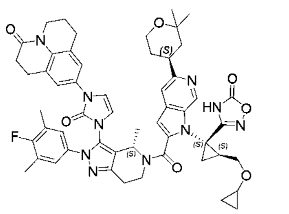 Using 9-bromo-2,3,6,7-tetrahydro-1H,5H-pyrido[3,2,1-ij]quinolin-5-one as a raw material | 977.4[M+H]+ | 424 | 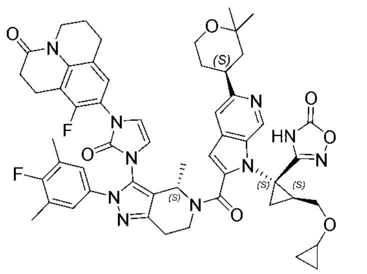 | 995.4[M+1] |
| 425 | 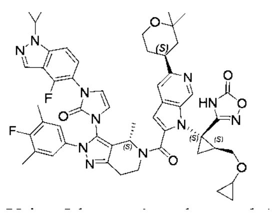 Using 5-bromo-1-cyclopropyl-4-fluoro-1H-indazole as a raw material | 966.4[M+1] | 426 | 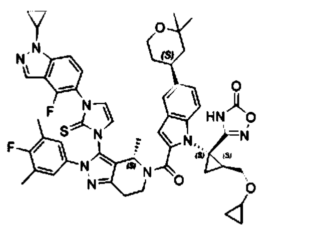 Using 5-bromo-1-cyclopropyl-4-fluoro-1H-indazole as a raw material | 981.4[M+1] |

(continued)

| Ex. | Structural formula of compound | MS m/z (ESI) | Ex. | Structural formula of compound | MS m/z (ESI) |
|---|---|---|---|---|---|
| 427 | 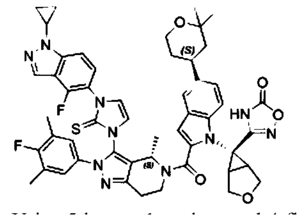 Using 5-bromo-1-cyclopropyl-4-fluoro-1H-indazole as a raw material | 953.4[M +1] | 428 | 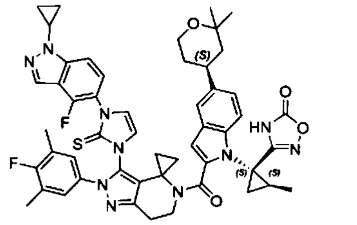 Using 5-bromo-1-cyclopropyl-4-fluoro-1H-indazole as a raw material | 937.4[M +1] |
| 429 | 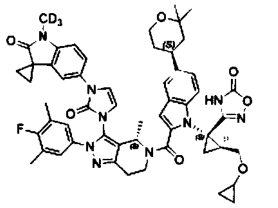 Using 5'-bromo-1'-(methyl-d3)spiro [cyclopropane-1,3'-dihydroindole]-2'-one as a raw material | 965.4[M +1] | | | |

The following examples can be prepared with reference to Example 65 or 65-1.

| Ex. | Structural formula of compound | MS m/z (ESI) | Ex. | Structural formula of compound | MS m/z (ESI) |
|---|---|---|---|---|---|
| 430 | 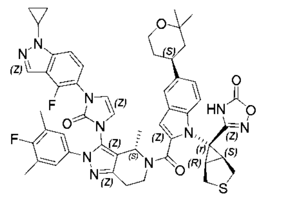 Using (3R,4S)-tetrahydrothiophene-3,4-diol as a raw material | 953.4 [M+1] | 431 | 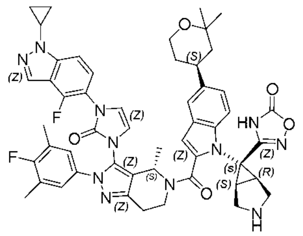 Using (3R,4R)-1-(tert-butyloxycarbonyl)-3,4-dihydroxypyrrolidine as a raw material | 936.4 [M+1] |
| 432 | 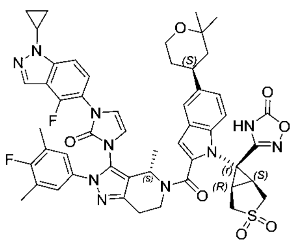 | 985.4 [M+1] | 433 | 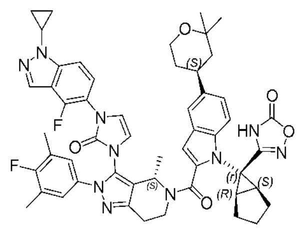 | 935.4 [M+1] |
| 434 | 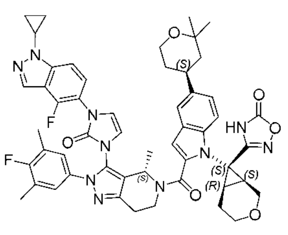 | 951.4 [M+1] | 435 | 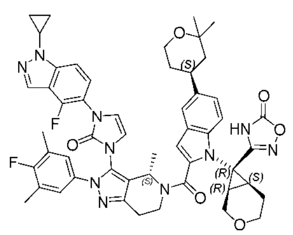 | 951.4 [M+1] |

The following examples can be prepared with reference to Example 82.

| Ex. | Structural formula of compound | MS m/z (ESI) | Ex. | Structural formula of compound | MS m/z (ESI) |
|---|---|---|---|---|---|
| 436 | 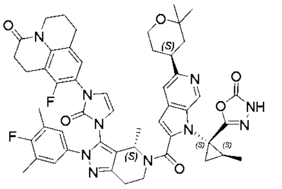 | 939.4 [M+1] | 437 | 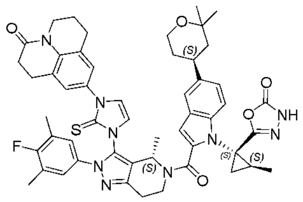 | 936.4[M+1] |
| 438 | 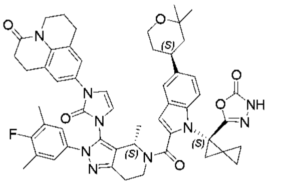 | 932.4 [M+1] | 439 | 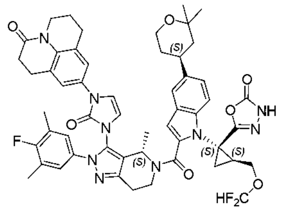 | 986.4[M+1] |
| 440 | 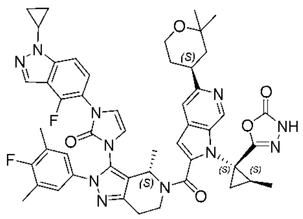 | 910.4 [M+1] | 441 | 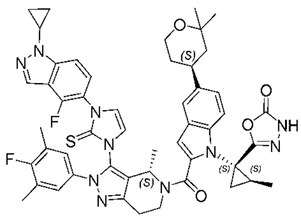 | 925.4[M+1] |
| 442 | 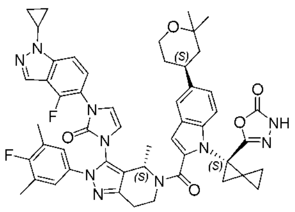 | 921.4 [M+1] | 443 | 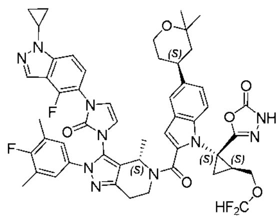 | 975.4[M+1] |
| 444 | 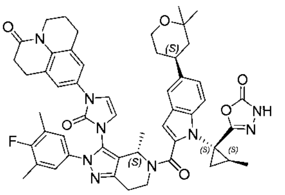 | 920.4 [M+1] | 445 | 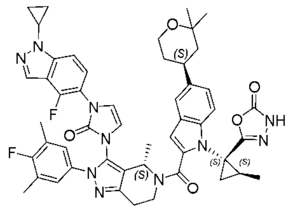 | 909.4[M+1] |
| 446 | 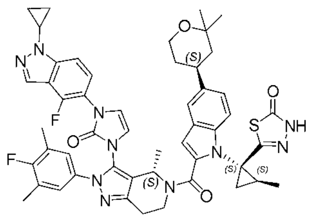 | 925.4 [M+1] | | | |

The following examples can be prepared with reference to Example 126.

| Ex. | Structural formula of compound | MS m/z (ESI) | Ex. | Structural formula of compound | MS m/z (ESI) |
|---|---|---|---|---|---|
| 447 | 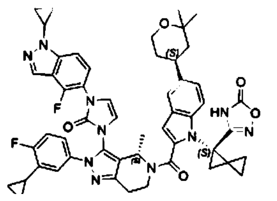 Using tert-butyl 2-(3-cyclopropyl-4-fluorophenyl)-3-(2-carbonyl-2,3-dihydro-1H-imidazol-1-yl)-2,4,6,7-tetrahydro-5H-pyrazolo[4,3-c]pyridine-5-carboxylate as a raw material | 933.4 [M+1] | 448 | 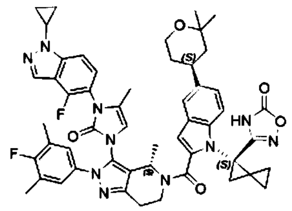 Using (S)-1-(2-(4-fluoro-3,5-dimethylphenyl)-4-methyl-4,5,6,7-tetrahydro-2H-pyrazolo[4,3-c]pyridin-3-yl)-4-methyl-1,3-dihydro-2H-imidazol-2-one as a raw material | 935.4 [M+1] |
| 449 | 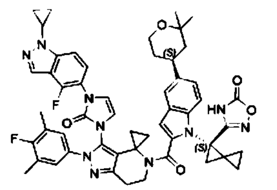 Using 1-(2'-(4-fluoro-3,5-dimethylphenyl)-2',5',6',7'-tetrahydrospiro [cyclopropane-1,4'-pyrazolo[4,3-c]pyridine]-3'-yl)-1,3-dihydro-2H-imidazol-2-one as a raw material | 933.4 [M+1] | 450 | 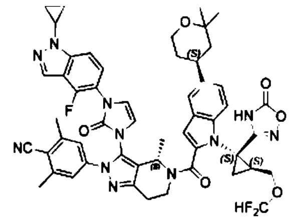 Using 1-((1S,2S)-2-((difluoromethoxy) methyl)-1-(5-carbonyl-4,5-dihydro-1,2,4-oxadiazol-3-yl)cyclopropyl)-5-((S)-2,2-dimethyltetrahydro-2H-pyran-4-yl)-1H-indole-2-carboxvlic acid as a raw material | 982.4 [M+1] |
| 451 | 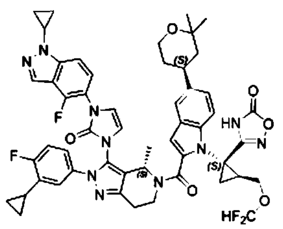 Using 1-((1S,2S)-2-((difluoromethoxy)methyl)-1-(5-carbonyl-4,5-dihydro-1,2,4-oxadiazol-3-yl)cyclopropyl)-5-((S)-2,2-dimethyltetrahydro-2H-pyran-4-yl)-1H-indole-2-carboxylic acid as a raw material | 987.4 [M+1] | 452 | 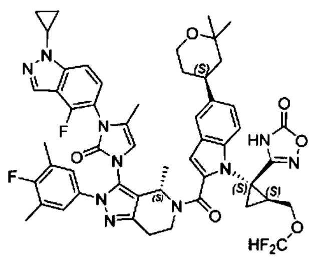 Using 1-((1S,2S)-2-((difluoromethoxy) methyl)-1-(5-carbonyl-4,5-dihydro-1,2,4-oxadiazol-3-yl)cyclopropyl)-5-((S)-2,2-dimethyltetrahydro-2H-pyran-4-yl)-1H-indole-2-carboxylic acid as a raw material | 989.4 [M+1] |
| 453 | 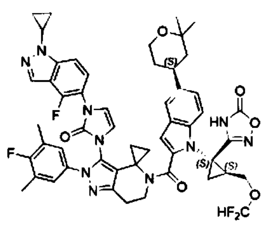 Using 1-((1S,2S)-2-((difluoromethoxy)methyl)-1-(5-carbonyl-4,5-dihydro-1,2,4-oxadiazol-3-yl)cyclopropyl)-5-((S)-2,2-dimethyltetrahydro-2H-pyran-4-yl)-1H-indole-2-carboxylic acid as a raw material | 987.4 [M+1] | | | |

The following examples can be prepared with reference to Example 135.

| Ex. | Structural formula of compound | MS m/z (ESI) | Ex. | Structural formula of compound | MS m/z (ESI) |
|---|---|---|---|---|---|
| 454 | 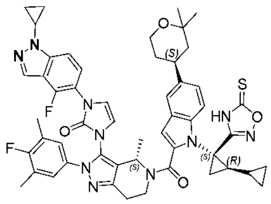 | 951.4 [M+H]+ | 455 | 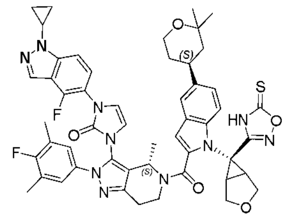<br>Using 3,4-dihydroxytetrahydrofuran as a raw material | 953.4 [M+H]+ |
| 458 | 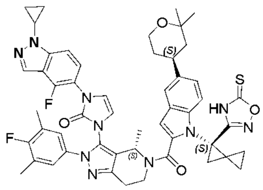<br>Using 1-(hydroxymethyl)cyclopropane-1-ol as a raw material | 937.4 [M+H]+ | 457 | 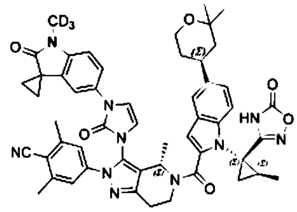<br>Using 5'-bromo-1'-(methyl-d3)spiro[cyclopropane-1,3'-dihydroindole]-2'-one and intermediate G as raw materials | 916.4 [M+1] |
| 458 | 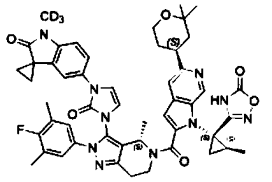<br>Using 5'-bromo-1'-(methyl-d3) spiro[cyclopropane-1,3'-dihydroindole]-2'-one and intermediate F as starting materials | 910.4 [M+1] | 459 | 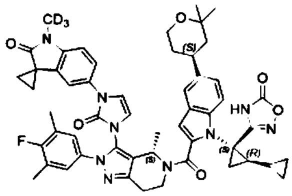<br>Using 5'-bromo-1'-(methyl-d3)spiro[cyclopropane-1,3'-dihydroindole]-2'-one as a raw material | 935.4 [M+1] |
| 460 | 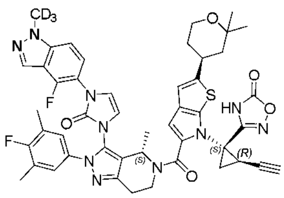<br>Using 6-(cyanomethyl)-2-[2,2-dimethyltetrahydropyran-4-yl]-N-methyl-N-phenylthieno[2,3-b]pyrrole-5-carboxamide as a raw material | 902.3 [M+1] | 461 | 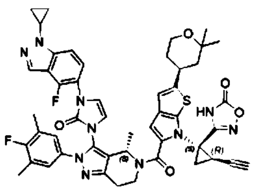<br>Using 5-bromo-1-cyclopropyl-4-fluoro-1H-indazole as a raw material | 925.3 [M+1] |
| 462 | 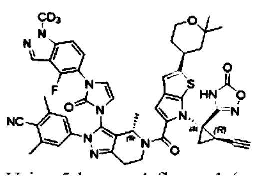<br>Using 5-bromo-4-fluoro-1-(methyl-d3)-1H-indazole as raw material | 909.3 [M+1] | | | |

The following examples can be prepared with reference to Example 144.

| Ex. | Structural formula of compound | MS m/z (ESI) | Ex. | Structural formula of compound | MS m/z (ESI) |
|---|---|---|---|---|---|
| 463 | 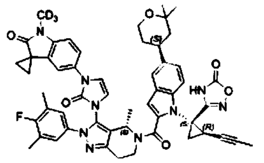 Using 5'-bromo-1'-(methyl-d3)spiro[cyclopropane-1,3'-dihydroindole]-2'-one as a raw material | 933.4[M +1] | 468 | 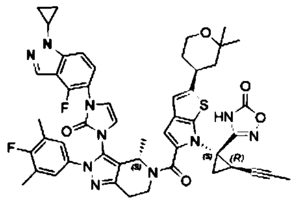 Using 5-bromo-1-cyclopropyl-4-fluoro-1H-indazole as a raw material | 939.3[M +1] |
| 465 | 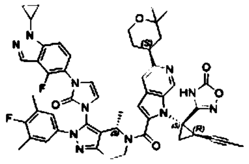 Using 3-((1S,2R)-1-(2-((S)-3-(3-(1-cyclopropyl-4-fluoro-1H-indazol-5-yl)-2-carbonyl-2,3-dihydro-1H-imidazol-1-yl)-2-(4-fluoro-3,5-dimethylphenyl)-4-methyl-4,5,6,7-tetrahydro-2H-pyrazolo [4,3-c]pyridine-5-carbonyl)-5-((S)-2,2-dimethyltetrahydro-2H-pyran-4-yl)-1H-pyrrolo[2,3-c]pyridin-1-yl)-2-ethynylcyclopropyl)-1,2,4-oxadiazol-5(4H)-one as a raw material | 934.4[M +1] | 466 | 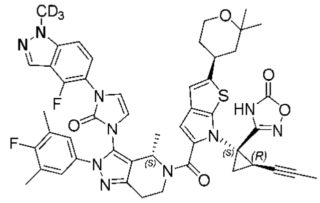 Using 3-((1S,2R)-1-(2-((S)-2,2-dimethyltetrahydro-2H-pyran-4-yl)-5-((S)-3-(3-(4-fluoro-1-(methyl-d3)-1H-indazol-5-yl)-2-carbonyl-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-6H-thieno[2,3-b]pyrrol-6-yl)-2-ethynylcyclopropyl)-1,2,4-oxadiazol-5(4H)-one as a raw material | 916.3[M +1] |
| 467 | 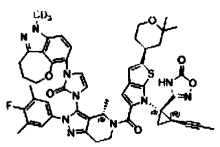 Using 3-((1S,2R)-1-(2-((S)-2,2-dimethyltetrahydro-2H-pyran-4-yl)-5-((S)-2-(4-fluoro-3,5-dimethylphenyl)-4-methyl-3-(3-(2-(methyl-d3)-2,7,8,9-tetrahydrooxaheptano [4,3,2-cd]indazol-5-yl)-2-carbonyl-2,3-dihydro-1H-imidazol-1-yl)-4,5,6,7-tetrahydro-2H-pyrazolo[4,3-c]pyridine-5-carbonyl)-6H-thieno[2,3-b]pyrrol-6-yl)-2-ethynylcyclopropyl)-1,2,4-oxadiazol-5(4H)-one as a raw material | 954.4 [M+H] | 464 | 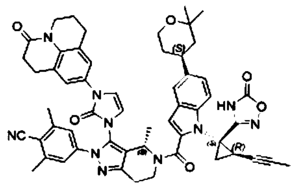 Using 3-((1S,2R)-1-(5-((S)-2,2-dimethyltetrahydro-2H-pyran-4-yl)-2-((S)-2-(4-fluoro-3,5-dimethylphenyl)-4-methyl-3-(2-carbonyl-3-(3-carbonyl-2,3,6,7-tetrahydro-1H,5H-pyrido[3,2,1-ij]quinolin-9-yl)-2,3-dihydro-1H-imidazol-1-yl)-4,5,6,7-tetrahydro-2H-pyrazolo[4,3-c]pyridine-5-carbonyl)-1H-pyrrolo[2,3-c]pyridin-1-yl)-2-ethynylcyclopropyl)-1,2,4-oxadiazol-5(4H)-one as a raw material | 951.4[M +1] |

(continued)

| Ex. | Structural formula of compound | MS m/z (ESI) | Ex. | Structural formula of compound | MS m/z (ESI) |
|---|---|---|---|---|---|
| 469 | 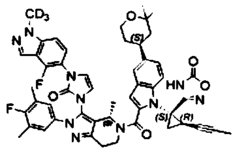 | 910.4 [M+1] | | | |

The following examples can be prepared with reference to Example 145.

| Ex. | Structural formula of compound | MS m/z (ESI) | Ex. | Structural formula of compound | MS m/z (ESI) |
|---|---|---|---|---|---|
| 470 | 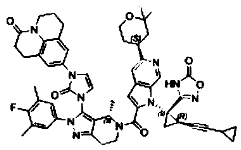 Using 3-((1S,2R)-1-(5-((S)-2,2-dimethyltetrahydro-2H-pyran-4-yl)-2-((S)-2-(4-fluoro-3,5-dimethylphenyl)-4-methyl-3-(2-carbonyl-3-(3-carbonyl-2,3,6,7-tetrahydro-1H,5H-pyrido[3,2,1-ij]quinolin-9-yl)-2,3-dihydro-1H-imidazol-1-yl)-4,5,6,7-tetrahydro-2H-pyrazolo[4,3-c]pyridine-5-carbonyl)-1H-pyrrolo[2,3-c]pyridin-1-yl)-2-ethynylcyclopropyl)-1,2,4-oxadiazol-5(4H)-one as a raw material | 971.4[M+1] | 471 | 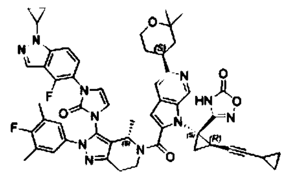 Using 3-((1S,2R)-1-(2-((S)-3-(3-(1-cyclopropyl-4-fluoro-1H-indazol-5-yl)-2-carbonyl-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-pyrrolo[2,3-c]pyridin-1-yl)-2-ethynylcyclopropyl)-1,2,4-oxadiazol-5(4H)-one as a raw material | 960.4[M+1] |

(continued)

| Ex. | Structural formula of compound | MS m/z (ESI) | Ex. | Structural formula of compound | MS m/z (ESI) |
|---|---|---|---|---|---|
| 472 | 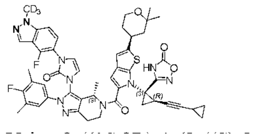 Using 3-((1S,2R)-1-(2-((S)-2,2-dimethyltetrahydro-2H-pyran-4-yl)-5-((S)-3-(3-(4-fluoro-1-(methyl-d3)-1H-indazol-5-yl)-2-carbonyl-2,3-dihydro-1H-imidazol-1-yl)-2-(4-fluoro-3,5-dimethylphenyl)-4-methyl-4,5,6,7-tetrahydro-2H-pyrazolo [4,3-c]pyridine-5-carbonyl)-6H-thieno[2,3-b]pyrrol-6-yl)-2-ethynylcyclopropyl)-1,2,4-oxadiazol-5(4H)-one as a raw material | 942.3 [M+1] | 473 | 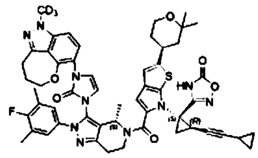 Using 3-((1S,2R)-1-(2-((s)-2,2-dimethyltetrahydro-2H-pyran-4-yl)-5-((S)-2-(4-fluoro-3,5-dimethylphenyl)-4-methyl-3-(3-(2-(methyl-d3)-2,7,8,9-tetrahydrooxaheptano [4,3,2-cd]indazol-5-yl)-2-carbonyl-2,3-dihydro-1H-imidazol-1-yl)-4,5,6,7-tetrahydro-2H-pyrazolo [4,3-c]pyridine-5-carbonyl)-6H-thieno[2,3-b]pyrrol-6-yl)-2-ethynylcyclopropyl)-1,2,4-oxadiazol-5(4H)-one as a raw material | 980.4[M+H] |
| 474 | 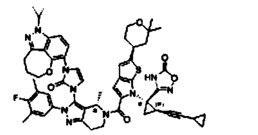 Using 5-bromo-2-cyclopropyl-2,7,8,9-tetrahydrooxepin[4,3,2-cd]indazole as a raw material | 1003.4[M+1] | 475 | 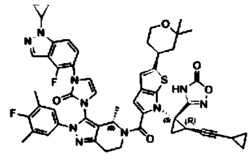 Using 5-bromo-1-cyclopropyl-4-fluoro-1H-indazole as a raw material | 965.3[M+1] |
| 476 | 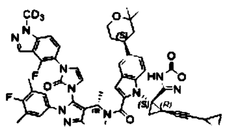 | 936.4 [M+1] | | | |

The following examples can be prepared with reference to Example 368.

| Ex. | Structural formula of compound | MS m/z (ESI) | Ex. | Structural formula of compound | MS m/z (ESI) |
|---|---|---|---|---|---|
| 477 | 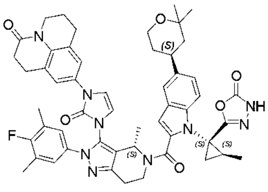 | 920.4[M+1] | 478 | 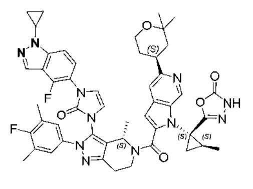 | 910.4 [M+1] |

(continued)

| Ex. | Structural formula of compound | MS m/z (ESI) | Ex. | Structural formula of compound | MS m/z (ESI) |
|---|---|---|---|---|---|
| 483 | 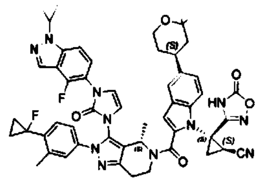 Using (4-(1-fluorocyclopropyl)-3-methylphenyl)hydrazine as a raw material | 946.4[M+1] | 480 | 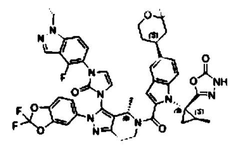 Using (2,2-difluorobenzo[d][1,3]dioxazol-5-yl)hydrazine as a raw material | 943.3 [M+1] |
| 481 | 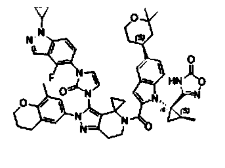 | 945.4[M+1] | 482 | 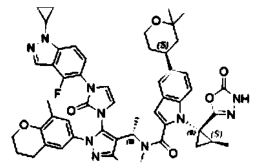 | 933.4 [M+1] |
| 479 | 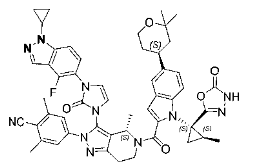 | 916.4[M+1] | | | |

The following examples can be prepared with reference to Example 182.

| Ex. | Structural formula of compound | MS m/z (ESI) | Ex. | Structural formula of compound | MS m/z (ESI) |
|---|---|---|---|---|---|
| 484 | 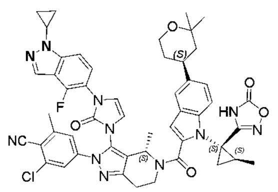 Using 4-bromo-2-chloro-6-methylbenzonitrile as a raw material | 936.3[ M+1] | 485 | 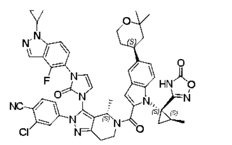 Using 4-bromo-2-chlorobenzonitrile as a raw material | 922.3[M +1] |
| 486 | 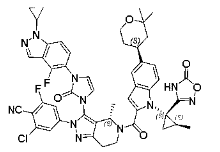 Using 4-bromo-2-chloro-6-fluorobenzonitrile as a raw material | 940.3[ M+1] | 487 | 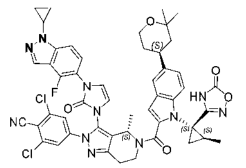 Using 4-bromo-2,6-dichlorobenzonitrile as a raw material | 956.3[M +1] |
| 488 | 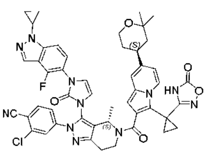 Using 4-bromo-2-chlorobenzonitrile as a raw material | 908.3[ M+1] | 489 | | 991.4[M +1] |

(continued)

| Ex. | Structural formula of compound | MS m/z (ESI) | Ex. | Structural formula of compound | MS m/z (ESI) |
|---|---|---|---|---|---|
| 490 | 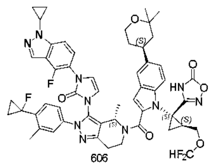 606 Using 5-bromo-2-(1-fluorocyclopropyl)-1,3-dimethylbenzene as a raw material | 1001.4[ M+1] | 491 | 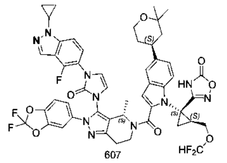 607 Using 5-bromo-2,2-difluorobenzo[d][1,3]dioxazole as a raw material | 1009.3[ M+1] |
| 492 | 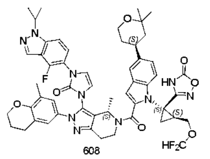 608 Using 6-bromo-8-methylchromane as a raw material | 999.4[ M+1] | 493 | 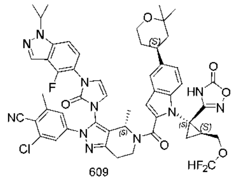 609 Using 4-bromo-2-chloro-6-methylbenzonitrile as a raw material | 1002.3[ M+1] |
| 494 | 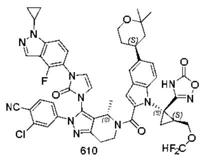 610 | 988.3[ M+1] | 495 | 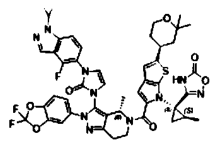 | 949.3[M +1] |
| | Using 4-bromo-2-chlorobenzonitrile as a raw material | | | Using (2,2-difluorobenzo [d][1,3]dioxazol-5-yl) hydrazine as a raw material | |
| 496 | 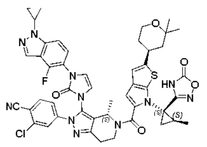 Using 4-bromo-2-chlorobenzonitrile as a raw material | 928.2[ M+1] | 497 | 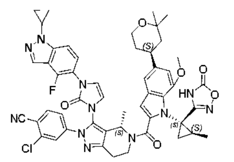 Using 4-bromo-2-chlorobenzonitrile as a raw material | 952.3[M +1] |
| 498 | 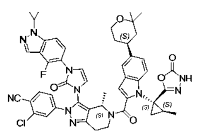 Using 4-bromo-2-chlorobenzonitrile as a raw material | 922.3[ M+1] | 499 | 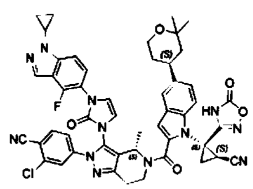 | 933.3[M +1] |
| 500 | 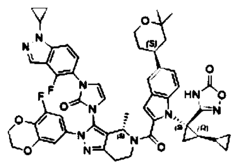 Using (8-fluoro-2,3-dihydrobenzo[b][1,4]dioxin-6-yl) hydrazine as a raw material | 965.4[ M+1] | 501 | 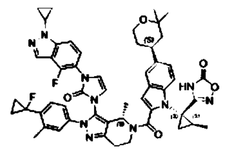 Using (4-(1-fluorocyclopropyl)-3-methylphenyl) hydrazine as a raw material | |

The following examples can be prepared with reference to Example 189.

| Ex. | Structural formula of compound | MS m/z (ESI) | Ex. | Structural formula of compound | MS m/z (ESI) |
|---|---|---|---|---|---|
| 502 | 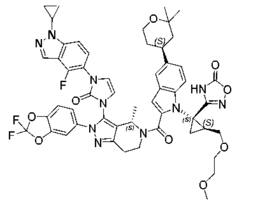 Using (2,2-difluorobenzo [d][1,3]dioxazol-5-yl)hydrazine as a raw material, with reference to intermediate G | 1017.3 [M+H]+ | 503 | 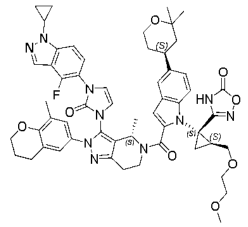 Using (8-methylchroman-6-yl)hydrazine as a raw material, with reference to intermediate G | 1007.4 [M+H] + |
| 504 | 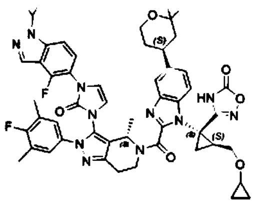 Using (4-fluoro-3,5-dimethylphenyl)hydrazine as a raw material | 966.4 [M+1] | 505 | 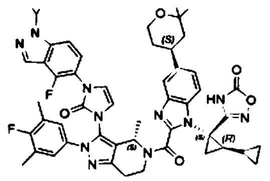 Using (4-fluoro-3,5-dimethylphenyl) hydrazine as a raw material | 936.4 [M+1] |
| 506 | 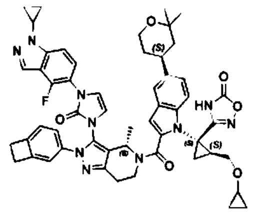 Using bicyclo[4.2.0] octa-1,3,5-trien-3-vlhvdrazine as a raw material | 945.4[M +1] | 507 | 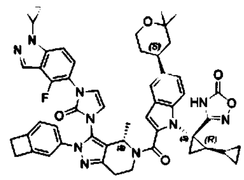 Using bicyclo[4.2.0]octa-1,3,5-trien-3-ylhydrazine as a raw material | 915.4[ M+1] |
| 508 | 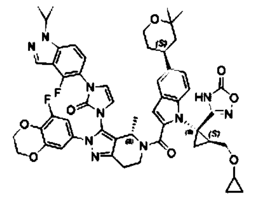 Using (8-fluoro-2,3-dihydrobenzo[b][1,4]dioxin-6-yl) hydrazine as a raw material | 995.4[M +1] | 509 | Using cis-4,4-difluorocyclopentane-1,2-diol as a raw material | 948.4[ M+1] |
| 510 | Using 5-(difluoromethylene)-2,2-dimethyl-1,3-dioxane as a raw material | 934.4[M +1] | 511 | Using 2-isopropylidenepropane-1,3-diol as a raw material | 926.4[ M+1] |
| 512 | Using cis-4,4-difluorocyclopentane-1,2-diol as a raw material | 971.4[M +1] | 513 | Using 5-(difluoromethylene)-2,2-dimethyl-1,3-dioxane as a raw material | 957.4[ M+1] |
| 514 | Using 2-isopropylidenepropane-1,3-diol as a raw material | 949.4[M +1] | | | |

**Biological Assays and Evaluations**

The present invention is further described and explained in conjunction with assay examples below. However, these examples are not intended to limit the scope of the present invention.

**I. Determination of the ability of the compound of the present invention to stimulate cAMP production in a cell line stably transformed with human GLP1 receptor**

1. Experimental purpose:

The purpose of this assay example is to test the ability of the compound to activate the human GLP-1 receptor on cell surface. The $EC_{50}$ for stimulated cAMP production after activation characterizes the ability of the compound to activate the human GLP-1 receptor.
In this experiment, the compound and cells were co-incubated for 30 min.

2. Experimental reagents and instruments

2.1 Experimental instruments:

Microplate reader (BioTek Synergy H1)
Pipette (Eppendorf & Rainin)

2.2 Experimental reagents

DMEM/F12, casein, 384-well plate, IBMX, Cisbio cAMP - Gs Dynamic kit

3. Experimental method:

Frozen cell line CHO-K1/GLP-1R/CRE-luc stably transformed with human GLP1 receptor was removed from a liquid nitrogen tank, and was placed in a 37°C water bath for rapid thawing. The cells were resuspended in DMEM/F12 medium, and after centrifugation, were washed once. The cells were resuspended in an experimental buffer, i.e., DMEM/F12 medium containing 0.1% casein. After centrifugation, cell density was adjusted using the experimental buffer. The cells were plated onto a 384-well plate at a density of 1500 cells/5 μL/well. Then, each well was added with 2.5 μL of IBMX working solution prepared with the buffer, the final concentration of IBMX being 0.5 mM, and 2.5 μL of a serially diluted compound sample (starting from 1000 to 333.33 nM, 3-fold dilution, 12 concentrations). The plate was centrifuged at 1000 rpm for 1 min, and was stood for incubation at 37°C for 0.5 h. Determination was performed using the Cisbio cAMP - Gs Dynamic kit. cAMP-d2 and anti-cAMP-Eu3+-Cryptate were diluted 18-fold and 20-fold respectively with cAMP Lysis & Detection Buffer, and were respectively mixed homogeneously. Each well was added with 5 μL of diluted cAMP-d2 solution, and the plate was centrifuged at 1000 rpm for 1 min, and shaken for 30 seconds for homogeneous mixing. Then, each well was added with 5 μL of diluted anti-cAMP-Eu3+-Cryptate solution, and the plate was centrifuged at 1000 rpm for 1 min and was incubated in the dark at room temperature for 1 h. HTRF signals were read using the Biotek Synergy H1 microplate reader, the excitation wavelength being 320 nm and the emission wavelengths being 620 nm and 665 nm.

4. Method for experimental data processing:

The signal-to-signal ratio was calculated (665 nm/620 nm * 10,000). Nonlinear fitting between the signal-to-signal ratio and the sample concentration was performed in GraphPad Prism 10 using a four-parameter equation to obtain the $EC_{50}$ value.

5. Experimental conclusion:

According to the above scheme, in the experiment of production of cAMP by the cell line stably transformed with hGLP1R under the stimulation of the compound of the present invention for 0.5 h, the $EC_{50}$ values for cAMP production as shown in Table 1 were exhibited.

Table 1 $EC_{50}$ values for cAMP production by the cell line stably transformed with human GLP1 receptor under the stimulation of the example compounds of the present invention

| Example | $EC_{50}$ (nM) | Example | $EC_{50}$ (nM) |
|---|---|---|---|
| 1 | 0.37 | 67 | 1.4 |
| 7 | 0.30 | 144 | 1.0 |
| 8 | 0.51 | 162 | 0.25 |
| 15 | 0.32 | 285 | 0.71 |
| 19 | 0.88 | 287 | 0.9 |
| 23 | 0.14 | 339 | 0.51 |
| 41 | 0.9 | 342 | 1.5 |
| 61 | 1.39 | | |

The experimental results show that the example compounds of the present invention exhibited a good biological activity in the experiment of production of cAMP by the cell line stably transformed with human GLP1 receptor under the stimulation thereof.

**II. Determination of the ability of the compound of the present invention to stimulate cAMP production in a cell line stably transformed with human GLP1 receptor**

1. Experimental purpose:

The purpose of this assay example is to test the ability of the compound to activate the human GLP-1 receptor on cell surface. The $EC_{50}$ for stimulated cAMP production after activation characterizes the ability of the compound to activate the human GLP-1 receptor.

In this experiment, the compound and cells were co-incubated for 2 h.

2. Experimental reagents and instruments

2.1 Experimental instruments:

Microplate reader, Pipette

2.2 Experimental reagents

DMEM/F12 medium, casein, 384-well plate, IBMX, Cisbio cAMP - Gs Dynamic kit

3. Experimental method:

Frozen cell line CHO-K1/GLP-1R/CRE-luc stably transformed with human GLP1 receptor was removed from a liquid nitrogen tank, and was placed in a 37°C water bath for rapid thawing. The cells were resuspended in DMEM/F12 medium, and after centrifugation, were washed once. The cells were resuspended in an experimental buffer, i.e., DMEM/F12 medium containing 0.1% casein. After centrifugation, cell density was adjusted using the experimental buffer. The cells were plated onto a 384-well plate at a density of 1500 cells/5 μL/well. Then, each well was added with 2.5 μL of IBMX working solution prepared with the buffer, the final concentration of IBMX being 0.5 mM, and 2.5 μL of a serially diluted compound sample (starting from 1000 to 333.33 nM, 3-fold dilution, 12 concentrations). The plate was centrifuged at 1000 rpm for 1 min, and was stood for incubation at 37°C for 2 h. Determination was performed using the Cisbio cAMP - Gs Dynamic kit. cAMP-d2 and anti-cAMP-Eu3+-Cryptate were diluted 18-fold and 20-fold respectively with cAMP Lysis & Detection Buffer, and were respectively mixed homogeneously. Each well was added with 5 μL of diluted cAMP-d2 solution, and the plate was centrifuged at 1000 rpm for 1 min, and shaken for 30 seconds for homogeneous mixing. Then, each well was added with 5 μL of diluted anti-cAMP-Eu3+-Cryptate solution, and the plate was centrifuged at 1000 rpm for 1 min and was incubated in the dark at room temperature for 1 h. HTRF signals were read using the Biotek Synergy H1 microplate reader, the excitation wavelength being 320 nm and the emission wavelengths being 620 nm and 665 nm.

4. Method for experimental data processing:

The signal-to-signal ratio was calculated (665 nm/620 nm * 10,000). Nonlinear fitting between the signal-to-signal ratio and the sample concentration was performed in GraphPad Prism 10 using a four-parameter equation to obtain the $EC_{50}$ value.

5. Experimental conclusion:

According to the above scheme, in the experiment of production of cAMP by the cell line stably transformed with hGLP1R under the stimulation of the compound of the present invention for 2 h, the $EC_{50}$ values for cAMP production as shown in Table 2 were exhibited.

Table 2 $EC_{50}$ values for cAMP production by the cell line stably transformed with human GLP1 receptor under the stimulation of the example compounds of the present invention

| Example | $EC_{50}$ (nM) | Example | $EC_{50}$ (nM) |
|---|---|---|---|
| 34 | 0.11 | 174 | 0.15 |
| 43 | 0.06 | 187 | 0.08 |
| 49 | 0.032 | 190 | 0.04 |
| 50 | 0.053 | 194 | 0.009 |
| 65-1 | 0.06 | 195 | 0.035 |
| 67 | 0.024 | 200 | 0.09 |
| 68 | 0.016 | 282 | 0.074 |
| 143 | 0.013 | 285 | 0.09 |
| 144 | 0.05 | 338 | 0.023 |
| 162 | 0.10 | | |

**III. Effect of single administration of the compound of the present invention on intraperitoneal glucose tolerance of GLP-1R humanized mice**

1. Experimental purpose:

To evaluate the effect of single administration of the compound of the present invention on blood glucose changes in the intraperitoneal glucose tolerance test (*ip*GTT) of GLP-1R humanized C57BL/6 mice.

2. Experimental materials:

C57BL/6_*hGLP-1R,* male, 5-8 weeks; ultra-clean bench; electronic balance; ACCU-CHEK Active blood glucose meter; glucose.

3 Experimental operation and data processing:

3.1. The day before the experiment, the animals were randomly grouped according to their body weight, with 5 animals in each group. All animals were deprived of food and fasted overnight for 16 h or more until administration;
3.2. A 0.2 g/mL glucose solution was prepared using pure water, and was filtered through a 0.22 μm filter membrane for later use;
3.3. On the day of assay, the blood sugar level of each animal prior to administration was measured by tail-cutting method, and was recorded as Baseline value;
Blood sugar assay method: The mouse was placed in a restrainer. The tail tip was disinfected using an alcohol cotton ball, and then a bit of the tail tip was cut off with scissors. After discarding the first drop of blood, the second drop of blood was dripped onto a prepared blood sugar test paper to detect the blood sugar value;
3.4. Administration was performed according to the body weight of the animals and the administration time of each

animal was recorded. One h after administration, the blood sugar value of each animal was measured, and was recorded as the blood sugar value at 0 min;

3.5. Then, according to the body weight of that day, pure water or glucose solution was immediately injected intraperitoneally in a volume of 10 mL/kg and a glucose dose of 2 g/kg;

3.6. The blood glucose value of each mouse was measured 15, 30, 60, 90 and 120 min after the injection of pure water or glucose solution, and the time and data were recorded;

3.7. After completion of the assay, all animals were allowed to resume eating.

3.8. Data processing:

A blood glucose (BG)-time curve was drawn, and the area under the blood glucose-time curve was calculated according to the following calculation formula:

AUC (mmol/L.hr)=(BG0+BG15)$\times$0.25/2 + (BG15+BG30)$\times$0.25/2 + (BG30+BG60)$\times$0.5/2 + (BG60+BG90)$\times$0.5/2 + (BG90+BG120)$\times$0.5/2.

Note: BG0, BG15, BG30, BG60, BG90 and BG120 represent the blood glucose values before glucose administration (0 min), and 15, 30, 60, 90 and 120 min after glucose administration, respectively.

The blood glucose reduction rate at each time point and AUC was calculated based on the average blood glucose value and blood glucose AUC at each time point according to the following calculation formula: blood glucose reduction rate = (blood glucose of model control group / AUC - blood glucose of drug administration group / AUC) / blood glucose of model control group / AUC $\times$ 100%.

The example compounds of the present invention were able to effectively reduce the blood glucose of mice. The blood glucose reduction rate was greater than 20%, preferably the blood glucose reduction rate of the compounds was greater than 40%, and more preferably the blood glucose reduction rate of the compounds was greater than 60%.

## IV. Effects of long-term administration of the compound of the present invention on the body weight and food intake of GLP-1R humanized mice fed with CDAHFD high-fat diet

1. Experimental purpose:

The purpose of this assay was to evaluate the effects of long-term administration of the compound on the body weight and food intake of GLP-1R humanized C57BL/6 mice fed with CDAHFD high-fat diet.

2. Experimental reagents and instruments

C57BL/6_*hGLP-1R,* male, 5-8 weeks, purchased from Biocytogen Pharmaceuticals (Beijing) Co., Ltd.

Choline-deficient, L-amino acid-defined, high-fat diet (CDAHFD), purchased from Shanghai Bopai Biotechnology Co., Ltd. (Research diet-A06071302)

Ultra-clean bench (CJ-2F, Suzhou Fengshi Experimental Animal Equipment Co., Ltd.

Electronic balance (BSA2202S-CW, Sartorius)

3. Experimental methods

3.1 On the day of starting feeding the high-fat diet, the C57BL/6 mice were randomly grouped according to body weight. The first group was Blank, with 7 mice, and was fed with normal control diet. The remaining animals were the modeling group, and were fed with the high-fat diet until the end of the experiment.

3.2 In week 4 of CDAHFD feeding, the animals in the modeling group were randomly grouped according to body weight, with 7 mice in each group. The first group was Vehicle group (Vehicle: 10%PEG400/10%PG/80% glycine buffer (100 mM glycine, 26 mM NaOH, pH 9) buffer), and was administered solvent; the remaining group was administration group. Administration regime: the corresponding compound was orally administered for a period of 21 days, once each day, the administration dose being 0.3 mg/kg, 1 mg/kg, 3 mg/kg, and 10 mg/kg, and the administration volume being 10 mL/kg. The Blank group continued to be fed with the normal diet, and was orally administered solvent once each day.

3.3 The day of administration was defined as Day 0.

3.4 The animals were weighed at each administration and the data were recorded. Oral administration was performed

according to body weight, and the administration volume was 10 mL/kg.

3.5 Starting from day 0 of the experiment, the food intake of the mice in each group was measured every three days. Specifically, after each weighing and administration, the diet was renewed, and the added amount and remaining amount were recorded.

3.6 On Day 21, the end day, all mice were euthanized sequentially according to grouping order. Plasma and tissues (liver, kidney, ileum, pancreas, and brain) were taken for PK determination, and serum was taken for blood biochemistry determination (ALT, AST, TG, TC, and LDL-C).

4. Experimental data processing and statistical analysis

The body weight and body weight change rate of the mice after administration were summarized and statistically analyzed. The body weight change rate was calculated according to (BWt - BW0) / BW0 × 100%, where BWt represents mouse body weight on day t of the experiment, and BW0 represents mouse body weight on day 0 of the experiment.

Food intake was calculated according to (added amount (g) - remaining amount (g)) / number of animals per cage. The cumulative food intake was the sum of the daily food intake of each animal during the administration period.

The experimental data were analyzed and plotted using GraphPad Prism software. Comparison between two groups was performed using the t-test method, and comparison among three or more groups was performed using the one-way ANOVA method. $p<0.05$ was defined as the difference being statistically significant.

The experimental results are shown in Table 3 below.

Table 3 Effects of long-term administration of the compound of the present invention on body weight and food intake of GLP-1R humanized mice fed with CDAHFD high-fat diet

| Example | Dose | Body weight change (%)- |
|---|---|---|
| Example 67 | 3 mg/kg | -20.34% |
| Example 143 | 3 mg/kg | -19.43% |
| Example 144 | 0.3mg/kg | -10.40% |
| | 1 mg/kg | -14.70% |

**V. Pharmacokinetic evaluation test in mice**

1. Research purpose:

Balb/c mice were used as test animals to study the pharmacokinetic behavior of the compound of the present invention in mice (plasma) at a dose of 5 mg/kg administered orally and 1 mg/kg administered intravenously.

2. Experimental regime:

2.1 Experimental drug:

The compound of the present invention, prepared in house.

2.2 Experimental animals:

Balb/c mice, 3 per group, male.

2.3 Formulation prescription:

Preparation of drug for oral administration: 10%PEG400/10%PG/80% glycine buffer (100 mM glycine, 64 Mm NaOH, pH 10) buffer.

10 ml of PEG400, 10 ml of PG, and 80 ml of (100 mM glycine, 64 Mm NaOH, pH = 9) were measured into a 100 ml volumetric flask, vortexed, mixed homogeneously, and sonicated to obtain a clear solution.

Single PK: An example compound was weighed into a 4 mL glass bottle. The solution was added, and sonication was performed for 10 min to obtain a colorless clear solution having a concentration of 0.5 mg/mL.

Cassette PK: Five example compounds were weighed at a similar weight into a 4 mL glass bottle and were mixed. The solution was added, and sonication was performed for 10 min to obtain a colorless clear solution having a

concentration of 0.5 mg/mL.
Preparation of drug for intravenous administration: 5% DMSO + 10% Solutol HS15 + 85% PBS

An example compound was weighed. 5% DMSO was first added according to the proportion in the total volume of administration, followed by vortexing and sonication for 2 min to achieve complete dissolution. Then 10% Solutol HS15 was added, followed by vortexing and sonication for 2 min to achieve complete dissolution. Finally, 85% PBS was added, followed by vortexing and sonication for 5 min and filtering through a 0.22 um filter membrane to obtain a colorless, transparent, clear solution having a concentration of 0.2 mg/mL.

2.4 Administration:

Three Balb/c mice, male, respectively administered p.o. after fasting overnight, at a dose of 5 mg/kg and in an administration volume of 10 mL/kg.

Three Balb/c mice, male, respectively administered IV after fasting overnight, at a dose of 1 mg/kg and in an administration volume of 5 mL/kg.

2.5 Sample collection:

Blood collection: 0.04 mL of blood was collected from the eye sockets of the mice before administration and at 0.083 (IV), 0.25, 0.5, 1, 2, 4, 6, 8, and 24 h after administration. The blood samples were placed in EDTA-K2 anticoagulant tubes and centrifuged at 6000 rpm at 4°C for 6 min to separate plasma, which was stored at -80°C. The mice were fed 4 h after administration.

2.6 Sample treatment:

1) 40 μL of a plasma sample was added with 160 μL of acetonitrile for precipitation, and after mixing, the mixture was centrifuged at 3500 × g for 5 to 20 min.
2) The supernatant solution after treatment was taken for LC/MS/MS analysis of the concentration of the test compound. The LC/MS/MS analysis instrument was AB Sciex API 4000 Qtrap.

2.7 Liquid phase analysis:

• Liquid phase condition: Shimadzu LC-20AD pump
• Chromatographic column: Agilent ZORBAX XDB-C18 (50×2.1 mm, 3.5 μm) Mobile phase: Solution A was 0.1% formic acid aqueous solution, and Solution B was acetonitrile
• Flow rate: 0.4 mL/min
• Elution time: 0-4.0 min, the eluent was as follows:

| Time/min | Solution A | Solution B |
|---|---|---|
| 0.01 | 90% | 10% |
| 0.5 | 90% | 10% |
| 0.8 | 5% | 95% |
| 2.4 | 5% | 95% |
| 2.5 | 90% | 10% |
| 4.0 | Stop | |

3. Experimental results and analysis

Main pharmacokinetic parameters were calculated using WinNonlin 6.1. The results of the mouse pharmacokinetic experiment are shown in Table 4 below:

Table 4: Pharmacokinetic parameters of oral administration of the compound of the present invention in mice

| Parameters | tmax (h) | Cmax (ng/mL) | AUC 0-t (ng/mL*h) | AUC 0-∞ (ng/mL*h) | tl/2 (h) | MRT 0-∞ (h) |
|---|---|---|---|---|---|---|
| Example 7 | 0.5 | 2970 | 10324 | 10768 | 1.6 | 2.8 |
| Example 25 | 2.0 | 2057 | 30498 | 39006 | 10.7 | 15.2 |
| Example 32 | 1.0 | 1044 | 8986 | 9322 | 5.3 | 6.7 |
| Example 34* | 1.0 | 1510 | 12052 | 13316 | 7.8 | 9.0 |
| Example 35* | 1.2 | 3073 | 32868 | 40998 | 10.3 | 13.9 |
| Example 49* | 1.0 | 2430 | 15698 | 16631 | 6.5 | 7.1 |
| Example 65-1* | 2.7 | 1220 | 10636 | 10946 | 4.8 | 6.5 |
| Example 67* | 4.0 | 1430 | 11219 | 12121 | 7.6 | 8.2 |
| Example 143* | 2.0 | 1204 | 12212 | 13260 | 6.9 | 9.0 |
| Example 144* | 3.0 | 2237 | 23506 | 35060 | 15.7 | 20.8 |
| Example 194 | 2.7 | 1843 | 19136 | 32185 | 19.9 | 26.6 |
| Example 282* | 2.0 | 1510 | 15026 | 18163 | 9.7 | 13.0 |
| Example 287* | 1.0 | 5323 | 27885 | 27973 | 3.0 | 4.2 |
| Note: * represents "single PK". | | | | | | |

3.4 Experimental conclusion:

The experimental results show that the example compounds of the present invention exhibited good metabolic properties, with both the exposure AUC and the maximum blood drug concentration $C_{max}$ being good.

**VI. Pharmacokinetic evaluation test in rats**

1. Research purpose:

SD rats were used as test animals to study the pharmacokinetic behavior of the following example compounds, administered orally at a dose of 5 mg/kg, in the plasma of the rats.

2. Experimental regime:

2.1 Experimental drug:

Example compounds of the present invention, prepared in house.

2.2 Experimental animals:

SD rats, 3 per group, male.

2.3 Formulation prescription:

Preparation of drug for oral administration: 10%PEG400/10%PG/80% glycine buffer (100 mM glycine, 64 Mm NaOH, pH 10) buffer.

10 ml of PEG400, 10 ml of PG, and 80 ml of (100 mM glycine, 64 Mm NaOH, pH = 9) were measured into a 100 ml volumetric flask, vortexed, mixed homogeneously, and sonicated to obtain a clear solution.

An example compound was weighed and added to a 4 mL glass bottle, and the solution was added. The pH was first adjusted with 1 M NaOH until the example compound dissolves to obtain a clear solution, and then the pH was readjusted to pH 9.0-9.5 with 1 M HCl. Sonication was performed for 10 min to obtain a colorless clear solution having a concentration of 0.5 mg/mL.

Preparation of drug for intravenous administration: 5% DMSO + 10% Solutol HS15 + 85% PBS

An example compound was weighed. 5% DMSO was first added according to the proportion in the total volume of

administration, followed by vortexing and sonication for 2 min to achieve complete dissolution. Then 10% Solutol HS15 was added, followed by vortexing and sonication for 2 min to achieve complete dissolution. Finally, 85% PBS was added, followed by vortexing and sonication for 5 min and filtering through a 0.22 um filter membrane to obtain a colorless, transparent, clear solution having a concentration of 0.2 mg/mL.

2.4 Administration:

Three rats, male, respectively administered p.o. after fasting overnight, at a dose of 5 mg/kg and in an administration volume of 10 mL/kg.

Three rats, male, respectively administered IV after fasting overnight, at a dose of 1 mg/kg and in an administration volume of 5 mL/kg.

2.5 Sample collection:

Before and after administration to the rats, 0.2 mL of blood was collected from the jugular vein at 0.083 (IV), 0.25, 0.5, 1, 2, 4, 6, 8 and 24 h. The blood samples were placed in EDTA-$K_2$ tubes and centrifuged at 6000 rpm at 4°C for 6 min to separate plasma, which was stored at -80°C. The rats were fed 4 h after administration.

2.6 Sample treatment:

1) 40 μL of a plasma sample was added with 160 μL of acetonitrile for precipitation, and after mixing, the mixture was centrifuged at 3500 × g for 5 to 20 min.
2) 100 μL of the supernatant solution after treatment was taken for LC/MS/MS analysis of the concentration of the test compound.

2.6 Liquid phase analysis

• Liquid phase conditions: Shimadzu LC-20AD pump
• Mass spectrometry conditions: AB Sciex API 4000 mass spectrometer
• Chromatographic column: phenomenex Gemiu 5 um C18 50 × 4.6 mm
• Mobile phase: Solution A was 0.1% formic acid aqueous solution, and Solution B was methanol
• Flow rate: 1.0 mL/min
• Elution time: 0-4.0 min, the eluent was as follows:

| Time/min | Solution A | Solution B |
|---|---|---|
| 0.01 | 90% | 10% |
| 0.5 | 90% | 10% |
| 0.8 | 5% | 95% |
| 2.4 | 5% | 95% |
| 2.5 | 90% | 10% |
| 4.0 | Stop | |

3. Experimental results and analysis

Main pharmacokinetic parameters were calculated using WinNonlin 8.2. The results of the rat pharmacokinetic experiment are shown in the table below:

Table 5: Pharmacokinetic parameters of oral administration of the compound of the present invention in rats

| Parameters | tmax(h) | Cmax(ng/mL) | AUC 0-t (ng/mL*h) | AUC 0-∞ (ng/mL*h) | t1/2(h) | MRT 0-∞ (h) |
|---|---|---|---|---|---|---|
| Example 25 | 6.0 | 1963 | 28488 | 37268 | 10.5 | 16.4 |

(continued)

| Parameters | tmax(h) | Cmax(ng/mL) | AUC 0-t (ng/mL*h) | AUC 0-∞ (ng/mL*h) | t1/2(h) | MRT 0-∞ (h) |
|---|---|---|---|---|---|---|
| Example 35 | 4.7 | 1111 | 12387 | 14093 | 7.2 | 11.5 |
| Example 282 | 6.0 | 971 | 12151 | 13888 | 7.3 | 12.0 |

The example compounds of the present invention exhibited good metabolic properties, with both the exposure AUC and the maximum blood drug concentration $C_{max}$ being good.

**VII. Pharmacokinetic evaluation test in rats**

1. Research purpose:

Beagles were used as test animals to study the pharmacokinetic behavior of the compound of the present invention, administered orally at a dose of 2 mg/kg, in beagles (plasma).

2. Experimental regime:

2.1 Experimental drug:

Example compounds of the present invention, prepared in house.

2.2 Experimental animals:

Beagles, 3 per group, male.

2.3 Formulation prescription:

Preparation of drug for oral administration: 10%PEG400/10%PG/80% glycine buffer (100 mM glycine, 64 Mm NaOH, pH 10) buffer.

10 ml of PEG400, 10 ml of PG, and 80 ml of (100 mM glycine, 64 Mm NaOH, pH = 9) were measured into a 100 ml volumetric flask, vortexed, mixed homogeneously, and sonicated to obtain a clear solution.

Single PK: The compound of the present invention was weighed into a 100 mL glass bottle, and the solution was added. The pH was first adjusted with 1 M NaOH until the compound dissolves to obtain a clear solution, and then the pH was readjusted to pH 9.0-9.5 with 1 M HCl. Sonication was performed for 10 min to obtain a colorless clear solution having a concentration of 0.4 mg/mL.

2.4 Administration:

Three beagles, male, respectively administered p.o. after fasting overnight, at a dose of 2 mg/kg and in an administration volume of 5 mL/kg.

2.5 Sample collection:

Blood collection: 0.3 mL of blood was collected from the forelimb vein of the beagles before administration and at 0.25, 0.5, 1, 2, 4, 6, 8, and 24 h after administration. The blood samples were placed in EDTA-$K_2$ anticoagulant tubes and centrifuged at 6000 rpm at 4°C for 6 min to separate plasma, which was stored at -80°C. The beagles were fed 4 h after administration.

2.6 Sample treatment:

1) 40 μL of a plasma sample was added with 160 μL of acetonitrile for precipitation, and after mixing, the mixture was centrifuged at 3500 × g for 5 to 20 min.
2) The supernatant solution after treatment was taken for LC/MS/MS analysis of the concentration of the test compound. The LC/MS/MS analysis instrument was AB Sciex API 4000 Qtrap.

2.7 Liquid phase analysis:

• Liquid phase condition: Shimadzu LC-20AD pump
• Chromatographic column: Agilent ZORBAX XDB-C18 (50×2.1 mm, 3.5 μm) Mobile phase: Solution A was 0.1% formic acid aqueous solution, and Solution B was acetonitrile
• Flow rate: 0.4 mL/min
• Elution time: 0-4.0 min, the eluent was as follows:

| Time/min | Solution A | Solution B |
|---|---|---|
| 0.01 | 90% | 10% |
| 0.5 | 90% | 10% |
| 0.8 | 5% | 95% |
| 2.4 | 5% | 95% |
| 2.5 | 90% | 10% |
| 4.0 | Stop | |

3. Experimental results and analysis

The results of the beagle pharmacokinetic experiment are shown in Table 6 below:

Table 6: Pharmacokinetic parameters of oral administration of the compound of the present invention in beagles

| Parameters | tmax(h) | Cmax(ng/mL) | AUC 0-t (ng/mL*h) | AUC 0-∞ (ng/mL*h) | t1/2(h) | MRT 0-∞ (h) |
|---|---|---|---|---|---|---|
| Example 34 | 1.7 | 1330 | 9088 | 9387 | 5.1 | 5.7 |
| Example 65-1 | 2.8 | 4362 | 40996 | 43882 | 5.9 | 8.1 |
| Example 67 | 1.5 | 2111 | 11497 | 11782 | 4.2 | 5.7 |
| Example 143 | 2.7 | 2044 | 13798 | 17797 | 3.3 | 4.8 |
| Example 144 | 1.7 | 2473 | 15894 | 16614 | 5.8 | 6.5 |
| Example 194 | 1.0 | 5830 | 40130 | 43742 | 7.6 | 8.3 |
| Example 282 | 2.0 | 858 | 9131 | 10795 | 8.5 | 12.1 |
| Example 285 | 1.5 | 3597 | 19167 | 19417 | 4.1 | 5.3 |
| Example 287 | 2.3 | 1880 | 17008 | 17250 | 3.7 | 6.1 |
| Example 338 | 1.3 | 3915 | 20922 | 21417 | 4.4 | 5.3 |

**VIII. Pharmacokinetic evaluation test in Macaca fascicularis monkeys**

**Oral administration**

1. Research purpose:

Macaca fascicularis monkeys were used as test animals to study the pharmacokinetic behavior of the compound of the present invention, administered orally at a dose of 5 mg/kg, in Macaca fascicularis monkeys (plasma).

2. Experimental regime:

2.1 Experimental drug:

Example compounds of the present invention, prepared in house.

2.2 Experimental animals:

Macaca fascicularis monkeys, 3 per group, male.

2.3 Formulation prescription:

Preparation of drug for oral administration: 10%PEG400/10%PG/80% glycine buffer (100 mM glycine, 64 Mm NaOH, pH 10) buffer.
10 ml of PEG400, 10 ml of PG, and 80 ml of (100 mM glycine, 64 Mm NaOH, pH = 9) were measured into a 100 ml volumetric flask, vortexed, mixed homogeneously, and sonicated to obtain a clear solution.
Single PK: The compound of the present invention was weighed into a 100 mL glass bottle, and the solution was added. The pH was first adjusted with 1 M NaOH until the compound dissolves to obtain a clear solution, and then the pH was readjusted to pH 9.0-9.5 with 1 M HCl. Sonication was performed for 10 min to obtain a colorless clear solution having a concentration of 1 mg/mL.

2.4 Administration:

Three Macaca fascicularis monkeys, male, respectively administered p.o. after fasting overnight, at a dose of 5 mg/kg and in an administration volume of 5 mL/kg.

2.5 Sample collection:

Blood collection: 0.3 mL of blood was collected from the forelimb vein of the Macaca fascicularis monkeys before administration and at 0.25, 0.5, 1, 2, 4, 6, 8, and 24 h after administration. The blood samples were placed in EDTA-$K_2$ anticoagulant tubes and centrifuged at 6000 rpm at 4°C for 6 min to separate plasma, which was stored at -80°C. The Macaca fascicularis monkeys were fed 4 h after administration.

2.6 Sample treatment:

1) 40 μL of a plasma sample was added with 160 μL of acetonitrile for precipitation, and after mixing, the mixture was centrifuged at 3500 × g for 5 to 20 min.
2) The supernatant solution after treatment was taken for LC/MS/MS analysis of the concentration of the test compound. The LC/MS/MS analysis instrument was AB Sciex API 4000 Qtrap.

2.7 Liquid phase analysis:

• Liquid phase condition: Shimadzu LC-20AD pump
• Chromatographic column: Agilent ZORBAX XDB-C18 (50×2.1 mm, 3.5 μm) Mobile phase: Solution A was 0.1% formic acid aqueous solution, and Solution B was acetonitrile
• Flow rate: 0.4 mL/min
• Elution time: 0-4.0 min, the eluent was as follows:

| Time/min | Solution A | Solution |
|---|---|---|
| 0.01 | 90% | 10% |
| 0.5 | 90% | 10% |
| 0.8 | 5% | 95% |
| 2.4 | 5% | 95% |
| 2.5 | 90% | 10% |
| 4.0 | Stop | |

3. Experimental results and analysis

The results of the Macaca fascicularis monkey pharmacokinetic experiment are shown in Table 7 below:

Table 7: Pharmacokinetic parameters of oral administration of the compound of the present invention in Macaca fascicularis monkeys

| Parameters | tmax(h) | Cmax(ng/mL) | AUC 0-t (ng/mL*h) | AUC 0-∞ (ng/mL*h) | t1/2(h) | MRT 0-∞ (h) |
|---|---|---|---|---|---|---|
| Example 65-1 | 2.0 | 4458 | 29678 | 30783 | 5.6 | 7.5 |
| Example 143 | 2.7 | 4044 | 26020 | 26180 | 3.2 | 5.1 |
| Example 282 | 4.0 | 570 | 7557 | 17047 | 47.6 | 68.9 |

**Intravenous administration**

1. Research purpose:

Macaca fascicularis monkeys were used as test animals to study the pharmacokinetic behavior of the compound of the present invention, administered intravenously at a dose of 0.2 mg/kg, in Macaca fascicularis monkeys (plasma).

2. Experimental regime:

2.1 Experimental drug:

Example compounds of the present invention, prepared in house.

2.2 Experimental animals:

Macaca fascicularis monkeys, 3 per group, male, Guangxi Xiongsen, Animal Production License No.: SCXK (Gui) 2021-0004).

2.3 Formulation prescription:

Preparation of drug for intravenous administration: 5%DMSO+10%Solutol HS15+85%PBS

The compound of the present invention was weighed. 5% DMSO was first added according to the proportion in the total volume, followed by vortexing and sonication for 2 min to achieve complete dissolution. Then 10% Solutol HS15 was added, followed by vortexing and sonication for 2 min to achieve complete dissolution. Finally, 85% PBS was added, followed by vortexing and sonication for 5 min and filtering through a 0.22 um filter membrane to obtain a colorless, transparent, clear solution having a concentration of 0.1 mg/mL.

2.4 Administration:

Three Macaca fascicularis monkeys, male, respectively administered I.V. after fasting overnight, at a dose of 0.2 mg/kg and in an administration volume of 2 mL/kg.

2.5 Sample collection:

0.5 mL of blood was collected from the forelimb vein of the Macaca fascicularis monkeys before administration and at 0.083, 0.25, 0.5, 1, 2, 4, 8, and 24 h after administration. The blood samples were placed in EDTA-$K_2$ tubes and centrifuged at 6000 rpm at 4°C for 6 min to separate plasma, which was stored at -80°C.

2.6 Sample treatment:

40 μL of a plasma sample was added with 160 μL of acetonitrile for precipitation, and after mixing, the mixture was centrifuged at 3500 × g for 5 to 20 min.

100 μL of the supernatant solution after treatment was taken for LC/MS/MS analysis of the concentration of the test compound.

2.7 Liquid phase analysis:

Liquid phase condition: Shimadzu LC-20AD pump
Mass spectrometry condition: AB Sciex API 4000 mass spectrometer
Chromatographic column: phenomenex Gemiu 5 um C18 50 × 4.6 mm
Mobile phase: Solution A was 0.1% formic acid aqueous solution, and Solution B was acetonitrile
Flow rate: 0.8 mL/min
Elution time: 0-4.0 min, the eluent was as follows:

| Time/min | Solution | Solution B |
|---|---|---|
| 0.01 | 90% | 10% |
| 0.5 | 90% | 10% |
| 0.8 | 5% | 95% |
| 2.4 | 5% | 95% |
| 2.5 | 90% | 10% |
| 4.0 | Stop | |

3. Experimental results and analysis

Main pharmacokinetic parameters were calculated using WinNonlin 8.2. The results of the Macaca fascicularis monkey pharmacokinetic experiment are shown in Table 8 below:

Table 8: Pharmacokinetic parameters of injection administration of the compound of the present invention in Macaca fascicularis monkeys

| Parameters | C0(ng/mL) | AUC 0-t (ng/mL *h) | AUC 0-∞ (ng/mL*h) | t1/2(h) | MRT 0-∞ (h) |
|---|---|---|---|---|---|
| Example 282 | 1076 | 1676 | 1929 | 9.8 | 10.3 |

**IX. Effects of long-term administration of the compound of the present invention on the body weight and food intake of GLP-1R humanized mice fed with HFD high-fat diet**

1. Experimental purpose:

The purpose of this assay was to evaluate the effects of long-term administration of the compound on the body weight and food intake of GLP-1R humanized C57BL/6 mice fed with high-fat diet.

2. Experimental reagents and instruments

C57BL/6_*hGLP-1R,* male, 60% high-fat diet (HFD), ultra-clean bench, electronic balance.

3. Experimental methods

3.1 On the day of starting feeding the high-fat diet, the C57BL/6 mice were randomly grouped according to body weight. The first group was Blank, with 7 mice, and was fed with normal control diet. The remaining animals were the modeling group, and were fed with the high-fat diet until the end of the experiment.
3.2 In week 8 of HFD feeding, the animals in the modeling group were randomly grouped according to body weight, with 7 mice in each group. The first group was Vehicle group (Vehicle: 10%PEG400/10%PG/80% glycine buffer (100 mM glycine, 26 mM NaOH, pH 9) buffer), and was administered solvent; the remaining group was administration group. Administration regime: the corresponding compound was orally administered for a period of 21 days, once each day, the administration dose being 10 mg/kg, and the administration volume being 10 mL/kg. The Blank group continued to be fed with the normal diet, and was orally administered solvent once each day.
3.3 The day of administration was defined as Day 0.

3.4 The animals were weighed at each administration and the data were recorded. Oral administration was performed according to body weight, and the administration volume was 10 mL/kg.

3.5 Starting from day 0 of the experiment, the food intake of the mice in each group was measured every three days. Specifically, after each weighing and administration, the diet was renewed, and the added amount and remaining amount were recorded.

3.7 On Day 21, the end day, all mice were euthanized sequentially according to grouping order. Plasma and tissues (liver, kidney, ileum, pancreas, and brain) were taken for PK determination, and serum was taken for blood biochemistry determination (ALT, AST, TG, TC, and LDL-C).

4. Experimental data processing and statistical analysis

The body weight and body weight change rate of the mice after administration were summarized and statistically analyzed. The body weight change rate was calculated according to (BWt - BW0) / BW0 × 100%, where BWt represents mouse body weight on day t of the experiment, and BW0 represents mouse body weight on day 0 of the experiment.
Food intake was calculated according to (added amount (g) - remaining amount (g)) / number of animals per cage. The cumulative food intake was the sum of the daily food intake of each animal during the administration period.
The experimental data were analyzed and plotted using GraphPad Prism software. Comparison between two groups was performed using the t-test method, and comparison among three or more groups was performed using the one-way ANOVA method. $p<0.05$ was defined as the difference being statistically significant.
Long-term administration of example compounds of the present invention had a good weight-reducing effect on GLP-1R humanized C57BL/6 mice fed with the high-fat diet, with the weight-reducing rate greater than 5%, and more preferably greater than 10%.

## Claims

**1.** A compound represented by a general formula (XIX), a prodrug thereof, a stereoisomer thereof, or a pharmaceutically acceptable salt thereof:

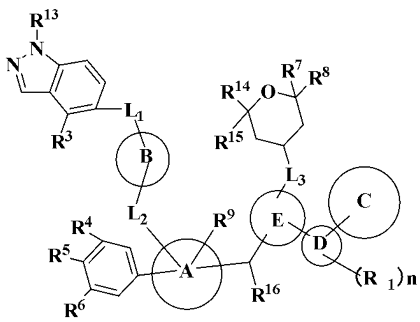

(XIX)

$R^{13}$ is selected from $C_{3-8}$ cycloalkyl, 3-8 membered heterocyclic, $C_{6-10}$ aryl, 5-10 membered heteroaryl, hydrogen, deuterium, halogen, amino, nitro, hydroxyl, cyano, oxo, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl or $C_{2-6}$ alkynyl, the $C_{3-8}$ cycloalkyl, 3-8 membered heterocyclic, $C_{6-10}$ aryl, 5-10 heteroaryl, amino, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl or $C_{2-6}$ alkynyl are optionally further substituted by one or more substituents selected from deuterium, halogen, amino, nitro, hydroxyl, cyano, oxo, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;
$L_1$, $L_2$ or $L_3$ are each independently selected from a bond, $C_{2-4}$ alkenylene, $C_{2-4}$ alkynylene or $-(CH_2)_{n1}-$;
ring A is selected from $C_{3-14}$ cycloalkyl, 3-14 membered heterocyclyl, $C_{6-14}$ aryl or 5-14 membered heteroaryl;
ring B is selected from $C_{3-12}$ cycloalkyl, 3-12 membered heterocyclyl, $C_{6-14}$ aryl or 5-14 membered heteroaryl, which are optionally further substituted with one or more substituents selected from deuterium, halogen, amino, nitro, hydroxyl, cyano, oxo, thiol, thio, $C_{1-6}$ alkyl, $C_{1-6}$ deuterated alkyl, $C_{1-6}$ haloalkyl, $C_{1-6}$ deuterated haloalkyl,

$C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy or $C_{1-6}$ deuterated haloalkoxy;

ring C is selected from $C_{3-14}$ cycloalkyl, 3-14 membered heterocyclyl, $C_{6-14}$ aryl or 5-14 membered heteroaryl, which are optionally further substituted by one or more substituents selected from deuterium, halogen, amino, nitro, hydroxyl, cyano, oxo, thiol, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy or $C_{1-6}$ deuterated haloalkoxy;

ring D is selected from $C_{3-12}$ cycloalkyl, 3-12 membered heterocyclyl, $C_{6-14}$ aryl or 5-14 membered heteroaryl;

ring E is selected from $C_{3-14}$ cycloalkyl, 3-14 membered heterocyclyl, $C_{6-14}$ aryl or 5-14 membered heteroaryl, which are optionally further substituted with one or more substituents selected from deuterium, halogen, amino, nitro, hydroxy, cyano, oxo, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

$R^1$, $R^3$, $R^4$, $R^5$, $R^6$, $R^7$, $R^8$, $R^9$, $R^{14}$, $R^{15}$ or $R^{16}$ is each independently selected from hydrogen, deuterium, halogen, amino, nitro, hydroxyl, cyano, oxo, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, C3-8 cycloalkyl, 3-8 membered heterocyclyl, C6-10 aryl, 5-10 membered heteroaryl, -(CH2)$_{m1}$OR$_{c1}$ or (=C)R$_{c3}$R$_{c4}$, the amino, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl, 5-10 membered heteroaryl, $-(CH_2)_{m1}OR_{c1}$ or $(=C)R_{c3}R_{c4}$ are optionally further substituted by one or more of deuterium, halogen, amino, nitro, hydroxyl, cyano, oxo, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

$R_{c1}$, $R_{c3}$ or $R_{c4}$ is each independently selected from hydrogen, deuterium, halogen, amino, nitro, hydroxyl, cyano, oxo, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl, 5-10 membered heteroaryl, $-(CH_2)_{m2}OR_{c2}$, $-(CH_2)_{m3}C(O)NR_{a1}R_{b1}$ or $-(CH_2)_{m4}C(O)OR_{a2}$, the amino, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, C3-8 cycloalkyl, 3-8 membered heterocyclic, C6-10 aryl, 5-10 membered heteroaryl, $-(CH_2)_{m2}OR_{c2}$, - $(CH_2)_{m3}C(O)NR_{a1}R_{b1}$ or $-(CH_2)_{m4}C(O)OR_{a2}$, are optionally further substituted by one or more of deuterium, halogen, amino, nitro, hydroxyl, cyano, oxo, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

$R_{a1}$, $R_{a2}$, $R_{b1}$ or $R_{c2}$ is each independently selected from hydrogen, deuterium, halogen, amino, nitro, hydroxyl, cyano, oxo, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, the amino, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl are optionally further substituted by one or more of deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

alternatively, $R^9$ or $R^{16}$ are respectively linked to atoms connected thereto to form $C_{3-10}$ cycloalkyl, 3-10 membered heterocyclic, $C_{6-12}$ aryl or 5-12 membered heteroaryl;

preferably, $R^9$ or $R^{16}$ are linked to the atoms connected thereto to form $C_{3-6}$ cycloalkyl, 3-6 membered heterocyclic containing 1-3 atoms selected from N, O or S, $C_{6-10}$ aryl, or 5-8 membered heteroaryl containing 1-3 atoms selected from N, O or S;

more preferably, $R^9$ or $R^{16}$ are linked to the atoms connected thereto to form cyclopropyl;
alternatively, when n is not 1,
$R^1$ and $R^1$, $R^4$ and $R^5$, $R^5$ and $R^6$, $R^7$ and $R^8$ are linked to the atoms connected thereto to form $C_{3-10}$ cycloalkyl, 3-10 membered heterocyclic, $C_{6-12}$ aryl or 5-12 membered heteroaryl, which are optionally further substituted by one or more substituents selected from deuterium, halogen, amino, nitro, hydroxyl, cyano, oxo, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;
n, n1, ml, m2, m3 or m4 is each independently selected from 0, 1, 2, 3, 4 or 5.

**2.** The compound, the prodrug thereof, the stereoisomer thereof or the pharmaceutically acceptable salt thereof according to claim 1, **characterized in that** the general formula (XIX) is further represented by a general formula (XVIII):

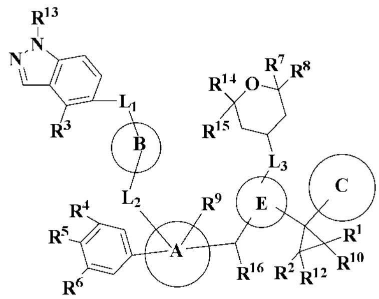

(XVIII)

wherein,

$R^1$, $R^2$, $R^3$, $R^4$, $R^5$, $R^6$, $R^7$, $R^8$, $R^9$, $R^{10}$, $R^{12}$, $R^{14}$, $R^{15}$ or $R^{16}$ is each independently selected from hydrogen, deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclyl, $C_{3-10}$ aryl, 5-8 $-(CH_2)_{m1}OR_{c1}$, the amino, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclic, $C_{6-10}$ aryl, 5-8 membered heteroaryl or $-(CH_2)_{m1}OR_{c1}$, are optionally further substituted by one or more substituents selected from deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl;
$R_{c1}$ is selected from hydrogen, deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclyl, $C_{6-10}$ aryl, 5-8 membered heteroaryl, $-(CH_2)_{m2}OR_{c2}$, $-(CH_2)_{m3}C(O)NR_{a1}R_{b1}$ or $-(CH_2)_{m4}C(O)OR_{a2}$, the amino, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, C3-6 cycloalkyl, 3-6 membered heterocyclic, C6-10 aryl, 5-8 membered heteroaryl, $-(CH_2)_{m2}OR_{c2}$, $-(CH_2)_{m3}C(O)NR_{a1}R_{b1}$ or $-(CH_2)_{m4}C(O)OR_{a2}$ are optionally further substituted by one or more substituents selected from deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl;
$R_{a1}$, $R_{a2}$, $R_{b1}$ or $R_{c2}$ are each independently selected from hydrogen, deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$

haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl, the amino, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, C3-6 cycloalkyl, 3-6 membered heterocyclic, C6-10 aryl or 5-8 membered heteroaryl are optionally further substituted with one or more substituents selected from deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl;

alternatively,

$R^1$ and $R^{10}$, $R^2$ and $R^{12}$, $R^4$ and $R^5$, $R^5$ and $R^6$, $R^7$ and $R^8$, $R^{10}$ and $R^{12}$ are linked to the atoms connected thereto to form $C_{3-6}$ cycloalkyl, 3-6 membered heterocyclic containing $_{1-3}$ atoms selected from N, O or S, $C_{6-10}$ aryl or 5-8 membered heteroaryl containing $_{1-3}$ atoms selected from N, O or S, which are optionally further substituted with one or more substituents selected from deuterium, halogen, amino, nitro, hydroxyl, cyano, oxo, thio, $C_{1-3}$ alkyl, $C_{1-3}$ deuterated alkyl, $C_{1-3}$ haloalkyl, $C_{1-3}$ deuterated haloalkyl, $C_{1-3}$ hydroxyalkyl, $C_{1-3}$ deuterated hydroxyalkyl, $C_{1-3}$ sulfanyl, $C_{1-3}$ deuterated sulfanyl, $C_{1-3}$ alkoxy, $C_{1-3}$ deuterated alkoxy, $C_{1-3}$ haloalkoxy, $C_{1-3}$ deuterated haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl;

preferably, $R^1$ and $R^{10}$, $R^2$ and $R^{12}$, $R^4$ and $R^5$, $R^5$ and $R^6$, $R^7$ and $R^8$, $R^{10}$ and $R^{12}$ are linked to the atoms connected thereto to form cyclopropyl,

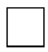、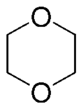、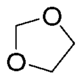、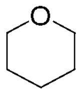、

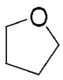、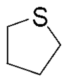 or 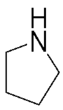,

which are optionally further substituted with one or more substituents selected from deuterium, fluorine, chlorine, bromine, amino, nitro, hydroxyl, cyano, oxo, thioxo, methyl or ethyl;

$R^{13}$, $L_1$, $L_2$, $L_3$, ring A, ring B, ring C, ring E are as set forth in claim 1;

n1, ml, m2, m3 or m4 is each independently selected from 0, 1, 2, 3, 4 or 5.

**3.** The compound, the prodrug thereof, the stereoisomer thereof or the pharmaceutically acceptable salt thereof according to claim 2, **characterized in that**,

$R^{13}$ is selected from $C_{3-8}$ cycloalkyl, 3-8 membered heterocyclic, $C_{6-10}$ aryl or 5-10 membered heteroaryl, $C_{1-6}$ deuterated alkyl, $C_{1-6}$ deuterated halogenated alkyl, $C_{1-6}$ deuterated hydroxyalkyl, $C_{1-6}$ deuterated alkoxy or $C_{1-6}$ deuterated halogenated alkoxy, the $C_{3-8}$ cycloalkyl, 3-8 membered heterocyclic, $C_{6-10}$ aryl, 5-10 membered heteroaryl, $C_{1-6}$ deuterated alkyl, $C_{1-6}$ deuterated halogenated alkyl, $C_{1-6}$ deuterated hydroxyalkyl, $C_{1-6}$ deuterated alkoxy or $C_{1-6}$ deuterated halogenated alkoxy are optionally further substituted with one or more substituents selected from deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

preferably, $R^{13}$ is selected from $C_{3-6}$ cycloalkyl, 3-6 membered heterocyclic, $C_{6-10}$ aryl, 5-8 membered heteroaryl, $C_{1-3}$ deuterated alkyl, $C_{1-3}$ deuterated halogenated alkyl, $C_{1-3}$ deuterated hydroxyalkyl, $C_{1-3}$ deuterated sulfanyl, $C_{1-3}$ deuterated alkoxy or $C_{1-3}$ deuterated halogenated alkoxy, the $C_{3-6}$ cycloalkyl, 3-6 membered heterocyclic, $C_{6-10}$ aryl, 5-8 membered heteroaryl, $C_{1-3}$ deuterated alkyl, $C_{1-3}$ deuterated halogenated alkyl, $C_{1-3}$ deuterated hydroxyalkyl, $C_{1-3}$ deuterated sulfanyl, $C_{1-3}$ deuterated alkoxy or $C_{1-3}$ deuterated halogenated alkoxy are optionally further substituted with one or more substituents selected from deuterium, halogen, amino, nitro, hydroxyl, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl;

more preferably, $R^{13}$ is selected from cyclopropyl or $-CD_3$, wherein the cyclopropyl or - $CD_3$ is optionally further

substituted with one or more substituents selected from deuterium, halogen, or $C_{1-3}$ alkyl;
further preferably, $R^{13}$ is selected from cyclopropyl, and the cyclopropyl is optionally further substituted by one or more substituents selected from deuterium, halogen, or $C_{1-3}$ alkyl.

**4.** The compound, the prodrug thereof, the stereoisomer thereof or the pharmaceutically acceptable salt thereof according to claim 2, **characterized in that** the general formula (XVIII) is further shown in a general formula (XVIII-A):

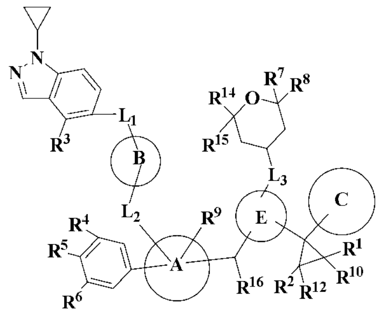

(XVIII-A)

alternatively, the general formula (XVIII) is further shown in a general formula (XVIII-B):

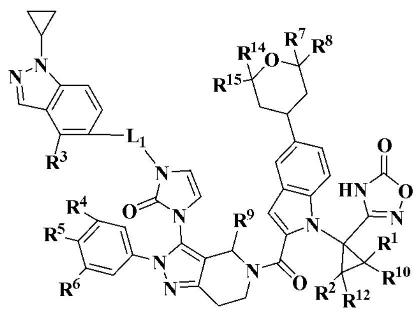

(XVIII-B)

wherein, ring A, ring B, ring C, ring E, $R^1$, $R^2$, $R^3$, $R^4$, $R^5$, $R^6$, $R^7$, $R^8$, $R^9$, $R^{10}$, $R^{12}$, $R^{14}$, $R^{15}$, $R^{16}$, L1, L2 and L3 are as defined in claim 2.

**5.** The compound, the prodrug thereof, the stereoisomer thereof or the pharmaceutically acceptable salt thereof according to claim 1, **characterized in that** the general formula (XIX) is further shown in a general formula (V):

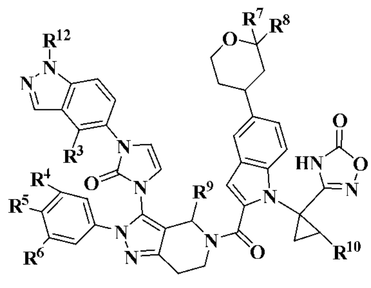

(V)

wherein,

$R^4$, $R^5$ or $R^6$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ sulfanyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, the amino, $C_{1-6}$ alkyl, $C_{1-6}$ deuterated alkyl, $C_{1-6}$ haloalkyl, $C_{1-6}$ hydroxyalkyl, $C_{1-6}$ sulfanyl, $C_{1-6}$ alkoxy, $C_{1-6}$

haloalkoxy, $C_{2-6}$ alkenyl, $C_{2-6}$ alkynyl, $C_{3-8}$ cycloalkyl, 3-8 membered heterocyclic, $C_{6-10}$ aryl and 5-10 membered heteroaryl are optionally further substituted with one or more substituents selected from halogen, amino, nitro, hydroxy, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl and 5-10 membered heteroaryl;

preferably, $R^4$, $R^5$ or $R^6$ is each independently selected from hydrogen, deuterium, halogen, amino, cyano, $C_{1-3}$ alkyl, $C_{1-3}$ sulfanyl, C2-4 alkynyl, C3-6 cycloalkyl, 3-6 membered heterocyclic containing $_{1-3}$ N, O or S atoms, which are optionally further substituted by one or more substituents selected from halogen, $C_{1-3}$ alkyl, $C_{1-3}$ deuterated alkyl and $C_{1-3}$ alkoxy;

more preferably, $R^4$, $R^5$ or $R^6$ is each independently selected from fluorine, methyl, cyano, ethynyl, methylthio, amino, cyclopropyl, oxetane, which are optionally further substituted by halogen, methoxy, deuterated methyl;

alternatively, $R^5$ and $R^6$ are linked to form $C_{3-8}$ cycloalkyl, 3-8 membered heterocyclic, $C_{6-10}$ aryl or 5-10 membered heteroaryl, the $C_{3-8}$ cycloalkyl, 3-8 membered heterocyclic, $C_{6-10}$ aryl and 5-10 membered heteroaryl are optionally further substituted by one or more of halogen, amino, nitro, hydroxy, $C_{1-6}$ cyanoalkyl, $C_{1-6}$ deuterated alkyl, $C_{1-6}$ haloalkyl, $C_{1-6}$ hydroxyalkyl, $C_{1-6}$ sulfanyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl and 5-10 membered heteroaryl;

$R^3$ is selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, 5-10 membered heteroaryl, $-NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$, the amino, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl and 5-10 membered heteroaryl are optionally further substituted by one or more of halogen, amino, nitro, hydroxyl, cyano, oxo, $-NS(O)R_{a1}R_{b1}$, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl and 5-10 membered heteroaryl;

$R_{a1}$, $R_{b1}$, $R_{a2}$, $R_{b2}$, $R_{a3}$, $R_{b3}$, $R_{a4}$, $R_{b4}$ or $R_{a5}$ is each independently selected from hydrogen, deuterium, halogen, amino, nitro, hydroxyl, cyano, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, 5-10 membered heteroaryl;

alternatively, $R_{a1}$ and $R_{b1}$, $R_{a2}$ and $R_{b2}$, $R_{a3}$ and $R_{b3}$, $R_{a4}$ and $R_{b4}$ are linked to form C3-8 cycloalkyl, 3-8 membered heterocyclic, $C_{6-10}$ aryl or 5-10 membered heteroaryl, the $C_{3-8}$ cycloalkyl, 3-8 membered heterocyclic, $C_{6-10}$ aryl and 5-10 membered heteroaryl are optionally further substituted by one or more substituents selected from halogen, aminonitrocyanocyano $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl and 5-10 membered heteroaryl;

preferably, $R_{a1}$ and $R_{b1}$, $R_{a2}$ and $R_{b2}$, $R_{a3}$ and $R_{b3}$, $R_{a4}$ and $R_{b4}$ are linked to form $C_{3-8}$ cycloalkyl, 3-6 membered heterocyclic, $C_{6-10}$ aryl or 5-8 membered heteroaryl;

$R^7$, $R^8$, $R^9$ or $R^{10}$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, which are optionally further substituted by one or more of halogen, amino, nitro, hydroxy, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl and 5-10 membered heteroaryl;

$R^{12}$ is selected from $C_{3-8}$ cycloalkyl, 3-8 membered heterocyclyl, $C_{6-10}$ aryl, 5-10 membered heteroaryl, hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl or $C_{2-6}$ alkynyl, which are optionally further substituted by one or more of halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, and 5-10 membered heteroaryl;

n1 is an integer of 0-5;

wherein when $R^7$ and $R^8$ are hydrogen, $R^9$, $R^{10}$, and $R^{12}$ are methyl, and $R^3$ and $R^5$ are fluorine, $R^4$ and $R^6$ are not methyl at the same time;

when $R^7$ and $R^8$ are hydrogen, $R^9$, $R^{10}$, and $R^{12}$ are methyl, $R^3$ is hydrogen, and $R^5$ is halogen, $R^4$ and $R^6$ are not methyl at the same time;

when $R^7$, $R^8$, $R^9$, $R^{10}$, and $R^{12}$ are methyl, $R^5$ is fluorine, and $R^4$ is halogen, trifluoromethyl, or cyclopropane, $R^6$ is not hydrogen;

when $R^7$, $R^8$, $R^9$, $R^{10}$ are methyl, $R^{12}$ is deuterated methyl, $R^3$ is fluorine, $R^5$ is halogen, $R^4$ is deuterated methyl, R6 is not deuterated methyl;

when $R^4$, $R^6$, $R^7$, $R^8$, $R^9$, $R^{10}$ are methyl, $R^3$, $R^5$ are fluorine, $R^{12}$ is not deuterated methyl;
when $R^7$, $R^8$, $R^9$, $R^{10}$, $R^{12}$ are methyl, $R^3$ is fluorine, $R^5$ is halogen, $R^4$ is methyl, $R^6$ is not methyl;
when $R^7$, $R^8$, $R^9$, $R^{10}$ are methyl, $R^{12}$ is deuterated methyl, $R^3$ is fluorine, $R^5$ is halogen, and $R^4$ is deuterated methyl, $R^6$ is not a deuterated methyl;
when $R^4$, $R^6$, $R^7$, $R^8$, $R^9$, $R^{10}$ are methyl, $R^3$ and $R^5$ are fluorine, $R^{12}$ is not deuterated methyl.

**6.** The compound, the prodrug thereof, the stereoisomer thereof or the pharmaceutically acceptable salt thereof according to claim 1, **characterized in that** the general formula (XIX) is further shown in a general formula (V):

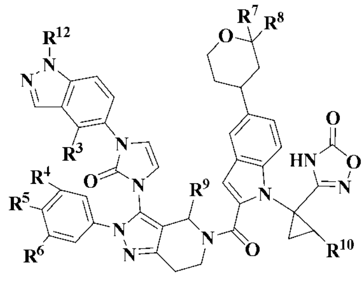

(V)

wherein,

$R^4$, $R^5$ or $R^6$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ sulfanyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, the amino, $C_{1-6}$ alkyl, $C_{1-6}$ deuterated alkyl, $C_{1-6}$ haloalkyl, $C_{1-6}$ hydroxyalkyl, $C_{1-6}$ sulfanyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl and 5-10 membered heteroaryl are optionally further substituted with one or more substituents selected from halogen, amino, nitro, hydroxy, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl and 5-10 membered heteroaryl;
preferably, $R^4$, $R^5$ or $R^6$ is each independently selected from hydrogen, deuterium, halogen, amino, cyano, $C_{1-3}$ alkyl, $C_{1-3}$ sulfanyl, C2-4 alkynyl, C3-6 cycloalkyl, 3-6 membered heterocyclic containing $_{1-3}$ N, O or S atoms, which are optionally further substituted by one or more substituents selected from halogen, $C_{1-3}$ alkyl, $C_{1-3}$ deuterated alkyl and $C_{1-3}$ alkoxy;
more preferably, $R^4$, $R^5$ or $R^6$ is each independently selected from fluorine, methyl, cyano, ethynyl, methylthio, amino, cyclopropyl, oxetane, which are optionally further substituted by halogen, methoxy, deuterated methyl;
alternatively, $R^5$ and $R^6$ are linked with adjacent atoms to form a $C_{3-8}$ cycloalkyl, a 3-8 membered heterocyclyl, a $C_{6-10}$ aryl or a 5-10 membered heteroaryl, the $C_{3-8}$ cycloalkyl, 3-8 membered heterocyclyl, $C_{6-10}$ aryl or 5-10 membered heteroaryl are optionally further substituted by one or more substituents of halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ sulfanyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;
$R^3$ is selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, 5-10 membered heteroaryl, $-NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$, $-(CH_2)_{n2}R_{a5}R_{b5}$ or

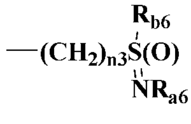,

the amino, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl are optionally further substituted by one or more substituents selected from halogen, amino, nitro, hydroxy, cyano, oxo, $-NS(O)R_{a1}R_{b1}$, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered

heteroaryl;

$R_{a1}$, $R_{b1}$, $R_{a2}$, $R_{b2}$, $R_{a3}$, $R_{b3}$, $R_{a4}$, $R_{b4}$, $R_{a5}$, $R_{b5}$, $R_{a6}$, or $R_{b6}$ is each independently selected from hydrogen, deuterium, halogen, amino, nitro, hydroxyl, cyano, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, 5-10 membered heteroaryl;

alternatively, $R_{a1}$ and $R_{b1}$, $R_{a2}$ and $R_{b2}$, $R_{a3}$ and $R_{b3}$, $R_{a4}$ and $R_{b4}$, $R_{a5}$ and $R_{b5}$, $R_{a6}$ and $R_{b6}$ are linked to adjacent atoms to form $C_{3-8}$ cycloalkyl, 3-8 membered heterocyclyl, $C_{6-10}$ aryl or 5-10 membered heteroaryl, and the $C_{3-8}$ cycloalkyl, 3-8 membered heterocyclyl, $C_{6-10}$ aryl or 5-10 membered heteroaryl, which are optionally further substituted by one or more substituents of halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

$R^7$, $R^8$, $R^9$ or $R^{10}$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, 5-10 membered heteroaryl or $-(CH_2)_{m1}OR_{c1}$, which are optionally further substituted with one or more substituents selected from halogen, amino, nitro, hydroxy, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

preferably, $R^7$, $R^8$, $R^9$ or $R^{10}$ is each independently selected from hydrogen, deuterium, halogen, amino, nitro, hydroxyl, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, C3-6 cycloalkyl, 3-6 membered heterocyclyl, $C_{6-10}$ aryl, 5-8 membered heteroaryl or $-(CH_2)_{m1}OR_{c1}$, which are optionally further substituted by one or more substituents selected from halogen, amino, nitro, hydroxy, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl;

$R_{c1}$ is selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ sulfanyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, or 5-10 membered heteroaryl, the amino, $C_{1-6}$ alkyl, $C_{1-6}$ deuterated alkyl, $C_{1-6}$ haloalkyl, $C_{1-6}$ hydroxyalkyl, $C_{1-6}$ sulfanyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl are optionally further substituted with one or more substituents selected from halogen, amino, nitro, hydroxy, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

preferably, $R_{c1}$ is selected from hydrogen, deuterium, halogen, amino, nitro, hydroxyl, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ sulfanyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl, which are optionally further substituted with one or more substituents selected from halogen, amino, nitro, hydroxy, cyano, oxo, $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ haloalkoxy, $C_{2-4}$ alkenyl, $C_{2-4}$ alkynyl, $C_{3-6}$ cycloalkyl, 3-6 membered heterocyclyl, $C_{6-10}$ aryl or 5-8 membered heteroaryl;

alternatively, $R^7$, $R^8$, $R^9$ or $R^{10}$ is linked to the atoms connected thereto to form $C_{3-10}$ cycloalkyl, 3-10 membered heterocyclyl, $C_{6-12}$ aryl, or a 5-12 membered heteroaryl;

$R^{12}$ is selected from $C_{3-8}$ cycloalkyl, 3-8 membered heterocyclyl, $C_{6-10}$ aryl, 5-10 membered heteroaryl, hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl or $C_{2-6}$ alkynyl, $-(CH_2)_{n2}R_{a5}R_{b5}$ or

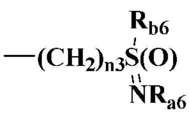,

which are optionally further substituted by one or more of halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, and 5-10 membered heteroaryl;

alternatively,

$R^{10}$ is linked to the atoms connected thereto and the adjacent atoms to form $C_{3-10}$ cycloalkyl, 3-10 membered heterocyclic, $C_{6-12}$ aryl, or 5-12 membered heteroaryl;

preferably, $R^{10}$ is linked to the atoms connected thereto and the adjacent atoms to form $C_{3-6}$ cycloalkyl, 3-6 membered heterocyclic, $C_{6-10}$ aryl or a 5-8 membered heteroaryl;

more preferably, $R^{10}$ is linked to the atoms connected thereto and the adjacent atoms to form

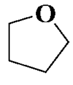;

n1-n3 are integers from 0 to 5;
m1 is an integer from 0 to 5.

**7.** The compound, the prodrug thereof, the stereoisomer thereof or the pharmaceutically acceptable salt thereof according to claim 1, wherein the compound has a structure as follows:

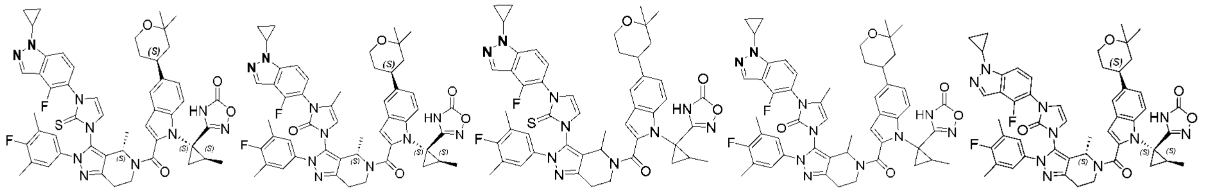

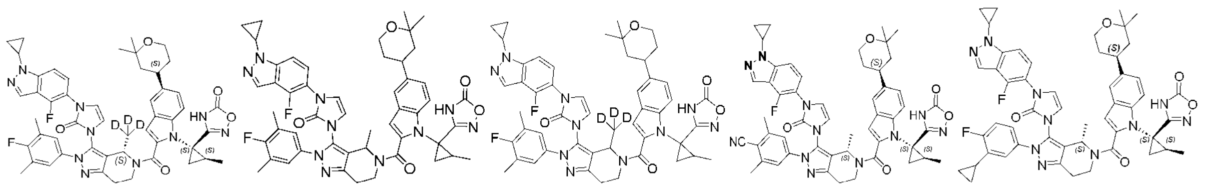

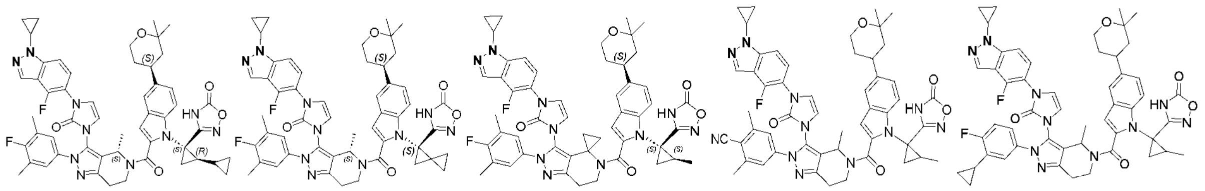

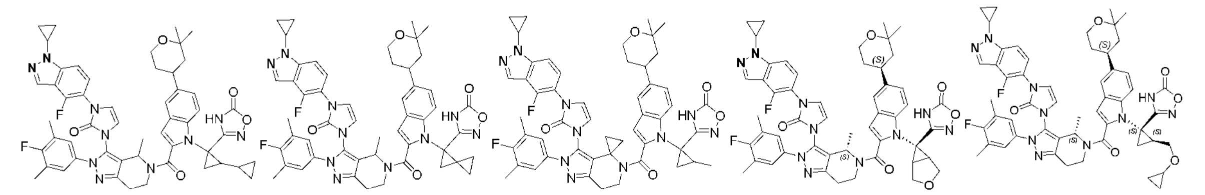

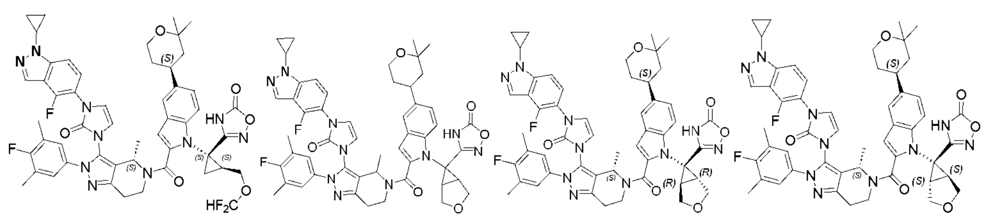

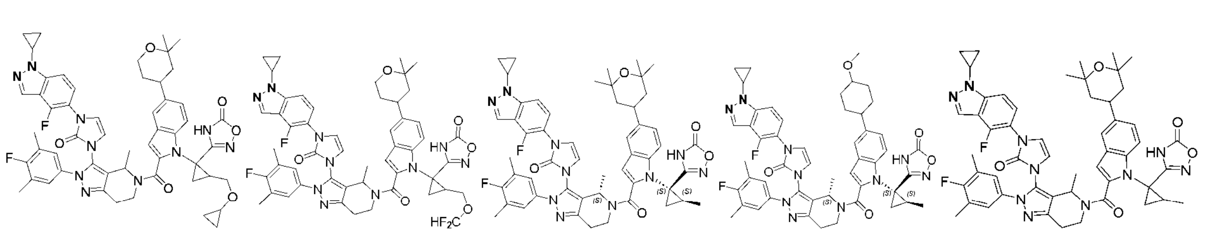

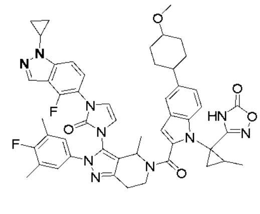
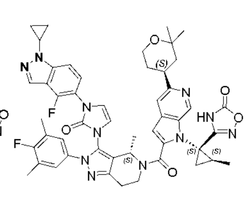
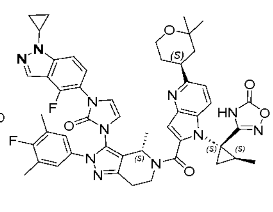
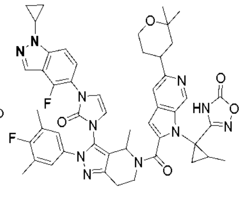

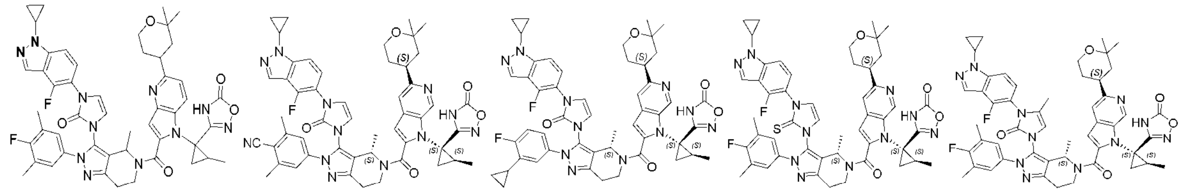

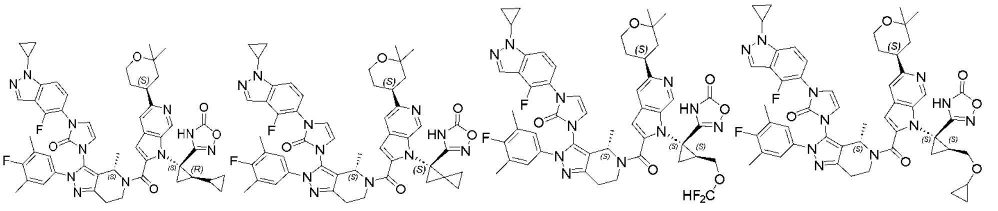

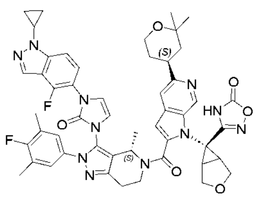
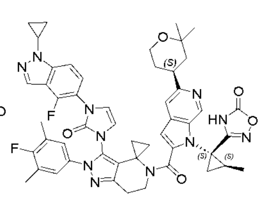
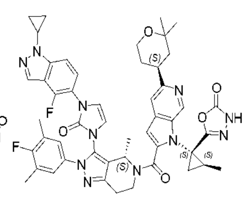
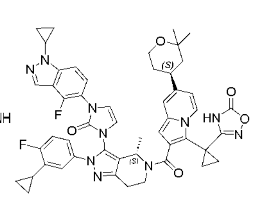

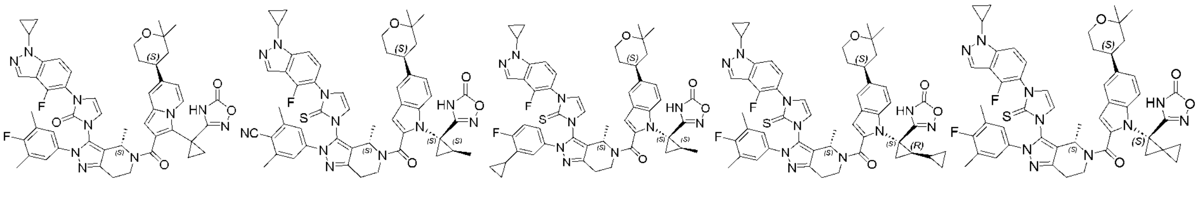

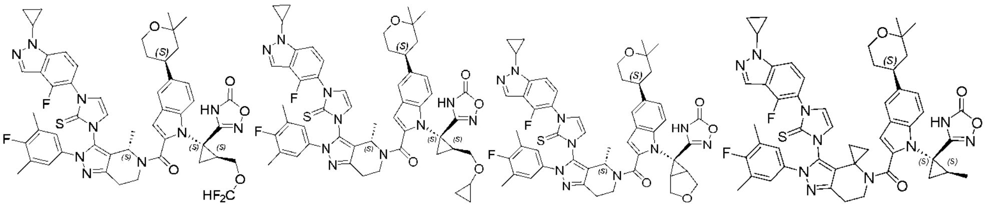

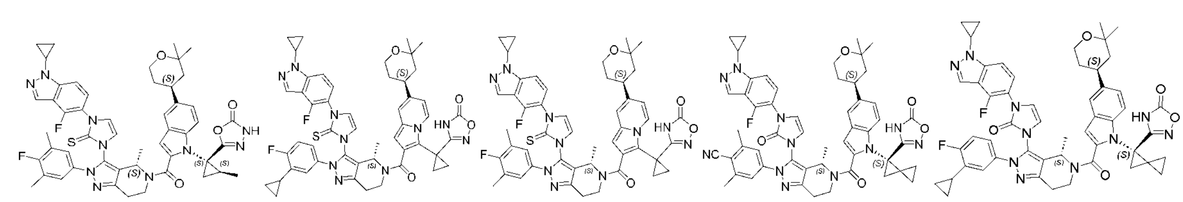

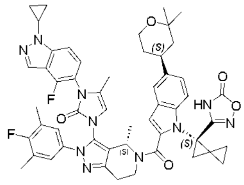
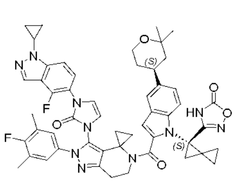
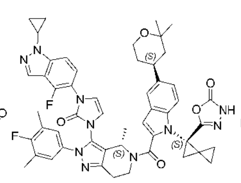
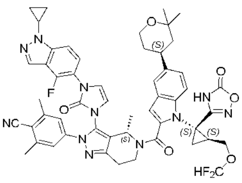
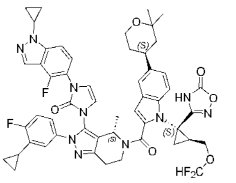

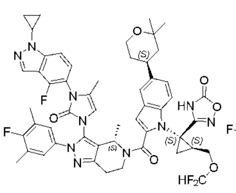
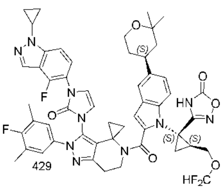
429
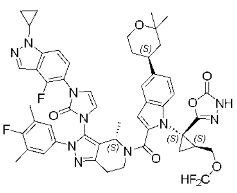
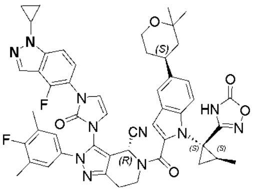
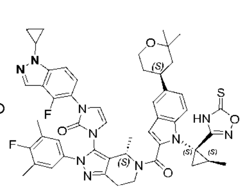

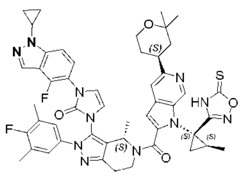
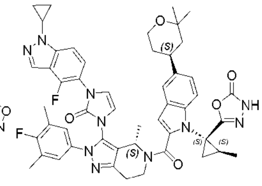
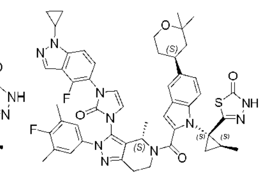
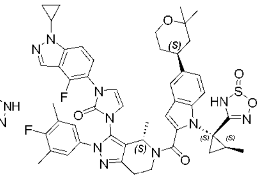
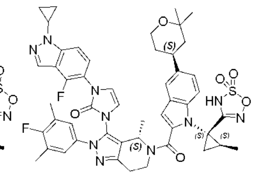

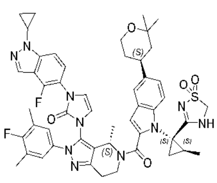
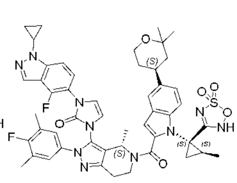
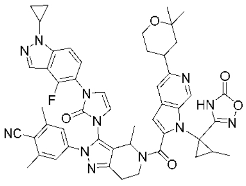
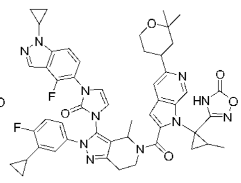
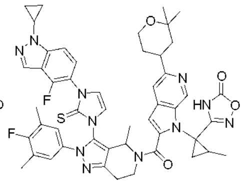

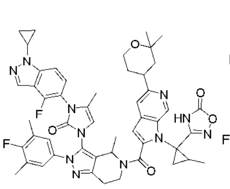
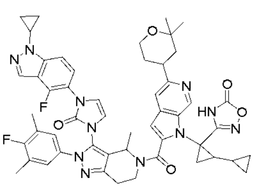
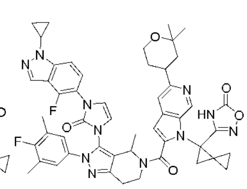
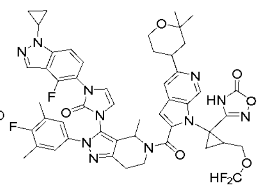
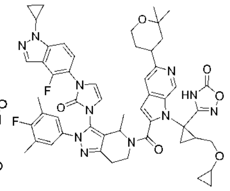

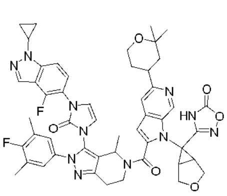
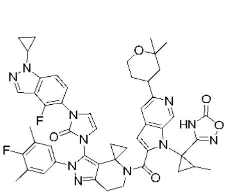
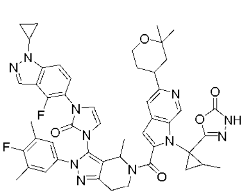
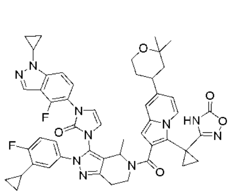
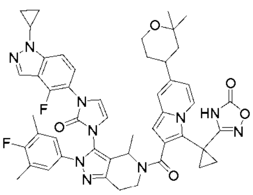

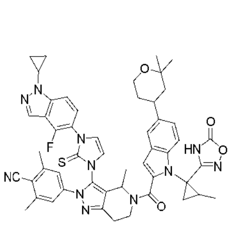
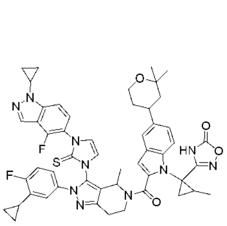
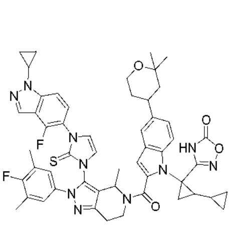
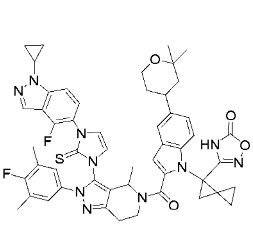
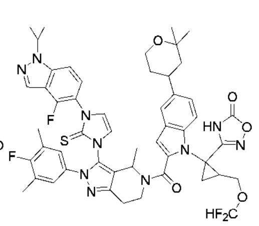

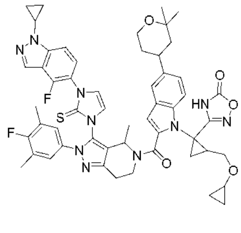 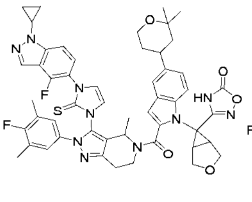 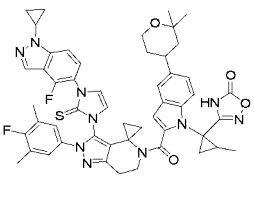 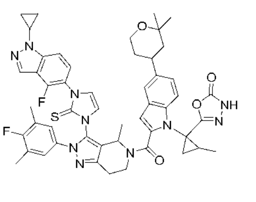

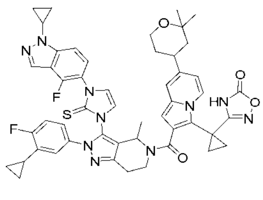 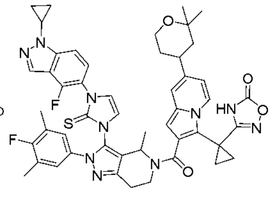 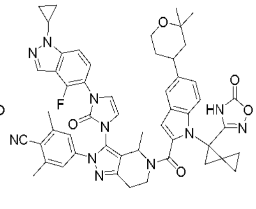 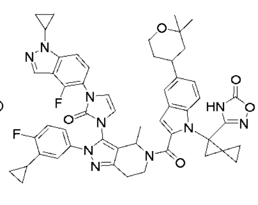

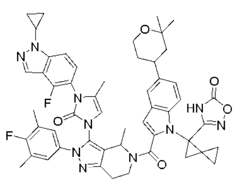 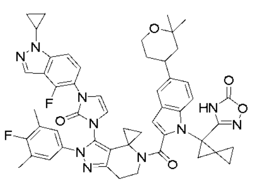 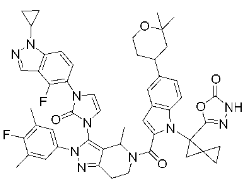 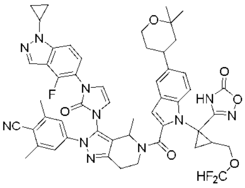 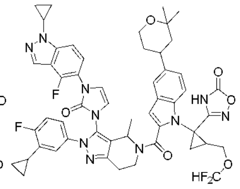

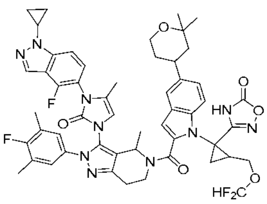 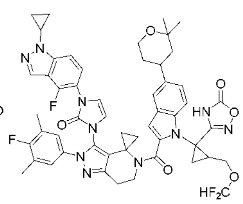 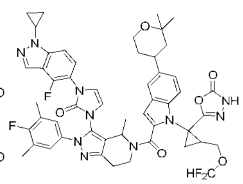 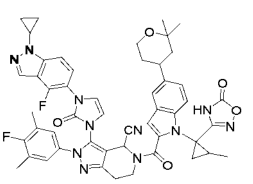

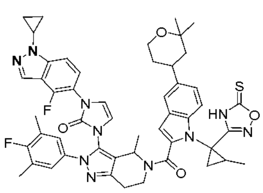 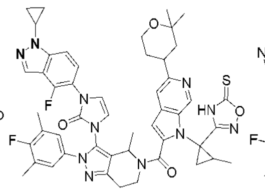 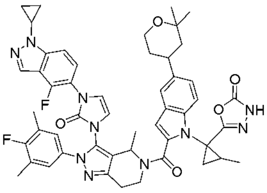 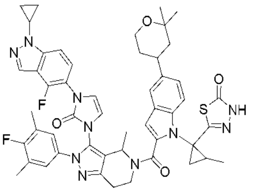

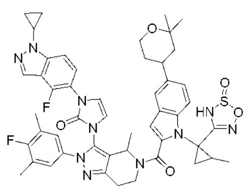 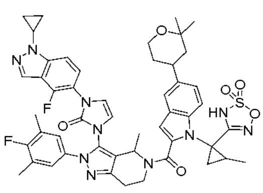 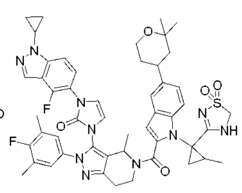 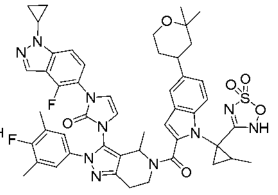

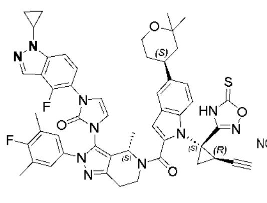 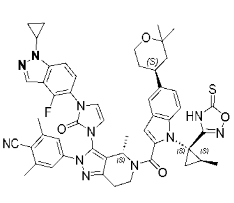 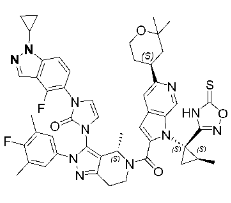 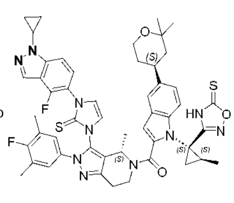 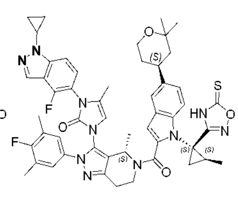

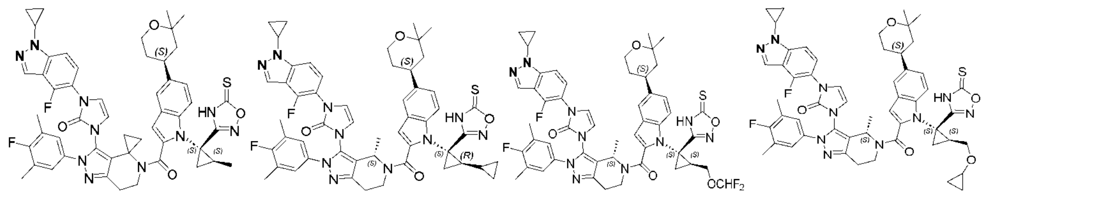

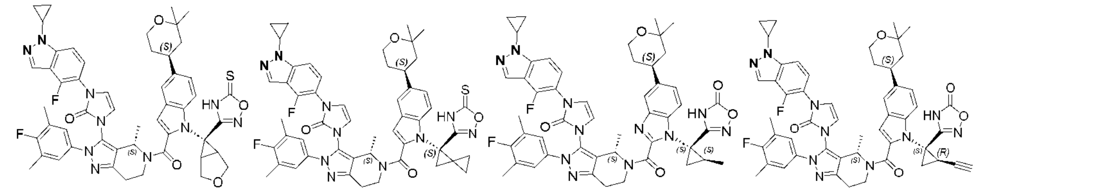

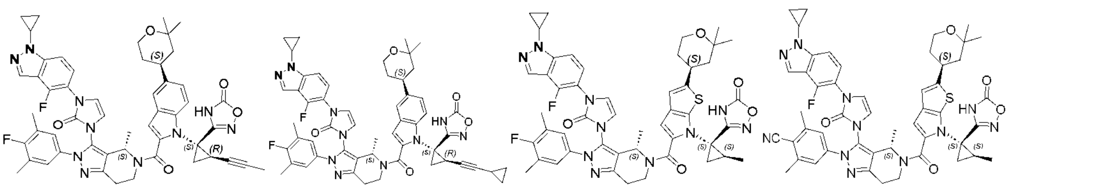

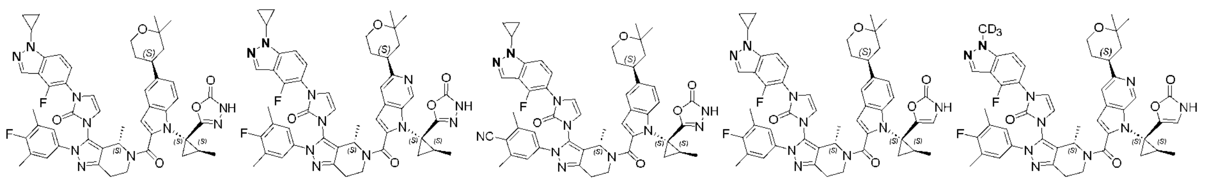

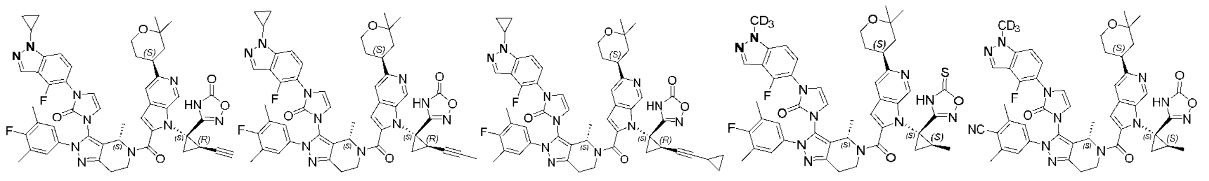

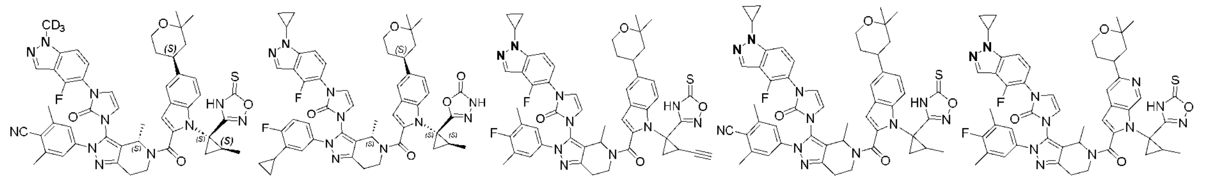

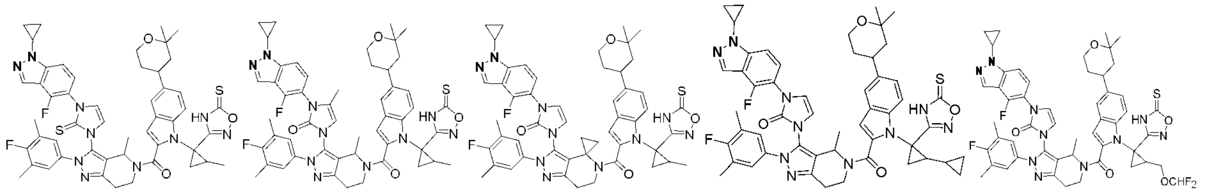

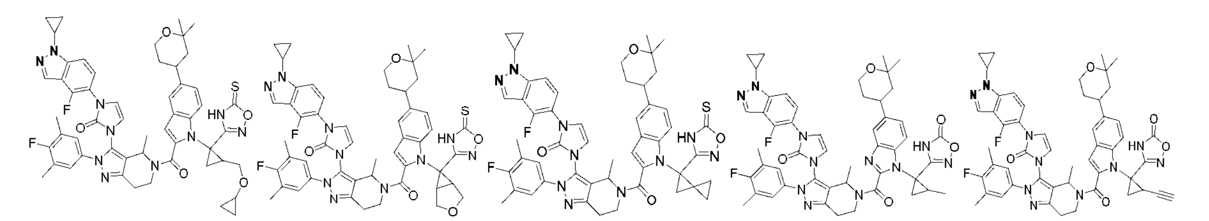

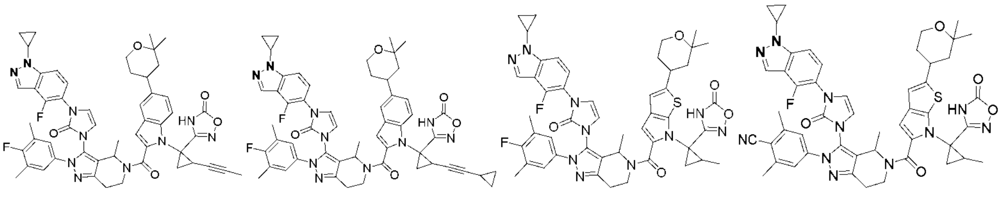

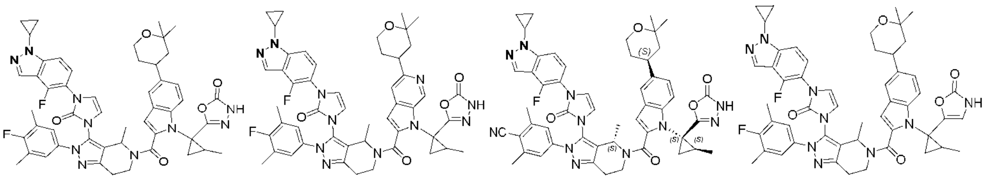

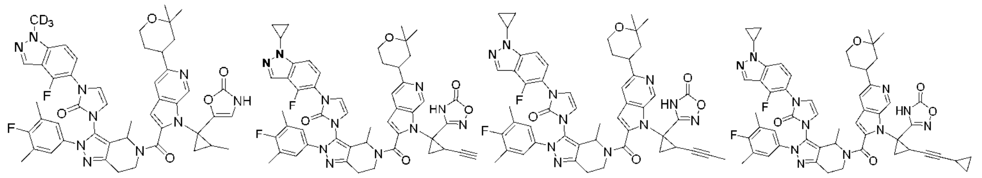

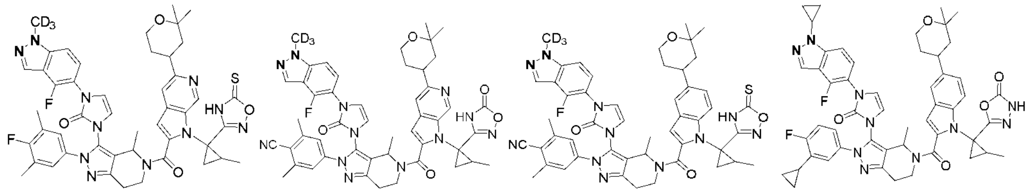

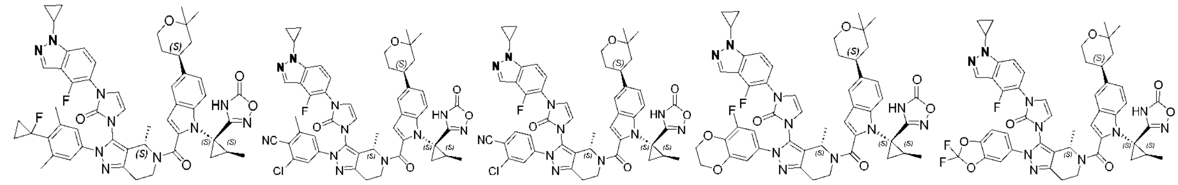

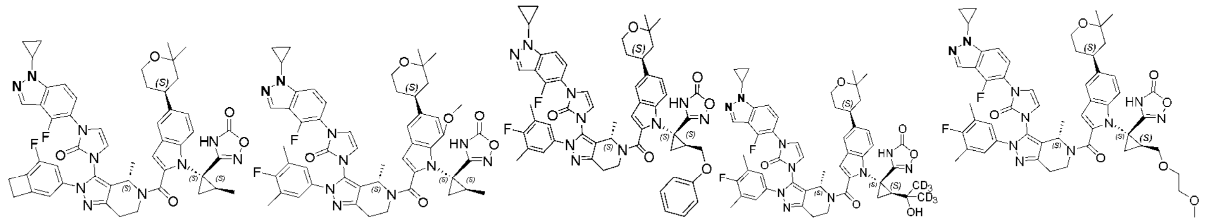

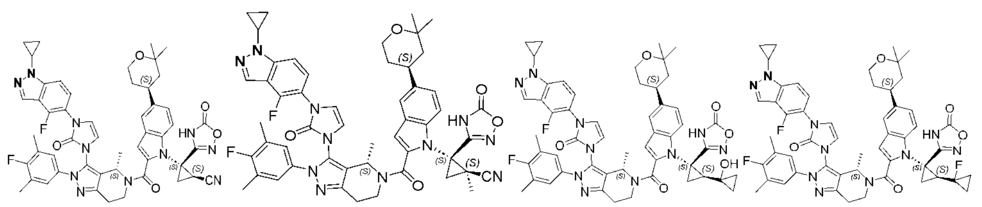

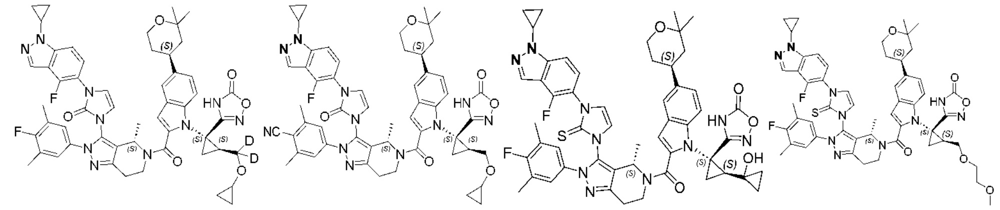

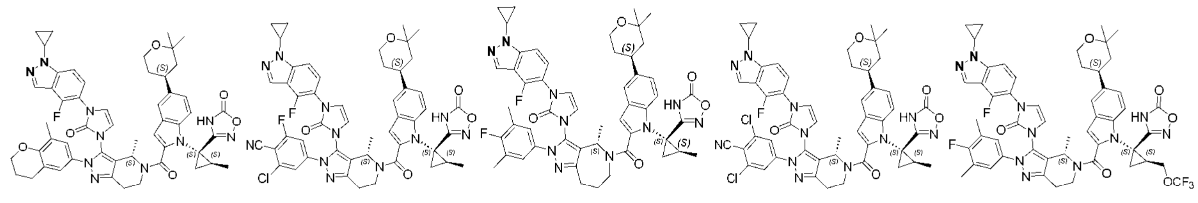

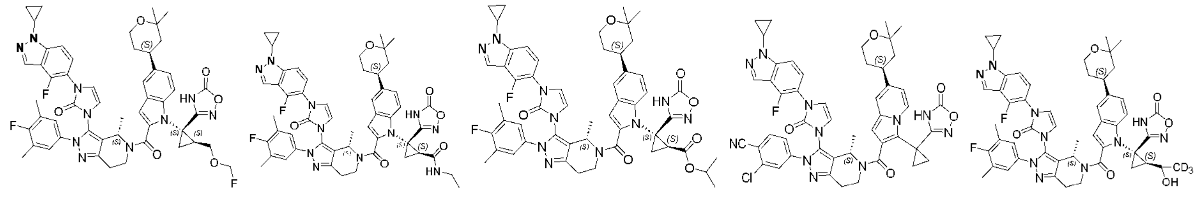

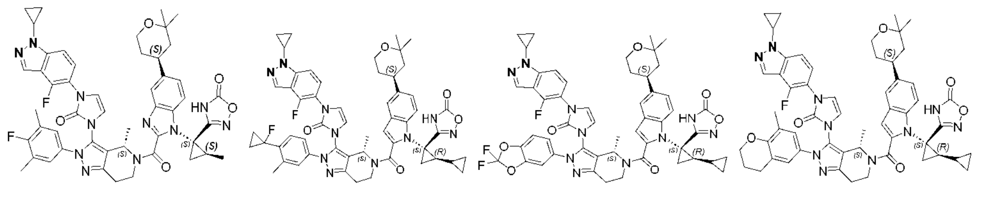

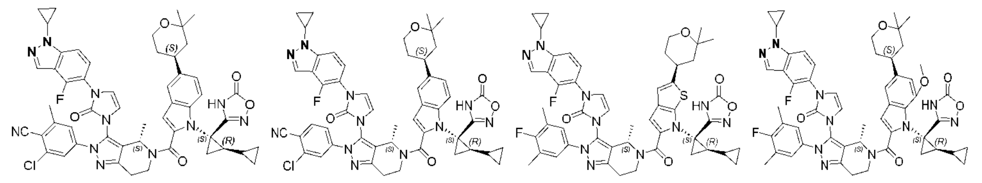

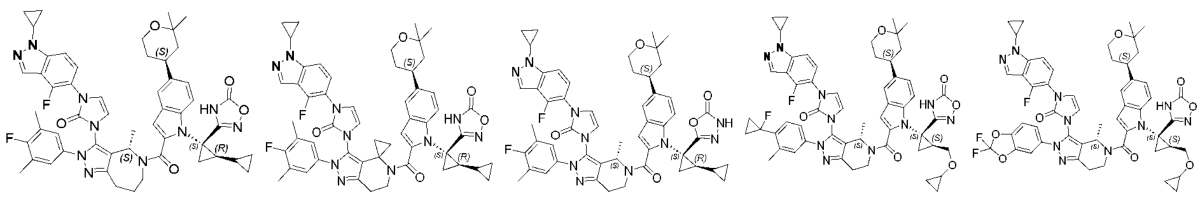

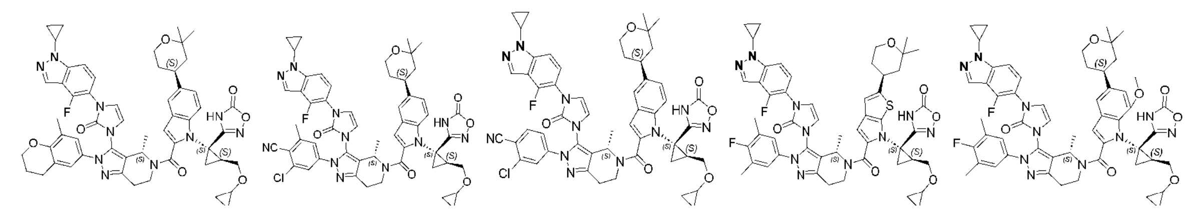

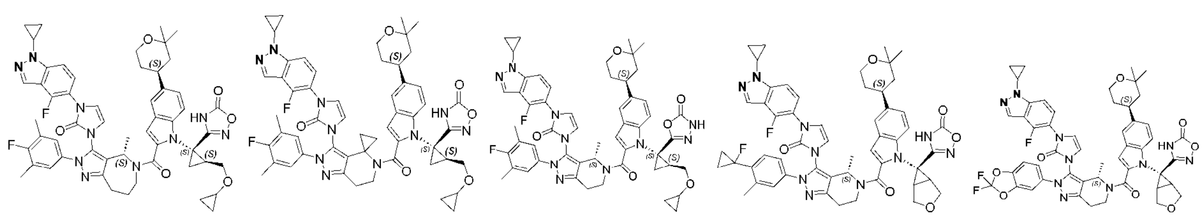

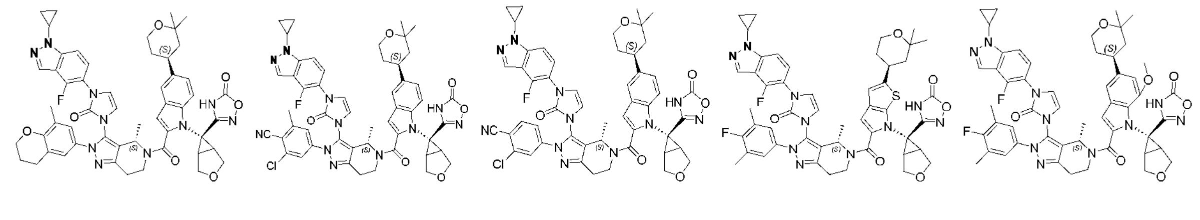

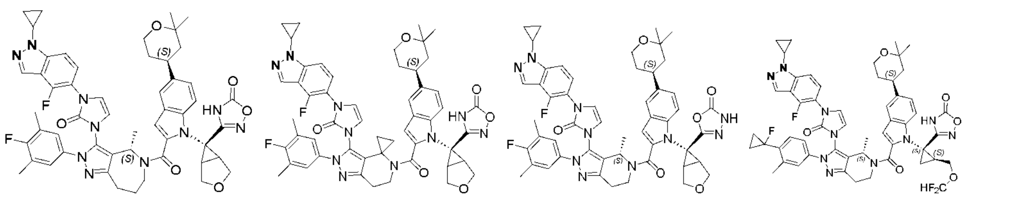

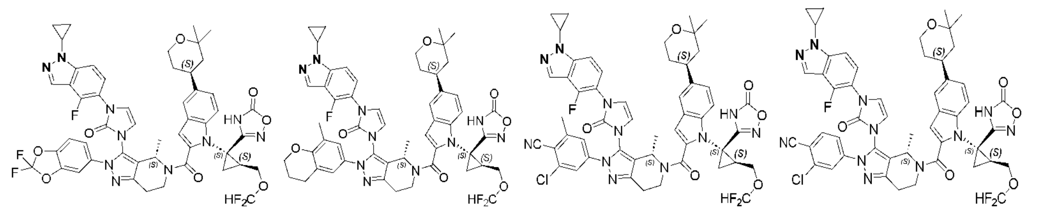

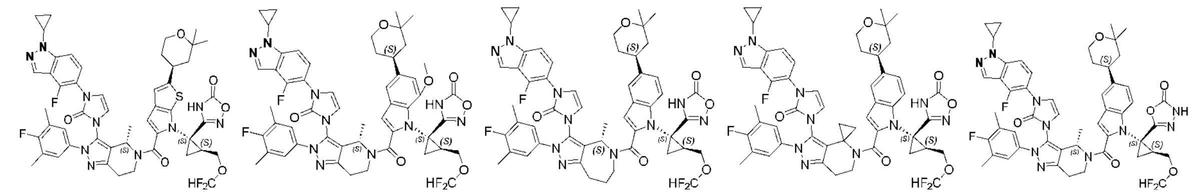

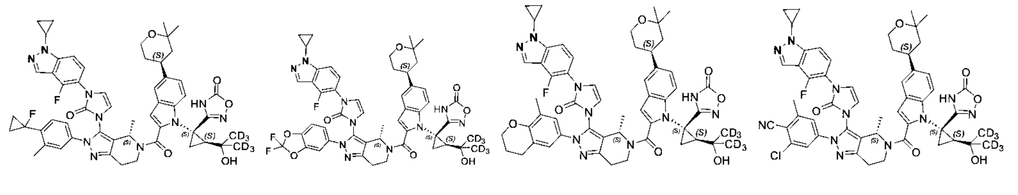

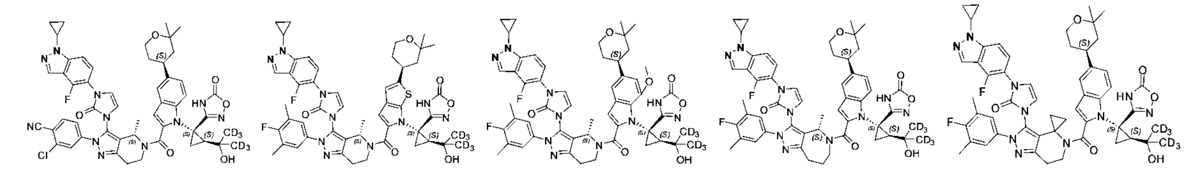

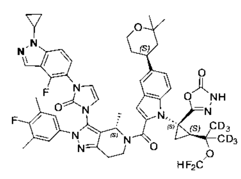
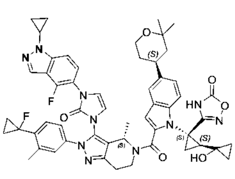
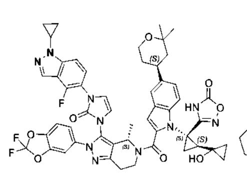
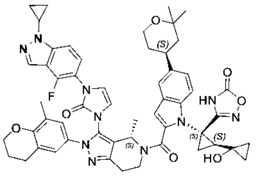
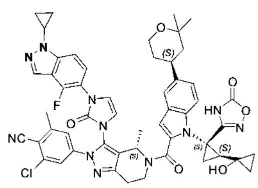
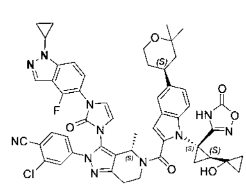
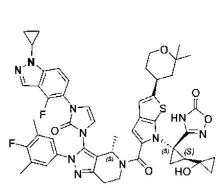
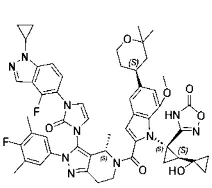
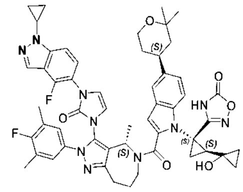
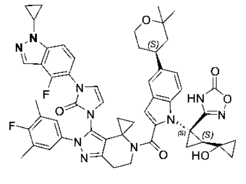
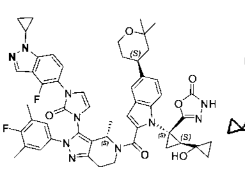
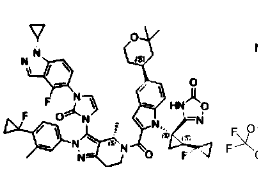
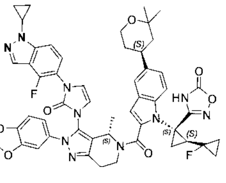
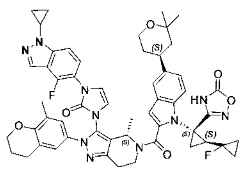
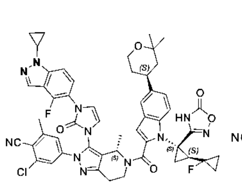
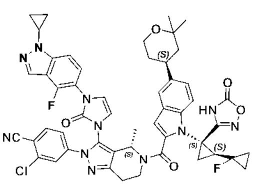
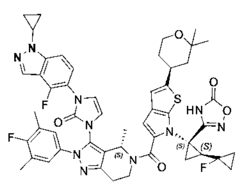
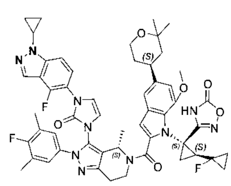
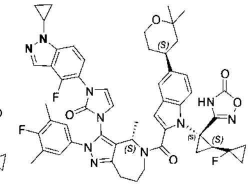
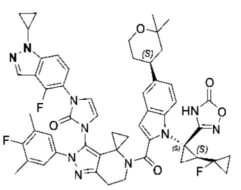
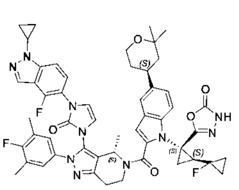
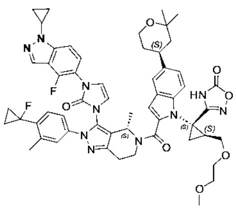
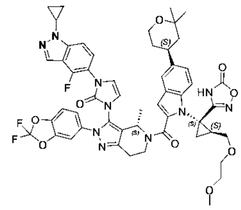
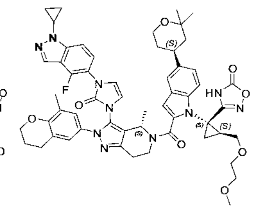
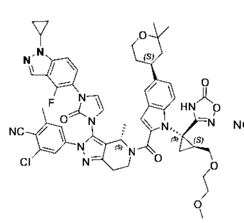
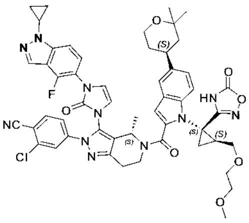
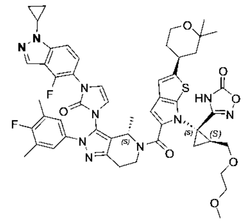
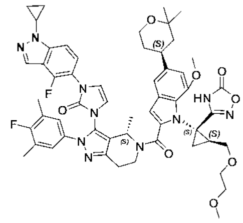
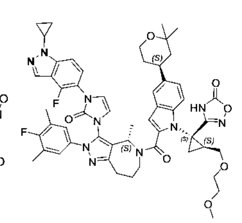
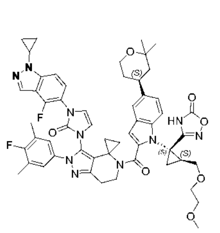
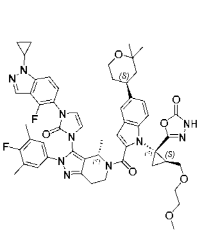
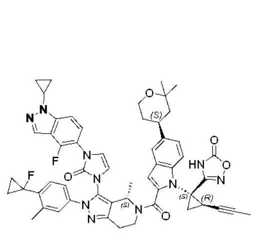
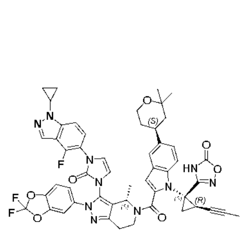
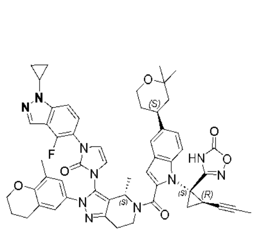

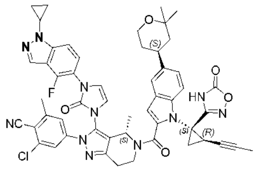
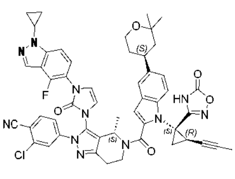
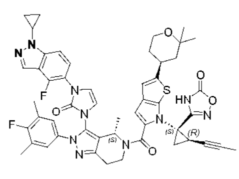
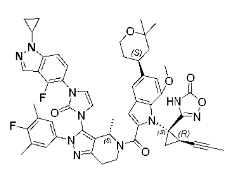
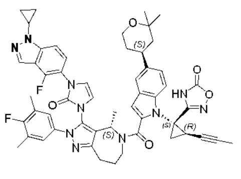

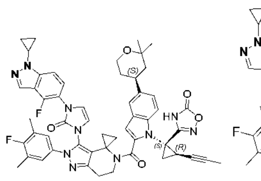
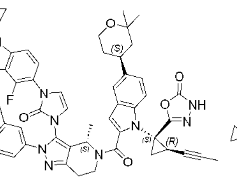
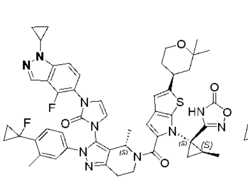
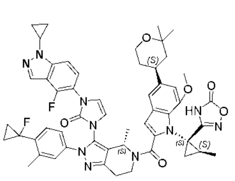
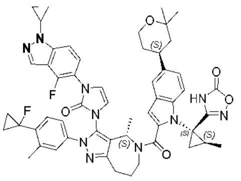

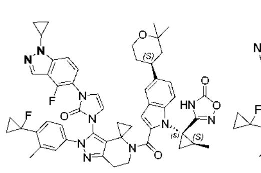
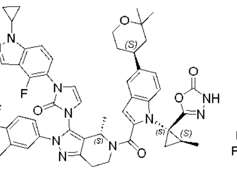
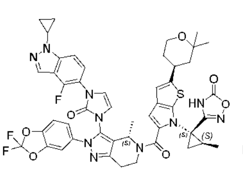
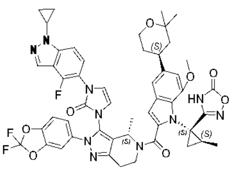
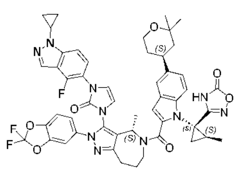

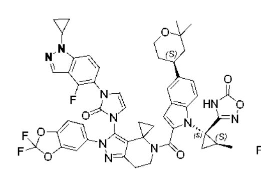
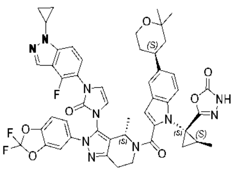
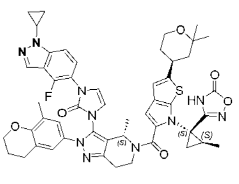
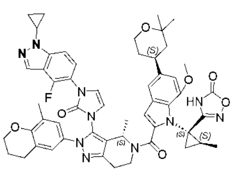
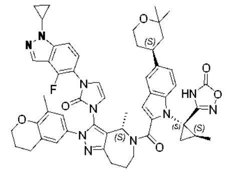

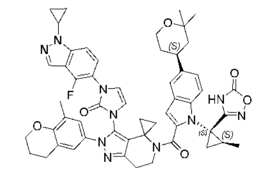
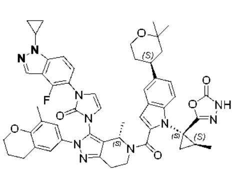
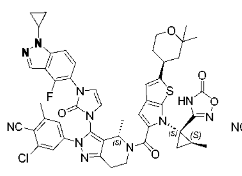
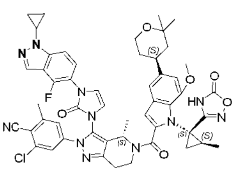
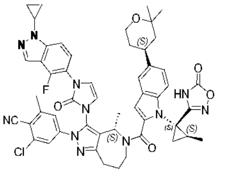

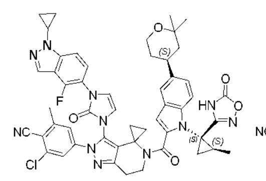
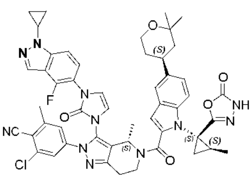
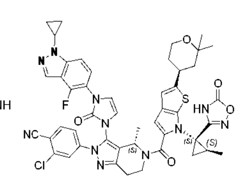
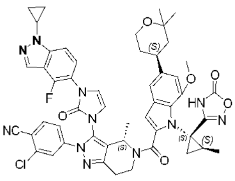
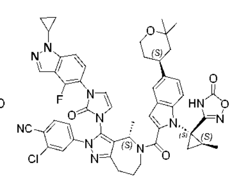

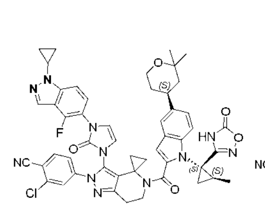
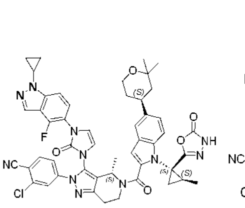
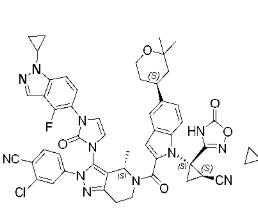
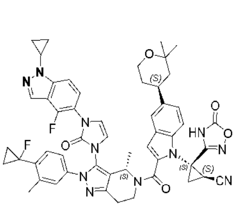
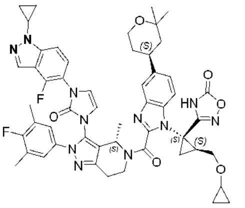

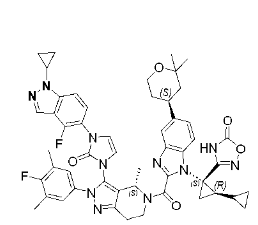
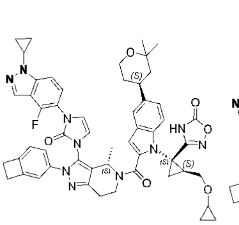
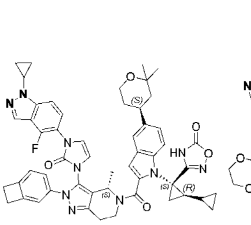
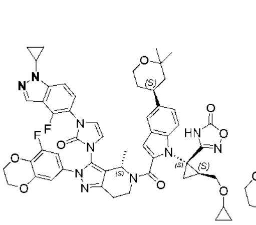
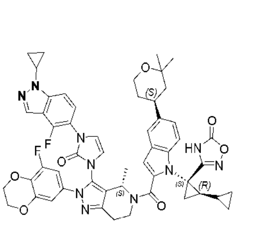

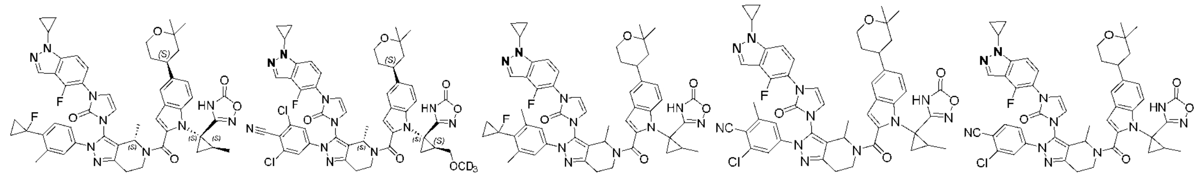

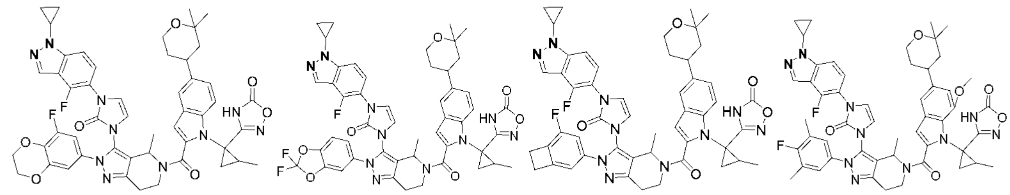

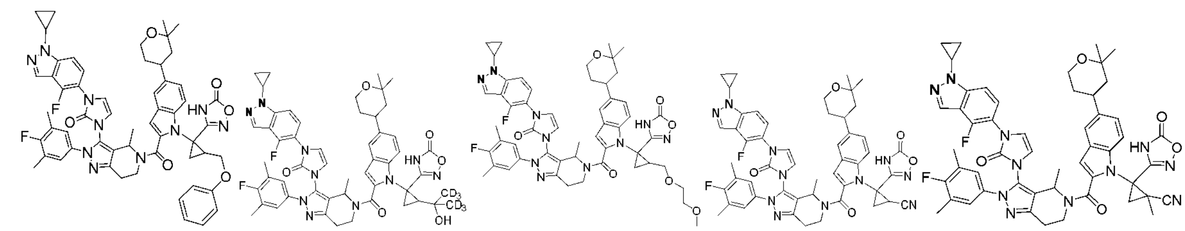

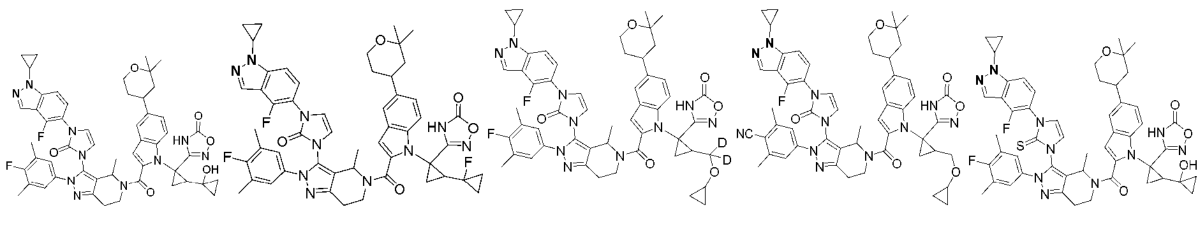

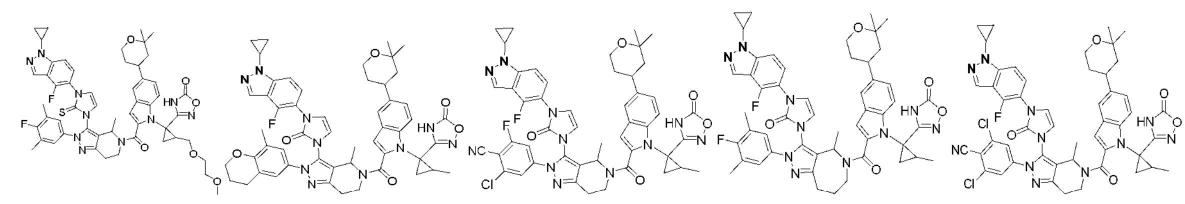

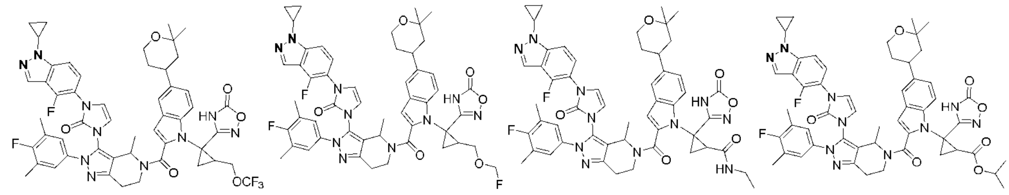

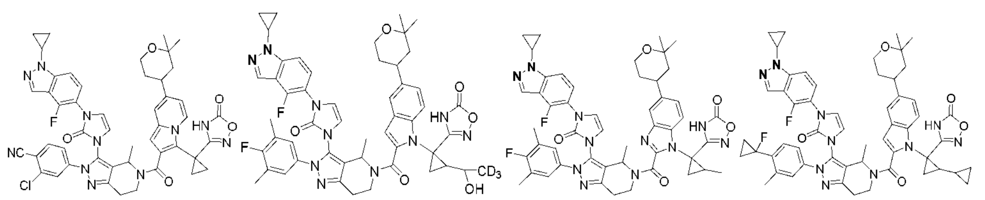

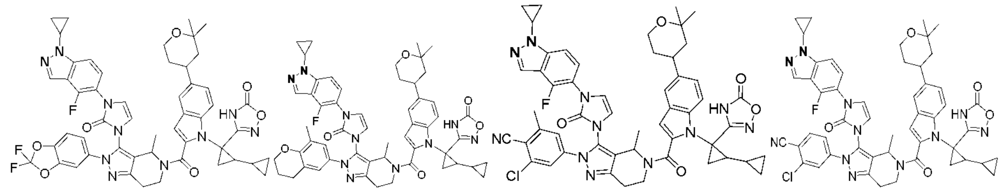

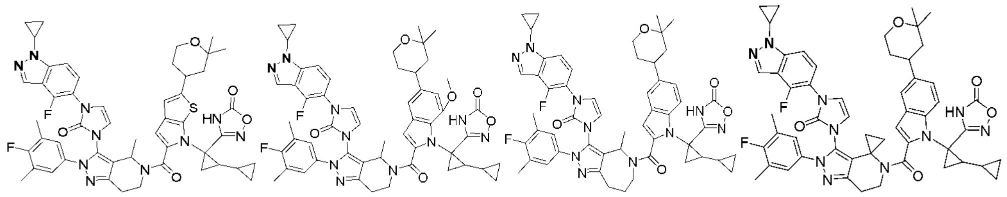

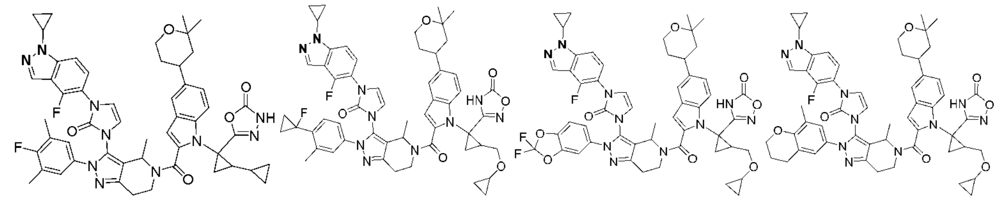

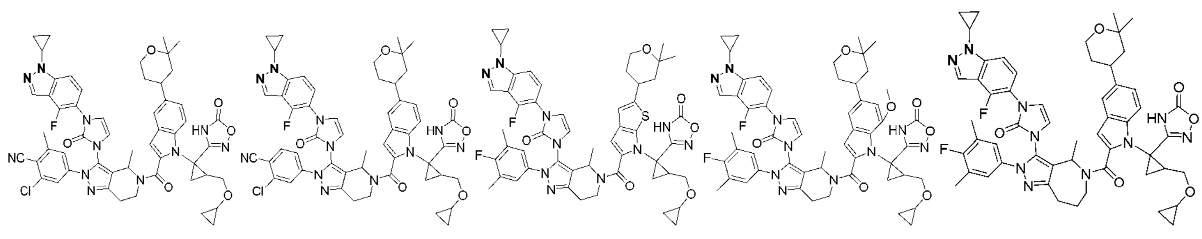

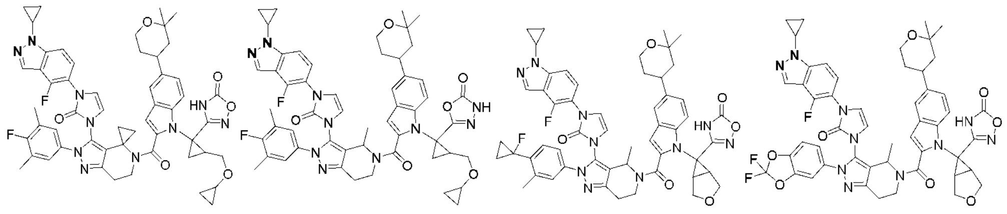

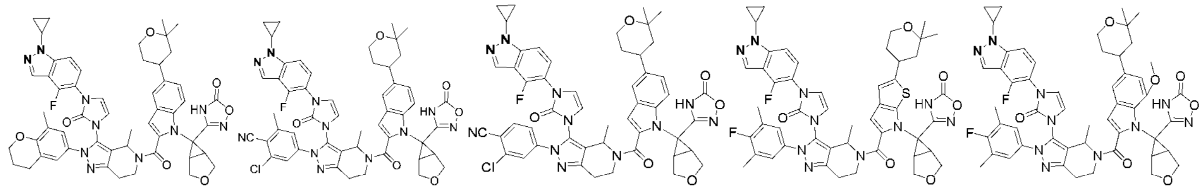

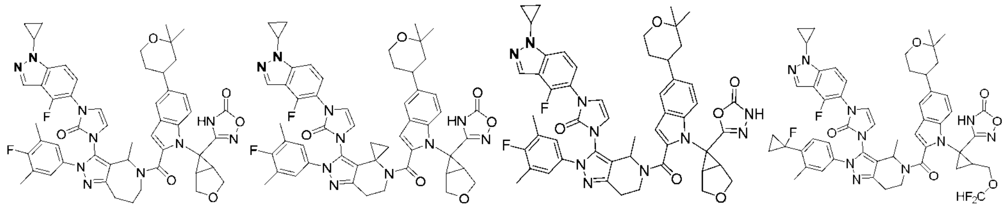

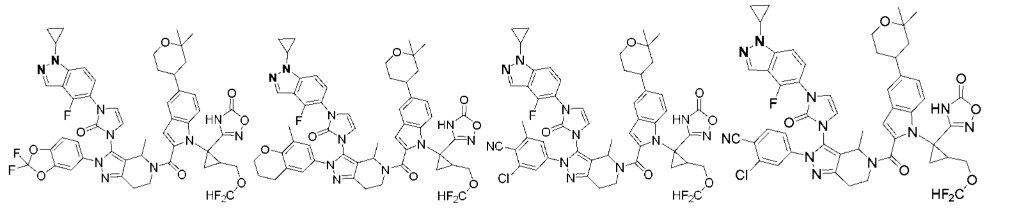

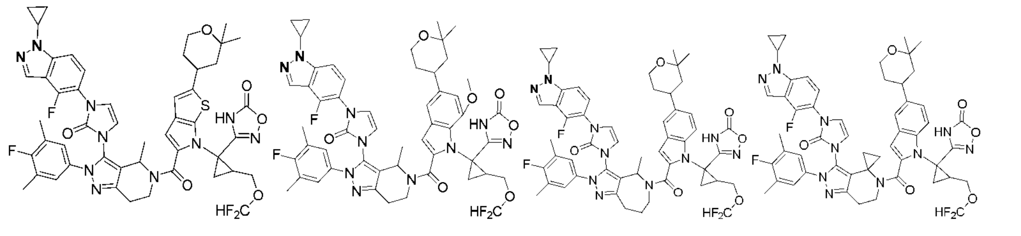

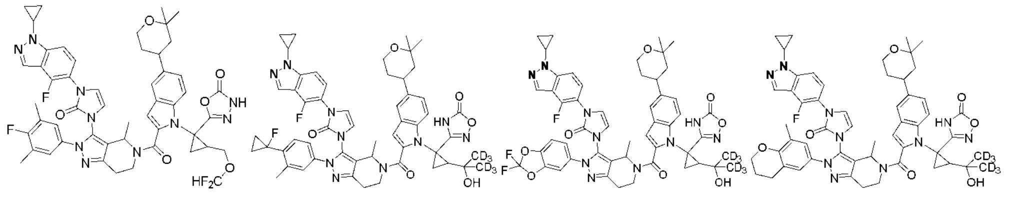

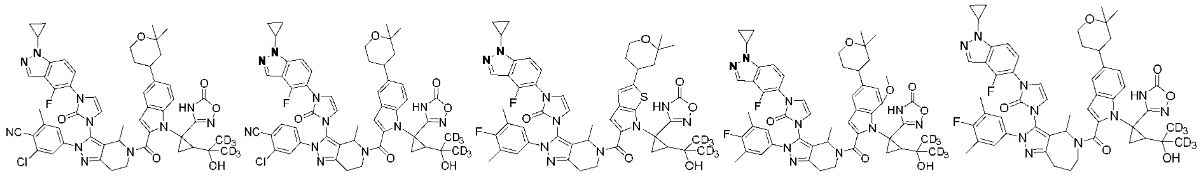

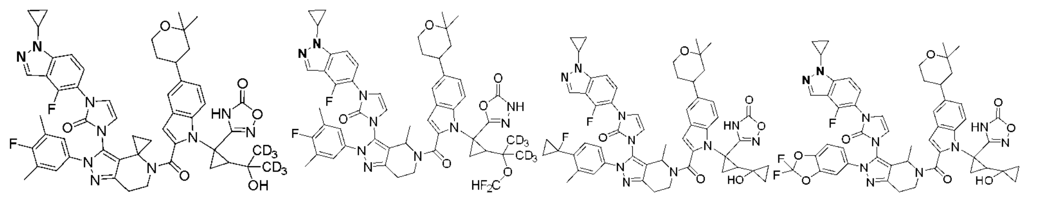

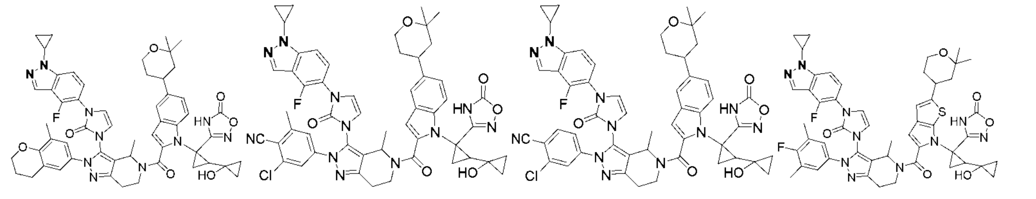

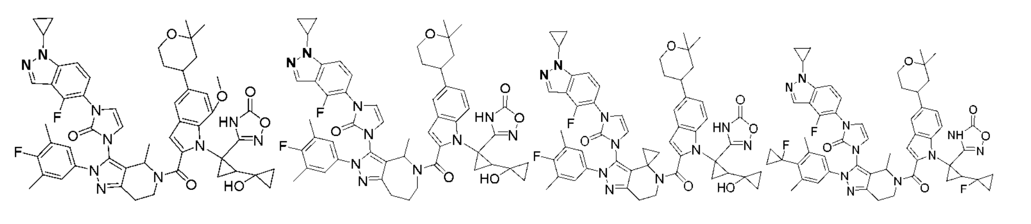

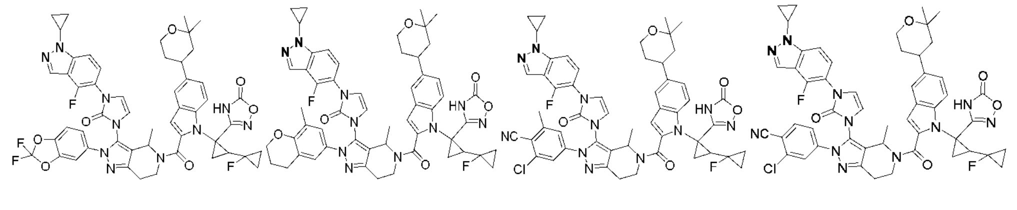

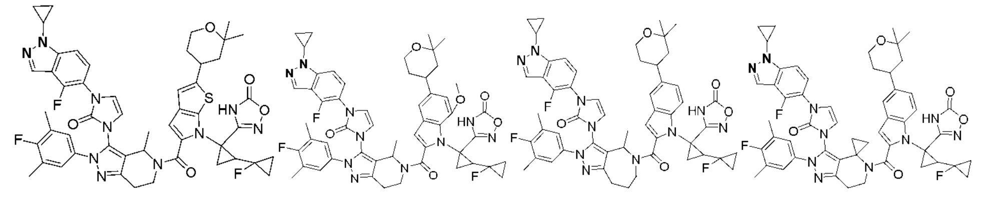

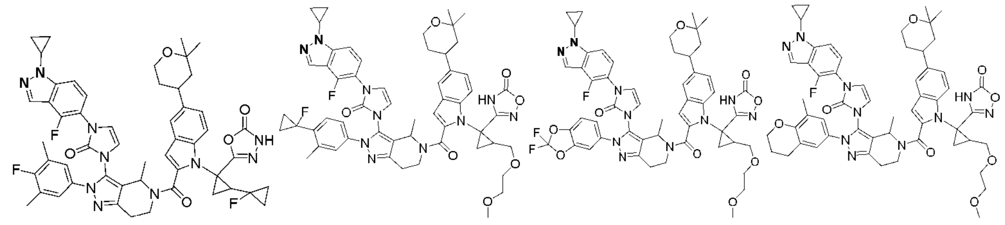

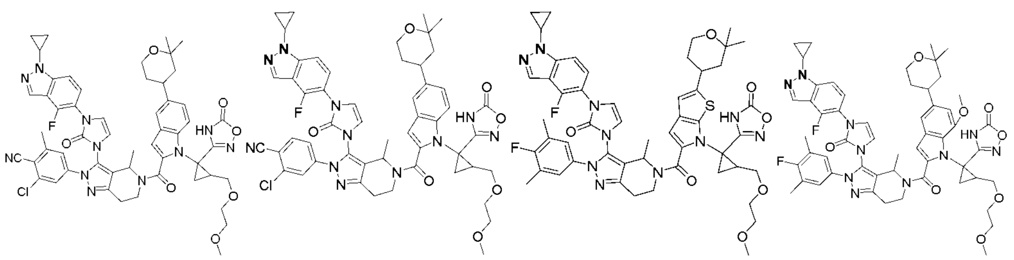

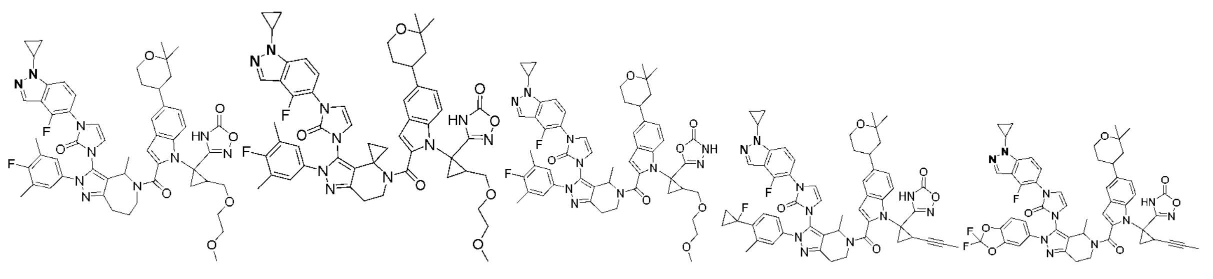

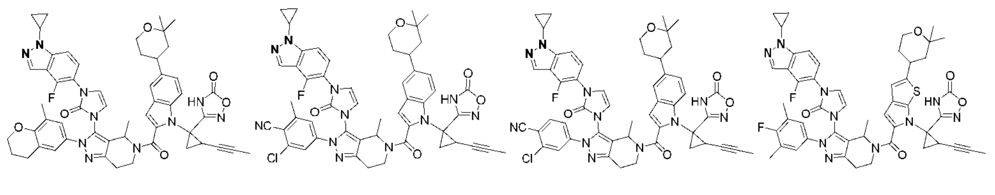

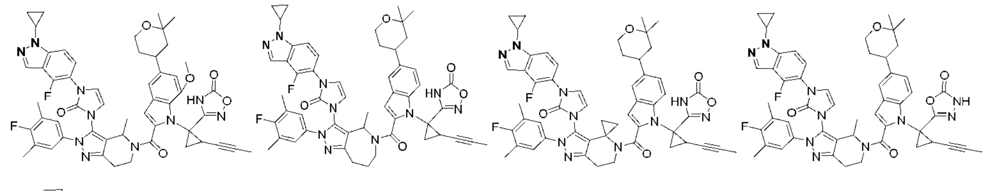

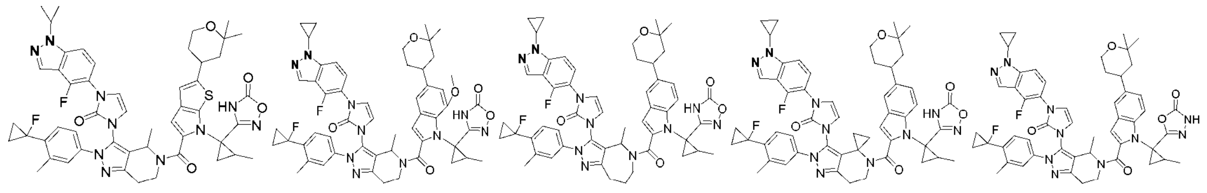

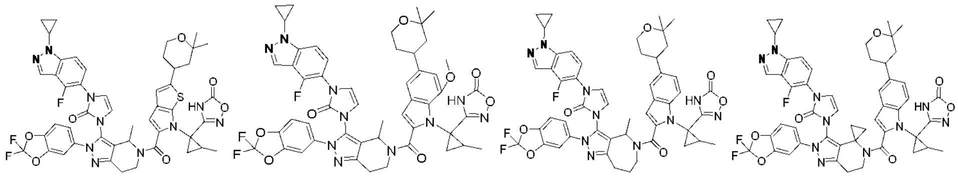

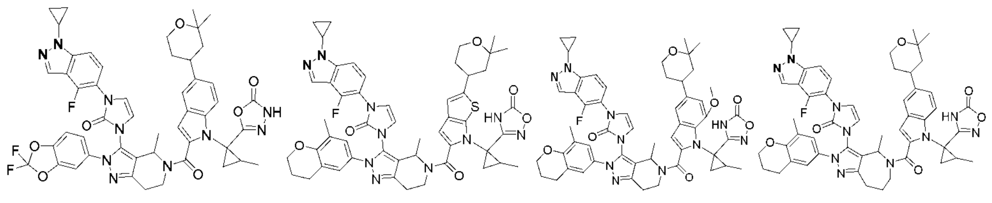

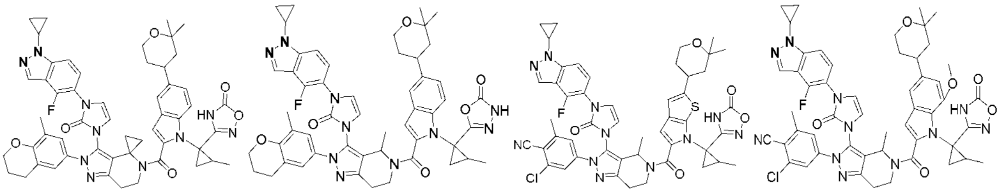

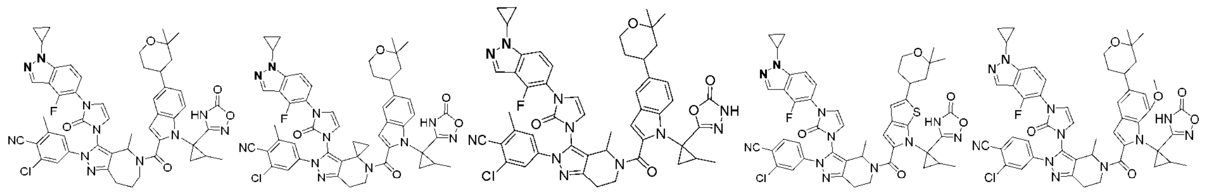

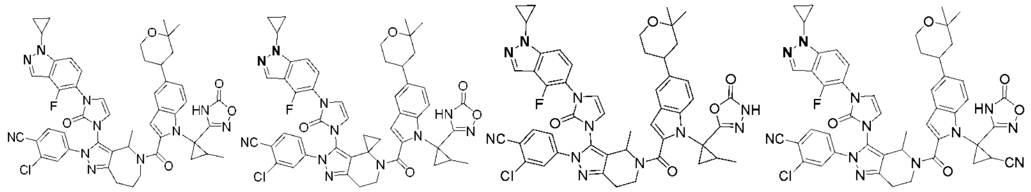

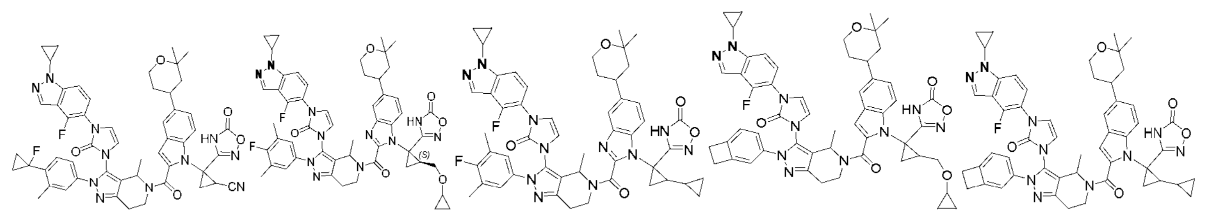

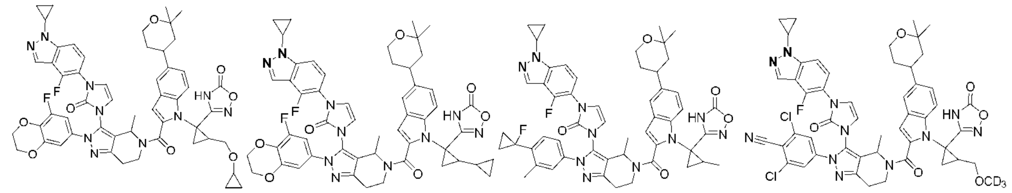

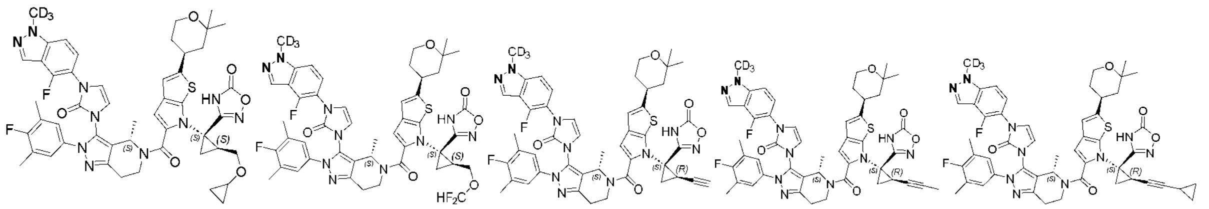

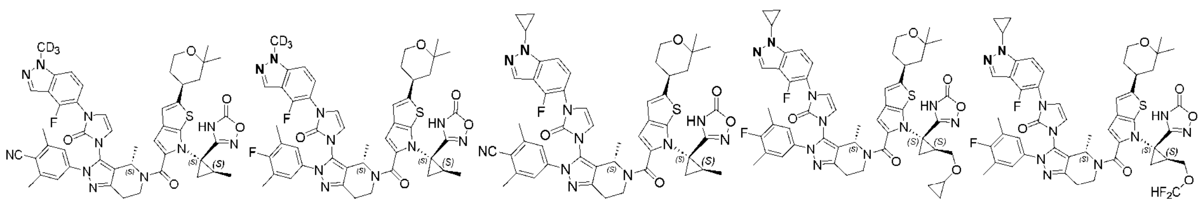

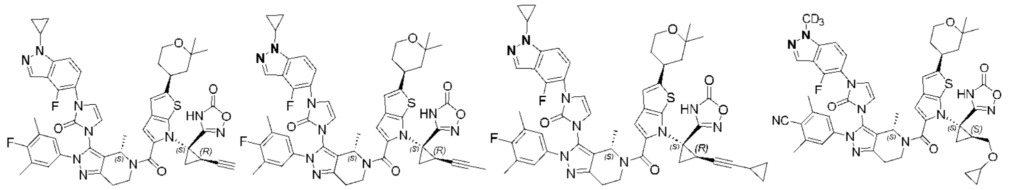

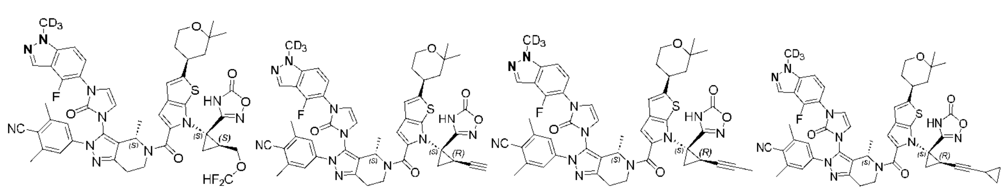

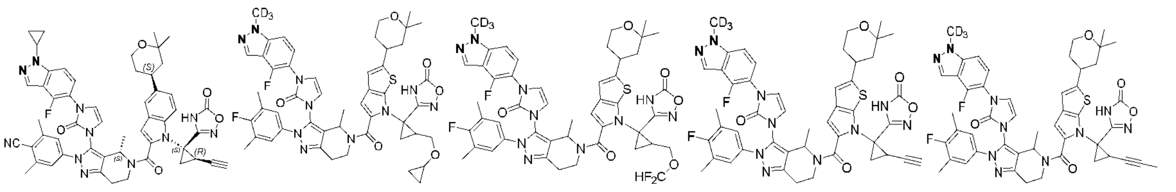

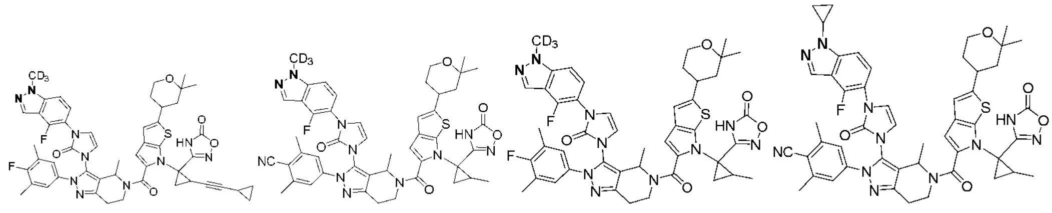

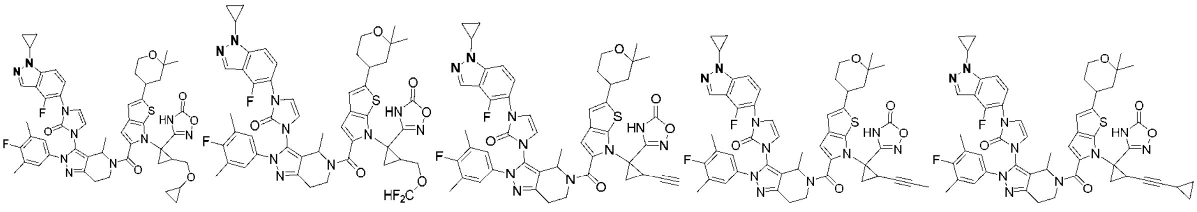

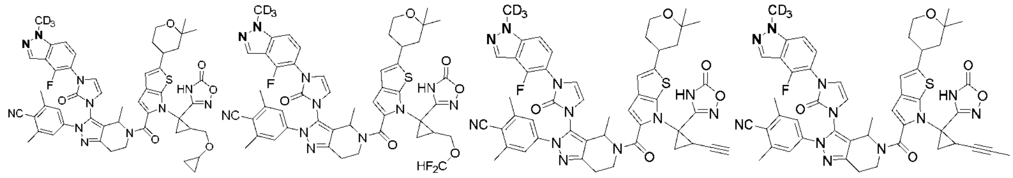

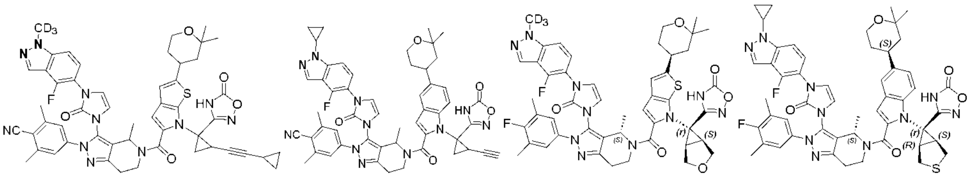

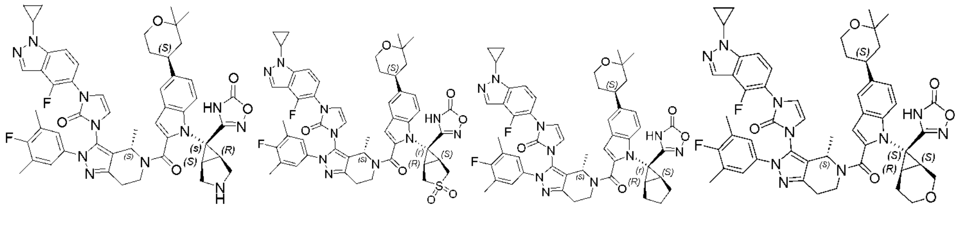

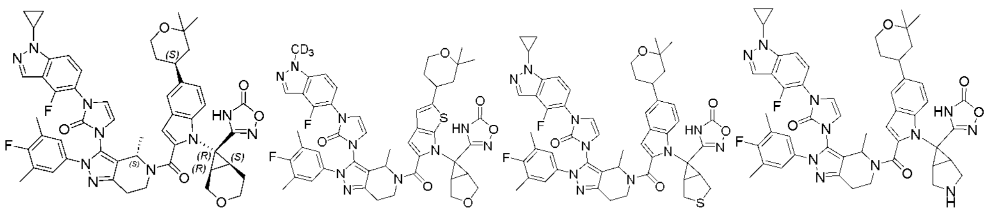

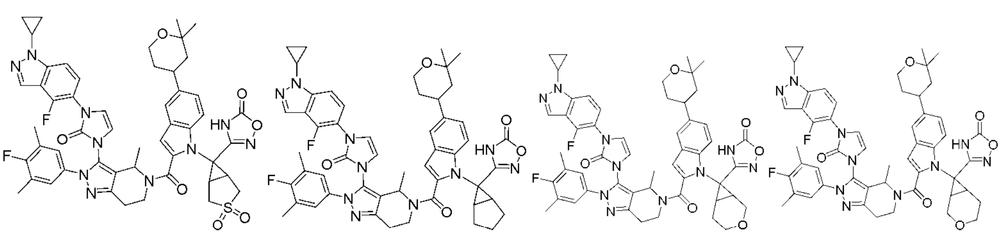

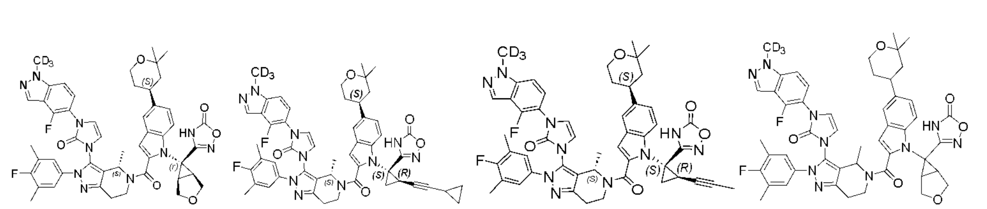

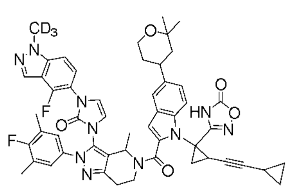 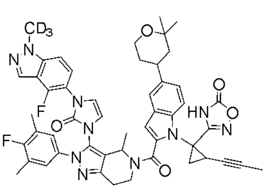 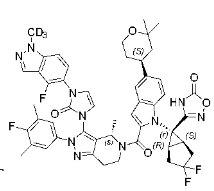 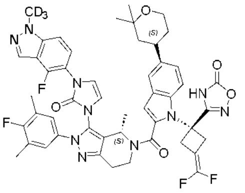

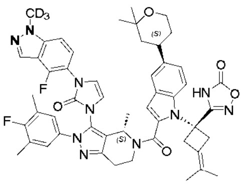 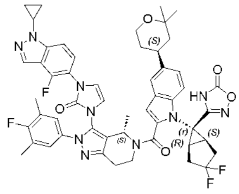 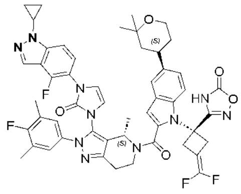 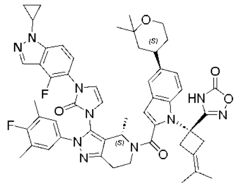 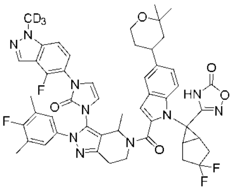

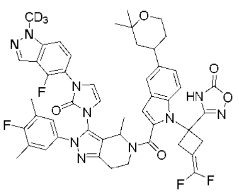 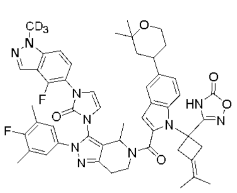 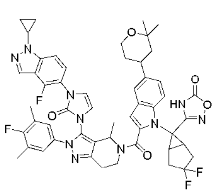 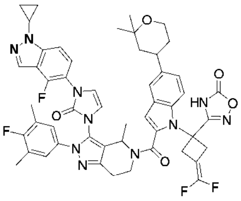 or 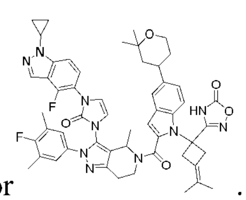 .

**8.** A compound represented by a general formula (XVI), a prodrug thereof, a stereoisomer thereof, or a pharmaceutically acceptable salt thereof:

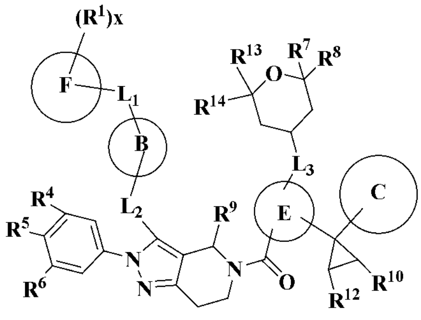

(XVI)

wherein,

ring F is selected from 3-18 membered heterocyclyl, $C_{3-18}$ cycloalkyl, $C_{6-18}$ aryl or 5-18 membered heteroaryl; preferably, ring F is selected from 3-14 membered heterocyclyl containing 1-3 heteroatoms selected from N, O or S, $C_{3-12}$ cycloalkyl, $C_{6-14}$ aryl or 5-14 membered heteroaryl containing 1-3 heteroatoms selected from N, O or S; more preferably, ring F is selected from 5-14 membered heterocyclyl containing 1-3 heteroatoms selected from N, O or S, $C_{3-10}$ cycloalkyl, $C_{6-10}$ aryl or 5-10 membered heteroaryl containing 1-3 heteroatoms selected from N, O or S;

further preferably, ring F is selected from 8-14 membered tricyclic fused heterocyclic containing 1-5 heteroatoms, C3-6 bridged cycloalkyl, 3-8 membered bridged heterocyclic containing 1-3 heteroatoms, fused heterocyclic containing 1-3 heteroatoms, bridged heterocyclic containing 1-3 heteroatoms, C6-10 aryl or 5-10 membered fused heteroaryl containing 1-3 heteroatoms;

furthermore preferably, ring F is selected from phenyl, pyridyl, pyrimidinyl, thiazolyl, pyridazinyl, oxazolyl, morpholinyl,

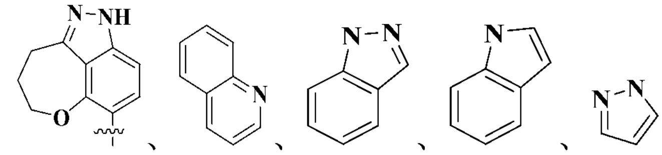
、 、 、 、 、

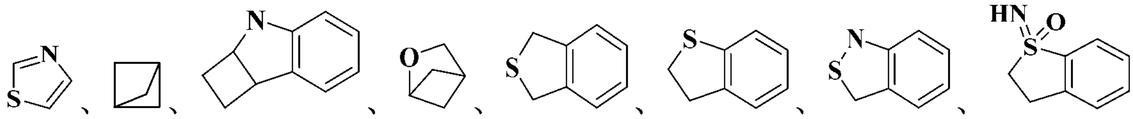
、 、 、 、 、 、 、 、

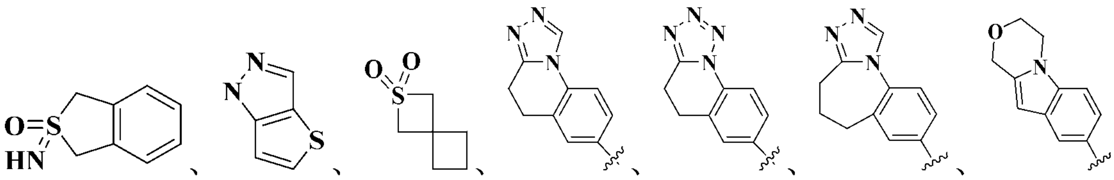
、 、 、 、 、 、 、

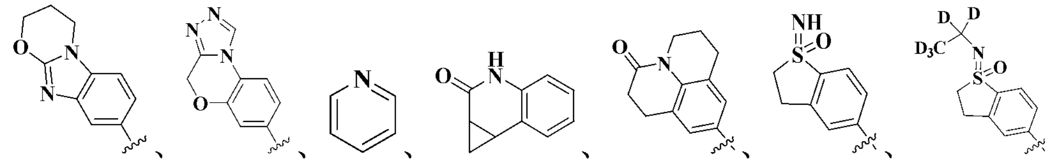
、 、 、 、 、 、 、

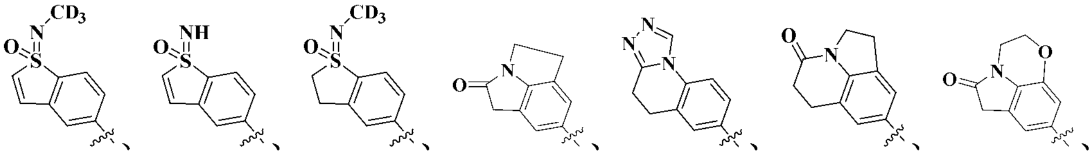
、 、 、 、 、 、 、

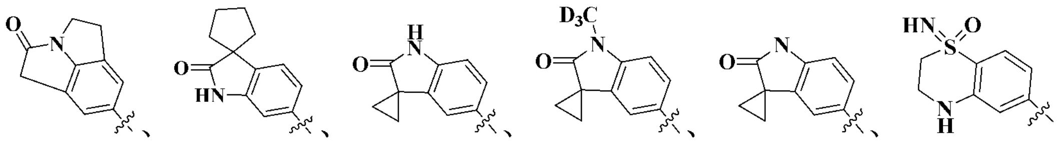
、 、 、 、 、 、

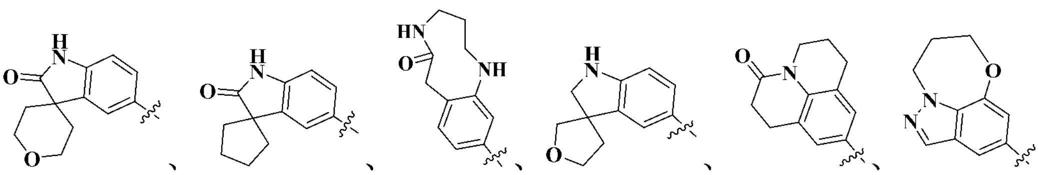
、 、 、 、 、 、

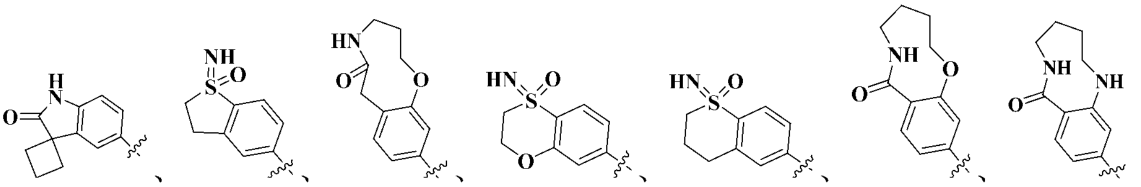
、 、 、 、 、 、 、

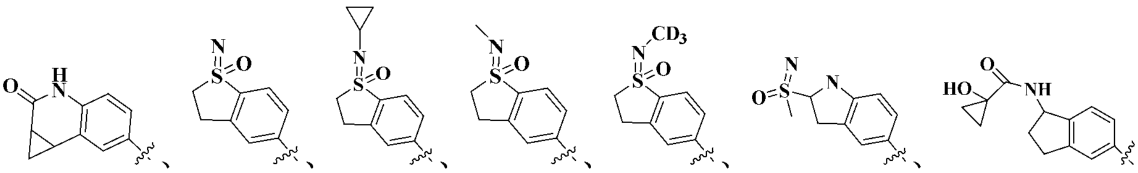
、 、 、 、 、 、 、

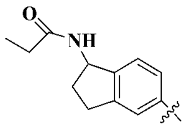、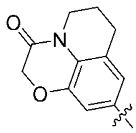、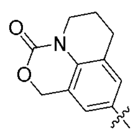、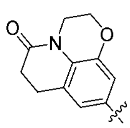、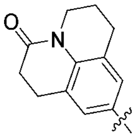、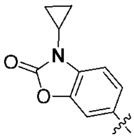、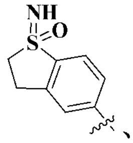、

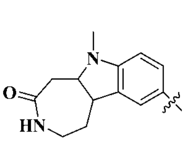、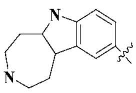、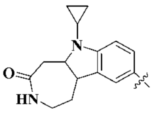、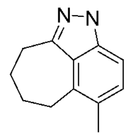、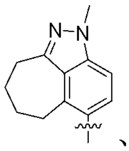、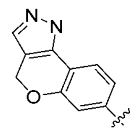、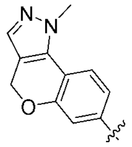、

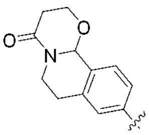、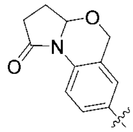、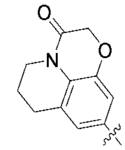、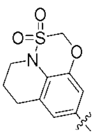、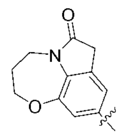、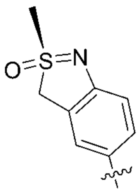、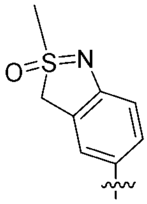、

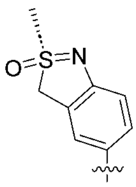、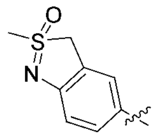、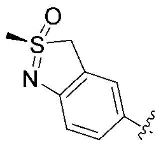、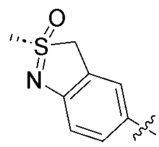、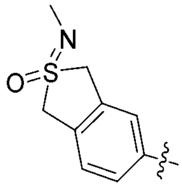、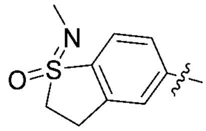、

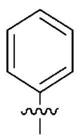、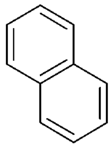、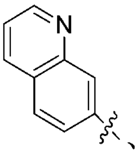、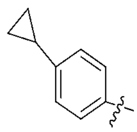、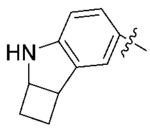、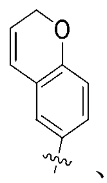、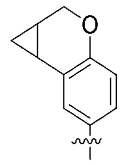、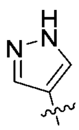、

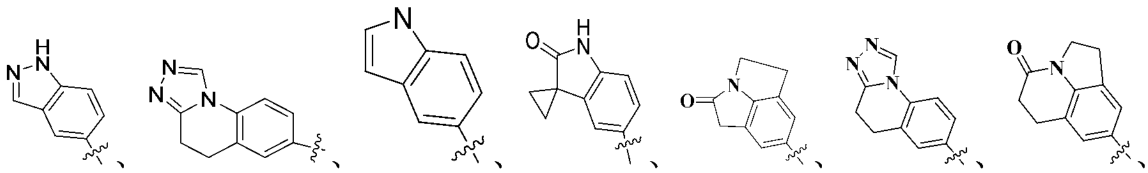、、、、、、、

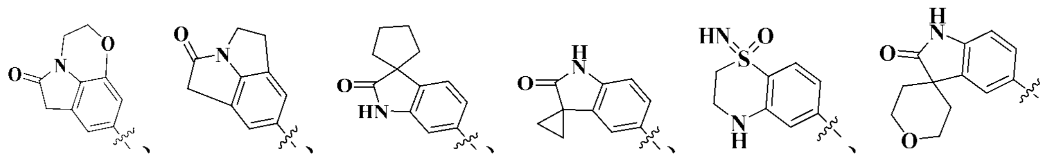、、、、、、

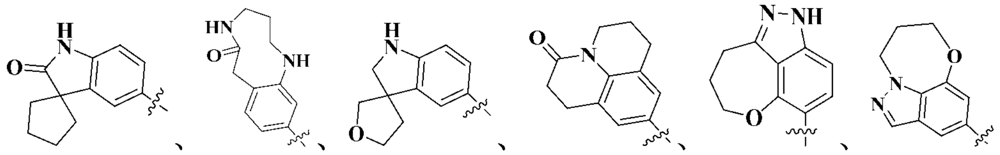、、、、、、

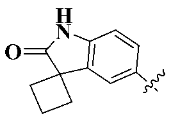、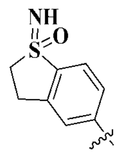、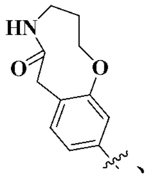、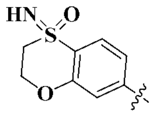、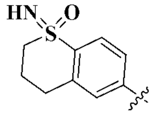、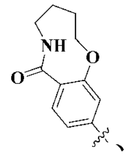、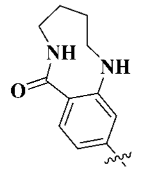、

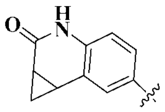、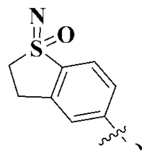、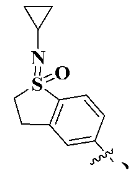、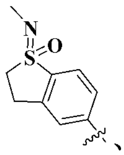、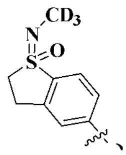、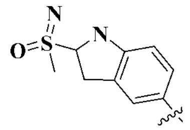、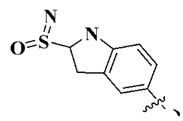、

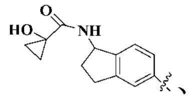、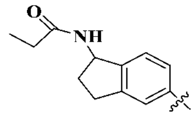、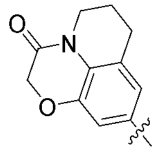、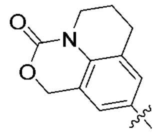、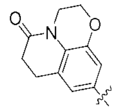、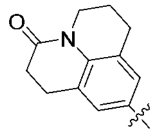、

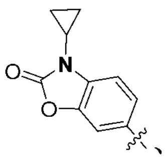、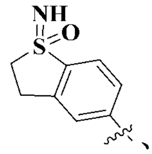、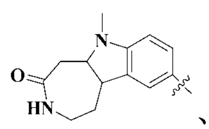、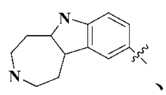、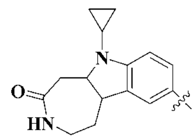、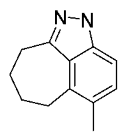、

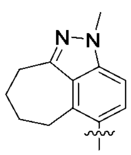、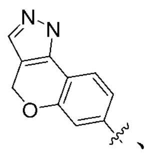、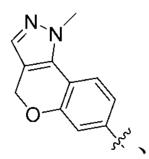、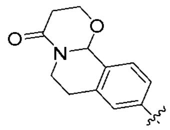、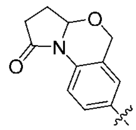、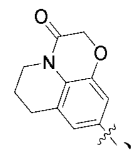、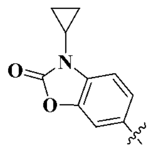或

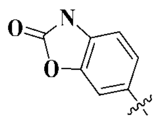;

ring B is selected from $C_{3-12}$ cycloalkyl, 3-12 membered heterocyclyl, $C_{6-14}$ aryl or 5-14 membered heteroaryl, which are optionally further substituted with one or more substituents selected from halogen, amino, nitro, hydroxyl, cyano, oxo, thiol, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ alkoxy, $C_{1-6}$ deuterated alkoxy, $C_{1-6}$ haloalkoxy or $C_{1-6}$ deuterated haloalkoxy;

ring C is selected from $C_{3-12}$ cycloalkyl, 3-12 membered heterocyclyl, $C_{6-14}$ aryl or 5-14 membered heteroaryl, which are optionally further substituted by one or more substituents selected from halogen, amino, nitro, hydroxyl, cyano, oxo, thiol, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy or $C_{1-6}$ deuterated haloalkoxy;

ring E is selected from $C_{3-12}$ cycloalkyl, 3-12 membered heterocyclyl, $C_{6-14}$ aryl or 5-14 membered heteroaryl, which are optionally further substituted by one or more substituents selected from deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl, the amino, $C_{1-6}$ alkyl, $C_{1-6}$ deuterated alkyl, $C_{1-6}$ haloalkyl, $C_{1-6}$ hydroxyalkyl, $C_{1-6}$ sulfanyl, $C_{1-6}$ alkoxy, $C_{1-6}$ haloalkoxy, $C_{2-6}$ alkenyl, $C_{2-6}$ alkynyl, C3-8 cycloalkyl, 3-8 membered heterocyclyl, C6-10 aryl or 5-10 membered heteroaryl;

$L_1$, $L_2$ or $L_3$ are each independently selected from a bond, $C_{2-4}$ alkenylene, $C_{2-4}$ alkynylene, $-(CH_2)_{n1}-$ or $-(CH_2)_{n5}NR_{b5}$, which are optionally further substituted by one or more substituents selected from halogen, amino,

nitro, hydroxyl, cyano, oxo, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

$R^1$ is independently selected from

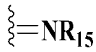
,

hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl, 5-10 membered heteroaryl, $-NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$, $-(CH_2)_{n1}O(CH_2)_{n2}R_{a5}$, $-(CH_2)_{n1}C(O)NR_{a6}R_{b6}$, $-(CH_2)_{n1}P(O)R_{a3}R_{b3}$,

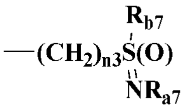
,

$-(CH_2)_{n1}NR_{a8}C(O)R_{b8}$, $-(CR_{a8}R_{b8})_{n4}C(O)NR_{a9}R_{b9}$, $-(CH_2)_{n1}S(O)_2R_{b5}$ or $-(CH_2)_{n5}C(O)NR_{a10}R_{b10}$, which are optionally further substituted by one or more substituents selected from

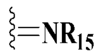
,

halogen, amino, nitro, hydroxyl, cyano, oxo, $-NS(O)R_{a1}R_{b1}$, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heteroaryl, $C_{6-10}$ aryl or 5-10 membered heteroaryl;

alternatively,

$R^1$ is respectively linked to the atoms connected thereto to form $C_{3-12}$ cycloalkyl, 3-12 membered heterocyclic, $C_{6-14}$ aryl or 5-14 membered heteroaryl, which are optionally further substituted by one or more substituents selected from

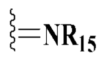
,

deuterium, halogen, amino, nitro, hydroxyl, cyano, oxo, thio, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl, 5-10 membered heteroaryl, $-NS(O)R_{a1}R_{b1}$, $-(CH_2)_{n1}S(O)NR_{a2}R_{b2}$, $-P(O)R_{a3}R_{b3}$, $-NR_{a5}S(O)NR_{a4}R_{b4}$, $-(CH2)_{n1}S(O)_2R_{a5}$, $-(CH_2)_{n1}O(CH_2)_{n2}R_{b5}$, $-(CH_2)_{n1}NR_{a8}C(O)R_{b8}$, $-(CR_{a8}R_{b8})_{n4}C(O)NR_{a9}R_{b9}$, $-(CH_2)_{n1}S(O)_2R_{b5}$ or $-(CH_2)_{n5}C(O)NR_{a10}R_{b10}$;

$R_{a1}$, $Rb_1$, $R_{a2}$, $Rb_2$, $R_{a3}$, $R_{b3}$, $R_{a4}$, $R_{b4}$, $R_{a5}$, $R_{a6}$, $R_{b6}$, $R_{a7}$, $R_{b7}$, $R_{a8}$, $R_{b8}$, $R_{a9}$, $R_{b9}$, $R_{a10}$ or $R_{b10}$ are each independently selected from hydrogen, deuterium, halogen, amino, nitro, hydroxyl, cyano, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, which are optionally further substituted by one or more substituents selected from halogen, amino, nitro, hydroxyl, cyano, oxo, $-NS(O)R_{a1}R_{b1}$, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

alternatively,

$R_{a1}$ and $R_{b1}$, $R_{a2}$ and $R_{b2}$, $R_{a3}$ and $R_{b3}$, $R_{a4}$ and $R_{b4}$, $R_{a6}$ and $R_{b6}$, $R_{a8}$ and $R_{b8}$, $R_{a9}$ and $R_{b9}$, $R_{a10}$ and $R_{b10}$ are linked with adjacent atoms to form $C_{3-10}$ cycloalkyl, 3-10 membered heterocyclic, $C_{6-12}$ aryl or 5-12 membered

heteroaryl, which are optionally further substituted by one or more substituents selected from halogen, amino, nitro, cyano, oxo, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R^4$, $R^5$ or $R^6$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, which are optionally further substituted by one or more substituents selected from Halogen, amino, nitro, hydroxyl, cyano, oxo, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R^7$, $R^8$, $R^9$, $R^{10}$, $R^{12}$, $R^{13}$, $R^{14}$ or $R^{15}$ are each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl, 5-10 membered heteroaryl or - $(CH_2)_{m1}OR_{c1}$, which are optionally further substituted by one or more substituents selected from deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R_{c1}$ is selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, the amino, $C_{1-6}$ alkyl, $C_{1-6}$ deuterated alkyl, $C_{1-6}$ haloalkyl, $C_{1-6}$ hydroxyalkyl, $C_{1-6}$ sulfanyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl, which are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ deuterated haloalkyl, $C_{1-6}$ hydroxyalkyl, $C_{1-6}$ deuterated hydroxyalkyl, $C_{1-6}$ sulfanyl, $C_{1-6}$ deuterated sulfanyl, $C_{1-6}$ alkoxy, $C_{1-6}$ deuterated alkoxy, $C_{1-6}$ haloalkoxy, $C_{1-6}$ deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

alternatively, $R^7$, $R^8$, $R^9$, $R^{10}$, $R^{12}$, $R^{13}$, $R^{14}$ or $R^{15}$ are linked to the atoms connected thereto to form a $C_{3-10}$ cycloalkyl, a 3-10 membered heterocyclic, a $C_{6-12}$ aryl, or 5-12 membered heteroaryl;

alternatively,

$R^{10}$ and $R^{12}$ are linked to the atoms connected thereto to form $C_{3-10}$ cycloalkyl, 3-10 membered heterocyclic, $C_{6-12}$ aryl or 5-12 membered heteroaryl;

preferably, $R^{10}$ and $R^{12}$ are linked to the atoms to form $C_{3-8}$ cycloalkyl, 3-8 membered heterocyclic containing 1-3 N, O or S atoms, $C_{6-10}$ aryl or 5-10 membered heteroaryl containing 1-3 N, O or S atoms;

n1-n5, m1 or x are each independently selected from 0, 1, 2, 3, 4 or 5.

**9.** The compound, the prodrug thereof, the stereoisomer thereof or the pharmaceutically acceptable salt thereof according to claim 8, **characterized in that** the general formula (XVI) is further represented by a general formula (XVI-A):

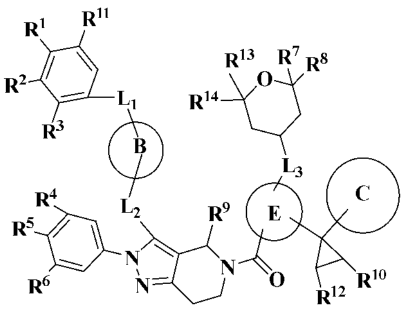

(XVI-A)

wherein,

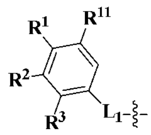

is further as shown in

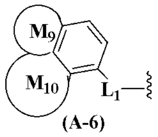
(A-6) 、
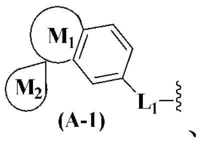
(A-1) 、
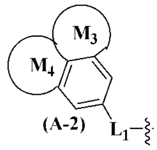
(A-2)
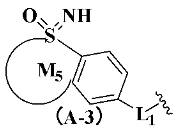
(A-3) 、
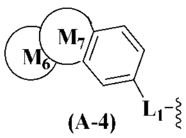
(A-4) or
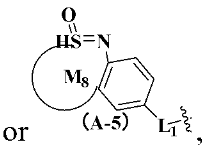
(A-5),

which are optionally further substituted by one or more substituents selected from halogen, methoxy, $C_{1-3}$ cycloalkyl, $C_{3-6}$ cycloalkyl or deuterated methyl;
ring $M_1$, ring $M_2$, ring $M_3$, ring $M_4$, ring $M_5$, ring $M_6$, ring $M_7$, ring $M_8$, $M_9$ or $M_{10}$ are each independently selected from $C_{3-12}$ cycloalkyl, 3-12 membered heterocyclyl, $C_{6-12}$ aryl or 5-12 membered heteroaryl, which are optionally further substituted by one or more substituents selected from halogen, amino, nitro, hydroxyl, cyano, oxo, C1-6 alkyl, C1-6 deuterated alkyl, C1-6 haloalkyl, C1-6 deuterated haloalkyl, C1-6 hydroxyalkyl, C1-6 deuterated hydroxyalkyl, C1-6 sulfanyl, C1-6 deuterated sulfanyl, C1-6 alkoxy, C1-6 deuterated alkoxy, C1-6 haloalkoxy, C1-6 deuterated haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;
preferably, ring $M_2$, ring $M_3$, ring $M_4$, ring $M_5$, ring $M_6$, ring $M_7$, ring $M_8$, $M_9$ or $M_{10}$ are each independently selected from $C_{3-8}$ cycloalkyl, 3-8 membered heterocyclic containing 1-3 N, O or S atoms, C6-10 aryl or 5-10 membered heteroaryl containing 1-3 N, O or S atoms, which are optionally further substituted by one or more substituents selected from halogen, amino, nitro, hydroxyl, cyano, oxo, C1-3 alkyl, C1-3 deuterated alkyl, C1-3 haloalkyl, C1-3 deuterated haloalkyl, C1-3 hydroxyalkyl, C1-3 deuterated hydroxyalkyl, C1-3 sulfanyl, C1-3 deuterated sulfanyl, C1-3 alkoxy, C1-3 deuterated alkoxy, C1-3 haloalkoxy, C1-3 deuterated haloalkoxy, C2-4 alkenyl, C2-4 alkynyl, $C_{3-6}$ cycloalkyl, 3-6 membered heterocyclyl, $C_{6-10}$ aryl or 5-8 membered heteroaryl;
further preferably, ring $M_2$, ring $M_3$, ring $M_4$, ring $M_5$, ring $M_6$, ring $M_7$, ring $M_8$, $M_9$ or $M_{10}$ are each independently selected from $C_{3-6}$ cycloalkyl, 3-6 membered heterocyclic containing 1-3 N, O or S atoms, $C_{6-10}$ aryl or 5-8 membered heteroaryl containing 1-3 N, O or S atoms, which are optionally further substituted by one or more substituents selected from halogen, methoxy, C1-3 cycloalkyl, $C_{3-6}$ cycloalkyl or deuterated methyl;
the definitions of ring B, ring C, ring E, $L_1$, $L_2$, $L_3$, $R^4$, $R^5$, $R^6$, $R^7$, $R^8$, $R^9$, $R^{10}$, $R^{12}$, $R^{13}$ and $R^{14}$ are as set forth in claim 9.

**10.** The compound, the prodrug thereof, the stereoisomer thereof or the pharmaceutically acceptable salt thereof according to claim 8, **characterized in that** the general formula (XVI) is further represented by a general formula (II):

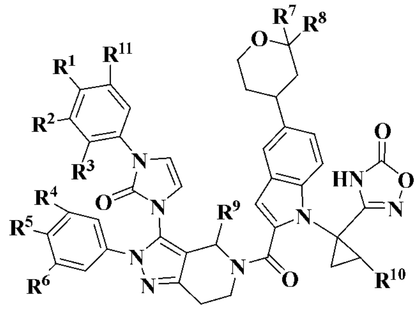

(II)

wherein,

$R^1$ and $R^2$, $R^1$ and $R^{11}$ are linked to adjacent atoms to form $C_{3-8}$ cycloalkyl, 3-8 membered heterocyclic, $C_{6-10}$ aryl or 5-10 membered heteroaryl, the $C_{3-8}$ cycloalkyl, 3-8 membered heterocyclic, C6-10 aryl or 5-10 membered heteroaryl are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

preferably, $R^1$ and $R^2$, $R^1$ and $R^{11}$ are linked to adjacent atoms to form $C_{3-6}$ cycloalkyl, 3-8 membered heterocyclic, $C_{6-10}$ aryl or 5-8 membered heteroaryl, which are optionally further substituted by one or more substituents selected from halogen, amino or oxo;

more preferably, $R^1$ and $R^2$, $R^1$ and $R^{11}$ are linked to adjacent atoms to form 5-7 membered heterocyclic containing 1-3 selected from N, O or S, and optionally further substituted by oxo;

$R^3$ is selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, 5-10 membered heteroaryl, $-NS(O)R_{a1}R_{b1}$, $-(CH_2)_{n1}S(O)NR_{a2}R_{b2}$, $-P(O)R_{a3}Rb_3$ or $-NR_{a5}S(O)NR_{a4}R_{b4}$, the amino, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl are optionally further substituted by one or more substituents selected from halogen, amino, nitro, hydroxyl, cyano, oxo, $-NS(O)R_{a1}R_{b1}$, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R_{a1}$, $Rb_1$, $R_{a2}$, $Rb_2$, $R_{a3}$, $R_{b3}$, $R_{a4}$ or $R_{b4}$ is each independently selected from hydrogen, deuterium, halogen, amino, nitro, hydroxyl, cyano, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

alternatively, $R_{a1}$, $R_{b1}$, $R_{a2}$, $Rb_2$, $R_{a3}$, $R_{b3}$, $R_{a4}$ or $R_{b4}$ is linked to adjacent atoms to form $C_{3-8}$ cycloalkyl, 3-8 membered heterocyclic, $C_{6-10}$ aryl or 5-10 membered heteroaryl, the $C_{3-8}$ cycloalkyl, 3-8 membered heterocyclic, $C_{6-10}$ aryl or 5-10 membered heteroaryl are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

alternatively, $R_{a1}$, $Rb_1$, $R_{a2}$, $Rb_2$, $R_{a3}$, $R_{b3}$, $R_{a4}$ or $R_{b4}$ is linked to adjacent atoms to form $C_{3-8}$ cycloalkyl, 3-6 membered heterocyclyl, $C_{6-10}$ aryl or 5-8 membered heteroaryl;

$R^4$, $R^5$ or $R^6$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, the amino, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R^7$, $R^8$, $R^9$ or $R^{10}$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, which

are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;
n1 is an integer from 0 to 5.

**11.** The compound, the prodrug thereof, the stereoisomer thereof or the pharmaceutically acceptable salt thereof according to claim 8, **characterized in that** the general formula (XVI) is further represented by a general formula (II):

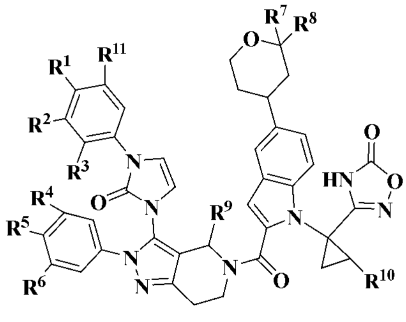

(II)

wherein,

$R^1$ and $R^2$, $R^1$ and $R^{11}$ are linked to adjacent atoms to form $C_{3-12}$ cycloalkyl, 3-12 membered heterocyclic, $C_{6-14}$ aryl or 5-14 membered heteroaryl, the $C_{3-12}$ cycloalkyl, 3-12 membered heterocyclic, $C_{6-14}$ aryl or 5-14 membered heteroaryl are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;
preferably, $R^1$ and $R^2$, $R^1$ and $R^{11}$ are linked to adjacent atoms to form $C_{3-6}$ cycloalkyl, 3-10 membered heterocyclyl, $C_{6-10}$ aryl or 5-8 membered heteroaryl, which are optionally further substituted by one or more substituents selected from halogen, $C_{1-3}$ alkyl, $C_{3-6}$ cycloalkyl, amino or oxo;
more preferably, $R^1$ and $R^2$, $R^1$ and $R^{11}$ are linked to adjacent atoms to form 5-7 membered heterocyclic containing 1-3 selected from N, O or S, which are optionally further substituted by $C_{3-6}$ cycloalkyl, oxo;
furthermore preferably, $R^1$ and $R^2$, $R^1$ and $R^{11}$ are linked to adjacent atoms to form

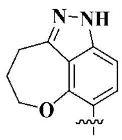 、 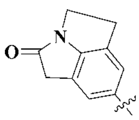 、 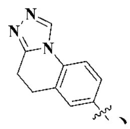 、 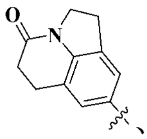 、 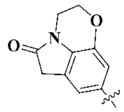 、 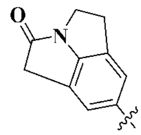 、 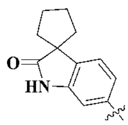 、

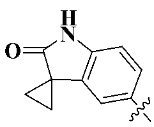 、 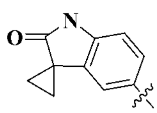 、 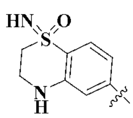 、 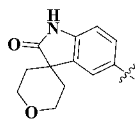 、 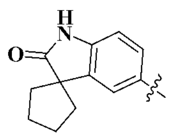 、 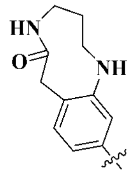 、

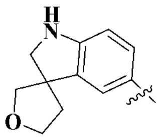 、 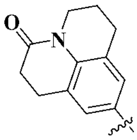 、 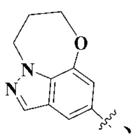 、 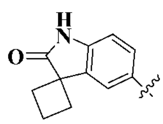 、 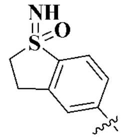 、 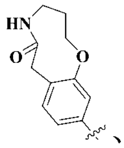 、 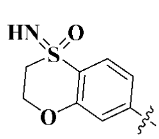 、

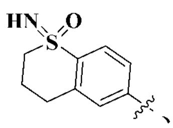, 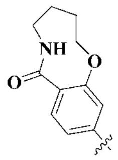, 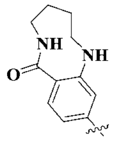, 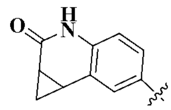, 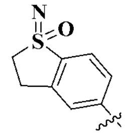, 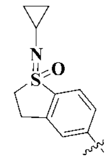, 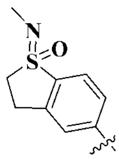,

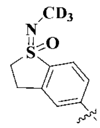, 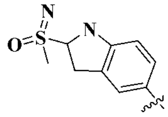, 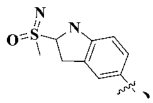, 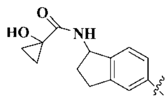, 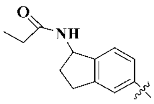, 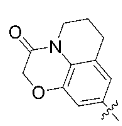, 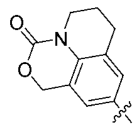,

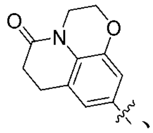, 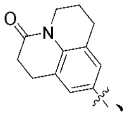, 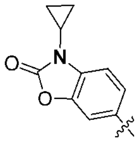, 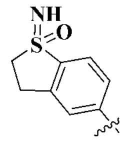, 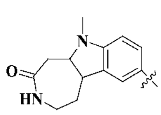, 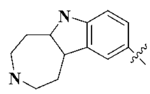, 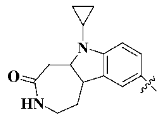,

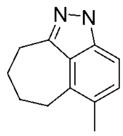, 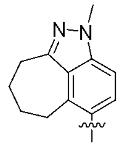, 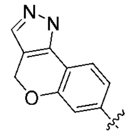, 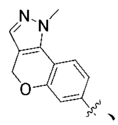, 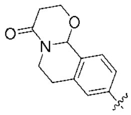, 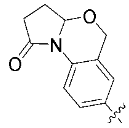, 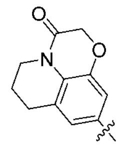, 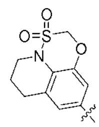 or

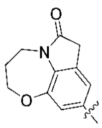,

which are optionally further substituted with one or more substituents selected from halogen, $C_{1-3}$ alkyl, $C_{1-3}$ deuterated alkyl, $C_{3-6}$ cycloalkyl, amino or oxo;

$R^1$, $R^2$, $R^3$ or $R^{11}$ is selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, 5-10 membered heteroaryl, $-NS(O)R_{a1}R_{b1}$, $-(CH_2)n1S(O)NR_{a2}R_{b2}$, $-P(O)R_{a3}R_{b3}$ or $-NR_{a5}S(O)NR_{a4}R_{b4}$, the amino, $C_{1-6}$ alkyl, $C_{1-6}$ deuterated alkyl, $C_{1-6}$ haloalkyl, $C_{1-6}$ hydroxyalkyl, $C_{1-6}$ Alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, which are optionally further substituted by one or more substituents selected from halogen, amino, nitro, hydroxyl, cyano, oxo, $-NS(O)R_{a1}R_{b1}$, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R_{a1}$, $R_{b1}$, $R_{a2}$, $R_{b2}$, $R_{a3}$, $R_{b3}$, $R_{a4}$ or $R_{b4}$ is each independently selected from hydrogen, deuterium, halogen, amino, nitro, hydroxyl, cyano, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl;

alternatively, $R_{a1}$, $R_{b1}$, $R_{a2}$, $R_{b2}$, $R_{a3}$, $R_{b3}$, $R_{a4}$ or $R_{b4}$ is respectively linked to adjacent atoms to form $C_{3-8}$ cycloalkyl, 3-8 membered heterocyclic, $C_{6-10}$ aryl or 5-10 membered heteroaryl, the $C_{3-8}$ cycloalkyl, 3-8 membered heterocyclic, $C_{6-10}$ aryl or 5-10 membered heteroaryl are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R^4$, $R^5$ or $R^6$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl, or 5-10 membered heteroaryl, the amino, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$

cycloalkyl, 3-8 membered heterocyclic, $C_{6-10}$ aryl or 5-10 membered heteroaryl;

$R^7$, $R^8$, $R^9$ or $R^{10}$ is each independently selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl, 5-10 membered heteroaryl or $-(CH_2)_{m1}OR_{c1}$, which are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

$R_{c1}$ is selected from hydrogen, deuterium, halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ sulfanyl, $C_{1-6}$ alkoxy, $C_{1-6}$ haloalkoxy, $C_{2-6}$ alkenyl, $C_{2-6}$ alkynyl, C3-8 cycloalkyl, 3-8 membered heterocyclic, C6-10 aryl or 5-10 membered heteroaryl, the amino, $C_{1-6}$ alkyl, $C_{1-6}$ deuterated alkyl, $C_{1-6}$ haloalkyl, $C_{1-6}$ hydroxyalkyl, $C_{1-6}$ sulfanyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclyl, $C_{6-10}$ aryl or 5-10 membered heteroaryl, which are optionally further substituted by one or more substituents selected from halogen, amino, nitro, hydroxyl, cyano, oxo, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-8}$ cycloalkyl, 3-8 membered heterocyclic, $C_{6-10}$ aryl or 5-10 membered heteroaryl;

alternatively,

$R_{10}$ is linked to and the atoms connected thereto and adjacent atoms to form $C_{3-10}$ cycloalkyl, 3-10 membered heterocyclic, $C_{6-12}$ aryl or a 5-12 membered heteroaryl;

n1 is an integer of 0-5;

m1 is an integer of 0-5.

**12.** The compound, the prodrug thereof, the stereoisomer thereof or the pharmaceutically acceptable salt thereof according to any one of claims 8-11, wherein the compound structure is as follows:

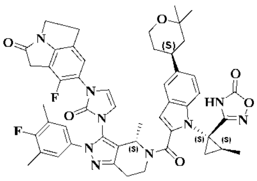
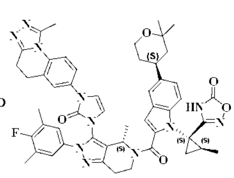
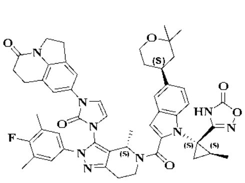
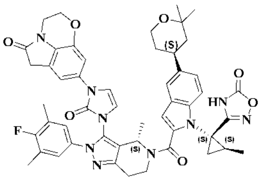

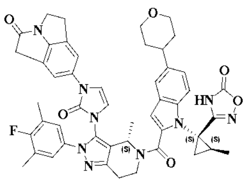
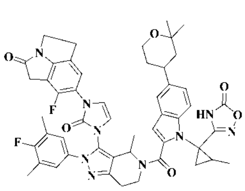
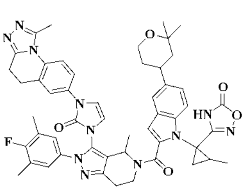
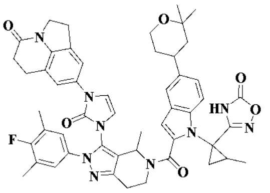

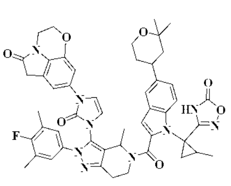
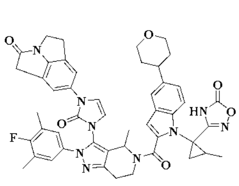
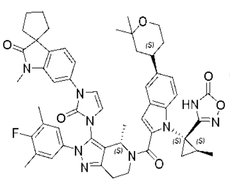
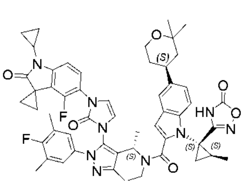
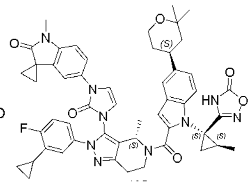

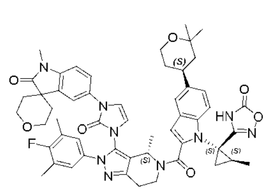
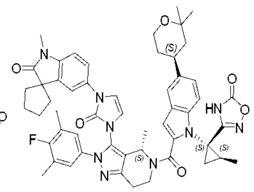
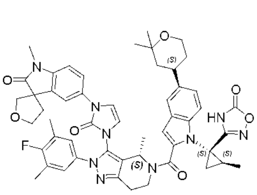
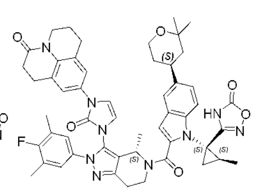

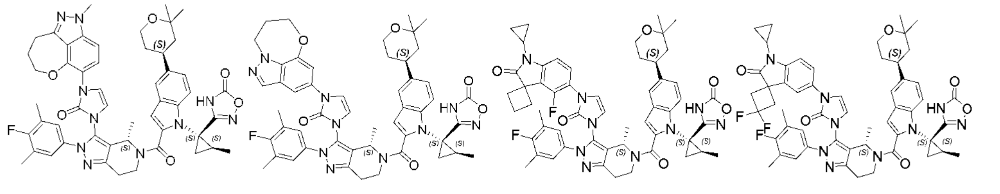

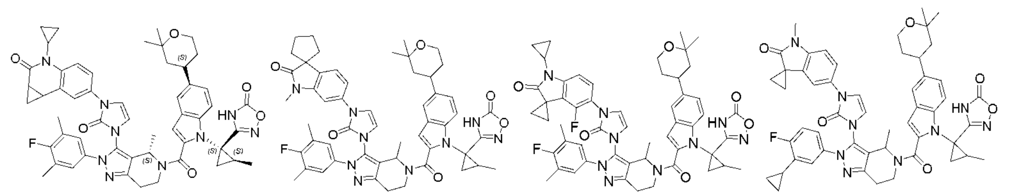

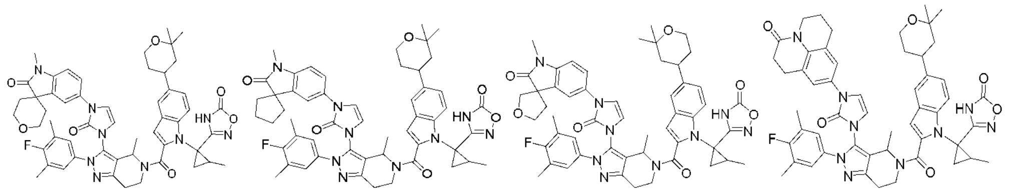

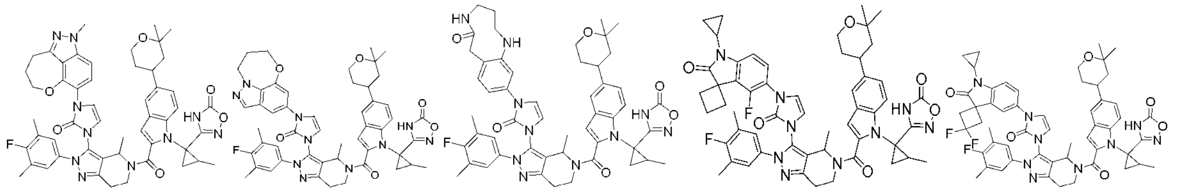

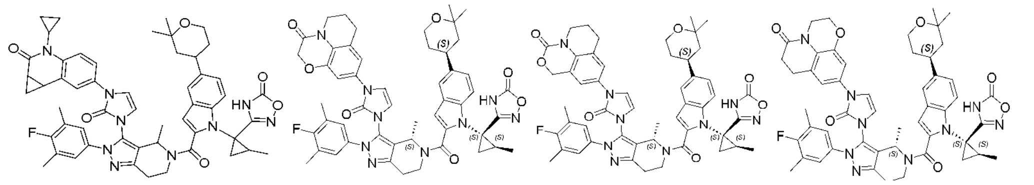

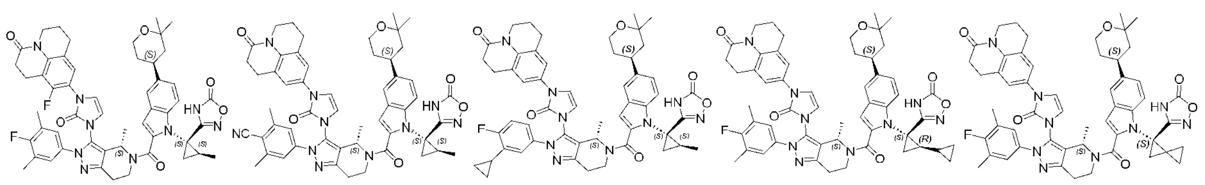

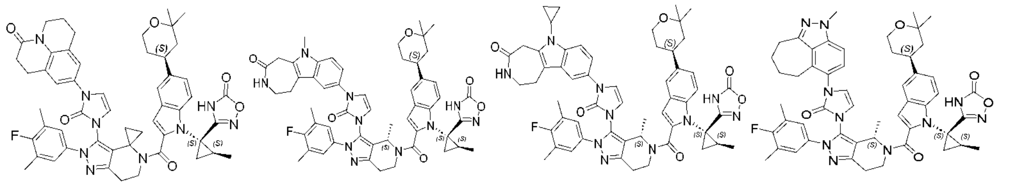

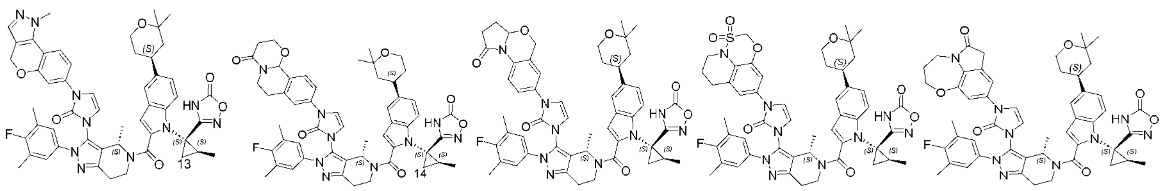

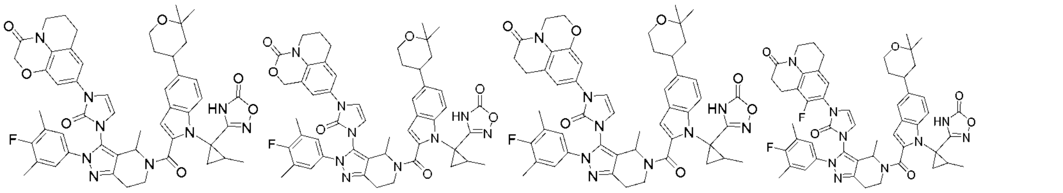

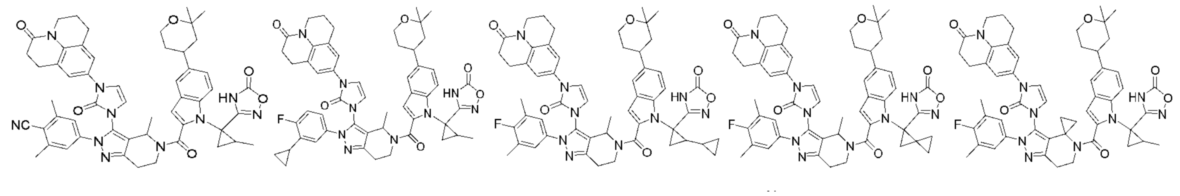

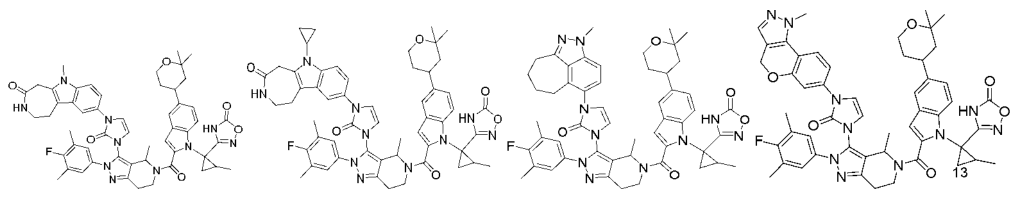

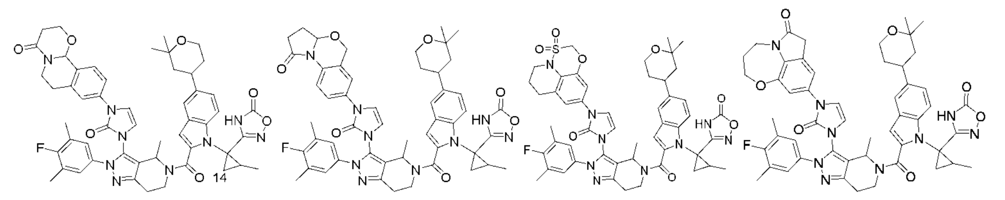

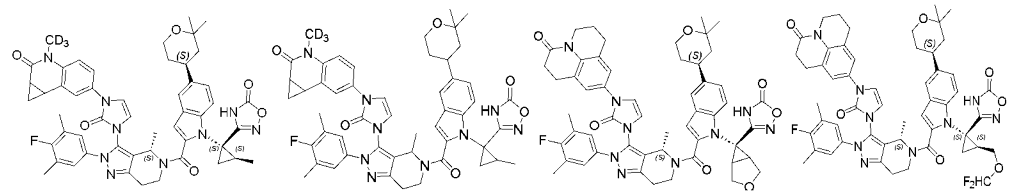

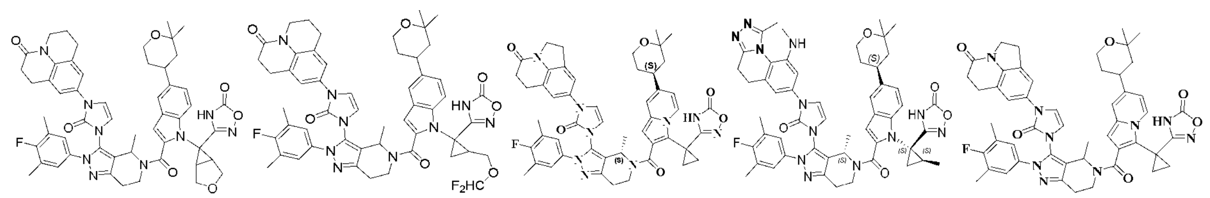

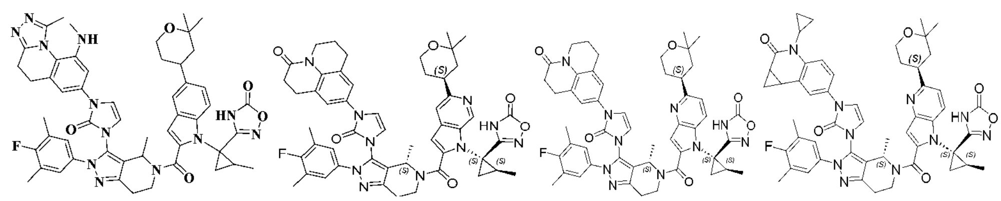

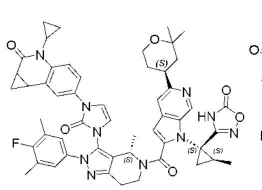 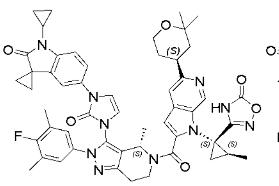 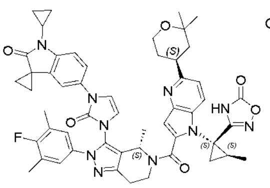 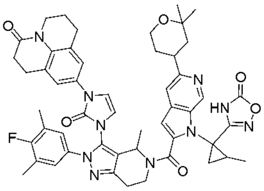

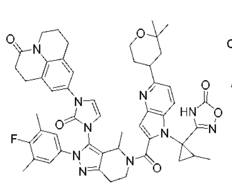 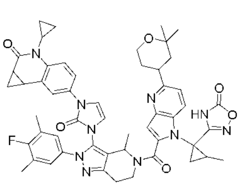 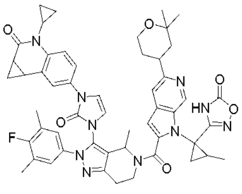 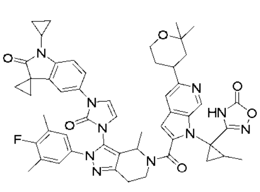 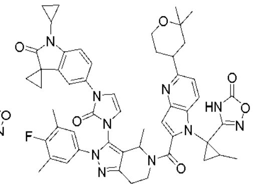

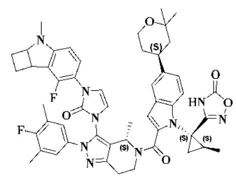 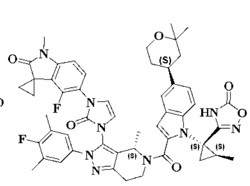 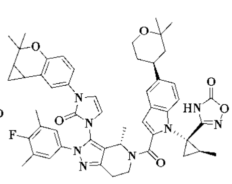 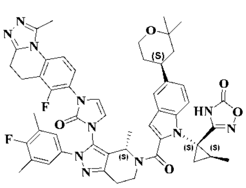 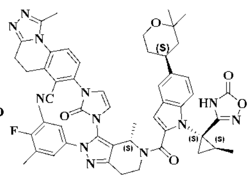

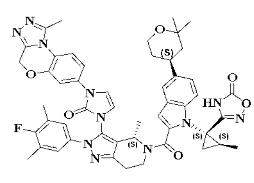 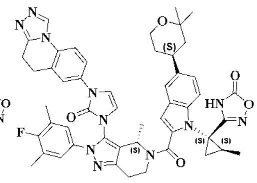 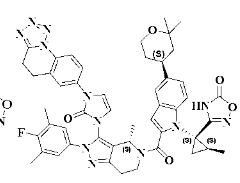 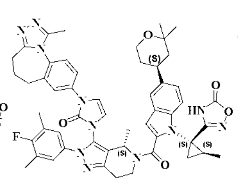 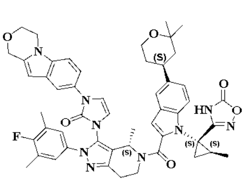

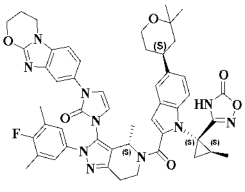 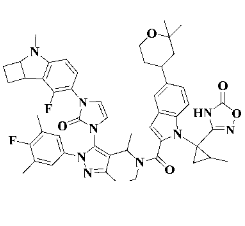 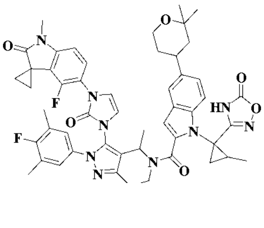 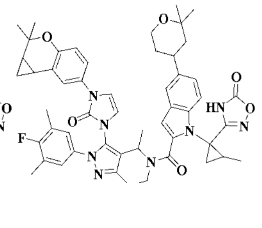

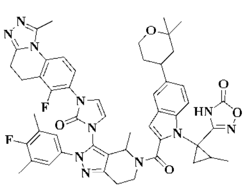 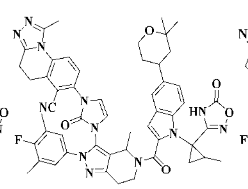 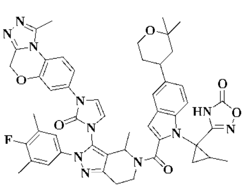 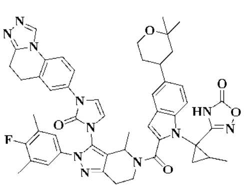 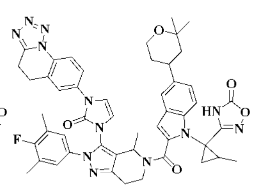

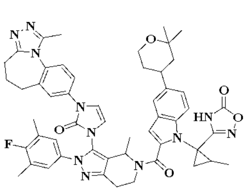 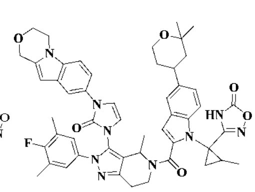 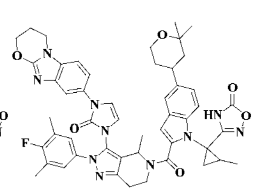 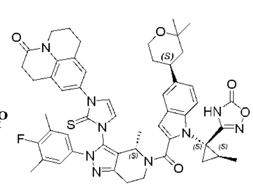 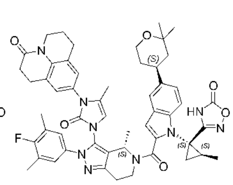

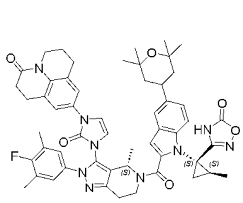 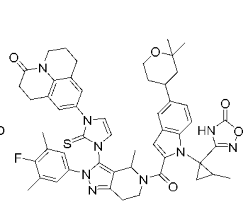 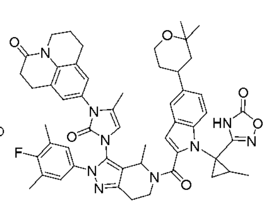 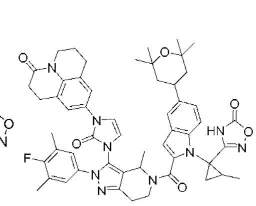 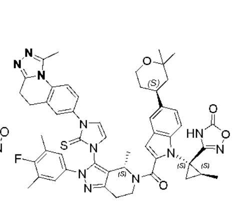

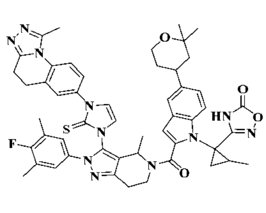
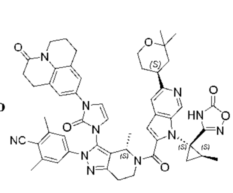
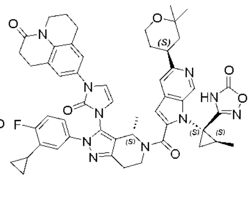
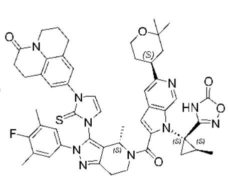
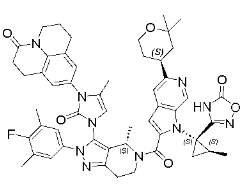

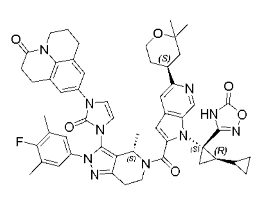
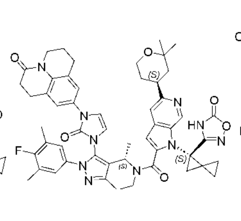
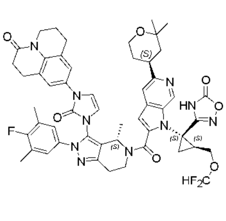
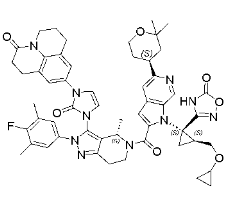
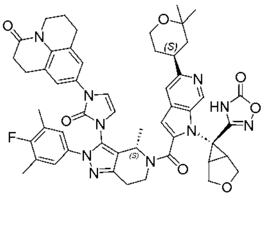

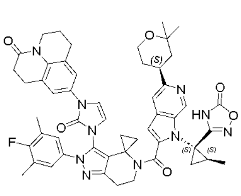
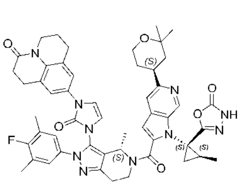
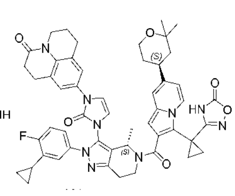
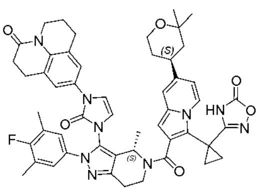
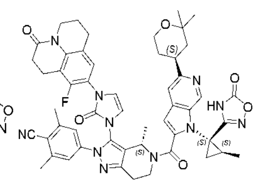

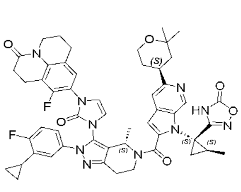
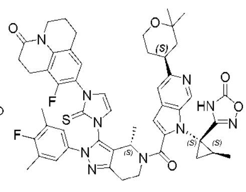
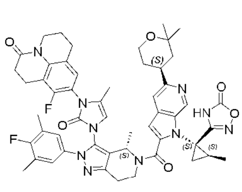
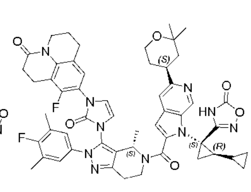
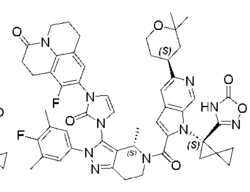

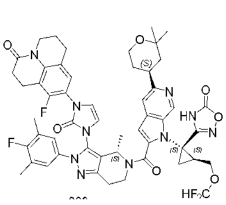
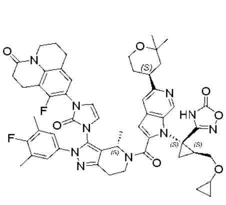
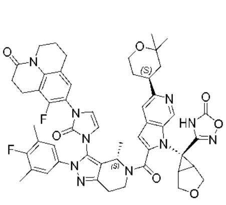
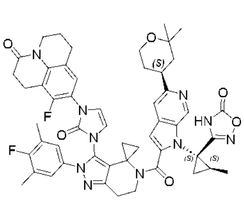
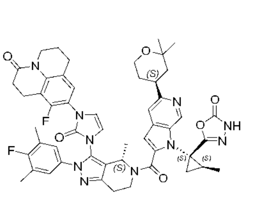

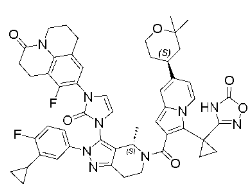
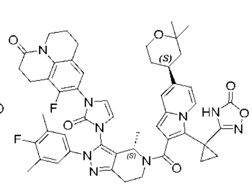
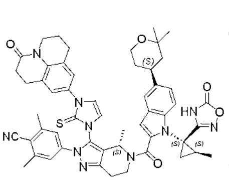
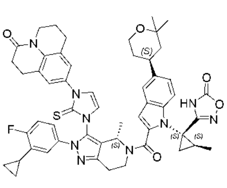
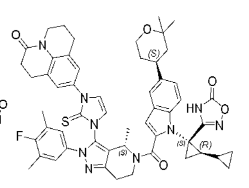

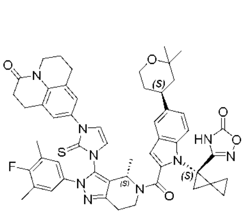
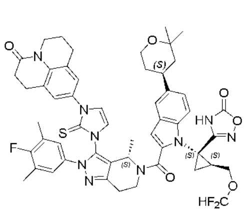
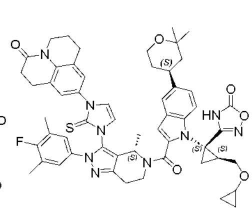
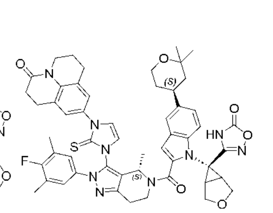

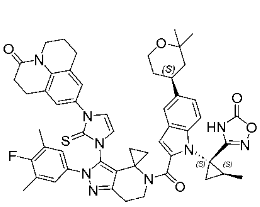
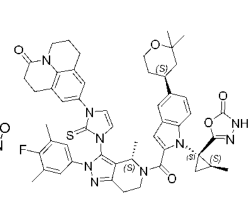
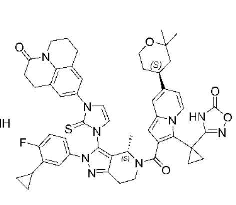
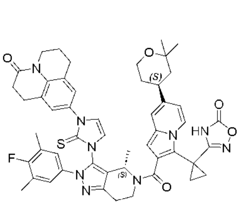
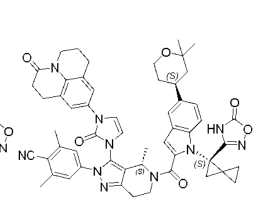

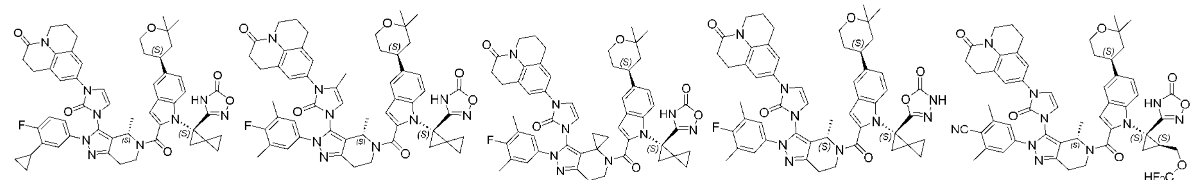

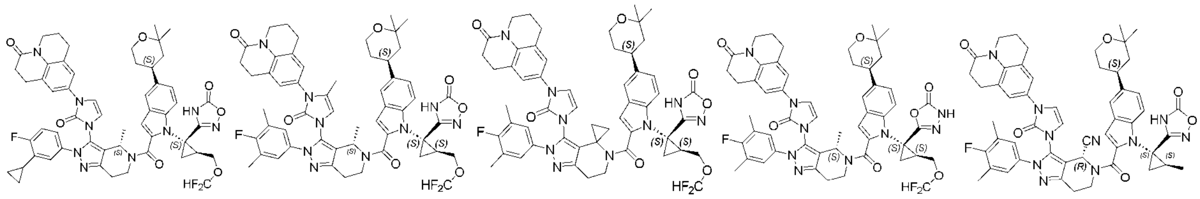

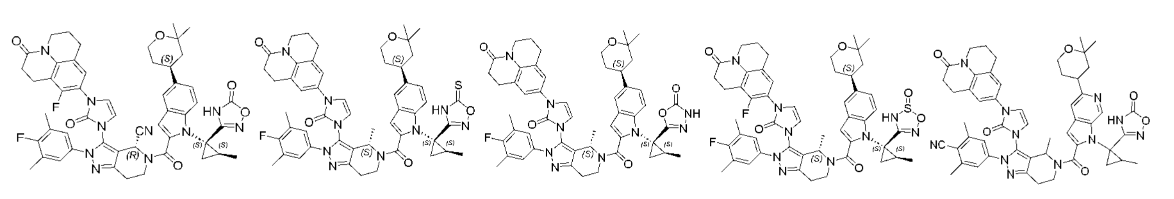

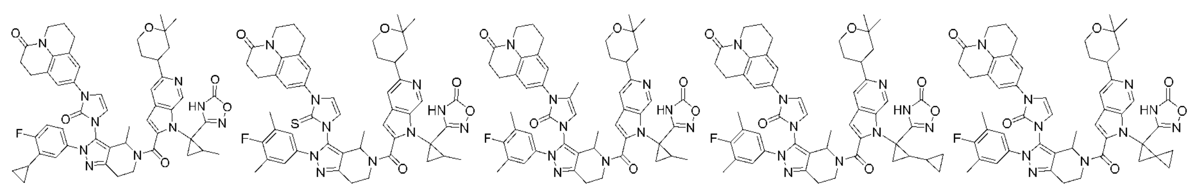

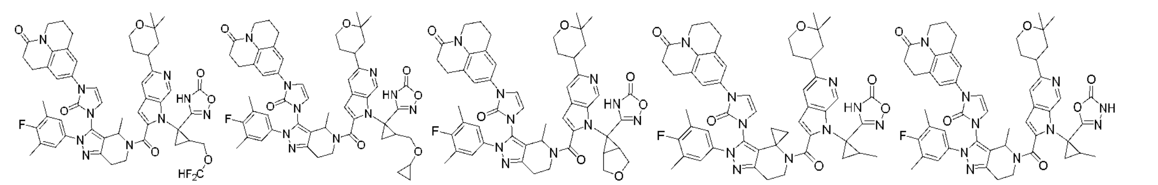

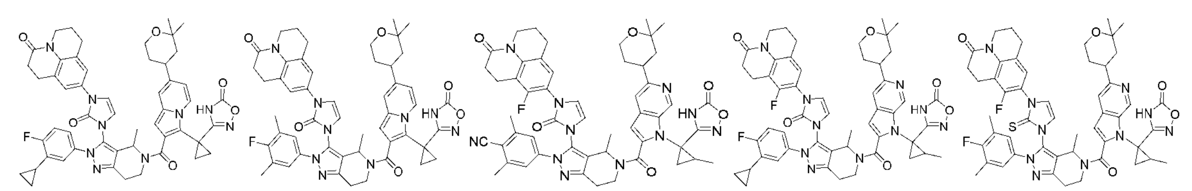

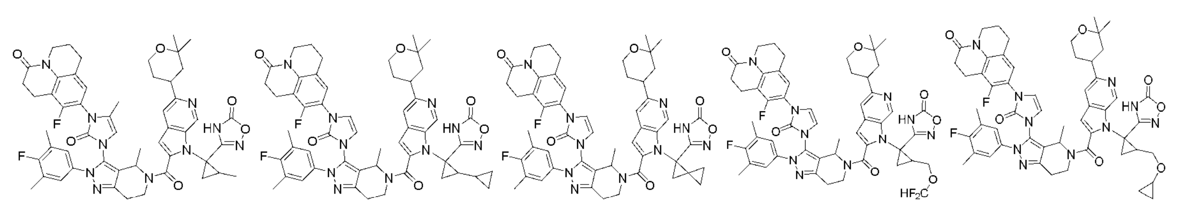

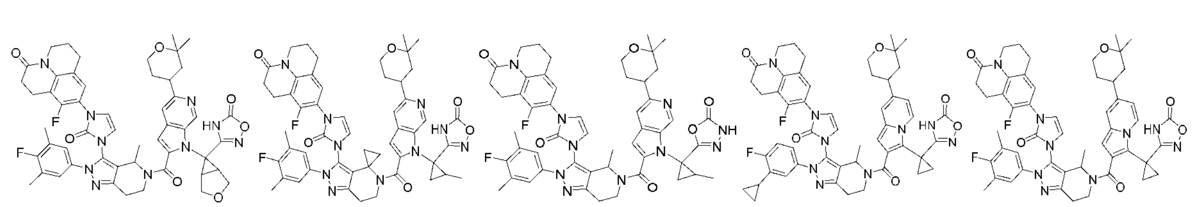

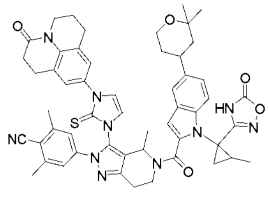 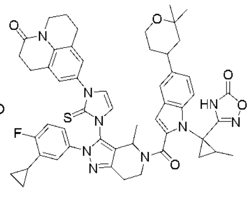 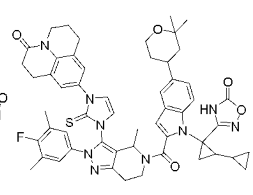 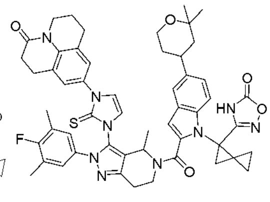

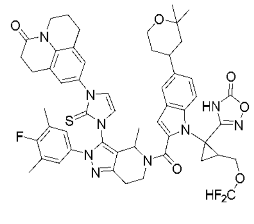 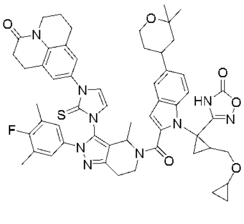 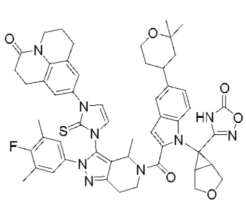 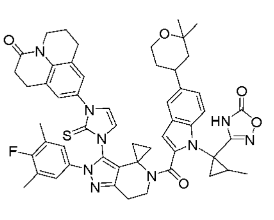

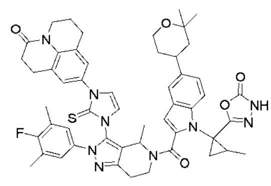 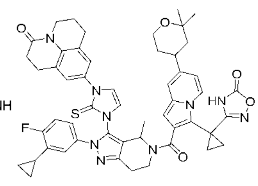 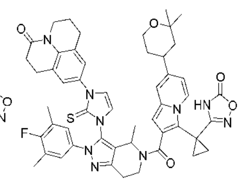 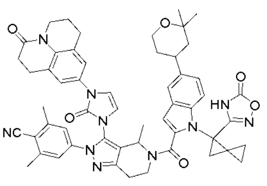

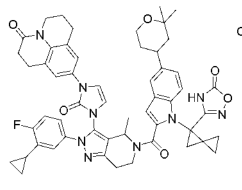 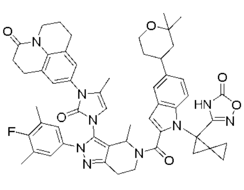 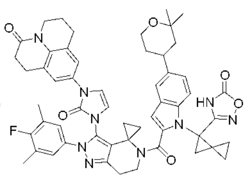 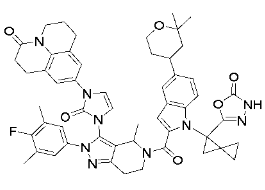

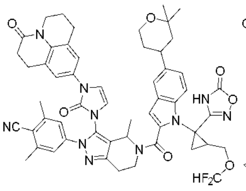 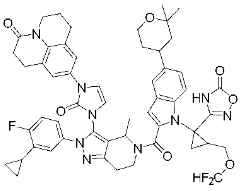 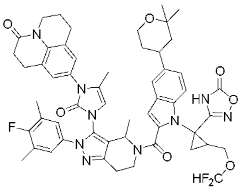 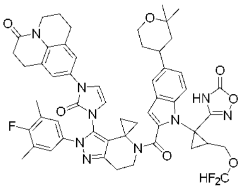 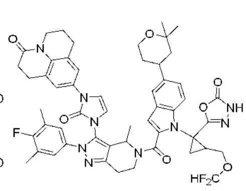

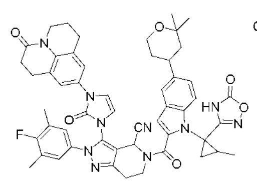 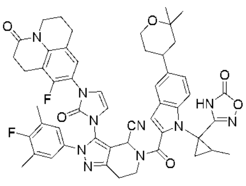 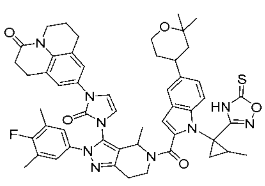 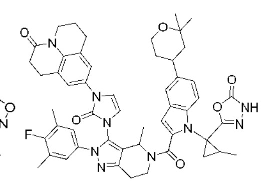

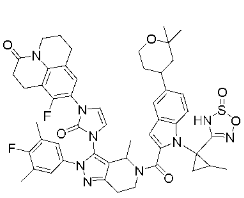 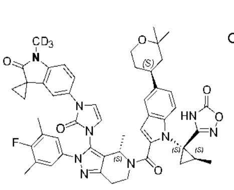 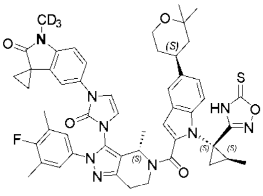 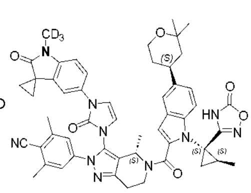 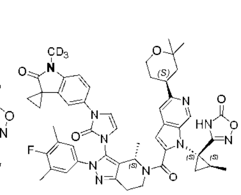

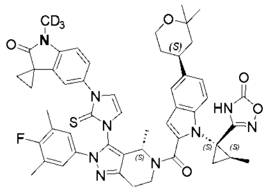 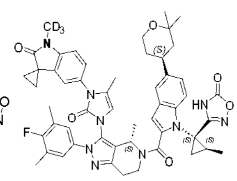 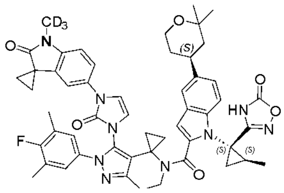 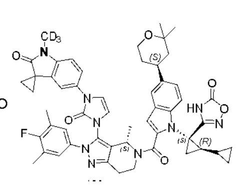

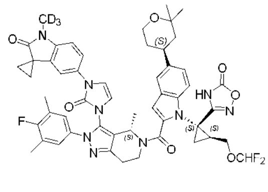 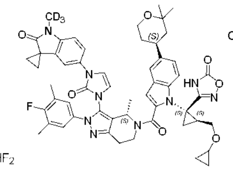 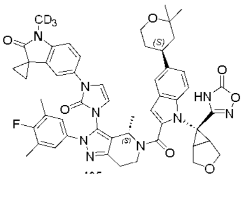 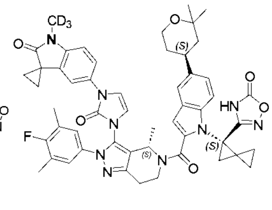 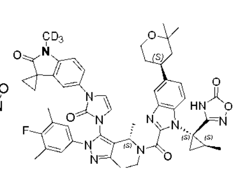

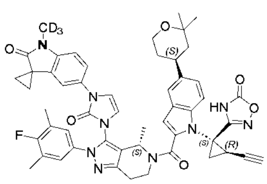 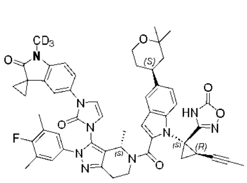 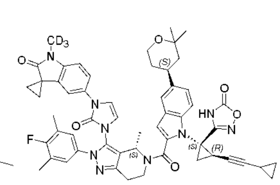 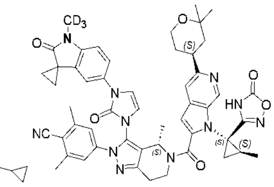

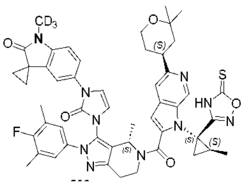 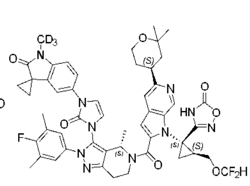 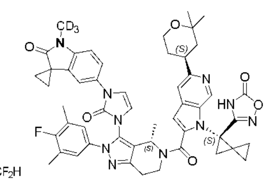 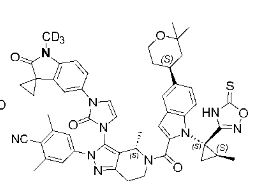

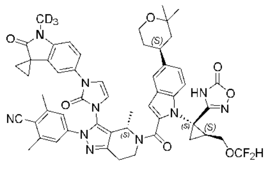 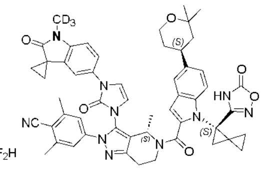 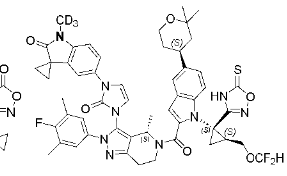 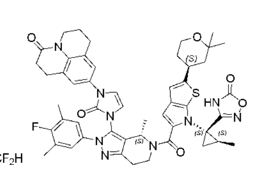

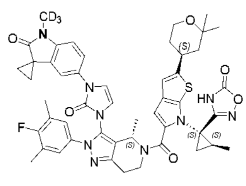 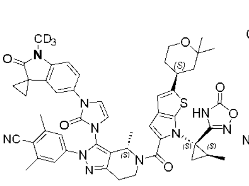 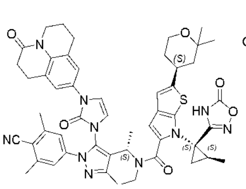 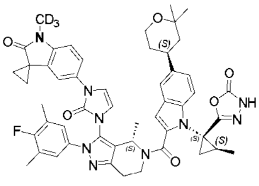 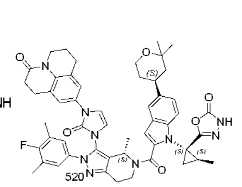
520

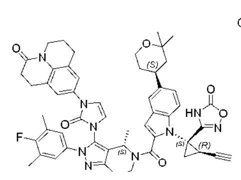 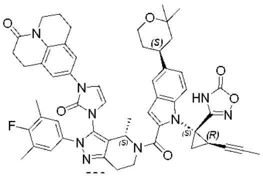 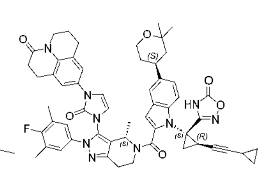 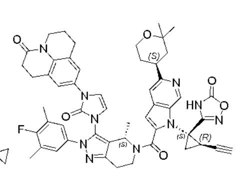

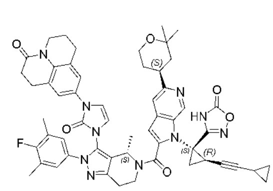 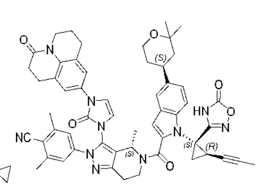 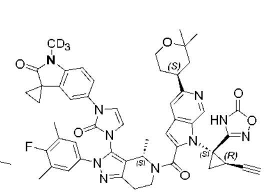 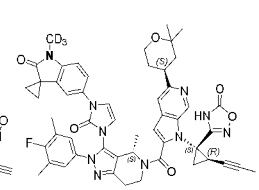

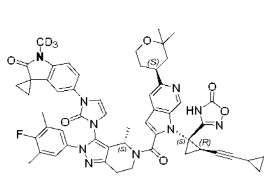
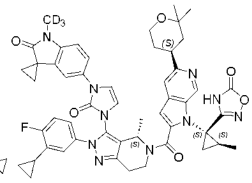
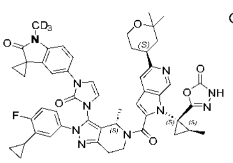
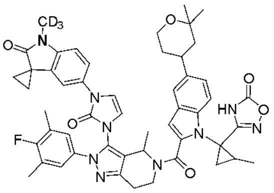

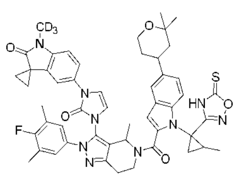
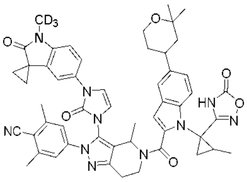
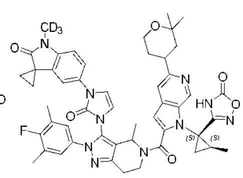
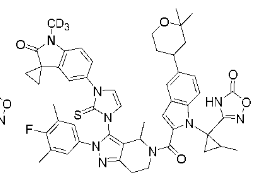
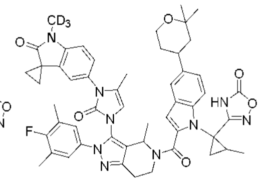

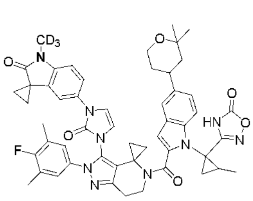
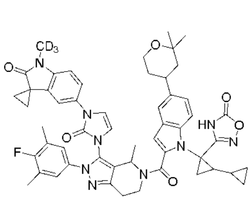
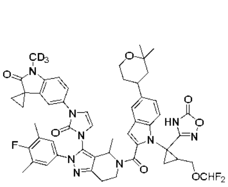
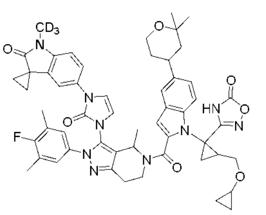

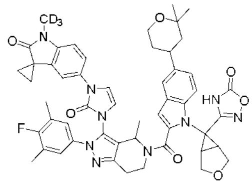
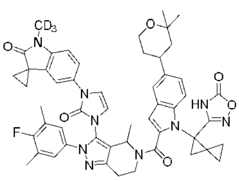
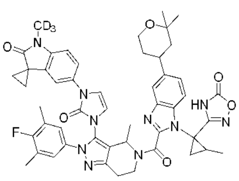
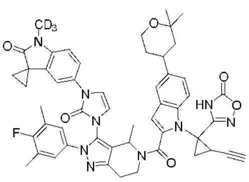

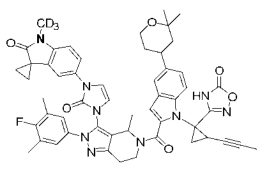
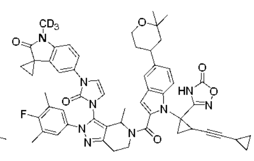
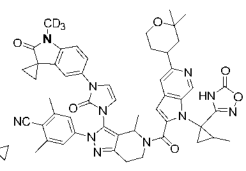
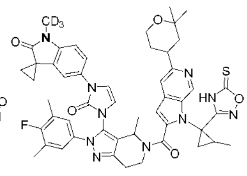
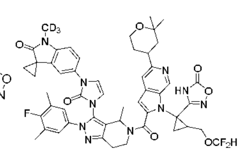

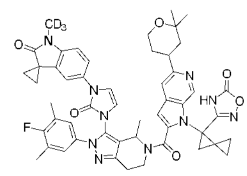
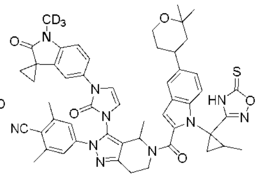
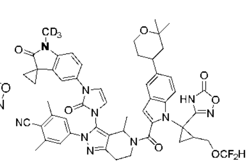
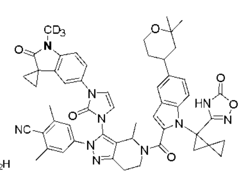

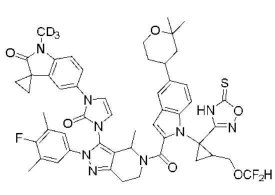
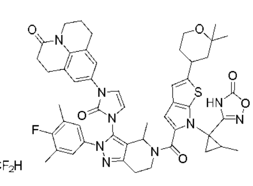
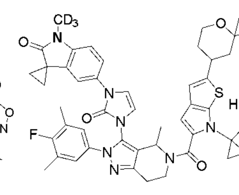
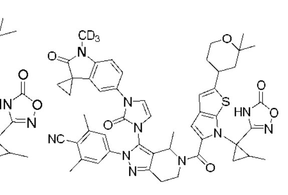

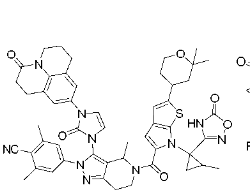
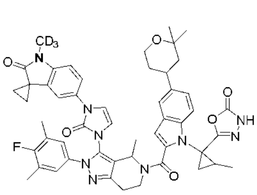
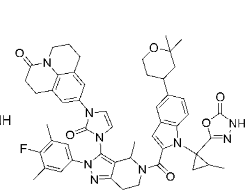
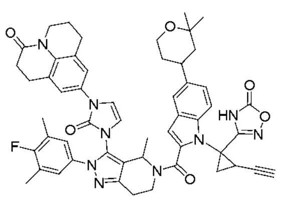

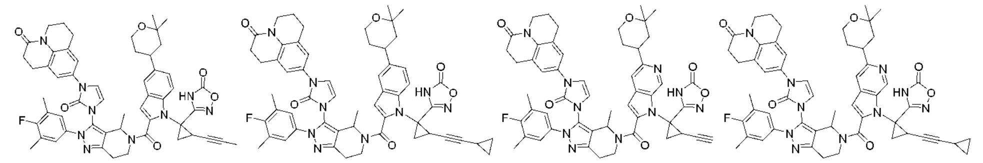

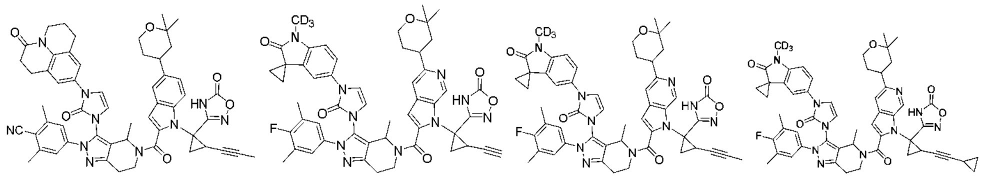

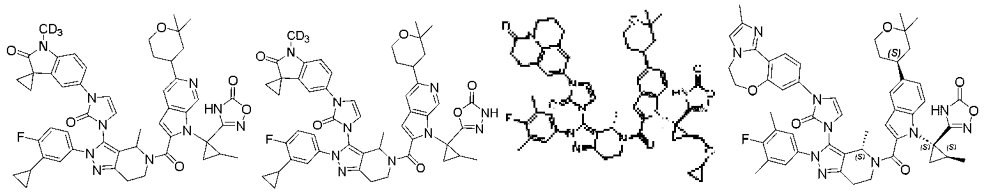

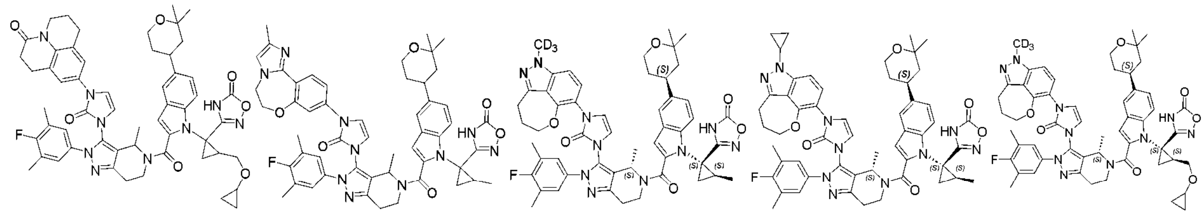

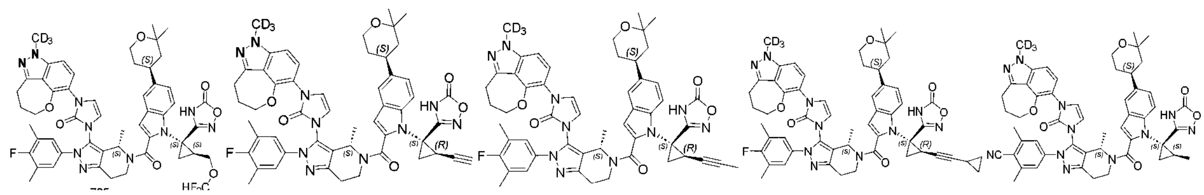

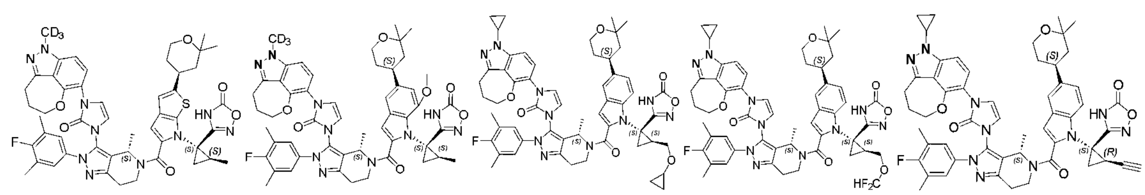

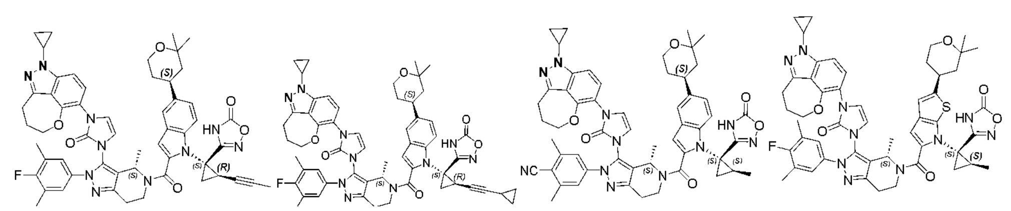

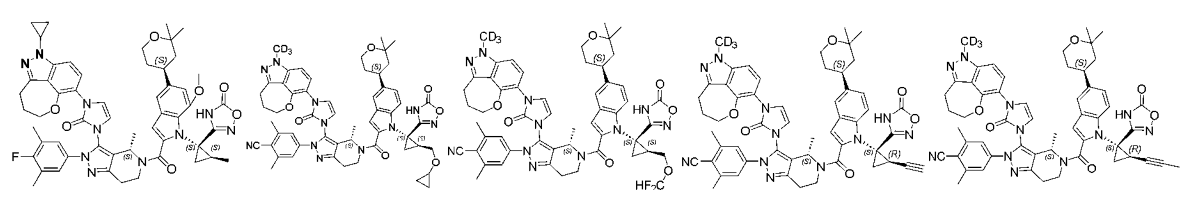

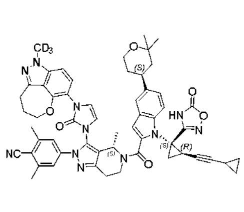 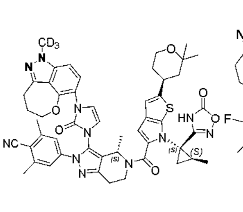 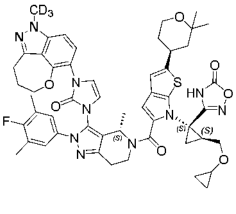 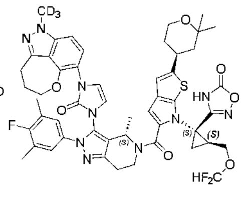 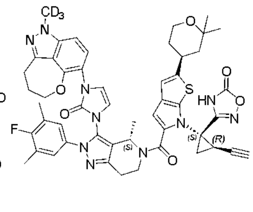

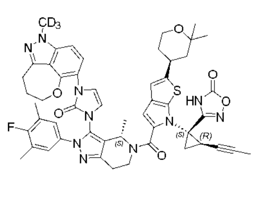 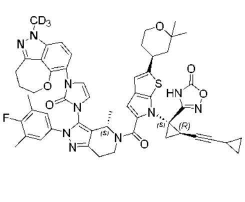 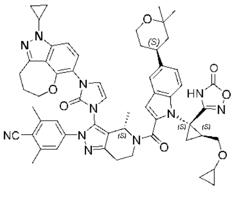 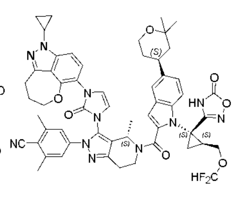 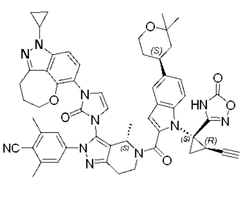

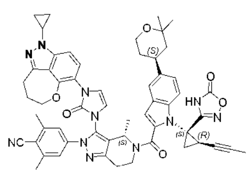 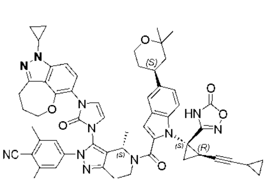 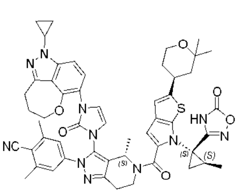 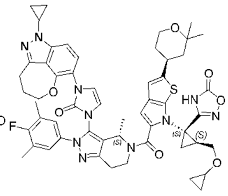 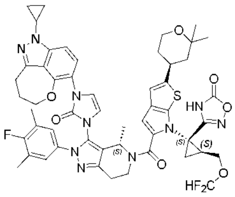

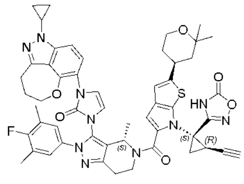 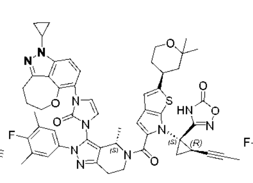 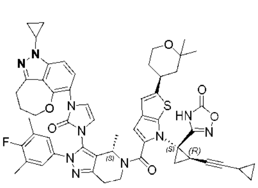 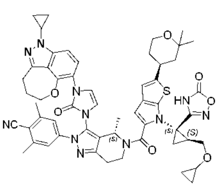 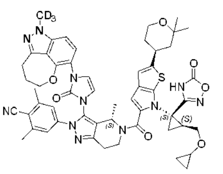

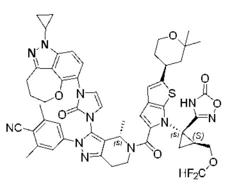 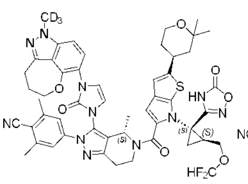 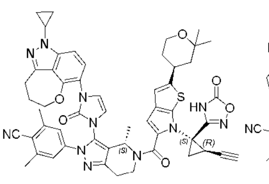 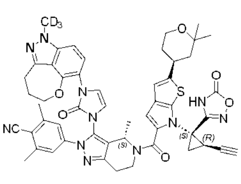 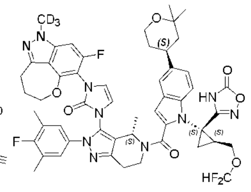

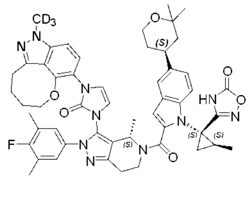 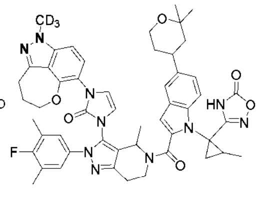 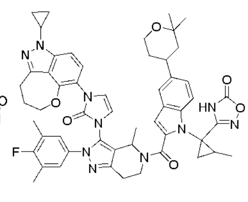 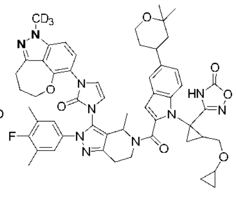 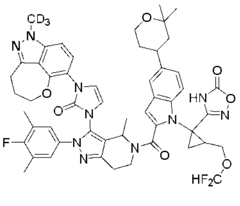

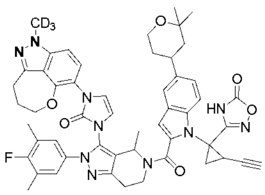 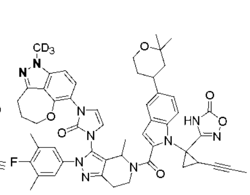 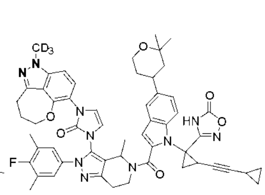 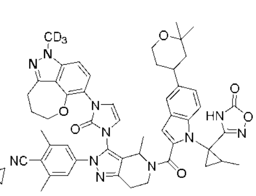

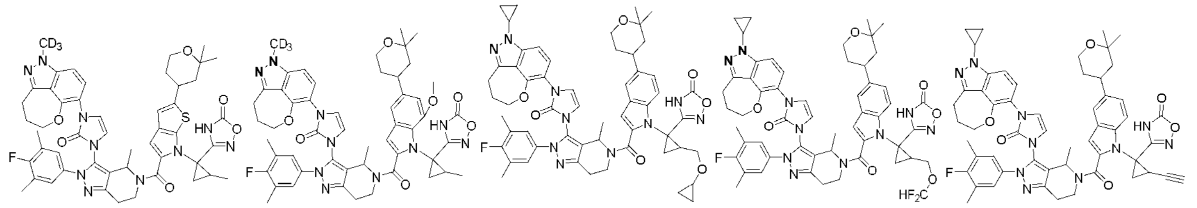

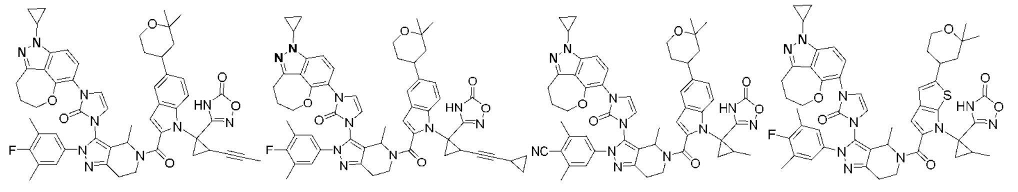

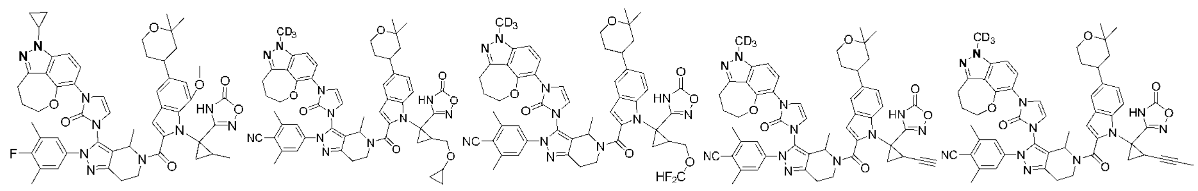

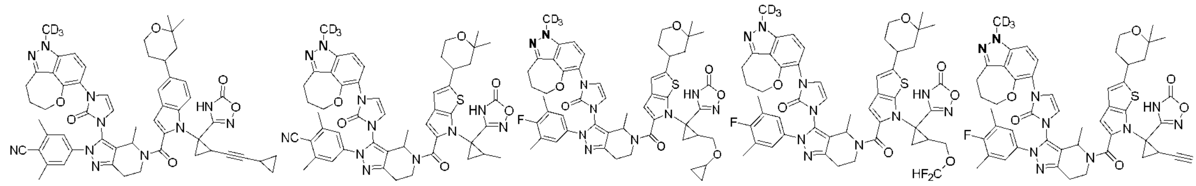

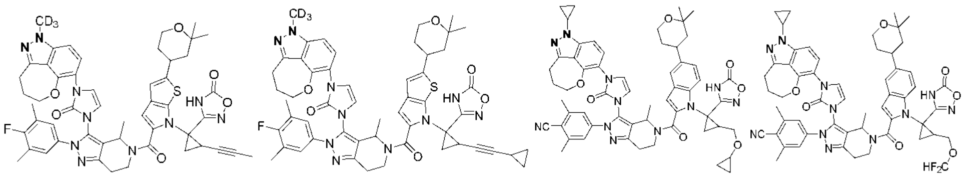

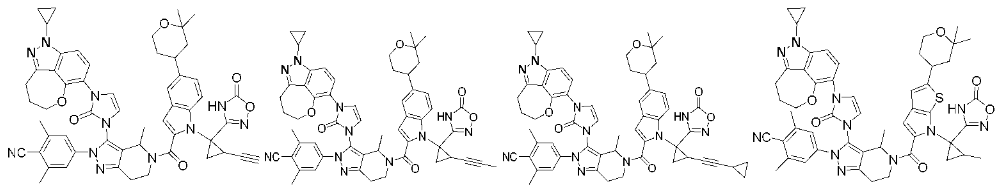

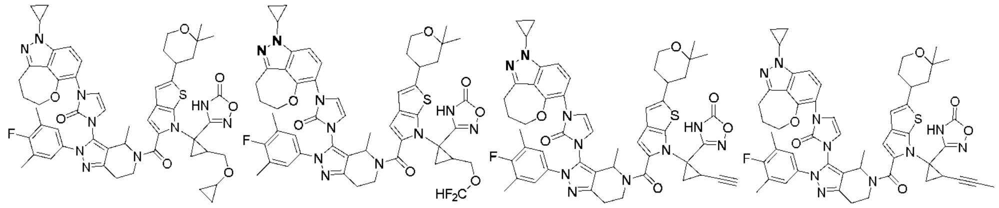

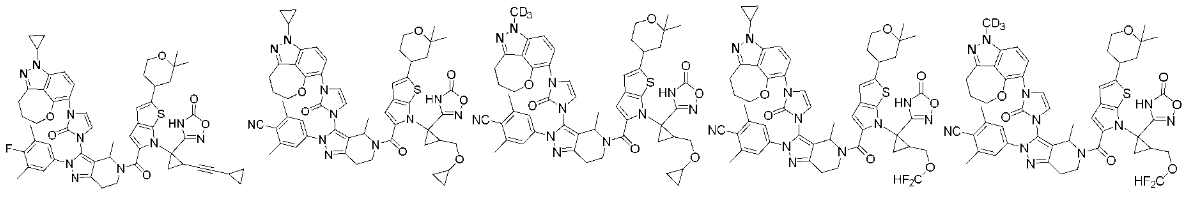

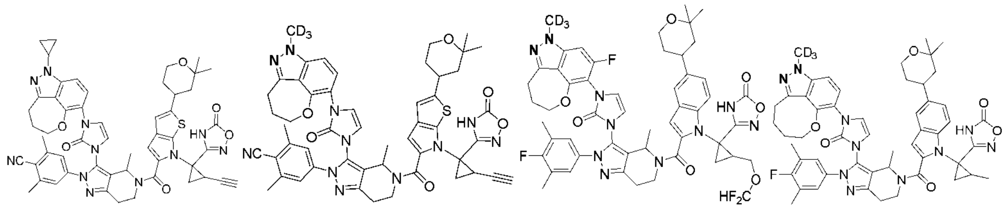

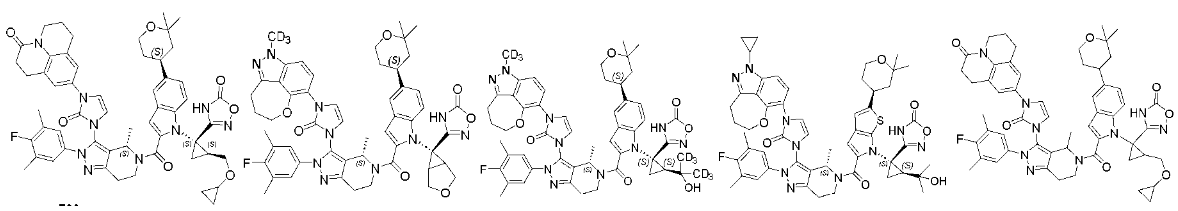

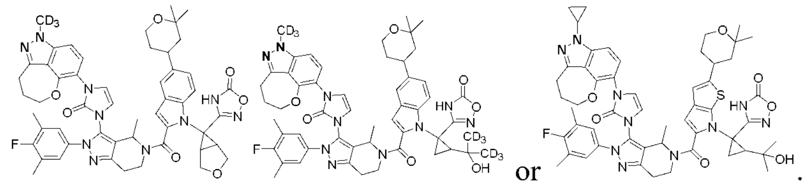

or .

**13.** A compound represented by the general formula (IN-A), (IN-B) or (IN-C), a prodrug thereof, a stereoisomer thereof or a pharmaceutically acceptable salt thereof:

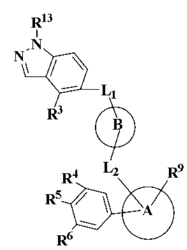

(IN-A)

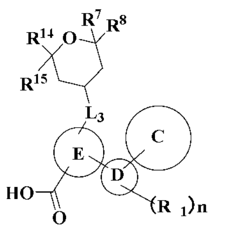

(IN-B)

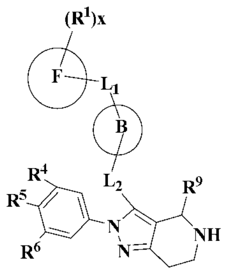

(IN-C)

wherein, in the general formula (IN-A), $R^{13}$ is selected from $C_{3-8}$ cycloalkyl, $C_{6-10}$ aryl, 5-10 membered heteroaryl; the $C_{3-8}$ cycloalkyl, $C_{6-10}$ aryl, 5-10 membered heteroaryl, which are optionally further substituted by one or more substituents selected from deuterium, halogen, amino, nitro, hydroxyl, cyano, oxy, $C_{1-6}$ alkyl; ring A, ring B, $L_1$, $L_2$, $R_3$, $R^4$, $R^5$, $R^6$, $R^9$ are as defined in claim 1;

in the general formula (IN-B), ring D is selected from $C_{3-8}$ bicyclic cycloalkyl, 3-8 membered bicyclic heterocyclic or 5-10 membered bicyclic heteroaryl containing 1-3 atoms selected from N, O or S; ring C, ring E, $L_3$, $R^1$, $R^7$, $R^8$, $R_{14}$, $R_{15}$, n are as defined in claim 1;

in the general formula (IN-C), ring F is selected from 8-14 membered tricyclic fused heterocyclic containing 1-5 heteroatoms; ring B, $L_1$, $L_2$, $R^1$, $R^4$, $R^5$, $R^6$, $R^9$, and x are as defined in claim 8.

**14.** A method for preparing a compound of the general formula as set forth in claims 1-12, **characterized in that** the method comprises the following steps:

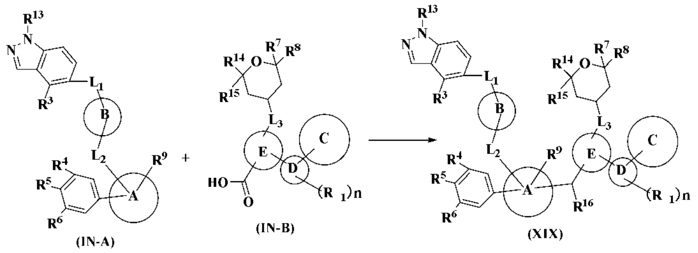

wherein: ring A, ring B, ring C, ring D, ring E, $L_1$, $L_2$, $L_3$, $R^1$, $R^4$, $R^5$, $R^6$, $R^7$, $R^8$, $R^9$, $R^{13}$, $R^{14}$, $R^{15}$, and $R^{16}$ are as defined in claim 1;
general formula (IN-A) and general formula (IN-B) react under the action of a condensing agent and a base to obtain the general formula (XIX);
the condensing agent is selected from EDC, DIC, DCC, TBTU, HATU, HBTU, HCTU, DEPBT, PyBOP or PyAOP, and preferably HATU;
the base is selected from DIPEA, DIEA, Et3N, NMP, DBU or DABCO, and preferably DIPEA;
alternatively, the method comprises the following steps:

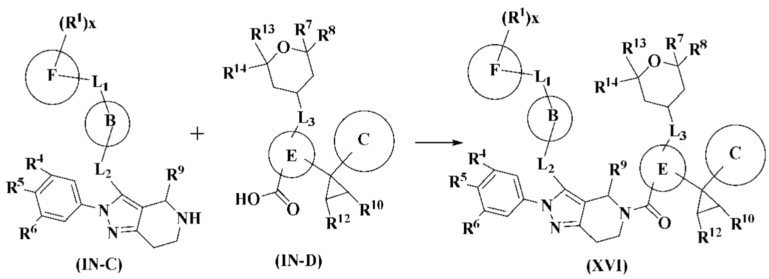

wherein: ring F, ring B, ring C, ring E, $L_1$, $L_2$, $L_3$, $R^1$, $R^4$, $R^5$, $R^6$, $R^7$, $R^8$, $R^9$, $R^{10}$, $R^{12}$, $R^{13}$, $R^{14}$, and x are as described in claim 8;
general formula (IN-C) and general formula (IN-D) react under the action of a condensing agent and a base to obtain the general formula (XVI);
the condensing agent is selected from EDC, DIC, DCC, TBTU, HATU, HBTU, HCTU, DEPBT, PyBOP or PyAOP, and preferably HATU;
the base is selected from DIPEA, DIEA, Et3N, NMP, DBU or DABCO, and preferably DIEA.

**15.** A pharmaceutical composition, comprising a therapeutically effective dose of the compound as set forth in any one of claims 1-12, a prodrug thereof, a stereoisomer thereof or a pharmaceutically acceptable salt thereof, and one or more pharmaceutically acceptable carriers or excipients.

**16.** Use of the compound according to any one of claims 1-12, a prodrug thereof, a stereoisomer thereof or a pharmaceutically acceptable salt thereof, or the pharmaceutical composition according to claim 15 in the preparation of a GLP-1 receptor agonist drug.

**17.** Use of the compound according to any one of claims 1-12, a prodrug thereof, a stereoisomer thereof or a pharmaceutically acceptable salt thereof, or the pharmaceutical composition according to claim 15 in the preparation of a medicament for treating metabolic-related diseases; the disease is selected from diabetes, obesity or non-alcoholic fatty liver disease-related diseases, or other related diseases caused by diabetes, obesity or non-alcoholic fatty liver disease.

**INTERNATIONAL SEARCH REPORT**

International application No.
**PCT/CN2024/108192**

**A. CLASSIFICATION OF SUBJECT MATTER**

C07D471/04(2006.01)i; C07D498/22(2006.01)i; A61K31/437(2006.01)i; A61K31/4709(2006.01)i; A61P1/16(2006.01)i; A61P3/04(2006.01)i; A61P3/10(2006.01)i

According to International Patent Classification (IPC) or to both national classification and IPC

**B. FIELDS SEARCHED**

Minimum documentation searched (classification system followed by classification symbols)

IPC: C07D, A61K, A61P

Documentation searched other than minimum documentation to the extent that such documents are included in the fields searched

Electronic data base consulted during the international search (name of data base and, where practicable, search terms used)

REGISTRY (STN), CAPLUS (STN), VEN, CNTXT, CNKI: 糖尿病, 肥胖, 肝炎, 胰高血糖素样肽, 吡啶并吡唑, 六氢吡喃, 环丙基, diabetes, obesity, hepatitis, hepatic, GLP-1, pyridopyrazole, hexahydropyran, cyclopropyl, STN中的结构式检索, structural formula search in STN

**C. DOCUMENTS CONSIDERED TO BE RELEVANT**

| Category* | Citation of document, with indication, where appropriate, of the relevant passages | Relevant to claim No. |
|---|---|---|
| X | CN 109790161 A (CHUGAI PHARMACEUTICAL CO., LTD.) 21 May 2019 (2019-05-21) abstract, claims 1-13, and description, page 42, embodiments 1-159 | 1-3, 5-6, 8, 10-11, 15-17 |
| Y | CN 109790161 A (CHUGAI PHARMACEUTICAL CO., LTD.) 21 May 2019 (2019-05-21) abstract, claims 1-13, and description, page 42, embodiments 1-159 | 4, 7 |
| A | CN 109790161 A (CHUGAI PHARMACEUTICAL CO., LTD.) 21 May 2019 (2019-05-21) entire document | 9, 12 |
| X | JP 2019099571 A (CHUGAI PHARMACEUTICAL CO., LTD.) 24 June 2019 (2019-06-24) abstract, claims 1-14, and description, embodiments 1-159 | 1-3, 5-6, 8, 10-11, 15-17 |
| X | CN 116390926 A (ECCOGENE (SHANGHAI) CO., LTD.) 04 July 2023 (2023-07-04) abstract, claims 1-62, and description, embodiments 1-103 | 1-3, 5-6, 8, 10-11, 13-17 |
| X | CN 116003403 A (SYNTRON INC.) 25 April 2023 (2023-04-25) abstract, claims 1-8, and description, paragraph 14, and embodiments 1-5 | 1-3, 5-6, 8, 10-11, 13, 15-17 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **14 October 2024** | **22 October 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
PCT/CN2024/108192

C. DOCUMENTS CONSIDERED TO BE RELEVANT

| Category* | Citation of document, with indication, where appropriate, of the relevant passages | Relevant to claim No. |
|---|---|---|
| X | CN 116368140 A (GASHERBRUM BIO, INC.) 30 June 2023 (2023-06-30) abstract, claims 1-182, and description, page 23, embodiments 1-17 | 1-4, 13, 15-17 |
| Y | CN 116368140 A (GASHERBRUM BIO, INC.) 30 June 2023 (2023-06-30) abstract, claims 1-182, and description, page 23, embodiments 1-17 | 4, 7 |
| X | CN 115698003 A (GASHERBRUM BIO, INC.) 03 February 2023 (2023-02-03) abstract, claims 1-258, and description, embodiments 1-9 | 8, 11, 13-17 |
| X | WO 2023016546 A1 (GASHERBRUM BIO, INC. et al.) 16 February 2023 (2023-02-16) abstract, claims 1-270, and description, embodiments 1-7 | 8, 11, 13-17 |
| X | WO 2022048665 A1 (GASHERBRUM BIO, INC. et al.) 10 March 2022 (2022-03-10) abstract, claims 1-204, and description, embodiments 1-2 | 8, 11, 13-17 |
| PX | CN 117069743 A (BEIJING SHENOGEN PHARMACEUTICAL TECHNOLOGY CO., LTD.) 17 November 2023 (2023-11-17) abstract, claims 1-8, and description, embodiments 1-2 | 1-3, 8, 11, 13-17 |
| PX | CN 117447493 A (SYNTRON INC.) 26 January 2024 (2024-01-26) abstract, claims 1-8, and description, paragraph 12, and embodiments 1-5 | 1-3, 5-6, 8, 11, 13-17 |
| PX | WO 2023169456 A1 (GASHERBRUM BIO, INC. et al.) 14 September 2023 (2023-09-14) abstract, claims 1-36, and description, embodiments 1-6 | 8, 11, 13, 15-17 |
| E | WO 2024169952 A1 (GASHERBRUM BIO, INC. et al.) 22 August 2024 (2024-08-22) description, pages 99-141, compounds 101, 103-139, 142-152, 155-156, 160, 164, 166-169, and 172-238, in particular, compound 101 | 8-11, 13-17 |

Form PCT/ISA/210 (second sheet) (July 2022)

**INTERNATIONAL SEARCH REPORT**
**Information on patent family members**

International application No.
**PCT/CN2024/108192**

| Patent document cited in search report | Publication date (day/month/year) | Patent family member(s) | Publication date (day/month/year) |
|---|---|---|---|
| CN 109790161 A | 21 May 2019 | PE 20190709 A1 | 17 May 2019 |
| | | IL 287166 A | 01 December 2021 |
| | | TW 201827426 A | 01 August 2018 |
| | | TWI 753946 B | 01 February 2022 |
| | | CR 20190149 A | 27 August 2019 |
| | | MX 2019003488 A | 16 December 2019 |
| | | UA 125586 C2 | 27 April 2022 |
| | | BR 112019005322 A2 | 02 July 2019 |
| | | EP 3517538 A1 | 31 July 2019 |
| | | EP 3517538 A4 | 10 June 2020 |
| | | EP 3517538 B1 | 13 March 2024 |
| | | DK 3517538 T3 | 25 March 2024 |
| | | PL 3517538 T3 | 10 June 2024 |
| | | HRP 20240499 T1 | 05 July 2024 |
| | | SG 11201901565 VA | 28 March 2019 |
| | | CO 2019002595 A2 | 29 March 2019 |
| | | AU 2017330733 A1 | 14 March 2019 |
| | | AU 2017330733 B2 | 28 May 2020 |
| | | MX 2022004071 A | 03 May 2022 |
| | | TN 2019000054 A1 | 15 July 2020 |
| | | HUE 066583 T2 | 28 August 2024 |
| | | US 2019225604 A1 | 25 July 2019 |
| | | US 10858356 B2 | 08 December 2020 |
| | | WO 2018056453 A1 | 29 March 2018 |
| | | FI 3517538 T3 | 20 May 2024 |
| | | IL 264945 A | 30 May 2019 |
| | | IL 264945 B | 31 October 2021 |
| | | US 2023382912 A1 | 30 November 2023 |
| | | US 2021017176 A1 | 21 January 2021 |
| | | US 11814381 B2 | 14 November 2023 |
| | | EA 201990518 A1 | 30 December 2019 |
| | | EA 037989 B1 | 21 June 2021 |
| | | NZ 751725 A | 26 March 2021 |
| | | PT 3517538 T | 09 April 2024 |
| | | AU 2022205222 A1 | 04 August 2022 |
| | | AU 2022205222 B2 | 04 April 2024 |
| | | RS 65398 B1 | 30 April 2024 |
| | | AU 2024201884 A1 | 11 April 2024 |
| | | KR 20190039591 A | 12 April 2019 |
| | | KR 102223227 B1 | 08 March 2021 |
| | | JP 2023100963 A | 19 July 2023 |
| | | JPWO 2018056453 A1 | 11 July 2019 |
| | | JP 6567778 B2 | 28 August 2019 |
| | | SA 519401409 B1 | 07 April 2022 |
| | | EP 4134367 A1 | 15 February 2023 |
| | | US 2024246971 A1 | 25 July 2024 |
| | | JOP 20190060 A1 | 26 March 2019 |
| | | CL 2019000663 A1 | 26 July 2019 |
| | | ECSP 19020228 A | 30 June 2019 |
| | | EA 202190802 A2 | 30 July 2021 |

Form PCT/ISA/210 (patent family annex) (July 2022)

**INTERNATIONAL SEARCH REPORT**
**Information on patent family members**

International application No.
**PCT/CN2024/108192**

| Patent document cited in search report | Publication date (day/month/year) | Patent family member(s) | Publication date (day/month/year) |
|---|---|---|---|
| | | EA 202190802 A3 | 29 October 2021 |
| | | EP 4349840 A2 | 10 April 2024 |
| | | EP 4349840 A3 | 10 July 2024 |
| | | AU 2020223687 A1 | 10 September 2020 |
| | | AU 2020223687 B2 | 14 April 2022 |
| | | CA 3038479 A1 | 29 March 2018 |
| | | CA 3038479 C | 22 June 2021 |
| | | JP 2019203015 A | 28 November 2019 |
| | | JP 6957564 B2 | 02 November 2021 |
| | | SI 3517538 T1 | 31 May 2024 |
| | | LT 3517538 T | 10 May 2024 |
| | | DOP 2019000074 A | 31 May 2019 |
| | | MA 46286 A | 31 July 2019 |
| | | US 2024262824 A1 | 08 August 2024 |
| | | JP 2022003087 A | 11 January 2022 |
| | | JP 7280929 B2 | 24 May 2023 |
| | | ZA 201901070 B | 30 June 2021 |
| | | PH 12019500659 A1 | 16 December 2019 |
| JP 2019099571 A | 24 June 2019 | JP 2024079762 A | 11 June 2024 |
| | | JP 7461104 B2 | 03 April 2024 |
| CN 116390926 A | 04 July 2023 | BR 112023000932 A2 | 03 October 2023 |
| | | EP 4182020 A1 | 24 May 2023 |
| | | IL 299704 A | 01 March 2023 |
| | | JP 2023534983 A | 15 August 2023 |
| | | CO 2023001407 A2 | 16 February 2023 |
| | | WO 2022017338 A1 | 27 January 2022 |
| | | AU 2021311567 A1 | 16 February 2023 |
| | | CA 3186217 A1 | 27 January 2022 |
| | | MX 2023000943 A | 22 February 2023 |
| | | US 2023051320 A1 | 16 February 2023 |
| | | US 12037339 B2 | 16 July 2024 |
| | | KR 20230048056 A | 10 April 2023 |
| | | CL 2023000196 A1 | 15 September 2023 |
| | | PE 20230854 A1 | 29 May 2023 |
| | | US 11584751 B1 | 21 February 2023 |
| CN 116003403 A | 25 April 2023 | None | |
| CN 116368140 A | 30 June 2023 | EP 4211139 A1 | 19 July 2023 |
| | | JP 2023540609 A | 25 September 2023 |
| | | US 2023331732 A1 | 19 October 2023 |
| | | WO 2022052958 A1 | 17 March 2022 |
| CN 115698003 A | 03 February 2023 | DOP 2022000158 A | 30 September 2022 |
| | | US 2022213130 A1 | 07 July 2022 |
| | | US 11492365 B2 | 08 November 2022 |
| | | PE 20231066 A1 | 17 July 2023 |
| | | AU 2021216324 A1 | 25 August 2022 |
| | | US 2023174565 A1 | 08 June 2023 |
| | | US 11926643 B2 | 12 March 2024 |
| | | FI 4097099 T3 | 30 July 2024 |
| | | KR 20220156535 A | 25 November 2022 |

Form PCT/ISA/210 (patent family annex) (July 2022)

**INTERNATIONAL SEARCH REPORT**
**Information on patent family members**

International application No.
**PCT/CN2024/108192**

| Patent document cited in search report | | | Publication date (day/month/year) | Patent family member(s) | | | Publication date (day/month/year) |
|---|---|---|---|---|---|---|---|
| | | | | EP | 4097099 | A1 | 07 December 2022 |
| | | | | EP | 4097099 | A4 | 27 September 2023 |
| | | | | EP | 4097099 | B1 | 26 June 2024 |
| | | | | CL | 2022002105 | A1 | 17 March 2023 |
| | | | | CR | 20220371 | A | 27 October 2022 |
| | | | | MX | 2022009524 | A | 11 January 2023 |
| | | | | CO | 2022011065 | A2 | 31 October 2022 |
| | | | | CA | 3169975 | A1 | 12 August 2021 |
| | | | | WO | 2021155841 | A1 | 12 August 2021 |
| | | | | DK | 4097099 | T3 | 15 July 2024 |
| | | | | PT | 4097099 | T | 19 July 2024 |
| | | | | JP | 2023513272 | A | 30 March 2023 |
| | | | | IL | 295281 | A | 01 October 2022 |
| | | | | TW | 202142538 | A | 16 November 2021 |
| WO | 2023016546 | A1 | 16 February 2023 | None | | | |
| WO | 2022048665 | A1 | 10 March 2022 | US | 2023322771 | A1 | 12 October 2023 |
| | | | | JP | 2023540558 | A | 25 September 2023 |
| | | | | EP | 4211133 | A1 | 19 July 2023 |
| CN | 117069743 | A | 17 November 2023 | None | | | |
| CN | 117447493 | A | 26 January 2024 | None | | | |
| WO | 2023169456 | A1 | 14 September 2023 | None | | | |
| WO | 2024169952 | A1 | 22 August 2024 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 202096871 A1
- WO 202176890 A1
- EP 32904192018 A1


### Non-patent literature cited in the description


- *Bioorganic and Medicinal Chemistry Letters*, 2021, vol. 47
- *Tetrahedron*, 2015, vol. 71 (26568), 6499-6505
- *European Journal of Medicinal Chemistry*, 2020, 190
- *Chemistry A European Journal*, 2022, vol. 28 (E202103135)
- *European Journal of Medicinal Chemistry*, 2020, vol. 190
- *Organic Letters*, 2007, vol. 9, 525-528
- *Bioorganic and Medicinal Chemistry Letters*, 2004, vol. 14, 2451-2457
- *Tetrahedron Letters*, 2018, vol. 59, 1501-1505
- *Journal of Organic Chemistry*, 2014, vol. 79, 1368-1376
- *European Journal of Medicinal Chemistry*, 2015, vol. 90, 788-796
- *Chemical Communications*, 2016, vol. 52 (8), 1665-1668
- *Journal of Organic Chemistry*, 1964, vol. 29, 11-15
- *Journal of Organic Chemistry*, 2017, vol. 82 (16), 8604-8610